# EUROPEAN PATENT SPECIFICATION

(11) **EP 3 728 285 B1**
(45) Date of publication and mention of the grant of the patent: **08.04.2026**
(21) Application number: 18839992.7
(22) Date of filing: 21.12.2018
(51) Int. Cl.: C07J 63/00, C07J 61/00, A61K 31/57, A61P 25/00

(54) **COMPOSITIONS AND METHODS FOR TREATING CNS DISORDERS**
ZUSAMMENSETZUNGEN UND VERFAHREN ZUR BEHANDLUNG VON STÖRUNGEN DES ZENTRALEN NERVENSYSTEMS
COMPOSITIONS ET MÉTHODES PERMETTANT DE TRAITER LES TROUBLES DU SNC

(30) Priority: 22.12.2017 US 201762610067 P; 29.12.2017 US 201762612164 P; 29.12.2017 US 201762612067 P; 29.12.2017 US 201762611977 P; 29.12.2017 US 201762612070 P; 17.08.2018 US 201862765164 P; 07.09.2018 US 201862728499 P; 27.09.2018 US 201862737559 P; 02.11.2018 US 201862754977 P
(43) Date of publication of application: 28.10.2020
(73) Proprietor: Sage Therapeutics, LLC, Rockville, MD 20850 (US)
(72) Inventor: ROBICHAUD, Albert, Jean, Boston, MA 02110 (US); SALITURO, Francesco, G., Marlborough, MA 01752 (US); BLANCO-PILLADO, Maria, Jesus, Arlington, MA 02474 (US); LA, Daniel, Randolph Road Chestnut Hill, MA 02467 (US); HARRISON, Boyd, L., Princeton Junction, NJ 08550 (US)
(74) Representative: Coles, Andrea Birgit
(86) International application number: PCT/US2018/067306
(87) International publication number: WO 2019/126761

(56) References cited:
- EP-A1- 3 909 967
- WO-A1-2014/169833
- WO-A1-2016/061527
- WO-A1-98/05337
- GB-A- 1 380 246
- GB-A- 1 494 097
- GB-A- 1 538 869
- GB-A- 1 570 394
- GB-A- 1 581 234
- GB-A- 1 581 235
- ADRIANA VELEIRO ET AL: "Structure-Activity Relationships of Neuroactive Steroids Acting on the GABAA Receptor", CURRENT MEDICINAL CHEMISTRY, vol. 16, no. 4, 1 February 2009 (2009-02-01), NL, pages 455 - 472, XP055570602, ISSN: 0929-8673, DOI: 10.2174/092986709787315522
- DI CHENNA P. ET AL: "Synthesis of D-Homo Analogs of Neurosteroids", MOLECULES, vol. 5, no. 3, 1 January 2000 (2000-01-01), CH, pages 447 - 448, XP093243573, ISSN: 1420-3049, DOI: 10.3390/50300447
- PHILLIPPS G H ET AL: "Water-soluble steroidal anaesthetics", JOURNAL OF STEROID BIOCHEMISTRY, PERGAMON PRESS PLC, GB, vol. 11, no. 1, 1 July 1979 (1979-07-01), pages 79 - 86, XP023413173, ISSN: 0022-4731, [retrieved on 19790701], DOI: 10.1016/0022-4731(79)90279-6
- SHINJI ITOH ET AL: "On the Acid-Catalyzed D-Homoannulation of Pregnanetriol 20-Sulfate and Its C-20 Isomeric Sulfate.", CHEMICAL AND PHARMACEUTICAL BULLETIN, vol. 42, no. 9, 1 January 1994 (1994-01-01), JP, pages 1736 - 1744, XP055569824, ISSN: 0009-2363, DOI: 10.1248/cpb.42.1736
- E.L. RONGONE ET AL: "In vivo metabolism of D-homotestosterone", STEROIDS, vol. 1, no. 6, 1 June 1963 (1963-06-01), US, pages 664 - 669, XP055569823, ISSN: 0039-128X, DOI: 10.1016/S0039-128X(63)80100-2
- PHILLIPPS G H: "STRUCTURE-ACTIVITY RELATIONSHIPS IN STEROIDAL ANAESTHETICS", NOL. MECH. GEN. ANAESTH. GLAXO SYMPOSIUM, XX, XX, 1 January 1974 (1974-01-01), pages 32 - 47, XP000600765
- BRYSON W. KATONA ET AL: "Neurosteroid analogues. 12. Potent enhancement of GABA-mediated chloride currents at GABAA receptors by ent-androgens", EUROPEAN JOURNAL OF MEDICINAL CHEMISTRY, vol. 43, no. 1, 1 January 2008 (2008-01-01), FR, pages 107 - 113, XP055303995, ISSN: 0223-5234, DOI: 10.1016/j.ejmech.2007.02.017
- MARIANGELA CHISARI ET AL: "The Influence of Neuroactive Steroid Lipophilicity on GABA A Receptor Modulation: Evidence for a Low-Affinity Interaction", JOURNAL OF NEUROPHYSIOLOGY, vol. 102, no. 2, 24 June 2009 (2009-06-24), US, pages 1254 - 1264, XP055570040, ISSN: 0022-3077, DOI: 10.1152/jn.00346.2009
- H.-J. SHU ET AL: "Photodynamic Effects of Steroid-Conjugated Fluorophores on GABAA Receptors", MOLECULAR PHARMACOLOGY, vol. 76, no. 4, 1 October 2009 (2009-10-01), US, pages 754 - 765, XP055304002, ISSN: 0026-895X, DOI: 10.1124/mol.109.057687

## Description

### Cross-Reference to Related Applications

This application claims priority to U.S. Provisional Application Nos. 62/610,067 filed December 22, 2017, 62/611,977 filed December 29, 2017, 62/765,164 filed August 17, 2018, 62/612,067 filed December 29, 2017, 62/728,499 filed September 7, 2018, 62/754,977 filed November 2, 2018, 62/612,070 filed December 29, 2017, 62/612,164 filed December 29, 2017, 62/737,559 filed September 27, 2018.

### Background of the Invention

Brain excitability is defined as the level of arousal of an animal, a continuum that ranges from coma to convulsions, and is regulated by various neurotransmitters. In general, neurotransmitters are responsible for regulating the conductance of ions across neuronal membranes. At rest, the neuronal membrane possesses a potential (or membrane voltage) of approximately -70 mV, the cell interior being negative with respect to the cell exterior. The potential (voltage) is the result of ion (K⁺, Na⁺, Cl⁻, organic anions) balance across the neuronal semipermeable membrane. Neurotransmitters are stored in presynaptic vesicles and are released under the influence of neuronal action potentials. When released into the synaptic cleft, a change of potential occurs from -70 mV to -50 mV. This effect is mediated by postsynaptic nicotinic receptors which are stimulated by acetylcholine to increase membrane permeability to Na⁺ ions. The reduced membrane potential stimulates neuronal excitability in the form of a postsynaptic action potential.

**In** the case of the GABA receptor complex (GRC), the effect on brain excitability is mediated by γ-aminobutyric acid (GABA), a neurotransmitter. GABA has a profound influence on overall brain excitability because up to 40% of the neurons in the brain utilize GABA as a neurotransmitter. GABA regulates the excitability of individual neurons by regulating the conductance of chloride ions across the neuronal membrane. GABA interacts with its recognition site on the GRC to facilitate the flow of chloride ions down an electrochemical gradient of the GRC into the cell. An intracellular increase in the levels of this anion causes hyperpolarization of the transmembrane potential, rendering the neuron less susceptible to excitatory inputs, *i.e.,* reduced neuron excitability. In other words, the higher the chloride ion concentration in the neuron, the lower the brain excitability and level of arousal.

It is well-documented that the GRC is responsible for the mediation of anxiety, seizure activity, and sedation. Thus, GABA and drugs that act like GABA or facilitate the effects of GABA *(e.g.,* the therapeutically useful barbiturates and benzodiazepines (BZs), such as Valium^{®}) produce their therapeutically useful effects by interacting with specific regulatory sites on the GRC. Accumulated evidence has now indicated that in addition to the benzodiazepine and barbiturate binding site, the GRC contains a distinct site for neuroactive steroids. See, *e.g.,* Lan, N. C. et al., Neurochem. Res. (1991) 16:347-356.

Neuroactive steroids can occur endogenously. The most potent endogenous neuroactive steroids are 3α-hydroxy-5-reduced pregnan-20-one and 3α-21-dihydroxy-5-reduced pregnan-20-one, metabolites of hormonal steroids progesterone and deoxycorticosterone, respectively. The ability of these steroid metabolites to alter brain excitability was recognized in 1986 (Majewska, M. D. et al., Science 232:1004-1007 (1986); Harrison, N. L. et al., J Pharmacol. Exp. Ther. 241:346-353 (1987)).

WO-A-2014/169833 and WO-A-98/05337 both disclose 3.alpha.-hydroxy, pregnan-20-one derivatives with a 3.beta.-aliphatic substitution with activity as GABA receptor modulators.

New and improved compounds are needed that act as modulating agents for brain excitability, as well as agents for the prevention and treatment of CNS-related diseases. The compounds, compositions, and methods described herein are directed toward this end.

### Summary of the Invention

Provided herein are compounds designed to act as GABA modulators. In some embodiments, such compounds are envisioned to be useful as therapeutic agents for treating a CNS-related disorder.

According to the present invention, there is provided the compound is of Formula **(I-Ia)** 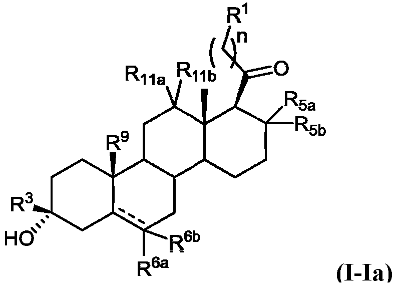 or a pharmaceutically acceptable salt thereof.

In some embodiments, the compound is of Formula **(I-Ib)** 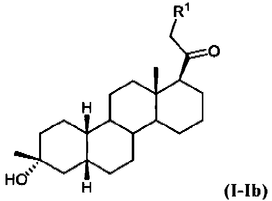 or a pharmaceutically acceptable salt thereof.

In some embodiments, the compound is of Formula **(I-Id)** 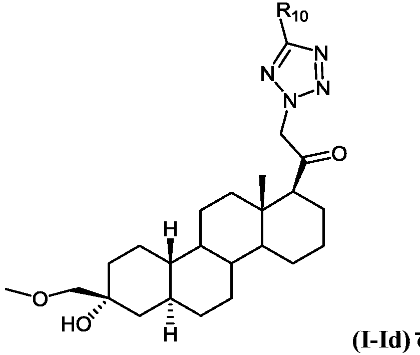 or a pharmaceutically acceptable salt thereof.

In some embodiments, the compound is of Formula **(I-Ie)** 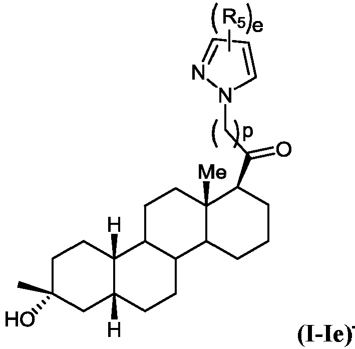 or a pharmaceutically acceptable salt thereof.

In some embodiments, the compound is of Formula **(I-If)** 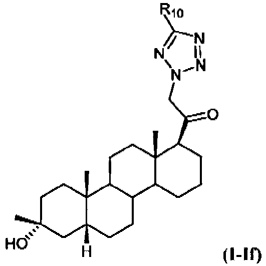 or a pharmaceutically acceptable salt thereof.

In some embodiments, the compound is of Formula **(I-Ig)** 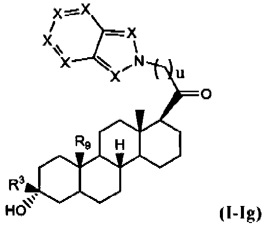 or a pharmaceutically acceptable salt thereof.

In some embodiments, the compound is of Formula **(I-Ih)** 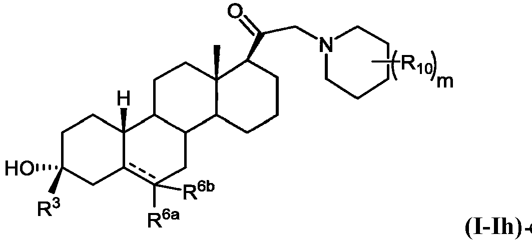 or a pharmaceutically acceptable salt thereof.

In some embodiments, the compound is of Formula **(I-Ii)** 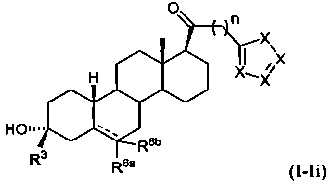 or a pharmaceutically acceptable salt thereof. The references to methods of treatment in the summary and detailed description of the invention in this description are to be interpreted as references to the compounds, pharmaceutical compositions and medicaments of the present invention for use in a method for treatment of the human (or animal) body by therapy (or for diagnosis).

In some embodiments, a pharmaceutical composition comprises a compound described herein or pharmaceutically acceptable salt thereof, and a pharmaceutically acceptable excipient.

In some embodiments, provided herein is a method of treating a CNS-related disorder in a subject in need thereof, comprising administering to the subject an effective amount of a compound described herein or a pharmaceutically acceptable salt thereof. In some embodiments, the CNS-related disorder is a sleep disorder, a mood disorder, a schizophrenia spectrum disorder, a convulsive disorder, a disorder of memory and/or cognition, a movement disorder, a personality disorder, autism spectrum disorder, pain, traumatic brain injury, a vascular disease, a substance abuse disorder and/or withdrawal syndrome, tinnitus, or status epilepticus. In some embodiments, the CNS-related disorder is depression. In some embodiments, the CNS-related disorder is postpartum depression. In some embodiments, the CNS-related disorder is major depressive disorder. In some embodiments, the major depressive disorder is moderate major depressive disorder. In some embodiments, the major depressive disorder is severe major depressive disorder.

In some embodiments, the compound is selected from the group consisting of the compounds of the invention as identified in **Table I-1** herein.

In one aspect, provided herein is a pharmaceutically acceptable salt of a compound of formula (I-Ia).

In one aspect, provided herein is a pharmaceutical composition comprising a compound of formula (I-Ia) or a pharmaceutically acceptable salt thereof, and a pharmaceutically acceptable excipient. In certain embodiments, the compound of the present invention is provided in an effective amount in the pharmaceutical composition. In certain embodiments, the compound of the present invention is provided in a therapeutically effective amount. In certain embodiments, the compound of the present invention is provided in a prophylactically effective amount.

Compounds of the present invention as described herein, act, in certain embodiments, as GABA modulators, e.g., effecting the GABA_{A} receptor in either a positive or negative manner. As modulators of the excitability of the central nervous system (CNS), as mediated by their ability to modulate GABA_{A} receptor, such compounds are expected to have CNS-activity.

Thus, in another aspect, provided are methods of treating a CNS-related disorder in a subject in need thereof, comprising administering to the subject an effective amount of a compound of the present invention. In certain embodiments, CNS-related disorder is a sleep disorder, a mood disorder, a schizophrenia spectrum disorder, a convulsive disorder, a disorder of memory and/or cognition, a movement disorder, a personality disorder, autism spectrum disorder, pain, traumatic brain injury, a vascular disease, a substance abuse disorder and/or withdrawal syndrome, tinnitus, or status epilepticus. In certain embodiments, the CNS-related disorder is depression. In certain embodiments, the CNS-related disorder is postpartum depression. In certain embodiments, the CNS-related disorder is major depressive disorder. In certain embodiments, the major depressive disorder is moderate major depressive disorder. In certain embodiments, the major depressive disorder is severe major depressive disorder. In certain embodiments, the compound is administered orally, subcutaneously, intravenously, or intramuscularly. In certain embodiments, the compound is administered orally. In certain embodiments, the compound is administered chronically. In certain embodiments, the compound is administered continuously, *e.g*., by continuous intravenous infusion.

### Detailed Description of Certain Embodiments of the Invention

As generally described herein, the present invention provides compounds designed, for example, to act as GABA modulators. In certain embodiments, such compounds are envisioned to be useful as therapeutic agents for treating a CNS-related disorder *(e.g.,* a disorder as described herein, for example depression, such as post-partum depression or major depressive disorder).

### Definitions

### Chemical definitions

Definitions of specific functional groups and chemical terms are described in more detail below. The chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Thomas Sorrell, Organic Chemistry, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987.

Isomers, *e.g*., stereoisomers, can be isolated from mixtures by methods known to those skilled in the art, including chiral high pressure liquid chromatography (HPLC) and the formation of chiral salts; or preferred isomers can be prepared by asymmetric syntheses. See, for example, Jacques et al., Enantiomers, Racemates and Resolutions (Wiley Interscience, New York, 1981); Wilen et al., Tetrahedron 33:2725 (1977); Eliel, Stereochemistry of Carbon Compounds (McGraw-Hill, NY, 1962); and Wilen, Tables of Resolving Agents and Optical Resolutions p. 268 (E.L. Eliel, Ed., Univ. of Notre Dame Press, Notre Dame, IN 1972). The invention additionally encompasses compounds described herein as individual isomers substantially free of other isomers, and alternatively, as mixtures of various isomers.

"Stereoisomers": It is also to be understood that compounds that have the same molecular formula but differ in the nature or sequence of bonding of their atoms or the arrangement of their atoms in space are termed "isomers." Isomers that differ in the arrangement of their atoms in space are termed "stereoisomers." Stereoisomers that are not mirror images of one another are termed "diastereomers" and those that are non-superimposable mirror images of each other are termed "enantiomers." When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (*i.e.,* as (+) or (-)-isomers respectively). A chiral compound can exist as either individual enantiomer or as a mixture thereof. A mixture containing equal proportions of the enantiomers is called a "racemic mixture".

As used herein a pure enantiomeric compound is substantially free from other enantiomers or stereoisomers of the compound (*i.e.,* in enantiomeric excess). In other words, an "S" form of the compound is substantially free from the "R" form of the compound and is, thus, in enantiomeric excess of the "R" form. The term "enantiomerically pure" or "pure enantiomer" denotes that the compound comprises more than 75% by weight, more than 80% by weight, more than 85% by weight, more than 90% by weight, more than 91% by weight, more than 92% by weight, more than 93% by weight, more than 94% by weight, more than 95% by weight, more than 96% by weight, more than 97% by weight, more than 98% by weight, more than 98.5% by weight, more than 99% by weight, more than 99.2% by weight, more than 99.5% by weight, more than 99.6% by weight, more than 99.7% by weight, more than 99.8% by weight or more than 99.9% by weight, of the enantiomer. In certain embodiments, the weights are based upon total weight of all enantiomers or stereoisomers of the compound.

As used herein, the term "diastereomeric purity" refers to the amount of a compound having the depicted absolute stereochemistry, expressed as a percentage of the total amount of the depicted compound and its diastereomers. The term "diastereomierically pure" denotes that the compound comprises more than 75% by weight, more than 80% by weight, more than 85% by weight, more than 90% by weight, more than 91% by weight, more than 92% by weight, more than 93% by weight, more than 94% by weight, more than 95% by weight, more than 96% by weight, more than 97% by weight, more than 98% by weight, more than 98.5% by weight, more than 99% by weight, more than 99.2% by weight, more than 99.5% by weight, more than 99.6% by weight, more than 99.7% by weight, more than 99.8% by weight or more than 99.9% by weight, of the diastereomer. Methods for determining diastereomeric and enantiomeric purity are well-known in the art. Diastereomeric purity can be determined by any analytical method capable of quantitatively distinguishing between a compound and its diastereomers, such as high performance liquid chromatography (HPLC).

In the compositions provided herein, an enantiomerically pure compound can be present with other active or inactive ingredients. For example, a pharmaceutical composition comprising enantiomerically pure R-compound can comprise, for example, about 90% excipient and about 10% enantiomerically pure R-compound. In certain embodiments, the enantiomerically pure R-compound in such compositions can, for example, comprise, at least about 95% by weight R-compound and at most about 5% by weight S-compound, by total weight of the compound. For example, a pharmaceutical composition comprising enantiomerically pure S-compound can comprise, for example, about 90% excipient and about 10% enantiomerically pure S-compound. In certain embodiments, the enantiomerically pure S-compound in such compositions can, for example, comprise, at least about 95% by weight S-compound and at most about 5% by weight R-compound, by total weight of the compound. In certain embodiments, the active ingredient can be formulated with little or no excipient or carrier.

The articles "a" and "an" may be used herein to refer to one or to more than one (*i.e.* at least one) of the grammatical objects of the article. By way of example "an analogue" means one analogue or more than one analogue.

When a range of values is listed, it is intended to encompass each value and sub-range within the range. For example "C₁₋₆ alkyl" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyl.

The following terms are intended to have the meanings presented therewith below and are useful in understanding the description and intended scope of the present invention.

"Alkyl" refers to a radical of a straight-chain or branched saturated hydrocarbon group having from 1 to 20 carbon atoms ("C₁₋₂₀ alkyl"). In some embodiments, an alkyl group has 1 to 12 carbon atoms ("C₁₋₁₂ alkyl"). In some embodiments, an alkyl group has 1 to 10 carbon atoms ("C₁₋₁₀ alkyl"). In some embodiments, an alkyl group has 1 to 9 carbon atoms ("C₁₋₉ alkyl"). In some embodiments, an alkyl group has 1 to 8 carbon atoms ("C₁₋₈ alkyl"). In some embodiments, an alkyl group has 1 to 7 carbon atoms ("C₁₋₇ alkyl"). In some embodiments, an alkyl group has 1 to 6 carbon atoms ("C₁₋₆ alkyl", also referred to herein as "lower alkyl"). In some embodiments, an alkyl group has 1 to 5 carbon atoms ("C₁₋₅ alkyl"). In some embodiments, an alkyl group has 1 to 4 carbon atoms ("C₁₋₄ alkyl"). In some embodiments, an alkyl group has 1 to 3 carbon atoms ("C₁₋₃ alkyl"). In some embodiments, an alkyl group has 1 to 2 carbon atoms ("C₁₋₂ alkyl"). In some embodiments, an alkyl group has 1 carbon atom ("C₁ alkyl"). In some embodiments, an alkyl group has 2 to 6 carbon atoms ("C₂₋₆ alkyl"). Examples of C₁₋₆ alkyl groups include methyl (C₁), ethyl (C₂), n-propyl (C₃), isopropyl (C₃), n-butyl (C₄), tert-butyl (C₄), sec-butyl (C₄), iso-butyl (C₄), n-pentyl (C₅), 3-pentanyl (C₅), amyl (C₅), neopentyl (C₅), 3-methyl-2-butanyl (C₅), tertiary amyl (C₃), and n-hexyl (C₆). Additional examples of alkyl groups include n-heptyl (C₇), n-octyl (C₈) and the like. Unless otherwise specified, each instance of an alkyl group is independently optionally substituted, *i.e.,* unsubstituted (an "unsubstituted alkyl") or substituted (a "substituted alkyl") with one or more substituents; e.g., for instance from 1 to 5 substituents, 1 to 3 substituents, or 1 substituent. In certain embodiments, the alkyl group is unsubstituted C₁₋₁₀ alkyl (*e.g.,* -CH₃). In certain embodiments, the alkyl group is substituted C₁₋₁₀ alkyl. Common alkyl abbreviations include Me (-CH₃), Et (-CH₂CH₃), iPr (-CH(CH₃)₂), nPr (-CH₂CH₂CH₃), n-Bu (-CH₂CH₂CH₂CH₃), or i-Bu (-CH₂CH(CH₃)₂).

"Alkylene" refers to an alkyl group wherein two hydrogens are removed to provide a divalent radical, and which may be substituted or unsubstituted. Unsubstituted alkylene groups include, but are not limited to, methylene (-CH₂-), ethylene (-CH₂CH₂-), propylene (-CH₂CH₂CH₂-), butylene (-CH₂CH₂CH₂CH₂-), pentylene (-CH₂CH₂CH₂CH₂CH₂-), hexylene (-CH₂CH₂CH₂CH₂CH₂CH₂-), and the like. Exemplary substituted alkylene groups, e.g., substituted with one or more alkyl (methyl) groups, include but are not limited to, substituted methylene (-CH(CH₃)-, (-C(CH₃)₂-), substituted ethylene (-CH(CH₃)CH₂-,-CH₂CH(CH₃)-, -C(CH₃)₂CH₂-,-CH₂C(CH₃)₂-), substituted propylene (-CH(CH₃)CH₂CH₂-, - CH₂CH(CH₃)CH₂-, -CH₂CH₂CH(CH₃)-, -C(CH₃)₂CH₂CH₂-, -CH₂C(CH₃)₂CH₂-, - CH₂CH₂C(CH₃)₂-), and the like. When a range or number of carbons is provided for a particular alkylene group, it is understood that the range or number refers to the range or number of carbons in the linear carbon divalent chain. Alkylene groups may be substituted or unsubstituted with one or more substituents as described herein.

"Alkenyl" refers to a radical of a straight-chain or branched hydrocarbon group having from 2 to 20 carbon atoms, one or more carbon-carbon double bonds *(e.g.,* 1, 2, 3, or 4 carbon-carbon double bonds), and optionally one or more carbon-carbon triple bonds *(e.g.,* 1, 2, 3, or 4 carbon-carbon triple bonds) ("C₂₋₂₀ alkenyl"). In certain embodiments, alkenyl does not contain any triple bonds. In some embodiments, an alkenyl group has 2 to 10 carbon atoms ("C₂₋₁₀ alkenyl"). In some embodiments, an alkenyl group has 2 to 9 carbon atoms ("C₂₋₉ alkenyl"). In some embodiments, an alkenyl group has 2 to 8 carbon atoms ("C₂₋₈ alkenyl"). In some embodiments, an alkenyl group has 2 to 7 carbon atoms ("C₂₋₇ alkenyl"). In some embodiments, an alkenyl group has 2 to 6 carbon atoms ("C₂₋₆ alkenyl"). In some embodiments, an alkenyl group has 2 to 5 carbon atoms ("C₂₋₅ alkenyl"). In some embodiments, an alkenyl group has 2 to 4 carbon atoms ("C₂₋₄ alkenyl"). In some embodiments, an alkenyl group has 2 to 3 carbon atoms ("C₂₋₃ alkenyl"). In some embodiments, an alkenyl group has 2 carbon atoms ("C₂ alkenyl"). The one or more carbon-carbon double bonds can be internal (such as in 2-butenyl) or terminal (such as in 1-butenyl). Examples of C₂₋₄ alkenyl groups include ethenyl (C₂), 1-propenyl (C₃), 2-propenyl (C₃), 1-butenyl (C₄), 2-butenyl (C₄), butadienyl (C₄), and the like. Examples of C₂₋₆ alkenyl groups include the aforementioned C₂₋₄ alkenyl groups as well as pentenyl (C₅), pentadienyl (C₅), hexenyl (C₆), and the like. Additional examples of alkenyl include heptenyl (C₇), octenyl (C₈), octatrienyl (C₈), and the like. Unless otherwise specified, each instance of an alkenyl group is independently optionally substituted, *i.e.,* unsubstituted (an "unsubstituted alkenyl") or substituted (a "substituted alkenyl") with one or more substituents *e.g*., for instance from 1 to 5 substituents, 1 to 3 substituents, or 1 substituent. In certain embodiments, the alkenyl group is unsubstituted C₂₋₁₀ alkenyl. In certain embodiments, the alkenyl group is substituted C₂₋₁₀ alkenyl.

"Alkynyl" refers to a radical of a straight-chain or branched hydrocarbon group having from 2 to 20 carbon atoms, one or more carbon-carbon triple bonds *(e.g.,* 1, 2, 3, or 4 carbon-carbon triple bonds), and optionally one or more carbon-carbon double bonds (*e.g.,* 1, 2, 3, or 4 carbon-carbon double bonds) ("C₂₋₂₀ alkynyl"). In certain embodiments, alkynyl does not contain any double bonds. In some embodiments, an alkynyl group has 2 to 10 carbon atoms ("C₂₋₁₀ alkynyl"). In some embodiments, an alkynyl group has 2 to 9 carbon atoms ("C₂₋₉ alkynyl"). In some embodiments, an alkynyl group has 2 to 8 carbon atoms ("C₂₋₈ alkynyl"). In some embodiments, an alkynyl group has 2 to 7 carbon atoms ("C₂₋₇ alkynyl"). In some embodiments, an alkynyl group has 2 to 6 carbon atoms ("C₂₋₆ alkynyl"). In some embodiments, an alkynyl group has 2 to 5 carbon atoms ("C₂₋₅ alkynyl"). In some embodiments, an alkynyl group has 2 to 4 carbon atoms ("C₂₋₄ alkynyl"). In some embodiments, an alkynyl group has 2 to 3 carbon atoms ("C₂₋₃ alkynyl"). In some embodiments, an alkynyl group has 2 carbon atoms ("C₂ alkynyl"). The one or more carbon-carbon triple bonds can be internal (such as in 2-butynyl) or terminal (such as in 1-butynyl). Examples of C₂₋₄ alkynyl groups include, without limitation, ethynyl (C₂), 1-propynyl (C₃), 2-propynyl (C₃), 1-butynyl (C₄), 2-butynyl (C₄), and the like. Examples of C₂₋₆ alkenyl groups include the aforementioned C₂₋₄ alkynyl groups as well as pentynyl (C₅), hexynyl (C₆), and the like. Additional examples of alkynyl include heptynyl (C₇), octynyl (C₈), and the like. Unless otherwise specified, each instance of an alkynyl group is independently optionally substituted, *i.e.,* unsubstituted (an "unsubstituted alkynyl") or substituted (a "substituted alkynyl") with one or more substituents; ***e.g.,*** for instance from 1 to 5 substituents, 1 to 3 substituents, or 1 substituent. In certain embodiments, the alkynyl group is unsubstituted C₂₋₁₀ alkynyl. In certain embodiments, the alkynyl group is substituted C₂₋₁₀ alkynyl.

The term "heteroalkyl," as used herein, refers to an alkyl group, as defined herein, which further comprises 1 or more *(e.g.,* 1, 2, 3, or 4) heteroatoms *(e.g.,* oxygen, sulfur, nitrogen, boron, silicon, phosphorus) within the parent chain, wherein the one or more heteroatoms is inserted between adjacent carbon atoms within the parent carbon chain and/or one or more heteroatoms is inserted between a carbon atom and the parent molecule, *i.e.,* between the point of attachment. In certain embodiments, a heteroalkyl group refers to a saturated group having from 1 to 10 carbon atoms and 1, 2, 3, or 4 heteroatoms ("heteroC₁₋₁₀ alkyl"). In some embodiments, a heteroalkyl group is a saturated group having 1 to 9 carbon atoms and 1, 2, 3, or 4 heteroatoms ("heteroC₁₋₉ alkyl"). In some embodiments, a heteroalkyl group is a saturated group having 1 to 8 carbon atoms and 1, 2, 3, or 4 heteroatoms ("heteroC₁₋₈ alkyl"). In some embodiments, a heteroalkyl group is a saturated group having 1 to 7 carbon atoms and 1, 2, 3, or 4 heteroatoms ("heteroC₁₋₇ alkyl"). In some embodiments, a heteroalkyl group is a group having 1 to 6 carbon atoms and 1, 2, or 3 heteroatoms ("heteroC₁₋₆ alkyl"). In some embodiments, a heteroalkyl group is a saturated group having 1 to 5 carbon atoms and 1 or 2 heteroatoms ("heteroC₁₋₅ alkyl"). In some embodiments, a heteroalkyl group is a saturated group having 1 to 4 carbon atoms and 1or 2 heteroatoms ("heteroC₁₋₄ alkyl"). In some embodiments, a heteroalkyl group is a saturated group having 1 to 3 carbon atoms and 1 heteroatom ("heteroC₁₋₃ alkyl"). In some embodiments, a heteroalkyl group is a saturated group having 1 to 2 carbon atoms and 1 heteroatom ("heteroC₁₋₂ alkyl"). In some embodiments, a heteroalkyl group is a saturated group having 1 carbon atom and 1 heteroatom ("heteroC₁ alkyl"). In some embodiments, a heteroalkyl group is a saturated group having 2 to 6 carbon atoms and 1 or 2 heteroatoms ("heteroC₂₋₆ alkyl"). Unless otherwise specified, each instance of a heteroalkyl group is independently unsubstituted (an "unsubstituted heteroalkyl") or substituted (a "substituted heteroalkyl") with one or more substituents. In certain embodiments, the heteroalkyl group is an unsubstituted heteroC₁₋₁₀ alkyl. In certain embodiments, the heteroalkyl group is a substituted heteroC₁₋₁₀ alkyl.

"Aryl" refers to a radical of a monocyclic or polycyclic (*e.g*., bicyclic or tricyclic) 4n+2 aromatic ring system (*e.g*., having 6, 10, or 14 π electrons shared in a cyclic array) having 6-14 ring carbon atoms and zero heteroatoms provided in the aromatic ring system ("C₆₋₁₄ aryl"). In some embodiments, an aryl group has six ring carbon atoms ("C₆ aryl"; *e.g.,* phenyl). In some embodiments, an aryl group has ten ring carbon atoms ("C₁₀ aryl"; *e.g.,* naphthyl such as 1-naphthyl and 2-naphthyl). In some embodiments, an aryl group has fourteen ring carbon atoms ("C₁₄ aryl"; *e.g*., anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Typical aryl groups include, but are not limited to, groups derived from aceanthrylene, acenaphthylene, acephenanthrylene, anthracene, azulene, benzene, chrysene, coronene, fluoranthene, fluorene, hexacene, hexaphene, hexalene, as-indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, and trinaphthalene. Particularly aryl groups include phenyl, naphthyl, indenyl, and tetrahydronaphthyl. Unless otherwise specified, each instance of an aryl group is independently optionally substituted, *i.e.,* unsubstituted (an "unsubstituted aryl") or substituted (a "substituted aryl") with one or more substituents. In certain embodiments, the aryl group is unsubstituted C₆₋₁₄ aryl. In certain embodiments, the aryl group is substituted C₆₋₁₄ aryl.

In certain embodiments, an aryl group substituted with one or more of groups selected from halo, C₁-C₈ alkyl, C₁-C₈ haloalkyl, cyano, hydroxy, C₁-C₈ alkoxy, and amino.

Examples of representative substituted aryls include the following 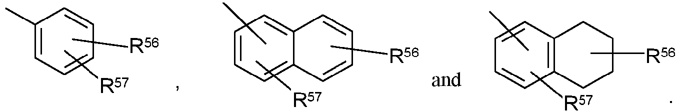 wherein one of R⁵⁶ and R⁵⁷ may be hydrogen and at least one of R⁵⁶ and R⁵⁷ is each independently selected from C₁-C₈ alkyl, C₁-C₈ haloalkyl, 4-10 membered heterocyclyl, alkanoyl, C₁-C₈ alkoxy, heteroaryloxy, alkylamino, arylamino, heteroarylamino, NR⁵⁸COR⁵⁹, NR⁵⁸SOR⁵⁹NR⁵⁸SO₂R⁵⁹, COOalkyl, COOaryl, CONR⁵⁸R⁵⁹, CONR⁵⁸OR⁵⁹, NR⁵⁸R⁵⁹, SO₂NR⁵⁸R⁵⁹, S-alkyl, SOalkyl, SO₂alkyl, Saryl, SOaryl, SO₂aryl; or R⁵⁶ and R⁵⁷ may be joined to form a cyclic ring (saturated or unsaturated) from 5 to 8 atoms, optionally containing one or more heteroatoms selected from the group N, O, or S. R⁶⁰ and R⁶¹ are independently hydrogen, C₁-C₈ alkyl, C₁-C₄ haloalkyl, C₃-C₁₀ cycloalkyl, 4-10 membered heterocyclyl, C₆-C₁₀ aryl, substituted C₆-C₁₀ aryl, 5-10 membered heteroaryl, or substituted 5-10 membered heteroaryl .

"Fused aryl" refers to an aryl having two of its ring carbon in common with a second aryl or heteroaryl ring or with a carbocyclyl or heterocyclyl ring.

"Heteroaryl" refers to a radical of a 5-10 membered monocyclic or bicyclic 4n+2 aromatic ring system (*e.g*., having 6 or 10 π electrons shared in a cyclic array) having ring carbon atoms and 1-4 ring heteroatoms provided in the aromatic ring system, wherein each heteroatom is independently selected from nitrogen, oxygen and sulfur ("5-10 membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. Bicyclic heteroaryl groups wherein one ring does not contain a heteroatom (*e.g.*, indolyl, quinolinyl, carbazolyl, and the like) the point of attachment can be on either ring, *i.e.,* either the ring bearing a heteroatom *(e.g.,* 2-indolyl) or the ring that does not contain a heteroatom *(e.g., 5-*indolyl).

In some embodiments, a heteroaryl group is a 5-10 membered aromatic ring system having ring carbon atoms and 1-4 ring heteroatoms provided in the aromatic ring system, wherein each heteroatom is independently selected from nitrogen, oxygen, and sulfur ("5-10 membered heteroaryl"). In some embodiments, a heteroaryl group is a 5-8 membered aromatic ring system having ring carbon atoms and 1-4 ring heteroatoms provided in the aromatic ring system, wherein each heteroatom is independently selected from nitrogen, oxygen, and sulfur ("5-8 membered heteroaryl"). In some embodiments, a heteroaryl group is a 5-6 membered aromatic ring system having ring carbon atoms and 1-4 ring heteroatoms provided in the aromatic ring system, wherein each heteroatom is independently selected from nitrogen, oxygen, and sulfur ("5-6 membered heteroaryl"). In some embodiments, the 5-6 membered heteroaryl has 1-3 ring heteroatoms selected from nitrogen, oxygen, and sulfur. In some embodiments, the 5-6 membered heteroaryl has 1-2 ring heteroatoms selected from nitrogen, oxygen, and sulfur. In some embodiments, the 5-6 membered heteroaryl has 1 ring heteroatom selected from nitrogen, oxygen, and sulfur. Unless otherwise specified, each instance of a heteroaryl group is independently optionally substituted, *i.e.,* unsubstituted (an "unsubstituted heteroaryl") or substituted (a "substituted heteroaryl") with one or more substituents. In certain embodiments, the heteroaryl group is unsubstituted 5-14 membered heteroaryl. In certain embodiments, the heteroaryl group is substituted 5-14 membered heteroaryl.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

Examples of representative heteroaryls include the following: 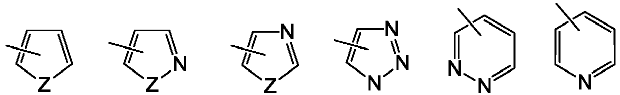 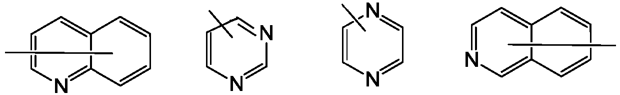 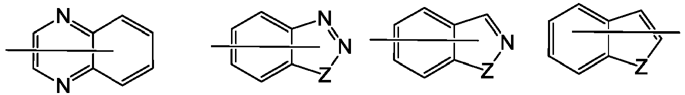 wherein each Z is selected from carbonyl, N, NR⁶⁵, O, and S; and R⁶⁵ is independently hydrogen, C₁-C₈ alkyl, C₃-C₁₀ cycloalkyl, 4-10 membered heterocyclyl, C₆-C₁₀ aryl, and 5-10 membered heteroaryl.

"Carbocyclyl" or "carbocyclic" refers to a radical of a non-aromatic cyclic hydrocarbon group having from 3 to 10 ring carbon atoms ("C₃₋₁₀ carbocyclyl") and zero heteroatoms in the non-aromatic ring system. In some embodiments, a carbocyclyl group has 3 to 8 ring carbon atoms ("C₃₋₈ carbocyclyl"). In some embodiments, a carbocyclyl group has 3 to 6 ring carbon atoms ("C₃₋₆ carbocyclyl"). In some embodiments, a carbocyclyl group has 3 to 6 ring carbon atoms ("C₃₋₆ carbocyclyl"). In some embodiments, a carbocyclyl group has 5 to 10 ring carbon atoms ("C₅₋₁₀ carbocyclyl"). Exemplary C₃₋₆ carbocyclyl groups include, without limitation, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), and the like. Exemplary C₃₋₈ carbocyclyl groups include, without limitation, the aforementioned C₃₋₆ carbocyclyl groups as well as cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptanyl (C₇), bicyclo[2.2.2]octanyl (C₈), and the like. Exemplary C₃₋₁₀ carbocyclyl groups include, without limitation, the aforementioned C₃₋₈ carbocyclyl groups as well as cyclononyl (C₉), cyclononenyl (C₉), cyclodecyl (C₁₀), cyclodecenyl (C₁₀), octahydro-1*H*-indenyl (C₉), decahydronaphthalenyl (C₁₀), spiro[4.5]decanyl (C₁₀), and the like. As the foregoing examples illustrate, in certain embodiments, the carbocyclyl group is either monocyclic ("monocyclic carbocyclyl") or contain a fused, bridged or spiro ring system such as a bicyclic system ("bicyclic carbocyclyl") and can be saturated or can be partially unsaturated. "Carbocyclyl" also includes ring systems wherein the carbocyclyl ring, as defined above, is fused with one or more aryl or heteroaryl groups wherein the point of attachment is on the carbocyclyl ring, and in such instances, the number of carbons continue to designate the number of carbons in the carbocyclic ring system. Unless otherwise specified, each instance of a carbocyclyl group is independently optionally substituted, *i.e.,* unsubstituted (an "unsubstituted carbocyclyl") or substituted (a "substituted carbocyclyl") with one or more substituents. In certain embodiments, the carbocyclyl group is unsubstituted C₃₋₁₀ carbocyclyl. In certain embodiments, the carbocyclyl group is a substituted C₃₋₁₀ carbocyclyl.

In some embodiments, "carbocyclyl" is a monocyclic, saturated carbocyclyl group having from 3 to 10 ring carbon atoms ("C₃₋₁₀ cycloalkyl"). In some embodiments, a cycloalkyl group has 3 to 8 ring carbon atoms ("C₃₋₈ cycloalkyl"). In some embodiments, a cycloalkyl group has 3 to 6 ring carbon atoms ("C₃₋₆ cycloalkyl"). In some embodiments, a cycloalkyl group has 5 to 6 ring carbon atoms ("C₃₋₆ cycloalkyl"). In some embodiments, a cycloalkyl group has 5 to 10 ring carbon atoms ("C₃₋₁₀ cycloalkyl"). Examples of C₅₋₆ cycloalkyl groups include cyclopentyl (C₅) and cyclohexyl (C₅). Examples of C₃₋₆ cycloalkyl groups include the aforementioned C₅₋₆ cycloalkyl groups as well as cyclopropyl (C₃) and cyclobutyl (C₄). Examples of C₃₋₈ cycloalkyl groups include the aforementioned C₃₋₆ cycloalkyl groups as well as cycloheptyl (C₇) and cyclooctyl (C₈). Unless otherwise specified, each instance of a cycloalkyl group is independently unsubstituted (an "unsubstituted cycloalkyl") or substituted (a "substituted cycloalkyl") with one or more substituents. In certain embodiments, the cycloalkyl group is unsubstituted C₃₋₁₀ cycloalkyl. In certain embodiments, the cycloalkyl group is substituted C₃₋₁₀ cycloalkyl.

"Heterocyclyl" or "heterocyclic" refers to a radical of a 3- to 10-membered non-aromatic ring system having ring carbon atoms and 1 to 4 ring heteroatoms, wherein each heteroatom is independently selected from nitrogen, oxygen, sulfur, boron, phosphorus, and silicon ("3-10 membered heterocyclyl"). In heterocyclyl groups that contain one or more nitrogen atoms, the point of attachment can be a carbon or nitrogen atom, as valency permits. A heterocyclyl group can either be monocyclic ("monocyclic heterocyclyl") or a fused, bridged or spiro ring system such as a bicyclic system ("bicyclic heterocyclyl"), and can be saturated or can be partially unsaturated. Heterocyclyl bicyclic ring systems can include one or more heteroatoms in one or both rings. "Heterocyclyl" also includes ring systems wherein the heterocyclyl ring, as defined above, is fused with one or more carbocyclyl groups wherein the point of attachment is either on the carbocyclyl or heterocyclyl ring, or ring systems wherein the heterocyclyl ring, as defined above, is fused with one or more aryl or heteroaryl groups, wherein the point of attachment is on the heterocyclyl ring, and in such instances, the number of ring members continue to designate the number of ring members in the heterocyclyl ring system. Unless otherwise specified, each instance of heterocyclyl is independently optionally substituted, *i.e.,* unsubstituted (an "unsubstituted heterocyclyl") or substituted (a "substituted heterocyclyl") with one or more substituents. In certain embodiments, the heterocyclyl group is unsubstituted 3-10 membered heterocyclyl. In certain embodiments, the heterocyclyl group is substituted 3-10 membered heterocyclyl.

In some embodiments, a heterocyclyl group is a 5-10 membered non-aromatic ring system having ring carbon atoms and 1-4 ring heteroatoms, wherein each heteroatom is independently selected from nitrogen, oxygen, sulfur, boron, phosphorus, and silicon ("5-10 membered heterocyclyl"). In some embodiments, a heterocyclyl group is a 5-8 membered non-aromatic ring system having ring carbon atoms and 1-4 ring heteroatoms, wherein each heteroatom is independently selected from nitrogen, oxygen, and sulfur ("5-8 membered heterocyclyl"). In some embodiments, a heterocyclyl group is a 5-6 membered non-aromatic ring system having ring carbon atoms and 1-4 ring heteroatoms, wherein each heteroatom is independently selected from nitrogen, oxygen, and sulfur ("5-6 membered heterocyclyl"). In some embodiments, the 5-6 membered heterocyclyl has 1-3 ring heteroatoms selected from nitrogen, oxygen, and sulfur. In some embodiments, the 5-6 membered heterocyclyl has 1-2 ring heteroatoms selected from nitrogen, oxygen, and sulfur. In some embodiments, the 5-6 membered heterocyclyl has one ring heteroatom selected from nitrogen, oxygen, and sulfur.

Exemplary 3-membered heterocyclyl groups containing one heteroatom include, without limitation, azirdinyl, oxiranyl, thiorenyl. Exemplary 4-membered heterocyclyl groups containing one heteroatom include, without limitation, azetidinyl, oxetanyl and thietanyl. Exemplary 5-membered heterocyclyl groups containing one heteroatom include, without limitation, tetrahydrofuranyl, dihydrofuranyl, tetrahydrothiophenyl, dihydrothiophenyl, pyrrolidinyl, dihydropyrrolyl and pyrrolyl-2,5-dione. Exemplary 5-membered heterocyclyl groups containing two heteroatoms include, without limitation, dioxolanyl, oxasulfuranyl, disulfuranyl, and oxazolidin-2-one. Exemplary 5-membered heterocyclyl groups containing three heteroatoms include, without limitation, triazolinyl, oxadiazolinyl, and thiadiazolinyl. Exemplary 6-membered heterocyclyl groups containing one heteroatom include, without limitation, piperidinyl, tetrahydropyranyl, dihydropyridinyl, and thianyl. Exemplary 6-membered heterocyclyl groups containing two heteroatoms include, without limitation, piperazinyl, morpholinyl, dithianyl, dioxanyl. Exemplary 6-membered heterocyclyl groups containing two heteroatoms include, without limitation, triazinanyl. Exemplary 7-membered heterocyclyl groups containing one heteroatom include, without limitation, azepanyl, oxepanyl and thiepanyl. Exemplary 8-membered heterocyclyl groups containing one heteroatom include, without limitation, azocanyl, oxecanyl and thiocanyl. Exemplary 5-membered heterocyclyl groups fused to a C₆ aryl ring (also referred to herein as a 5,6-bicyclic heterocyclic ring) include, without limitation, indolinyl, isoindolinyl, dihydrobenzofuranyl, dihydrobenzothienyl, benzoxazolinonyl, and the like. Exemplary 6-membered heterocyclyl groups fused to an aryl ring (also referred to herein as a 6,6-bicyclic heterocyclic ring) include, without limitation, tetrahydroquinolinyl, tetrahydroisoquinolinyl, and the like.

"Nitrogen-containing heterocyclyl" group means a 4- to 7- membered non-aromatic cyclic group containing at least one nitrogen atom, for example, but without limitation, morpholine, piperidine (*e.g*. 2-piperidinyl, 3-piperidinyl and 4-piperidinyl), pyrrolidine (*e.g*. 2-pyrrolidinyl and 3-pyrrolidinyl), azetidine, pyrrolidone, imidazoline, imidazolidinone, 2-pyrazoline, pyrazolidine, piperazine, and N-alkyl piperazines such as N-methyl piperazine. Particular examples include azetidine, piperidone and piperazone.

"Hetero" when used to describe a compound or a group present on a compound means that one or more carbon atoms in the compound or group have been replaced by a nitrogen, oxygen, or sulfur heteroatom. Hetero may be applied to any of the hydrocarbyl groups described above such as alkyl, *e.g.,* heteroalkyl, cycloalkyl, *e.g.,* heterocyclyl, aryl, *e.g,.* heteroaryl, cycloalkenyl, *e.g,.* cycloheteroalkenyl, and the like having from 1 to 5, and particularly from 1 to 3 heteroatoms.

"Acyl" refers to a radical -C(O)R²⁰, where R²⁰ is hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl, as defined herein. "Alkanoyl" is an acyl group wherein R²⁰ is a group other than hydrogen. Representative acyl groups include, but are not limited to, formyl (-CHO), acetyl (-C(=O)CH₃), cyclohexylcarbonyl, cyclohexylmethylcarbonyl, benzoyl (-C(=O)Ph), benzylcarbonyl (-C(=O)CH₂Ph), -C(O)-C₁-C₈ alkyl, -C(O)-(CH₂)ₜ(C₆-C₁₀ aryl), -C(O)-(CH₂)ₜ(5-10 membered heteroaryl), -C(O)-(CH₂)ₜ(C₃-C₁₀ cycloalkyl), and -C(O)-(CH₂)ₜ(4-10 membered heterocyclyl), wherein t is an integer from 0 to 4. In certain embodiments, R²¹ is C₁-C₈ alkyl, substituted with halo or hydroxy; or C₃-C₁₀ cycloalkyl, 4-10 membered heterocyclyl, C₆-C₁₀ aryl, arylalkyl, 5-10 membered heteroaryl or heteroarylalkyl, each of which is substituted with unsubstituted C₁-C₄ alkyl, halo, unsubstituted C₁-C₄ alkoxy, unsubstituted C₁-C₄ haloalkyl, unsubstituted C₁-C₄ hydroxyalkyl, or unsubstituted C₁-C₄ haloalkoxy or hydroxy.

"Alkoxy" refers to the group -OR²⁹ where R²⁹ is substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl. Particular alkoxy groups are methoxy, ethoxy, n-propoxy, isopropoxy, n-butoxy, tert-butoxy, sec-butoxy, n-pentoxy, n-hexoxy, and 1,2-dimethylbutoxy. Particular alkoxy groups are lower alkoxy, *i.e.* with between 1 and 6 carbon atoms. Further particular alkoxy groups have between 1 and 4 carbon atoms.

In certain embodiments, R²⁹ is a group that has 1 or more substituents, for instance from 1 to 5 substituents, and particularly from 1 to 3 substituents, in particular 1 substituent, selected from the group consisting of amino, substituted amino, C₆-C₁₀ aryl, aryloxy, carboxyl, cyano, C₃-C₁₀ cycloalkyl, 4-10 membered heterocyclyl, halogen, 5-10 membered heteroaryl, hydroxyl, nitro, thioalkoxy, thioaryloxy, thiol, alkyl-S(O)-, aryl-S(O)-, alkylS(O)₂- and aryl-S(O)₂-. Exemplary 'substituted alkoxy' groups include, but are not limited to, -O-(CH₂)ₜ(C₆-C₁₀ aryl), -O-(CH₂)ₜ(5-10 membered heteroaryl), -O-(CH₂)ₜ(C₃-C₁₀ cycloalkyl), and -O-(CH₂)ₜ(4-10 membered heterocyclyl), wherein t is an integer from 0 to 4 and any aryl, heteroaryl, cycloalkyl or heterocyclyl groups present, may themselves be substituted by unsubstituted C₁-C₄ alkyl, halo, unsubstituted C₁-C₄ alkoxy, unsubstituted C₁-C₄ haloalkyl, unsubstituted C₁-C₄ hydroxyalkyl, or unsubstituted C₁-C₄ haloalkoxy or hydroxy. Particular exemplary 'substituted alkoxy' groups are -OCF₃, -OCH₂CF₃, -OCH₂Ph, -OCH₂-cyclopropyl, -OCH₂CH₂OH, and -OCH₂CH₂NMe₂.

"Amino" refers to the radical -NH₂.

"Oxo group" refers to -C(=O)-.

"Substituted amino" refers to an amino group of the formula -N(R³⁸)₂ wherein R³⁸ is hydrogen, substituted or unsubstituted alkyl, substituted or unsubstitued alkenyl, substituted or unsubstitued alkynyl, substituted or unsubstitued carbocyclyl, substituted or unsubstituted heterocyclyl, substituted or unsubstituted aryl, substituted or unsubstitued heteroaryl, or an amino protecting group, wherein at least one of R³⁸ is not a hydrogen. In certain embodiments, each R³⁸ is independently selected from hydrogen, C₁-C₈ alkyl, C₃-C₈ alkenyl, C₃-C₈ alkynyl, C₆-C₁₀ aryl, 5-10 membered heteroaryl, 4-10 membered heterocyclyl, or C₃-C₁₀ cycloalkyl; or C₁-C₈ alkyl, substituted with halo or hydroxy; C₃-C₈ alkenyl, substituted with halo or hydroxy; C₃-C₈ alkynyl, substituted with halo or hydroxy, or - (CH₂)ₜ(C₆-C₁₀ aryl), -(CH₂)ₜ(5-10 membered heteroaryl), -(CH₂)ₜ(C₃-C₁₀ cycloalkyl), or - (CH₂)ₜ(4-10 membered heterocyclyl), wherein t is an integer between 0 and 8, each of which is substituted by unsubstituted C₁-C₄ alkyl, halo, unsubstituted C₁-C₄ alkoxy, unsubstituted C₁-C₄ haloalkyl, unsubstituted C₁-C₄ hydroxyalkyl, or unsubstituted C₁-C₄ haloalkoxy or hydroxy; or both R³⁸ groups are joined to form an alkylene group.

Exemplary "substituted amino" groups include, but are not limited to, -NR³⁹-C₁-C₈ alkyl, -NR³⁹-(CH₂)ₜ(C₆-C₁₀ aryl), -NR³⁹-(CH₂)ₜ(5-10 membered heteroaryl), -NR³⁹-(CH₂)ₜ(C₃-C₁₀ cycloalkyl), and -NR³⁹-(CH₂)ₜ(4-10 membered heterocyclyl), wherein t is an integer from 0 to 4, for instance 1 or 2, each R³⁹ independently represents H or C₁-C₈ alkyl; and any alkyl groups present, may themselves be substituted by halo, substituted or unsubstituted amino, or hydroxy; and any aryl, heteroaryl, cycloalkyl, or heterocyclyl groups present, may themselves be substituted by unsubstituted C₁-C₄ alkyl, halo, unsubstituted C₁-C₄ alkoxy, unsubstituted C₁-C₄ haloalkyl, unsubstituted C₁-C₄ hydroxyalkyl, or unsubstituted C₁-C₄ haloalkoxy or hydroxy. For the avoidance of doubt the term 'substituted amino' includes the groups alkylamino, substituted alkylamino, alkylarylamino, substituted alkylarylamino, arylamino, substituted arylamino, dialkylamino, and substituted dialkylamino as defined below. Substituted amino encompasses both monosubstituted amino and disubstituted amino groups.

"Carboxy" refers to the radical -C(O)OH.

"Cyano" refers to the radical -CN.

"Halo" or "halogen" refers to fluoro (F), chloro (Cl), bromo (Br), and iodo (I). In certain embodiments, the halo group is either fluoro or chloro.

"Haloalkyl" refers to an alkyl radical in which the alkyl group is substituted with one or more halogens. Typical haloalkyl groups include, but are not limited to, trifluoromethyl, difluoromethyl, fluoromethyl, chloromethyl, dichloromethyl, dibromoethyl, tribromomethyl, tetrafluoroethyl, and the like.

"Hydroxy" refers to the radical -OH.

"Nitro" refers to the radical -NO₂.

"Thioketo" refers to the group =S.

Alkyl, alkenyl, alkynyl, carbocyclyl, heterocyclyl, aryl, and heteroaryl groups, as defined herein, are optionally substituted (*e*.*g*., "substituted" or "unsubstituted" alkyl, "substituted" or "unsubstituted" alkenyl, "substituted" or "unsubstituted" alkynyl, "substituted" or "unsubstituted" carbocyclyl, "substituted" or "unsubstituted" heterocyclyl, "substituted" or "unsubstituted" aryl or "substituted" or "unsubstituted" heteroaryl group). In general, the term "substituted", whether preceded by the term "optionally" or not, means that at least one hydrogen present on a group *(e.g.,* a carbon or nitrogen atom) is replaced with a permissible substituent, e.g., a substituent which upon substitution results in a stable compound, *e.g.,* a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. The term "substituted" is contemplated to include substitution with all permissible substituents of organic compounds, any of the substituents described herein that results in the formation of a stable compound. The present invention contemplates any and all such combinations in order to arrive at a stable compound. For purposes of this invention, heteroatoms such as nitrogen may have hydrogen substituents and/or any suitable substituent as described herein which satisfy the valencies of the heteroatoms and results in the formation of a stable moiety.

Exemplary carbon atom substituents include, but are not limited to, halogen, -CN, -NO₂, -N₃, -SO₂H, -SO₃H, -OH, -OR^{aa}, -ON(R^{bb})₂, -N(R^{bb})₂, -N(R^{bb})₃⁺X⁻, -N(OR^{cc})R^{bb}, - SH, -SR^{aa}, -SSR^{cc}, -C(=O)R^{aa}, -CO₂H, -CHO, -C(OR^{cc})₂, -CO₂R^{aa}, -OC(=O)R^{aa}, - OCO₂R^{aa}, -C(=O)N(R^{bb})₂, -OC(=O)N(R^{bb})₂, -NR^{bb}C(=O)R^{aa}, -NR^{bb}CO₂R^{aa}, - NR^{bb}C(=O)N(R^{bb})₂, -C(=NR^{bb})R^{aa}, -C(=NR^{bb})OR^{aa}, -OC(=NR^{bb})R^{aa}, -OC(=NR^{bb})OR^{aa}, - C(=NR^{bb})N(R^{bb})₂, -OC(=NR^{bb})N(R^{bb})₂, -NR^{bb}C(=NR^{bb})N(R^{bb})₂, -C(=O)NR^{bb}SO₂R^{aa},-NR^{bb}SO₂R^{aa}, -SO₂N(R^{bb})₂, -SO₂R^{aa}, -SO₂OR^{aa}, -OSO₂R^{aa}, -S(=O)R^{aa}, -OS(=O)R^{aa}, - Si(R^{aa})₃, -OSi(R^{aa})₃ -C(=S)N(R^{bb})₂, -C(=O)SR^{aa}, -C(=S)SR^{aa}, -SC(=S)SR^{aa}, -SC(=O)SR^{aa}, -OC(=O)SR^{aa}, -SC(=O)OR^{aa}, -SC(=O)R^{aa}, -P(=O)₂R^{aa}, -OP(=O)₂R^{aa}, -P(=O)(R^{aa})₂, - OP(=O)(R^{aa})₂, -OP(=O)(OR^{cc})₂, -P(=O)₂N(R^{bb})₂, -OP(=O)₂N(R^{bb})₂, -P(=O)(NR^{bb})₂, - OP(=O)(NR^{bb})₂, -NR^{bb}P(=O)(OR^{cc})₂, -NR^{bb}P(=O)(NR^{bb})₂, -P(R^{cc})₂, -P(R^{cc})₃, -OP(R^{cc})₂, - OP(R^{cc})₃, -B(R^{aa})₂, -B(OR^{cc})₂, -BR^{aa}(OR^{cc}), C₁₋₁₀ alkyl, C₁₋₁₀ haloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, wherein each alkyl, alkenyl, alkynyl, carbocyclyl, heterocyclyl, aryl, and heteroaryl is independently substituted with 0, 1, 2, 3, 4, or 5 R^{dd} groups; or two geminal hydrogens on a carbon atom are replaced with the group =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb}, or =NOR^{cc};
each instance of R^{aa} is, independently, selected from C₁₋₁₀ alkyl, C₁₋₁₀ haloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, or two R^{aa} groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{dd} groups;
each instance of R^{bb} is, independently, selected from hydrogen, -OH, -OR^{aa}, - N(R^{cc})₂, -CN, -C(=O)R^{aa}, -C(=O)N(R^{cc})₂, -CO₂R^{aa}, -SO₂R^{aa}, -C(=NR^{cc})OR^{aa}, - C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, - C(=S)SR^{cc}, -P(=O)₂R^{aa}, -P(=O)(R^{aa})₂, -P(=O)₂N(R^{cc})₂, -P(=O)(NR^{cc})₂, C₁₋₁₀ alkyl, C₁₋₁₀ haloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, or two R^{bb} groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{dd} groups;
each instance of R^{cc} is, independently, selected from hydrogen, C₁₋₁₀ alkyl, C₁₋₁₀ haloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, or two R^{cc} groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{dd} groups;
each instance of R^{dd} is, independently, selected from halogen, -CN, -NO₂, -N₃, - SO₂H, -SO₃H, -OH, -OR^{ee}, -ON(R^{ff})₂, -N(R^{ff})₂, -N(R^{ff})₃⁺X⁻, -N(OR^{ee})R^{ff}, -SH, -SR^{ee}, - SSR^{ee}, -C(=O)R^{cc}, -CO₂H, -CO₂R^{ee}, -OC(=O)R^{ee}, -OCO₂R^{ee}, -C(=O)N(R^{ff})₂, - OC(=O)N(R^{ff})₂, -NR^{ff}C(=O)R^{ee}, -NR^{ff}CO₂R^{ee}, -NR^{ff}C(=O)N(R^{ff})₂, -C(=NR^{ff})OR^{ee}, - OC(=NR^{ff})R^{ee}, -OC(=NR^{ff})OR^{ee}, -C(=NR^{ff})N(R^{ff})₂, -OC(=NR^{ff})N(R^{ff})₂, - NR^{ff}C(=NR^{ff})N(R^{ff})₂,-NR^{ff}SO₂R^{ee}, -SO₂N(R^{ff})₂, -SO₂R^{ee}, -SO₂OR^{ee}, -OSO₂R^{ee}, -S(=O)R^{ee}, -Si(R^{ee})₃, -OSi(R^{ee})₃, -C(=S)N(R^{ff})₂, -C(=O)SR^{ee}, -C(=S)SR^{ee}, -SC(=S)SR^{ee}, -P(=O)₂R^{ee}, - P(=O)(R^{ee})₂, -OP(=O)(R^{ee})₂, -OP(=O)(OR^{ee})₂, C₁₋₆ alkyl, C₁₋₆ haloalkyl, C₂₋₆ alkenyl, C₂₋₆ alkynyl, C₃₋₁₀ carbocyclyl, 3-10 membered heterocyclyl, C₆₋₁₀ aryl, 5-10 membered heteroaryl, wherein each alkyl, alkenyl, alkynyl, carbocyclyl, heterocyclyl, aryl, and heteroaryl is independently substituted with 0, 1, 2, 3, 4, or 5 R^{gg} groups, or two geminal R^{dd} substituents can be joined to form =O or =S;
each instance of R^{ee} is, independently, selected from C₁₋₆ alkyl, C₁₋₆ haloalkyl, C₂₋₆ alkenyl, C₂₋₆ alkynyl, C₃₋₁₀ carbocyclyl, C₆₋₁₀ aryl, 3-10 membered heterocyclyl, and 3-10 membered heteroaryl, wherein each alkyl, alkenyl, alkynyl, carbocyclyl, heterocyclyl, aryl, and heteroaryl is independently substituted with 0, 1, 2, 3, 4, or 5 R^{gg} groups;
each instance of R^{ff} is, independently, selected from hydrogen, C₁₋₆ alkyl, C₁₋₆ haloalkyl, C₂₋₆ alkenyl, C₂₋₆ alkynyl, C₃₋₁₀ carbocyclyl, 3-10 membered heterocyclyl, C₆₋₁₀ aryl and 5-10 membered heteroaryl, or two R^{ff} groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{gg} groups; and
each instance of R^{gg} is, independently, halogen, -CN, -NO₂, -N₃, -SO₂H, -SO₃H, -OH, -OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺X⁻, -NH₂(C₁₋₆ alkyl)⁺X⁻, -NH₃⁺X⁻, -N(OC₁₋₆ alkyl)(C₁₋₆ alkyl), -N(OH)(C₁₋₆ alkyl), -NH(OH), -SH, -SC₁₋₆ alkyl, -SS(C₁₋₆ alkyl), -C(=O)(C₁₋₆ alkyl), -CO₂H, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, - OC(=O)NH(C₁₋₆ alkyl), -NHC(=O)( C₁₋₆ alkyl), -N(C₁₋₆ alkyl)C(=O)( C₁₋₆ alkyl), - NHCO₂(C₁₋₆ alkyl), -NHC(=O)N(C₁₋₆ alkyl)₂, -NHC(=O)NH(C₁₋₆ alkyl), -NHC(=O)NH₂, - C(=NH)O(C₁₋₆ alkyl),-OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, -OC(=NH)N(C₁₋₆ alkyl)₂, -OC(NH)NH(C₁₋₆ alkyl), -OC(NH)NH₂, -NHC(NH)N(C₁₋₆ alkyl)₂, -NHC(=NH)NH₂, -NHSO₂(C₁₋₆ alkyl), - SO₂N(C₁₋₆ alkyl)₂, -SO₂NH(C₁₋₆ alkyl), -SO₂NH₂,-SO₂C₁₋₆ alkyl, -SO₂OC₁₋₆ alkyl, - OSO₂C₁₋₆ alkyl, -SOC₁₋₆ alkyl, -Si(C₁₋₆ alkyl)₃, -OSi(C₁₋₆ alkyl)₃ -C(=S)N(C₁₋₆ alkyl)₂, C(=S)NH(C₁₋₆ alkyl), C(=S)NH₂, -C(=O)S(C₁₋₆ alkyl), -C(=S)SC₁₋₆ alkyl, -SC(=S)SC₁₋₆ alkyl, -P(=O)₂(C₁₋₆ alkyl), -P(=O)(C₁₋₆ alkyl)₂, -OP(=O)(C₁₋₆ alkyl)₂, -OP(=O)(OC₁₋₆ alkyl)₂, C₁₋₆ alkyl, C₁₋₆ haloalkyl, C₂₋₆ alkenyl, C₂₋₆ alkynyl, C₃₋₁₀ carbocyclyl, C₆₋₁₀ aryl, 3-10 membered heterocyclyl, 5-10 membered heteroaryl; or two geminal R^{gg} substituents can be joined to form =O or =S; wherein X⁻ is a counterion.

A "counterion" or "anionic counterion" is a negatively charged group associated with a cationic quaternary amino group in order to maintain electronic neutrality. Exemplary counterions include halide ions (*e.g.,* F⁻, Cl⁻, Br⁻, I⁻), NO₃⁻, ClO₄⁻, OH⁻, H₂PO₄⁻, HSO₄⁻, sulfonate ions (*e.g*., methansulfonate, trifluoromethanesulfonate, p-toluenesulfonate, benzenesulfonate, 10-camphor sulfonate, naphthalene-2-sulfonate, naphthalene-1-sulfonic acid-5-sulfonate, ethan-1-sulfonic acid-2-sulfonate, and the like), and carboxylate ions *(e.g.,* acetate, ethanoate, propanoate, benzoate, glycerate, lactate, tartrate, glycolate, and the like).

These and other exemplary substituents are described in more detail in the **Detailed Description,** and **Claims.** The invention is not intended to be limited in any manner by the above exemplary listing of substituents.

### Other definitions

As used herein, the term "modulation" refers to the inhibition or potentiation of GABA receptor function. A "modulator" (*e.g*., a modulator compound) may be, for example, an agonist, partial agonist, antagonist, or partial antagonist of the GABA receptor.

"Pharmaceutically acceptable" means approved or approvable by a regulatory agency of the Federal or a state government or the corresponding agency in countries other than the United States, or that is listed in the U.S. Pharmacopoeia or other generally recognized pharmacopoeia for use in animals, and more particularly, in humans.

"Pharmaceutically acceptable salt" refers to a salt of a compound of the invention that is pharmaceutically acceptable and that possesses the desired pharmacological activity of the parent compound. In particular, such salts are non-toxic may be inorganic or organic acid addition salts and base addition salts. Specifically, such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, *e.g.,* an alkali metal ion, an alkaline earth ion, or an aluminum ion; or coordinates with an organic base such as ethanolamine, diethanolamine, triethanolamine, N-methylglucamine and the like. Salts further include, by way of example only, sodium, potassium, calcium, magnesium, ammonium, tetraalkylammonium, and the like; and when the compound contains a basic functionality, salts of non-toxic organic or inorganic acids, such as hydrochloride, hydrobromide, tartrate, mesylate, acetate, maleate, oxalate and the like. The term "pharmaceutically acceptable cation" refers to an acceptable cationic counterion of an acidic functional group. Such cations are exemplified by sodium, potassium, calcium, magnesium, ammonium, tetraalkylammonium cations, and the like. See, *e.g.,* Berge, et al., J. Pharm. Sci. (1977) 66(1): 1-79.

"Tautomers" refer to compounds that are interchangeable forms of a particular compound structure, and that vary in the displacement of hydrogen atoms and electrons. Thus, two structures may be in equilibrium through the movement of π electrons and an atom (usually H). For example, enols and ketones are tautomers because they are rapidly interconverted in the presence of either acid or base. Another example of tautomerism is the aci- and nitro- forms of phenylnitromethane, that are likewise formed in the presence of acid or base. Tautomeric forms may be relevant to the attainment of the optimal chemical reactivity and biological activity of a compound of interest.

A "subject" to which administration is contemplated includes, but is not limited to, humans (*i.e.,* a male or female of any age group, *e.g.,* a pediatric subject (*e.g,* infant, child, adolescent) or adult subject *(e.g.,* young adult, middle-aged adult or senior adult)) and/or a non-human animal, *e.g.,* a mammal such as primates *(e.g.,* cynomolgus monkeys, rhesus monkeys), cattle, pigs, horses, sheep, goats, rodents, cats, and/or dogs. In certain embodiments, the subject is a human. In certain embodiments, the subject is a non-human animal.

In certain embodiments, the substituent present on an oxygen atom is an oxygen protecting group (also referred to as a hydroxyl protecting group). Oxygen protecting groups include, but are not limited to, -R^{aa}, -N(R^{bb})₂, -C(=O)SR^{aa}, -C(=O)R^{aa}, -CO₂R^{aa}, - C(=O)N(R^{bb})₂, -C(=NR^{bb})R^{aa}, -C(=NR^{bb})OR^{aa}, -C(=NR^{bb})N(R^{bb})₂, -S(=O)R^{aa}, -SO₂R^{aa}, - Si(R^{aa})₃, -P(R^{cc})₂, -P(R^{cc})₃, -P(=O)₂R^{aa}, -P(=O)(R^{aa})₂, -P(=O)(OR^{cc})₂, -P(=O)₂N(R^{bb})₂, and - P(=O)(NR^{bb})₂, wherein R^{aa}, R^{bb}, and R^{cc} are as defined herein. Oxygen protecting groups are well known in the art and include those described in detail in Protecting Groups in Organic Synthesis, T. W. Greene and P. G. M. Wuts, 3rd edition, John Wiley & Sons, 1999.

Exemplary oxygen protecting groups include, but are not limited to, methyl, methoxylmethyl (MOM), 2-methoxyethoxymethyl (MEM), benzyl (Bn), triisopropylsilyl (TIPS), *t*-butyldimethylsilyl (TBDMS), t-butylmethoxyphenylsilyl (TBMPS), methanesulfonate (mesylate), and tosylate (Ts).

In certain embodiments, the substituent present on an sulfur atom is an sulfur protecting group (also referred to as a thiol protecting group). Sulfur protecting groups include, but are not limited to, -R^{aa}, -N(R^{bb})₂, -C(=O)SR^{aa}, -C(=O)R^{aa}, -CO₂R^{aa}, - C(=O)N(R^{bb})₂, -C(=NR^{bb})R^{aa}, -C(=NR^{bb})OR^{aa}, -C(=NR^{bb})N(R^{bb})₂, -S(=O)R^{aa}, -SO₂R^{aa}, - Si(R^{aa})₃, -P(R^{cc})₂, -P(R^{cc})₃, -P(=O)₂R^{aa}, -P(=O)(R^{aa})₂, -P(=O)(OR^{cc})₂, -P(=O)₂N(R^{bb})₂, and - P(=O)(NR^{bb})₂, wherein R^{aa}, R^{bb}, and R^{cc} are as defined herein. Sulfur protecting groups are well known in the art and include those described in detail in Protecting Groups in Organic Synthesis, T. W. Greene and P. G. M. Wuts, 3rd edition, John Wiley & Sons, 1999.

In certain embodiments, the substituent present on a nitrogen atom is an amino protecting group (also referred to herein as a nitrogen protecting group). Amino protecting groups include, but are not limited to, -OH, -OR^{aa}, -N(R^{cc})₂, -C(=O)R^{aa}, -C(=O)OR^{aa}, - C(=O)N(R^{cc})₂, -S(=O)₂R^{aa}, -C(=NR^{cc})R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, C₁₋₁₀ alkyl, C₂₋₁₀ alkenyl, C₂₋₁₀ alkynyl, C₃₋₁₀ carbocyclyl, 3-14-membered heterocyclyl, C₆₋₁₄ aryl, and 5-14-membered heteroaryl groups, wherein each alkyl, alkenyl, alkynyl, carbocyclyl, heterocyclyl, aryl, and heteroaryl is independently substituted with 0, 1, 2, 3, 4, or 5 R^{dd} groups, and wherein R^{aa}, R^{bb}, R^{cc} and R^{dd} are as defined herein. Amino protecting groups are well known in the art and include those described in detail in Protecting Groups in Organic Synthesis, T. W. Greene and P. G. M. Wuts, 3rd edition, John Wiley & Sons, 1999.

Exemplary amino protecting groups include, but are not limited to amide groups (*e.g.,* -C(=O)R^{aa}), which include, but are not limited to, formamide and acetamide; carbamate groups (*e.g.,* -C(=O)OR^{aa}), which include, but are not limited to, 9-fluorenylmethyl carbamate (Fmoc), *t*-butyl carbamate (BOC), and benzyl carbamate (Cbz); sulfonamide groups (*e.g*., -S(=O)₂R^{aa}), which include, but are not limited to, *p*-toluenesulfonamide (Ts), methanesulfonamide (Ms), and *N*-[2-(trimethylsilyl)ethoxy]methylamine (SEM).

Disease, disorder, and condition are used interchangeably herein.

As used herein, and unless otherwise specified, the terms "treat," "treating" and "treatment" contemplate an action that occurs while a subject is suffering from the specified disease, disorder or condition, which reduces the severity of the disease, disorder or condition, or retards or slows the progression of the disease, disorder or condition. In an alternate embodiment, the present invention contemplates administration of the compounds of the present invention as a prophylactic before a subject begins to suffer from the specified disease, disorder or condition.

In general, the "effective amount" of a compound refers to an amount sufficient to elicit the desired biological response, *e.g.*, to treat a CNS-related disorder, is sufficient to induce anesthesia or sedation. As will be appreciated by those of ordinary skill in this art, the effective amount of a compound of the invention may vary depending on such factors as the desired biological endpoint, the pharmacokinetics of the compound, the disease being treated, the mode of administration, and the age, weight, health, and condition of the subject. An effective amount encompasses therapeutic and prophylactic treatment.

As used herein, and unless otherwise specified, a "therapeutically effective amount" of a compound is an amount sufficient to provide a therapeutic benefit in the treatment of a disease, disorder or condition, or to delay or minimize one or more symptoms associated with the disease, disorder or condition. A therapeutically effective amount of a compound means an amount of therapeutic agent, alone or in combination with other therapies, which provides a therapeutic benefit in the treatment of the disease, disorder or condition. The term "therapeutically effective amount" can encompass an amount that improves overall therapy, reduces or avoids symptoms or causes of disease or condition, or enhances the therapeutic efficacy of another therapeutic agent.

As used herein, and unless otherwise specified, a "prophylactically effective amount" of a compound is an amount sufficient to prevent a disease, disorder or condition, or one or more symptoms associated with the disease, disorder or condition, or prevent its recurrence. A prophylactically effective amount of a compound means an amount of a therapeutic agent, alone or in combination with other agents, which provides a prophylactic benefit in the prevention of the disease, disorder or condition. The term "prophylactically effective amount" can encompass an amount that improves overall prophylaxis or enhances the prophylactic efficacy of another prophylactic agent.

### Compounds

In an aspect, provided herein is a compound of Formula **(I-Ia)** 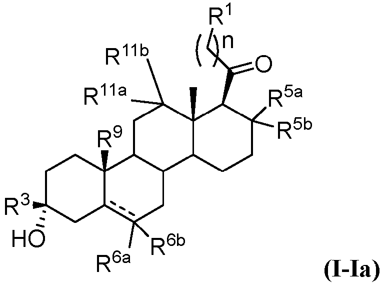 or a pharmaceutically acceptable salt thereof, wherein
R³ is substituted or unsubstituted C₁₋₃ alkyl;
R⁹ is hydrogen or unsubstituted C₁₋₃ alkyl;
each of R^{5a}, R^{5b}, R^{6a}, R^{6b}, R^{11a}, and R^{11b} is hydrogen;
R¹ is substituted or unsubstituted alkyl, substituted or unsubstituted carbocyclyl, substituted or unsubstituted heterocyclyl, substituted or unsubstituted aryl, substituted or unsubstituted heteroaryl, -OR^{A1}, -SR^{A1}, -N(R^{A1})₂, -N(R^{A1}), -OC(=O)R^{A1}, -OC(=O)OR^{A1}, - OC(=O)SR^{A1}, -OC(=O)N(R^{A1})₂, -SC(=O)R^{A2}, -SC(=O)OR^{A1}, -SC(=O)SR^{A1}, -SC(=O)N(R^{A1})₂, -NHC(=O)R^{A1}, -NHC(=O)OR^{A1}, -NHC(=O)SR^{A1}, -NHC(=O)N(R^{A1})₂, -OS(=O)₂R^{A2} -OS(=O)₂OR^{A1}, -S-S(=O)₂R^{A2}, -S-S(=O)₂OR^{A1}, -S(=O)R^{A2}, -SO₂R^{A2}, or -S(=O)₂OR^{A1}
wherein each instance of R^{A1} is independently hydrogen, substituted or unsubstituted alkyl, substituted or unsubstituted carbocyclyl, substituted or unsubstituted heterocyclyl, substituted or unsubstituted aryl, substituted or unsubstituted heteroaryl, or two R^{A1} groups are joined to form an substituted or unsubstituted heterocyclic or heteroaryl ring; and R^{A2} is substituted or unsubstituted alkyl, substituted or unsubstituted carbocyclyl, substituted or unsubstituted heterocyclyl, substituted or unsubstituted aryl, or substituted or unsubstituted heteroaryl; and
n is 0, 1, 2, or 3; and
wherein - - - - - - represents a single bond.

### Formula (I-I): Group R³

According to the invention, R³ is substituted or unsubstituted C₁₋₃-alkyl.

In some embodiments, R³ is substituted C₁₋₃-alkyl. In some embodiments, R³ is unsubstituted C₁₋₃-alkyl. In some embodiments, R³ is methyl.

### Formula (I-I): Group R⁹

In some aspects, R⁹ is hydrogen.

In some embodiments, R⁹ is unsubstituted C₁₋₃-alkyl.

In some embodiments, R⁹ is methyl In some aspects, R⁹ is ethyl.

### Formula (I-I): Groups R^{6a} and R^{6b}

According to the invention, both R^{6a} and R^{6b} are hydrogen.

### Formula (I-I): Groups R^{11a} and R^{11b}

According to the invention, R^{11a} and R^{11b} are both hydrogen.

### Formula (I-I): Groups R^{5a} and R^{5b}

According to the invention, R^{5a} and R^{5b} are each hydrogen.

According to the invention, the compound is of Formula **(I-Ia)** 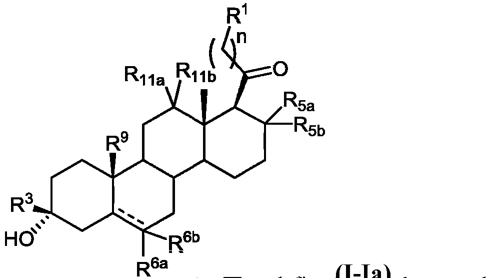 or a pharmaceutically acceptable salt thereof; as defined both above and in claim 1.

In some embodiments, the compound is of Formula **(I-Ib)** 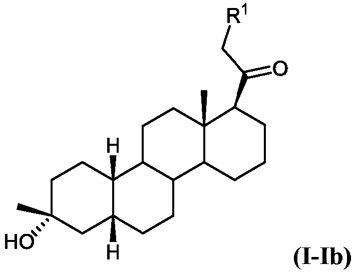 or a pharmaceutically acceptable salt thereof.

In some embodiments, R¹ is substituted or unsubstituted alkyl, substituted or unsubstituted carbocyclyl, substituted or unsubstituted heterocyclyl, substituted or unsubstituted aryl, substituted or unsubstituted heteroaryl.

In some embodiments, R¹ is 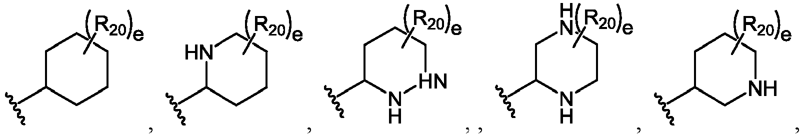 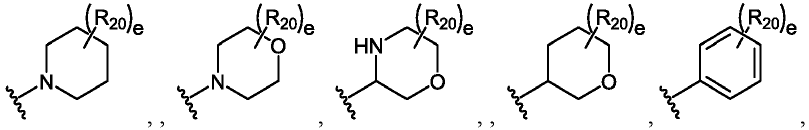 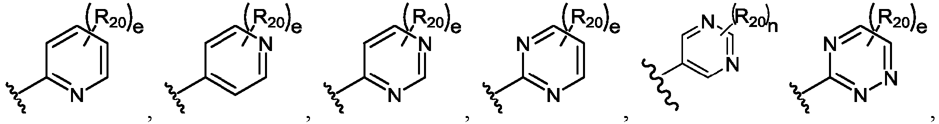 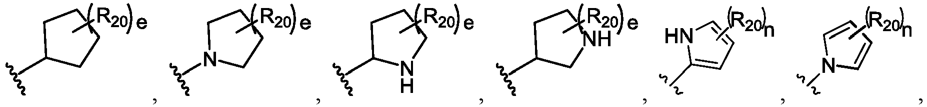 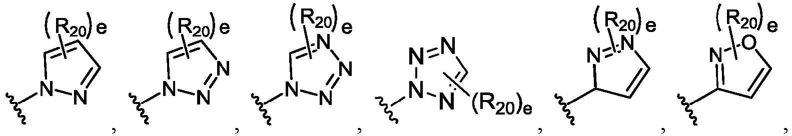 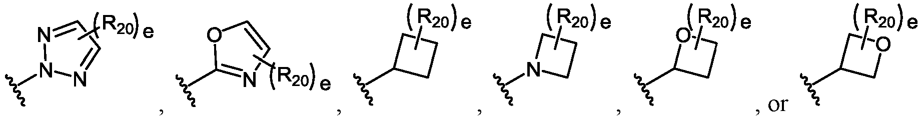 wherein each instance of R₂₀ is, independently, halogen, -NO₂, -CN, -OR^{GA}, -N(R^{GA})₂, - C(=O)R^{GA}, -C(=O)OR^{GA}, -OC(=O)R^{GA}, -OC(=O)OR^{GA}, -C(=O)N(R^{GA})₂, -N(R^{GA})C(=O)R^{GA}, -OC(=O)N(R^{GA})₂, -N(R^{GA})C(=O)OR^{GA}, -S(=O)₂R^{GA}, -S(=O)₂OR^{GA}, -OS(=O)₂R^{GA}, - S(=O)₂N(R^{GA})₂, or -N(R^{GA})S(=O)₂R^{GA}; substituted or unsubstituted C₁₋₆ alkyl, substituted or unsubstituted C₂₋₆ alkenyl, substituted or unsubstituted C₂₋₆ alkynyl, substituted or unsubstituted C₃₋₄ carbocylyl, substituted or unsubstituted 3- to 4- membered heterocylyl, or optionally two R^{GA} are taken with the intervening atoms to form a substituted or unsubstituted 3- to 4- membered carbocyclic or heterocyclic ring; wherein each instance of R^{GA} is independently hydrogen, substituted or unsubstituted C₁₋₆ alkyl, substituted or unsubstituted C₂₋₆ alkenyl, substituted or unsubstituted C₂₋₆ alkynyl, substituted or unsubstituted C₃₋₆ carbocylyl, substituted or unsubstituted 3- to 6- membered heterocylyl, substituted or unsubstituted aryl, substituted or unsubstituted heteroaryl, an oxygen protecting group when attached to oxygen, a nitrogen protecting group when attached to nitrogen, or two R^{GA} groups are taken with the intervening atoms to form a substituted or unsubstituted carbocyclic or heterocyclic ring; and n or e is 0, 1, 2, 3, 4, or 5.

In some embodiments, R¹ is 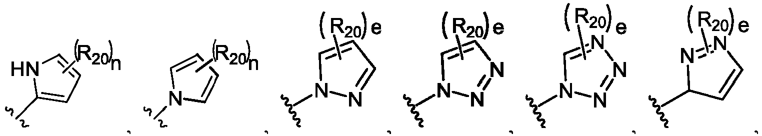 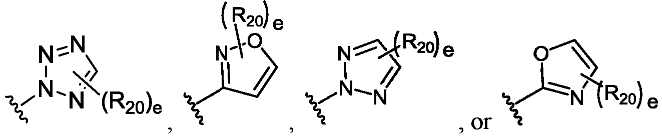 wherein each instance of R₂₀ is, independently, halogen, -NO₂, -CN, -OR^{GA}, -N(R^{GA})₂, - C(=O)R^{GA}, -C(=O)OR^{GA}, -OC(=O)R^{GA}, -OC(=O)OR^{GA}, -C(=O)N(R^{GA})₂, -N(R^{GA})C(=O)R^{GA}, -OC(=O)N(R^{GA})₂, -N(R^{GA})C(=O)OR^{GA}, -S(=O)₂R^{GA}, -S(=O)₂OR^{GA}, -OS(=O)₂R^{GA}, - S(=O)₂N(R^{GA})₂, or -N(R^{GA})S(=O)₂R^{GA}; substituted or unsubstituted C₁₋₆ alkyl, substituted or unsubstituted C₂₋₆ alkenyl, substituted or unsubstituted C₂₋₆ alkynyl, substituted or unsubstituted C₃₋₄ carbocylyl, substituted or unsubstituted 3- to 4- membered heterocylyl, or optionally two R^{GA} are taken with the intervening atoms to form a substituted or unsubstituted 3- to 4- membered carbocyclic or heterocyclic ring; wherein each instance of R^{GA} is independently hydrogen, substituted or unsubstituted C₁₋₆ alkyl, substituted or unsubstituted C₂₋₆ alkenyl, substituted or unsubstituted C₂₋₆ alkynyl, substituted or unsubstituted C₃₋₆ carbocylyl, substituted or unsubstituted 3- to 6- membered heterocylyl, substituted or unsubstituted aryl, substituted or unsubstituted heteroaryl, an oxygen protecting group when attached to oxygen, a nitrogen protecting group when attached to nitrogen, or two R^{GA} groups are taken with the intervening atoms to form a substituted or unsubstituted carbocyclic or heterocyclic ring; and n or e is 0, 1, 2, 3, 4, or 5.

In some embodiments, the compound is of Formula **(I-Id)** 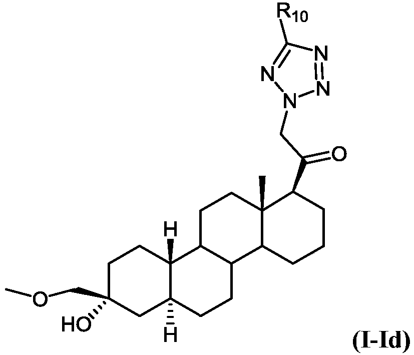 or a pharmaceutically acceptable salt thereof; wherein R₁₀ is independently hydrogen, halogen, substituted or unsubstituted alkyl, hydroxyl, or cyano.

In some embodiments, the compound is of Formula **(I-Ie)** 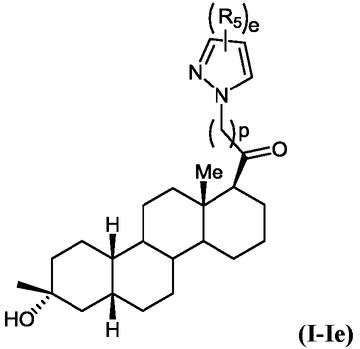 or a pharmaceutically acceptable salt thereof, wherein e is 0, 1, 2 or 3; p is 0, 1, 2, or 3; each R₅ is independently halogen, alkyl, hydroxyl, or cyano.

In some embodiments, the compound is of Formula **(I-If)** 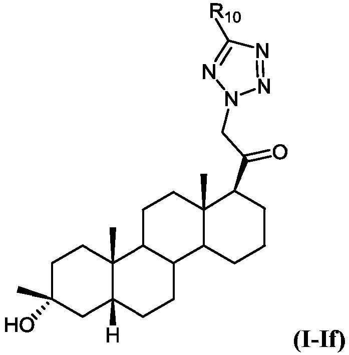 or a pharmaceutically acceptable salt thereof; wherein R₁₀ is independently hydrogen, halogen, substituted or unsubstituted alkyl, hydroxyl, or cyano.

In some embodiments, the compound is of Formula **(I-Ig)** 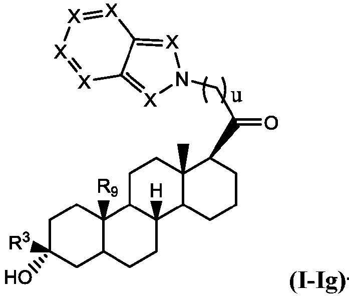
or a pharmaceutically acceptable salt thereof, wherein u is 0, 1, or 2; each X is independently -C(R^{N})-, -C(R^{N})₂-, -O-, -S-, -N-, or N(R^{N})- wherein R^{N} is independently hydrogen, substituted or unsubstituted C₁₋₆ alkyl, C(=O)R^{GA}, -C(=O)OR^{GA}, -C(=O)N(R^{GA})₂, - S(=O)₂R^{GA}, or -S(=O)₂N(R^{GA})₂; and
each instance of R^{GA} is independently hydrogen, substituted or unsubstituted C₁₋₆ alkyl, substituted or unsubstituted C₂₋₆ alkenyl, substituted or unsubstituted C₂₋₆ alkynyl, substituted or unsubstituted C₃₋₆ carbocylyl, substituted or unsubstituted 3- to 6- membered heterocyclyl, substituted or unsubstituted aryl, substituted or unsubstituted heteroaryl, an oxygen protecting group when attached to oxygen, nitrogen protecting group when attached to nitrogen, or two R^{GA} groups are taken with the intervening atoms to form a substituted or unsubstituted heterocyclyl or heteroaryl ring.

In some embodiments, the compound is of Formula **(I-Ih)** 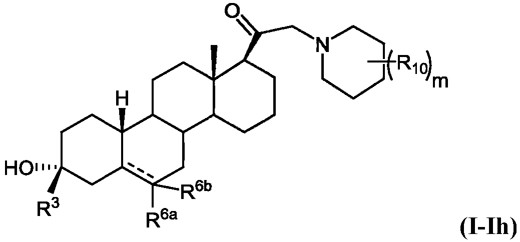 or a pharmaceutically acceptable salt thereof, wherein each R₁₀ is independently halogen, alkyl, hydroxyl, or cyano; and m is 0, 1, 2 or 3.

In some embodiments, the compound is of Formula **(I-Ii)** 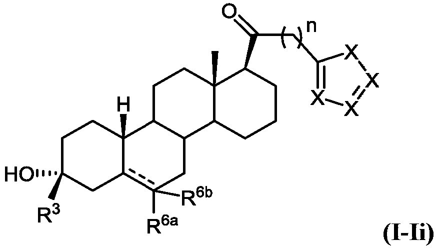
or a pharmaceutically acceptable salt thereof, wherein n is 0, 1, or 2; and each X is independently -C(R^{N})-, -C(R^{N})₂-, -O-, -S-, -N-, or N(R^{N})- wherein R^{N} is independently hydrogen, substituted or unsubstituted C1-6 alkyl, C(=O)R^{GA}, -C(=O)OR^{GA}, -C(=O)N(R^{GA})₂, -S(=O)₂R^{GA}, or -S(=O)₂N(R^{GA})₂; and
each instance of R^{GA} is independently hydrogen, substituted or unsubstituted C₁₋₆ alkyl, substituted or unsubstituted C₂₋₆ alkenyl, substituted or unsubstituted C₂₋₆ alkynyl, substituted or unsubstituted C₃₋₆ carbocylyl, substituted or unsubstituted 3- to 6- membered heterocylyl, substituted or unsubstituted aryl, substituted or unsubstituted heteroaryl, an oxygen protecting group when attached to oxygen, nitrogen protecting group when attached to nitrogen, or two R^{GA} groups are taken with the intervening atoms to form a substituted or unsubstituted heterocylyl or heteroaryl ring.

In some embodiments, the compound is selected from the group consisting of the compounds identified in **Table I-1** below. Of the compounds of table I-1, compounds I-E4, I-14, 1-15, 1-16, I-17, I-D7, I-D9, I-20, I-G14, I-G16, I-M3, 1-25, I-26, 1-27, I-K2, I-B11, I-P9, I-L14, I-L17, I-L19, I-S3, I-D7a, I-20a, I-G16a, I-S3a and I-U11 are compounds according to the present invention. Of these compounds, compounds I-D7a and I-S3a do not fall within the scope of formula I-Ia, as they have the wrong stereochemistry at position 17a. However, these are still compounds of the present invention and are claimed per se with all of the other compounds mentioned above (which do fall within the scope of formula I-Ia) in independent claim 11. All other compounds of table I-1 are for the purposes of comparison only.

**Table I-1.**

| **STRUCTURE** | **ID** |
|---|---|
| 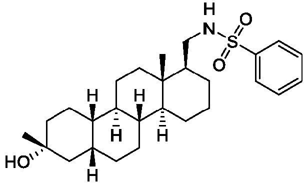 | **I-A7** |
| 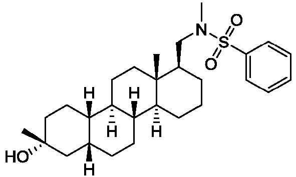 | **I-A8** |
| 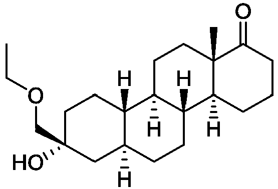 | **I-B6** |
| 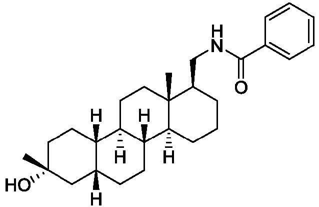 | **I-C4** |
| 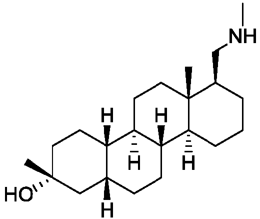 | **I-C5** |
| 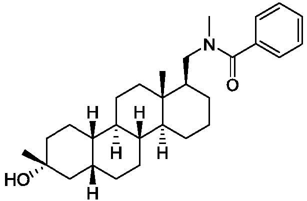 | **I-C6** |
| 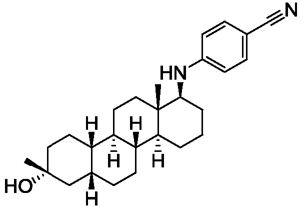 | **I-7** |
| 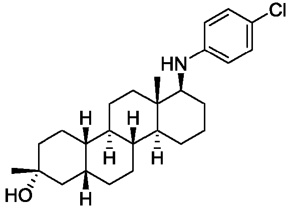 | **I-8** |
| 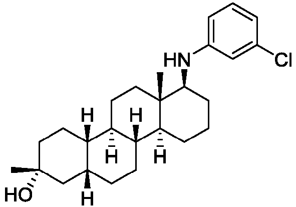 | **I-9** |
| 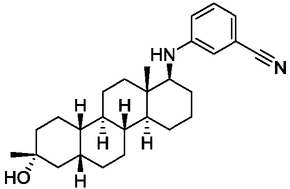 | **I-10** |
| 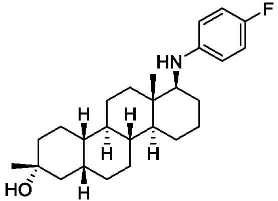 | **I-11** |
| 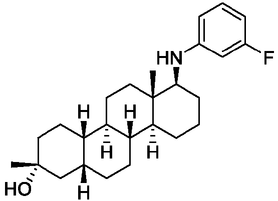 | **I-12** |
| 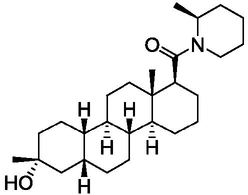 | **I-E4** |
| 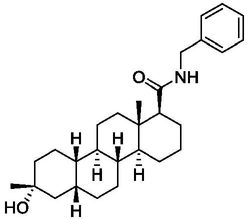 | **I-14** |
| 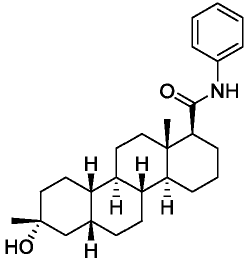 | **I-15** |
| 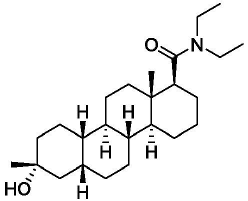 | **I-16** |
| 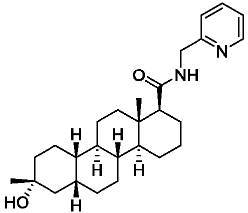 | **I-17** |
| 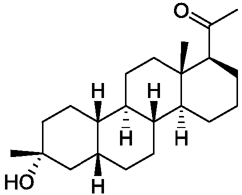 | **I-D7** |
| 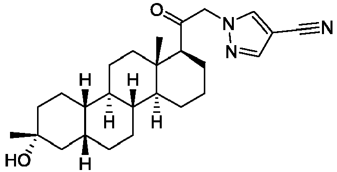 | **I-D9** |
| 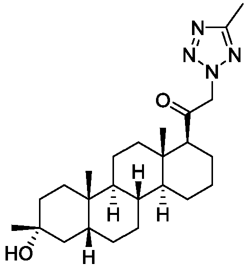 | **I-20** |
| 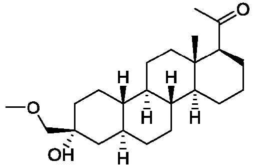 | **I-G14** |
| 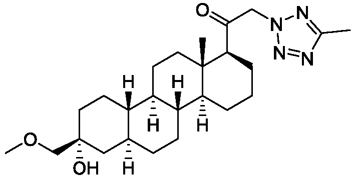 | **I-G16** |
| 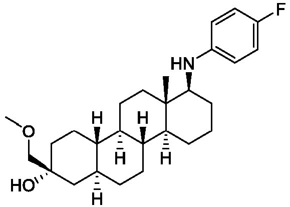 | **I-23** |
| 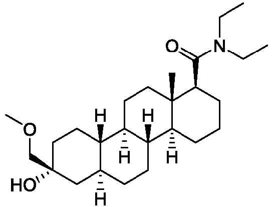 | **I-M3** |
| 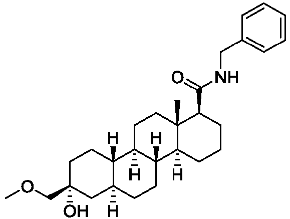 | **I-25** |
| 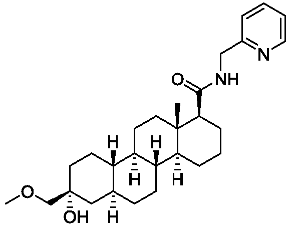 | **I-26** |
| 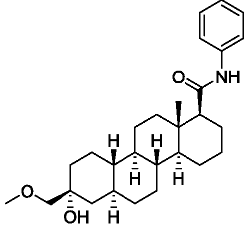 | **I-27** |
| 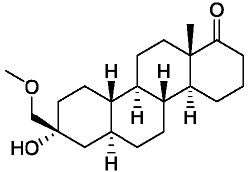 | **I-H5** |
| 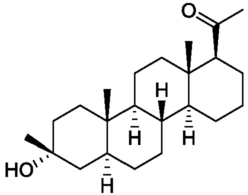 | **I-K2** |
| 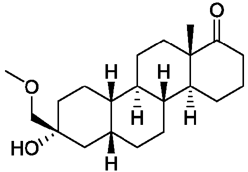 | **I-L6** |
| 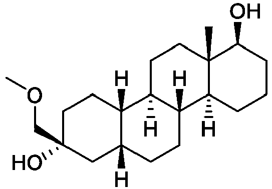 | **I-L8** |
| 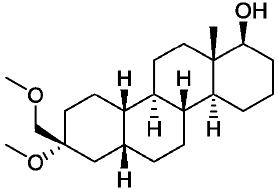 | **I-L9** |
| 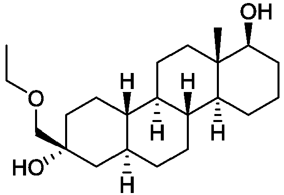 | **I-39** |
| 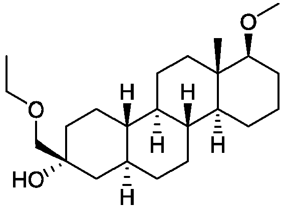 | **I-40** |
| 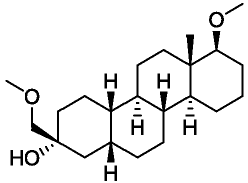 | **I-N11** |
| 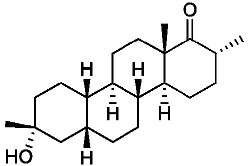 | **I-50** |
| 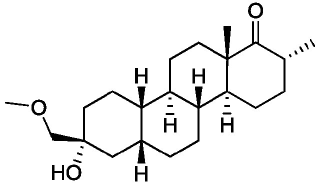 | **I-53** |
| 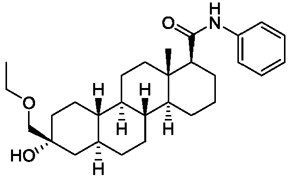 | **I-B11** |
| 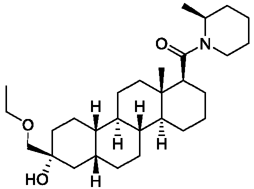 | **I-P9** |
| 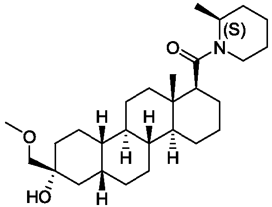 | **I-L14** |
| 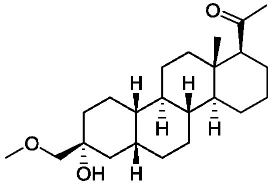 | **I-L17** |
| 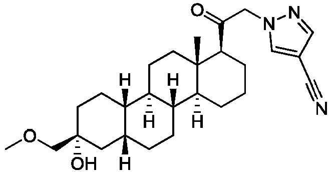 | **I-L19** |
| 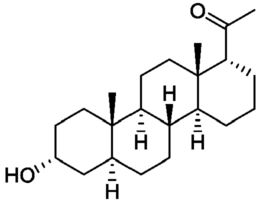 | **I-Q8a** |
| 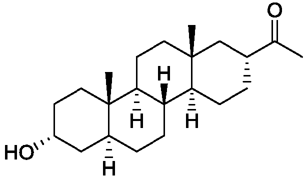 | **I-Q8** |
| 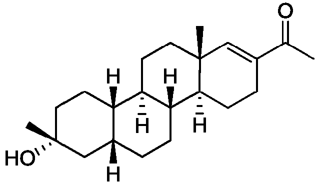 | **I-R13** |
| 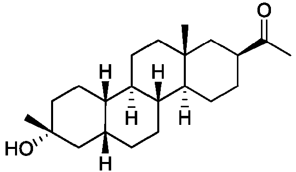 | **I-R14** |
| 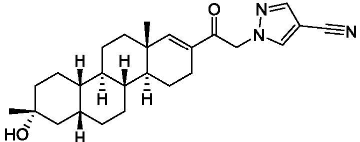 | **I-R16** |
| 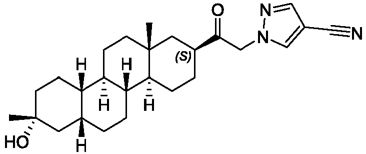 | **I-R17** |
| 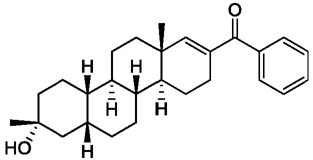 | **I-R20** |
| 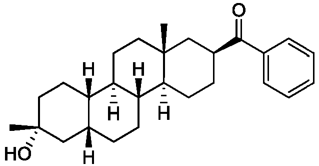 | **I-R21** |
| 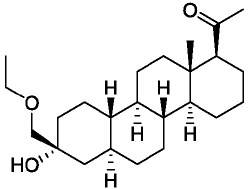 | **I-S3** |
| 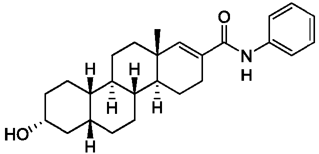 | **I-R23** |
| 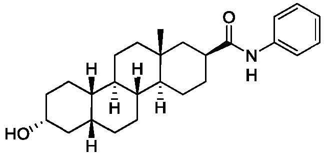 | **I-R24** |
| 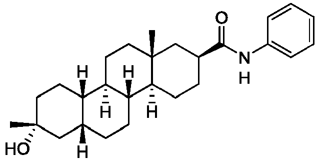 | **I-R26** |
| 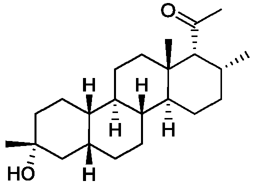 | **I-T6a** |
| 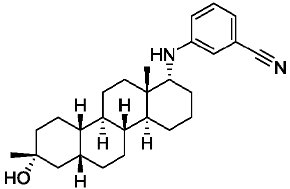 | **I-10a** |
| 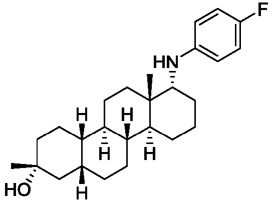 | **I-11a** |
| 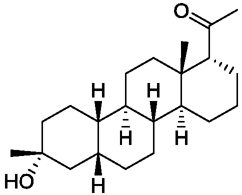 | **I-D7a** |
| 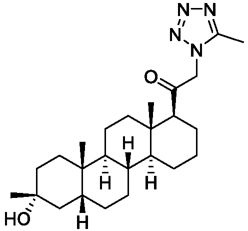 | **I-20a** |
| 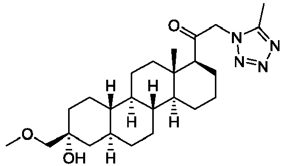 | **I-G16a** |
| 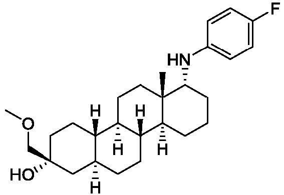 | **I-23a** |
| 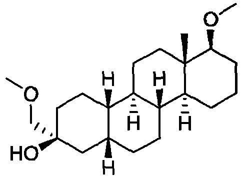 | **I-N11a** |
| 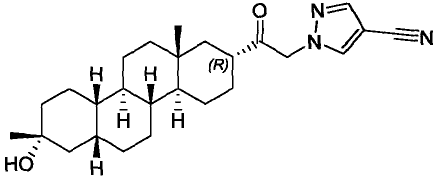 | **I-R17a** |
| 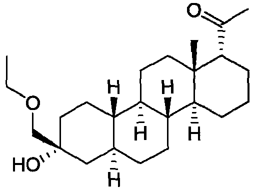 | **I-S3a** |
| 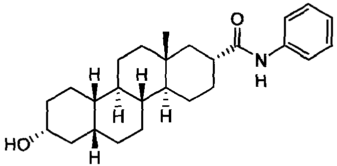 | **I-R24a** |
| 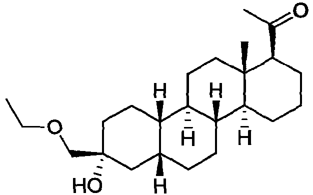 | **I-U11** |

Table I-2 below lists compounds which are not part of the present invention and which are all disclosed herein for the purposes of comparison only:

**Table I-2**

| **STRUCTURE** | **Compound ID** |
|---|---|
| 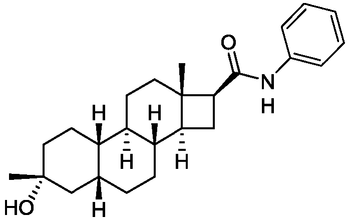 | **I-AA6** |
| 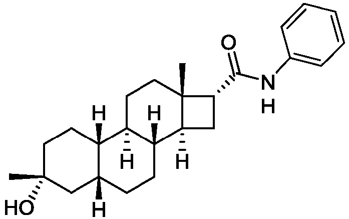 | **I-AA6a** |
| 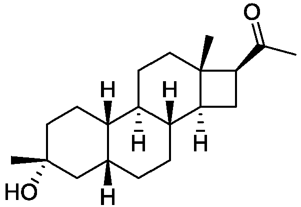 | **I-AA8** |
| 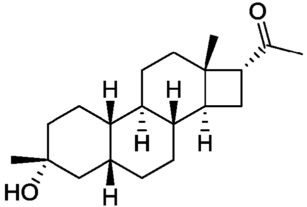 | **I-AA8a** |
| 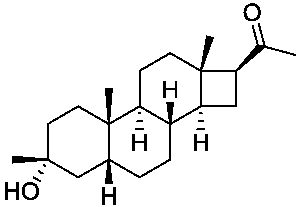 | **I-103** |
| 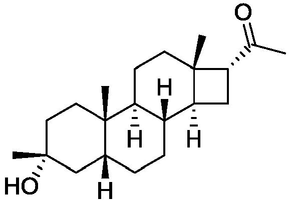 | **I-103a** |
| 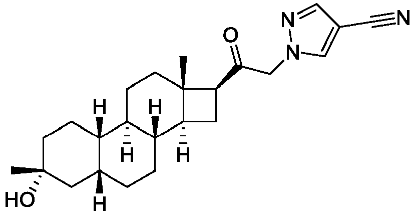 | **I-AA10** |
| 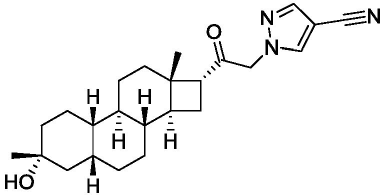 | **I-105** |
| 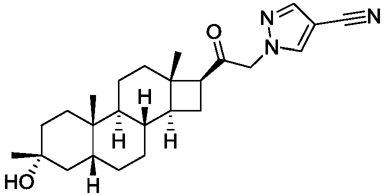 | **I-106** |
| 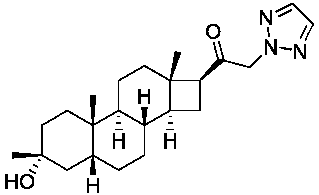 | **I-107** |
| 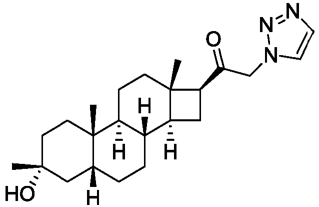 | **I-107a** |
| 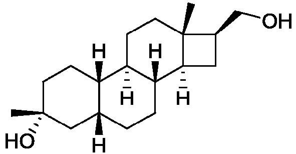 | **I-AB1** |
| 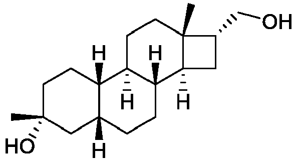 | **I-AB1a** |
| 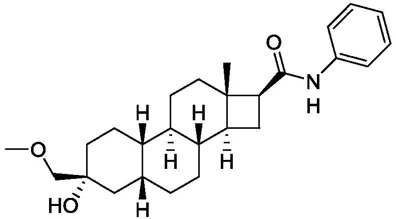 | **I-AC8** |
| 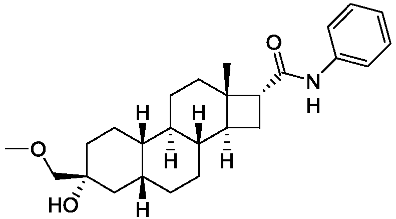 | **I-AC8a** |
| 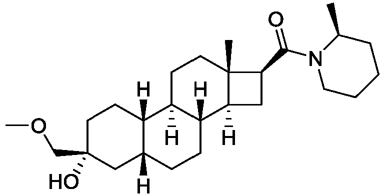 | **I-AC9** |
| 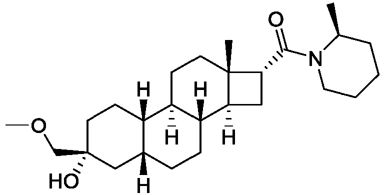 | **I-AC9a** |
| 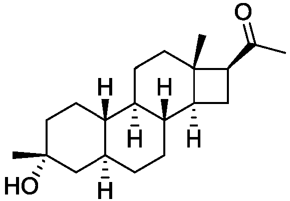 | **I-AD8** |
| 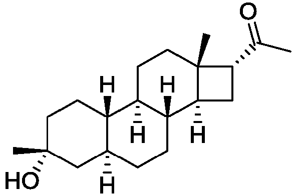 | **I-AD8a** |
| 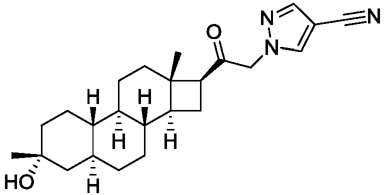 | **I-AD10** |
| 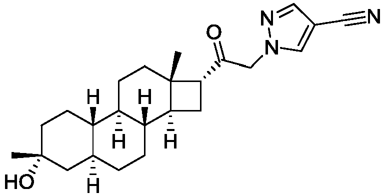 | **I-AD10a** |
| 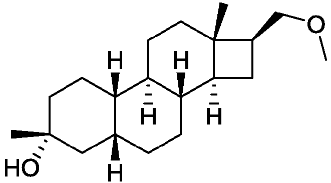 | **I-117** |
| 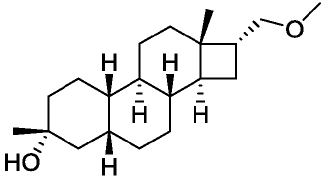 | **I-118** |
| 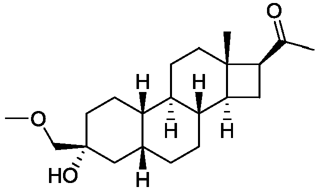 | **I-AC11** |
| 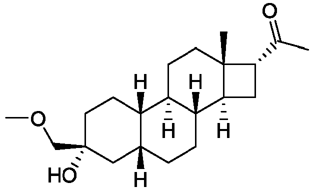 | **I-AC12** |
| 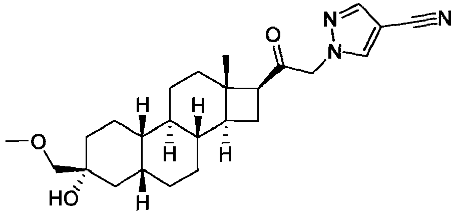 | **I-AC14** |
| 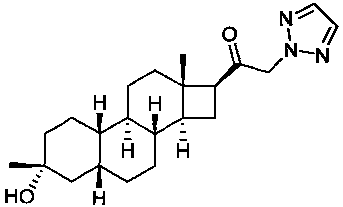 | **I-AA11** |
| 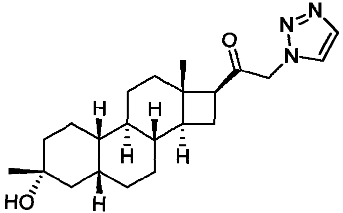 | **I-AA12** |

Table I-3 below lists compounds which are not part of the present invention and which are all disclosed herein for the purposes of comparison only:

**Table I-3**

| **STRUCTURE** | **Compound ID** |
|---|---|
| 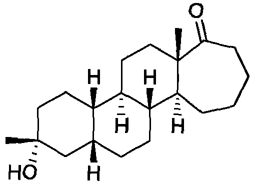 | **I-BA3** |
| 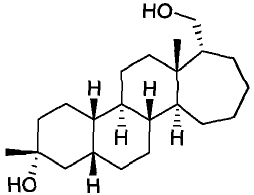 | **I-BA5a** |
| 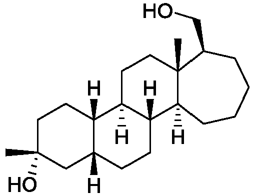 | **I-BA5b** |
| 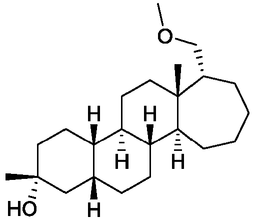 | **I-BA6a** |
| 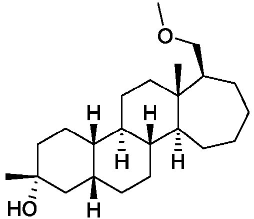 | **I-BA6** |
| 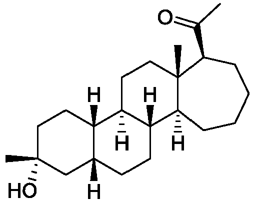 | **I-BA9a** |
| 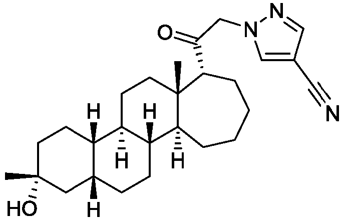 | **I-BA11** |
| 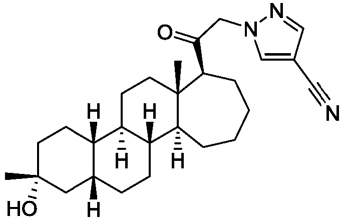 | **I-BA11a** |
| 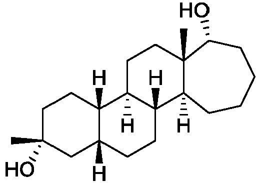 | **I-BA12** |
| 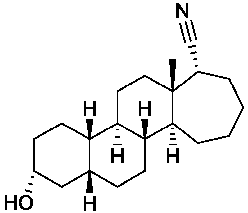 | **I-BA16a** |
| 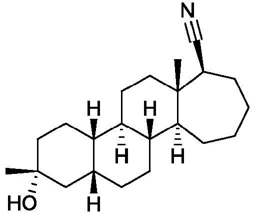 | **I-BA16** |
| 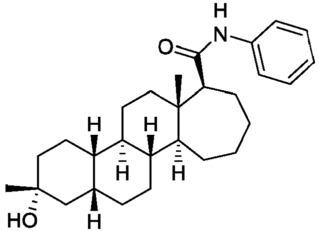 | **I-BA17** |
| 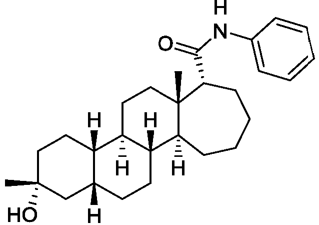 | **I-BA17a** |
| 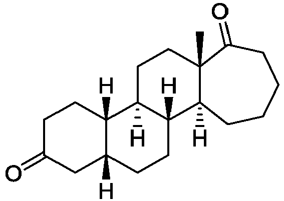 | **I-BB6** |
| 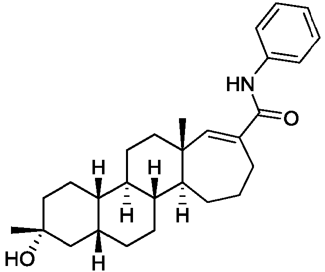 | **I-BC10** |
| 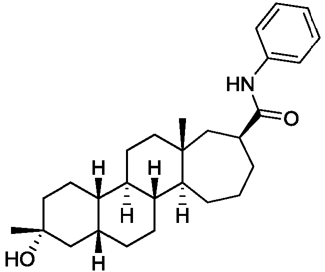 | **I-BC11** |
| 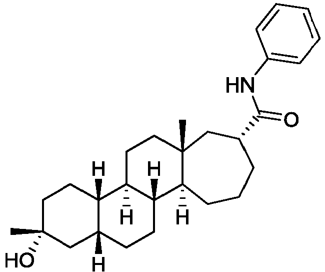 | **I-BC11a** |
| 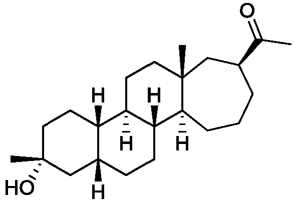 | **I-BC14a** |
| 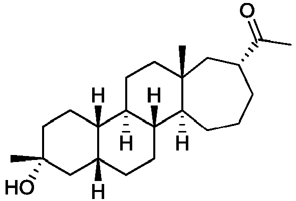 | **I-BC14b** |
| 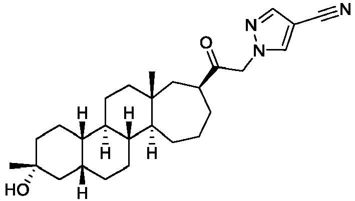 | **I-BC16** |
| 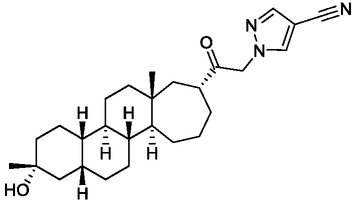 | **I-BC16a** |

It should be appreciated that formulas described herein may reference particular carbon atoms, such as C17, C3, C19, etc. These references are based on the position of carbon atoms according to steroid nomenclature known and used in the industry, as shown below: 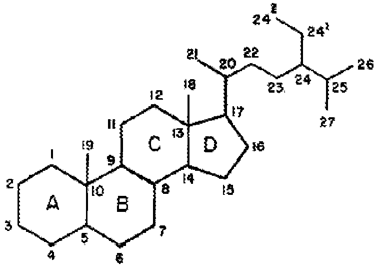 For example, C17 refers to the carbon at position 17 and C3 refers to the carbon at position 3. It should be appreciated that the stereochemistry at C17 could be depicted in any of the following but equivalent ways: 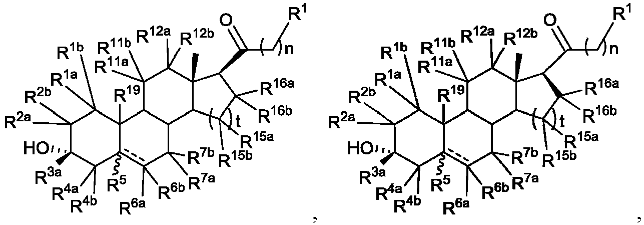 or

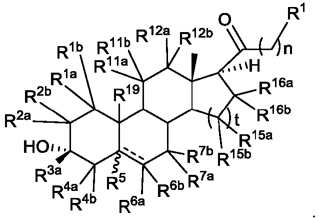

Table II-1 below lists compounds which are not part of the present invention and which are all disclosed herein for the purposes of comparison only:

**Table II-1**

| **Compound ID** | **Structure** |
|---|---|
| **II-C2a** | 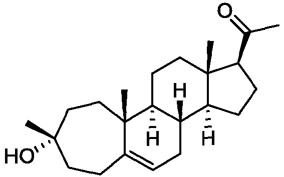 |
| **II-C2b** | 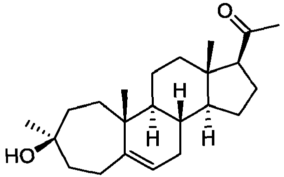 |
| **II-C3** | 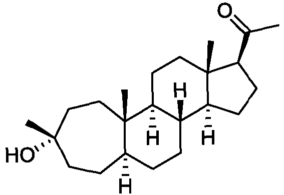 |
| **II-C5** | 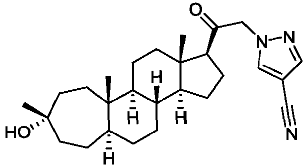 |
| **II-B5** | 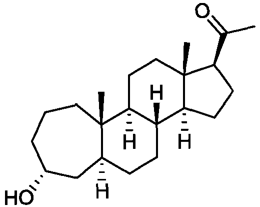 |
| **II-C6** | 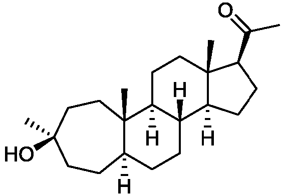 |
| **II-C8** | 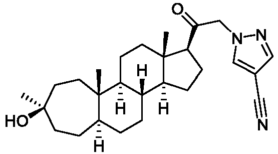 |
| **II-D2a** | 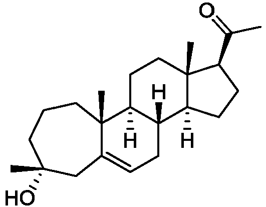 |
| **II-D2b** | 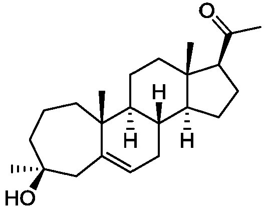 |
| **II-D3b** | 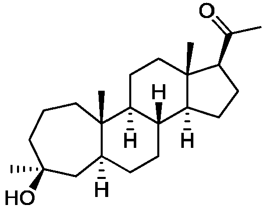 |
| **II-D3a** | 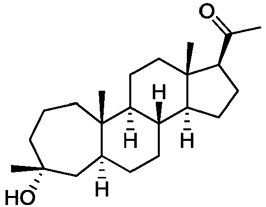 |
| **II-D5a** | 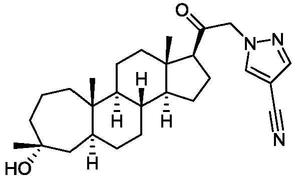 |
| **II-D5b** | 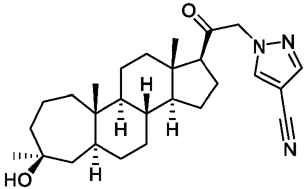 |
| **II-E7a** | 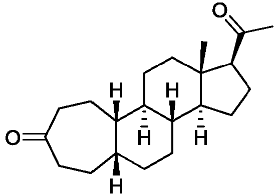 |
| **II-E7b** | 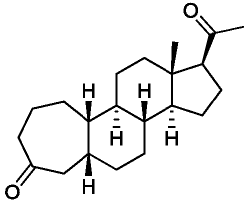 |
| **II-E8** | 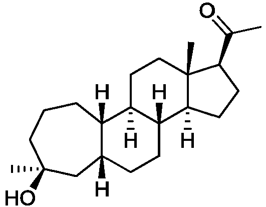 |
| **II-E8a** | 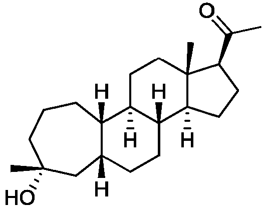 |
| **II-E10** | 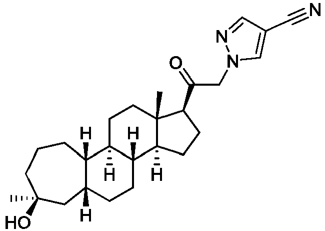 |
| **II-E10a** | 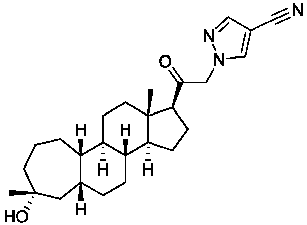 |
| **II-C10** | 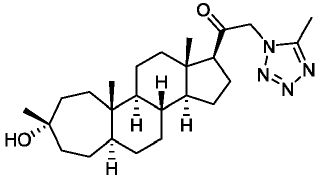 |
| **II-C11** | 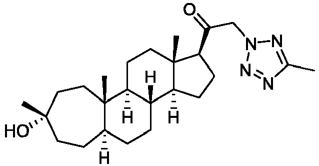 |
| **II-C12** | 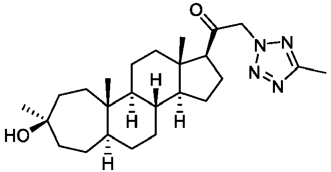 |
| **II-F3** | 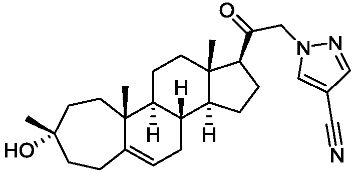 |
| **II-F6** | 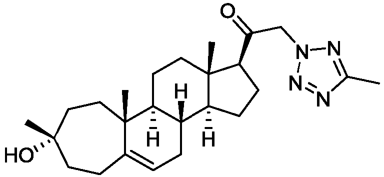 |
| **II-F7** | 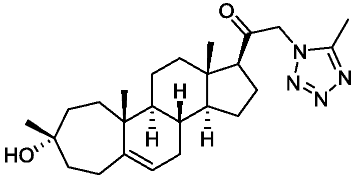 |
| **II-F8** | 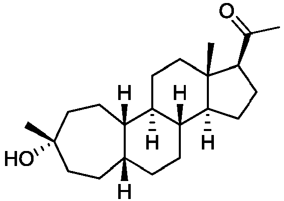 |
| **II-F9** | 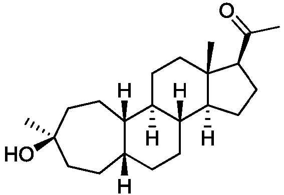 |
| **II-F11** | 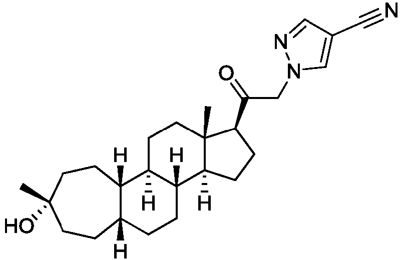 |
| **II-F12** | 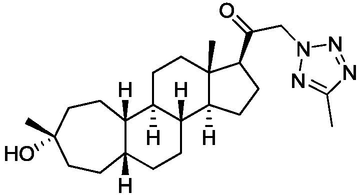 |
| **II-F13** | 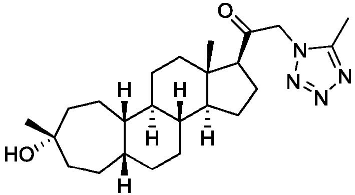 |
| **II-F19** | 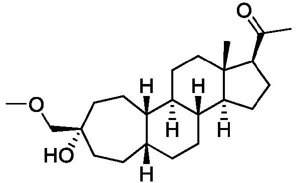 |
| **II-F21** | 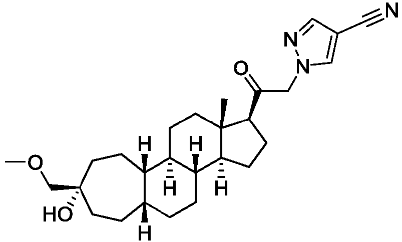 |
| **II-G8** | 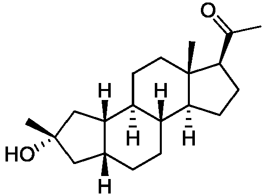 |
| **II-G10** | 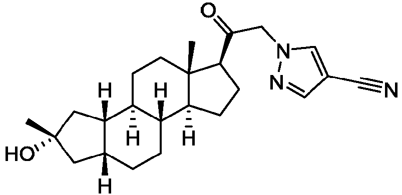 |
| **II-H11** | 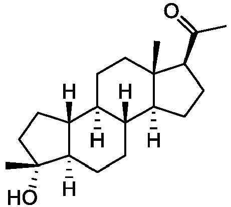 |
| **II-H13** | 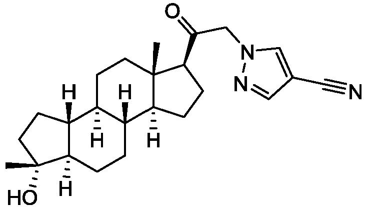 |
| **II-H17** | 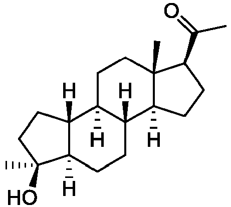 |
| **II-H19** | 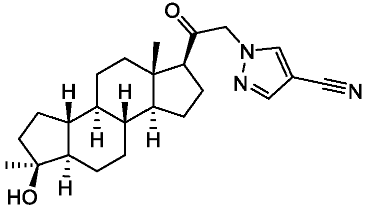 |
| **II-J9** | 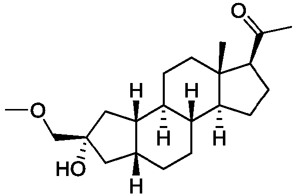 |
| **II-J11** | 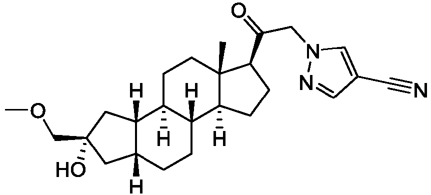 |
| **II-G11** | 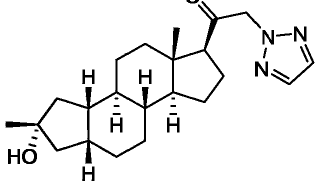 |
| **II-G12** | 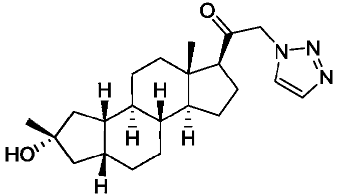 |

Table IV-1 below lists compounds which are not part of the present invention and which are all disclosed herein for the purposes of comparison only:

**Table IV-1.**

| **Intermediate** | **STRUCTURE** |
|---|---|
| IV-A14 | 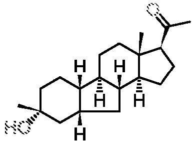 |
| IV-A16 | 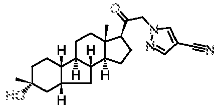 |
| IV-B7 | 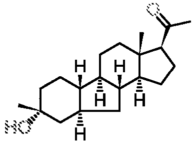 |
| IV-B9 | 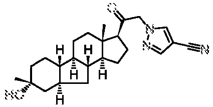 |

Table V-1 below lists compounds which are not part of the present invention and which are all disclosed herein for the purposes of comparison only:

**Table V-1:**

| **Compound ID** | **STRUCTURE** |
|---|---|
| V**-A8** | 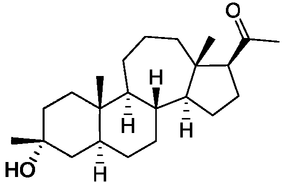 |
| V**-A10** | 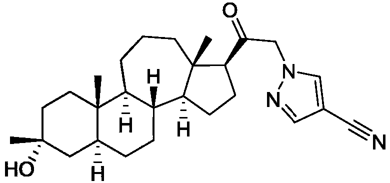 |

In one aspect, provided herein is a pharmaceutically acceptable salt of a compound of the invention according to formula (I-Ia) and sub-formulae thereof, as defined above and in claims 1-10 and also as identified as being part of the invention in the introduction to table I-1 and as appearing in independent claim 11.

In one aspect, provided herein is a pharmaceutical composition comprising a compound of the invention according to formula (I-Ia) and sub-formulae thereof, as defined above and in claims 1-10 and also as identified as being part of the invention in the introduction to table I-1 and as appearing in independent claim 11. In certain embodiments, the compound of the present invention is provided in an effective amount in the pharmaceutical composition. In certain embodiments, the compound of the present invention is provided in a therapeutically effective amount. In certain embodiments, the compound of the present invention is provided in a prophylactically effective amount.

Compounds of the present invention as described herein, act, in certain embodiments, as GAB_{A} modulators, *e.g.*, effecting the GABA_{A} receptor in either a positive or negative manner. As modulators of the excitability of the central nervous system (CNS), as mediated by their ability to modulate GABA_{A} receptor, such compounds are expected to have CNS-activity.

Thus, in another aspect, provided are methods of treating a CNS-related disorder in a subject in need thereof, comprising administering to the subject an effective amount of a compound of the present invention. In certain embodiments, CNS-related disorder is a sleep disorder, a mood disorder, a schizophrenia spectrum disorder, a convulsive disorder, a disorder of memory and/or cognition, a movement disorder, a personality disorder, autism spectrum disorder, pain, traumatic brain injury, a vascular disease, a substance abuse disorder and/or withdrawal syndrome, tinnitus, or status epilepticus. In certain embodiments, the CNS-related disorder is depression. In certain embodiments, the CNS-related disorder is postpartum depression. In certain embodiments, the CNS-related disorder is major depressive disorder. In certain embodiments, the major depressive disorder is moderate major depressive disorder. In certain embodiments, the major depressive disorder is severe major depressive disorder. In certain embodiments, the compound is administered orally, subcutaneously, intravenously, or intramuscularly. In certain embodiments, the compound is administered orally. In certain embodiments, the compound is administered chronically. In certain embodiments, the compound is administered continuously, e.g., by continuous intravenous infusion.

As used herein, an "episodic dosing regimen" is a dosing regimen wherein a compound of a Formula described herein or a composition comprising a compound of a Formula described herein is administered to a subject for a finite period of time in response to the diagnosis of a disorder or symptom thereof, e.g, a diagnosis or symptom of depression. an episode of major depressive disorder, bipolar depression, anxiety, or postpartum depression. In some embodiments, the major depressive disorder is moderate major depressive disorder. In some embodiments, the major depressive disorder is severe major depressive disorder In some embodiments, the compound is formulated as individual dosage units, each unit comprising a compound of a Formula described herein and one or more suitable pharmaceutical excipients. In some embodiments, the episodic dosing regimen has a duration of a plurality of weeks, e.g. about 8 weeks. In contrast with chronic administration as defined herein, episodic dosing of a compound occurs over a finite period of time, e.g., from about 2 weeks to about 8 weeks, in response to a diagnosis of a disorder, e.g., depression, or a symptom thereof. In some embodiments, episodic dosing occurs once per day across a plurality of weeks, e.g., from about 2 weeks to about 6 weeks. In one embodiment, the episodic dosing has a duration of two weeks. In some embodiments, more than one episodic dosing regimen is administered to the subject, e.g., two or more episodic regimens throughout the subject's life.

Exemplary compounds of the invention may be synthesized from the following known starting materials using methods known to one skilled in the art or certain references, In one aspect, provided herein is a pharmaceutically acceptable salt of a compound of the invention.

### Alternative Embodiments

In an alternative embodiment, compounds described herein may also comprise one or more isotopic substitutions. For example, hydrogen may be ²H (D or deuterium) or ³H (T or tritium); carbon may be, for example, ¹³C or ¹⁴C; oxygen may be, for example, ¹⁸O; nitrogen may be, for example, ¹⁵N, and the like. In other embodiments, a particular isotope *(e.g.,* ³H, ¹³C, ¹⁴C, ¹⁸O, or ¹⁵N) can represent at least 1%, at least 5%, at least 10%, at least 15%, at least 20%, at least 25%, at least 30%, at least 35%, at least 40%, at least 45%, at least 50%, at least 60%, at least 65%, at least 70%, at least 75%, at least 80%, at least 85%, at least 90%, at least 95%, at least 99%, or at least 99.9% of the total isotopic abundance of an element that occupies a specific site of the compound.

### Pharmaceutical Compositions

In one aspect, provided herein is a pharmaceutical composition comprising a compound of the invention or a pharmaceutically acceptable salt thereof, and a pharmaceutically acceptable excipient. In certain embodiments, the compound of the present invention is provided in an effective amount in the pharmaceutical composition. In certain embodiments, the compound of the present invention is provided in a therapeutically effective amount. In certain embodiments, the compound of the present invention is provided in a prophylactically effective amount.

In certain embodiments, the pharmaceutical composition comprises an effective amount of the active ingredient. In certain embodiments, the pharmaceutical composition comprises a therapeutically effective amount of the active ingredient. In certain embodiments, the pharmaceutical composition comprises a prophylactically effective amount of the active ingredient.

The pharmaceutical compositions provided herein can be administered by a variety of routes including, but not limited to, oral (enteral) administration, parenteral (by injection) administration, rectal administration, transdermal administration, intradermal administration, intrathecal administration, subcutaneous (SC) administration, intravenous (IV) administration, intramuscular (IM) administration, and intranasal administration.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

When used to prevent the onset of a CNS-disorder, the compounds provided herein will be administered to a subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The pharmaceutical compositions provided herein can also be administered chronically ("chronic administration"). Chronic administration refers to administration of a compound or pharmaceutical composition thereof over an extended period of time, *e.g.,* for example, over 3 months, 6 months, 1 year, 2 years, 3 years, 5 years, *etc,* or may be continued indefinitely, for example, for the rest of the subject's life. In certain embodiments, the chronic administration is intended to provide a constant level of the compound in the blood, *e.g.,* within the therapeutic window over the extended period of time.

The pharmaceutical compositions of the present invention may be further delivered using a variety of dosing methods. For example, in certain embodiments, the pharmaceutical composition may be given as a bolus, *e.g.,* in order to raise the concentration of the compound in the blood to an effective level. The placement of the bolus dose depends on the systemic levels of the active ingredient desired throughout the body, *e.g.,* an intramuscular or subcutaneous bolus dose allows a slow release of the active ingredient, while a bolus delivered directly to the veins (*e.g*., through an IV drip) allows a much faster delivery which quickly raises the concentration of the active ingredient in the blood to an effective level. In other embodiments, the pharmaceutical composition may be administered as a continuous infusion, *e.g*., by IV drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1 to about 50% by weight or preferably from about 1 to about 40% by weight) with the remainder being various vehicles or excipients and processing aids helpful for forming the desired dosing form.

With oral dosing, one to five and especially two to four and typically three oral doses per day are representative regimens. Using these dosing patterns, each dose provides from about 0.01 to about 20 mg/kg of the compound provided herein, with preferred doses each providing from about 0.1 to about 10 mg/kg, and especially about 1 to about 5 mg/kg.

Transdermal doses are generally selected to provide similar or lower blood levels than are achieved using injection doses, generally in an amount ranging from about 0.01 to about 20% by weight, preferably from about 0.1 to about 20% by weight, preferably from about 0.1 to about 10% by weight, and more preferably from about 0.5 to about 15% by weight.

Injection dose levels range from about 0.1 mg/kg/hour to at least 20 mg/kg/hour, all for from about 1 to about 120 hours and especially 24 to 96 hours. A preloading bolus of from about 0.1 mg/kg to about 10 mg/kg or more may also be administered to achieve adequate steady state levels. The maximum total dose is not expected to exceed about 5 g/day for a 40 to 80 kg human patient.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable excipients known in the art. As before, the active compound in such compositions is typically a minor component, often being from about 0.05 to 10% by weight with the remainder being the injectable excipient and the like.

Transdermal compositions are typically formulated as a topical ointment or cream containing the active ingredient(s). When formulated as an ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or Formulation. All such known transdermal formulations and ingredients are included within the scope provided herein.

The compounds provided herein can also be administered by a transdermal device. Accordingly, transdermal administration can be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania.

The compounds of the present invention can also be administered in sustained release forms or from sustained release drug delivery systems. A description of representative sustained release materials can be found in *Remington's Pharmaceutical Sciences.*

The present invention also relates to the pharmaceutically acceptable acid addition salt of a compound of the present invention. The acid which may be used to prepare the pharmaceutically acceptable salt is that which forms a non-toxic acid addition salt, *i.e.,* a salt containing pharmacologically acceptable anions such as the hydrochloride, hydroiodide, hydrobromide, nitrate, sulfate, bisulfate, phosphate, acetate, lactate, citrate, tartrate, succinate, maleate, fumarate, benzoate, para-toluenesulfonate, and the like.

In another aspect, the invention provides a pharmaceutical composition comprising a compound of the present invention and a pharmaceutically acceptable excipient, *e.g.,* a composition suitable for injection, such as for intravenous (IV) administration.

Pharmaceutically acceptable excipients include any and all diluents or other liquid vehicles, dispersion or suspension aids, surface active agents, isotonic agents, preservatives, lubricants and the like, as suited to the particular dosage form desired, *e.g*., injection. General considerations in the formulation and/or manufacture of pharmaceutical compositions agents can be found, for example, in Remington's Pharmaceutical Sciences, Sixteenth Edition, E. W. Martin (Mack Publishing Co., Easton, Pa., 1980), and Remington: The Science and Practice of Pharmacy, 21st Edition (Lippincott Williams & Wilkins, 2005).

For example, injectable preparations, such as sterile injectable aqueous suspensions, can be formulated according to the known art using suitable dispersing or wetting agents and suspending agents. Exemplary excipients that can be employed include, but are not limited to, water, sterile saline or phosphate-buffered saline, or Ringer's solution.

In certain embodiments, the pharmaceutical composition further comprises a cyclodextrin derivative. The most common cyclodextrins are α-, β- and γ- cyclodextrins consisting of 6, 7 and 8 α-l ,4-linked glucose units, respectively, optionally comprising one or more substituents on the linked sugar moieties, which include, but are not limited to, substituted or unsubstituted methylated, hydroxyalkylated, acylated, and sulfoalkylether substitution. In certain embodiments, the cyclodextrin is a sulfoalkyl ether β-cyclodextrin, *e.g.,* for example, sulfobutyl ether β-cyclodextrin, also known as CAPTISOL^{®}. See, *e.g.,* U.S. 5,376,645. In certain embodiments, the composition comprises hexapropyl-β-cyclodextrin. In a more particular embodiment, the composition comprises hexapropyl-β-cyclodextrin (10-50% in water).

The injectable composition can be sterilized, for example, by filtration through a bacterial-retaining filter, or by incorporating sterilizing agents in the form of sterile solid compositions which can be dissolved or dispersed in sterile water or other sterile injectable medium prior to use.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, response of the individual patient, the severity of the patient's symptoms, and the like.

The compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include pre-filled, premeasured ampules or syringes of the liquid compositions. In such compositions, the compound is usually a minor component (from about 0.1% to about 50% by weight or preferably from about 1% to about 40% by weight) with the remainder being various vehicles or carriers and processing aids helpful for forming the desired dosing form.

The compounds provided herein can be administered as the sole active agent, or they can be administered in combination with other active agents. In one aspect, the present invention provides a combination of a compound of the present invention and another pharmacologically active agent. Administration in combination can proceed by any technique apparent to those of skill in the art including, for example, separate, sequential, concurrent, and alternating administration.

Although the descriptions of pharmaceutical compositions provided herein are principally directed to pharmaceutical compositions which are suitable for administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and/or perform such modification with ordinary experimentation. General considerations in the formulation and/or manufacture of pharmaceutical compositions can be found, for example, in Remington: The Science and Practice of Pharmacy 21st ed., Lippincott Williams & Wilkins, 2005.

In one aspect, provided is a kit comprising a composition (*e*.*g*., a solid composition) comprising a compound of the invention.

### Medicinal Indications

The references to methods of treatment in the summary and detailed description of the invention in this description are to be interpreted as references to the compounds, pharmaceutical compositions and medicaments of the present invention for use in a method for treatment of the human (or animal) body by therapy (or for diagnosis).

In an aspect, compounds of the invention are envisioned to be useful as therapeutic agents for treating a CNS-related disorder (*e*.*g*., sleep disorder, a mood disorder such as depression, a schizophrenia spectrum disorder, a convulsive disorder, epileptogenesis, a disorder of memory and/or cognition, a movement disorder, a personality disorder, autism spectrum disorder, pain, traumatic brain injury, a vascular disease, a substance abuse disorder and/or withdrawal syndrome, or tinnitus) in a subject in need (*e.g*., a subject with Rett syndrome, Fragile X syndrome, or Angelman syndrome). Exemplary CNS conditions related to GABA-modulation include, but are not limited to, sleep disorders [*e.g*., insomnia], mood disorders [*e.g*., depression (*e.g*., major depressive disorder (MDD)), dysthymic disorder (*e*.*g*., mild depression), bipolar disorder (*e.g.,* I and/or II), anxiety disorders (*e.g.,* generalized anxiety disorder (GAD), social anxiety disorder), stress, post-traumatic stress disorder (PTSD), compulsive disorders (*e*.*g*., obsessive compulsive disorder (OCD))], schizophrenia spectrum disorders [*e*.*g*., schizophrenia, schizoaffective disorder], convulsive disorders [*e.g*., epilepsy (*e.g.,* status epilepticus (SE)), seizures], disorders of memory and/or cognition [*e*.*g*., attention disorders (*e.g.,* attention deficit hyperactivity disorder (ADHD)), dementia (*e.g*., Alzheimer's type dementia, Lewis body type dementia, vascular type dementia], movement disorders [*e*.*g*., Huntington's disease, Parkinson's disease], personality disorders [*e*.*g*., anti-social personality disorder, obsessive compulsive personality disorder], autism spectrum disorders (ASD) [*e.g*., autism, monogenetic causes of autism such as synaptophathy's, *e*.*g*., Rett syndrome, Fragile X syndrome, Angelman syndrome], pain [*e*.*g*., neuropathic pain, injury related pain syndromes, acute pain, chronic pain], traumatic brain injury (TBI), vascular diseases [*e*.*g*., stroke, ischemia, vascular malformations], substance abuse disorders and/or withdrawal syndromes [*e*.*g*., addition to opiates, cocaine, and/or alcohol], and tinnitus.

In certain embodiments, CNS-related disorder is a sleep disorder, a mood disorder, a schizophrenia spectrum disorder, a convulsive disorder, a disorder of memory and/or cognition, a movement disorder, a personality disorder, autism spectrum disorder, pain, traumatic brain injury, a vascular disease, a substance abuse disorder and/or withdrawal syndrome, tinnitus, or status epilepticus. In certain embodiments, the CNS-related disorder is depression. In certain embodiments, the CNS-related disorder is postpartum depression. In certain embodiments, the CNS-related disorder is major depressive disorder. In certain embodiments, the major depressive disorder is moderate major depressive disorder. In certain embodiments, the major depressive disorder is severe major depressive disorder.

In an aspect, provided is a method of alleviating or preventing seizure activity in a subject, comprising administering to the subject in need of such treatment an effective amount of a compound of the present invention. In some embodiments, the method alleviates or prevents epileptogenesis.

In yet another aspect, provided is a combination of a compound of the present invention and another pharmacologically active agent. The compounds provided herein can be administered as the sole active agent or they can be administered in combination with other agents. Administration in combination can proceed by any technique apparent to those of skill in the art including, for example, separate, sequential, concurrent and alternating administration.

In another aspect, provided is a method of treating or preventing brain excitability in a subject susceptible to or afflicted with a condition associated with brain excitability, comprising administering to the subject an effective amount of a compound of the present invention to the subject.

In yet another aspect, provided is a method of treating or preventing stress or anxiety in a subject, comprising administering to the subject in need of such treatment an effective amount of a compound of the present invention, or a composition thereof.

In yet another aspect, provided is a method of alleviating or preventing insomnia in a subject, comprising administering to the subject in need of such treatment an effective amount of a compound of the present invention, or a composition thereof.

In yet another aspect, provided is a method of inducing sleep and maintaining substantially the level of REM sleep that is found in normal sleep, wherein substantial rebound insomnia is not induced, comprising administering an effective amount of a compound of the present invention.

In yet another aspect, provided is a method of alleviating or preventing premenstrual syndrome (PMS) or postnatal depression (PND) in a subject, comprising administering to the subject in need of such treatment an effective amount of a compound of the present invention.

In yet another aspect, provided is a method of treating or preventing mood disorders in a subject, comprising administering to the subject in need of such treatment an effective amount of a compound of the present invention. In certain embodiments the mood disorder is depression.

In yet another aspect, provided is a method of cognition enhancement or treating memory disorder by administering to the subject a therapeutically effective amount of a compound of the present invention. In certain embodiments, the disorder is Alzheimer's disease. In certain embodiments, the disorder is Rett syndrome.

In yet another aspect, provided is a method of treating attention disorders by administering to the subject a therapeutically effective amount of a compound of the present invention. In certain embodiments, the attention disorder is ADHD.

In certain embodiments, the compound is administered to the subject chronically. In certain embodiments, the compound is administered to the subject orally, subcutaneously, intramuscularly, or intravenously.

### Neuroendocrine Disorders and Dysfunction

Provided herein are methods that can be used for treating neuroendocrine disorders and dysfunction. As used herein, "neuroendocrine disorder" or "neuroendocrine dysfunction" refers to a variety of conditions caused by imbalances in the body's hormone production directly related to the brain. Neuroendocrine disorders involve interactions between the nervous system and the endocrine system. Because the hypothalamus and the pituitary gland are two areas of the brain that regulate the production of hormones, damage to the hypothalamus or pituitary gland, *e.g.,* by traumatic brain injury, may impact the production of hormones and other neuroendocrine functions of the brain. In some embodiments, the neuroendocrine disorder or dysfunction is associated with a women's health disorder or condition (*e.g.,* a women's health disorder or condition described herein). In some embodiments, the neuroendocrine disorder or dysfunction is associated with a women's health disorder or condition is polycystic ovary syndrome.

Symptoms of neuroendocrine disorder include, but are not limited to, behavioral, emotional, and sleep-related symptoms, symptoms related to reproductive function, and somatic symptoms; including but not limited to fatigue, poor memory, anxiety, depression, weight gain or loss, emotional lability, lack of concentration, attention difficulties, loss of lipido, infertility, amenorrhea, loss of muscle mass, increased belly body fat, low blood pressure, reduced heart rate, hair loss, anemia, constipation, cold intolerance, and dry skin.

### Neurodegenerative Diseases and Disorders

The methods described herein can be used for treating neurodegenerative diseases and disorders. The term "neurodegenerative disease" includes diseases and disorders that are associated with the progressive loss of structure or function of neurons, or death of neurons. Neurodegenerative diseases and disorders include, but are not limited to, Alzheimer's disease (including the associated symptoms of mild, moderate, or severe cognitive impairment); amyotrophic lateral sclerosis (ALS); anoxic and ischemic injuries; ataxia and convulsion (including for the treatment and prevention and prevention of seizures that are caused by schizoaffective disorder or by drugs used to treat schizophrenia); benign forgetfulness; brain edema; cerebellar ataxia including McLeod neuroacanthocytosis syndrome (MLS); closed head injury; coma; contusive injuries (*e*.*g*., spinal cord injury and head injury); dementias including multi-infarct dementia and senile dementia; disturbances of consciousness; Down syndrome; drug-induced or medication-induced Parkinsonism (such as neuroleptic-induced acute akathisia, acute dystonia, Parkinsonism, or tardive dyskinesia, neuroleptic malignant syndrome, or medication-induced postural tremor); epilepsy; fragile X syndrome; Gilles de la Tourette's syndrome; head trauma; hearing impairment and loss; Huntington's disease; Lennox syndrome; levodopa-induced dyskinesia; mental retardation; movement disorders including akinesias and akinetic (rigid) syndromes (including basal ganglia calcification, corticobasal degeneration, multiple system atrophy, Parkinsonism-ALS dementia complex, Parkinson's disease, postencephalitic parkinsonism, and progressively supranuclear palsy); muscular spasms and disorders associated with muscular spasticity or weakness including chorea (such as benign hereditary chorea, drug-induced chorea, hemiballism, Huntington's disease, neuroacanthocytosis, Sydenham's chorea, and symptomatic chorea), dyskinesia (including tics such as complex tics, simple tics, and symptomatic tics), myoclonus (including generalized myoclonus and focal cyloclonus), tremor (such as rest tremor, postural tremor, and intention tremor) and dystonia (including axial dystonia, dystonic writer's cramp, hemiplegic dystonia, paroxysmal dystonia, and focal dystonia such as blepharospasm, oromandibular dystonia, and spasmodic dysphonia and torticollis); neuronal damage including ocular damage, retinopathy or macular degeneration of the eye; neurotoxic injury which follows cerebral stroke, thromboembolic stroke, hemorrhagic stroke, cerebral ischemia, cerebral vasospasm, hypoglycemia, amnesia, hypoxia, anoxia, perinatal asphyxia and cardiac arrest; Parkinson's disease; seizure; status epilecticus; stroke; tinnitus; tubular sclerosis, and viral infection induced neurodegeneration (*e*.*g*., caused by acquired immunodeficiency syndrome (AIDS) and encephalopathies). Neurodegenerative diseases also include, but are not limited to, neurotoxic injury which follows cerebral stroke, thromboembolic stroke, hemorrhagic stroke, cerebral ischemia, cerebral vasospasm, hypoglycemia, amnesia, hypoxia, anoxia, perinatal asphyxia and cardiac arrest. Methods of treating or preventing a neurodegenerative disease also include treating or preventing loss of neuronal function characteristic of neurodegenerative disorder.

### Mood disorders

Also provided herein are methods for treating a mood disorder, for example clinical depression, postnatal depression or postpartum depression, perinatal depression, atypical depression, melancholic depression, psychotic major depression, cataonic depression, seasonal affective disorder, dysthymia, double depression, depressive personality disorder, recurrent brief depression, minor depressive disorder, bipolar disorder or manic depressive disorder, depression caused by chronic medical conditions, treatment-resistant depression, refractory depression, suicidality, suicidal ideation, or suicidal behavior. In some embodiments, the method described herein provides therapeutic effect to a subject suffering from depression (*e*.*g*., moderate or severe depression). In some embodiments, the mood disorder is associated with a disease or disorder described herein (*e*.*g*., neuroendocrine diseases and disorders, neurodegenerative diseases and disorders (*e*.*g*., epilepsy), movement disorders, tremor (*e*.*g*., Parkinson's Disease), women's health disorders or conditions).

**Clinical depression** is also known as major depression, major depressive disorder (MDD), severe depression, unipolar depression, unipolar disorder, and recurrent depression, and refers to a mental disorder characterized by pervasive and persistent low mood that is accompanied by low self-esteem and loss of interest or pleasure in normally enjoyable activities. Some people with clinical depression have trouble sleeping, lose weight, and generally feel agitated and irritable. Clinical depression affects how an individual feels, thinks, and behaves and may lead to a variety of emotional and physical problems. Individuals with clinical depression may have trouble doing day-to-day activities and make an individual feel as if life is not worth living.

**Peripartum depression** refers to depression in pregnancy. Symptoms include irritability, crying, feeling restless, trouble sleeping, extreme exhaustion (emotional and/or physical), changes in appetite, difficulty focusing, increased anxiety and/or worry, disconnected feeling from baby and/or fetus, and losing interest in formerly pleasurable activities.

**Postnatal depression (PND)** is also referred to as **postpartum depression (PPD),** and refers to a type of clinical depression that affects women after childbirth. Symptoms can include sadness, fatigue, changes in sleeping and eating habits, reduced sexual desire, crying episodes, anxiety, and irritability. In some embodiments, the PND is a treatment-resistant depression (*e*.*g*., a treatment-resistant depression as described herein). In some embodiments, the PND is refractory depression (*e*.*g*., a refractory depression as described herein).

In some embodiments, a subject having PND also experienced depression, or a symptom of depression during pregnancy. This depression is referred to herein as) **perinatal depression.** In an embodiment, a subject experiencing perinatal depression is at increased risk of experiencing PND.

**Atypical depression (AD)** is characterized by mood reactivity (*e*.*g*., paradoxical anhedonia) and positivity, significant weight gain or increased appetite. Patients suffering from AD also may have excessive sleep or somnolence (hypersomnia), a sensation of limb heaviness, and significant social impairment as a consequence of hypersensitivity to perceived interpersonal rejection.

**Melancholic depression** is characterized by loss of pleasure (anhedonia) in most or all activities, failures to react to pleasurable stimuli, depressed mood more pronounced than that of grief or loss, excessive weight loss, or excessive guilt.

**Psychotic major depression (PMD)** or psychotic depression refers to a major depressive episode, in particular of melancholic nature, where the individual experiences psychotic symptoms such as delusions and hallucinations.

**Catatonic depression** refers to major depression involving disturbances of motor behavior and other symptoms. An individual may become mute and stuporose, and either is immobile or exhibits purposeless or bizarre movements.

**Seasonal affective disorder (SAD)** refers to a type of seasonal depression wherein an individual has seasonal patterns of depressive episodes coming on in the fall or winter.

**Dysthymia** refers to a condition related to unipolar depression, where the same physical and cognitive problems are evident. They are not as severe and tend to last longer (*e.g.,* at least 2 years).

**Double depression** refers to fairly depressed mood (dysthymia) that lasts for at least 2 years and is punctuated by periods of major depression.

**Depressive Personality Disorder (DPD)** refers to a personality disorder with depressive features.

**Recurrent Brief Depression (RBD)** refers to a condition in which individuals have depressive episodes about once per month, each episode lasting 2 weeks or less and typically less than 2-3 days.

**Minor depressive disorder** or minor depression refers to a depression in which at least 2 symptoms are present for 2 weeks.

**Bipolar disorder or manic depressive disorder** causes extreme mood swings that include emotional highs (mania or hypomania) and lows (depression). During periods of mania the individual may feel or act abnormally happy, energetic, or irritable. They often make poorly thought out decisions with little regard to the consequences. The need for sleep is usually reduced. During periods of depression there may be crying, poor eye contact with others, and a negative outlook on life. The risk of suicide among those with the disorder is high at greater than 6% over 20 years, while self-harm occurs in 30-40%. Other mental health issues such as anxiety disorder and substance use disorder are commonly associated with bipolar disorder.

**Depression caused by chronic medical conditions** refers to depression caused by chronic medical conditions such as cancer or chronic pain, chemotherapy, chronic stress.

**Treatment-resistant depression** refers to a condition where the individuals have been treated for depression, but the symptoms do not improve. For example, antidepressants or physchological counseling (psychotherapy) do not ease depression symptoms for individuals with treatment-resistant depression. In some cases, individuals with treatment-resistant depression improve symptoms, but come back. **Refractory depression** occurs in patients suffering from depression who are resistant to standard pharmacological treatments, including tricyclic antidepressants, MAOIs, SSRIs, and double and triple uptake inhibitors and/or anxiolytic drugs, as well as non-pharmacological treatments (*e*.*g*., psychotherapy, electroconvulsive therapy, vagus nerve stimulation and/or transcranial magnetic stimulation).

**Post-surgical depression** refers to feelings of depression that follow a surgical procedure (*e.g.,* as a result of having to confront one's mortality). For example, individuals may feel sadness or empty mood persistently, a loss of pleasure or interest in hobbies and activities normally enjoyed, or a persistent felling of worthlessness or hopelessness.

**Mood disorder associated with conditions or disorders of women's health** refers to mood disorders (*e.g.,* depression) associated with (*e.g.,* resulting from) a condition or disorder of women's health (*e.g.,* as described herein).

**Suicidality, suicidal ideation, suicidal behavior** refers to the tendency of an individual to commit suicide. Suicidal ideation concerns thoughts about or an unusual preoccupation with suicide. The range of suicidal ideation varies greatly, from *e.g.,* fleeting thoughts to extensive thoughts, detailed planning, role playing, incomplete attempts. Symptoms include talking about suicide, getting the means to commit suicide, withdrawing from social contact, being preoccupied with death, feeling trapped or hopeless about a situation, increasing use of alcohol or drugs, doing risky or self-destructive things, saying goodbye to people as if they won't be seen again.

**Symptoms** of depression include persistent anxious or sad feelings, feelings of helplessness, hopelessness, pessimism, worthlessness, low energy, restlessness, difficulty sleeping, sleeplessness, irritability, fatigue, motor challenges, loss of interest in pleasurable activities or hobbies, loss of concentration, loss of energy, poor self-esteem, absence of positive thoughts or plans, excessive sleeping, overeating, appetite loss, insomnia,self-harm, thoughts of suicide, and suicide attempts. The presence, severity, frequency, and duration of symptoms may vary on a case to case basis. Symptoms of depression, and relief of the same, may be ascertained by a physician or psychologist (*e*.*g*., by a mental state examination).

In some embodiments, the method comprises monitoring a subject with a known depression scale, *e.g.,* the Hamilton Depression (HAM-D) scale, the Clinical Global Impression-Improvement Scale (CGI), and the Montgomery-Åsberg Depression Rating Scale (MADRS). In some embodiments, a therapeutic effect can be determined by reduction in Hamilton Depression (HAM-D) total score exhibited by the subject. Reduction in the HAM-D total score can happen within 4, 3, 2, or 1 days; or 96, 84, 72, 60, 48, 24, 20, 16, 12, 10, 8 hours or less. The therapeutic effect can be assessed across a specified treatment period. For example, the therapeutic effect can be determined by a decrease from baseline in HAM-D total score after administering a compound of the present invention (*e.g*., 12, 24, or 48 hours after administration; or 24, 48, 72, or 96 hours or more; or 1 day, 2 days, 14 days, 21 days, or 28 days; or 1 week, 2 weeks, 3 weeks, or 4 weeks; or 1 month, 2 months, 6 months, or 10 months; or 1 year, 2 years, or for life).

In some embodiments, the subject has a mild depressive disorder, *e*.*g*., mild major depressive disorder. In some embodiments, the subject has a moderate depressive disorder, *e*.*g*., moderate major depressive disorder. In some embodiments, the subject has a severe depressive disorder, *e*.*g*., severe major depressive disorder. In some embodiments, the subject has a very severe depressive disorder, *e*.*g*., very severe major depressive disorder. In some embodiments, the baseline HAM-D total score of the subject (i.e., prior to treatment with a compound of the present invention) is at least 24. In some embodiments, the baseline HAM-D total score of the subject is at least 18. In some embodiments, the baseline HAM-D total score of the subject is between and including 14 and 18. In some embodiments, the baseline HAM-D total score of the subject is between and including 19 and 22. In some embodiments, the HAM-D total score of the subject before treatment with a compound of the present invention greater than or equal to 23. In some embodiments, the baseline score is at least 10, 15, or 20. In some embodiments, the HAM-D total score of the subject after treatment with a compound of the present invention, is about 0 to 10 (*e.g.,* less than 10; 0 to 10, 0 to 6, 0 to 4, 0 to 3, 0 to 2, or 1.8). In some embodiments, the HAM-D total score after treatment with a compound of the present invention, is less than 10, 7, 5, or 3. In some embodiments, the decrease in HAM-D total score is from a baseline score of about 20 to 30 (*e.g.,* 22 to 28, 23 to 27, 24 to 27, 25 to 27, 26 to 27) to a HAM-D total score at about 0 to 10 (*e.g.,* less than 10; 0 to 10, 0 to 6, 0 to 4, 0 to 3, 0 to 2, or 1.8) after treatment with a compound of the present invention. In some embodiments, the decrease in the baseline HAM-D total score to HAM-D total score after treatment with a compound of the present invention, is at least 1, 2, 3, 4, 5, 7, 10, 25, 40, 50, or 100 fold). In some embodiments, the percentage decrease in the baseline HAM-D total score to HAM-D total score after treatment with a compound of the present invention, is at least 50% (*e.g.,* 60%, 70%, 80%, or 90%). In some embodiments, the therapeutic effect is measured as a decrease in the HAM-D total score after treatment with a compound of the present invention, relative to the baseline HAM-D total score (*e.g.,* 12, 24, 48 hours after administration; or 24, 48, 72, 96 hours or more; or 1 day, 2 days, 14 days, or more) is at least 10, 15, or 20 points.

In some embodiments, the method of treating a depressive disorder, *e.g*., major depressive disorder provides a therapeutic effect (*e.g.,* as measured by reduction in Hamilton Depression Score (HAM-D)) within 14, 10, 4, 3, 2, or 1 days, or 24, 20, 16, 12, 10, or 8 hours or less. In some embodiments, the method of treating the depressive disorder, *e*.*g*., major depressive disorder, provides a therapeutic effect (*e.g.,* as determined by a statistically significant reduction in HAM-D total score) within the first or second day of the treatment with a compound of the present invention. In some embodiments, the method of treating the depressive disorder, *e*.*g*., major depressive disorder, provides a therapeutic effect (*e.g.,* as determined by a statistically significant reduction in HAM-D total score) within less than or equal to 14 days since the beginning of the treatment with a compound of the present invention. In some embodiments, the method of treating the depressive disorder, *e*.*g*., major depressive disorder, provides a therapeutic effect (*e.g.,* as determined by a statistically significant reduction in HAM-D total score) within less than or equal to 21 days since the beginning of the treatment with a compound of the present invention. In some embodiments, the method of treating the depressive disorder, *e*.*g*., major depressive disorder, provides a therapeutic effect (*e.g.,* as determined by a statistically significant reduction in HAM-D total score) within less than or equal to 28 days since the beginning of the treatment with a compound of the present invention. In some embodiments, the therapeutic effect is a decrease from baseline in HAM-D total score after treatment with a compound of the present invention (*e.g*., treatment with a compound of the present invention once a day for 14 days). In some embodiments, the HAM-D total score of the subject before treatment with a compound of the present invention, is at least 24. In some embodiments, the HAM-D total score of the subject before treatment with a compound of the present invention, is at least 18. In some embodiments, the HAM-D total score of the subject before treatment with a compound of the present invention, is between and including 14 and 18. In some embodiments, the decrease in HAM-D total score after treating the subject with a compound of the present invention, relative to the baseline HAM-D total score is at least 10. In some embodiments, the decrease in HAM-D total score after treating the subject with a compound of the present invention, relative to the baseline HAM-D total score is at least 15 (*e.g.,* at least 17). In some embodiments, the HAM-D total score associated with treating the subject with a compound of the present invention, is no more than a number ranging from 6 to 8. In some embodiments, the HAM-D total score associated with treating the subject with a compound of the present invention, is no more than 7.

In some embodiments, the method provides therapeutic effect (*e.g.,* as measured by reduction in Clinical Global Impression-Improvement Scale (CGI)) within 14, 10, 4, 3, 2, or 1 days, or 24, 20, 16, 12, 10, or 8 hours or less. In some embodiments, the CNS-disorder is a depressive disorder, *e*.*g*., major depressive disorder. In some embodiments, the method of treating the depressive disorder, *e*.*g*., major depressive disorder provides a therapeutic effect within the second day of the treatment period. In some embodiments, the therapeutic effect is a decrease from baseline in CGI score at the end of a treatment period (*e.g.,* 14 days after administration).

In some embodiments, the method provides therapeutic effect (*e.g.,* as measured by reduction in Montgomery-Åsberg Depression Rating Scale (MADRS) within 14, 10, 4, 3, 2, or 1 days, or 24, 20, 16, 12, 10, or 8 hours or less. In some embodiments, the CNS-disorder is a depressive disorder, *e*.*g*., major depressive disorder. In some embodiments, the method of treating the depressive disorder, *e*.*g*., major depressive disorder provides a therapeutic effect within the second day of the treatment period. In some embodiments, the therapeutic effect is a decrease from baseline in MADRS score at the end of a treatment period *(e.g.,* 14 days after administration).

A therapeutic effect for major depressive disorder can be determined by a reduction in Montgomery-Åsberg Depression Rating Scale (MADRS) score exhibited by the subject. For example, the MADRS score can be reduced within 4, 3, 2, or 1 days; or 96, 84, 72, 60, 48, 24, 20, 16, 12, 10, 8 hours or less. The Montgomery-Åsberg Depression Rating Scale (MADRS) is a ten-item diagnostic questionnaire (regarding apparent sadness, reported sadness, inner tension, reduced sleep, reduced appetite, concentration difficulties, lassitude, inability to feel, pessimistic thoughts, and suicidal thoughts) which psychiatrists use to measure the severity of depressive episodes in patients with mood disorders.

In some embodiments, the method provides therapeutic effect (*e.g.,* as measured by reduction in Edinburgh Postnatal Depression Scale (EPDS)) within 4, 3, 2, 1 days; 24, 20, 16, 12, 10, 8 hours or less. In some embodiments, the therapeutic effect is an improvement measured by the EPDS.

In some embodiments, the method provides therapeutic effect (*e.g.,* as measured by reduction in Generalized Anxiety Disorder 7-Item Scale (GAD-7)) within 4, 3, 2, 1 days; 24, 20, 16, 12, 10, 8 hours or less.

### Anxiety Disorders

Provided herein are methods for treating anxiety disorders (*e*.*g*., generalized anxiety disorder, panic disorder, obsessive compulsive disorder, phobia, post-traumatic stress disorder). **Anxiety disorder** is a blanket term covering several different forms of abnormal and pathological fear and anxiety. Current psychiatric diagnostic criteria recognize a wide variety of anxiety disorders.

**Generalized anxiety disorder** is a common chronic disorder characterized by long-lasting anxiety that is not focused on any one object or situation. Those suffering from generalized anxiety experience non-specific persistent fear and worry and become overly concerned with everyday matters. Generalized anxiety disorder is the most common anxiety disorder to affect older adults.

**In panic disorder,** a person suffers from brief attacks of intense terror and apprehension, often marked by trembling, shaking, confusion, dizziness, nausea, difficulty breathing. These panic attacks, defined by the APA as fear or discomfort that abruptly arises and peaks in less than ten minutes, can last for several hours and can be triggered by stress, fear, or even exercise; although the specific cause is not always apparent. In addition to recurrent unexpected panic attacks, a diagnosis of panic disorder also requires that said attacks have chronic consequences: either worry over the attacks' potential implications, persistent fear of future attacks, or significant changes in behavior related to the attacks. Accordingly, those suffering from panic disorder experience symptoms even outside of specific panic episodes. Often, normal changes in heartbeat are noticed by a panic sufferer, leading them to think something is wrong with their heart or they are about to have another panic attack. In some cases, a heightened awareness (hypervigilance) of body functioning occurs during panic attacks, wherein any perceived physiological change is interpreted as a possible life threatening illness (i.e. extreme hypochondriasis).

**Obsessive compulsive disorder** is a type of anxiety disorder primarily characterized by repetitive obsessions (distressing, persistent, and intrusive thoughts or images) and compulsions (urges to perform specific acts or rituals). The OCD thought pattern may be likened to superstitions insofar as it involves a belief in a causative relationship where, in reality, one does not exist. Often the process is entirely illogical; for example, the compulsion of walking in a certain pattern may be employed to alleviate the obsession of impending harm. And in many cases, the compulsion is entirely inexplicable, simply an urge to complete a ritual triggered by nervousness. In a minority of cases, sufferers of OCD may only experience obsessions, with no overt compulsions; a much smaller number of sufferers experience only compulsions.

The single largest category of anxiety disorders is that of **phobia,** which includes all cases in which fear and anxiety is triggered by a specific stimulus or situation. Sufferers typically anticipate terrifying consequences from encountering the object of their fear, which can be anything from an animal to a location to a bodily fluid.

**Post-traumatic stress disorder or PTSD** is an anxiety disorder which results from a traumatic experience. Post-traumatic stress can result from an extreme situation, such as combat, rape, hostage situations, or even serious accident. It can also result from long term (chronic) exposure to a severe stressor, for example soldiers who endure individual battles but cannot cope with continuous combat. Common symptoms include flashbacks, avoidant behaviors, and depression.

### Women's Health Disorders

Provided herein are methods for treating conditions or disorders related to women's health. Conditions or disorders related to women's health include, but are not limited to, gynecological health and disorders (*e*.*g*., premenstrual syndrome (PMS), premenstrual dysphoric disorder (PMDD)), **pregnancy issues** (*e*.*g*., miscarriage, abortion), infertility and related disorders (*e*.*g*., polycystic ovary syndrome (PCOS)), other disorders and conditions, and issues related to women's overall health and wellness (*e*.*g*., menopause).

**Gynecological health and disorders** affecting women include menstruation and menstrual irregularities; urinary tract health, including urinary incontinence and pelvic floor disorders; and such disorders as bacterial vaginosis, vaginitis, uterine fibroids, and vulvodynia.

**Premenstrual syndrome (PMS)** refers to physical and emotional symptoms that occur in the one to two weeks before a women's period. Symptoms vary but can include bleeding, mood swings, tender breasts, food cravings, fatigue, irritability, acne, and depression.

**Premenstrual dysphoric disorder (PMDD)** is a severe form of PMS. The symptoms of PMDD are similar to PMS but more severe and may interfere with work, social activity, and relationships. PMDD symptoms include mood swings, depressed mood or feelings of hopelessness, marked anger, increased interpersonal conflicts, tension and anxiety, irritability, decreased interest in usual activities, difficulty concentrating, fatigue, change in appetite, feeling out of control or overwhelmed, sleep problems, physical problems (*e*.*g*., bloating, breast tenderness, swelling, headaches, joint or muscle pain).

**Pregnancy issues** include preconception care and prenatal care, pregnancy loss (miscarriage and stillbirth), preterm labor and premature birth, sudden infant death syndrome (SIDS), breastfeeding, and birth defects.

**Miscarriage** refers to a pregnancy that ends on its own, within the first 20 weeks of gestation.

**Abortion** refers to the deliberate termination of a pregnancy, which can be performed during the first 28 weeks of pregnancy.

**Infertility and related disorders** include uterine fibroids, polycystic ovary syndrome, endometriosis, and primary ovarian insufficiency.

**Polycystic ovary syndrome (PCOS)** refers to an endocrine system disorder among women of reproductive age. PCOS is a set of symptoms resulting from an elevated male hormone in women. Most women with PCOS grow many small cysts on their ovaries. Symptoms of PCOS include irregular or no menstrual periods, heavy periods, excess body and facial hair, acne, pelvic pain, difficulty getting pregnant, and patches of thick, darker, velvety skin. PCOS may be associated with conditions including type 2 diabetes, obesity, obstructive sleep apnea, heart disease, mood disorders, and endometrial cancer.

**Other disorders and conditions** that affect only women include Turner syndrome, Rett syndrome, and ovarian and cervical cancers.

**Issues related to women's overall health and wellness** include violence against women, women with disabilities and their unique challenges, osteoporosis and bone health, and menopause.

**Menopause** refers to the 12 months after a woman's last menstrual period and marks the end of menstrual cycles. Menopause typically occurs in a woman's 40s or 50s. Physical symptoms such as hot flashes and emotional symptoms of menopause may disrupt sleep, lower energy, or trigger anxiety or feelings of sadness or loss. Menopause includes natural menopause and surgical menopause, which is a type of induced menopause due to an event such as surgery (*e*.*g*., hysterectomy, oophorectomy; cancer). It is induced when the ovaries are gravely damaged by, *e*.*g*., radiation, chemotherapy, or other medications.

### Epilepsy

The compounds of the invention, or pharmaceutically acceptable salt, or a pharmaceutically acceptable composition thereof, can be used in a method described herein, for example in the treatment of a disorder described herein such as epilepsy, status epilepticus, or seizure.

Epilepsy is a brain disorder characterized by repeated seizures over time. Types of epilepsy can include, but are not limited to generalized epilepsy, *e*.*g*., childhood absence epilepsy, juvenile nyoclonic epilepsy, epilepsy with grand-mal seizures on awakening, West syndrome, Lennox-Gastaut syndrome, partial epilepsy, *e.g*., temporal lobe epilepsy, frontal lobe epilepsy, benign focal epilepsy of childhood.

### Epileptogenesis

The compounds and methods described herein can be used to treat or prevent epileptogenesis. Epileptogenesis is a gradual process by which a normal brain develops epilepsy (a chronic condition in which seizures occur). Epileptogenesis results from neuronal damage precipitated by the initial insult (*e*.*g*., status epilepticus).

### Status epilepticus (SE)

Status epilepticus (SE) can include, *e.g.,* convulsive status epilepticus, *e.g.,* early status epilepticus, established status epilepticus, refractory status epilepticus, super-refractory status epilepticus; non-convulsive status epilepticus, *e*.*g*., generalized status epilepticus, complex partial status epilepticus; generalized periodic epileptiform discharges; and periodic lateralized epileptiform discharges. Convulsive status epilepticus is characterized by the presence of convulsive status epileptic seizures, and can include early status epilepticus, established status epilepticus, refractory status epilepticus, super-refractory status epilepticus. Early status epilepticus is treated with a first line therapy. Established status epilepticus is characterized by status epileptic seizures which persist despite treatment with a first line therapy, and a second line therapy is administered. Refractory status epilepticus is characterized by status epileptic seizures which persist despite treatment with a first line and a second line therapy, and a general anesthetic is generally administered. Super refractory status epilepticus is characterized by status epileptic seizures which persist despite treatment with a first line therapy, a second line therapy, and a general anesthetic for 24 hours or more.

Non-convulsive status epilepticus can include, *e.g.,* focal non-convulsive status epilepticus, *e*.*g*., complex partial non-convulsive status epilepticus, simple partial non-convulsive status epilepticus, subtle non-convulsive status epilepticus; generalized non-convulsive status epilepticus, *e*.*g*., late onset absence non-convulsive status epilepticus, atypical absence non-convulsive status epilepticus, or typical absence non-convulsive status epilepticus.

The compounds of the invention or pharmaceutically acceptable salt, or a pharmaceutically acceptable composition thereof, can also be administered as a prophylactic to a subject having a CNS disorder *e*.*g*., a traumatic brain injury, status epilepticus, *e.g.,* convulsive status epilepticus, *e.g.,* early status epilepticus, established status epilepticus, refractory status epilepticus, super-refractory status epilepticus; non-convulsive status epilepticus, *e*.*g*., generalized status epilepticus, complex partial status epilepticus; generalized periodic epileptiform discharges; and periodic lateralized epileptiform discharges; prior to the onset of a seizure.

### Seizure

A seizure is the physical findings or changes in behavior that occur after an episode of abnormal electrical activity in the brain. The term "seizure" is often used interchangeably with "convulsion." Convulsions are when a person's body shakes rapidly and uncontrollably. During convulsions, the person's muscles contract and relax repeatedly.

Based on the type of behavior and brain activity, seizures are divided into two broad categories: generalized and partial (also called local or focal). Classifying the type of seizure helps doctors diagnose whether or not a patient has epilepsy.

Generalized seizures are produced by electrical impulses from throughout the entire brain, whereas partial seizures are produced (at least initially) by electrical impulses in a relatively small part of the brain. The part of the brain generating the seizures is sometimes called the focus.

There are six types of generalized seizures. The most common and dramatic, and therefore the most well-known, is the generalized convulsion, also called the grand-mal seizure. In this type of seizure, the patient loses consciousness and usually collapses. The loss of consciousness is followed by generalized body stiffening (called the "tonic" phase of the seizure) for 30 to 60 seconds, then by violent jerking (the "clonic" phase) for 30 to 60 seconds, after which the patient goes into a deep sleep (the "postictal" or after-seizure phase). During grand-mal seizures, injuries and accidents may occur, such as tongue biting and urinary incontinence.

Absence seizures cause a short loss of consciousness (just a few seconds) with few or no symptoms. The patient, most often a child, typically interrupts an activity and stares blankly. These seizures begin and end abruptly and may occur several times a day. Patients are usually not aware that they are having a seizure, except that they may be aware of "losing time."

Myoclonic seizures consist of sporadic jerks, usually on both sides of the body. Patients sometimes describe the jerks as brief electrical shocks. When violent, these seizures may result in dropping or involuntarily throwing objects.

Clonic seizures are repetitive, rhythmic jerks that involve both sides of the body at the same time.

Tonic seizures are characterized by stiffening of the muscles.

Atonic seizures consist of a sudden and general loss of muscle tone, particularly in the arms and legs, which often results in a fall.

Seizures described herein can include epileptic seizures; acute repetitive seizures; cluster seizures; continuous seizures; unremitting seizures; prolonged seizures; recurrent seizures; status epilepticus seizures, *e*.*g*., refractory convulsive status epilepticus, non-convulsive status epilepticus seizures; refractory seizures; myoclonic seizures; tonic seizures; tonic-clonic seizures; simple partial seizures; complex partial seizures; secondarily generalized seizures; atypical absence seizures; absence seizures; atonic seizures; benign Rolandic seizures; febrile seizures; emotional seizures; focal seizures; gelastic seizures; generalized onset seizures; infantile spasms; Jacksonian seizures; massive bilateral myoclonus seizures; multifocal seizures; neonatal onset seizures; nocturnal seizures; occipital lobe seizures; post traumatic seizures; subtle seizures; Sylvan seizures; visual reflex seizures; or withdrawal seizures. In some embodiments, the seizure is a generalized seizure associated with Dravet Syndrome, Lennox-Gastaut Syndrome, Tuberous Sclerosis Complex, Rett Syndrome or PCDH19 Female Pediatric Epilepsy.

### Movement Disorders

Also described herein are methods for treating a movement disorder. As used herein, "movement disorders" refers to a variety of diseases and disorders that are associated with hyperkinetic movement disorders and related abnormalities in muscle control. Exemplary movement disorders include, but are not limited to, Parkinson's disease and parkinsonism (defined particularly by bradykinesia), dystonia, chorea and Huntington's disease, ataxia, tremor (*e*.*g*., essential tremor), myoclonus and startle, tics and Tourette syndrome, Restless legs syndrome, stiff person syndrome, and gait disorders.

### Tremor

The methods described herein can be used to treat tremor, for example the compounds of the invention can be used to treat cerebellar tremor or intention tremor, dystonic tremor, essential tremor, orthostatic tremor, parkinsonian tremor, physiological tremor, psychogenic tremor, or rubral tremor. Tremor includes hereditary, degenerative, and idiopathic disorders such as Wilson's disease, Parkinson's disease, and essential tremor, respectively; metabolic diseases (*e*.*g*., thyroid-parathyroid-, liver disease and hypoglycemia); peripheral neuropathies (associated with Charcot-Marie-Tooth, Roussy-Levy, diabetes mellitus, complex regional pain syndrome); toxins (nicotine, mercury, lead, CO, Manganese, arsenic, toluene); drug-induced (narcoleptics, tricyclics, lithium, cocaine, alcohol, adrenaline, bronchodilators, theophylline, caffeine, steroids, valproate, amiodarone, thyroid hormones, vincristine); and psychogenic disorders. Clinical tremor can be classified into physiologic tremor, enhanced physiologic tremor, essential tremor syndromes (including classical essential tremor, primary orthostatic tremor, and task- and position-specific tremor), dystonic tremor, parkinsonian tremor, cerebellar tremor, Holmes' tremor (i.e., rubral tremor), palatal tremor, neuropathic tremor, toxic or drug-induced tremor, and psychogenic tremor.

**Tremor** is an involuntary, at times rhythmic, muscle contraction and relaxation that can involve oscillations or twitching of one or more body parts (*e*.*g*., hands, arms, eyes, face, head, vocal folds, trunk, legs).

**Cerebellar tremor or intention tremor** is a slow, broad tremor of the extremities that occurs after a purposeful movement. Cerebellar tremor is caused by lesions in or damage to the cerebellum resulting from, *e.g.,* tumor, stroke, disease (*e.g.,* multiple sclerosis, an inherited degenerative disorder).

**Dystonic tremor** occurs in individuals affected by dystonia, a movement disorder in which sustained involuntary muscle contractions cause twisting and repetitive motions and/or painful and abnormal postures or positions. Dystonic tremor may affect any muscle in the body. Dystonic tremors occurs irregularly and often can be relieved by complete rest.

**Essential tremor** or benign essential tremor is the most common type of tremor. Essential tremor may be mild and nonprogressive in some, and may be slowly progressive, starting on one side of the body but affect both sides within 3 years. The hands are most often affected, but the head, voice, tongue, legs, and trunk may also be involved. Tremor frequency may decrease as the person ages, but severity may increase. Heightened emotion, stress, fever, physical exhaustion, or low blood sugar may trigger tremors and/or increase their severity. Symptoms generally evolve over time and can be both visible and persistent following onset.

**Orthostatic tremor** is characterized by fast (*e.g.,* greater than 12 Hz) rhythmic muscle contractions that occurs in the legs and trunk immediately after standing. Cramps are felt in the thighs and legs and the patient may shake uncontrollably when asked to stand in one spot. Orthostatic tremor may occurs in patients with essential tremor.

**Parkinsonian tremor** is caused by damage to structures within the brain that control movement. Parkinsonian tremor is often a precursor to Parkinson's disease and is typically seen as a "pill-rolling" action of the hands that may also affect the chin, lips, legs, and trunk. Onset of parkinsonian tremor typically begins after age 60. Movement starts in one limb or on one side of the body and can progress to include the other side.

**Physiological tremor** can occur in normal individuals and have no clinical significance. It can be seen in all voluntary muscle groups. Physiological tremor can be caused by certain drugs, alcohol withdrawal, or medical conditions including an overactive thyroid and hypoglycemia. The tremor classically has a frequency of about 10 Hz.

**Psychogenic tremor** or hysterical tremor can occur at rest or during postural or kinetic movement. Patient with psychogenic tremor may have a conversion disorder or another psychiatric disease.

**Rubral tremor** is characterized by coarse slow tremor which can be present at rest, at posture, and with intention. The tremor is associated with conditions that affect the red nucleus in the midbrain, classical unusual strokes.

**Parkinson's Disease** affects nerve cells in the brain that produce dopamine. Symptoms include muscle rigidity, tremors, and changes in speech and gait. **Parkinsonism** is characterized by tremor, bradykinesia, rigidity, and postural instability. Parkinsonism shares symptoms found in Parkinson's Disease, but is a symptom complex rather than a progressive neurodegenerative disease.

**Dystonia** is a movement disorder characterized by sustained or intermittent muscle contractions causing abnormal, often repetitive movements or postures. Dystonic movements can be patterned, twisting, and may be tremulous. Dystonia is often initiated or worsened by voluntary action and associated with overflow muscle activation.

**Chorea** is a neurological disorder characterized by jerky involuntary movements typically affecting the shoulders, hips, and face. **Huntington's Disease** is an inherited disease that causes nerve cells in the brain to waste away. Symptoms include uncontrolled movements, clumsiness, and balance problems. Huntington's disease can hinder walk, talk, and swallowing.

**Ataxia** refers to the loss of full control of bodily movements, and may affect the fingers, hands, arms, legs, body, speech, and eye movements.

**Myloclonus and Startle** is a response to a sudden and unexpected stimulus, which can be acoustic, tactile, visual, or vestibular.

**Tics** are an involuntary movement usually onset suddenly, brief, repetitive, but non-rhythmical, typically imitating normal behavior and often occurring out of a background of normal activity. Tics can be classified as motor or vocal, motor tics associated with movements while vocal tics associated with sound. Tics can be characterized as simple or complex. For example simple motor tics involve only a few muscles restricted to a specific body part. **Tourette Syndrome** is an inherited neuropsychiatric disorder with onset in childhood, characterized by multiple motor tics and at least one vocal tic.

**Restless Legs Syndrome** is a neurologic sensorimotor disorder characterized by an overwhelming urge to move the legs when at rest.

**Stiff Person Syndrome** is a progressive movement disorder characterized by involuntary painful spasms and rigidity of muscles, usually involving the lower back and legs. Stiff-legged gait with exaggerated lumbar hyperlordosis typically results. Characteristic abnormality on EMG recordings with continuous motor unit activity of the paraspinal axial muscles is typically observed. Variants include "stiff-limb syndrome" producing focal stiffness typically affecting distal legs and feet.

**Gait disorders** refer to an abnormality in the manner or style of walking, which results from neuromuscular, arthritic, or other body changes. Gait is classified according to the system responsible for abnormal locomotion, and include hemiplegic gait, diplegic gait, neuropathic gait, myopathic gait, parkinsonian gait, choreiform gait, ataxic gait, and sensory gait.

### Anesthesia / Sedation

Anesthesia is a pharmacologically induced and reversible state of amnesia, analgesia, loss of responsiveness, loss of skeletal muscle reflexes, decreased stress response, or all of these simultaneously. These effects can be obtained from a single drug which alone provides the correct combination of effects, or occasionally with a combination of drugs (*e.g*., hypnotics, sedatives, paralytics, analgesics) to achieve very specific combinations of results. Anesthesia allows patients to undergo surgery and other procedures without the distress and pain they would otherwise experience.

Sedation is the reduction of irritability or agitation by administration of a pharmacological agent, generally to facilitate a medical procedure or diagnostic procedure.

Sedation and analgesia include a continuum of states of consciousness ranging from minimal sedation (anxiolysis) to general anesthesia.

**Minimal sedation is** also known as anxiolysis. Minimal sedation is a drug-induced state during which the patient responds normally to verbal commands. Cognitive function and coordination may be impaired. Ventilatory and cardiovascular functions are typically unaffected.

**Moderate sedation/analgesia (conscious sedation) is** a drug-induced depression of consciousness during which the patient responds purposefully to verbal command, either alone or accompanied by light tactile stimulation. No interventions are usually necessary to maintain a patent airway. Spontaneous ventilation is typically adequate. Cardiovascular function is usually maintained.

**Deep sedation/analgesia is a** drug-induced depression of consciousness during which the patient cannot be easily aroused, but responds purposefully (not a reflex withdrawal from a painful stimulus) following repeated or painful stimulation. Independent ventilatory function may be impaired and the patient may require assistance to maintain a patent airway. Spontaneous ventilation may be inadequate. Cardiovascular function is usually maintained.

**General anesthesia is a** drug-induced loss of consciousness during which the patient is not arousable, even to painful stimuli. The ability to maintain independent ventilatory function is often impaired and assistance is often required to maintain a patent airway. Positive pressure ventilation may be required due to depressed spontaneous ventilation or drug-induced depression of neuromuscular function. Cardiovascular function may be impaired.

Sedation in the intensive care unit (ICU) allows the depression of patients' awareness of the environment and reduction of their response to external stimulation. It can play a role in the care of the critically ill patient, and encompasses a wide spectrum of symptom control that will vary between patients, and among individuals throughout the course of their illnesses. Heavy sedation in critical care has been used to facilitate endotracheal tube tolerance and ventilator synchronization, often with neuromuscular blocking agents.

In some embodiments, sedation (*e*.*g*., long-term sedation, continuous sedation) is induced and maintained in the ICU for a prolonged period of time (*e.g*., 1 day, 2 days, 3 days, 5 days, 1 week, 2 week, 3 weeks, 1 month, 2 months). Long-term sedation agents may have long duration of action. Sedation agents in the ICU may have short elimination half-life.

Procedural sedation and analgesia, also referred to as conscious sedation, is a technique of administering sedatives or dissociative agents with or without analgesics to induce a state that allows a subject to tolerate unpleasant procedures while maintaining cardiorespiratory function.

### Examples

In order that the invention described herein may be more fully understood, the following examples are set forth. The synthetic and biological examples described in this application are offered to illustrate the compounds, pharmaceutical compositions, and methods provided herein and are not to be construed in any way as limiting their scope.

Of the following examples, the following compounds are examples of the present invention as are the biological tests carried out thereon: compounds I-D7a and I-E4 of example I-13; compounds I-14, I-15, I-16 and I-17 of examples I-14 to I-17; compounds I-D7 and I-D7a of examples I-18 and I-18a; compounds I-D7 and I-D9 of example I-19; compounds I-20 and I-20a of examples I-20 and I-20a; compound I-G14 of example I-21; compounds I-G16 and I-G16a of examples I-22 and I-22a; compound I-M3 of example I-24; compounds I-25, I-26 and I-27 of examples I-25 to I-27; compound I-K2 of example I-34; compound I-B11 of example I-57; compound I-P9 of example I-58; compound I-L14 of example I-59; compound I-L17 of example I-60; compound I-L19 of example I-61; compounds I-S3 and I-S3a of examples I-70 and I-70a and compound I-U11 of example I-75. Other compounds appearing in the present examples are provided either as intermediates for the preparation of the example compounds of the present invention as previously identified or are provided for the purposes of comparison only.

### Materials and Methods

The compounds provided herein can be prepared from readily available starting materials using the following general methods and procedures. It will be appreciated that where typical or preferred process conditions (*i.e*., reaction temperatures, times, mole ratios of reactants, solvents, pressures, *etc*.) are given, other process conditions can also be used unless otherwise stated. Optimum reaction conditions may vary with the particular reactants or solvent used, but such conditions can be determined by one skilled in the art by routine optimization.

Additionally, as will be apparent to those skilled in the art, conventional protecting groups may be necessary to prevent certain functional groups from undergoing undesired reactions. The choice of a suitable protecting group for a particular functional group as well as suitable conditions for protection and deprotection are well known in the art. For example, numerous protecting groups, and their introduction and removal, are described in T. W. Greene and P. G. M. Wuts, Protecting Groups in Organic Synthesis, Second Edition, Wiley, New York, 1991, and references cited therein.

The compounds provided herein may be isolated and purified by known standard procedures. Such procedures include (but are not limited to) trituration, column chromatography, HPLC, or supercritical fluid chromatography (SFC). The following schemes are presented with details as to the preparation of representative oxysterols that have been listed herein. The compounds provided herein may be prepared from known or commercially available starting materials and reagents by one skilled in the art of organic synthesis. Exemplary chiral columns available for use in the separation/purification of the enantiomers/diastereomers provided herein include, but are not limited to, CHIRALPAK^{®} AD-10, CHIRALCEL^{®} OB, CHIRALCEL^{®} OB-H, CHIRALCEL^{®} OD, CHIRALCEL^{®} OD-H, CHIRALCEL^{®} OF, CHIRALCEL^{®} OG, CHIRALCEL^{®} OJ and CHIRALCEL^{®} OK.

**¹H-NMR** reported herein (*e.g.,* for the region between δ (ppm) of about 0.5 to about 4 ppm) will be understood to be an exemplary interpretation of the NMR spectrum (*e*.*g*., exemplary peak integratations) of a compound. Exemplary general method for preparative HPLC: Column: Waters RBridge prep 10 µm C18, 19*250 mm. Mobile phase: acetonitrile, water (NH₄HCO₃) (30 L water, 24 g NH₄HCO₃, 30 mL NH₃.H₂O). Flow rate: 25 mL/min.

Exemplary general method for analytical HPLC: Mobile phase: A: water (10 mM NH₄HCO₃), B: acetonitrile Gradient: 5%-95% B in 1.6 or 2 min Flow rate: 1.8 or 2 mL/min; Column: XBridge C18, 4.6*50mm, 3.5 µm at 45 C.

Exemplary general method for LC-ELSD/MS: Mobile Phase: 1.5mL/4L TFA in water (solvent A) and 0.75mL/4L TFA in acetonitrile (solvent B), using the elution gradient 30%-90% (solvent B) over 0.9 minutes and holding at 90% for 0.6 minutes at a flow rate of 1.2 mL/min; Column: Xtimate C18 2.1*30mm, 3µm; Column temperature: 50°C; PDA, Wavelength: UV 220 nm; MS ionization: ESI & ELSD.

Exemplary general method for SFC: Column: CHIRALPAK^{®} AD CSP (250 mm * 30 mm, 10 µm), Gradient: 45% B, A= NH₃H₂O, B= MeOH, flow rate: 60 mL/min. For example, AD_3_EtOH_DEA_5_40_25ML would indicate: "Column: Chiralpak AD-3 150×4.6mm I.D., 3um Mobile phase: A: CO2 B:ethanol (0.05% DEA) Gradient: from 5% to 40% of B in 5 min and hold 40% for 2.5 min, then 5% of B for 2.5 min Flow rate: 2.5mL/min Column temp: 35°C".

### Abbreviations:

PE: petroleum ether; EtOAc: ethyl acetate; THF: tetrahydrofuran; PCC: pyridinium chlorochromate; TLC: thin layer chromatography; PCC: pyridinium chlorochromate; t-BuOK: potassium tert-butoxide; 9-BBN: 9-borabicyclo[3.3.1]nonane; Pd(t-Bu₃P)₂: bis(tri-tert-butylphosphine)palladium(0); AcCl: acetyl chloride; *i*-PrMgCl: Isopropylmagnesium chloride; TBSCl: tert-Butyl(chloro)dimethylsilane; (*i*-PrO)₄Ti: titanium tetraisopropoxide; BHT: 2,6-di-t-butyl-4-methylphenoxide; Me: methyl; *i*-Pr: iso-propyl; *t-*Bu: tert-butyl; Ph: phenyl; Et: ethyl; Bz: benzoyl; BzCl: benzoyl chloride; CsF: cesium fluoride; DCC: dicyclohexylcarbodiimide; DCM: dichloromethane; DMAP: 4-dimethylaminopyridine; DMP: Dess-Martin periodinane; EtMgBr: ethylmagnesium bromide; EtOAc: ethyl acetate; TEA: triethylamine; AlaOH: alanine; Boc: t-butoxycarbonyl. Py: pyridine; TBAF: tetra-n-butylammonium fluoride; THF: tetrahydrofuran; TBS: t-butyldimethylsilyl; TMS: trimethylsilyl; TMSCF₃: (Trifluoromethyl)trimethylsilane; Ts: p-toluenesulfonyl; Bu: butyl;
Ti(OiPr)₄: tetraisopropoxytitanium; LAH: Lithium Aluminium Hydride; LDA: lithium diisopropylamide; LiOH.H₂O: lithium hydroxide hydrates; MAD: methyl aluminum bis(2,6-di-t-butyl-4-methylphenoxide); MeCN: acetonitrile; NBS: N-bromosuccinimide; Na₂SO₄: sodium sulfate; Na₂S₂O₃: sodium thiosulfate; PE: petroleum ether; MeCN: acetonitrile; MeOH: methanol; Boc: t-butoxycarbonyl; MTBE: methyl tert-butyl ether; K-selectride: Potassium tri(s-butyl)borohydride.

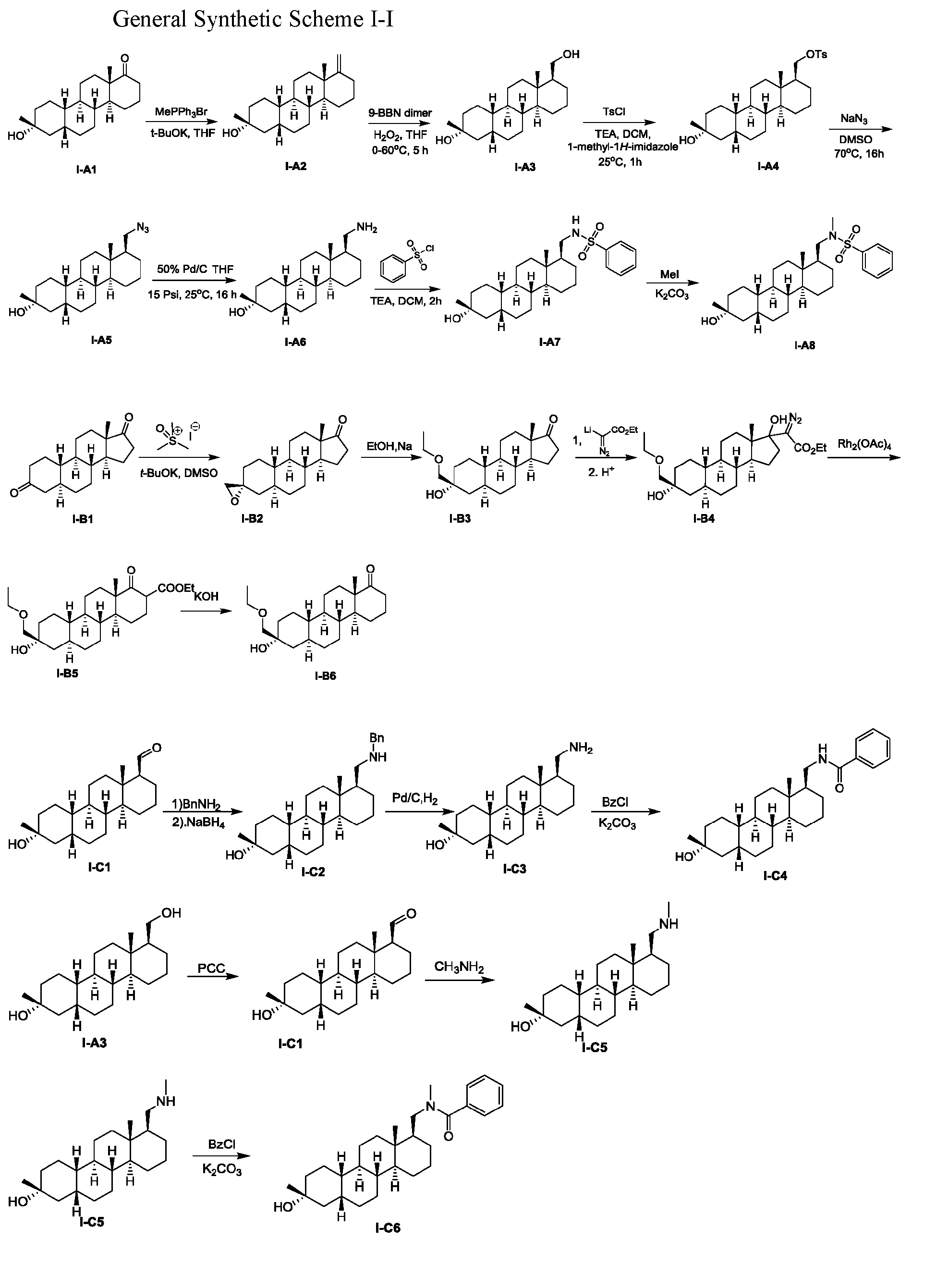

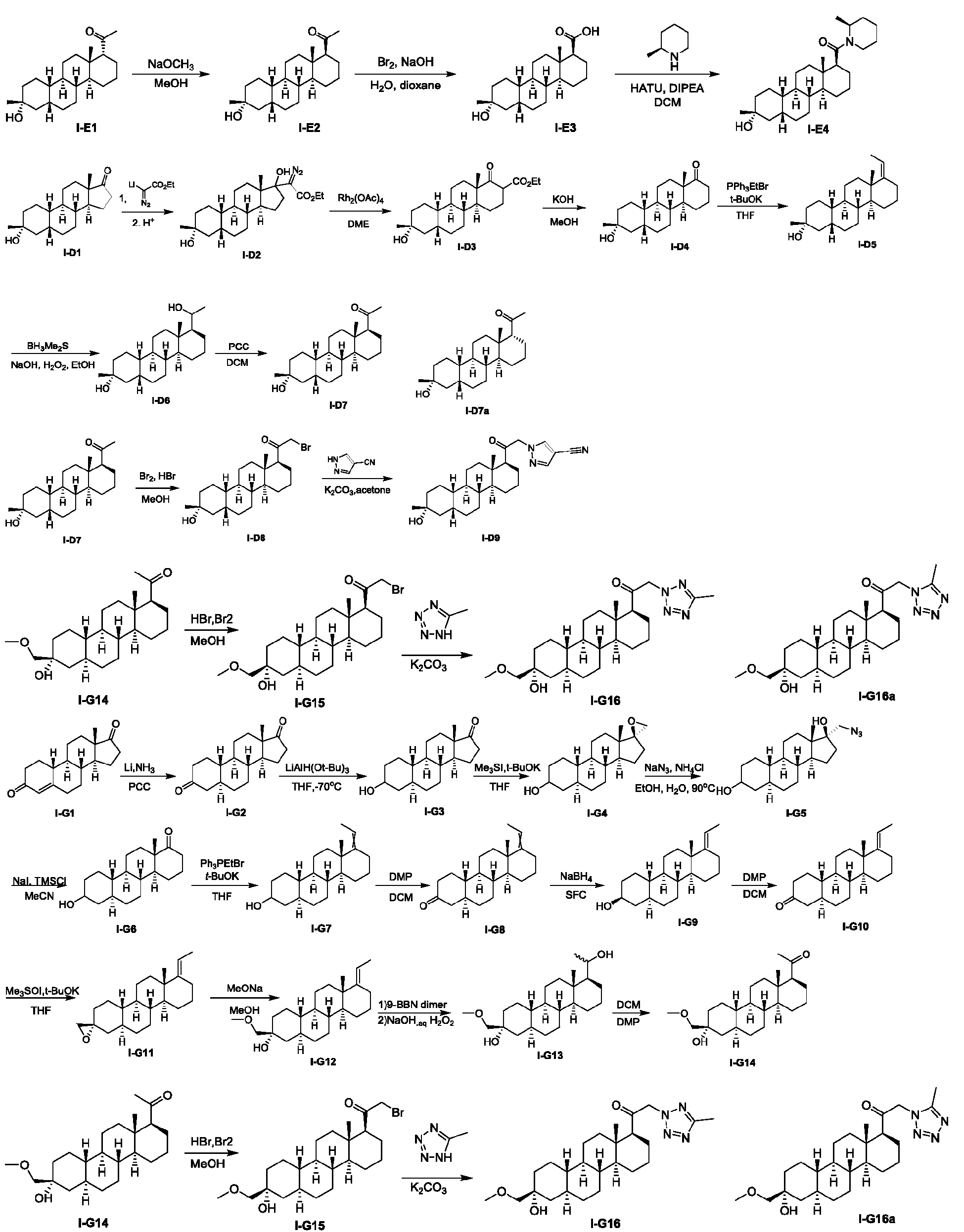

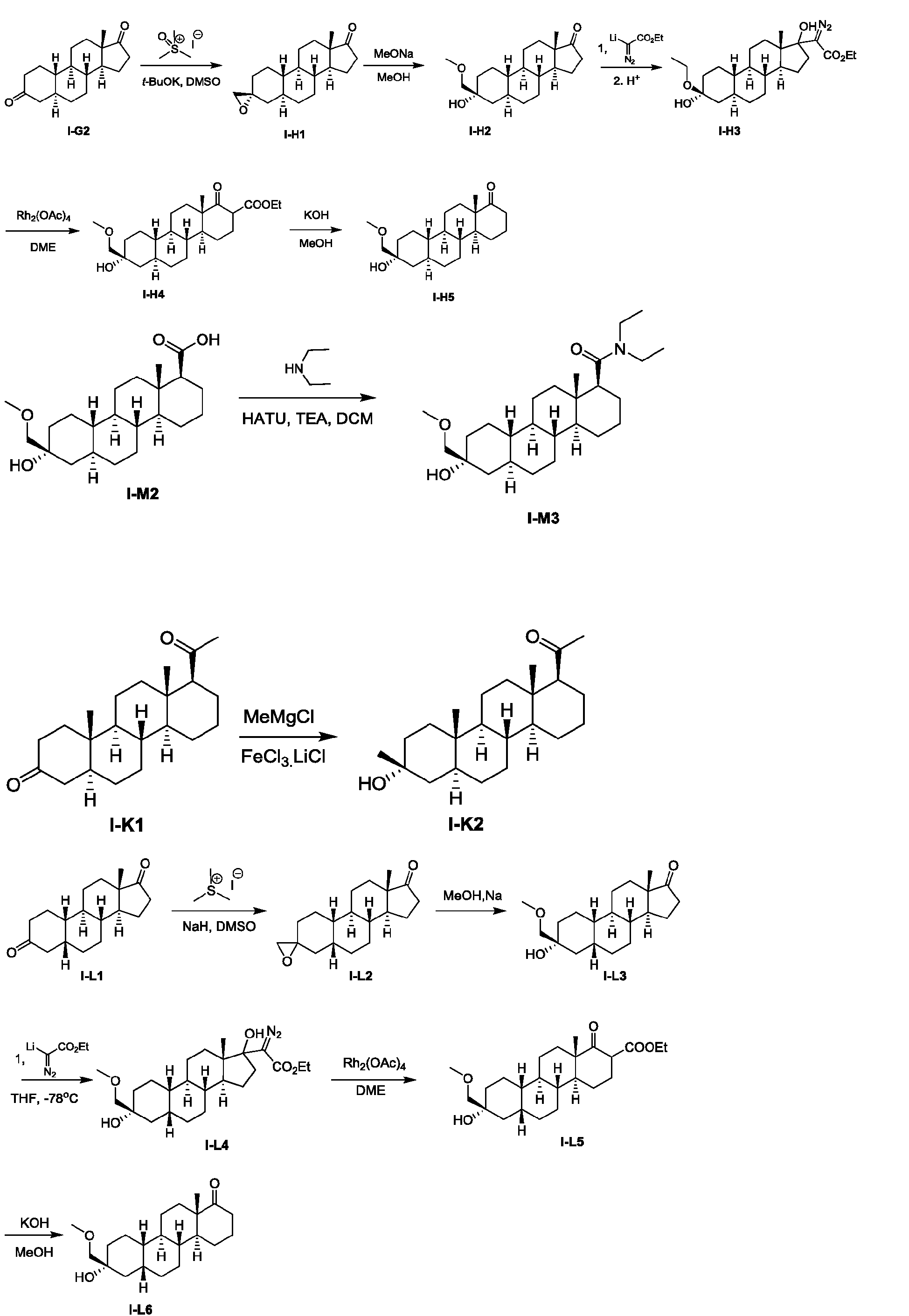

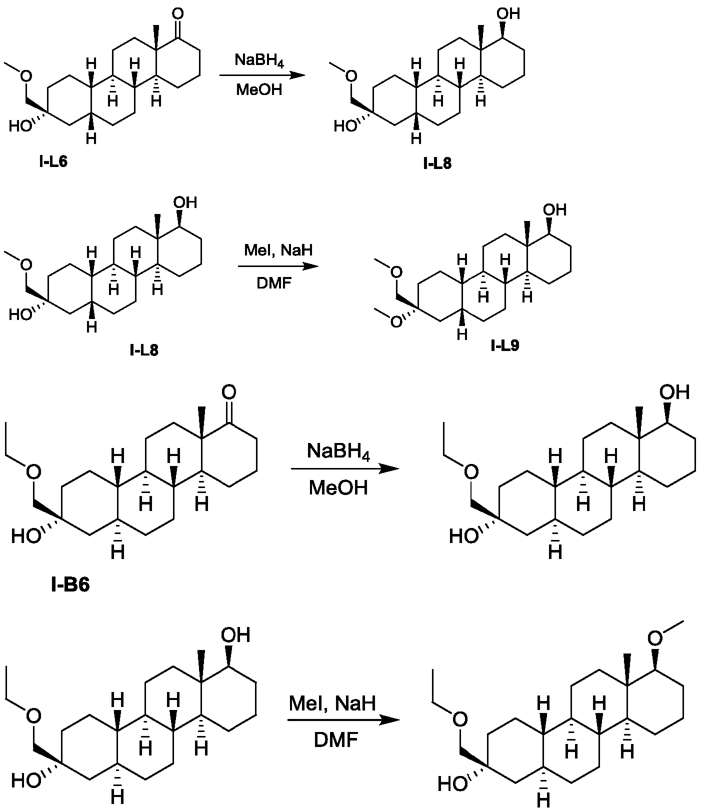

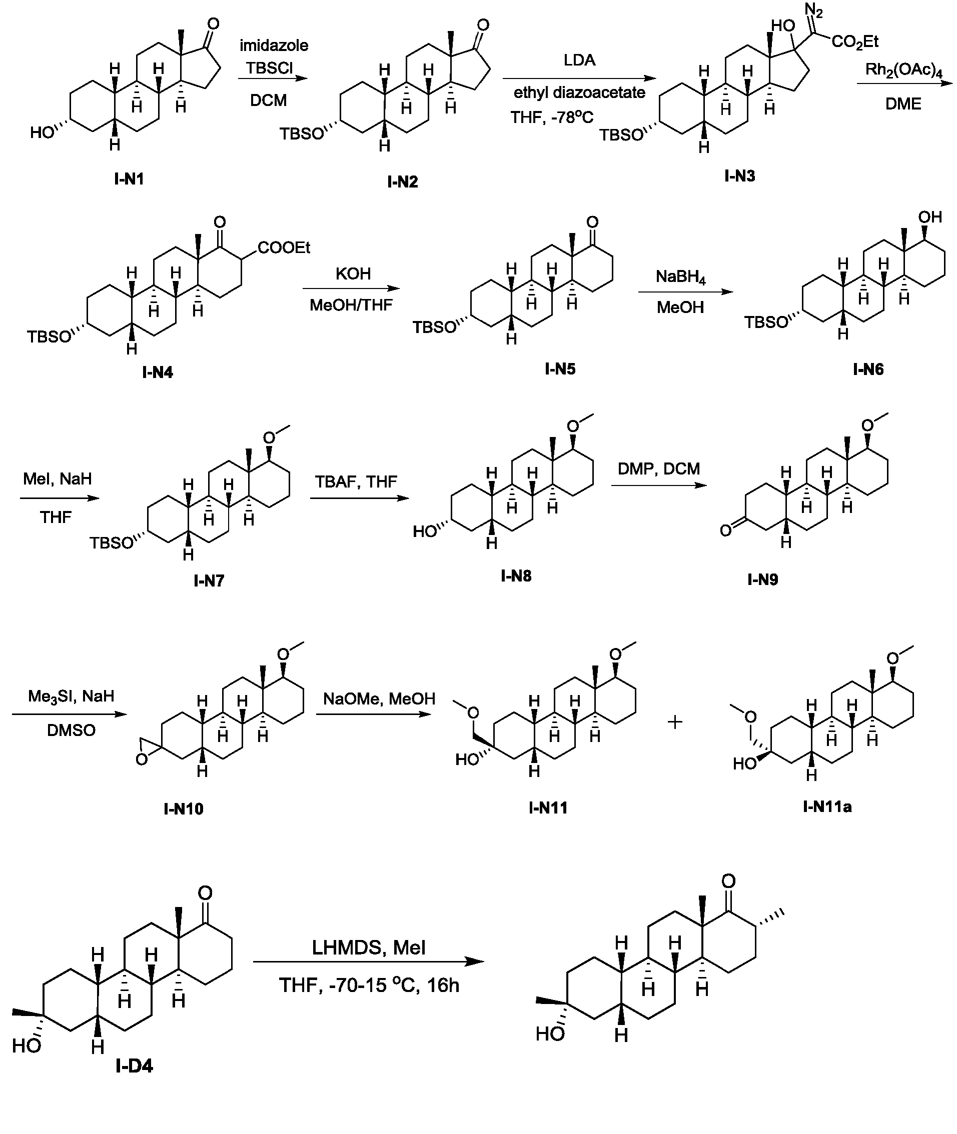

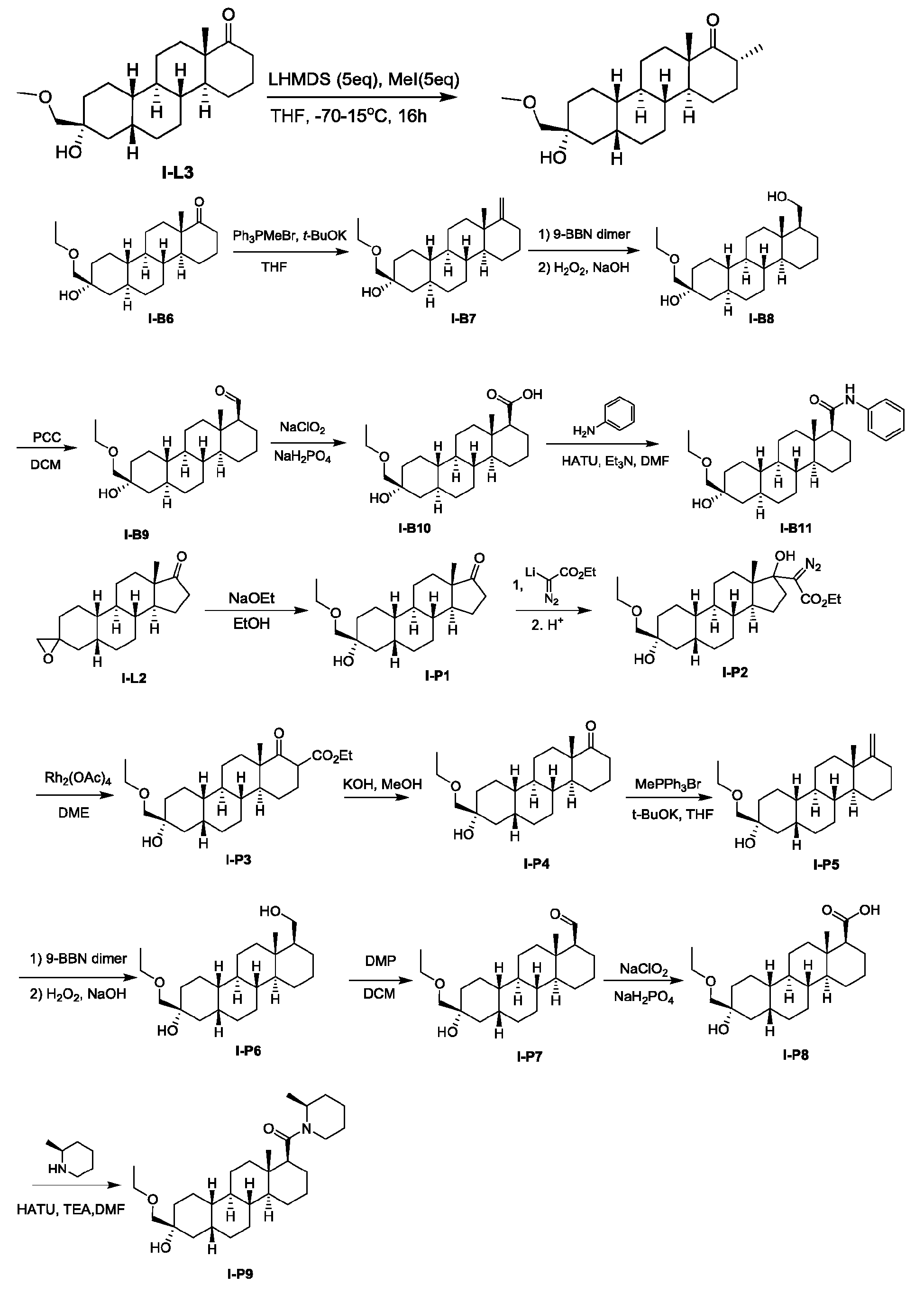

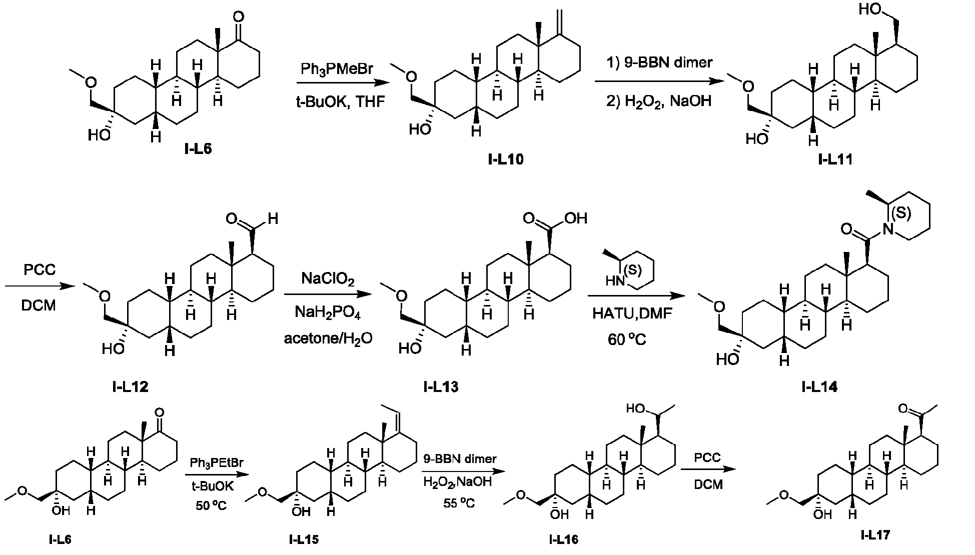

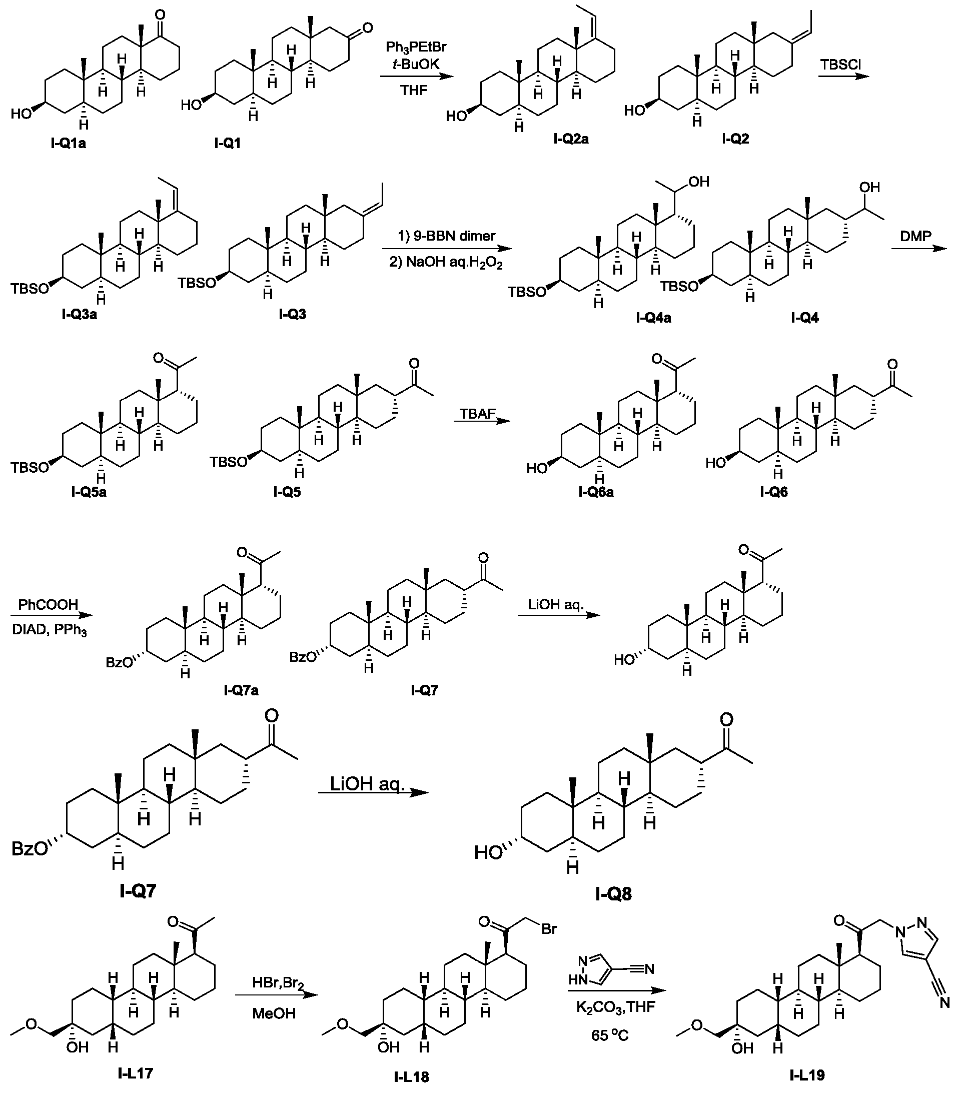

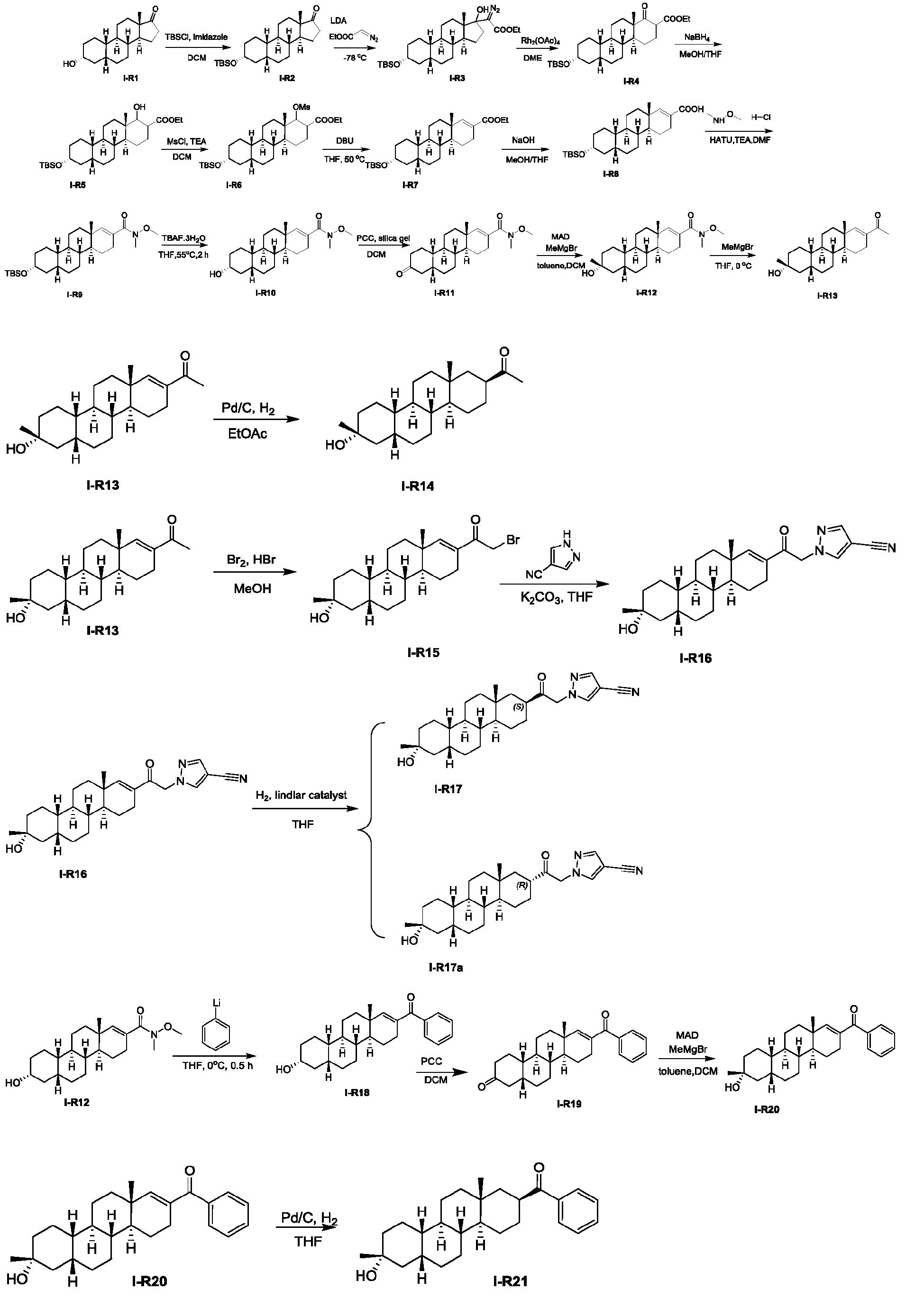

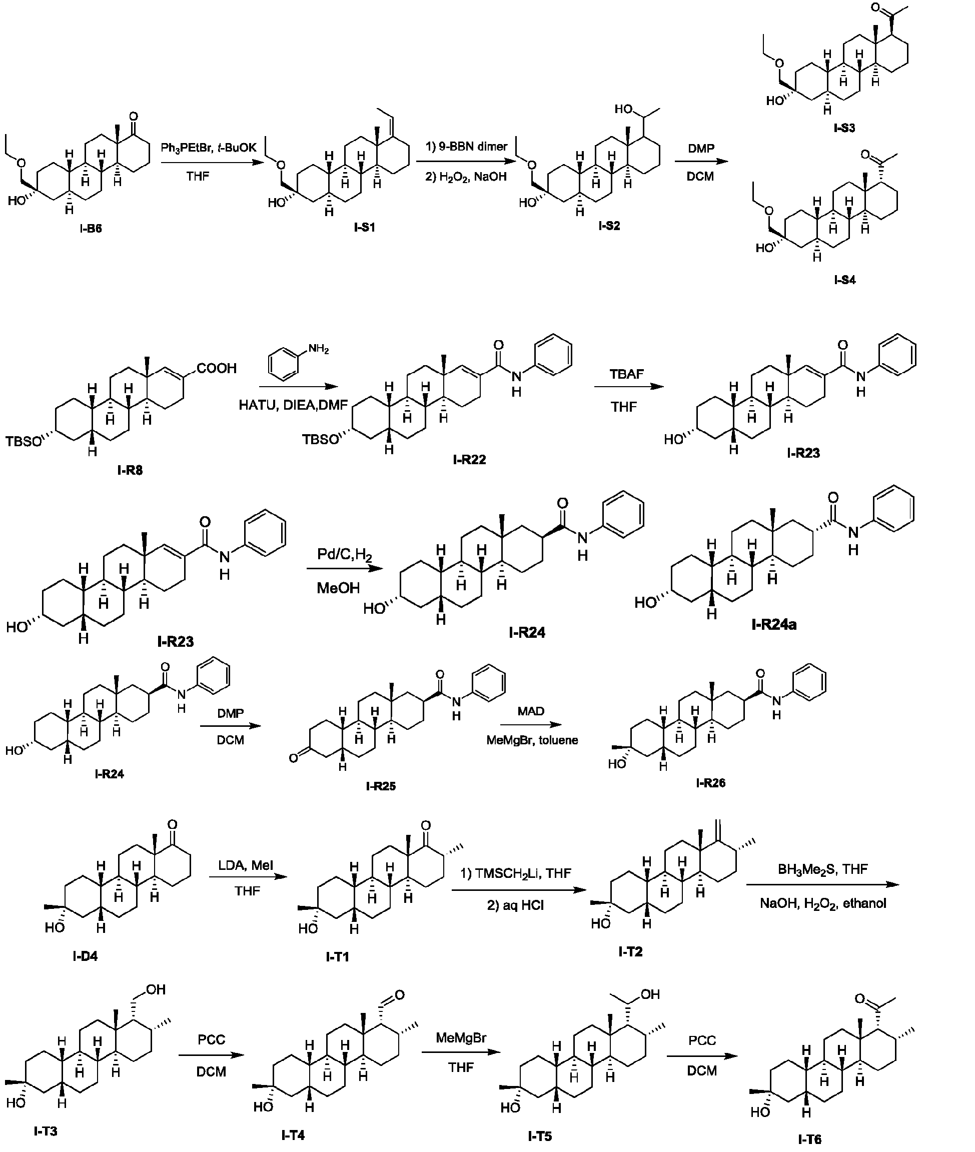

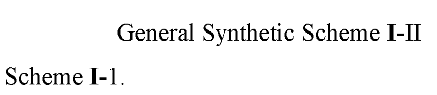

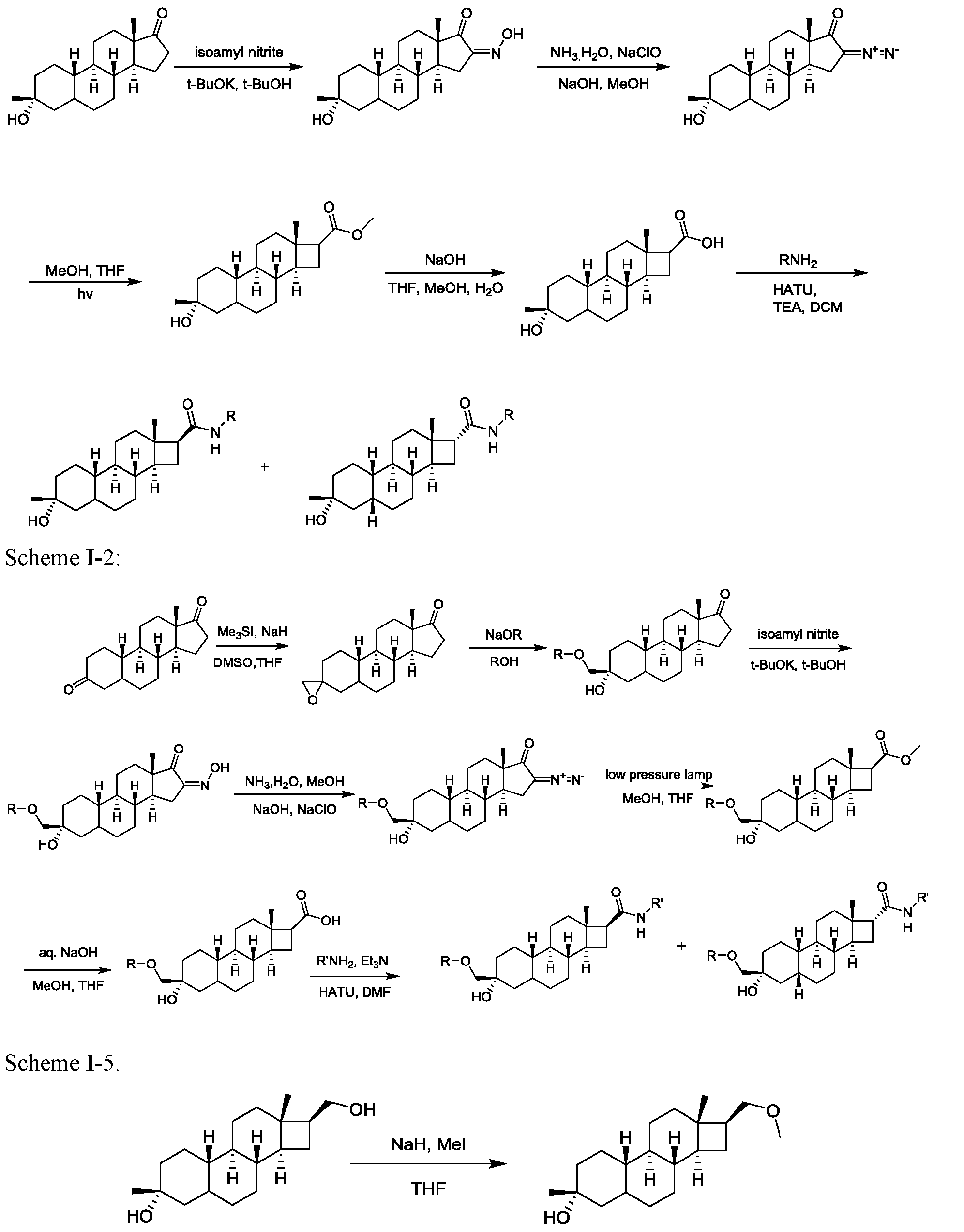

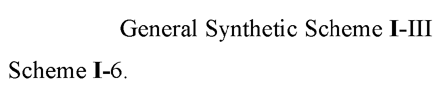

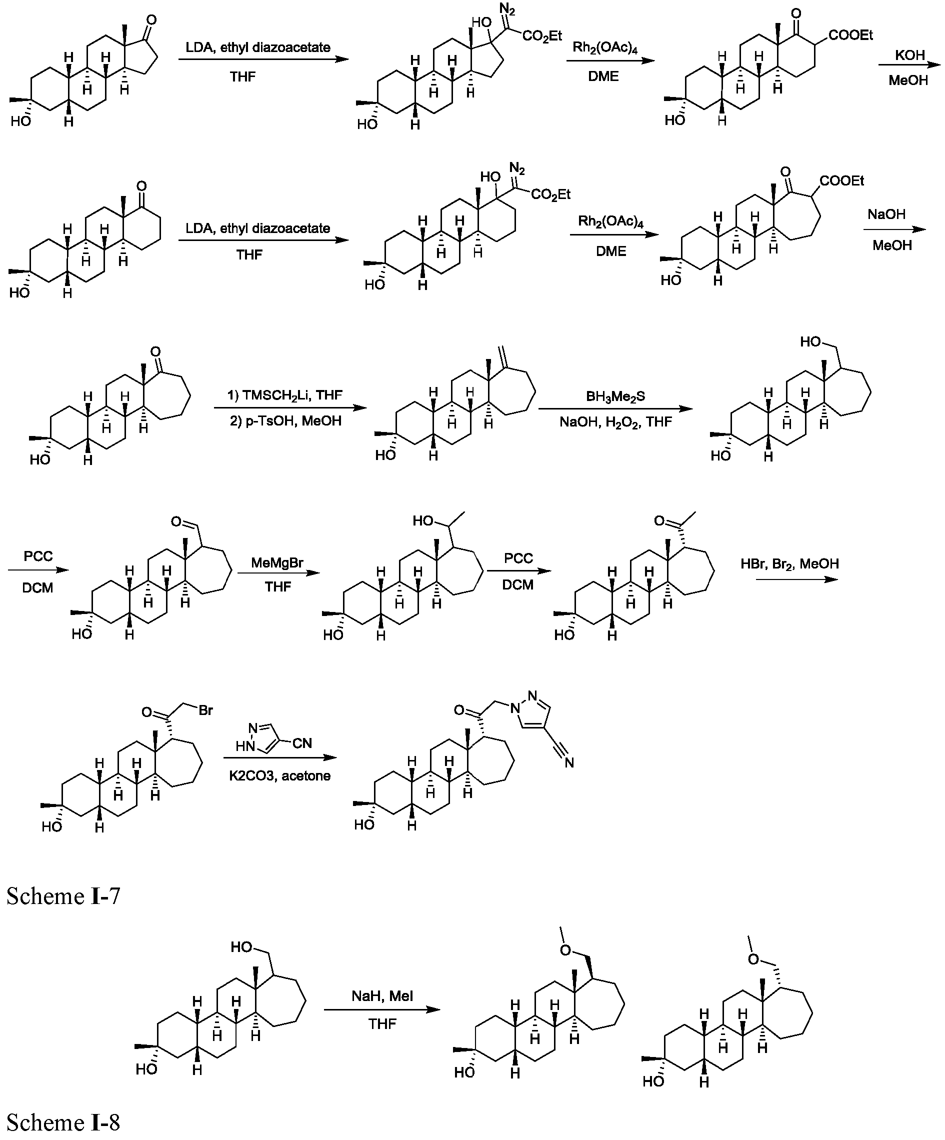

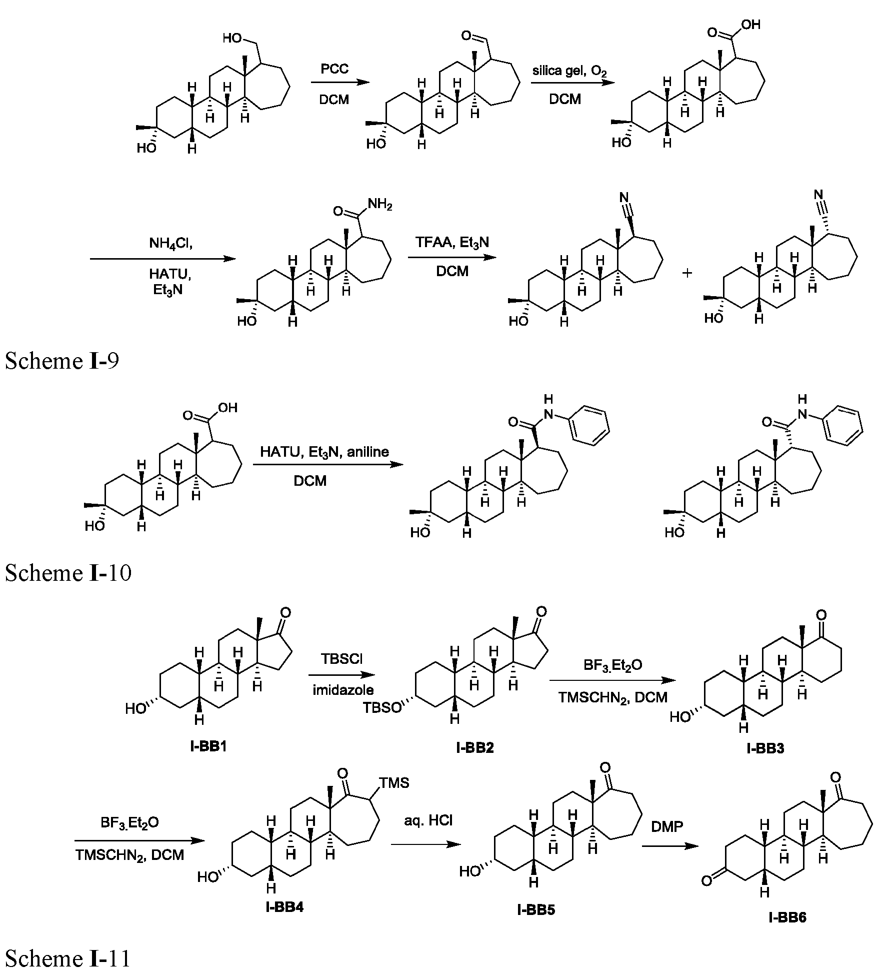

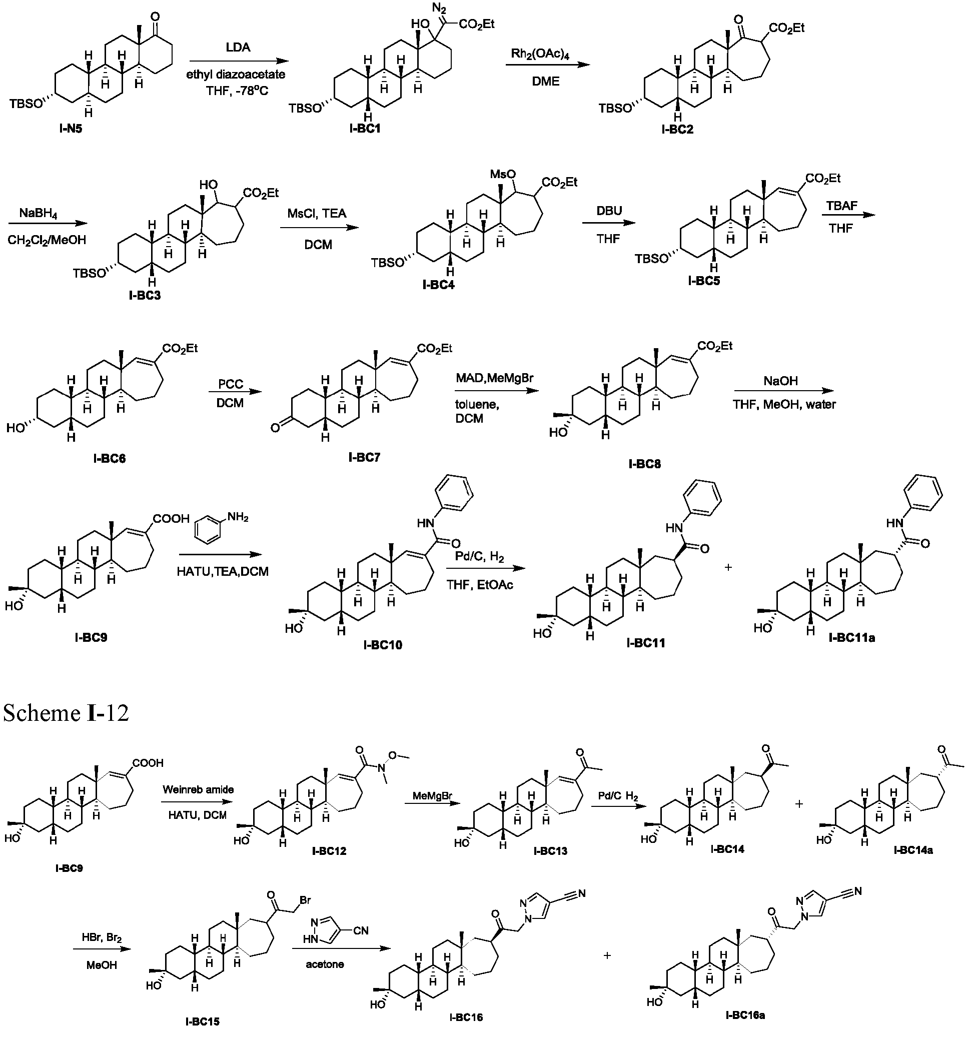

### Example I-1a, I-1 & I-2: Synthesis of (2R,4aS,4bR,6aS,7S,10aS,10bR,12aR)-7-(hydroxymethyl)-2,6a-dimethyloctadecahydrochrysen-2-ol (I-A3), N-(((1S,4aS,4bR,6aR,8R,10aS,10bR,12aS)-8-hydroxy-8,12a-dimethyloctadecahydrochrysen-1-yl)methyl)benzenesulfonamide (I-A7), and N-(((1S,4aS,4bR,6aR,8R,10aS,10bR,12aS)-8-hydroxy-8,12a-dimethyloctadecahydrochrysen-1-yl)methyl)-N-methylbenzenesulfonamide (I-A8)

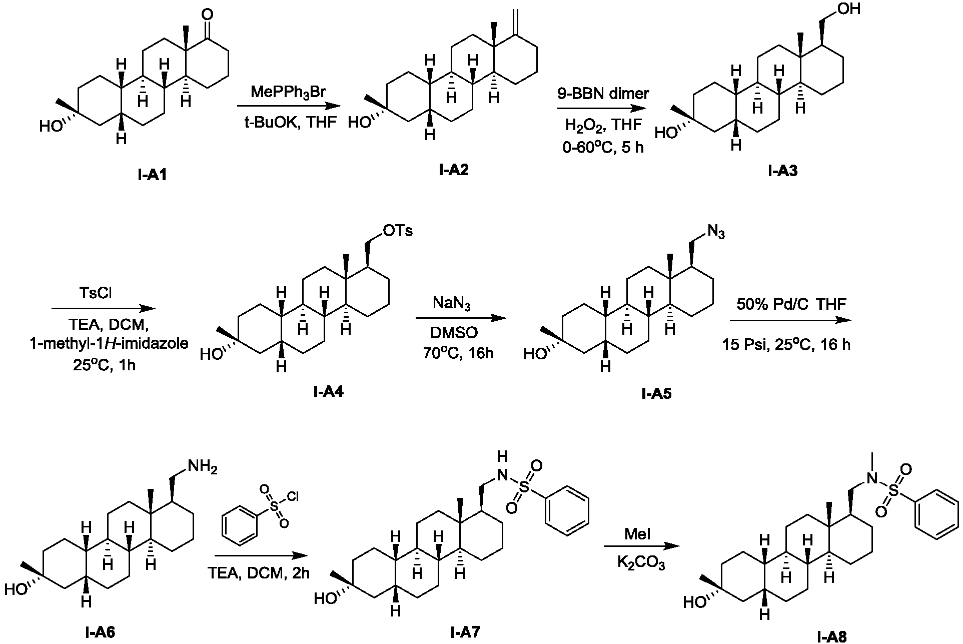

The experimental of intermediate **I-A1** could be found in **Example I-18 (I-D4)** herein.

### Synthesis of I-A2

To a suspension of Ph₃PMeBr (23.3 g, 65.5 mmol) in anhydrous THF (50 mL) was added t-BuOK (7.34 g, 65.5 mmol) at 25°C under N₂. The reaction mixture was stirred at 25°C for 20 min. A solution of **I-A1** (4 g, 13.1 mmol) in anhydrous THF (50 mL) was drop-wise after stirring at 50°C for 1 h. The mixture was poured into ice-water (200 mL) and stirred for 10 mins. The aqueous phase was extracted with EtOAc (2 x 100 mL). The combine organic phase was washed with saturated brine (100 mL), filtered and concentrated. The residue was purified by flash column (0~10% of EtOAc in PE) to give **I-A2** (3.5 g, 88%) as an oil. **¹H NMR** (400 MHz, CDCl₃) δH 4.60-4.56 (m, 2H), 2.36-2.30 (m, 1H), 2.12-2.07 (m, 1H), 1.94-1.64 (m, 8H), 1.51-1.40 (m, 5H), 1.35-1.24 (m, 9H), 1.13-1.05 (m, 2H), 0.98-0.88 (m, 6H)

### Synthesis of I-A3

To a solution of **I-A2** (3.5 g, 11.5 mmol) in anhydrous THF (60 mL) was added 9-BBN dimer (6.94 g, 28.7 mmol) at 25°C under N₂. After stirring at 25°C for 1 h, the mixture was cooled, quenched by EtOH (15 mL) at 0°C. NaOH (11.4 mL, 5M, 57.4 mmol) was added very slowly. After stirring at 60°C for another 1 h the mixture was cooled. The mixture was poured into water (300 mL). The suspension was filtered and the filter cake was washed with Na₂SO₃ (100 mL) and water (100 mL) and concentrated in vacuum to give **I-A3** (3 g) as a solid.

**¹H NMR** (400 MHz, CDCl₃) δH 3.95-3.85 (m, 1H), 3.32-3.23 (m, 1H), 2.42-2.24 (m, 1H), 2.04-1.99 (m, 1H), 1.89-1.81 (m, 3H), 1.68-1.66 (m, 3H), 1.65-1.63 (m, 3H), 1.44-1.37 (m, 4H), 1.32-1.23 (m, 5H), 1.21-1.08 (m, 5H), 0.99-0.94 (m, 3H), 0.89-0.85 (m, 3H), 0.73 (s, 3H).

### Synthesis of I-A4

To a solution of **I-A3** (3 g, 9.35 mmol), TEA (1.41 g, 14 mmol) and 1-methyl-1H-imidazole (767 mg, 9.35 mmol) in DCM (30 mL) was added 4-methylbenzene-1-sulfonyl chloride (2.13 g, 11.2 mmol) at 0°C. The reaction solution was stirred at 25°C for 10 hrs. The reaction mixture was quenched with water (50 mL) and extracted with DCM (20 mL). The combined organic layer was washed with 1N HCl (50 mL), brine (100 mL), dried over Na₂SO₄, filtered and concentrated. The residue was purified by column (0~20% of EtOAc in PE) to give **I-A4** (3.5 g, 79%) as an oil.

**¹H NMR** (400 MHz, CDCl₃) δH 7.82-7.76 (m, 2H), 7.35 (t, *J* = 8Hz, 2H), 4.14-4.09 (m, 2H), 3.75-3.70 (m, 1H), 2.46-2.43 (m, 3H), 2.04 (s, 2H), 1.86-1.74 (m, 3H), 1.66-1.62 (m, 3H), 1.52-1.50 (m, 1H), 1.40-1.36 (m, 3H), 1.27-1.22 (m, 9H), 1.18-1.04 (m, 3H), 0.97-0.78 (m, 6H), 0.69 (s, 2H).

### Synthesis of I-A5

To a solution of **I-A4** (3.5 g, 7.37 mmol) in DMSO (50 mL) was added NaN₃ (955 mg, 14.7 mmol) at 25°C. The mixture was stirred at 70°C for 16 hrs. To the mixture was added aqueous 10% NaHCO₃.aq (100 mL) until pH > 8 and extracted with EtOAc (2 x 80 mL). The combined organic layer was washed with brine (100 mL), dried over Na₂SO₄, filtered and concentrated. The residue was purified by flash column (PE/EtOAc = 2/1) to give **I-A5** (2 g, 79%) as a solid.

**¹H NMR** (400 MHz, CDCl₃) δH 3.59-3.53 (m, 1H), 2.90-2.84 (m, 1H), 1.88-1.84 (m, 1H), 1.81-1.74 (m, 3H), 1.70-1.65 (m, 3H), 1.42-1.36 (m, 6H), 1.32-1.22 (m, 9H), 1.14-1.05 (m, 2H), 0.97-0.94(m, 3H), 0.90-0.85 (m, 3H), 0.74 (s, 3H).

### Synthesis of I-A6

To a solution of **I-A5** (2 g, 5.78 mmol) in THF (30 mL) was added Pd/C (0.5 g, 50% water).

Then the solution was hydrogenated under 103421 Pa (15 psi) of hydrogen at 25°C for 16 hrs. The mixture was filtered through a pad of celite and the filtrate was concentrated in vacuum to afford I-A6 (1.2 g) as an oil.

**¹H NMR** (400 MHz, CDCl₃) δH 2.95-2.91 (m, 1H), 2.25-2.19 (m, 1H), 1.86-1.78 (m, 5H), 1.67-1.58 (m, 5H), 1.44-1.39 (m, 5H), 1.26-1.25 (m, 8H), 1.14-1.04 (m, 4H), 0.95-0.89 (m, 5H), 0.69 (s, 3H).

### Synthesis of I-A7

To a solution of **I-A6** (300 mg, 0.938 mmol) and TEA (236 mg, 2.34 mmol) in DCM (5 mL) was added benzenesulfonyl chloride (247 mg, 1.4 mmol) at 0°C. The mixture was stirred at 25°C for 2 hrs. The mixture was poured into water (10 mL) and extracted with DCM (2 x 20 mL). The combined organic layer was washed with brine (30 mL), dried over Na₂SO₄, filtered and concentrated. The residue was purified by HPLC separation (column: YMC-Actus Triart C18 100*30mm*5um, gradient: 65-95% B (water (0.05%HCl)-ACN), flow rate: 25 mL/min) to give **I-A7** (200 mg) as a solid.

The material was purified by flash column (5% acetone in DCM) to give **I-A7** (120 mg, 70%, 26 mg) as a solid.

**¹H NMR** (400 MHz, CDCl₃) δH 7.87-7.84 (m, 2H), 7.61-7.57 (m, 1H), 7.54-7.50 (m, 2H), 4.25-4.22 (m, 1H), 3.23-3.18 (m, 1H), 2.60-2.53 (m, 1H), 1.93-1.79 (m, 1H), 1.76-1.57 (m, 8H), 1.52-1.31 (m, 4H), 1.29-1.16 (m, 8H), 1.13-1.00 (m, 3H), 0.76-0.72 (m, 6H), 0.66 (s, 3H).

**LC-ELSD/MS** Rt = 1.244 min in 2 min chromatography, 30-90AB_2 min. Lcm. (Mobile Phase: 1.5ML/4LTFA in water (solvent A) and 0.75ML/4LTFA in acetonitrile (solvent B), using the elution gradient 30%-90% (solvent B) over 0.9 minutes and holding at 90% for 0.6 minutes at a flow rate of 1.2 ml/min; Column: Xtimate C18 2.1*30mm, 3um; Wavelength: UV 220 nm; Column temperature: 50°C; MS ionization: ESI; Detector: PDA&ELSD), purity 99%, **MS ESI** calcd. for C₂₇H₄₀NO₂S [M+H-H₂O]⁺ 442, found 442.

### Synthesis of I-A8

To a solution of **I-A7** (94 mg, 0.204 mmol), Cs₂CO₃ (167 mg, 0.51 mmol) in DMF (3 mL) was added iodomethane (34.6 mg, 0.244 mmol) at 25°C. The mixture was stirred at 25°C for 16 hrs. The mixture was poured into water (10 mL) and extracted with EtOAc (2 x 20mL). The combined organic layer was washed with brine (30 mL), dried over Na₂SO₄, filtered and concentrated. The residue was purified by flash column (PE/EtOAc=5/1 to 3/1) to give **I-A8** (26 mg, 27%) as a solid.

**¹H NMR** (400 MHz, CDCl₃) δH 7.78-7.76 (m, 2H), 7.61-7.50 (m, 3H), 2.99-2.93 (m, 1H), 2.80-2.76 (m, 1H), 2.67 (s, 3H), 1.88-1.62 (m, 9H), 1.54-1.15 (m, 15H), 1.08-0.81 (m, 6H), 0.75 (s, 3H).

**LC-ELSD/MS** Rt = 1.330 min in 2 min chromatography, 30-90AB_2 min. Lcm. (Mobile Phase: 1.5ML/4LTFA in water (solvent A) and 0.75ML/4LTFA in acetonitrile (solvent B), using the elution gradient 30%-90% (solvent B) over 0.9 minutes and holding at 90% for 0.6 minutes at a flow rate of 1.2 ml/min; Column: Xtimate C18 2.1*30mm, 3um; Wavelength: UV 220 nm; Column temperature: 50°C; MS ionization: ESI; Detector: PDA&ELSD), purity 99%, MS ESI calcd. for C₂₇H₄₄NO₃S [M+H]⁺ 474, found 474.

### Example I-3: Synthesis of (4aS,4bR,6aS,8R,10aS,10bR,12aS)-8-(ethoxymethyl)-8-hydroxy-12a-methylhexadecahydrochrysen-1(2H)-one (I-B6)

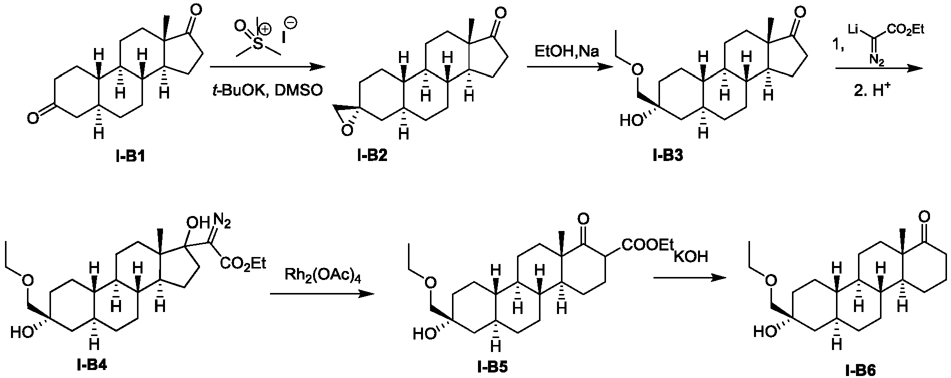

### Synthesis of I-B2

To a suspension of Me₃SOI (8.4 g, 38.2 mmol) in DMSO(150 mL) was added t-BuOK (4.27 g, 38.2 mmol) at 20°C under N₂. The reaction mixture was stirred at 20°C for 30 minutes. A solution of **I-B1** (CAS# 5696-51-5) (10 g, 36.4 mmol) in DMSO (100 mL) was added dropwise. After stirring at 20°C for 1 h, the reaction mixture was poured into water (1500 mL) with stirring and the mixture was filtered. The filter cake was washed with water (2 x 500 mL) and dried to give **I-B2** (10 g) as a solid, which was used in next step directly. **¹H NMR** (400 MHz, CDCl₃) δH 2.64 (s, 2H), 2.50-2.38 (m, 1H), 2.13-2.02 (m, 1H), 2.00-1.73 (m, 6H), 1.71-1.59 (m, 2H), 1.54-1.13 (m, 9H), 1.11-0.98 (m, 2H), 0.92-0.72 (m, 5H).

### Synthesis of I-B3

Na (4.12 g, 72 mmol) was carefully added to anhydrous EtOH (300 mL) in portions. After stirring at 20°C for 1h, a solution of **I-B2** (10 g, 34.6 mmol) in THF (50 mL) was added in portions. After stirring at 80°C under N₂ for 16 hrs, the mixture was quenched with 10% NH₄Cl (500 mL) and extracted with EtOAc (3 x 200 mL). The combined organic phase was washed with 10% NH₄Cl (500 mL), dried over Na₂SO₄, filtered, concentrated and purified by combi-flash (0-20% of EtOAc in PE) to give desired product **I-B3** (8 g, 69%) as a solid.

**¹H NMR** (400 MHz, CDCl₃) δH 3.56-3.49 (m, 2H), 3.21 (s, 2H),2.50-2.39 (m, 1H), 2.15-2.02 (m, 2H), 1.98-1.85 (m, 2H), 1.83-1.71 (m, 4H), 1.67-1.56 (m, 3H), 1.54-1.40 (m, 2H), 1.37-1.17 (m, 7H), 1.15-0.96 (m, 4H), 0.87 (s, 3H), 0.82-0.71 (m, 2H)

### Synthesis of I-B4

A cold (-78°C) solution of lithium diisopropylamine from addition of n-butyl-lithium in hexane (34.4 mL, 2.5 M, 86 mmol) to diisopropylamine (9.79, 96.8 mmol) in THF (100 mL) was added to a stirred solution of **I-B3** (9 g, 26.9 mmol) and ethyl 2-diazoacetate (9.19 g, 80.6 mmol) in THF (500 mL) at -78°C. The mixture was stirred at -78°C for 1 hour. Then acetic acid (14.8 g, 247 mmol) in THF (100 mL) was added. The mixture then warmed to 20°C. After stirring at 20°C for 12 h, the reaction mixture was quenched with water (2 L) and extracted with EtOAc (2 x 600 mL). The combined organic phase was washed with brine (1000 mL), dried by Na₂SO₄, concentrated and purified by combi flash (0-20% of EtOAc in PE) to give **I-B4** (9 g, 75%) as an oil.

**¹H NMR** (400 MHz, CDCl₃) δH 4.31-4.19 (m, 2H), 3.56-3.47 (m, 2H), 3.20 (s, 2H), 2.19-2.09 (m, 1H), 2.06 (s, 1H), 1.94-1.69 (m, 6H), 1.68-1.62 (m, 1H), 1.52-1.32 (m, 3H), 1.31-1.26 (m, 4H), 1.25-1.17 (m, 6H), 1.16-0.93 (m, 7H), 0.92 (s, 3H), 0.75-0.63. (m, 2H).

### Synthesis of I-B5

To a solution of **I-B4** (9 g, 20 mmol) in DME (300 mL) was added Rh₂(OAc)₄ (176 mg, 0.4 mmol) in one portion at 20°C. After stirring at 20°C for 16 hours, the mixture was concentrated to give **I-B5** (8 g) as a solid, which was used directly in next step directly.

### Synthesis of I-B6

To a solution of **I-B5** (8 g, 19 mmol) in MeOH (200 mL) was added KOH (5.32 g, 95 mmol). After stirring at 60°C for 2 h, the mixture was quenched with 10% NH₄Cl (300 mL) and extracted with EtOAc (2 x 150 mL). The combined organic phase was washed with brine (300 mL), dried over Na₂SO₄, filtered, concentrated and purified by combi-flash (0-20% of EtOAc in PE) to give **I-B6** (6.1 g, 92%) as a solid.

**¹H NMR** (400 MHz, CDCl₃) δ_{H} 3.58-3.45 (m, 2H), 3.21 (s, 2H), 2.68-2.55 (m, 1H), 2.22-2.15 (m, 1H), 2.12-2.02 (m, 2H), 1.94-1.72 (m, 6H), 1.65-1.60 (m, 2H), 1.54-1.33 (m, 4H), 1.23-1.15 (m, 6H), 1.08 (s, 3H), 1.06-0.84 (m, 5H), 0.78-0.63 (m, 2H).

**LC-ELSD/MS** Rt = 1.178 min in 30-90AB_2min_E (Column: Xtimate C18 2.1*30mm,3um; Mobile Phase: A: water (4 L)+TFA (1.5 mL) B: acetonitrile (4 L)+TFA (0.75 mL); Gradient: from 30% to 90% of B in 0.9 min and hold 90% for 0.6 min, then 30% of B for 0.5 min; Flow Rate: 1.2mL/min; wavelength: UV 220nm; Oven Temp: 50°C; MS ionization: ESI), purity 99%, **MS ESI** calcd. For C₂₂H₃₅O₂ [M+H-H_{w}O]⁺ 331, found 331.

### Example I-4: Synthesis of N-(((1S,4aS,4bR,6aR,8R,10aS,10bR,12aS)-8-hydroxy-8,12a-dimethyloctadecahydrochrysen-1-yl)methyl)benzamide (I-C4)

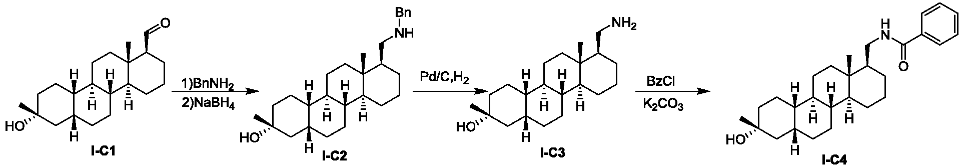

The experimental of intermediate **I-C1** could be found in **Example I-5** herein.

### Synthesis of I-C2

To a solution of **I-C1** (600 mg, 1.88 mmol) in MeOH (10 mL) was added 1-phenylmethanamine (10 mL) at 25°C under N₂. After stirring at 60°C for 30 min, NaBH₄ (213 mg, 5.64 mmol) was added at 25°C. After stirring at 25°C for 30 min, the mixture was poured into water (50 mL), stirred for 10 min and treated with saturated citric acid (50 mL). The aqueous phase was extracted with EtOAc (2 x 100 mL). The organic phase was washed with saturated brine (2 x 50 mL), drive over anhydrous Na₂SO₄, filtered and concentrated. The residue was purified by flash column (0~10% of DCM in MeOH) to give **I-C2** (1 g) as a solid.

500 mg of the **I-C2** was purified by preparative-HPLC (Instrument: FE, Column:YMC-Actus Triart C18 100*30mm*5um, Condition:water(0.05%HCl)-ACN, Begin B:20, End B:90, Gradient Time(min): 10, 100%B Hold Time(min): 1, FlowRate(ml/min): 25) to give **I-C2** (150 mg, 30%) as a solid.

**¹H NMR** (400 MHz, CDCl₃) δH 7.64-7.61 (m, 2H), 7.41-7.38 (m, 3H), 4.25-3.98 (m, 2H), 3.22-2.89 (m, 1H), 2.48-2.30 (m, 1H), 2.13-2.00 (m, 1H), 1.88-1.56 (m, 14H) 1.25-1.00 (m, 10H), 0.96-0.73 (m, 6H), 0.61 (s, 3H).

### Synthesis of I-C3

To a solution of **I-C2** (150 mg, 0.366 mmol) and Pd/C (100 mg, dry) in MeOH (5 mL) was hydrogenated under 103421 Pa (15 psi) of hydrogen at 25°C for 16 hours. The reaction mixture was filtered through a pad of celite and the filter cake was washed with MeOH (3 x 30 mL). The filter liquor was concentrated to give **I-C3** (100 mg) as an oil, which was used directly for the next step.

### Synthesis of I-C4

To a solution of **I-C3** (100 mg, 0.313 mmol) in anhydrous THF (5 mL) was added K₂CO₃ (87.7 mg, 0.623 mmol) and BzCl (87.9 mg, 0.623 mmol) at 25°C under N₂. After stirring at 25°C for 4 h, the mixture was quenched by water (20 mL) and extracted with EtOAc (2 x 50 mL). The organic phase was washed with saturated brine (2 x 50 mL), dried over anhydrous Na₂SO₄, filtered and concentrated. The residue was purified by flash column (5~15% of EtOAc in PE) to give **I-C4** (27 mg, 20%) as a solid.

**¹H NMR** (400 MHz, CDCl₃) δH 7.79-7.67 (m, 2H), 7.51-7.41 (m, 3H), 6.01 (s, 1H), 3.68-3.63 (m, 1H), 3.19-3.14 (m, 1H), 1.98-1.58 (m, 10H), 1.45-1.10 (m, 15H), 1.05-0.84 (m, 5H), 0.82 (s, 3H).

**LC-ELSD/MS** Rt = 1.195 min in 2 min chromatography, 30-90AB_2MIN_E (Column: Xtimate C18 2.1*30mm,3um; Mobile Phase: A: water(4L)+TFA(1.5mL) B: acetonitrile(4L)+TFA(0.75mL); Gradient: from 30% to 90% of B in 0.9 min and hold 90% for 0.6 min, then 30% of B for 0.5 min; Flow Rate: 1.2mL/min; wavelength: UV 220nm; Oven Temp: 50°C; MS ionization: ESI; Detector: PDA, ELSD), purity 99%, **MS ESI** calcd. for C₂₈H₄₂NO₂ [M+H]⁺ 424, found 424.

### Example I-5: Synthesis of (2R,4aS,4bR,6aS,7S,10aS,10bR,12aR)-2,6a-dimethyl-7-((methylamino)methyl)octadecahydrochrysen-2-ol (I-C5)

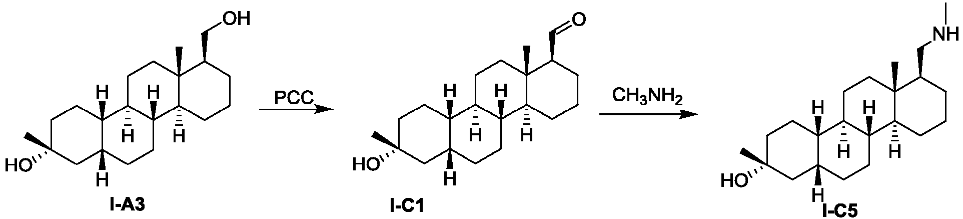

### Synthesis of I-C1

To a solution of **I-A3** (5 g) in DCM (100 mL) was added silica gel (15 g) and PCC (9.99 g, 46.5 mol) at 25°C. After stirring at 25°C for 1 h, PE (100 mL) was added. The mixture was filtered through a pad of silica gel and the filter cake was washed with PE/DCM (2 x 100 mL/100 mL). The filtrate was concentrated in vacuum and purified by silica gel chromatography (PE/EtOAc = 20/1 to 10/1) to afford **I-C1** (2 g, 40%) as an oil.

**¹H NMR** (400 MHz, CDCl₃) δH 10.08 (s, 0.2H), 9.82-9.81 (m, 0.8 H), 2.03-1.96 (m, 1H), 1.98-1.82 (m, 1H), 1.80-1.62 (m, 8H), 1.45-1.21 (m, 15H), 1.00-0.89 (m, 8H).

### Synthesis of I-C5

A solution of **I-C1** (600 mg, 1.88 mmol) in CH₃NH₂ (30 mL, 2M in EtOH) was stirred at 25°C a for 2 h under N₂, followed by adding NaBH₄ (142 mg, 3.76 mmol) at 25°C. After stirring at 25°C for 30 min, the mixture was poured into NH₄Cl (100 mL) and extracted with EtOAc (2 x 100 mL). The organic phase was washed with saturated brine (2 x 50 mL), dried over anhydrous Na₂SO₄, filtered and concentrated. The residue was triturated from EtOAc (20 mL) at 25°C to give **I-C5** (250 mg, 40%) as a solid.

50 mg of **I-C5** (50 mg, 0.150 mmol) was triturated with EtOAc (1 mL) at 70°C to give **I-C5** (31 mg, 77%) as a solid.

**¹H NMR** (400 MHz, CDCl₃) δH 2.75-2.70 (m, 1H), 2.41 (s, 3H), 2.20-2.15 (m, 1H), 1.89-1.44 (m, 12H), 1.38-1.09 (m, 14H), 1.07-0.78 (m, 5H), 0.71 (s, 3H).

**LC-ELSD/MS** Rt = 0.771 min in 2 min chromatography, 30-90AB_2MIN_E (Column: Xtimate C18 2.1*30mm,3um; Mobile Phase: A: water(4L)+TFA(1.5mL) B: acetonitrile(4L)+TFA(0.75mL); Gradient: from 30% to 90% of B in 0.9 min and hold 90% for 0.6 min, then 30% of B for 0.5 min; Flow Rate: 1.2mL/min; wavelength: UV 220nm; Oven Temp: 50°C; MS ionization: ESI; Detector: PDA, ELSD), purity 99%, **MS ESI** calcd. for C₂₂H₄₀NO[M+H]⁺ 334, found 334.

### Example I-6: Synthesis of N-(((1S,4aS,4bR,6aR,8R,10aS,10bR,12aS)-8-hydroxy-8,12a-dimethyloctadecahydrochrysen-1-yl)methyl)-N-methylbenzamide (I-C6)

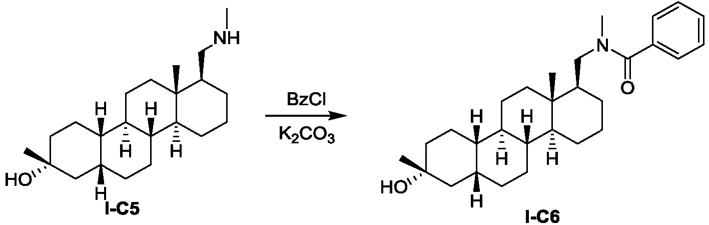

To a solution of **I-C5** (200 mg, 0.599 mmol) in anhydrous THF (10 mL) was added K₂CO₃ (250 mg, 1.79 mmol) and BzCl (251 mg, 1.79 mmol) at 25°C under N₂. After stirring at 25°C for 12 h, the mixture was poured into water (20 mL) and extracted with EtOAc (2 x 50 mL). The organic phase was washed with saturated brine (2 x 50 mL), dried over anhydrous Na₂SO₄, filtered and concentrated. The residue was purified by flash column (5~15% of EtOAc in PE) to give I-C6 (102 mg, 39%) as a solid.

**¹H NMR** (400 MHz, DMSO t=80) δH 7.45-7.37 (m, 3H), 7.35-7.31 (m, 2H), 3.95-3.80 (m, 1H), 3.46-3.28 (m, 2H), 2.88 (s, 3H), 1.83-1.35 (m, 13H), 1.34-1.03 (m, 12H), 0.99-0.79 (m, 5H), 0.74-0.53 (m, 2H).

**LC-ELSD/MS** Rt = 1.263 min in 2 min chromatography, 30-90AB_2MIN_E (Column: Xtimate C18 2.1*30mm,3um; Mobile Phase: A: water (4L)+TFA(1.5mL) B: acetonitrile(4L)+TFA(0.75mL); Gradient: from 30% to 90% of B in 0.9 min and hold 90% for 0.6 min, then 30% of B for 0.5 min; Flow Rate: 1.2mL/min; wavelength: UV 220nm; Oven Temp: 50oC; MS ionization: ESI; Detector: PDA, ELSD), purity 99%, **MS ESI** calcd. for C₂₉H₄₄NO₂ [M+H]⁺ 438, found 438.

### Example I-7: Synthesis 4-(((1S,4aS,4bR,6aR,8R,10aS,10bR,12aS)-8-hydroxy-8,12a-dimethyloctadecahydrochrysen-1-yl)amino)benzonitrile

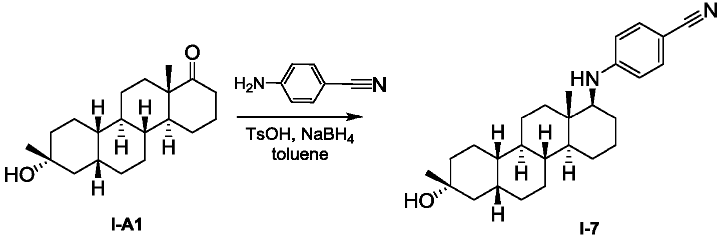

To a solution of **I-A1** (100 mg, 0.328 mmol) in toluene (20 mL) was added 4-aminobenzonitrile (154 mg, 1.31 mmol) and TsOH (28.2 mg, 0.164 mmol) at 25°C under N₂. The mixture was refluxed at 110°C for 10 hrs. After cooling, the reaction was diluted with MeOH (20 mL), followed by adding NaBH₄ (123 mg, 3.26mmol) at 0°C under N₂. The mixture was stirred at 0°C for 1 hrs. The mixture was poured into water (15 mL) and extracted with EtOAc (2 x 20 mL). The combined organic layers was washed with brine (20 mL), dried over Na₂SO₄, filtered and concentrated in vacuum. The residue was purified by HPLC (Instrument: BP ; Method : Column Waters Xbridge 150*25 5um Condition: Water(10mM NH4HCO3)-ACN ; Gradient 67%-87%B ; Gradient Time(min) : 6) to give 4-(((1S,4aS,4bR,6aR,8R,10aS,10bR,12aS)-8-hydroxy-8,12a-dimethyloctadecahydrochrysen-1-yl)amino)benzonitrile (40 mg) as a solid, which was further triturated from MeCN (2 mL) and H₂O (2 mL) at 25°C to give 4-(((1S,4aS,4bR,6aR,8R,10aS,10bR,12aS)-8-hydroxy-8,12a-dimethyloctadecahydrochrysen-1-yl)amino)benzonitrile (17 mg, 29%) as a solid.

**¹H NMR** (400MHz, CDCl₃), 7.37 (d, *J*=8 Hz, 2H), 6.53 (d, *J*=8 Hz, 2H), 3.99-3.93 (m, 1H), 3.12-2.99 (m, 1H), 1.85-1.65 (m, 8H), 1.42-1.22 (m, 14H), 1.17-0.90 (m, 7H), 0.86 (m, 3H). **LC-ELSD/MS** Rt = 1.026 min in 2 min chromatography, 30-90AB_2MIN_E, purity 99%, MS ESI calcd. for C₂₇H₃₉N₂O [M+H]⁺ 407, found 407.

### Examples I-8- I-12

Following examples were made from the listed aniline and **I-A1**. In some cases, the minor diastereomer from reduction at C17 were isolated.

| **Example** | **Aniline** | **Compound Name** | **¹H NMR (400 MHz, CDCl3)** | **MS ESI** |
|---|---|---|---|---|
| **I-8** | 4-chloroani line | (2R,4aS,4bR,6aS ,7S,10aS,10bR,1 2aR)-7-((4-chlorophenyl)am ino)-2,6a-dimethyloctadeca hydrochrysen-2-ol **(I-8)** | δ_{H} 7.06 (d, J=8.8 Hz, 2H), 6.50 (d, J=9.2 Hz, 2H), 3.38 ( s, 1H), 2.92 (s, 1H), 1.86 (d, J=12.8 Hz, 2H), 1.81-1.71 (m, 4H), 1.71-1.59 (m, 4H), 1.54-1.46 (m, 1H), 1.44-1.37 (m, 2H), 1.36-1.27 (m, 6H), 1.26 (s, 4H), 1.15-1.04 (m, 1H), 1.03-0.86 (m, 5H), 0.84 (s, 3H) | calcd. for C26H39ClN O [M+H]+ 416, found 416 |
| **I-9** | 3-chloroani line | (2R,4aS,4bR,6aS ,7S,10aS,10bR,1 2aR)-7-((3-chlorophenyl)am ino)-2,6a-dimethyloctadeca hydrochrysen-2-ol **(I-9)** | δ_{H} 7.05-6.98 (m, 1H), 6.59-6.54 (m, 2H), 6.46-6.41 (m, 1H), 3.47 ( s, 1H), 2.95 (d, J=7.2 Hz, 1H), 1.90-1.81 (m, 2H), 1.81-1.72 (m, 4H), 1.72-1.59 (m, 4H), 1.54-1.47 (m, 1H), 1.44-1.30 (m, 7H), 1.29-1.23 (m, 5H), 1.16-1.05 (m, 1H), 1.04-0.86 (m, 5H), 0.84 (s, 3H) | calcd. for C26H39ClN O [M+H]+ 416, found 416 |
| **I-10** | 3-aminobe nzonitrile | 3-(((1S,4aS,4bR,6a R,8R,10aS,10bR, 12aS)-8-hydroxy-8,12a-dimethyloctadeca hydrochrysen-1-yl)amino)benzon itrile (**I-10**) | δ_{H} 7.19-7.13 (m, 1H), 6.89-6.85 (m, 1H), 6.78-6.70 (m, 2H), 3.65-3.60 (m, 1H), 3.01-2.90 (m, 1H), 1.93-1.56 (m, 10H), 1.50-0.98 (m, 13H), 1.15-0.86 (m, 6H), 0.85 (s, 3H). | calcd. for C27H39N2 O [M +H]+ 407, found 407 |
| **I-10a** | 3-aminobe nzonitrile | 3-(((1R,4aS,4bR,6a R,8R,10aS,10bR, 12aS)-8-hydroxy-8,12a-dimethyloctadeca hydrochrysen-1-yl)amino)benzon itrile **(I-10a)** | δ_{H} 7.22-7.15 (m, 1H), 6.89-6.85 (m, 1H), 6.80-6.76 (m, 2H), 4.17-4.11 (m, 1H), 3.15-3.10 (m, 1H), 1.89-1.56 (m, 11H), 1.50-1.15 (m, 13H), 1.15-0.86 (m, 8H). | calcd. for C27H39N2 O [M +H]+ 407, found 407 |
| **I-11** | 4-fluoroani line | (2R,4aS,4bR,6aS ,7S,10aS,10bR,1 2aR)-7-((4-fluorophenyl)ami no)-2,6a-dimethyloctadeca hydrochrysen-2-ol **(I-11)** | δ_{H} 6.85-6.81 (m, 2H), 6.54-6.48 (m, 2H), 2.90-2.82 (m, 1H), 1.95-1.55 (m, 10H), 1.50-1.21 (m, 14H), 1.15-0.90 (m, 6H),0.84 (s, 3H). | calcd. for C₂₆H₃₉FNO [M+H]⁺ 400, found 400 |
| **I-11a** | 4-fluoroani line | (2R,4aS,4bR,6aS ,7R,10aS,10bR,1 2aR)-7-((4-fluorophenyl)ami no)-2,6a-dimethyloctadeca hydrochrysen-2-ol **(I-11a)** | δ_{H} 6.89-6.81 (m, 2H), 6.55-6.48 (m, 2H), 3.04 (s, 1H), 1.90-1.55 (m, 12H), 1.50-1.15 (m, 13H), 1.15-0.94 (m, 8H). | calcd. for C₂₆H₃₉FNO [M+H]⁺ 400, found 400 |
| **I-12** | 3-fluoroani line | (2R,4aS,4bR,6aS ,7S,10aS,10bR,1 2aR)-7-((3-fluorophenyl)ami no)-2,6a-dimethyloctadeca hydrochrysen-2-ol **(I-12)** | δ_{H} 7.08-6.98 (m, 1H), 6.38-6.23 (m, 3H), 3.52 (brs, 1H), 2.96-2.91 (m, 1H), 1.88-1.56 (m, 10H), 1.45-1.20 (m, 13H), 1.15-0.85 (m, 6H), 0.84 (s, 3H). | calcd. for C₂₆H₃₉FNO [M +H]⁺ 400, found 400 |

### Example I-13: Synthesis of ((15,4aS,4bR,6aR,8R,10aS,10bR,12aS)-8-hydroxy-8,12a-dimethyloctadecahydrochrysen-1-yl)((S)-2-methylpiperidin-1-yl)methanone (I-E4)

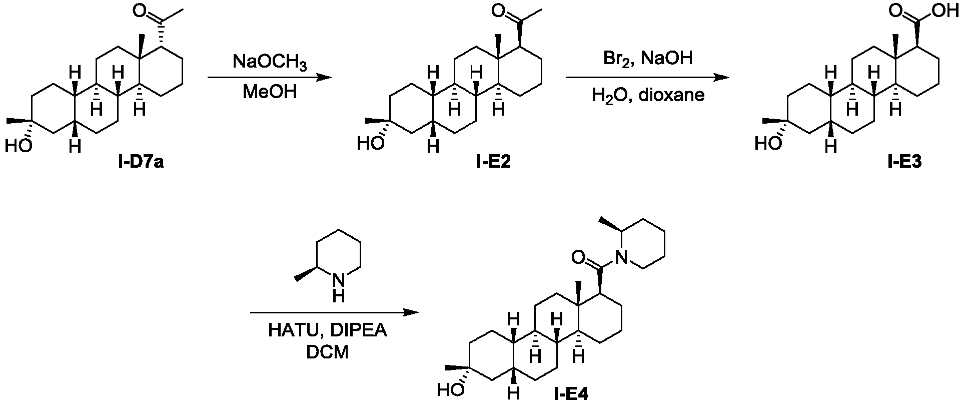

The experimental of intermediate **I-D7a** could be found in **Example I-18.**

### Synthesis of I-E2

To a solution of **I-D7a** (2.1 g, 6.31 mmol) in MeOH (50 mL) was added MeONa (3.40 g, 63.0 mmol). The mixture was stirred at 60°C for 40 hours. After cooling, the mixture was quenched by adding water (10 mL) and ethyl acetate (50 mL). The mixture was poured into ice water (50 mL) and extracted with EtOAc (2 x 100mL). The combined organic phase was washed with saturated brine (100 mL), dried over anhydrous Na₂SO₄, filtered and concentrated in vacuum. The residue was triturated by PE (40 mL) to give **I-E2** (1.1 g, 53%) as a solid and the mother liquid was concentrated to give **I-E2** (1.0 g) as an oil.

**¹H NMR** (400 MHz, CDCl3) δ 2.30 (dd, *J* = 3.2, 12.8 Hz, 1H), 2.14 (s, 3H), 1.91-1.57 (m, 10H), 1.54-1.28 (m, 9H), 1.26 (s, 3H), 1.04-0.93 (m, 4H), 0.92 (s, 3H), 0.89-0.81 (m, 3H).

### Synthesis of I-E3

Liquid bromine (1.44 g, 9.00 mmol) was added slowly to a vigorously stirred sodium hydroxide aqueous (9 mL, 4 M, 36.0 mmol) at 0°C. When all the bromine was dissolved, the mixture was added slowly to a stirred solution of **I-E2** (500 mg, 1.50 mmol) in dioxane (20 mL) and water (5 mL). The homogeneous solution slowly formed a precipitate, and the reaction mixture was stirred at 25°C for 16 hours. The remaining oxidizing reagent was quenched by Na₂S₂O₃ aqueous (50 mL) and the mixture was then heated at 80°C until the solid material was dissolved. Acidification of the solution with hydrochloride acid (3 N, 10 mL) furnished a precipitate. The solid was filtered and washed with water (3 x 100 mL) to give a solid, which was purified by flash column chromatography (ethyl acetate in PE, 15%) to afford **I-E3** (100 mg, 20%) as a solid.

**¹H NMR** (400 MHz, CDCl₃) δH 1.96 (dd, *J* = 3.6, 12.8 Hz, 1H), 1.78-1.37 (m, 12H), 1.35 (s, 2H), 1.33-1.12 (m, 8H), 1.09 (s, 3H), 0.99-0.80 (m, 8H).

### Synthesis of I-E4

To a solution **I-E3** (100 mg, 0.2989 mmol) and DIPEA (115 mg, 0.8967 mmol) in DMF (4 mL) was added HATU (227 mg, 0.5978 mmol) at 25°C, followed by adding (2S)-2-methylpiperidine (44.4 mg, 0.4483 mmol). After stirring at 25°C for 16 hours, the reaction mixture was extracted with ethyl acetate (3 x 60 mL). The combined organic phase was washed with water (3 x 100 mL), brine (60 mL), dried over Na₂SO₄, filtered and concentrated under vacuum to give a solid, which was purified by prep-HPLC (column: Boston Green ODS 150*30 5u, Condition: water(0.1%TFA)-ACN, Begin B: 55%, End B:85%, Gradient Time: 8 min, FlowRate:25 ml/min) to give **I-E4** (13 mg, 10%) as a solid.

**¹H NMR** (400 MHz, CDCl₃) δH 5.02-4.93 (m, 0.6H), 4.58-4.50 (m, 0.4H), 4.31-4.26 (m, 0.4H), 3.93-3.85 (m, 0.6H), 3.16-3.02 (m, 0.6H), 2.61 (*J* = 13.2 Hz, 0.4H), 2.41-2.28 (m, 1H), 1.96-1.73 (m, 5H), 1.71-1.60 (m, 7H), 1.59-1.50 (m, 7H), 1.50-1.20 (m, 13H), 1.13-0.79 (m, 9H).

**LC-ELSD/MS** Rt = 1.199 min in 2 min chromatography, 30-90AB_2MIN_E.M, purity 98%, **MS ESI** calcd. for C₂₇H₄₆NO₂ [M+H]⁺ 416, found 416.

### Examples I-14- I-17: Synthesis of (1S,4aS,4bR,6aR,8R,10aS,10bR,12aS)-N-benzyl-8-hydroxy-8,12a-dimethyloctadecahydrochrysene-1-carboxamide (I-14), (1S,4aS,4bR,6aR,8R,10aS,10bR,12aS)-8-hydroxy-8,12a-dimethyl-N-phenyloctadecahydrochrysene-1-carboxamide (I-15), (1S,4aS,4bR,6aR,8R,10aS,10bR,12aS)-N,N-diethyl-8-hydroxy-8,12a-dimethyloctadecahydrochrysene-1-carboxamide (I-16), and (1S,4aS,4bR,6aR,8R,10aS,10bR,12aS)-8-hydroxy-8,12a-dimethyl-N-(pyridin-2-ylmethyl)octadecahydrochrysene-1-carboxamide (I-17).

The following examples were made from **I-E3** with the listed amines instead of (2S)-2-methylpiperidine.

| **Example** | **amine** | **Compound Name** | **¹H NMR (400 MHz, CDCl₃)** | **MS ESI** |
|---|---|---|---|---|
| **I**-14 | Benzyl amine | (1S,4aS,4bR,6aR,8R,10 aS,10bR,12aS)-N-benzyl-8-hydroxy-8,12a-dimethyloctadecahydro chrysene-1-carboxamide (**I-14**) | δ_{H} 7.41-7.27 (m, 5H), 5.65-5.55 (m, 1H), 4.49-4.35 (m, 2H), 1.95-1.59 (m, 11H), 1.53-1.28 (m, 9H), 1.26 (s, 3H), 1.23-1.05 (m, 3H), 1.00 (s, 3H), 0.98-0.89 (m, 4H). | calcd. for C₂₈H₄₂NO₂ [M+H]⁺ 424, found 424 |
| **I**-15 | aniline | (1S,4aS,4bR,6aR,8R, 10 aS,10bR,12aS)-8-hydroxy-8,12a-dimethyl-N-phenyloctadecahydroch rysene-1-carboxamide (**I-15**) | δ_{H} 7.51-7.49 (m, 2H), 7.39-7.29 (m, 2H), 7.15-7.08 (m, 1H), 7.07-7.01 (m, 1H), 1.95-1.59 (m, 13H), 1.53-1.28 (m, 7H), 1.27 (s, 3H), 1.25-1.05 (m, 3H), 1.03 (s, 3H), 1.01-0.89 (m, 4H). | calcd. for C₂₇H₄₀NO₂ [M+H]⁺ 410, found 410. |
| **I**-16 | diethyl amine | (1S,4aS,4bR,6aR,8R,10 aS,10bR,12aS)-N,N-diethyl-8-hydroxy-8,12a-dimethyloctadecahydro chrysene-1-carboxamide (**I-16**) | δ_{H} 3.65-3.51 (m, 2H), 3.25-3.05 (m, 2H), 2.27-2.24 (m, 1H), 1.95-1.59 (m, 10H), 1.53-1.28 (m, 10H), 1.26 (s, 3H), 1.24-1.09 (m, 7H), 1.07 (s, 3H), 0.99-0.89 (m, 5H) | calcd. for C₂₅H₄₄NO₂ [M+H]⁺ 390, found 390 |
| **I**-17 | 1-(pyridin -2-yl) methanamine | (1S,4aS,4bR,6aR,8R,10 aS,10bR,12aS)-8-hydroxy-8,12a-dimethyl-N-(pyridin-2-ylmethyl)octadecahydr ochrysene-1-carboxamide (**I-17**) | δ_{H} 8.55-8.53 (m, 1H), 7.66-7.65 (m, 1H), 7.25-7.24 (m, 1H), 7.23-7.21 (m, 1H), 6.66-6.59 (m, 1H), 4.55-4.45 (m, 2H), 1.95-1.56 (m, 11H), 1.53-1.28 (m, 10H), 1.26 (s, 3H), 1.23-1.02 (m, 3H), 0.99 (s, 3H), 0.98-0.89 (m, 3H). | calcd. for C₂₇H₄₁N₂O ₂ [M+H]⁺ 425, found 425 |

### Example I-18 and 1-18a: Synthesis of 1-((1S,4aS,4bR,6aR,8R,10aS,10bR,12aS)-8-hydroxy-8,12a-dimethyloctadecahydrochrysen-1-yl)ethan-1-one (I-D7) and 1-((1R,4aS,4bR,6aR,8R,10aS,10bR,12aS)-8-hydroxy-8,12a-dimethyloctadecahydrochrysen-1-yl)ethan-1-one (I-D7a)

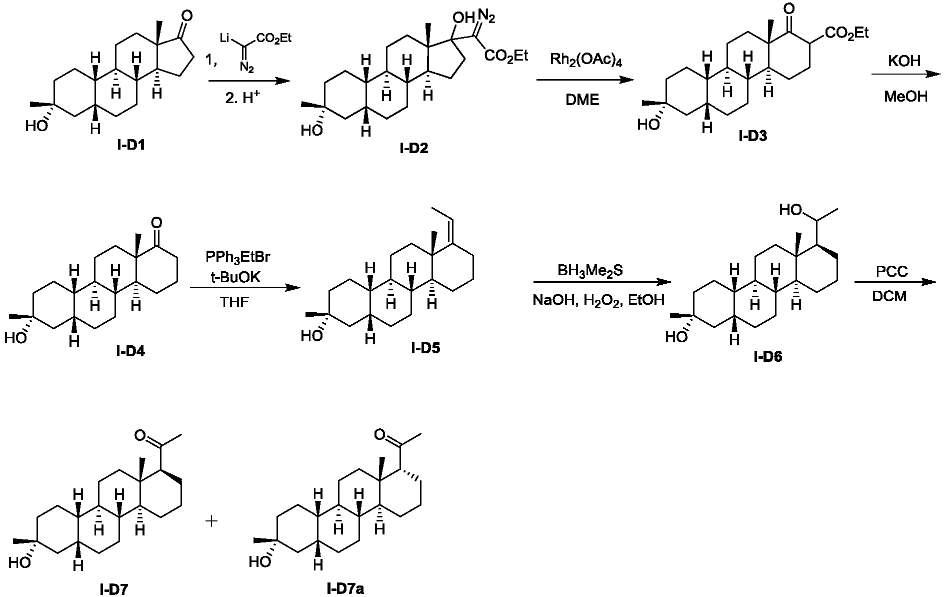

The experimental of intermediate **I-D1** could be found in WO 2014/169833, Example 1.

### Synthesis of I-D2

A cold (-78°C) solution of lithium di-isopropylamide prepared from n-butyl-lithium (6.55 mL 2.5 M in hexane, 16.4 mmol) with di-isopropylamine (2.59 mL, 0.72g/mL, 18.5 mmol) in THF (20 mL) was added to a stirred solution of **I-D1** (3 g, 10.3 mmol) and ethyl diazoacetate (1.75 g, 15.4 mmol) in THF (60 mL) at -78°C. The mixture was stirred at -78°C for 1 hours. Then acetic acid (1.1 g, 18.5 mmol) in THF (20 mL) was added to quench the reaction at - 78°C. The mixture was then warmed to 20°C. Water (100 mL) was added. The aqueous solution was extracted with diethyl ether (3 x 100 mL). The combined organic layers were washed with brine, dried over Na₂SO₄, and evaporated under reduced pressure to give the product as an oil, and then purified by combi flash (0-5% of EtOAc in PE) to give **I-D2** (2.6 g, 63%) as an oil.

**¹H NMR** (400 MHz, CDCl₃) δH 4.75-4.65 (m, 1H), 4.27-4.24 (m, 2H), 2.20-2.09 (m, 2H), 1.94-1.58 (m, 8H), 1.52-1.36 (m, 7H), 1.32 (t, *J =* 7.2 Hz, 4H), 1.27 (s, 3H), 1.21-0.96 (m, 6H), 0.92 (s, 3H).

### Synthesis of I-D3

To a solution of **I-D2** (2.6 g, 6.42 mmol) in DME (25 mL) was added Rh₂(OAc)₄ (25.5 mg, 0.0577 mmol) in one portion at 20°C. The mixture was stirred at 20°C for 18 hrs. The reaction mixture was concentrated to give **I-D3** (2.2 g, 91%) as an oil.

**¹H NMR** (400 MHz, CDCl₃) δH 3.70 (dd, *J=*13.6, 6.0 Hz, 1H), 2.39-2.28 (m, 1H), 1.80-1.75 (m, 3H), 1.65-1.49 (m, 10H), 1.32-1.29 (m, 4H), 1.29-1.25 (m, 9H), 1.11 (d, *J =* 11.6 Hz, 4H), 1.08-0.85 (m, 4H).

### Synthesis of I-D4

To **I-D3** (2.2 g, 5.84 mmol) was added a solution of KOH (19.5 g, 35 mmol) in MeOH (220 mL) at 20°C. The reaction mixture was refluxed at 70°C for 1 hour. The reaction was poured into brine (200 mL), then extracted with DCM (3 x 400 mL). The combined organic layers were washed with HCl (1 M, 200 mL), saturated NaHCO₃ (200 mL), brine (200 mL), dried over Na₂SO₄, filtered and concentrated to give the **I-D4** (1.7 g, 96%) as a solid.

**¹H NMR** (400 MHz, CDCl₃) δH 2.65-2.57 (m, 1H), 2.23-2.16 (m, 1H), 2.08-2.01 (m, 1H), 1.92-1.60 (m, 9H), 1.51-1.27 (m, 10H), 1.26-1.24 (m, 4H), 1.23-1.09 (m, 2H), 1.08 (s, 3H), 1.07-0.91 (m, 1H).

### Synthesis of I-D5

To a mixture of bromo(ethyl)triphenylphosphorane (14.5 g, 39.3 mmol) in THF (80 mL) was added t-BuOK (4.40 g, 39.3 mmol) at 20°C. The mixture was stirred at 50°C for 30 min and then **I-D4** (2 g, 6.56 mmol) in THF (20 mL) was added dropwise at 30°C. The reaction mixture was stirred at 40°C under N₂ for 16 hours. The mixture was cooled to 0°C and quenched with H₂O (200 mL). The mixture was extracted with EtOAc (3 x 100 mL). The combined organic phase was washed with brine (200 mL), dried over Na₂SO₄, filtered and evaporated to give product as an oil. The product was purified by combi flash (0-5% of EtOAc in PE) to give **I-D5** (1.8 g, 87%) as an oil.

**¹H NMR** (400 MHz, CDCl₃) δH 5.19-5.11 (m, 1H), 2.52-2.48 (m, 1H), 2.25-2.10 (m, 1H), 2.01-1.68 (m, 9H), 1.65-1.58 (m, 3H), 1.53-1.28 (m, 8H), 1.28-1.23 (m, 6H), 1.19-1.06 (m, 2H), 1.05-1.04 (m, 1H), 1.03-0.94 (m, 2H), 0.92-0.91 (m, 2H).

### Synthesis of I-D6

To a solution of **I-D5** (1.4 g, 4.42 mmol) in THF (10 mL) was added BH₃.Me₂S (2.21 mL, 22.1 mmol, 10 M) dropwise at 0°C. The reaction mixture was stirred at 15°C for 3 hours. The reaction mixture was cooled to 0°C. Ethanol (2.03 g, 44.2 mmol) was dropwise at 0°C. NaOH aqueous (8.84 mL, 44.2 mmol, 5 M) was added dropwise followed by hydrogen peroxide (4.42 mL, 44.2 mmol, 10.0 M) at 0°C. The suspension was stirred at 70°C for 1 hour. The mixture was extracted with ethyl acetate (3 x 100 mL). The combined organic phase was washed with saturated Na₂S₂O₃ aqueous (2 x 50 mL), brine (50 mL), dried over Na₂SO₄, filtered and evaporated to give **I-D6** (1.5 g) as a solid.

**¹H NMR** (400 MHz, CDCl₃) δH 1.93-1.63 (m, 12H), 1.31-1.29 (m, 2H), 1.28-1.24 (m, 11H), 1.17-1.10 (m, 4H), 0.99-0.89 (m, 8H), 0.79-0.77 (m, 1 H).

### Synthesis of I-D7 and I-D7a

To a solution of **I-D6** (1.5 g, 4.48 mmol) in DCM (20 mL) was added silica gel (5 g) followed by PCC (1.92 g, 8.96 mmol) at 20°C. The reaction was stirred at 20°C for 1 hour. The mixture was filtered and the filter cake was washed with DCM (3 x 50 mL). The combined filtrate was evaporated to give a residue, which was purified by flash column (EtOAc in PE = 0-10%) to give **I-D7** (130 mg, 8%), **I-D7a** (200 mg), and **I-D7a** (300 mg, 20%) as a solid.

The **I-D7a** (200 mg) was re-purified by flash column (EtOAc in PE = 0-5%) to give **I-D7** (51 mg, 25% recovered) and **I-D7a** (50 mg) as an oil.

### I-D7:

**¹H NMR** (400 MHz, CDCl₃) δH 2.30 (dd, *J=* 12.8, 3.2 Hz, 1H), 2.14 (s, 3H), 1.90-1.64 (m, 8H), 1.52-1.49 (m, 2H), 1.43-1.29 (m, 8H), 1.27-1.25 (m, 4H), 0.99-0.96 (m, 4H), 0.92 (s, 3H), 0.91-0.82 (m, 3H).

**LC-ELSD/MS** Rt = 1.063 min in 2 min chromatography, 30-90AB_2MIN_E, purity 99%, **MS ESI** calcd. for C₂₂H₃₅O[M+H-2H₂O]⁺ 315, found 315.

### I-D7a:

**¹H NMR** (400 MHz, CDCl₃) δH 2.47 (d, *J =* 5.6 Hz, 1H), 2.13 (s, 3H), 1.89-1.59 (m, 10H), 1.54-1.26 (m, 12H), 1.24 (s, 3H), 1.11-0.96 (m, 2H), 0.92 (s, 3H), 0.92-0.83 (m, 2H). **LC-ELSD/MS** Rt = 1.145 min in 2 min chromatography, 30-90AB_2MIN_E, purity 99%, **MS ESI** calcd. for C₂₂H₃₅O[M+H-2H₂O]⁺ 315, found 315.

### Example I-19: Synthesis of 1-(2-((1S,4aS,4bR,6aR,8R,10aS,10bR,12aS)-8-hydroxy-8,12a-dimethyloctadecahydrochrysen-1-yl)-2-oxoethyl)-1H-pyrazole-4-carbonitrile (I-D9)

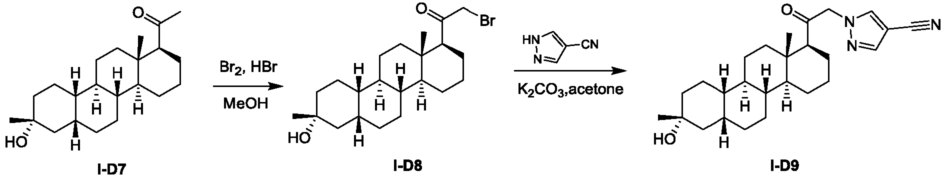

### Synthesis of I-D8

To a solution of **I-D7** (160 mg, 0.481 mmol) in MeOH (10 mL) was added one drop of HBr (7.79 mg, 0.0962 mmol) and Br₂ (84.6 mg, 0.529 mmol). The final reaction mixture was stirred at 20°C for 1 h. The mixture was quenched with 50% NaHCO₃ (50 mL) and extracted with EtOAc (2 x 30 mL). The combined organic layer was washed with brine (50 mL), dried over Na₂SO₄, filtered, concentrated to give **I-D8** (1.4 g, 95%) as a solid.

**¹H NMR** (400 MHz, CDCl₃) δH 4.01-3.87 (m, 2H), 2.58 (dd, *J=* 12.4, 3.0 Hz, 1H), 1.92-1.62 (m, 10H), 1.53-1.30 (m, 13H), 1.26 (s, 3H), 0.94 (s, 3H), 0.93-0.87 (m, 3H).

### Synthesis of I-D9

To a solution of **I-D8** (170 mg, 0.413 mmol) in acetone (10 mL) was added 1H-pyrazole-4-carbonitrile (57.6 mg, 0.619 mmol) followed by K₂CO₃ (85.4 mg, 0.619 mmol). The reaction mixture was stirred for 2 hours. The mixture was quenched with 50% NaHCO₃ (50 mL) and extracted with EtOAc (3 x 30 mL). The combined organic layer was washed with brine (50 mL), dried over Na₂SO₄, filtered, concentrated to give **I-D9,** which was purified by flash column (EtOAc/PE=0-20%) to give **I-D9** (80 mg) as a solid. The solid was triturated with n-hexane to give **I-D9** (57 mg, 0.134 mmol, 71.6%) as a solid.

**¹H NMR** (400 MHz, CDCl₃) δH 7.82 (d, *J =* 4.4 Hz, 2H), 5.08-4.91 (m, 3H), 2.32 (dd, *J =* 12.4, 3.6 Hz, 2H), 1.92-1.56 (m, 12H), 1.45-1.32 (m, 7H), 1.27 (s, 3H), 1.02-0.96 (m, 3H), 0.96 (s, 3H), 0.95-0.93 (m, 2H).

**LC-ELSD/MS** Rt = 1.051 min in 2 min chromatography, 30-90AB_2MIN_E, purity 99%, **MS ESI** calcd. for C₂₆H₃₆N₃O[M+H-H₂O]⁺ 406, found 406.

### Example I-20 and I-20a: Synthesis of 1-((1S,4aS,4bR,6aR,8R,10aS,10bS,12aS)-8-hydroxy-8,10a,12a-trimethyloctadecahydrochrysen-1-yl)-2-(5-methyl-2H-tetrazol-2-yl)ethan-1-one (I-20), and 1-((1S,4aS,4bR,6aR,8R,10aS,10bS,12aS)-8-hydroxy-8,10a,12a-trimethyloctadecahydrochrysen-1-yl)-2-(5-methyl-1H-tetrazol-1-yl)ethan-1-one (I-20a)

The following examples were made from **I-D8** with 5-methyl-2H-1, 2, 3, 4-tetrazole instead of 1H-pyrazole-4-carbonitrile yielding a mixture of regioisomers.

| **Example** | **ArNH** | **Compound Name** | **¹H NMR (400 MHz, CDCl₃)** | **MS ESI** |
|---|---|---|---|---|
| **I-20** | 5-methyl -2H-1, 2, 3, 4-tetrazole | 1-((1S,4aS,4bR,6aR,8R,10aS ,10bS,12aS)-8-hydroxy-8,10a,12a-trimethyloctadecahydrochr ysen-1-yl)-2-(5-methyl-2H-tetrazol-2-yl)ethan-1-one (**I-20**) | δ_{H} 5.38 (s, 2H), 2.56 (s, 3H), 2.33 (dd, *J =* 2.8, 12.8 Hz, 1H), 1.90-1.47 (m, 15H), 1.39-1.33 (m, 5H), 1.26-1.18 (m, 8H), 0.95 (s, 3H), 0.93 (s, 3H). | calcd. for C₂₅H₄₁N₄O ₂ [M+H]⁺ 429, found 429. |
| **I-20a** | 5-methyl -2H-1, 2, 3, 4-tetrazole | 1-((1S,4aS,4bR,6aR,8R,10aS ,10bS,12aS)-8-hydroxy-8,10a,12a-trimethyloctadecahydrochr ysen-1-yl)-2-(5-methyl-1H-tetrazol-1-yl)ethan-1-one (**I-20a**) | δ_{H} 5.13 (dd, *J =* 18.4, 27.2 Hz, 2H), 2.44 (s, 3H), 2.39 (dd, *J =* 3.6, 12.8 Hz, 1H), 1.92-1.60 (m, 12H), 1.43-1.21 (m, 16H), 0.95 (s, 3H), 0.94 (s, 3H). | calcd. for C₂₅H₄₁N₄O ₂ [M+H]⁺ 429, found 429 |

### Example I-21: Synthesis of 1-((1S,4aS,4bR,6aS,8R,10aS,10bR,12aS)-8-hydroxy-8-(methoxymethyl)-12a-methyloctadecahydrochrysen-1-yl)ethan-1-one (I-G14)

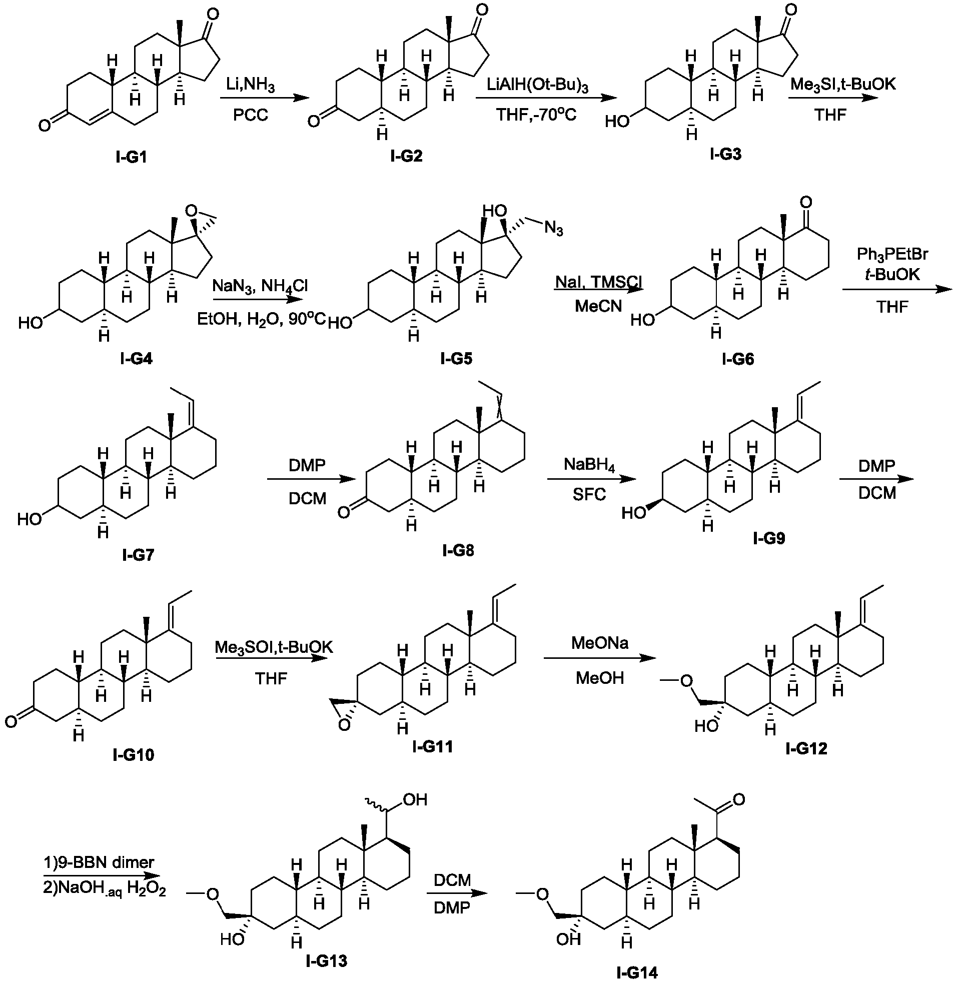

### Synthesis of I-G2

Lithium (12.7 g, 1.83 mol) was added to fresh prepared liquid ammonia (1.5 L) in portions at -70°C. After stirring at -70°C for 1 h, a solution of **I-G1** (50 g, 183 mmol) in dry THF (500 mL) and a solution of t-butanol (27.1 g, 366 mmol) in dry THF (100 mL) were added to this mixture under strong stir in turns. The temperature maintained below -60°C. The resultant mixture was stirred at -70°C for 1 h. Ammonium chloride (150 g) was added to reaction mixture; the mixture was warmed to room temperature and stirred for 16 h. The reaction mixture was neutralized with HCl (2.5 M, 1500 mL), extracted with EtOAc (3 x 1 L), washed with brine (1 L), dried over anhydrous sodium sulfate, filtered and concentrated to give a solid which was used directly without further purification. To a solution of the solid (150 g, 538 mmol) in DCM (2 L) was added PCC (230 g, 1.07 mol) and silica gel (230 g) at 25°C. The solution was stirred at 25°C for 3 h. The mixture was added PE (2 L) then filtered and the residue was washed with anhydrous DCM (2 x 1 L) and PE (2 x 1 L). The combined filtrate was concentrated in vacuum to give **I-G2** (90 g) as a solid.

**¹H NMR** (400 MHz, CDCl₃) δH 2.50-2.30 (m, 3H), 2.28-2.20 (m, 3H), 2.12-2.00 (m, 3H), 1.98-1.25 (m, 6H), 1.30-1.12 (m, 7H), 0.90 (s, 3H), 0.88-0.67 (m, 1H).

### Synthesis of I-G3

To a solution of **I-G2** (60 g, 218 mmol) in anhydrous THF (300 mL) was added a solution of LiAlH(Ot-Bu)₃ (80.3 g, 316 mmol) in anhydrous THF (300 mL) drop-wise at -70°C over a period of 30 min under N₂, during which the temperature was maintained below -60°C. The reaction mixture was stirred for 30 min at -70°C, poured into saturated NH₄Cl (1 L) at 0°C and stirred for 30 min. The aqueous phase was extracted with EtOAc (2 x 600 mL). The combined organic phase was washed with saturated brine (2 x 500 mL), dried over anhydrous Na₂SO₄, filtered and concentrated in vacuum to give a solid. The residue was purified by silica gel chromatography (PE/EtOAc = 8/1 to 5/1) to afford **I-G3** (51 g) as a solid.

**¹H NMR** (400 MHz, CDCl₃) δ 4.10-3.55 (m, 1H), 2.45-2.30 (m, 1H), 2.00-1.30 (m, 12H), 1.25-1.10 (m, 8H), 0.89-0.86 (m, 3H), 0.85-0.80 (m, 3H).

### Synthesis of I-G4

To a suspension of Me₃SI (75.0 g, 368 mmol) in anhydrous THF (400 mL) was added t-BuOK (41.2 g, 368 mmol) at 25°C under N₂. After stirring for 30 min at 25°C, a solution of **I-G3** (51 g) in anhydrous THF (400 mL) was added. The reaction mixture was warmed to 40°C and stirred for another 1 h. The mixture poured into ice-water (1.5 L) at 0°C. The aqueous phase was extracted with EtOAc (2 x 700 mL). The combined organic phase was washed with saturated brine (2 x 500 mL), dried over anhydrous Na₂SO₄, filtered and concentrated. The residue was triturated form PE (600 mL) at 25°C to give **I-G4** (30 g, 56%) as a solid.

**¹H NMR** (400 MHz, CDCl₃) δH 4.15-3.50 (m, 1H), 2.91-2.85 (m, 1H), 2.61-2.58 (m, 1H), 2.01-1.83 (m, 3H), 1.78-1.43 (m, 15H), 1.25-1.05 (m, 6H), 0.90-0.86 (m, 3H).

### Synthesis of I-G5

A solution of **I-G4** (30 g, 103 mmol), sodium azide (26.7 g, 412 mmol), and ammonium chloride (24.7 g, 463 mmol) in ethanol (700 mL) and water (140 mL) was heated at 90°C for 6 h. The reaction mixture was cooled to 25°C and treated with water (1 L). The precipitate was filtered and washed with water (3 x 500 mL) to get a solid. The residue was dissolved in EtOAc (2 x 500 mL), washed with saturated aqueous brine (2 x 500 mL), filtered and concentrated to give **I-G5** (36 g) as a solid.

**¹H NMR** (400 MHz, CDCl₃) δH 3.60-3.50 (m, 2H), 3.25-3.20 (m, 1H), 2.00-1.75 (m, 8H), 1.69-1.50 (m, 7H), 1.25-1.00 (m, 7H), 0.89-0.86 (m, 3H), 0.75-0.65 (m, 3H).

### Synthesis of I-G6

To a solution of **I-G5** (36 g) in anhydrous MeCN (700 mL) was added NaI (160 g, 1.07 mol) and TMSCl (116 g, 1.07 mol) drop-wise at 0°C under N₂. After stirring for 30 mins, the reaction mixture was warmed to 40°C and stirred for another 12 h. The mixture was poured into ice-water (1 L) and was added saturated aqueous Na₂S₂O₃ (1 L). The aqueous phase was extracted with EtOAc (2 x 600 mL). The combine organic phase was washed with saturated aqueous brine (2 x 500 mL), filtered and concentrated in vacuum to give **I-G6** (36 g) as a solid.

**¹H NMR** (400 MHz, CDCl₃) δH 4.12-4.02 (m, 1H), 3.65-3.50 (m, 1H), 2.60-2.50 (m, 1H), 2.12-1.50 (m, 11H), 1.48-1.10 (m, 10H),1.07-1.05 (m, 4H), 0.13-0.12 (m, 2H).

### Synthesis of I-G7

To a suspension of Ph₃PEtBr (91.3 g, 246 mmol) in anhydrous THF (400 mL) was added t-BuOK (27.6 g, 246 mmol) at 0°C under. The reaction mixture was stirred 30 min. Then a solution of **I-G6** (36 g, 123 mmol) in anhydrous THF (400 mL) was drop-wise. The reaction mixture was warmed to 50°C and stirred for 1 h. The mixture was cooled and poured into ice-water (800 mL) stirred for 10 min. The aqueous phase was extracted with EtOAc (2 x 500 mL). The combine organic phase was washed with saturated brine (2 x 500 mL).filtered and concentrated. The residue was triturated from MeOH/H₂0 (1/1, 400/400 mL) at 25°C to give **I-G7** (20 g) as a soild. The G7 (20 g) was purified by flash column (0~30% EtOAc in PE) to give **I-G7** (15 g) as a solid.

**¹H NMR** (400 MHz, CDCl₃) δH 5.28-5.10 (m, 1H), 3.60-3.50 (m, 1H), 2.25-2.12 (m, 1H), 2.00-1.61 (m, 17H), 1.25-1.00 (m, 14H).

### Synthesis of I-G8

To a solution of **I-G7** (15 g, 49.5 mmol) in DCM (300 ml) was added DMP (41.9 g, 99.0 mmol) at 25°C. The reaction mixture warmed to 40°C and stirred for 30 mins, treated with water (1 mL) and stirred for another 30 mins. The reaction mixture was quenched with saturated NaHCO₃, aqueous pH 7~8 at below 10°C. The Suspension was filtered. The DCM phase in filtrate was separated and washed with saturated NaHCO₃/Na₂S₂O₃ aqueous (1:1, 2 x 300 mL). The organic phase was washed with saturated brine (2 x 300 mL). dried over anhydrous Na₂SO₄, filtered and concentrated. The residue was purified by flash column (0~30% of EtOAc in PE) to give **I-G8** (10 g, 68%) as an oil.

**¹H NMR** (400 MHz, CDCl₃) δH 5.20-5.13 (m, 1H), 2.52-2.49 (m, 1H), 2.25-2.16 (m, 7H), 1.75-1.50 (m, 10H), 1.48-1.25 (m, 2H), 1.25-0.95 (m, 10H), 0.93-0.91 (m, 1H).

### Synthesis of I-G9

To a solution of **I-G8** (10 g, 33.2 mmol) in MeOH (50 mL) was slowed added NaBH₄ (2.52 g, 66.4 mmol) at 0°C. The mixture was stirred 30 min. To the reaction mixture was added water (200 mL). The aqueous phase was extracted with DCM (2 x 100 mL). The combined organic phase was washed with saturated brine (2 x 100 mL), dried over anhydrous Na₂SO₄, filtered and concentrated. The residue was triturated with MeCN (50 mL) at reflux for 1 h. The mixture stirred was cool 25°C. The suspension was filtration in vacuum to get 3 g of a solid, which was purified by SFC (Column:AD(250mm*50mm,10um), Condition:0.1%NH₃H₂O ETOH, Begin B:25%) to afford **I-G9** (PK2: 1 g, 33%) as a solid and **I-G9a** (PK1: 1 g, 33%) as a solid.

The structures of **I-G9** and **I-G9a** were randomly assigned.

**¹H NMR** (400 MHz, CDCl₃) δH 5.16-5.13 (m, 1H), 3.60-3.55 (m, 1H), 2.20-2.09 (m, 2H), 2.05-1.97 (m, 3H), 1.95-1.32 (m, 11H), 1.22-0.90 (m, 12H), 0.88-0.78 (m, 2H), 0.68-0.54 (m, 2H).

### Synthesis of I-G10

To a solution of **I-G9** (1 g, 3.30 mmol) in DCM (20 ml) was added DMP (2.79 g, 6.60 mmol) at 25°C. The reaction mixture warmed to 40°C and stirred for 30 mins. To the mixture was added one drop of water and stirred for another 30 mins. The reaction mixture was quenched with saturated NaHCO₃, aqueous pH 7~8 at below 10°C. The suspension was filtered. The DCM phase in filtrate was separated and washed with saturated NaHCO₃/Na₂S₂O₃ aqueous (1/1, 2 x 10 mL). The organic phase was washed with saturated brine (2 x 30 mL). dried over anhydrous Na2SO4, filtered and concentrated in vacuum to give **I-G10** (0.9 g, 91%) as a solid.

**¹H NMR** (400 MHz, CDCl₃) δ_{H} 5.16-5.14 (m, 1H), 2.48-2.43 (m, 1H), 2.35-2.12 (m, 8H), 1.72-1.57 (m, 7H), 1.45-1.25 (m, 2H), 1.21-1.05 (m, 10H), 0.80-0.75 (m, 3H).

### Synthesis of I-G11

To a suspension of Me₃SOI (1.31 g, 5.98 mmol) in DMSO (10 mL) was added t-BuOK (671 mg, 5.98 mmol). After stirring at 25°C for 30 min, a solution of **I-G10** (0.9 g) in DMSO/THF (10/10 mL) was added. The reaction mixture and stirred at 50°C for 1 hr and treated with NH₄Cl .aq (50 mL), extracted with EtOAc (2 x 50 mL). The combined organic phase was washed with saturated brine (2 x 30 mL), dried over anhydrous Na₂SO₄, filtered and concentrated in vacuum to give **I-G11** (1 g) as an oil.

**¹H NMR** (400 MHz, CDCl₃) δH 5.16-5.12 (m, 1H), 2.67-2.60 (m, 1H), 2.23-2.10 (m, 2H), 2.05-1.89 (m, 3H), 1.75-1.50 (m, 7H), 1.32-1.10 (m, 16H), 0.88-0.65 (m, 4H).

### Synthesis of I-G12

To a solution of **I-G11** (1 g) in MeOH (20 mL) was added MeONa (3.42 g, 63.4 mmol). After stirring at 25°C for 10 mins under N₂, the mixture was warmed to 60°C and stirred for 3 h. After cooling, the mixture was treated water (50 mL), extracted with EtOAc (2 x 50 mL). The combined organic phase was washed with saturated brine (2 x 50 mL), drive over anhydrous Na₂SO₄, filtered and concentrated. The residue was purified by flash column (0~10% of EtOAc in PE) to give **I-G12** (0.6 g, 55%) as an oil.

**¹H NMR** (400 MHz, CDCl₃) δH 5.15-5.12 (m, 1H), 3.38 (s, 3H), 3.17 (s, 2H), 2.25-2.10 (m, 2H), 2.08-1.93 (m, 3H), 1.89-1.50 (m, 10H), 1.25-0.80 (m, 17H).

### Synthesis of I-G13

To a solution of **I-G12** (0.6 g, 1.73 mmol) in anhydrous THF (10 mL) was added 9-BNN dimer (1.04 g, 4.32 mmol) and stirred at 0°C for 30 min under N₂. After stirring at 50°C for 1 h, the reaction mixture was cooled and quenched with EtOH (10 mL) at 0°C, followed by adding NaOH (3.46 mL, 5M, 17.3 mmol) very slowly. After addition, H₂O₂ (1.96 g, 17.3 mmol, 30% in water) was added slowly until the inner temperature no longer rises and the inner temperature was maintained below 30°C. The mixture was stirred at 50°C for another 1 h. The aqueous phase was extracted with DCM (3 x 50 mL). The combine organic phase was washed with saturated Na₂S₂O₃ (2 x 30 mL), brine (2 x 30 mL), drive over anhydrous Na₂SO₄, filtered and concentrated in vacuum to give **I-G13** (1 g) as an oil, which was used directly for the next step.

**¹H NMR** (400 MHz, CDCl₃) δH 4.23-4.21 (m, 1H), 3.38 (s, 3H), 3.18 (s, 2H), 1.99 (s, 1H), 1.89-1.75 (m, 8H), 1.50-1.12 (m, 10H), 1.10-0.75 (m, 15H).

### Synthesis of I-G14

To a solution of **I-G13** (1 g, 2.74 mmol) in DCM (10 ml) was added DMP (1.74 g, 4.11 mmol) at 25°C. The reaction mixture warmed to 40°C and stirred for 30 mins, then treated with one drop water and stirred for another 30 mins. The reaction mixture was quenched with saturated NaHCO₃, aqueous pH 7~8 at below 10°C. The suspension was filtered. The DCM phase in filtrate was separated and washed with saturated NaHCO₃/Na₂S₂O₃ aqueous (1/1, 2 x 10 mL). The organic phase was washed with saturated brine (2 x 30 mL), dried over anhydrous Na₂SO₄, filtered and concentrated in vacuum to give **I-G14** (0.25 g, 25%) as a solid.

**¹H NMR** (400 MHz, CDCl₃) δH 3.38 (s, 3H), 3.17 (s, 2H), 2.31-2.27 (m, 1H), 2.13 (s, 3H), 2.20 (s, 1H), 1.85-1.50 (m, 12H), 1.48-1.10 (m, 12H), 0.91-0.56 (m, 4H).

### Example I-22 and 1-22a: Synthesis of 1-((1S,4aS,4bR,6aS,8R,10aS,10bR,12aS)-8-hydroxy-8-(methoxymethyl)-12a-methyloctadecahydrochrysen-1-yl)-2-(5-methyl-2H-tetrazol-2-yl)ethan-1-one (I-G16) and 1-((1S,4aS,4bR,6aS,8R,10aS,10bR,12aS)-8-hydroxy-8-(methoxymethyl)-12a-methyloctadecahydrochrysen-1-yl)-2-(5-methyl-1H-tetrazol-1-yl)ethan-1-one (I-G16a)

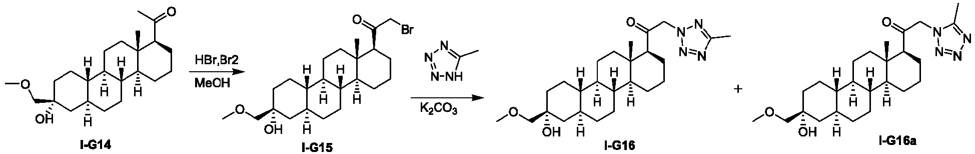

### Synthesis of I-G15

To a solution of **I-G14** (0.2 g, 0.551 mmol) in methanol (10 mL) was added HBr (40%, 111 g, 0.551 mmol) and Br₂ (96.8 mg, 0.606 mmol) drop wise at 25°C. The reaction mixture was warmed to 40°C and stirred for 1 h. The reaction mixture was poured into water (30 mL) and stirred for another 30 min. The precipitate was collected by filtration, washed with saturated NaHCO₃.aq/Na₂S₂O₃.aq (3 x 30/30 mL) and dried in the air to give **I-G15** (0.3 g) as a solid. **¹H NMR** (400 MHz, CDCl₃) δH 3.98-3.91 (m, 2H), 3.38 (s, 3H), 3.17 (s, 2H), 2.57-2.55 (m, 1H), 2.10-1.98 (m, 2H), 1.88-1.50 (m, 7H), 1.48-1.10 (m, 8H), 1.08-0.68 (m, 11H), 0.68-0.58 (m, 1H).

### Synthesis of I-G16 and I-G16a

To a solution of **I-G15** (0.3 g) in anhydrous THF (10 mL) was added 5-methyl-1H-tetrazole (113 mg, 1.35 mmol) and K₂CO₃ (186 mg, 1.35 mmol) at 25°C under N₂. After stirring at 50°C for 12 h, the reaction mixture was cooled, treated with water (20 mL) and stirred for 30 min. The aqueous phases was extracted with EtOAc (2 x 50 mL). The combined organic phase was washed with saturated brine (2 x 20 mL), dried over anhydrous Na₂SO₄, filtered and concentrated. The residue was purified by flash column (0~50% of EtOAc in PE) to give **I-G16** (12 mg, 4%) as a solid and **I-G16a** (6 mg, 2%) as a solid.

### I-G16:

**¹H NMR** (400 MHz, CDCl₃) δH 5.40-5.36 (m, 2H), 3.38 (s, 3H), 3.18 (s, 2H), 2.55 (s, 3H), 2.30-2.25 (m, 1H), 2.10-1.98 (m, 1H), 1.88-1.60 (m, 8H), 1.48-1.25 (m, 5H), 1.21-0.75 (m, 15H).

HPLC Rt = 4.57 min in 10 min chromatography, 30-90_AB_1.2ml_E, purity 97%, **MS ESI** calcd. for C₂₅H₄₁N₄O₃ [M+H]⁺ 445, found 445.

### I-G16a:

**¹H NMR** (400 MHz, CDCl₃) δH 5.15-5.12 (m, 2H), 3.38 (s, 3H), 3.18 (s, 2H), 2.43-2.40 (m, 4H), 2.02 (s, 1H), 1.89-65 (m, 7H), 1.64-1.1.50 (m, 3H), 1.48-0.79 (m, 17H), 0.75-0.70 (m, 1H).

**LC-ELSD/MS** Rt = 0.991 min in 2 min chromatography, 30-90AB_2MIN_E, purity 99%, **MS ESI** calcd. for C₂₅H₄₁N₄O₃ [M+H]⁺ 445, found 445.

### Example I-23 and I-23a: Synthesis of (2R,4aS,4bR,6aS,7S,10aS,10bR,12aS)-7-((4-fluorophenyl)amino)-2-(methoxymethyl)-6a-methyloctadecahydrochrysen-2-ol (I-23) and (2R,4aS,4bR,6aS,7R,10aS,10bR,12aS)-7-((4-fluorophenyl)amino)-2-(methoxymethyl)-6a-methyloctadecahydrochrysen-2-ol (I-23a)

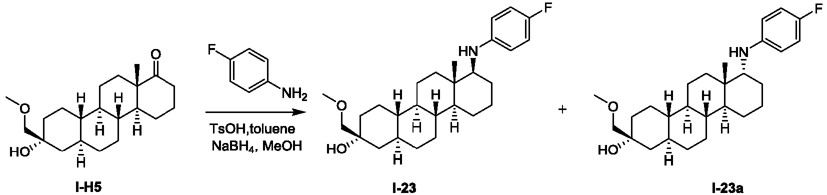

The experimental of intermediate **I-H5** could be found in **Example I-28** herein. To a solution of **I**-H5 (200 mg, 0.597 mmol) in toluene (20 mL) was added 4-fluoroaniline (132 mg, 1.19 mmol) and 4-methylbenzenesulfonic acid (10.2 mg, 0.0597 mmol) at 25°C under N₂. The mixture was refluxed at 130°C for 6 hrs. After cooling, the reaction mixture was diluted with MeOH (20 mL) and treated with NaBH₄ (225 mg, 5.96 mmol) in one portion at 0°C. After the addition, the mixture was stirred at 25°C for 1 h. The mixture was quenched with saturated NH₄Cl (20 mL). The mixture was extracted with EtOAc (2 x 20 mL). The combined organic phase was washed with brine (2 x 20 mL), dried over Na₂SO₄, filtered, concentrated to give (220 mg) as a solid, which was purified by HPLC (Column: Waters Xbridge 150*25 5u; Condition: water (10mM NH₄HCO₃)-ACN; Gradient 76%-96%B; Gradient Time (min):6) to afford **I-23** (152 mg, 60%) and **I-23a** (16 mg, 6%)as a solid.

### I-23:

**¹H NMR** (400 MHz, CDCl₃) δH 6.91-6.78 (m, 2H), 6.53-6.48 (m, 2H), 3.38 (s, 3H), 3.18 (s, 2H), 2.90-2.84 (m, 1H), 2.01-1.51 (m, 13H), 1.40-0.90 (m, 12H), 0.94-0.76 (m, 5H).

**LC-ELSD/MS** Rt = 1.024 min in 2 min chromatography, 30-90AB_2MIN_E.M, purity 99%, **MS ESI** calcd. for C₂₇H₄₁FNO₂ [M+H]⁺ 430, found 430.

(Mobile Phase: 1.5mL/4LTFA in water (solvent A) and 0.75mL/4LTFA in acetonitrile (solvent B), using the elution gradient 30%-90% (solvent B) over 0.9 minutes and holding at 90% for 0.6 minutes at a flow rate of 1.2 mL/min; Column: Xtimate C18 2.1*30mm, 3um; Wavelength: UV 220 nm; Column temperature: 50°C; MS ionization: ESI; Detector: PDA&ELSD).

### I-23a:

**¹H NMR** (400 MHz, CDCl₃) δH 6.91-6.78 (m, 2H), 6.53-6.48 (m, 2H), 3.38 (s, 3H), 3.17 (s, 2H), 3.04 (s, 1H), 2.01-1.51 (m, 10H), 1.40-0.90 (m, 13H), 0.89-0.76 (m, 7H).

**LC-ELSD/MS** Rt = 1.179 min in 2 min chromatography, 30-90AB_2MIN_E.M, purity 99%, **MS ESI** calcd. for C₂₇H₄₁FNO₂ [M+H]⁺ 430, found 430.

(Mobile Phase: 1.5mL/4LTFA in water (solvent A) and 0.75mL/4LTFA in acetonitrile (solvent B), using the elution gradient 30%-90% (solvent B) over 0.9 minutes and holding at 90% for 0.6 minutes at a flow rate of 1.2 mL/min; Column: Xtimate C18 2.1*30mm, 3um; Wavelength: UV 220 nm; Column temperature: 50°C; MS ionization: ESI; Detector: PDA&ELSD).

### Example I-24: Synthesis of (1S,4aS,4bR,6aS,8R,10aS,10bR,12aS)-N,N-diethyl-8-hydroxy-8-(methoxymethyl)-12a-methyloctadecahydrochrysene-1-carboxamide (I-M3)

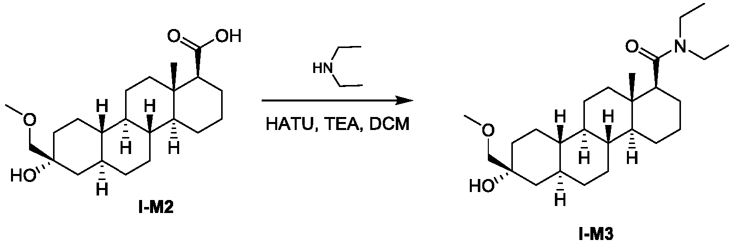

The experimental of intermediate **I-M2** could be found in **Example I-17.** To a solution of **I**-M2 (300 mg, 0.82 mmol) in DCM (4 mL) was added HATU (623 mg, 1.64 mmol) and Et₃N (414 mg, 4.1 mmol) at 25°C. The reaction mixture was stirred at 25°C for 0.5 hour. Diethylamine (119 mg, 1.64 mmol) was added to the reaction mixture at 25°C. The reaction mixture was stirred at 25°C for 10 hours. The mixture was treated by water (10 mL) and extracted with EtOAc (2 x 10 mL). The combined organic phase was concentrated under vacuum. The residual was resolved in EtOAc and washed with water (2 x 10 mL), brine (10 mL), dried over anhydrous Na₂SO₄, filtered and concentrated to give **I-M3** (300 mg) as a solid. The product was purified by HPLC (column: Xtimate C18 150*25mm*5um), condition: water (0.225%FA)-ACN, gradient: 50-80% B, Gradient Time: 11.3 mins, 100%B Hold Time: 2 min, flow rate: 30 mL/min) to give **I-M3** (30 mg, 10%) as a solid.

**¹H NMR** (400 MHz, CDCl₃) δH 3.67-3.52 (m, 2H), 3.38 (s, 3H), 3.23-3.06 (m, 4H), 2.32-2.23 (m, 1H), 2.05-1.59 (m, 12H), 1.43-1.08 (m, 12H), 1.07 (s, 3H), 1.04-0.91 (m, 4H), 0.87-0.58 (m, 4H).

**LC-ELSD/MS** Rt = 1.037 min in 2 min chromatography, 30-90AB_2MIN_E.M, purity 99%, **MS ESI** calcd. for C₂₆H₄₆NO₃ [M+H]⁺ 420, found 420.

(Mobile Phase: 1.5ML/4LTFA in water (solvent A) and 0.75ML/4LTFA in acetonitrile (solvent B), using the elution gradient 30%-90% (solvent B) over 0.9 minutes and holding at 90% for 0.6 minutes at a flow rate of 1.2 ml/min; Column: Xtimate C18 2.1*30mm, 3um; Wavelength: UV 220 nm; Column temperature: 50°C; MS ionization: ESI; Detector: PDA&ELSD).

### Example I-25- I-27: Synthesis of (1S,4aS,4bR,6aS,8R,10aS,10bR,12aS)-N-benzyl-8-hydroxy-8-(methoxymethyl)-12a-methyloctadecahydrochrysene-1-carboxamide (I-25), (1S,4aS,4bR,6aS,8R,10aS,10bR,12aS)-8-hydroxy-8-(methoxymethyl)-12a-methyl-N-(pyridin-2-ylmethyl)octadecahydrochrysene-1-carboxamide (I-26), and (1S,4aS,4bR,6aS,8R,10aS,10bR,12aS)-8-hydroxy-8-(methoxymethyl)-12a-methyl-N-phenyloctadecahydrochrysene-1-carboxamide (I-27)

The following examples were made from **I-M2** with the listed amines replacing used above.

| **Example** | **amine** | **Compound Name** | **¹H NMR (400 MHz, CDCl3)** | **MS ESI** |
|---|---|---|---|---|
| **I-25** | Benzylamine | (1S,4aS,4bR,6aS,8R, 10aS,10bR,12aS)-N-benzyl-8-hydroxy-8-(methoxymethyl)-12a-methyloctadecahydro chrysene-1-carboxamide (**I-25**) | δ_{H} 7.36-7.30 (m, 2H), 7.29-7.27 (m, 3H), 5.64-5.56 (m, 1H), 4.48-4.35 (m, 2H), 3.38 (s, 3H), 3.17 (s, 2H), 2.01 (brs, 1H), 1.93-1.59 (m, 11H), 1.42-1.01 (m, 8H), 1.00 (s, 3H), 0.99-0.89 (m, 3H), 0.84-0.61 (m, 4H). | calcd. for C₂₉H₄₄NO₃ [M+H]⁺ 454, found 454 |
| **I-26** | 1-(pyridin-2-yl)methana mine | (1S,4aS,4bR,6aS,8R, 10aS,10bR, 12aS)-8-hydroxy-8-(methoxymethyl)-12a-methyl-N-(pyridin-2-ylmethyl)octadecahy drochrysene-1-carboxamide (**I-26**) | δ_{H} 8.57-8.51 (d, *J =* 4.4 Hz, 1H), 7.69-7.61 (m, 1H), 7.24 (s, 1H), 7.21-7.15 (m, 1H), 6.65-6.59 (m, 1H), 4.61-4.46 (m, 2H), 3.37 (s, 3H), 3.17 (s, 2H), 2.08-1.62 (m, 11H), 1.57-1.01 (m, 9H), 0.99 (s, 3H), 0.98-0.57 (m, 7H). | calcd. for C₂₈H₄₃N₂O ₃ [M+H]⁺ 455, found 455 |
| **I-27** | aniline | (1S,4aS,4bR,6aS,8R, 10aS,10bR,12aS)-8-hydroxy-8-(methoxymethyl)-12a-methyl-N-phenyloctadecahydro chrysene-1-carboxamide (**I-27**) | δ_{H} 7.53-7.48 (d, *J =* 8 Hz, 2H), 7.33-7.27 (t, *J=* 8 Hz, 2H), 7.12-7.03 (m, 2H), 3.38 (s, 3H), 3.18 (s, 2H), 2.06-1.62 (m, 12H), 1.44-1.04 (m, 8H), 1.02 (s, 3H), 1.01-0.58 (s, 7H). | calcd. for C₂₈H₄₂NO₃ [M+H]⁺ 440, found 440 |

### Example I-28: Synthesis of (4aS,4bR,6aS,8R,10aS,10bR,12aS)-8-hydroxy-8-(methoxymethyl)-12a-methylhexadecahydrochrysen-1(2H)-one (I-H5)

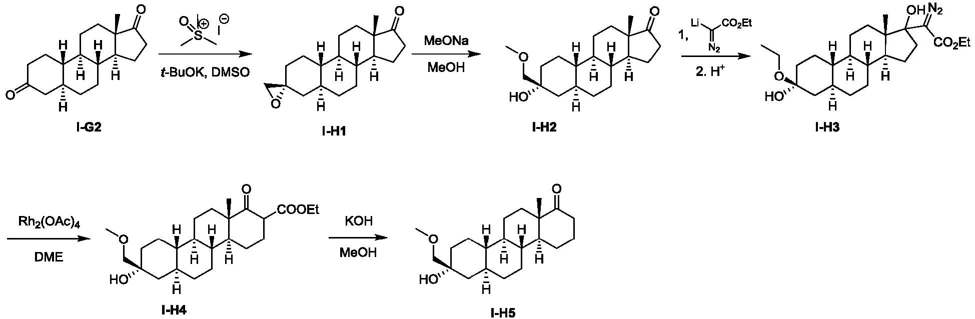

The synthesis of **I-G2** could be found in **Example I-21.**

### Synthesis of I-H1

A stirred solution of trimethylsulfoxonium iodide (26.1 g, 119 mmol) and t-BuOK (13.3 g, 119 mmol) in DMSO (300 mL) was heated at 40°C for 1.0 h under N₂. The reaction mixture was added to a solution of **I-G2** (30 g, 109 mmol) in THF (100 mL) and stirred at 40°C for 30 mins. The reaction mixture was combined with the other batches from 1 g and 10 g of G2 respectively. The reaction was treated with water (1000 mL). The mixture was extracted with EtOAc (2 x 500 mL). The combined organic phase was washed with water (2 x 300mL), brine (300 mL), dried over anhydrous Na₂SO₄, filtered and concentrated in vacuum to afford **I-H1** (40 g, 95%) as a solid.

**¹H NMR** (400 MHz, CDCl₃) δH 2.63 (s, 2H), 2.50-2.38 (m, 1H), 2.15-2.02 (m, 1H), 2.02-1.85 (m, 4H), 1.85-1.71 (m, 2H), 1.71-1.61 (m, 1H), 1.61-1.40 (m, 2H), 1.40-0.99 (m, 10H), 0.95-0.73 (m, 5H).

### Synthesis of I-H2

To a solution of **I-H1** (41 g, 142 mmol) in MeOH (500 mL) was added MeONa (38.3 g, 710 mmol) at 25°C under N₂. The mixture was stirred at 70°C at reflux for 16 h under N₂. The reaction was treated with water (1000 mL). The aqueous phase was extracted with EtOAc (2 x 500 mL). The combined organic phase was washed with saturated brine (300 mL), dried over anhydrous Na₂SO₄, filtered and concentrated in vacuum. The residue was purified by flash column (0~10% of EtOAc in PE) to give **I-H2** (30 g, 66%) as a solid.

**¹H NMR** (400 MHz, CDCl₃) δH 3.38 (s, 3H), 3.18 (s, 2H), 2.50-2.40 (m, 1H), 2.15-2.00 (m, 2H), 2.00-1.85 (m, 2H), 1.85-1.70 (m, 4H), 1.70-1.58 (m, 2H), 1.58-1.40 (m, 2H), 1.40-1.19 (m, 5H), 1.19-0.98 (m, 4H), 0.87 (s, 3H), 0.85-0.70 (m, 2H).

### Synthesis of I-H3

A cold (0°C) solution of lithium di-isopropylamide from addition of n-butyl-lithium in hexane (19.9 mL, 49.9 mmol, 2.5 M in hexane) to di-isopropylamine (7.87 mL, 56.1 mmol, 0.72 g/mL) in THF (20 mL) at -78°C was added over 30 min to a stirred solution of **I-H2** (5 g, 15.6 mmol) and ethyl diazoacetate (4.88 mL, 46.7 mmol, 1.09 g/mL) in THF (20 mL) at - 78°C. The mixture was stirred at -78°C for 2 hours after which acetic acid (3.19 mL, 56.1 mmol, 1.050 g/mL) in THF (10 mL) was added during 20 min. The mixture was then allowed to warm to 25°C and stirred 2 hours. Water (100 mL) was then added. The organic layer separated and the aqueous solution extracted with EtOAc (3 x 100 mL). The combined organic phase was washed with brine (2 x 50 mL), dried over Na₂SO₄, filtered, concentrated in vacuum. The residue was purified by flash column (0~10% of EtOAc in PE) to give **I-H3** (3 g, 44%) as a solid.

**¹H NMR** (400 MHz, CDCl₃) δH 4.71 (s, 1H), 4.30-4.15 (m, 2H), 3.38 (s, 3H), 3.17 (s, 2H), 2.20-2.10 (m, 1H), 2.10-1.95 (m, 1H), 1.95-1.55 (m, 2H), 1.55-1.20 (m, 11H), 1.20-0.82 (m, 13H), 0.80-0.60 (m, 2H).

### Synthesis of I-H4

To a solution of **I-H3** (3 g, 6.90 mmol) in DME (50 mL) was added Rhodium(II) acetate dimer (15.2 mg, 0.0345 mmol) in one portion at 25°C. The mixture was stirred at 25°C for 2 hours. The mixture was concentrated to give **I-H4** (2.7 g) as an oil which was used directly in the next step.

### Synthesis of I-H5

To a solution of **I-H4** (2.7 g, 6.64 mmol) in MeOH (20 mL) was added KOH (3.72 g, 66.4 mmol) in one portion at 25°C. The mixture was stirred at 65°C for 16 hours and treated with water (100 mL). The mixture was extracted with EtOAc (3 x 50 mL). The combined organic phase was washed with brine (30 mL), dried over Na₂SO₄, filtered, concentrated in vacuum. The residue was purified by flash column (0~10% of EtOAc in PE) to give **I-H5** (1.8 g, 81%) as a solid.

**¹H NMR** (400 MHz, CDCl₃) δH 3.37 (s, 3H), 3.17 (s, 2H), 2.68-2.52 (m, 1H), 2.25-2.15 (m, 1H), 2.10-2.00 (m, 2H), 1.95-1.65 (m, 7H), 1.65-1.30 (m, 5H), 1.30-0.85 (m, 11H), 0.80-0.55 (m, 2H).

**LC-ELSD/MS** Rt = 1.038 min in 2 min chromatography, 30-90AB_ELSD, purity 100.0%, **MS ESI** calcd. for C₂₁H₃₃O₂ [M+H-H2O]+ 317, found 317.

### Example I-34: 1-((1S,4aS,4bR,6aS,8R,10aS,10bS,12aS)-8-hydroxy-8,10a,12a-trimethyloctadecahydrochrysen-1-yl)ethan-1-one (I-K2)

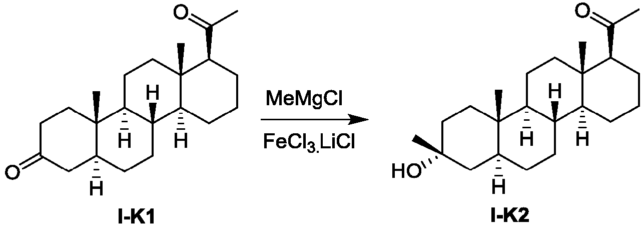

To anhydrous THF (3 mL) under nitrogen at 10°C was added anhydrous LiCl (21.5 mg, 0.5 mmol). The mixture was stirred at 10°C for 10 min, and anhydrous FeCl₃ (43.1 mg, 0.27 mmol) was added immediately. The resulting mixture was cooled to -30°C, and MeMgBr (3 M in THF, 0.322 mL, 0.968 mmol) was added dropwise maintaining the inner temperature below -15°C. The dark solution was stirred at -15°C for 10 min. A solution of **I-K1** (56722-72-6) (80 mg, 0.242 mmol) in anhydrous THF (2 mL) was added dropwise to the mixture. The resulting mixture was stirred at -15°C for 3 hrs. The reaction mixture was quenched with NH₄Cl (10 mL) and extracted with EtOAc (2 x 10 mL). The combined organic layer was dried over anhydrous sodium sulfate, filtered and concentrated to give **I-K2** (120 mg) as an oil The material was purified by silica gel chromatography (PE/EtOAc = 30/1 to 1/1) to afford **I-K2** (40 mg, 49%) as a solid.

**¹H NMR** (400 MHz, CDCl₃) δ_{H} 2.32-2.26 (m, 1H), 2.13 (s, 3H), 1.88-1.26(m, 20H),1.20 (s, 3H), 1.17-1.09(m, 1H) , 0.98-0.93 (m, 3H), 0.71(s, 3H); MS ESI calcd. for C₂₃H₃₆O [M-H₂O]⁺ 329, found 329.

### Example I-35: Synthesis of (4aS,4bR,6aR,8R,10aS,10bR,12aS)-8-hydroxy-8-(methoxymethyl)-12a-methylhexadecahydrochrysen-1(2H)-one (I-L6)

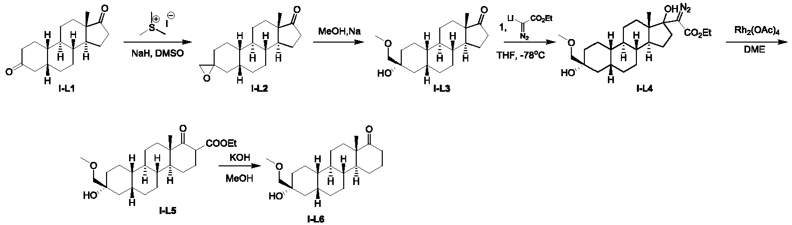

### Synthesis of I-L2

A stirred solution of iodotrimethyl-4-sulfane (27.1 g, 133.0 mmol) and NaH (5.31 g, 133.0 mmol, 60%) in DMSO (300 mL) was cooled at 0°C for 1 h under N₂. The mixture was added to a solution of **I-L1** (35 g, 127.0 mmol, reported in patent 'WO2014/169833 A1, 2014') in DMSO (100 mL) at 25°C for 16 hrs. The reaction was treated with water (300 mL), extracted with EtOAc (2 x 200 mL). The combined organic phase was washed with water (2 x 200 mL), brine (200 mL), dried over anhydrous Na₂SO₄, filtered and concentrated in vacuum to give **I-L2** (40 g) as a solid.

### Synthesis of I-L3

To freshly prepared methoxysodium (693.0 mmol) in MeOH (400 mL) was added **I-L2** (20 g, 69.3 mmol) and the resulting mixture was stirred at 70°C for 16 hrs. The reaction was treated with water (200 mL). The aqueous phase was extracted with EtOAc (2 x 200 mL). The combined organic phase was washed with brine (300 mL), dried over anhydrous Na₂SO₄, filtered and concentrate in vacuum. The residue was purified by flash column (0~30% of EtOAc in PE) to give **I-L3** (10 g, 47.8%) as a solid.

**¹H NMR** (400 MHz, CDCl₃) δ_{H} 3.41-3.36 (m, 5H), 2.61 (s, 1H), 1.97-1.73 (m, 5H), 1.68-1.33 (m, 10H), 1.32-0.99 (m, 8H), 0.91-0.81 (m, 3H).

### Synthesis of I-L4

A cold (0°C) solution of lithium di-isopropylamide prepared from addition of n-butyl-lithium in hexane (79.6 mL, 199 mmol, 2.5 M in hexane) to di-isopropylamine (31.3 mL, 224 mmol) in THF (100 mL) at -78°C was added over 30 mins to a stirred solution of **I-L3** (10 g, 31.2 mmol) and ethyl diazoacetate (19.5 mL, 187 mmol) in THF (50 mL) at -78°C. The mixture was stirred at -78°C for 45 min, after which acetic acid (12.7 m, 224 mmol) in THF (50 mL) was added during 20 min. The mixture was then allowed to warm to 25°C. Water (200 mL) was then added. The organic layer was separated and the aqueous solution was extracted with EtOAc (3 x 100 mL). The combined organic phase was washed with brine (300 mL), dried over Na₂SO₄, filtered and concentrated in vacuum. The residue was purified by flash column (0~30% of EtOAc in PE) to give **I-L4** (5.0 g, 37%) as a solid.

**¹H NMR** (400 MHz, CDCl₃) δ_{H} 3.47-3.32 (m, 5H), 2.69-2.56 (m, 1H), 2.23-2.08 (m, 1H), 1.93-1.59 (m, 8H), 1.51-1.41 (m, 8H), 1.35-1.25 (m, 10H), 1.15-1.03 (m, 5H), 0.92 (s, 3H).

### Synthesis of I-L5

To a solution of **I-L4** (4 g, 9.2 mmol) in DME (50 mL) was added Rhodium(II) acetate dimer (20.3 mg, 0.046 mmol) in one portion at 25°C. The mixture was stirred at 25°C for 2 hours. The mixture was concentrated to give **I-L5** (4 g) as an oil.

### Synthesis of I-L6

To a solution of **I-L5** (4.0 g, 9.8 mmol) in MeOH (50 mL) was added KOH (551 mg, 9.8 mmol) in one portion at 25°C. The mixture was stirred at 65°C for 16 hours. Water (100 mL) was added. The mixture was extracted with EtOAc (3 x 50 mL). The combined organic phase was washed with brine (30 mL), dried over Na₂SO₄, filtered and concentrated in vacuum. The residue was purified by flash column (0~20% of EtOAc in PE) to give L6 (1.8 g, 81%) as a solid. The material (200 mg) was purified by flash column (0~20% of EtOAc in PE) to give **I-L6** (90 mg, 45 %) as a solid.

**¹H NMR** (400 MHz, CDCl3) δ_{H} 3.44-3.33 (m, 5H), 2.66-2.57 (m, 1H), 2.56 (s, 1H), 2.20 (br d, J=12.0 Hz, 1H), 2.10-1.99 (m, 1H), 1.91-1.68 (m, 6H), 1.68-1.55 (m, 3H), 1.53-1.53 (m, 1H), 1.53-1.39 (m, 3H), 1.39-1.20 (m, 6H), 1.14 (br d, J=9.8 Hz, 1H), 1.08 (s, 3H), 1.06-0.91 (m, 2H); **MS ESI** calcd. for C21H35O3Na [M+Na]+ 357, found 357.

### Example I-37: Synthesis of (1S,4aS,4bR,6aR,8R,10aS,10bR,12aS)-8-(methoxymethyl)-12a-methyloctadecahydrochrysene-1,8-diol (I-L8)

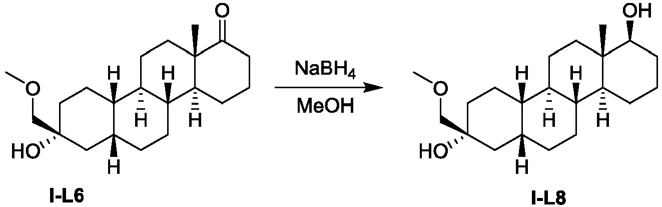

To a solution of **I-L6** (200 mg, 0.6 mmol) in MeOH (2 mL) was added NaBH₄ (67.7 mg, 1.8 mmol) in one portion at 0°C. After the addition, the mixture was stirred at 25 C for 0.5 h. The mixture was quenched with NH₄Cl (10 mL). The mixture was extracted with EtOAc (2 x 10 mL). The combined organic phase was washed with brine (2 x 10 mL), dried over Na₂SO₄, filtered and concentrated to give **I-L8** (210 mg) as a solid. The product was purified by flash column (0~20% of EtOAc in PE) to give **I-L8** (72 mg, 34%) as a solid. The stereochemistry at C17a was confirmed by NOE. H17a has a correlation signal with H14 and no correlation signal with H18.

**¹H NMR** (400 MHz, CDCl3) δH 3.45-3.32 (m, 5H), 3.24-3.15 (m, 1H), 2.60 (s, 1H), 1.95-1.81 (m, 2H), 1.79-1.57 (m, 8H), 1.56-1.19 (m, 10H), 1.12-0.81 (m, 6H), 0.79 (s, 3H); **MS ESI** calcd. for C21H36O3Na [M+Na]+ 359, found 359.

### Example I-38: Synthesis of (1S,4aS,4bR,6aR,8R,10aS,10bR,12aS)-8-methoxy-8-(methoxymethyl)-12a-methyloctadecahydrochrysen-1-ol (I-L9)

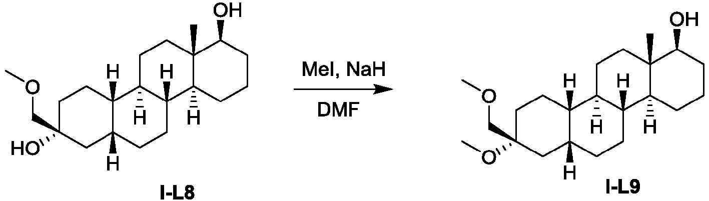

To a mixture of sodium hydride (94.6 mg, 2.4 mmol, 60%) in DMF (2 mL) at 0°C was added a solution of **I-L8** (200 mg, 0.6 mmol) in DMF (2 mL) dropwise. The mixture was stirred at 25°C for 30 min. Then iodomethane (101 mg, 0.7 mmol) was added. The mixture was stirred at 25°C for 1 h. Then, the reaction was quenched with water (10 mL) and the mixture was extracted with EtOAc (2 x 10 mL). The combined organic phase was washed with brine (10 mL) and dried over Na₂SO₄ concentrated to give **I-L9** (150 mg) as a solid, which was purified by flash column (0~30% of EtOAc in PE) to give **I-L9** (50 mg, 34%) as a solid.

**¹H NMR** (400 MHz, CDCl3) δ_{H} 3.50-3.47 (m, 2H), 3.39 (s, 3H), 3.28 (s, 3H), 3.24-3.15 (m, 1H), 1.94-1.84 (m, 2H), 1.80-1.56 (m, 9H), 1.52-1.17 (m, 9H), 1.13-0.83 (m, 6H), 0.80 (s, 3H); **MS ESI** calcd. for C22H38O3Na [M+Na]+ 373, found 373.

### Example I-39: Synthesis of (1S,4aS,4bR,6aS,8R,10aS,10bR,12aS)-8-(ethoxymethyl)-12a-methyloctadecahydrochrysene-1,8-diol

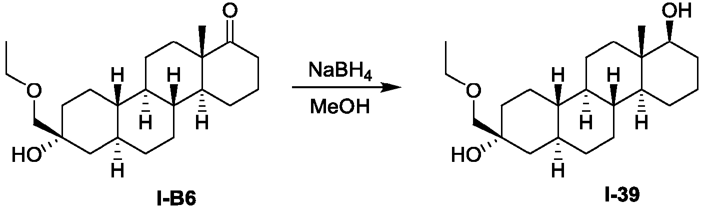

To a solution of **I-B6** (1.0 g, 2.9 mmol) in MeOH (20 mL) was added NaBH₄ (108 mg, 2.9 mmol) at 15°C. The reaction mixture was stirred for 1h at 15°C. The reaction mixture was added into saturated NH₄Cl (100 mL). The aqueous layer was extracted with EtOAc (3 x 50 mL). The combined organic layer was washed with saturated brine (2 x 100 mL), dried over anhydrous Na₂SO₄, filtered and concentrated to give the product. The residue was triturated by PE (10 mL) to give **I-39** (370 mg, 37%) as a solid and an oil (600 mg).

**¹H NMR** (400 MHz, CDCl₃) δ_{H} 3.53 (q, *J =* 7.2 Hz, 2H), 3.25-3.13 (m, 3H), 2.08 (s, 1H), 1.95-1.59 (m, 9H), 1.51-1.15 (m, 10H), 1.13-0.82 (m, 8H), 0.80 (s, 3H), 0.78-0.58 (m, 2H); **MS ESI** calcd. for C₂₂H₃₇O₂ [M-H₂O+H]⁺ 333, found 333.

### Example I-40: Synthesis of (2R,4aS,4bR,6aS,7S,10aS,10bR,12aS)-2-(ethoxymethyl)-7-methoxy-6a-methyloctadecahydrochrysen-2-ol

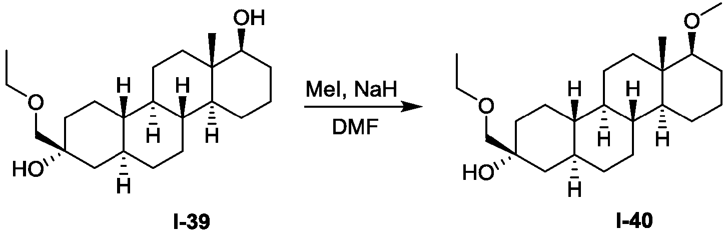

To a solution of **I-39** (300 mg, 0.9 mmol) in DMF (5 mL) was added NaH (51.1 mg, 1.3 mmol, 60% in oil) at 0°C. The reaction mixture was stirred for 30 min at 0°C. Then CH₃I (181 mg, 1.3 mmol) was added to the above mixture and the resulting mixture was stirred at 15°C for 12 h. Saturated NH₄Cl (10 mL) was added to the mixture. The aqueous layer was extracted with EtOAc (3 x 10 mL). The combined organic layers were washed with saturated brine (10 mL), dried over anhydrous Na₂SO₄, filtered and concentrated to give the product. The product was purified by flash column (0-6% of EtOAc in PE) to give **I-40** (14 mg, 5%) as a solid.

**¹H NMR** (400 MHz, CDCl₃) δ_{H} 3.52 (q, *J =* 7.2 Hz, 2H), 3.33 (s, 3H), 3.21 (s, 2H), 2.64 (dd, *J=* 4.0,11.2 Hz, 1H), 2.08 (s, 1H), 2.04-1.93 (m, 1H), 1.82-1.72 (m, 5H), 1.63-1.58 (m, 2H), 1.42-1.12 (m, 9H), 1.06-0.61 (m, 14H); **MS ESI** calcd. for C₂₃H₃₉O₂ [M-H₂O+H]⁺ 347, found 347.

### Example I-49 and I-49a: Synthesis of (2R,4aS,4bR,6aS,7S,10aS,10bR,12aR)-7-methoxy-2-(methoxymethyl)-6a-methyloctadecahydrochrysen-2-ol (I-N11) and (2S,4aS,4bR,6a8,7S,10aS,10bR,12aR)-7-methoxy-2-(methoxymethyl)-6a-methyloctadecahydrochrysen-2-ol (I-N11a)

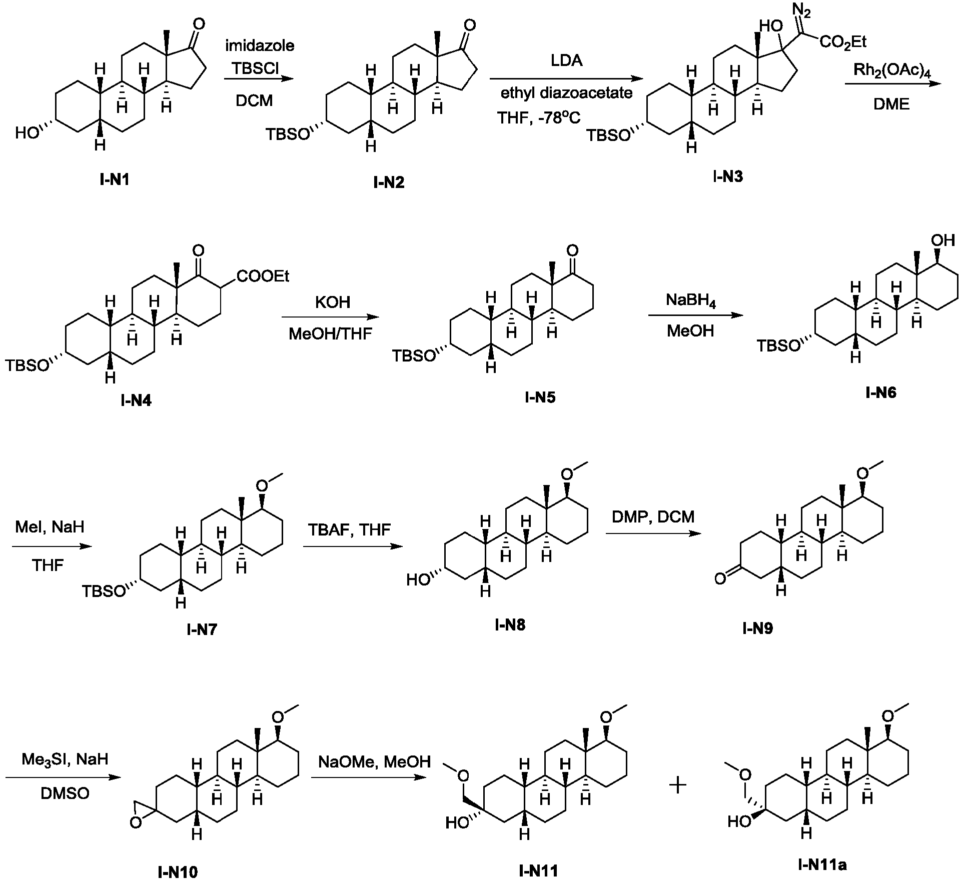

### Synthesis of I-N2

To a solution of 19-norandrosterone (**I-N1**) (66 g, 238 mmol) in DCM (500 mL) were added imidazole (48.5 g, 714 mmol) and TBSCl (64.5 g, 428 mmol) at 10°C. The reaction mixture was stirred for 1h. The reaction mixture was filtered and the mother liquor was washed with saturated NH₄Cl (2 x 200 mL), dried over anhydrous Na₂SO₄, filtered and concentrated to give **I-N2** (110 g) as an oil. **I-N2** (110 g) was purified by flash column (0-5% of EtOAc in PE) to give **I-N2** (94 g, 86%) as an oil.

**¹H NMR** (400 MHz, CDCl₃) δ_{H} 3.60-3.50 (m, 1H), 2.43 (dd, *J=* 8.8, 19.6 Hz, 1H), 2.15-2.00 (m, 1H), 1.89-1.54 (m, 7H), 1.48-1.13 (m, 12H), 1.08-0.75 (m, 14H), 0.05 (s, 6H).

### Synthesis of I-N3

A cold (-78 °C) solution of lithium di-isopropylamide from addition of n-butyl-lithium in hexane (230 mL, 2.5 M, 575 mmol) to di-isopropylamine (84 mL, 0.72 g/mL, 598 mmol) in THF (200 mL). To a stirred a solution of **I-N2** (45.0 g, 115 mmol) and ethyl diazoacetate (65.6 g, 575 mmol) in THF (600 mL) was added LDA (575 mmol) at -78°C. The reaction mixture was stirred for 1 hour at -78°C. Acetic acid (34.5 g, 575 mmol) in THF (200 mL) was added to the reaction mixture at -78°C. The reaction mixture was stirred for 16 hours at 10°C. The reaction mixture was added into water (1 L). The aqueous phase was extracted with EtOAc (3 x 500 mL). The combined organic layers were washed with brine (1 L), dried over anhydrous Na₂SO₄, filtered and concentrated to give the product. The residue was purified by flash column (0-5% of EtOAc in PE) to give **I-N3** as an oil.

**¹H NMR** (400 MHz, CDCl₃) δ_{H} 4.27-4.24 (m, 2H), 3.60-3.57 (m, 1H), 2.10-2.00 (m, 1H), 1.75-1.50 (m, 9H), 1.48-1.00 (m, 20H), 0.88-0.68 (m, 9H), 0.06 (s, 6H).

### Synthesis of I-N4

To a solution **of I-N3** (112.0 g, 221 mmol) in DME (600 mL) was added Rh₂ (OAc)₄ (1.0 g, 2.3 mmol) at 10°C. The reaction mixture was stirred for 2 hours. The reaction mixture was added into water (800 mL). The aqueous phase was extracted with EtOAc (2 x 500 mL). The combined organic layer was washed with saturated brine (1 L), dried over anhydrous Na₂SO₄, filtered and concentrated to give **I-N4** (98.0 g) as an oil.

**¹H NMR** (400 MHz, CDCl₃) δ_{H} 12.42 (s, 1H), 4.20 (q, *J =* 7.2 Hz, 2H), 3.60-3.50 (m, 1H), 2.30-2.25 (m, 1H), 2.18-2.00 (m, 2H), 1.98-1.65 (m, 9H), 1.61-1.34 (m, 10H), 1.31-1.08 (m, 5H), 0.98-0.88 (m, 11H), 0.06 (s, 6H).

### Synthesis of I-N5

To a solution of **I-L4** (24.0 g) in MeOH (200 mL) and THF (100 mL) was added KOH (22.5 g, 402 mmol) at 25°C. After stirring at 75°C for 2 h, the reaction mixture was poured into water (300 mL). The aqueous phase was extracted with EtOAc (2 x 200 mL). The combined organic phase was washed with saturated brine (2 x 200 mL), dried over anhydrous Na₂SO₄, filtered and concentrated. The residue was purified by flash column (0-5% of EtOAc in PE) to give **I-L5** (20.0 g) as an oil.

**¹H NMR** (400 MHz, CDCl₃) δ_{H} 3.58-3.56 (m, 1H), 2.63-2.58 (m, 1H), 2.25-1.89 (m, 2H), 1.83-1.54 (m, 8H), 1.44-0.75 (m, 26H), 0.05 (s, 6H).

### Synthesis of I-N6

To a mixture of **I-N5** (2.0 g, 4.9 mmol) in MeOH (40 mL) was added NaBH₄ (186 mg, 4.9 mmol). The mixture was stirred at 15°C for 5 hrs. Then to the mixture was added saturated NH₄Cl (aq, 50 mL). The mixture was extracted with EtOAc (3 x 30 mL). The combined organic layers were dried over anhydrous Na₂SO₄, and filtered. The filtrate was concentrated under vacuum to afford the product, which was purified by flash chromatography on silica gel (5% - 10% EtOAc in PE) to afford **I-N6** (740 mg, 37%) as a solid.

**¹H NMR** (400MHz, CDCl₃) δ_{H} 3.65 - 3.51 (m, 1H), 3.24 - 3.12 (m, 1H), 1.95 - 1.84 (m, 2H), 1.77 - 1.59 (m, 6H), 1.53 - 1.39 (m, 4H), 1.35 - 1.13 (m, 7H), 1.11 - 0.91 (m, 5H), 0.89 (s, 9H), 0.87 - 0.81 (m, 2H), 0.79 (s, 3H), 0.05 (s, 6H).

### Synthesis of I-N7

To a solution of **I-N6** (740 mg, 1.8 mmol) in THF (10 mL) was added NaH (431 mg, 60%, 10.8 mmol) at 0°C. The reaction mixture was stirred for 1h at 20°C. Then MeI (1.5 g, 1.1 mmol) was added into the reaction mixture at 20°C. The reaction mixture was stirred for 16 hours at 20°C. The reaction mixture was added into saturated NH₄Cl (50 mL). The aqueous layer was extracted with EtOAc (2 x 50 mL). The combined organic layer was washed with saturated brine (100 mL), dried over anhydrous Na₂SO₄ and filtered. The filtrate was concentrated under vacuum to afford **I-N7** (950 mg) as an oil.

**¹H NMR** (400MHz, CDCl₃) δ_{H} 3.64 - 3.53 (m, 1H), 3.33 (s, 3H), 2.63 (dd, *J*=4.0, 11.6 Hz, 1H), 2.02 - 1.97 (m, 1H), 1.94 - 1.74 (m, 4H), 1.68 - 1.64 (m, 2H), 1.58 - 1.54 (m, 3H), 1.45 - 1.39 (m, 2H), 1.33 - 1.29 (m, 2H), 1.22 - 1.16 (m, 4H), 0.99 - 0.95 (m, 3H), 0.89 (s, 9H), 0.86 - 0.82 (m, 4H), 0.78 (s, 3H), 0.06 (s, 6H).

### Synthesis of I-N8

To a mixture of **I-N7** (1.3 g, 3.1 mmol) in THF (15 mL) was added TBAF (15.4 mL, 1 M, 15.4 mmol) at 15°C. The mixture was stirred at 15°C for 16h. Then the mixture was treated with water (30 mL). The aqueous layer was extracted with EtOAc (3 x 30 mL). The combined organic layers were washed with brine (2 x 10 mL), dried over anhydrous Na₂SO₄, and filtered. The filtrate was concentrated under vacuum to afford **I-N8** (1.1 g) as a solid. **¹H NMR** (400MHz, CDCl₃) δ_{H} 3.63 (tt, *J*=5.2, 10.0 Hz, 1H), 3.33 (s, 3H), 2.64 (dd, *J=*4.0, 11.2 Hz, 1H), 2.03 - 1.93 (m, 2H), 1.86 - 1.74 (m, 3H), 1.69 - 1.65 (m, 2H), 1.60 - 1.54 (m, 5H), 0.78 (s, 3H).

### Synthesis of I-N9

To a mixture of **I-N8** (1.1 g, 3.6 mmol) in DCM (20 mL) was added DMP (3.0 g, 7.2 mmol). The mixture was stirred at 15°C for 2h. The mixture was quenched by saturated NaHCO₃ aqueous (50 mL). The aqueous layer was extracted with EtOAc (3 x 50 mL). The combined organic layers were washed with brine (2 x 30 mL), dried over anhydrous Na₂SO₄, and filtered. The filtrate was concentrated under vacuum to afford **I-N9** (500 mg) as an oil.

**¹H NMR** (400MHz, CDCl₃) δ_{H} 3.34 (s, 3H), 2.66 (dd, *J*=4.4, 11.2 Hz, 1H), 2.62 - 2.52 (m, 1H), 2.28 - 2.11 (m, 4H), 2.10 - 2.02 (m, 2H), 1.89 - 1.59 (m, 7H), 1.56 - 1.47 (m, 2H), 1.39 - 0.87 (m, 9H), 0.82 (s, 3H).

### Synthesis ofI-N10

To a mixture of Me₃SI (334 mg, 1.6 mmol) in DMSO (4 mL) and THF (2 mL) was added NaH (65.4 mg, 1.6 mmol) at 0°C. The mixture was stirred at 0°C for 1h. Then the mixture was added to the stirring mixture of **I-N9** (250 mg, 0.8 mmol) in DMSO (2 mL) at 0°C. The reaction mixture was stirred at 20°C for 4h. Water (20 mL) was then added. The aqueous layer was extracted with EtOAc (3 x 30 mL). The combined organic layers were washed with brine (2 x 20 mL), dried over anhydrous Na₂SO₄, and filtered. The filtrate was concentrated under vacuum to afford **I-N10** (420 mg) as an oil.

**¹H NMR** (400MHz, CDCl₃) δ_{H} 3.33 (d, *J*=1.2 Hz, 3H), 2.68 - 2.62 (m, 1H), 2.58 (s, 1H), 2.26 - 2.17 (m, 1H), 2.12 - 2.05 (m, 1H), 1.94 - 1.80 (m, 3H), 1.70 - 1.61 (m, 2H), 1.54 - 1.39 (m, 3H), 1.36 - 1.29 (m, 2H), 1.22 - 1.14 (m, 2H), 1.10 - 0.82 (m, 12H), 0.80 (d, J=2.0 Hz, 3H).

### Synthesis of I-N11 and I-N11a

To a mixture of **I-N10** (420 mg, 1.3 mmol) in THF (4 mL) was added MeONa (707 mg, 13.1 mmol in 8 mL MeOH). The mixture was stirred at 60°C for 16h. Then to the mixture was added water (20 mL). The mixture was extracted with EtOAc (3 x 20 mL). The combined organic layers were dried over anhydrous Na₂SO₄, and filtered. The filtrate was concentrated under vacuum. The residue was purified by flash chromatography on silica gel (10% - 15% EtOAc in PE) to afford **I-N11** (71 mg, 15.5%) and **I-N11a** (66 mg, 14.4%) both as solids.

### I-N11:

**¹H NMR** (400MHz, CDCl₃) δ_{H} 3.44 - 3.35 (m, 5H), 3.33 (s, 3H), 2.64 (dd, *J*=4.4, 11.2 Hz, 1H), 2.58 (s, 1H), 2.03 - 1.96 (m, 1H), 1.91 - 1.65 (m, 6H), 1.58 - 1.10 (m, 12H), 1.07 - 0.80 (m, 6H), 0.78 (s, 3H); **MS ESI** calcd. for C22H38O3Na [M+Na]⁺ 373, found 373.

### I-N11a:

**¹H NMR** (400MHz, CDCl₃) δ_{H} 3.39 (s, 3H), 3.33 (s, 3H), 3.20 (s, 2H), 2.64 (dd, *J*=4.0, 11.2 Hz, 1H), 2.13 - 2.04 (m, 1H), 1.99 (s, 1H), 1.86 - 1.72 (m, 3H), 1.71 - 1.55 (m, 6H), 1.50 - 0.80 (m, 15H), 0.79 (s, 3H); **MS ESI** calcd. for C22H38O3Na [M+Na]⁺ 373, found 373.

### Example I-50: Synthesis of (2R,4aS,4bR,6aR,8R,10aS,10bR,12aS)-8-hydroxy-2,8,12a-trimethylhexadecahydrochrysen-1(2H)-one

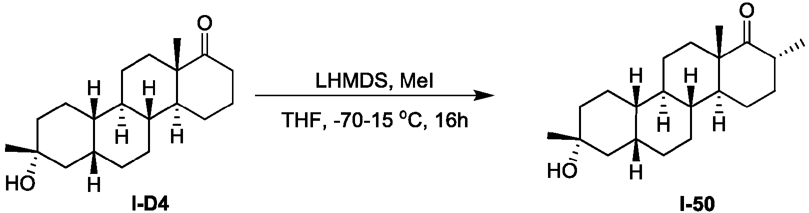

To a solution of **I-D4** (700 mg, 2.29 mmol) in THF (20 mL) was added LHMDS (11.4 mL, 11.4 mmol, 1M) at -70°C. The reaction mixture was stirred for 1 hour at -70°C. MeI (1.61 g, 11.4 mmol) was added to the reaction mixture and stirred for 16 hours at 20°C. The reaction mixture was poured into water (20 mL), extracted with EtOAc (2 x 20 mL). The combined organic layer was washed with brine (100 mL), dried over Na₂SO₄, filtered and concentrated to give the product. Then was purified by combi flash (0-10% of EtOAc in PE) to give **I-50** (14 mg, 2%) as a solid.

**¹H NMR** (400 MHz, CDCl₃) δ 2.78-2.72 (m, 1H), 2.15-2.06 (m, 1H), 1.95-1.59 (m, 11H), 1.51-1.29 (m, 7H), 1.26 (s, 3H), 1.24-1.11 (m, 4H), 1.08 (s, 3H), 1.06-1.00 (m, 1H), 0.99-0.96 (m, 3H); NOE showed signal between H-17a and Me-18 meaning that H-17a and Me-18 should be cis- position. **MS ESI** calcd. for C₂₁H₃₃O [M+H-H₂O]⁺ 301, found 301.

### Example I-53: Synthesis of (2R,4aS,4bR,6aR,8R,10aS,10bR,12aS)-8-hydroxy-8-(methoxymethyl)-2,12a-dimethylhexadecahydrochrysen-1(2H)-one

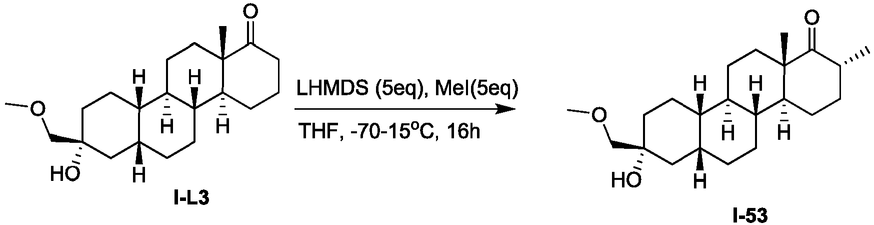

To a solution of **I-L3** (495 mg, 1.48 mmol) in THF (20 mL) was added into LHMDS (7.5 mL, 7.50 mmol, 1 M) at -70°C. The reaction mixture was stirred for 1 hour at 20 °C. MeI (7.6 g, 53.5 mmol) was added into the reaction mixture at 20°C. The reaction mixture was stirred for 16 hours at 20°C. The reaction mixture was poured into water (20 mL), then extracted with EtOAc (2 x 50 mL). The combined organic layer was washed with brine (100 mL), dried over Na₂SO₄, filtered and concentrated to give the product, which was purified by combi flash (0-10% of EtOAc in PE) to give **I-53** (64 mg, 12%) as a solid.

**¹H NMR** (400 MHz, CDCl₃) δ 3.45-3.35 (m, 5H), 2.75-2.65 (m, 1H), 2.61-2.52 (m, 1H), 2.12-2.02 (m, 1H), 1.95-1.59 (m, 9H), 1.49-1.12 (m, 11H), 1.07 (s, 3H), 1.06-0.95 (m, 5H); **MS ESI** calcd. for C₂₂H₃₅O₂ [M+H-H₂O]⁺ 331, found 331. The structure was confirmed by NOE. (There was signal between H-17a and Me-18. Thus, H-17a and Me-18 should be at cis-position.)

### Example I-57: Synthesis of (1S,4aS,4bR,6aS,8R,10aS,10bR,12aS)-8-(ethoxymethyl)-8-hydroxy-12a-methyl-N-phenyloctadecahydrochrysene-1-carboxamide (I-B11)

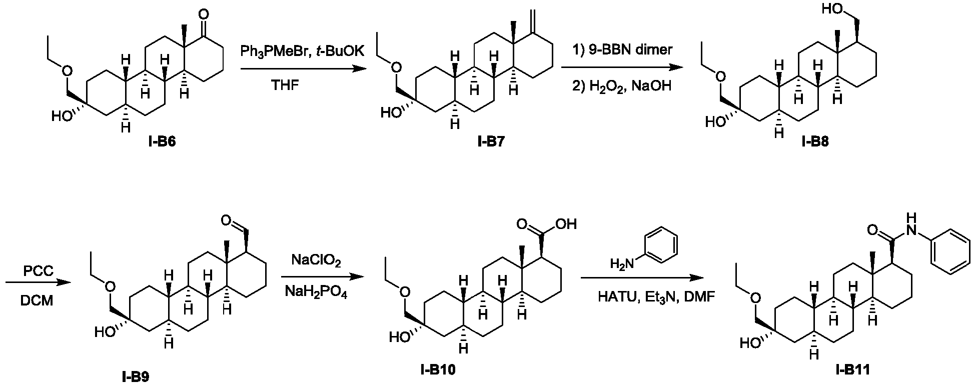

### Synthesis of I-B7

To a solution of MePPh₃Br (6.12 g, 17.2 mmol) in THF (10 mL) was added t-BuOK (1.92 g, 17.2 mmol) at 15°C. The reaction mixture was stirred for 0.5h at 50°C. A solution of **I-B6** (1.2 g, 3.4 mmol) in THF (10 mL) was added to the reaction mixture at 30°C. The reaction mixture was stirred for 1 hour at 60°C. The reaction mixture was added into water (100 mL). The aqueous layer was extracted with EtOAc (3 x 50 mL). The combined organic layer was washed with saturated brine (100 mL), dried over anhydrous Na₂SO₄, filtered and concentrated. The residue was purified by flash column (0-10% of EtOAc in PE) to give **I-B7** (1.3 g) as a solid.

**¹H NMR** (400 MHz, CDCl₃) δ_{H} 4.65-4.50 (m, 2H), 3.52 (q, *J=* 6.8 Hz, 2H), 3.21 (s, 2H), 2.35-2.24 (m, 1H), 2.15-2.04 (m, 2H), 1.85-1.64 (m, 9H), 1.30-1.13 (m, 9H), 1.05-0.94 (m, 3H), 0.80-0.25 (m, 8H).

### Synthesis of I-B8

To a solution of **I-B7** (1.3 g, 3.8 mmol) in THF (40 mL) was added dimer 9-borabicyclo [3.3.1] nonane (5.49 g, 22.5 mmol) at 0°C under N₂. The reaction mixture was stirred at 25°C for 2 h. Then an aqueous solution of sodium hydroxide (10%, 45 mmol) and hydrogen peroxide (30%, 45 mmol) were added and the mixture was stirred for 2h at 10°C. Then sodium sulfite (15 g) was added. The mixture was extracted with ethyl acetate (3 x 20 mL). The combined organic layer was washed with brine (2 x 30 mL), dried over anhydrous Na₂SO₄, filtered and concentrated to give **I-B8** (660 mg,48.5%) as a solid.

**¹H NMR** (400 MHz, CDCl₃) δ_{H} 3.96-3.83 (m, 1H), 3.52 (q, *J=* 6.8 Hz, 2H), 3.33-3.26 (m, 1H), 3.21 (s, 2H), 2.17 (s, 1H), 2.11-2.06 (m, 1H), 1.87-1.65 (m, 8H), 1.48-1.08 (m, 12H), 1.01-0.56 (m, 12H).

### Synthesis of I-B9

To a solution of **I-B8** (260 mg, 0.71 mmol) in DCM (4 mL) was added PCC (227 mg, 1.1 mmol) and silica gel (0.5 g) at 15°C. The mixture was stirred at 15°C for 3 h. The suspension was filtered and the filter cake was washed with DCM (2 x 10 mL). The combined filtrate was concentrated to afford **I-B9** (200 mg) as a solid.

### Synthesis of I-B10

To a mixture of **I-B9** (200 mg, 0.55 mmol) and 2-methyl-2-butene (2 mL) in acetone (6 mL) was added a solution of NaClO₂ (248 mg, 2.75 mmol) and NaH₂PO₄ (329 mg, 2.75 mmol) in H₂O (3 mL) at 0°C. The reaction mixture was stirred at 15 °C for 12 hrs. The reaction mixture was diluted with H₂O (15 mL) and extracted with EtOAc (3 x 15 mL). The combined organic phase was washed with saturated brine (20 mL), dried over anhydrous Na₂SO₄, filtered and concentrated to give the product. The product was purified by flash column (25~30% of EtOAc in PE) to give **I-B10** (100 mg, 48.0%) as a solid.

**¹H NMR** (400 MHz, CDCl₃) δ_{H} 3.52 (q, *J =* 7.2 Hz, 2H), 3.21 (s, 2H), 2.16-2.08 (m, 1H), 1.86-1.54 (m, 13H), 1.45-1.29 (m, 2H), 1.28-1.11 (m, 7H), 1.10-0.92 (m, 7H), 0.90-0.73 (m, 3H), 0.69-0.57 (m, 1H).

### Synthesis of I-B11

To a solution of **I-B10** (100 mg, 0.26 mmol) in DMF (5 mL) were added HATU (250 mg, 0.66 mmol), TEA (66.6 mg, 0.66 mmol) and aniline (24.5 mg, 0.2641 mmol) at 60°C. The mixture was stirred at 60°C for 5 hrs. The reaction mixture was poured into water (10 mL) and the aqueous layer was extracted with EtOAc (2 x 15 mL). The combined organic phase was washed with saturated brine (2 x 50 mL), dried over anhydrous Na₂SO₄, filtered and concentrated in vacuum to afford the product. The product was purified by flash chromatography (EtOAc in petroleum ether =20%-25%) to give **I-B11** (90 mg) as a solid. The material (90 mg) was purified by prep-HPLC (Column: Boston Prime C18 150*30mm 5µm; Condition: water (0.05% ammonia hydroxide v/v)-ACN; Begin B: 78; End B: 100; Gradient Time (min): 8; 100%B Hold Time(min): 2) to give **I-B11** (22.8 mg, 19.1%) as a solid.

**¹H NMR** (400 MHz, CDCl₃) δ_{H} 7.51 (d, *J=* 7.6 Hz, 2H), 7.30 (t, *J=* 7.2 Hz, 2H), 7.12-7.06 (m, 1H), 7.03 (s, 1H), 3.52 (q, *J =* 6.8 Hz, 2H), 3.21 (s, 2H), 1.94-1.80 (m, 4H), 1.79-1.58 (m, 8H), 1.45-1.16 (m, 9H), 1.05-0.94 (m, 7H), 0.93-0.73 (m, 4H), 0.69-0.58 (m, 1H); **MS ESI** calcd. for C₂₉H₄₄NO₃ [M+H]⁺ 454, found 454.

### Example I-58: Synthesis of ((1S,4aS,4bR,6aR,8R,10aS,10bR,12aS)-8-(ethoxymethyl)-8-hydroxy-12a-methyloctadecahydrochrysen-1-yl)((S)-2-methylpiperidin-1-yl)methanone (I-P9)

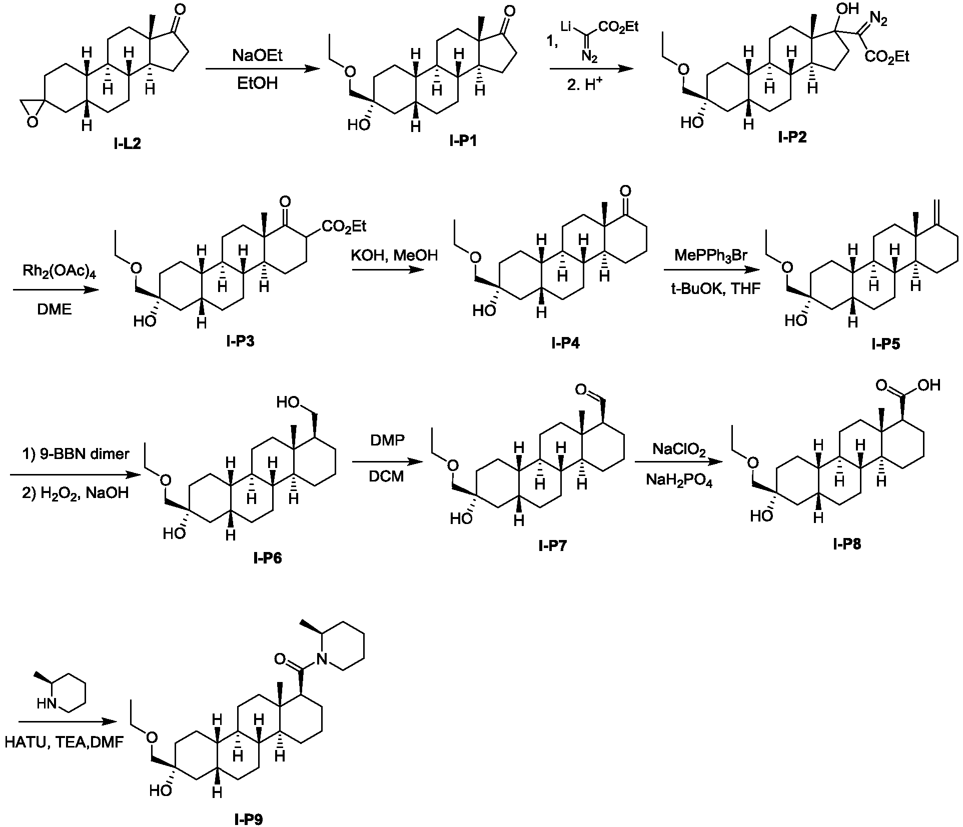

### Synthesis of I-P1

To a mixture of **I-L2** (17.8 g, 61.9 mmol) in anhydrous EtOH (50 mL) was added the fresh prepared NaOEt (23.6 g, 347 mmol) in 300 mL EtOH at 15°C. The reaction mixture was stirred at 80°C for 16 h. Water (200 mL) was added. The reaction mixture was concentrated to remove most of the solvent. The mixture was extracted with EtOAc (2 x 200 mL). The combined organic phase was washed with saturated brine (2 x 100 mL), dried over anhydrous Na₂SO₄, filtered, concentrated. The residue was purified by flash column (5% - 13% EtOAc in PE) to give **I-P1** (8.0 g, 38.6 %) as an oil.

**¹H NMR** (400 MHz, CDCl₃) δ_{H} 3.49 (q, *J*=6.8 Hz, 2H), 3.39 (q, *J*=9.2 Hz, 2H), 2.46-2.33 (m, 1H), 2.11-2.01 (m, 1H), 1.95-1.85 (m, 1H), 1.84-1.68 (m, 5H), 1.66-1.23 (m, 12H), 1.19-0.96 (m, 6H), 0.83 (s, 3H).

### Synthesis of I-P2

To a solution of **I-P1** (2.0 g, 6.0 mmol) and ethyl diazoacetate (3.4 g, 29.8 mmol) in THF (40 mL) was added LDA (26.8 mmol in 15 mL THF) at -70°C. The reaction mixture was stirred for 2 hour at -70°C. Acetic acid (1.8 g, 29.8 mmol) in THF (40 mL) was added to the reaction mixture at -70°C. The reaction mixture was stirred for 16 hours at 10°C. The mixture was added into water (100 mL). The mixture was extracted with EtOAc (3 x 100 mL). The combined organic layers were washed with brine (50 mL), dried over anhydrous Na₂SO₄, and filtered. The filtrate was concentrated under vacuum. The residue was purified by flash chromatography on silica gel (10% - 20% EtOAc in PE) to afford **I-P2** (1.5 g, 56.1%) as an oil.

**¹H NMR** (400MHz, CDCl₃) δ_{H} 4.32 - 4.19 (m, 2H), 3.53 (q, *J*=7.2 Hz, 2H), 3.46 - 3.37 (m, 2H), 2.75 (s, 1H), 2.20 - 2.07 (m, 1H), 1.92 - 1.61 (m, 7H), 1.49 - 1.36 (m, 6H), 1.33 (t, *J*=7.2 Hz, 4H), 1.29 - 1.24 (m, 2H), 1.20 (t, *J*=7.2 Hz, 3H), 1.15 - 1.00 (m, 5H), 0.91 (s, 3H), 0.89 - 0.83 (m, 2H).

### Synthesis of I-P3

To a solution of **I-P2** (1.5 g, 3.3 mmol) in DME (15 mL) was added Rh₂(OAc)₄ (15 mg, 0.03 mmol) in one portion at 20°C. The mixture was stirred at 20°C for 16 hrs. Then the mixture was concentrated under vacuum to afford **I-P3** (1.2 g, 85.7%) as an oil.

**¹H NMR** (400MHz, CDCl₃) δ_{H} 4.19 (q, *J*=7.2 Hz, 2H), 3.56 - 3.49 (m, 3H), 3.46 - 3.39 (m, 3H), 2.73 (br s, 1H), 2.37 - 2.28 (m, 1H), 2.16 - 2.04 (m, 2H), 1.94 - 1.72 (m, 5H), 1.70 - 1.51 (m, 5H), 1.46 - 1.34 (m, 4H), 1.29 (t, *J*=7.2 Hz, 4H), 1.20 (t, *J*=7.2 Hz, 4H), 1.17 - 1.11 (m, 1H), 1.09 (s, 3H), 1.06 - 1.00 (m, 2H).

### Synthesis of I-P4

To a mixture of **I-P3** (1.5 g, 3.5 mmol) in MeOH (120 mL) was added KOH (970 mg, 17.3 mmol) at 20°C. The mixture was stirred at 70°C for 2h. The reaction mixture was poured into brine (200 mL), extracted with DCM (3 x 200 mL). The combined organic layers were washed with HCl (1 M, 200 mL), saturated NaHCO₃ (200 mL), brine (200 mL). Then the organic layer was dried over anhydrous Na₂SO₄, and filtered. The filtrate was concentrated under vacuum to afford **I-P4** (1.2 g) as a solid.

**¹H NMR** (400MHz, CDCl₃) δ_{H} 3.52 (q, *J*=6.8 Hz, 2H), 3.42 (q, *J*=9.2 Hz, 2H), 2.68 (s, 1H), 2.60 (dt, *J*=6.8, 14.0 Hz, 1H), 2.24 - 2.15 (m, 1H), 2.10 - 1.99 (m, 1H), 1.89 - 1.75 (m, 3H), 1.73 - 1.70 (m, 2H), 1.65 - 1.47 (m, 8H), 1.46 - 1.32 (m, 5H), 1.31 - 1.24 (m, 2H), 1.22 - 1.18 (m, 3H), 1.07 (s, 3H), 1.04 - 0.85 (m, 2H).

### Synthesis of I-P5

To a mixture of MePPh₃Br (5.1 g, 14.3 mmol) in THF (10 mL) was added t-BuOK (1.6 g, 14.3 mmol) at 20°C under N₂. The resulting mixture was stirred at 50°C for 30 min. **I-P4** (1.0 g, 2.9 mmol) was added in portions below 50°C. The reaction mixture was stirred at 50°C for 16 hours. The reaction mixture was quenched with saturated NH₄Cl aqueous (30 mL) at 15°C. THF layer was separated. The aqueous layer was extracted with EtOAc (3 x 30 mL). The combined organic layers were dried over anhydrous Na₂SO₄, and filtered. The filtrate was concentrated under vacuum to give a solid, which was purified by trituration with MeOH/H₂O (1:1, 50 mL) at reflux to give **I-P5** (1.0 g) as a solid.

**¹H NMR** (400MHz, CDCl₃) δ_{H} 4.62 - 4.51 (m, 2H), 3.53 (q, *J*=7.2 Hz, 2H), 3.48 - 3.37 (m, 2H), 2.67 (s, 1H), 2.32 (dt, *J*=5.2, 13.6 Hz, 1H), 2.15 - 2.00 (m, 1H), 1.91 - 1.76 (m, 3H), 1.71 - 1.63 (m, 2H), 1.62 - 1.57 (m, 3H), 1.53 - 1.47 (m, 2H), 1.43 - 1.34 (m, 4H), 1.27 - 1.24 (m, 2H), 1.20 (t, *J*=7.2 Hz, 4H), 1.12 - 0.97 (m, 4H), 0.95 (s, 3H), 0.93 - 0.83 (m, 2H).

### Synthesis of I-P6

To a mixture of **I-P5** (1.2 g, 3.5 mmol) in THF (40 mL) was added 9-BBN dimer (5.1 g, 20.7 mmol) at 15°C under N₂. The reaction mixture was stirred at 15°C for 2 hours. NaOH aqueous (6.9 mL, 5 M, 34.6 mmol) was added dropwise below 15°C. H₂O₂ (3.9 g, 30%, 34.6 mmol) was added dropwise below 15°C. Then saturated Na₂S₂O₃ (50 mL) was added. The mixture was poured into water (100 mL), and filtered. The filter cake was dissolved in DCM (20 mL), dried over anhydrous Na₂SO₄, and filtered. The filtrate was concentrated under vacuum to give **I-P6** (560 mg, 44.4%) as an oil.

**¹H NMR** (400MHz, CDCl₃) δ_{H} 3.76 - 3.73 (m, 2H), 3.53 (q, *J*=7.2 Hz, 2H), 3.42 (q, *J*=9.2 Hz, 2H), 2.72 - 2.65 (m, 1H), 1.87 - 1.82 (m, 4H), 1.81 - 1.75 (m, 2H), 1.73 - 1.65 (m, 4H), 1.53 - 1.47 (m, 2H), 1.43 (s, 2H), 1.41 - 1.32 (m, 4H), 1.20 (t, *J*=7.2 Hz, 5H), 1.14 - 1.07 (m, 2H), 0.97 - 0.92 (m, 3H), 0.88 - 0.84 (m, 2H), 0.73 (s, 3H)

### Synthesis of I-P7

To a mixture of **I-P6** (560 mg, 1.5 mmol) in DCM (10 mL) was added DMP (1.3 g, 3.1 mmol). The mixture was stirred at 15°C for 2h. The mixture was quenched by saturated NaHCO₃ aqueous (30 mL). The aqueous layer was extracted with EtOAc (3 x 30 mL). The combined organic layers were washed with brine (2 x 20 mL), dried over anhydrous Na₂SO₄, and filtered. The filtrate was concentrated under vacuum to afford **I-P7** (360 mg) as an oil.

**¹H NMR** (400MHz, CDCl₃) δ_{H} 10.14 - 9.77 (m, 1H), 3.53 (q, *J*=7.2 Hz, 2H), 3.42 (q, *J*=9.2 Hz, 2H), 2.81 - 2.55 (m, 1H), 2.03 - 1.96 (m, 1H), 1.92 - 1.74 (m, 4H), 1.72 - 1.65 (m, 3H), 1.64 - 1.46 (m, 7H), 1.46 - 1.37 (m, 4H), 1.36 - 1.32 (m, 1H), 1.30 - 1.22 (m, 2H), 1.22 - 1.18 (m, 2H), 0.97 (s, 5H), 0.94 - 0.91 (m, 3H).

### Synthesis of I-P8

To a mixture of **I-P7** (360 mg, 1.0 mmol) and 2-methyl-2-butene (2 mL) in acetone (10 mL) was added NaClO₂ (448 mg, 5.0 mmol) and NaH₂PO₄ (595 mg, 5.0 mmol) in H₂O (5 mL) at 0°C. The reaction mixture was stirred at 15°C for 48h. The mixture was treated with water (30 mL). The aqueous layer was extracted with EtOAc (3 x 30 mL). The combined organic layers were washed with brine (2 x 20 mL), dried over anhydrous Na₂SO₄, and filtered. The filtrate was concentrated under vacuum to afford **I-P8** (360 mg) as an oil.

**¹H NMR** (400MHz, CDCl₃) δ_{H} 3.53 (q, *J*=6.8 Hz, 2H), 3.46 - 3.38 (m, 2H), 2.19 - 2.10 (m, 1H), 1.88 - 1.78 (m, 3H), 1.74 - 1.65 (m, 6H), 1.61 - 1.55 (m, 3H), 1.45 - 1.38 (m, 3H), 1.36 - 1.32 (m, 2H), 1.31 - 1.25 (m, 4H), 1.20 (s, 4H), 1.03 - 0.97 (m, 2H), 0.95 (s, 3H), 0.93 - 0.82 (m, 3H).

### Synthesis of I-P9

To a mixture of **I-P8** (120 mg, 0.3 mmol) in DMF (2 mL) was added HATU (180 mg, 0.5 mmol) and Et₃N (159 mg, 1.6 mmol). The mixture was stirred at 20°C for 1h. Then to the mixture was added (2S)-2-methylpiperid (156 mg, 1.6 mmol). The reaction mixture was stirred at 20°C for 16h. Water (10 mL) was added. The mixture was extracted with EtOAc (3 x 10 mL). The combined organic layers were washed with brine (2 x 10 mL), dried over anhydrous Na₂SO₄, and filtered. The filtrate was concentrated under vacuum to afford the product, which was purified by flash chromatography on silica gel (10% - 15% EtOAc in PE) to afford **I-P9** (53 mg, 24.3%) as a solid.

**¹H NMR** (400MHz, CDCl₃) δ_{H} 4.98 (br s, 0.6H), 4.54 (m, 0.4H), 4.30 (m, 0.4H), 3.98 - 3.84 (m, 0.6H), 3.53 (q, *J*=7.2 Hz, 2H), 3.47 - 3.36 (m, 2H), 3.16 - 3.03 (m, 0.6H), 2.71 (s, 1H), 2.66 - 2.54 (m, 0.4H), 2.41 - 2.28 (m, 1H), 1.90 - 1.73 (m, 4H), 1.72 - 1.62 (m, 5H), 1.58 - 1.47 (m, 5H), 1.45 - 1.31 (m, 6H), 1.31 - 1.24 (m, 3H), 1.20 (t, *J*=6.8 Hz, 3H), 1.12 - 0.97 (m, 9H), 0.96 - 0.80 (m, 2H); **MS ESI** calcd. for C29H50NO3 [M+H]⁺ 460, found 460.

### Example I-59: Synthesis of ((1S,4aS,4bR,6aR,8R,10aS,10bR,12aS)-8-hydroxy-8-(methoxymethyl)-12a-methyloctadecahydrochrysen-1-yl)((S)-2-methylpiperidin-1-yl)methanone (I-L14)

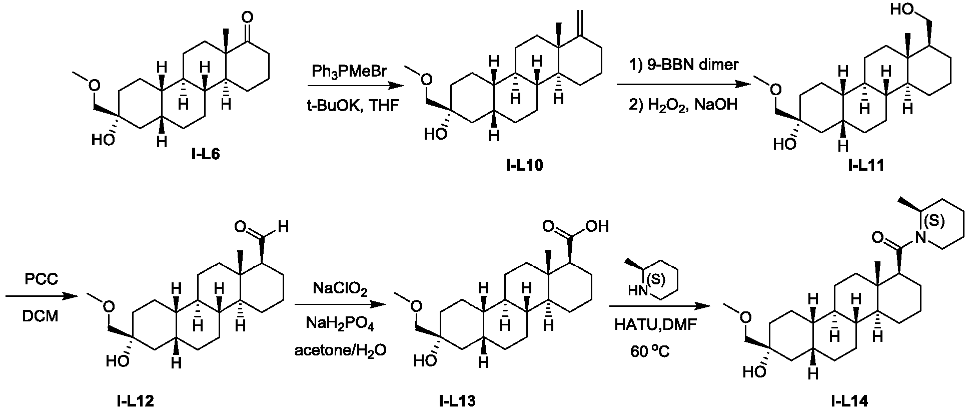

### Synthesis of I-L10

To a suspension of Ph₃PMeBr (22.3 g, 62.5 mmol) in anhydrous THF (100 mL) was added t-BuOK (7.0 g, 62.5 mmol) at 25°C under N₂. The reaction mixture was stirred at 25°C for 20 min. A solution of **I-L6** (4.2 g, 12.5 mmol) in anhydrous THF (50 mL) was added. After stirring at 25°C for 1h, the mixture was poured into ice-water (300 mL). The aqueous phase was extracted with EtOAc (2 x 200 mL). The combined organic phase was washed with saturated brine (2 x 100 mL), dried over anhydrous Na₂SO₄, filtered and concentrated. The residue was purified by column (0~10% of EtOAc in PE) to give **I-L10** (4.0 g, 96%) as an oil.

**¹H NMR** (400 MHz, CDCl₃) δ_{H} 4.56 (d, *J =* 12.0 Hz, 1H), 3.45-3.37 (m, 5H), 2.56 (s, 1H), 2.38-2.27 (m, 1H), 2.13-2.05 (m, 1H), 1.92-1.50 (m, 12H), 1.48-1.25 (m, 9H), 1.13-0.94 (m, 6H).

### Synthesis of I-L11

To a solution of **I-L10** (4.0 g, 12.0 mmol) in anhydrous THF (100 mL) was added 9-BBN dimer (11.6 g, 48.0 mmol) at 25°C under N₂. After stirring at 60°C for 1 h. then the mixture was cooled. The reaction mixture was quenched by EtOH (20 mL). NaOH (19.2 mL, 5M, 96.0 mmol) was added very slowly. After addition, H₂O₂ (13.6 g, 120 mmol, 30% in water) was added slowly until the inner temperature no longer rises and the inner temperature was maintained below 30°C. The mixture was stirred at 60°C for another 1h. The mixture was poured into water (1 L) to give a suspension, filtered in vacuum to give a solid. The residue was purified by flash column (10~30% of EtOAc in PE) to give **I-L11** (3.3 g, 78%) as an oil. **¹H NMR** (400 MHz, CDCl₃) δ_{H} 3.97-3.87 (m, 1H), 3.40-3.38 (m, 5H), 3.35-3.27 (m, 1H), 1.89-1.50 (m, 16H), 1.25-1.07 (m, 6H), 1.00-0.75 (m, 6H), 0.72 (s, 3H).

### Synthesis of I-L12

To a solution of **I-L11** (3.0 g, 8.5 mmol) in DCM (50 mL) was added silica gel (10 g) and PCC (5.5 g, 25.6 mmol) at 25°C. After stirring at 25°C for 30 min, the mixture was filtered through a pad of silica gel and the solid was washed with PE/DCM (2 X 100 mL/100 mL), filtered and concentrated in vacuum. The residue was purified by silica gel chromatography (PE/EtOAc = 10/1 to 8/1) to afford **I-L12** (1.5 g, 51%) as an oil.

**¹H NMR** (400 MHz, CDCl₃) δ_{H} 10.09 (s, 0.2H), 9.82 (s, 0.7H), 3.45-3.36 (m, 5H), 2.60-2.45 (m, 1H), 2.30-2.20 (m, 1H), 2.18-1.95 (m, 2H), 1.88-1.25 (m, 18H), 1.22-0.88 (m, 8H).

### Synthesis of I-L13

To a mixture of **I-L12** (1.5 g, 4.3 mmol) and 2-methyl-2-butene (6 mL) in acetone (60 mL) was added NaClO₂ (1.9 g, 21.4 mmol) and NaH₂PO₄ (2.6 g, 21.4 mmol) in H₂O (15 mL) at 0°C. The reaction mixture was stirred at 25°C for 2h. The mixture was treated with water (100 mL). The aqueous layer was extracted with EtOAc (3 x 50 mL). The combined organic layers were washed with brine (2 x 50 mL), dried over anhydrous Na₂SO₄, filtered. The filtrate was concentrated under vacuum to afford **I-L13** (1.1 g) as an oil.

**¹H NMR** (400 MHz, CDCl₃) δ_{H} 3.95-3.93 (m, 5H), 2.56-2.50 (m, 1H), 2.32-2.18 (m, 3H), 1.89-1.15 (m, 20H), 1.14-0.85 (m, 7H).

### Synthesis of I-L14

To a solution of **I-L13** (200 mg, 0.5 mmol) in DMF (10 mL) were added HATU (623 mg, 1.6 mmol), TEA (165 mg, 1.6 mmol) and (2S)-2-methylpiperidine (108 mg, 1.1 mmol) at 25°C. The mixture was stirred at 60°C for 12 hrs. The reaction was poured into water (50 mL). The aqueous phase was extracted with EtOAc (2 x 50 mL). The combined organic phase was washed with saturated brine (2 x 50 mL), dried over anhydrous Na₂SO₄, filtered and concentrated. The residue was purified by flash column (10~25% of EtOAc in PE) to afford **I-L14** (100 mg, 41%) as a solid. The material (100 mg, 0.2 mmol) was purified by flash column (10~25% of EtOAc in PE) to give **I-L14** (43 mg, 43%) as a solid.

**¹H NMR** (400 MHz, CDCl₃) δ_{H} 4.97 (s, 0.6H), 4.54 (d, *J=* 13.2 Hz, 0.4H), 4.37-4.27 (s, 0.4H), 3.90 (d, *J =* 13.2 Hz, 0.6H), 3.39-3.38 (m, 5H), 3.09 (t, *J =* 2.4, 13.2 Hz, 0.6H), 2.62-2.59 (m, 1.4H), 2.37-2.34 (m, 1H), 1.88-1.15 (m, 24H), 1.13-0.75 (m, 13H); **MS ESI** calcd. for C₂₈H₄₈NO₃ [M+H]⁺ 446, found 446.

### Example I-60: Synthesis of 1-((1S,4aS,4bR,6aR,8R,10aS,10bR,12aS)-8-hydroxy-8-(methoxymethyl)-12a-methyloctadecahydrochrysen-1-yl)ethan-1-one (I-L17)

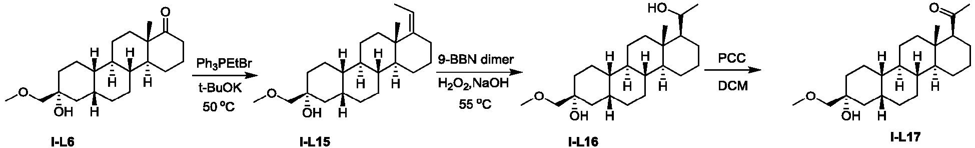

### Synthesis of I-L15

To a suspension of Ph₃PEtBr (23.2 g, 62.5 mmol) in anhydrous THF (100 mL) was added t-BuOK (7.0 g, 62.5 mmol) at 25°C under N₂. The reaction mixture was stirred at 25°C for 20 min to get a dark red suspension. A solution of **I-L6** (4.2 g, 12.5 mmol) in anhydrous THF (50 mL) was added. After stirring at 50°C for 6h, the mixture was poured into ice-water (300 mL). The aqueous phase was extracted with EtOAc (2 x 200 mL). The combined organic phase was washed with saturated brine (2 x 100 mL), dried over anhydrous Na₂SO₄, filtered and concentrated. The residue was purified by column (0~10% of EtOAc in PE) to give **I-L15** (3 g, 69%) as an oil.

**¹H NMR** (400 MHz, CDCl₃) δ_{H} 5.20-5.10 (m, 1H), 3.45-3.35 (m, 5H), 2.60-2.45 (m, 2H), 2.25-1.55 (m, 21H), 1.25-0.88 (m, 9H).

### Synthesis of I-L16

To a solution of **I-L15** (3.0 g, 8.6 mmol) in anhydrous THF (50 mL) was added 9-BBN dimer (8.4 g, 34.6 mmol) at 25°C under N₂. After stirring at 55°C for 5 h, then the mixture was cooled, quenched by EtOH (20 mL). NaOH (17.2 mL, 5M, 86.4 mmol) was added very slowly. After addition, H₂O₂ (9.8 g, 86.4 mmol, 30% in water) was added slowly until the inner temperature no longer rises and the inner temperature was maintained below 30°C. After stirring at 60°C for another 1 h, the mixture was poured into water (100 mL). The aqueous phase was extracted with EtOAc (3 x 100 mL). The combined organic phase was washed with 10% aqueous Na₂S₂O₃ (2 x 50 mL), saturated brine (2 x 100 mL), filtered and concentrated. The residue was purified by flash column (10~20% of EtOAc in PE) to give **I-L16** (1.5 g, 48%) as an oil.

**¹H NMR** (400 MHz, CDCl₃) δ_{H} 4.27-4.18 (m, 1H), 3.45-3.35 (m, 5H), 1.95-1.82 (m, 3H), 1.75-1.12 (m, 23H), 1.04-0.66 (m, 8H).

### Synthesis of I-L17

To a solution of **I-L16** (1.5 g, 4.1 mmol) in DCM (50 mL) was added silica gel (10 g) and PCC (4.4 g, 20.5 mmol) at 25°C. After stirring at 25°C for 12 h, the mixture was filtered through a pad of silica gel and the solid was washed with PE/DCM (2 X 100 mL/100 mL), filtered and concentrated in vacuum. The residue was purified by silica gel chromatography (PE/EtOAc = 8/1 to 5/1) to afford **I-L17** (0.5 g, 34%) as a solid.

**¹H NMR** (400 MHz, CDCl₃) δ_{H} 3.45-3.35 (m, 5H), 2.59 (s, 1H), 2.30 (dd, *J* = 3.2, 12.8 Hz, 1H), 2.14 (s, 3H), 1.90-1.15 (m, 20H), 1.00-0.75 (m, 8H). **MS ESI** calcd. for C₂₃H₃₇O₂ [M+H-H₂O]⁺ 345, found 345.

### Example I-61: Synthesis of 1-(2-((1S,4aS,4bR,6aR,8R,10aS,10bR,12aS)-8-hydroxy-8-(methoxymethyl)-12a-methyloctadecahydrochrysen-1-yl)-2-oxoethyl)-1H-pyrazole-4-carbonitrile (I-L19)

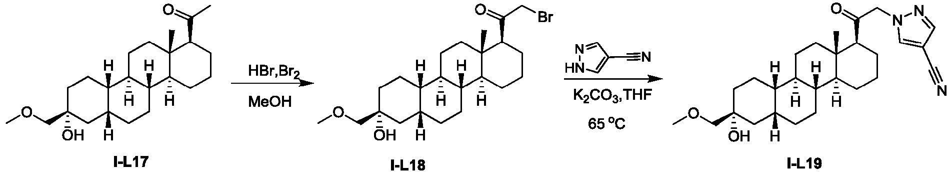

### Synthesis of I-L18

To a solution of **I-L17** (500 mg, 1.4 mmol) in methanol (20 mL) was added HBr (40%, 27.4 mg, 0.14 mmol) and Br₂ (262 mg, 1.6 mmol) drop wise. After stirring at 25°C for 1 h, saturated Na₂S₂O₃ (50 mL) was added. The aqueous phase was extracted with EtOAc (2 x 50 mL). The combined organic phase was washed with saturated brine (2 x 50 mL), filtered and concentrated to give **I-L18** (450 mg, 75%) as an oil.

**¹H NMR** (400 MHz, CDCl₃) δ_{H} 3.95 (dd, *J =* 13.2, 22.4 Hz, 2H), 3.45-3.35 (m, 5H), 2.60-2.56 (m, 2H), 1.90-1.17 (m, 20H), 1.12-0.85 (m, 8H).

### Synthesis of I-L19

To a solution of **I-L18** (150 mg) in anhydrous THF (10 mL) was added 1H-pyrazole-4-carbonitrile (94.0 mg, 1.0 mmol) and K₂CO₃ (141 mg, 1.0 mmol) at 25°C under N₂. The mixture was stirred at 65°C for 12 h. The reaction mixture was poured into ice-water (50 mL) and extracted with EtOAc (3 x 50 mL). The combined organic phase was washed with saturated brine (2 x 50 mL), dried over anhydrous Na₂SO₄, filtered and concentrated. The residue was purified by flash column (30~40% of EtOAc in PE) to give **I-L19** (46.5 mg, 30%) as a solid.

**¹H NMR** (400 MHz, CDCl₃) δ_{H} 7.82 (s, 1H), 7.81 (s, 1H), 4.99 (dd, *J =* 18.0, 31.2 Hz, 2H), 3.43-3.35 (m, 5H), 2.63 (brs, 1H), 2.32 (dd, *J =* 3.2, 12.4 Hz, 1H), 1.88-1.10 (m, 20H), 1.08-0.88 (m, 8H); **MS ESI** calcd. for C₂₇H₃₈N₃O₂ [M+H-H₂O]⁺ 436, found 436.

### Example I-62: Synthesis of 1-((1R,4aS,4bR,6aS,8R,10aS,10bS,12aS)-8-hydroxy-10a,12a-dimethyloctadecahydrochrysen-1-yl)ethan-1-one (I-Q8a)

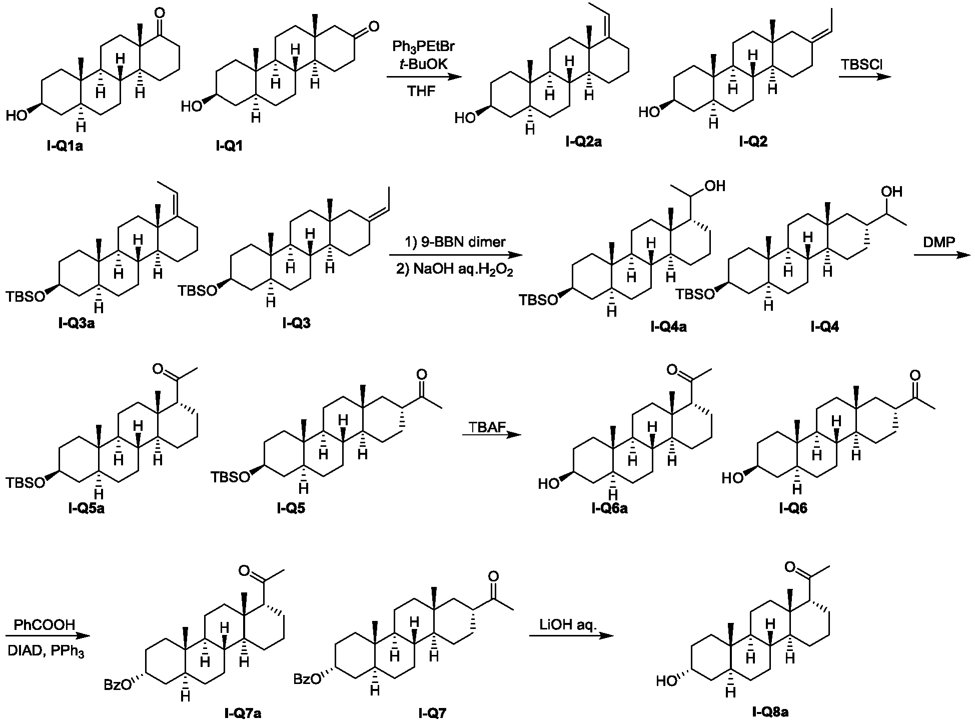

### Synthesis of I-Q2a and I-Q2

To a suspension of EtPPh₃Br (21.1 g, 57.0 mmol) in THF (150 mL) was added t-BuOK (6.38 g, 57.0 mmol) at 15°C under N₂. After stirring at 50°C for 30 mins, a mixture of **I-Q1a & I-Q1** (CAS# 26729-16-8 & 51057-15-9) (3.5 g, 11.4 mmol) in THF (30 mL) was added to the mixture in portions below 65°C. The mixture was stirred at 50°C for 1 h and treated with sat. NH₄Cl (150 mL), extracted with EtOAc (3 x 100 mL). The organic layer was separated, concentrated in vacuum to give a product, which was purified by flash column (0~20% of EtOAc in PE) to give a mixture of **I-Q2a** & **I-Q2** (3 g, 83%) as a solid.

**¹H NMR** (400 MHz, CDCl₃) δ 5.29-4.96 (m, 1H), 3.85-3.65 (m, 1H), 3.65-3.41 (m, 1H), 2.70-2.43 (m, 1H), 2.30-2.08 (m, 1H), 2.01-1.47 (m, 10H), 1.46-1.01 (m, 11H), 1.00-0.56 (m, 10H).

### Synthesis of I-Q3a & I-Q3

To a solution of a mixture of **I-Q2a** & **I-Q2** (3.0 g, 9.47 mmol) in DCM (30 mL) was added 1H-imidazole (1.28 g, 18.9 mmol) and TBSCl (2.14 g, 14.2 mmol) at 25°C. After stirring at 25°C for 16 hours, the mixture was diluted with DCM (60 mL), washed with water (2 x 30 mL), brine (30 mL), dried over Na₂SO₄, filtered and concentrated under vacuum to give a residue, which was purified by flash column (0~5% of EtOAc in PE) to give a mixture of **I-Q3a** & **I-Q3** (4.3 g) as a solid.

**¹H NMR** (400 MHz, CDCl₃) δ 5.27-4.98 (m, 1H), 3.61-3.48 (m, 1H), 2.71-2.45 (m, 1H), 2.30-2.09 (m, 1H), 2.04-1.51 (m, 8H), 1.49-0.95 (m, 14H), 0.94-0.90 (m, 3H), 0.89-0.86 (m, 9H), 0.83-0.57 (m, 6H), 0.05 (s, 6H).

### Synthesis of I-Q4a & I-Q4

To a solution of a mixture of **I-Q3a** & **I-Q3** (4.3 g, 9.98 mmol) in THF (50 mL) was added 9-BBN dimer (4.85 g, 19.9 mmol) at 25°C. The reaction mixture was stirred at 60°C under N₂ for 3 hrs and treated with ethanol (5.73 mL, 99.8 mmol), followed by adding NaOH aqueous (19.9 mL, 5 M, 99.8 mmol) and H₂O₂ (9.97 mL, 10 M, 99.8 mmol) dropwise at 0°C. Then the mixture was warmed to 65°C and stirred for 1 hr, diluted with water (1.5 L). The reaction mixture was extracted with EtOAc (2 x 800 mL). The combined organic layer was added saturated aqueous Na₂S₂O₃ (60 mL) and stirred for 15 min. Then the organic phase was washed with saturated brine (2 x 50 mL), dried over anhydrous Na₂SO₄, filtered and concentrated in vacuum to give a mixture of **I-Q4a** & **I-Q4** (4.5 g) as a solid.

**¹H NMR** (400 MHz, CDCl₃) δ 3.63-3.43 (m, 1H), 2.64-2.23 (m, 1H), 2.21-1.96 (m, 3H), 1.89-1.62 (m, 5H), 1.56-1.36 (m, 5H), 1.36-0.92 (m, 10H), 0.89-0.60 (m, 13H), 0.84-0.62 (m, 8H), 0.04 (s, 6H).

### Synthesis of I-Q5a & I-Q5

To a solution of a mixture of **I-Q4a** & **I-Q4** (4.5 g, 10.0 mmol) in DCM (100 mL) was added DMP (12.7 g, 30.0 mmol). The reaction mixture was stirred at 25°C for 60 mins and treated with water (80 mL), followed by adding NaHCO₃ solid (12.7 g, 150 mmol) in portions. The mixture was filtered. The filtrate was washed with saturated Na₂S₂O₃ (2 x 80 mL) aqueous, extracted with DCM (2 x 100 mL). The combined organic layer was washed with brine (150 mL), dried over Na₂SO₄, filtered and concentrated in vacuum to give a product which was purified by flash column (0~10% of EtOAc in PE) to give a mixture of **I-Q5a** & **I-Q5** (1.8 g, 40%) as a solid and 2.6 g of product. The material (2.6 g, 5.81 mmol) was purified by flash column (0~10% of EtOAc in PE) to give **I-Q5a** & **I-Q5** (0.8 g, 31%) as a solid and 1.3 g of product.

**¹H NMR** (400 MHz, CDCl₃) δ 3.60-3.46 (m, 1H), 2.50-2.21 (m, 1H), 2.16-2.06 (m, 3H), 1.89-1.50 (m, 3H), 1.56-1.36 (m, 6H), 1.35-1.10 (m, 8H), 1.07-0.84 (m, 16H), 0.83-0.59 (m, 6H), 0.05 (s, 6H).

### Synthesis of I-Q6a & I-Q6

To a solution of a mixture of **I-Q5a** & **I-Q5** (2.9 g, 6.19 mmol) in THF (30 mL) was added TBAF (12.9 mL, 1 M in THF, 12.9 mmol) in portions. After stirring at 25°C for 16 hrs, the reaction was quenched with sat. NH₄Cl (50 mL), extracted with EtOAc (2 x 50 mL). The combined organic phase was washed with water (2 x 60 mL), dried over Na₂SO₄, filtered and concentrated to give a mixture of **I-Q6a** & **I-Q6** (2.5 g) as a solid. The product was used directly without further purification.

**¹H NMR** (400 MHz, CDCl₃) δ 3.65-3.53 (m, 1H), 2.67-2.23 (m, 1H), 2.17-2.07 (m, 3H), 1.99-1.62 (m, 6H), 1.56-1.14 (m, 10H), 1.13-0.60 (m, 15H).

### Synthesis of I-Q7a & I-Q7

To a solution of a mixture of **I-Q6a** & **I-Q6** (2.4 g, 7.21 mmol) in THF (150 mL) was added benzoic acid (1.31 mg, 10.8 mmol) and triphenylphosphine (2.83 g, 10.8 mmol) at 25°C under N₂. After stirring at 25°C for 20 mins, DIAD (2.18 g, 10.8 mmol) was added dropwise at 0°C under N₂. The mixture was stirred at 25°C for 16 h. The reaction mixture was quenched with water (100 mL), extracted with ethyl acetate (2 x 100 mL). The combined organic phase was washed with brine (150 mL), dried over Na₂SO₄, filtered and evaporated to give a residue, which was purified by flash column (0~10% of EtOAc in PE) to give **I-Q7** (3.04 g) as an oil. The oil was purified by flash column (0~10% of EtOAc in PE) to give **I-Q7** (340 mg) as a solid and **I-Q7a** (240 mg) as an oil.

### I-Q7a:

**¹H NMR** (400 MHz, CDCl₃) δ 8.11-8.03 (m, 2H), 7.72-7.42 (m, 3H), 2.52-2.45 (m, 1H), 2.14 (s, 3H), 1.94-1.66 (m, 7H), 1.65-1.40 (m, 7H), 1.39-1.03 (m, 8H), 1.00-0.83 (m, 7H), 0.80 (s, 3H).

### Synthesis of I-Q8a

To a solution of **I-Q7a** (100 mg, 0.2290 mmol) in THF (1 mL), MeOH (0.5 mL) and water (0.5 mL) was added LiOH (54.8 mg, 2.29 mmol). After stirring at 25°C for 24 hrs, the mixture was added water (20 mL) and extracted with EtOAc (2 x 10 mL). The combined organic layer was washed with brine (20 mL), dried over Na₂SO₄, filtered and concentrated. The residue was purified by flash column (0-15% of EtOAc in PE) to give **I-Q8a** (35 mg) as an oil, which was triturated with MeCN (5 mL) at 82°C to give **I-Q8a** (8 mg, 23%) as a solid. The structure was confirmed by NOE.

### I-Q8a:

**¹H NMR** (400 MHz, CDCl₃) δ 4.05-3.99 (m, 1H), 2.50-2.41 (m, 1H), 2.16-2.08 (s, 3H), 1.88-1.58 (m, 7H), 1.52-1.33 (m, 7H), 1.33-1.04 (m, 8H), 0.97-0.80 (m, 6H), 0.74 (s, 3H); MS ESI calcd. for C₂₂H₃₅O [M+H-H₂O]⁺ 315, found 315.

### Example I-63: Synthesis of 1-((2R,4aS,4bS,6aS,8R,10aS,10bS,12aR)-8-hydroxy-10a,12a-dimethyloctadecahydrochrysen-2-yl)ethan-1-one (I-Q8)

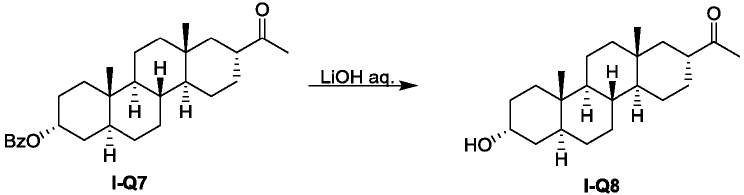

To a solution of **I-Q7** (240 mg, 0.5496 mmol) in THF (2 mL), MeOH (1 mL) and water(1 mL) was added LiOH (131 mg, 5.49 mmol). The mixture was stirred at 25°C for 16 hrs. The mixture was added water (15 mL) and extracted with EtOAc (3 x 10 mL). The combined organic layer was washed with brine (20 mL), dried over Na₂SO₄, filtered and concentrated in vacuum to give a residue which was purified by flash column (0-15% of EtOAc in PE) to give **I-Q8** (51 mg, 28%) as a solid.

**¹H NMR** (400 MHz, CDCl₃) δ 4.06-4.01 (m, 1H), 2.64-2.53 (m, 1H), 2.12 (s, 3H), 1.99-1.69 (m, 3H), 1.68-1.56 (m, 3H), 1.54-1.35 (m, 7H), 1.33-1.10 (m, 8H), 1.07-0.91 (m, 1H), 0.88-0.78 (m, 5H), 0.77-0.72 (m, 4H); **MS ESI** calcd. for C₂₂H₃₅O [M+H-H₂O]⁺ 315, found 315. The structure was confirmed by NOE.

### Example I-64: Synthesis of 1-((4aS,4bR,6aR,8R,10aS,10bR,12aS)-8-hydroxy-8,12a-dimethyl-3,4,4a,4b,5,6,6a,7,8,9,10,10a,10b,11,12,12a-hexadecahydrochrysen-2-yl)ethan-1-one (I-R13)

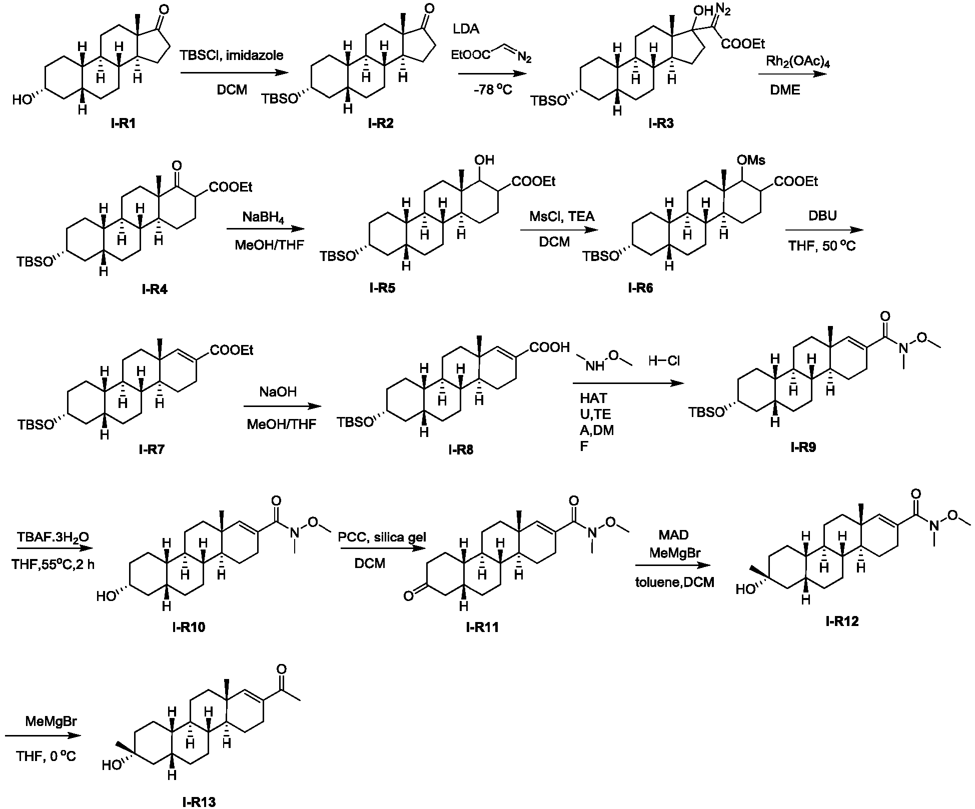

### Synthesis of I-R2

To a solution of **I-R1** (6.5 g, 23.5 mmol) in DCM (50 mL) was added imidazole (2.4 g, 35.2 mmol) and TBSCl (5.3 g, 35.2 mmol) at 25°C. The mixture was stirred at 25°C for 16 hours. The mixture was poured into water (50 mL). The aqueous phase was extracted with DCM (3 x 60 mL). The combined organic phase was washed with brine (100 mL), dried over anhydrous Na₂SO₄, filtered and concentrated under vacuum to give **I-R2** (9.0 g) as an oil.

**¹H NMR** (400 MHz, CDCl₃) δ_{H} 3.75-3.50 (m, 1H), 2.50-2.34 (m, 1H), 2.12-1.00 (m, 23H), 0.98-0.85 (m, 11H), 0.09-0.03 (m, 6H).

### Synthesis of I-R3

A cold (-70°C) LDA solution (73.6 mL, 1.0 M, 73.6 mmol) was added to a stirred solution of **I-R2** (9.0 g, 23.0 mmol) and ethyl diazoacetate (7.9 g, 69.0 mmol) in THF (120 mL) at - 78°C. The mixture was stirred at -70°C for 1h. Then acetic acid (5.0 g, 82.8 mmol) in THF (60 mL) was added and the mixture was then warm to 20°C. Water (100 mL) was added. The aqueous phase was extracted with EtOAc (3 x 100 mL). The combined organic layers were washed with brine (100 mL), dried over anhydrous Na₂SO₄, filtered and evaporated under reduced pressure to give the product as an oil. The residue was purified by flash column (0-5% of EtOAc in PE) to give **I-R3** (6.5 g, 56%) as a solid.

**¹H NMR** (400 MHz, CDCl₃) δ_{H} 4.71-4.69 (m, 1H), 4.30-4.20 (m, 2H), 3.61-3.58 (m, 1H), 2.25-2.17 (m, 1H), 2.00-1.75 (m, 2H), 1.74-1.25 (m, 20H), 1.22-0.75 (m, 15H), 0.06 (s, 6H).

### Synthesis of I-R4

To a solution **of I-R3** (6.7 g, 13.2 mmol) in DME (100 mL) was added Rh₂ (OAc)₄ (100 mg, 0.2 mmol) at 25°C. The reaction mixture was stirred at 25°C for 2 hours. The reaction mixture was extracted with ethyl acetate (3 x 80 mL). The combined organic phase was washed with water (120 mL), brine (120 mL), dried over anhydrous Na₂SO₄, filtered and concentrated under vacuum to give a residue, which was triturated in DCM/PE (15 mL/ 80 mL), no solid was precipitated. The triturated solution was concentrated under vacuum to give **I-R4** (7.1 g) as an oil. The material (7.1 g) was purified by flash column chromatography (ethyl acetate in PE, 10%) to give **I-R4** (6.1 g, 86%) as an oil.

**¹H NMR** (400 MHz, CDCl₃) δ_{H} 12.4 (s, 1H), 4.25-4.11 (m, 2H), 3.75-3.50 (m, 1H), 2.70-1.50 (m, 14H), 1.47-1.00 (m, 15H), 0.88-0.85 (m, 9H), 0.05 (s, 6H).

### Synthesis of I-R5

To a solution of **I-R4** (5.1 g, 10.6 mmol) in MeOH/THF (35 mL/35 mL) was added NaBH₄ (400 mg, 10.6 mmol) at 20°C. The reaction mixture was stirred at 20°C for 20 min. The reaction mixture was quenched by saturated aqueous NH₄Cl (60 mL). The suspension was extracted with ethyl acetate (3 x 80 mL). The combined organic layer was washed with brine (100 mL), dried over anhydrous Na₂SO₄, filtered and concentrated under vacuum to give **I-R5** (4.2 g, 83%) as a solid.

**¹H NMR** (400 MHz, CDCl₃) δ_{H} 4.35-4.00 (m, 2H), 3.70-3.50 (m, 1H), 2.20-0.75 (m, 41H), 0.05 (s, 6H).

### Synthesis of I-R6

To a solution of **I-R5** (4.2 g, 8.8 mmol) in DCM (50 mL) was added TEA (17.6 g, 175 mmol) and 1-methyl-1H-imidazole (20 mL) at 20°C. The mixture was cooled to 0°C; MsCl (7.6 g, 66.4 mmol) in DCM (20 mL) was added. The reaction mixture was stirred at 20°C for 16 hours. The reaction mixture was quenched with water (100 mL). The aqueous phase was extracted with DCM (2 x 100 mL). The combined organic phase was washed with brine (200 mL), dried over anhydrous Na₂SO₄, filtered and concentrated under vacuum to give **I-R6** (6.2 g) as an oil.

**¹H NMR** (400 MHz, CDCl₃) δ_{H} 4.25-4.00 (m, 1H), 3.70-3.54 (m, 1H), 3.32-2.85 (m, 3H), 2.00-0.75 (m, 41H), 0.05 (s, 6H).

### Synthesis of I-R7

To a solution of **I-R6** (6.2 g, 11.1 mmol) in THF (15 mL) was added DBU (6 mL) at 20°C. The reaction mixture was stirred at 50°C for 16 hours. The reaction mixture was extracted with ethyl acetate (2 x 100 mL). The combined organic phase was washed with water (2 x 100 mL), brine (100 mL), dried over anhydrous Na₂SO₄, filtered and concentrated under vacuum to give a residue, which was triturated in MeCN (10 mL) at 20°C to give R7 (8.5 g) as an oil. The material (8.5 g) was purified by flash column chromatography (ethyl acetate in PE, 1%) to give **I-R7** (2.3 g, 27%) as an oil.

**¹H NMR** (400 MHz, CDCl₃) δ_{H} 6.66 (s, 1H), 4.17 (q, *J =* 7.2 Hz, 2H), 3.62-3.55 (m, 1H), 2.40 (dd, *J =* 6.4, 18.4 Hz, 1H), 2.25-2.18 (m, 1H), 1.89-1.75 (m, 2H), 1.70-1.00 (m, 20H), 0.89-0.85 (m, 14H), 0.05 (s, 6H).

### Synthesis of I-R8

To a solution **of I-R7** (2.7 g, 5.8 mmol) in MeOH/THF (20 mL/15 mL) was added NaOH (1.5 g, 37.5 mmol) in water (3 mL). The reaction mixture was stirred at 50°C for 1 hour. The reaction solution was extracted with EtOAc (3 x 80 mL). The combined organic phases were washed with brine (100 mL), dried over anhydrous Na₂SO₄, filtered and concentrated under vacuum to give **I-R8** (3.0 g) as a solid.

**¹H NMR** (400 MHz, CDCl₃) δ_{H} 6.80 (s, 1H), 3.61-3.54 (m, 2H), 2.40 (dd, *J=* 5.6, 18.0 Hz, 1H), 2.25-2.18 (m, 1H), 1.90-1.80 (m, 3H), 1.74-1.50 (m, 8H), 1.43-1.00 (m, 12H), 0.90 (s, 3H), 0.88 (s, 9H), 0.05 (s, 6H).

### Synthesis of I-R9

To a solution of **I-R8** (2.0 g, 4.6 mmol) in DMF (30 mL) were added HATU (2.6 g, 6.9 mmol), TEA (933 mg, 9.2 mmol) and N,O-dimethylhydroxylamine hydrochloride (423 mg, 6.9 mmol) at 25°C. The mixture was stirred at 25°C for 5 hrs. The reaction mixture was poured into water (200 mL). The aqueous phase was extracted with EtOAc (2 x 100 mL). The combined organic layers was washed with 1M HCl (80 mL), brine (2 x 100 mL), dried over anhydrous Na₂SO₄, filtered and concentrated under vacuum. The residue was purified by flash column (10~15% of EtOAc in PE) to afford **I-R9** (1.8 g, 82%) as an oil.

**¹H NMR** (400 MHz, CDCl₃) δ_{H} 5.82 (s, 1H), 3.62-3.54 (m, 4H), 3.22 (s, 3H), 2.38-2.20 (m, 2H), 1.90-1.80 (m, 2H), 1.74-1.51 (m, 15H), 1.38-0.96 (m, 16H), 0.05 (s, 6H).

### Synthesis of I-R10

To a solution of **I-R9** (1.8 g, 3.8 mmol) in THF (50 mL) was added TBAF.3H₂O (5.9 g, 18.9 mmol). After stirring at 55°C for 5 hrs, the mixture was poured into water (100 mL). The aqueous phase was extracted with EtOAc (2 x 100 mL). The combined organic phase was washed with saturated brine (2 x 50 mL), dried over anhydrous Na₂SO₄, filtered and concentrated in vacuum to give **I-R10** (1.5 g) as an oil.

**¹H NMR** (400 MHz, CDCl₃) δ_{H} 5.82 (s, 1H), 3.70-3.60 (m, 4H), 3.45-3.32 (m, 1H), 3.22 (s, 3H), 2.38-2.21 (m, 2H), 2.00-1.45 (m, 15H), 1.14-0.86 (m, 9H).

### Synthesis of I-R11

To a solution of **I-R10** (1.0 g) in DCM (60 mL) was added silica gel (10.0 g) and PCC (5.9 g, 27.5 mol) at 25°C. After stirring at 25°C for 1 h, the mixture was filtered through a pad of silica gel and the filter cake was washed with PE/DCM (2 x 100 mL/100 mL). The filtrate was concentrated in vacuum to give a product as an oil. The residue was purified by silica gel chromatography (PE/EtOAc = 8/1 to 6/1) to afford **I-R11** (0.9 g, 91%) as an oil.

**¹H NMR** (400 MHz, CDCl₃) δ_{H} 5.85 (s, 1H), 3.64 (s, 3H), 3.32 (s, 3H), 2.58 (t, *J =* 16.0 Hz, 1H), 2.28-2.04 (m, 7H), 1.92-1.46 (m, 10H), 1.28-1.10 (m, 5H), 0.96 (s, 3H).

### Synthesis of I-R12

To a solution of 2, 6-di-tert-butyl-4-methylphenol (7.3 g, 33.1 mmol) in toluene (10 mL) was added AlMe₃ (8.25 mL, 16.5 mmol, 2 M in toluene) drop-wise at 0°C. The mixture was warmed to 25°C and stirred at 25°C for 30 min. a solution of **I-R11** (0.9 g, 2.5 mmol) in anhydrous DCM (10 mL) was added to above MAD solution at -70°C and stirred at -70°C for 1 h. MeMgBr (5.0 mL, 15.0 mmol, 3M in ethyl ether) was added drop-wise at -70°C and stirred at -70°C for 20 min. The reaction mixture was slowly poured into saturated aqueous citric acid (50 mL). The aqueous phase was extracted with EtOAc (2 x 100 mL). The combined organic layer was separated, washed with brine (2 x 100 mL), dried over anhydrous Na₂SO₄, filtered and concentrated in vacuum. The residue was purified by flash column (40~60% of EtOAc in PE) to give **I-R12** (0.8 g, 85%) as an oil.

**¹H NMR** (400 MHz, CDCl₃) δ_{H} 5.82 (s, 1H), 3.64 (s, 3H), 3.22 (s, 3H), 2.39-2.18 (m, 2H), 1.89-1.47 (m, 13H), 1.40-1.20 (m, 9H), 1.17-1.97 (m, 3H), 0.91 (s, 3H).

### Synthesis of I-R13

To a solution of **I-R12** (0.8 g, 2.1 mmol) in anhydrous THF (30 mL) was added MeMgBr (7.1 mL, 21.3 mmol, 3M in ether) drop-wise at 0°C under N₂. The reaction mixture was stirred at 0°C for 30 min. The reaction mixture was quenched by saturated citric acid (50 mL) at 0°C. The aqueous phase was extracted with EtOAc (2 x 80 mL). The combined organic phase washed was with saturated brine (2 x 50 mL), dried anhydrous Na₂SO₄, filtered and concentrated in vacuum. The residue was purified by flash column (15~20% of EtOAc in PE) to give **I-R13** (450 mg, 64%) as a solid.

**¹H NMR** (400 MHz, CDCl₃) δ_{H} 6.55 (s, 1H), 2.42 (dd, *J =* 6.0, 18.0 Hz, 1H), 2.28 (s, 3H), 2.13-2.03 (m, 1H), 1.90-1.58 (m, 9H), 1.46-0.99 (m, 16H), 0.93 (s, 3H); **MS ESI** calcd. For C₂₂H₃₅O₂ [M+H] ⁺ 331, found 331.

### Example I-65: Synthesis of 1-((2S,4aS,4bR,6aR,8R,10aS,10bR,12aR)-8-hydroxy-8,12a-dimethyloctadecahydrochrysen-2-yl)ethan-1-one (I-R14)

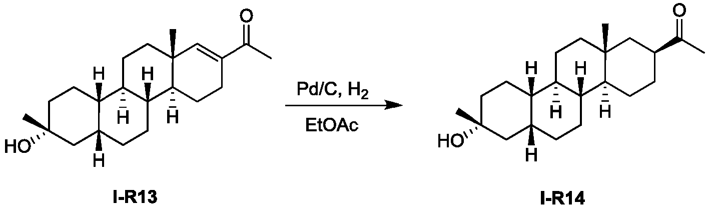

To a solution of **I-R13** (150 mg, 0.45 mmol) in EtOAc (10 mL) was added Pd-C (wet, 10%, 80 mg) under N₂. The suspension was degassed under vacuum and purged with H₂ for three times. 103421 Pa (15 psi) of hydrogen at 25°C for 12 hrs was applied to the resulting solution. The reaction mixture was filtered through a pad of Celite and washed with THF (2 x 50 mL), the filtrate was concentrated. The residue was purified by column (10~15% of EtOAc in PE) to give **I-R14** (50 mg, 33%) as a solid.

**¹H NMR** (400 MHz, CDCl₃) δ_{H} 2.45-2.42 (m, 1H), 2.30 (d, *J =* 12.0 Hz, 1H), 2.19 (s, 3H), 2.04-2.00 (m, 1H), 1.85-1.76 (m, 3H), 1.64-1.24 (m, 17H), 1.18-0.81 (m, 7H), 0.66 (s, 3H); **MS ESI** calcd. For C₂₂H₃₅O [M+H-H₂O]⁺ 315, found 315.

### Example I-66: Synthesis of 1-(2-((4aS,4bR,6aR,8R,10aS,10bR,12aS)-8-hydroxy-8,12a-dimethyl-3,4,4a,4b,5,6,6a,7,8,9,10,10a,10b,11,12,12a-hexadecahydrochrysen-2-yl)-2-oxoethyl)-1H-pyrazole-4-carbonitrile (I-R16)

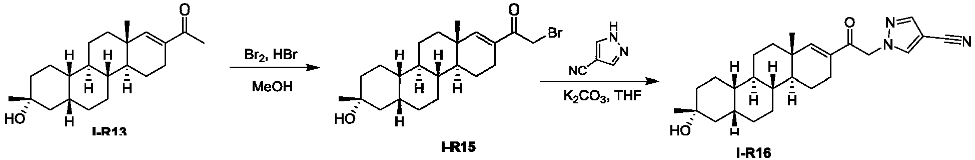

### Synthesis of I-R15

To a solution of **I-R13** (200 mg, 0.6 mmol) in methanol (10 mL) was added HBr (40%, 60.5 mg, 0.3 mmol) and Br₂ (115 mg, 0.7 mmol) drop-wise at 25°C. After stirring at 25°C for 1 h, the reaction was quenched by aqueous Na₂S₂O₃ (50 mL). The aqueous phase was extracted with EtOAc (2 x 50 mL). The combined organic phase was washed with brine (2 x 50 mL), dried over anhydrous Na₂SO₄, filtered and concentrated under vacuum to give **I-R15** (200 mg) as an oil.

**¹H NMR** (400 MHz, CDCl₃) δ_{H} 6.63 (s, 1H), 4.20-4.18 (m, 2H), 2.46 (s, 1H), 1.93-2.56 (m, 15H), 1.43-1.28 (m, 10H), 0.97-0.94 (m, 4H).

### Synthesis of I-R16

To a solution of **I-R15** (200 mg) in anhydrous THF (20 mL) was added 1H-pyrazole-4-carbonitrile (90.9 mg, 1.0 mmol) and K₂CO₃ (134 mg, 1.0 mmol) at 25°C under N₂. The mixture was stirred at 65°C for 12 h. The reaction mixture was poured into ice-water (30 mL). The aqueous phase was extracted with EtOAc (2 x 50 mL). The combined organic phase was washed with saturated brine (2 x 50 mL), dried over anhydrous Na₂SO₄, filtered and concentrated. The residue was purified by flash column (30~40% of EtOAc in PE) to give **I-R16** (130 mg, 63%) as an oil.

**¹H NMR** (400 MHz, CDCl₃) δ_{H} 7.88 (s, 1H), 7.82 (s, 1H), 6.65 (s, 1H), 5.39-5.30 (m, 2H), 2.43 (dd, *J* = 6.0, 18.4 Hz, 1H), 2.16-2.07 (m, 1H), 2.00 (s, 1H), 1.97-1.49 (m, 7H), 1.43-0.99 (m, 17H), 0.96 (s, 3H).

### Example I-67 and I-67a: Synthesis of 1-(2-((2S,4aS,4bR,6aR,8R,10aS,10bR,12aR)-8-hydroxy-8,12a-dimethyloctadecahydrochrysen-2-yl)-2-oxoethyl)-1H-pyrazole-4-carbonitrile (I-R17) & 1-(2-((2R,4aS,4bR,6aR,8R,10aS,10bR,12aR)-8-hydroxy-8,12a-dimethyloctadecahydrochrysen-2-yl)-2-oxoethyl)-1H-pyrazole-4-carbonitrile (I-R17a)

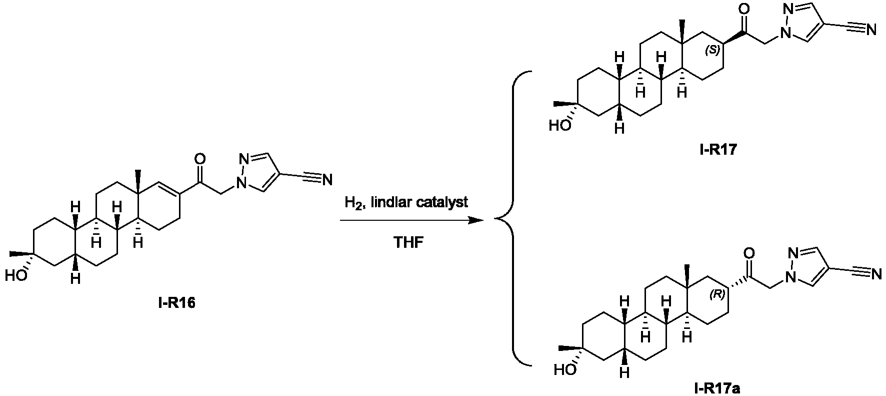

To a solution of **I-R16** (120 mg, 0.3 mmol) in THF (20 mL) was added lindlar catalyst (100 mg) under N₂. The suspension was degassed under vacuum and purged with H₂ for three times. 103421 Pa (15 psi) of hydrogen at 25°C for 18 hrs was applied to the resulting solution. The reaction mixture was filtered through a pad of Celite and washed with THF (2 x 50 mL). The filtrate was concentrated. The residue was purified by flash column (25~35% of EtOAc in PE) to give **I-R17** (5 mg, 4%) as a solid and **I-R17a** (22 mg, 18%) as a solid.

### I-R17:

**¹H NMR** (400 MHz, CDCl₃) δ_{H} 7.88 (s, 1H), 7.82 (s, 1H), 5.05 (s, 2H), 2.75-2.69 (m, 1H), 2.00-1.60 (m, 7H), 1.50-1.23 (m, 15H), 1.18-0.91 (m, 6H), 0.86-0.83 (m, 4H); **MS ESI** calcd. For C₂₆H₃₆N₃O [M+H-H₂O] ⁺ 406, found 406.

### I-R17a:

**¹H NMR** (400 MHz, CDCl₃) δ_{H} 7.86 (s, 1H), 7.81 (s, 1H), 5.27 (d, *J =* 17.6 Hz, 1H), 5.02 (d, *J=* 17.2 Hz, 1H), 2.68-2.62 (m, 1H), 2.35-2.27 (m, 1H), 2.02-1.96 (m, 1H), 1.86 (d, *J =* 8.0 Hz, 1H), 1.79-1.61 (m, 5H), 1.47-1.22 (m, 14H), 1.17-0.81 (m, 7H), 0.70 (s, 3H); **MS ESI** calcd. For C₂₆H₃₆N₃O [M+H-H₂O]⁺ 406, found 406.

### Example I-68: ((4aS,4bR,6aR,8R,10aS,10bR,12aS)-8-hydroxy-8,12a-dimethyl-3,4,4a,4b,5,6,6a,7,8,9,10,10a,10b,11,12,12a-hexadecahydrochrysen-2-yl)(phenyl)methanone (I-R20)

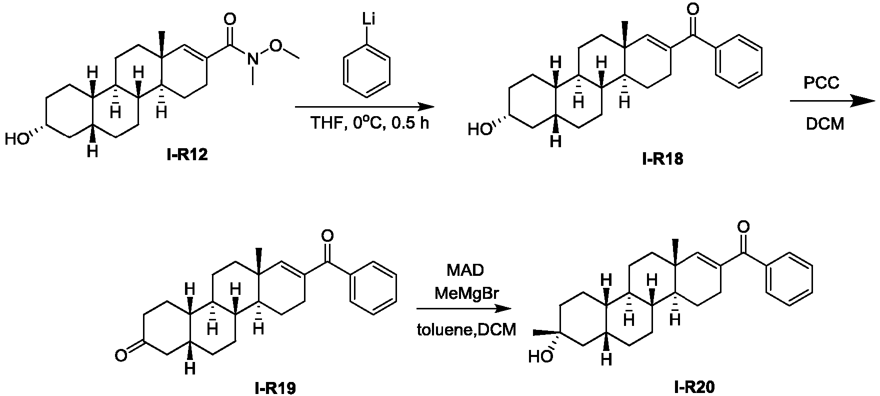

### Synthesis of I-R18

To a solution of **I-R12** (500 mg) in anhydrous THF (10 mL) was added phenyllithium (2.10 mL, 4.1 mmol, 2M in THF) drop-wise at 0°C. The reaction mixture was stirred at 0°C for 30 min under N₂. The reaction was quenched by saturated aqueous NH₄Cl (20 mL). The aqueous phase was extracted with EtOAc (2 x 50 mL). The combined organic phase was washed with saturated brine (2 x 50 mL), dried over anhydrous Na₂SO₄, filtered and concentrated in vacuum to give **I-R18** (500 mg) as an oil.

**¹H NMR** (400 MHz, CDCl₃) δ_{H} 7.66-7.28 (m, 5H), 6.26 (s, 1H), 3.67-3.58 (m, 2H), 3.43-3.22 (m, 1.5H), 2.67-2.54 (m, 1H), 2.10-1.55 (m, 5H), 1.41-1.18 (m, 7H), 0.97-0.85 (m, 12H).

### Synthesis of I-R19

To a solution of **I-R18** (500 mg) in DCM (20 mL) was added silica gel (2 g) and PCC (1.1 g, 5.3 mol) at 25°C. After stirring at 25°C for 30 min, the mixture was filtered through a pad of silica gel and the filter cake was washed with PE/DCM (2 x 30 mL/30 mL), filtered and concentrated in vacuum to give a product. The residue was purified by silica gel chromatography (PE/EtOAc = 10/1 to 8/1) to afford **I-R19** (250 mg, 50%) as a solid.

**¹H NMR** (400 MHz, CDCl₃) δ_{H} 7.64 (d, *J =* 6.8 Hz, 2H), 7.53-7.49 (m, 1H), 7.42 (t, *J =* 6.8 Hz, 2H), 6.27 (s, 1H), 2.66-2.56 (m, 2H), 2.39-1.98 (m, 8H), 1.82-1.57 (m, 7H), 1.48-1.06 (m, 6H), 1.01 (s, 3H).

### Synthesis of I-R20

To a solution of 2, 6-di-tert-butyl-4-methylphenol (3.5 g, 15.8 mmol) in toluene (8 mL) was added AlMe₃ (3.95 mL, 7.9 mmol, 2 M in toluene) drop-wise at 0°C. The mixture was stirred at 25°C for 30 min. a solution of **I-R19** (250 mg, 0.7 mmol) in anhydrous DCM (5 mL) was added drop-wise to above MAD solution at -70°C, during which the temperature was maintained below -60°C. After stirring at -70°C for 1 h, MeMgBr (2.65 mL, 8.0 mmol, 3M in ethyl ether) was added drop-wise at -70°C and stirried at -70°C for 1 h. The reaction mixture was slowly poured into saturated aqueous citric acid (30 mL). The aqueous phase was extracted with EtOAc (2 x 50 mL). The combined organic layer was separated, washed with brine (2 x 30 mL), dried over Na₂SO₄, filtered and concentrated in vacuum. The residue was purified by flash column (10~20% of EtOAc in PE) to give **I-R20** (150 mg, 58%) as a solid. **¹H NMR** (400 MHz, CDCl₃) δ_{H} 7.65-7.61 (m, 2H), 7.55-7.48 (m, 1H), 7.41 (t, *J =* 8.0 Hz, 2H), 6.25 (s, 1H), 2.60 (dd, *J =* 6.0, 18.4 Hz, 1H), 2.36-2.26 (m, 1H), 1.98-1.94 (m, 1H), 1.92-1.61 (m, 8H), 1.52-1.22 (m, 12H), 1.20-1.03 (m, 4H), 0.96 (s, 3H); **MS ESI** calcd. For C₂₇H₃₇O₂ [M+H]⁺ 393, found 393.

### Example I-69: Synthesis of ((2S,4aS,4bR,6aR,8R,10aS,10bR,12aR)-8-hydroxy-8,12a-dimethyloctadecahydrochrysen-2-yl)(phenyl)methanone (I-R21)

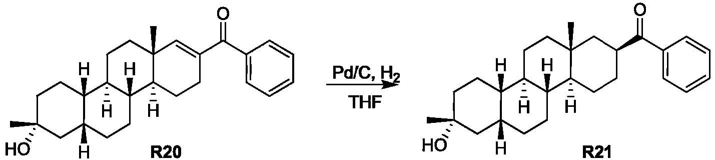

To a solution of **I-R20** (120 mg, 0.3 mmol) in THF (10 mL) was added Pd-C (wet, 10%, 50 mg) under N₂. The suspension was degassed under vacuum and purged with H₂ for three times. 103421 Pa (15 psi) of hydrogen at 25°C for 12 hrs was applied to the resulting solution. The reaction mixture was filtered through a pad of Celite and washed with THF (2 x 50 mL). The filtrate was concentrated and residue was purified by column (10~20% of EtOAc in PE) to give **I-R21** (45 mg, 38%) as a solid.

**¹H NMR** (400 MHz, CDCl₃) δ_{H} 7.80 (d, *J =* 8.0 Hz, 2H), 7.55-7.41 (m, 3H), 3.40-3.36 (m, 1H), 2.43-2.37 (m, 1H), 1.94 (d, *J =* 16.0 Hz, 1H), 1.86-1.74 (m, 3H), 1.70-1.35 (m, 15H), 1.33-1.27 (m, 2H), 1.07-0.80 (m, 7H), 0.54 (s, 3H); **MS ESI** calcd. For C₂₇H₃₇O [M+H-H₂O] ⁺ 377, found 377.

### Example I-70 and I-70a: Synthesis of 1-((1S,4aS,4bR,6aS,8R,10aS,10bR,12aS)-8-(ethoxymethyl)-8-hydroxy-12a-methyloctadecahydrochrysen-1-yl)ethan-1-one (I-S3) & 1-((1R,4aS,4bR,6aS,8R,10aS,10bR,12aS)-8-(ethoxymethyl)-8-hydroxy-12a-methyloctadecahydrochrysen-1-yl)ethan-1-one (I-S3a)

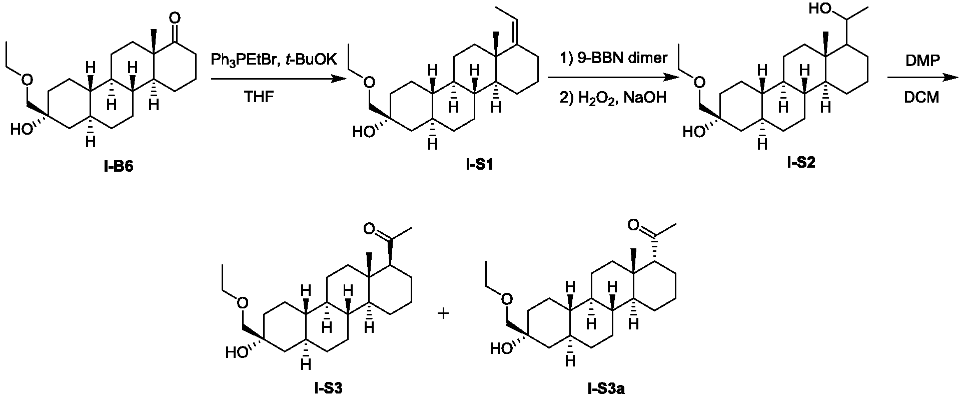

### Synthesis of I-S1

To a suspension of bromo(ethyl)triphenylphosphorane (38.1 g, 103 mmol) in anhydrous THF (300 mL) under N₂ at 20°C was added t-BuOK (11.5 g, 103 mmol). After stirring at 40°C for 30minutes, a solution of **I-B6** (6 g, 17.2 mmol) in anhydrous THF (100 mL) was added. The resulting mixture was stirred at 40°C for 30 minutes, quenched with 10% NH₄Cl (400 mL), extracted with EtOAc (2 x 200 mL). The combined organic phase was concentrated to give a residue, which was dissolved in MeOH (500 mL). The solution was slowly poured to water (500 mL) with stirring. After stirring at 20°C for 30 minutes, the above clear solution was decanted. The remaining oil was diluted with EtOAc (200 mL) and the mixture was washed with 10% NH₄Cl (200 mL), dried over Na₂SO₄, filtered and concentrated to give 5.5 g of **IS1** as an oil.

**¹H NMR** (400 MHz, CDCl₃) δ 5.21 - 5.11 (m, 1H), 3.56-3.48 (m, 2H), 3.21 (s, 2H), 2.56-2.46 (m, 1H), 2.34 - 2.12 (m, 1H), 2.07 (s, 1H), 1.93 - 1.68 (m, 8H), 1.64-1.58 (m, 4H), 1.42 - 1.31 (m, 2H), 1.23-1.17 (m, 3H), 1.15-0.95 (m, 7H), 0.93 (s, 3H), 0.89 - 0.60 (m, 5H).

### Synthesis of I-S2

To a mixture of **I-S1** (5.5 g, 15.2 mmol) in THF (200 mL) was added 9-BBN dimmer (11.1 g, 45.6 mmol) at 15°C under N₂. After stirring at 50°C for 16 hours, the mixture was cooled to 15°C. NaOH aqueous (30.4 mL, 5 M, 152 mmol) was added dropwise below 15°C. H₂O₂ (17.2 g, 30%, 152 mmol) was added dropwise below 15°C. The mixture was extracted with EtOAc (2 x 100 mL). The combined organic phase was washed with sat.Na₂S₂O₃ (5 x 100 mL), dried over Na₂SO₄, filtered and concentrated to give 10 g of **I-S2**, which was used in next step directly.

### Synthesis of I-S3 & I-S3a

To a solution of **I-S2** (9 g, 23.7 mmol) in DCM (400 mL) was added DMP (30.1 g, 71.1 mmol). After stirring at 30°C for 30min, the reaction mixture was quenched with saturated NaHCO₃ and Na₂S₂O₃ solution (3 x 600 mL, v: v = 1:1). The organic phase was separated, dried over Na₂SO₄, filtered, concentrated and purified by combi-flash (0-30% of EtOAc in PE) to give **I-S3** & **I-S3a** (1.80 g) as an oil. The material was purified by column (0-10% of Acetone in PE: DCM= 1:1) to give pure **I-S3** (1.1 g) and a mixture of **I-S3** & **I-S3a** (2:1) (300 mg), which was separated by ELSD-HPLC ((column: Agela DuraShell 150mm_25mm_5um), gradient: 60-90% B (A= water(10mM NH₄HCO₃), B= MeCN), flow rate: 25 mL/min) to give **I-S3** (40 mg, 13%) and **I-S3a** (17 mg, 5.6%) as a solid.

### I-S3:

**¹H NMR** (400 MHz, CDCl₃) δ 3.57-3.49 (m, 2H), 3.21 (s, 2H), 2.34-2.26 (m, 1H), 2.14 (s, 3H), 2.08 (s, 1H), 1.87-1.55 (m, 10H), 1.51-1.23 (m, 4H), 1.22-1.15 (m, 5H), 1.11-0.91 (m, 8H), 0.88-0.62 (m, 4H); **MS ESI** calcd. For C₂₄H₃₉O₂ [M+H-H₂O]⁺ 359, found 359.

### I-S3a:

**¹H NMR** (400 MHz, CDCl₃) δ 3.57-3.47 (m, 2H), 3.20 (s, 2H), 2.47 (d, *J*=5.5 Hz, 1H), 2.12 (s, 3H), 2.03 (s, 1H), 1.85-1.58 (m, 9H), 1.56-1.23 (m, 6H), 1.21-1.18 (m, 4H), 1.17-1.02 (m, 2H), 1.00-0.73 (m, 9H), 0.69 - 0.55 (m, 1H); **MS ESI** calcd. For C₂₄H₃₉O₂ [M+H-H₂O]⁺ 359, found 359.

### Example I-71: Synthesis of (4aS,4bR,6aR,8R,10aS,10bR,12aS)-8-hydroxy-12a-methyl-N-phenyl-3,4,4a,4b,5,6,6a,7,8,9,10,10a,10b,11,12,12a-hexadecahydrochrysene-2-carboxamide (I-R23)

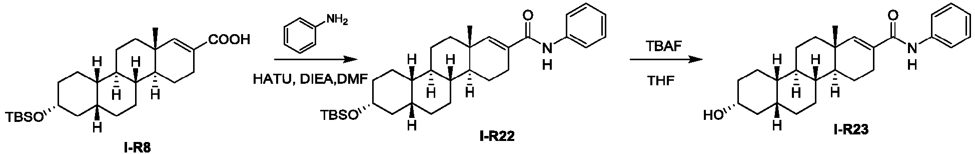

### Synthesis of I-R22

To a solution of **I-R8** (500 mg, 1.15 mmol) and aniline (214 mg, 2.30 mmol)in DMF (6 mL) was added HATU (1.30 g, 3.44 mmol) followed by DIPEA (2 mL). The reaction mixture was stirred at 15°C for 16 hours. The reaction was extracted with ethyl acetate (2 x 120 mL). The combined organic phase was washed with 1% LiCl aqueous (2 x 100 mL), brine (150 mL), dried over Na₂SO₄, filtered and concentrated under vacuum to give **I-R22** (600 mg) as a solid.

### Synthesis of I-R23

To a solution of **I-R22** (600 mg, 1.18 mmol) in THF (10 mL) was added TBAF (4.7 mL, 1.0 m in THF, 4.7 mmol) at 20°C. The reaction mixture was stirred at 60°C for 16 hours. The combined mixture was diluted with ethyl acetate (150 mL) and washed with water (2 x 100 mL), brine (100 mL), dried over Na₂SO₄, filtered and concentrated under vacuum to give R23 (630 mg) as an oil, which was purified by flash column chromatography (ethyl acetate in PE, 20%) to give **I-R23** (390 mg) as a solid. **I-R23** (20 mg) was further purified by prep-HPLC ((column: YMC-Actus Triart C18 100*30mm*5um), gradient: 7.5 min 70-93% (A= water(0.05%HCl), B= MeCN), flow rate: 25 mL/min) to give **I-R23** (7.7 mg) as a solid.

**¹H NMR** (400 MHz, CDCl₃) δ_{H} 7.56 (d, *J =* 7.6 Hz, 2H), 7.38 (s, 1H), 7.33 (t, *J =* 7.6 Hz, 2H), 7.13 - 7.06 (m, 1H), 6.43 (s, 1H), 3.70 - 3.59 (m, 1H), 2.56 - 2.46 (m, 1H), 2.36 - 2.23 (m, 1H), 2.00 - 1.91 (m, 2H), 1.82 - 1.54 (m, 10H), 1.41 - 1.03 (m, 10H), 0.95 (s, 3H); **MS ESI** calcd. For C₂₆H₃₆NO₂ [M+H]⁺ 394, found 394.

### Example I-72 and I-72a: Synthesis of (2S,4aS,4bR,6aR,8R,10aS,10bR,12aR)-8-hydroxy-12a-methyl-N-phenyloctadecahydrochrysene-2-carboxamide (I-R24) & (2R,4aS,4bR,6aR,8R,10aS,10bR,12aR)-8-hydroxy-12a-methyl-N-phenyloctadecahydrochrysene-2-carboxamide (I-R24a)

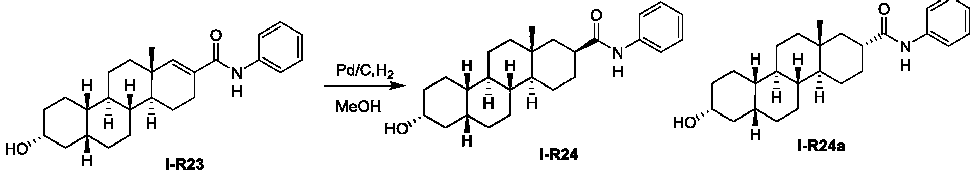

A mixture of **I-R23** (330 mg, 0.8 mmol) and Pd/C (100 mg, dry) in MeOH (15 mL) was stirred at 20°C under H₂ (103421 Pa = 15 psi) for 1 hour. The reaction mixture was filtered and concentrated under vacuum to give **I-R24** & **I-R24a** (330 mg) as a solid. The material (210 mg) was purified by SFC (column: Phenomenex-Cellulose-2 (250mm*30mm,5um), gradient: 40-40% B (A= 0.1%NH3H2O, B= EtOH), flow rate: 50 mL/min) to give **I-R24** (Peak 1, 145 mg, 69%) and **I-R24a** (Peak 2, 32 mg, 15%) as a solid.

### I-R24:

**¹H NMR** (400 MHz, CDCl₃) δ_{H} 7.49 (d, *J =* 8.0 Hz, 2H), 7.32 (t, *J =* 7.6 Hz, 2H), 7.13 - 7.07 (m, 1H), 3.70 - 3.56 (m, 1H), 2.68 - 2.59 (m, 1H), 2.38 - 2.27 (m, 1H), 2.00 - 1.88 (m, 2H), 1.77 - 1.56 (m, 11H), 1.52 - 0.86 (m, 13H), 0.82 (s, 3H); **MS ESI** calcd. For C₂₆H₃₈NO₂ [M+H] ⁺ 396, found 396.

### I-R24a:

**¹H NMR** (400 MHz, CDCl₃) δ_{H} 7.52 (d, *J =* 7.6 Hz, 2H), 7.31 (t, *J =* 7.6 Hz, 2H), 7.13 - 7.06 (m, 2H), 3.68-3.59 (m, 1H), 2.52-2.43 (m, 1H), 2.08 - 1.92 (m, 2H), 1.86 - 1.57 (m, 9H), 1.53 - 1.14 (m, 10H), 1.14 - 0.90 (m, 5H), 0.88 (s, 3H); **MS ESI** calcd. For C₂₆H₃₈NO₂ [M+H]⁺ 396, found 396.

### Example I-73: (2S,4aS,4bR,6aR,8R,10aS,10bR,12aR)-8-hydroxy-8,12a-dimethyl-N-phenyloctadecahydrochrysene-2-carboxamide (I-R26)

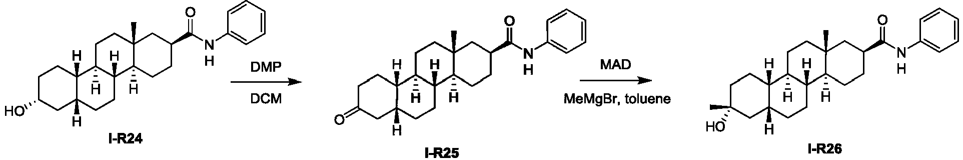

### Synthesis of I-R25

To a solution of **I-R24** (100 mg, 0.3 mmol) in DCM (5 mL) was added DMP (214 mg, 0.5 mmol) at 15°C. The reaction mixture was stirred for 1h at 15°C. The reaction mixture was added into saturated NaHCO₃ (50 mL) and stirred for 5 min. The aqueous layer was extracted with DCM (2 x 50 mL). The combined organic layer was washed with saturated Na₂SO₃ (3 x 50 mL), saturated brine (50 mL), dried over anhydrous Na₂SO₄, filtered and concentrated to give **I-R25** (100 mg) as an oil.

**¹H NMR** (400 MHz, CDCl₃) δ_{H} 7.55-7.45 (m, 2H), 7.40-7.30 (m, 2H), 7.15-7.03 (m, 1H), 3.70-3.55 (m, 1H), 2.68-2.58 (m, 1H), 2.39-2.26 (m, 1H), 1.99-1.83 (m, 1H), 1.34-1.18 (m, 12H), 1.16-0.84 (m, 11H), 0.82 (s, 3H).

### Synthesis of I-R26

To a solution of MAD (0.8 mmol) in toluene was added **I-R25** (100 mg, 0.3 mmol) at -78°C. The reaction mixture was stirred for 1 h at -78°C. Then MeMgBr (6.0 mmol, 2 mL, 3M in Et₂O) was added to the reaction mixture at -78°C. The reaction mixture was stirred for 1h at - 78°C. The reaction mixture was added into ice-cooled aqueous citric acid (50 mL). The aqueous layer was extracted with EtOAc (3 x 50 mL). The combined organic layer was washed with saturated brine (100 mL), dried over anhydrous Na₂SO₄, filtered and concentrated to give a product. The residue was purified by combi flash (0-10% of EtOAc in PE) to give **I-R26** (50 mg) as a solid. The material (50 mg) was triturated by hexane (5 mL) to give R26 (10 mg, 20%) as a solid.

**¹H NMR** (400 MHz, CDCl₃) δ_{H} 7.55-7.45 (m, 2H), 7.40-7.30 (m, 2H), 7.15-7.03 (m, 1H), 2.69-2.56 (m, 1H), 2.40-1.90 (m, 5H), 1.80-1.58 (m, 7H), 1.51-1.24 (m, 9H), 1.22 (s, 3H), 1.21-0.98 (m, 3H), 0.96-0.84 (m, 3H), 0.82 (s, 3H); **MS ESI** calcd. for C₂₇H₄₀NO₂[M+H]⁺ 410, found 410.

### Example I-74: 1-((1R,2R4aS,4bR,6aR,8R,10aS,10bR,12aS)-8-hydroxy-2,8,12a-trimethyloctadecahydrochrysen-1-yl)ethan-1-one (I-T6a)

### Synthesis of I-T1

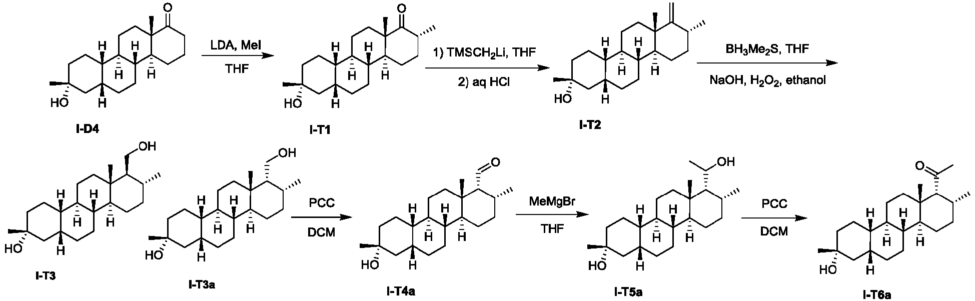

To a solution of **I-D4** (4.9 mmol) in THF (30 mL) was added LDA (24.6 mmol) at -78°C. The reaction mixture was stirred at -78°C for 1h. MeI (3.49 g, 24.6 mmol) was added into the reaction mixture at -78°C. Then the reaction mixture was stirred at 15°C for 16 hours. Water (50 mL) was poured to the mixture. The mixture was extracted with EtOAc (3 x 100 mL). The combined organic layer was washed with saturated brine (2 x 100 mL), filtered and concentrated under reduced pressure. The residue was purified by silica gel chromatography (0-45% of EtOAc in PE) to afford **I-T1** (800 mg, 51.2%) as a solid.

**¹H NMR** (400 MHz, CDCl₃) δ_{H} 2.75-2.55 (m, 1H), 2.10-2.05 (m, 1H), 2.00-1.95 (m, 1H), 1.90-1.60 (m, 12H), 1.51-1.20 (m, 6H), 1.15-0.70 (m, 13H).

### Synthesis of I-T2

To a solution of TMSCH₂Li (44.6 mL, 25.0 mmol, 0.56 M in hexane) in THF (20 mL) was added dropwise a solution of **I-T1** (1.6 g, 5.0 mmol) in THF (20 mL) at -40°C. After addition, the resulting mixture was allowed to warm to 30°C and stirred for 16 hrs. The reaction mixture concentrated and dissolved in MeOH (20 mL). P-TsOH (500 mg) was added into the mixture and stirred for 10min at 20°C. The reaction mixture was added into saturated NaHCO₃ (100 mL). The aqueous layer was extracted with EtOAc (3 x 50 mL). The combined organic layer was washed with saturated brine (100 mL), dried over anhydrous Na₂SO₄, filtered and concentrated. The residue was purified by flash column (0-8% of EtOAc in PE) to give **I-T2** (1.5 g, 95%) as an oil.

**¹H NMR** (400 MHz, CDCl₃) δ_{H} 4.88-4.59 (m, 2H), 2.38-2.25 (m, 1H), 2.00-1.60 (m, 8H), 1.52-1.28 (m, 6H), 1.26 (s, 3H), 1.24-1.98 (m, 9H), 0.95 (s, 3H), 0.93-0.75 (m, 4H).

### Synthesis of I-T3 and I-T3a

To a solution of **I-T2** (1.5 g, 4.7 mmol) in THF (20 mL) was added BH₃.Me₂S (2.4 mL, 23.9 mmol, 10 M) dropwise at 0°C. The reaction mixture was stirred at 15°C for 3 hours. The reaction mixture was cooled to 0°C. Ethanol (2.17 g, 47.3 mmol) was added dropwise at 0°C. NaOH (10 mL, 5M, 50.0 mmol) was added dropwise followed by 30% H₂O₂ (5 g, 52.6 mmol) at 0°C. The suspension was stirred at 70°C for 1 hour. The mixture was extracted with EtOAc (3 x 50 mL). The combined organic phase was washed with saturated aqueous Na₂S₂O₃ solution (2 x 50 mL) and saturated brine (100 mL), dried over anhydrous Na₂SO₄, filtered and concentrated. The residue was purified by flash column (0-20% of EtOAc in PE) to give **I-T3** (300 mg), **I-T3a** (800 mg, 51%) and **I-T3a** (200 mg) as an oil.

**¹H NMR** (400 MHz, CDCl₃) δ_{H} 3.85-3.64 (m, 3H), 1.93-1.28 (m, 15H), 1.25 (s, 3H), 1.23-0.75 (m, 17H).

### Synthesis of I-T4a

To a solution of **I-T3a** (400 mg, 1.2 mmol) in DCM (5 mL) was added silica gel (1 g) and PCC (511 mg, 2.4 mmol) at 15°C. The reaction mixture was stirred for 0.5h at 15°C. The mixture was filtered and the filter cake was washed with DCM (3 x 50 mL). The mother liquor was concentrated to give **I-T4a** (500 mg) as an oil.

**¹H NMR** (400 MHz, CDCl₃) δ_{H} 10.10-9.95 (m, 1H), 1.95-1.59 (m, 15H), 1.53-1.29 (m, 5H), 1.26 (s, 3H), 1.23-0.75 (m, 12H).

### Synthesis of I-T5a

To a solution of **I-T4a** (500 mg, 1.5 mmol) in THF (5 mL) was added bromo(methyl)magnesium (5 mL, 3M in Et₂O, 15 mmol) at 0°C. The reaction mixture was stirred for 1h at 20°C. The reaction mixture was added into saturated NH₄Cl (50 mL). The aqueous layer was extracted with EtOAc (3 x 50 mL). The combined organic layer was washed with saturated brine (2 x 100 mL), dried over anhydrous Na₂SO₄, filtered and concentrated to give **I-T5a** (300 mg) as an oil.

**¹H NMR** (400 MHz, CDCl₃) δ_{H} 3.09-3.02 (m, 1H), 1.95-1.59 (m, 8H), 1.53-1.28 (m, 12H), 1.26 (s, 3H), 1.23-0.75 (m, 16H).

### Synthesis of I-T6a

To a solution of **I-T5a** (300 mg) in DCM (5 mL) was added silica gel (500 mg) and PCC (369 mg, 1.7 mmol) at 15°C. The reaction mixture was stirred for 1h at 15°C. The reaction mixture was filtered and the filter cake was washed by DCM (3 x 50 mL). The mother liquor was concentrated. The residue was purified by flash column (0-5 % of EtOAc in PE) to give **I-T6a** (32 mg, 11%) as a solid. The structure was confirmed by NOE. (1. H18 and H19 showed signal; 2. H18 and H14 showed no signal. Thus, H18 and H19 should be cis-position.)

**¹H NMR** (400 MHz, CDCl₃) δ_{H} 2.57-2.52 (m, 1H), 2.13 (s, 3H), 2.04-1.91 (m, 1H), 1.89-1.59 (m, 9H), 1.53-1.26 (m, 11H), 1.24 (s, 3H), 1.22-0.96 (m, 3H), 0.94 (s, 3H), 0.92-0.76 (m, 4H); **MS ESI** calcd. for C₂₃H₃₇O[M+H-H₂O]⁺ 329, found 329.

### EXAMPLE I-75: Synthesis of 1-((1S,4aS,4bR,6aR,8R,10aS,10bR,12aS)-8-(ethoxymethyl)-8-hydroxy-12a-methyloctadecahydrochrysen-1-yl)ethan-1-one (I-U11)

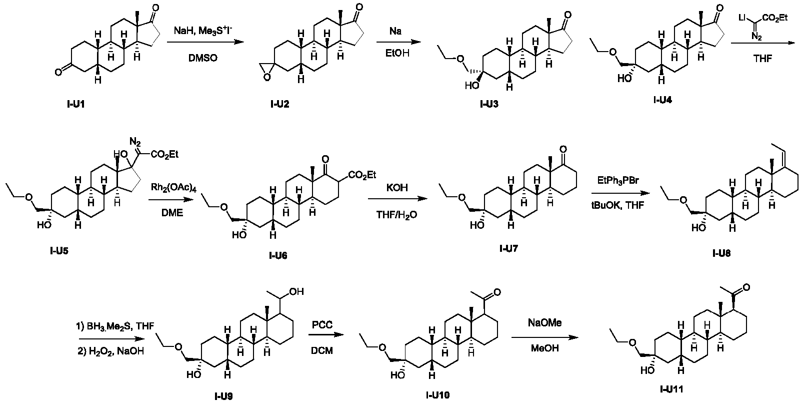

### Synthesis of I-U2

To a stirred solution of trimethylsulfonium iodide (16.8 g, 87.3 mmol) in DMSO (100 mL) and THF (50 mL) was added NaH (8.71 g, 218 mmol, 60 % in oil) at 0°C. After stirring for 1.0 h under N₂. a solution of **I-U1** (50 g, 182 mmol, reported in "WO2014/169833, 2014, A1") in DMSO (200 mL) at 0°C. After stirring at 25°C for 16 h, the reaction was diluted with water (500 mL) and extracted with EtOAc (2 x 300 mL). The combined organic solution was washed with water (2 x 300 mL), brine (300 mL), dried over anhydrous Na₂SO₄, filtered and concentrated in vacuum to give **I-U2** (45 g) as an oil. **¹H NMR** (400 MHz, CDCl₃) δ_{H} 2.64-2.56 (m, 2H), 2.53-2.40 (m, 1H), 2.30-2.15 (m, 3H), 2.10-1.65 (m, 7H), 1.60-1.35 (m, 6H), 1.25-1.05 (m, 4H), 1.05-0.80 (m, 5H).

### Synthesis of I-U3 & I-U4

To anhydrous EtOH (400 mL) was added Na (17.9 g, 780 mmol) at 25°C. After stirring at 75°C for 1h, a solution of **I-U2** (45 g, 156 mmol) in anhydrous ethanol (200 mL) was added. After stirring at 75°C for 16 h, the reaction was diluted with water (200 mL), concentrated to remove most of the solvent, and extracted with EtOAc (2 x 300 mL). The combined organic solution was washed with saturated brine (300 mL), dried over anhydrous Na₂SO₄, filtered and concentrated, to give mixture of **I-U3** and **I-U4** (46 g) as an oil. The residue was purified by silica gel chromatography (PE/EtOAc = 5:1) to afford **I-U3** (18 g, 39%) and **I-U4** (27 g, 59 %) as solids.
**I-U4: ¹H NMR** (400 MHz, CDCl₃) δ_{H} 3.53 (q, *J =* 7.2 Hz, 2H), 3.43 (q, *J =* 9.6 Hz, 2H), 2.71 (s, 1H), 2.43 (dd, *J =* 8.8, 19.2 Hz, 1H), 2.16-2.00 (m, 1H), 1.99-1.60 (m, 8H), 1.55-1.26 (m, 10H), 1.25-1.00 (m, 6H), 0.86 (s, 3H)
**I-U3: ¹H NMR** (400 MHz, CDCl₃) δ_{H} 3.53 (q, *J =* 7.2 Hz, 2H), 3.27-3.17 (m, 2H), 2.44 (dd, *J* = 8.4, 20.0 Hz, 1H), 2.25-2.15(m, 1H), 2.12-2.00 (m, 2H), 1.98-1.69 (m, 6H), 1.54-1.25 (m, 9H), 1.24-1.09 (m, 8H), 0.87 (s, 3H)

### Synthesis of I-U5

To freshly prepared LDA (made from DIPA (25.7 g, 254 mmol) and n-BuLi (101 mL, 2.5 M in hexane, 254 mmol)) was added to a solution of **I-U4** (17 g, 50.8 mmol) and ethyl diazoacetate (32.1 g, 254 mmol, 90%) in THF (140 mL) at -78°C. After stirring at -70°C for 2 h, acetic acid (254 mmol) in THF (100 mL) was added. After warming to rt, the reaction was diluted with water (200 mL) and extracted with EtOAc (3 x 200 mL). The combined organic solutions were washed with brine (200 mL), dried over anhydrous Na₂SO₄, filtered and concentrated. The residue was purified by silica gel chromatography (PE/EtOAc = 5:1) to afford **I-U5** (14 g, 70 %) as an oil. **¹H NMR** (400 MHz, CDCl₃) δ_{H} 4.26-4.15 (m, 2H), 3.69 (dd, *J =* 6.0*,* 13.6 Hz, 1H), 3.52 (q, *J =* 10.4 Hz, 2H), 3.41 (q, *J =* 9.2 Hz, 2H), 2.25-2.15 (m, 1H), 2.05-1.60 (m, 9H), 1.50-1.24 (m, 9H), 1.24-1.10 (m, 8H), 1.10-0.87 (m, 4H)

### Synthesis of I-U6

To a solution of **I-U5** (14 g) in DME (100 mL) was added Rh₂(OAc)₄ (206 mg, 467 µmol) at 30°C. After stirring at 30 °C for 12 h, the reaction mixture was extracted with ethyl acetate (2 x 200 mL). The combined organic solution was washed with water (2 x 200 mL), brine (2 x 200 mL), dried over Na₂SO₄, filtered and concentrated under vacuum to give **I-U6** (12 g) as an oil. **¹H NMR** (400 MHz, CDCl₃) δ_{H} 12.42 (s, 1H), 4.20 (q, *J =* 7.2 Hz, 2H), 3.53 (q, *J =* 6.8 Hz, 2H), 3.43 (q, *J =* 8.8 Hz, 2H), 2.70 (br, 1H), 2.30-2.36 (m, 1H), 2.18-2.02 (m, 2H), 1.99-1.57 (m, 8H), 1.55-1.24 (m, 10H), 1.24-1.12 (m, 5H), 1.12-0.92 (m, 6H)

### Synthesis of I-U7

To a solution of **I-U6** (12 g, 3.5 mmol) in MeOH (100 mL), THF (50 mL) and H₂O (50 mL) was added KOH (7.96 g, 142 mmol) at 20°C. After stirring at 70°C for 2 h, the reaction mixture was poured into brine (500 mL) and extracted with EtOAC (3 x 200 mL). The combined organic solutions were washed with saturated NaHCO₃ (200 mL) and brine (200 mL), dried over anhydrous Na₂SO₄, filtered and concentrated. The residue was purified by flash column (0-10% of EtOAc in PE) to give **I-U7** (6 g, 75 %) as a solid. **¹H NMR** (400 MHz, CDCl₃) δ_{H} 3.52 (q, *J =* 7.2 Hz, 2H), 3.42 (q, *J* = 9.2 Hz, 2H), 2.72-2.54 (m, 2H), 2.19 ( dd, *J* = 1.6, 13.2 Hz, 1H), 2.10-1.99 (m, 2H), 1.90-1.64 (m, 6H), 1.55-1.29 (m, 8H), 1.28 - 1.14 (m, 7H), 1.07 (s, 3H), 1.04 - 0.82 (m, 3H)

### Synthesis of I-U8

To a suspension of Ph₃PEtBr (50.8 g, 137 mmol) in anhydrous THF (150 mL) was added t-BuOK (15.3 g, 173 mmol) at 25°C under N₂. After stirring at 60°C for 30 min, a solution of **I-U7** (8.0 g, 22.9 mmol) in anhydrous THF (50 mL) was added dropwise. After stirring at 60°C for 16 h, the mixture was poured into saturated NH₄Cl (500 mL) and extracted with EtOAc (2 x 200 mL). The combine organic solution was washed with saturated brine (2 x 200 mL), filtered and concentrated. The residue was purified by flash column (0-10% of EtOAc in PE) to give **I-U8** (6 g, 75 %) as an oil. **¹H NMR** (400 MHz, CDCl₃) δ_{H} 5.21-5.09 (m, 1H), 3.53 (q, *J =* 6.8 Hz, 2H), 3.43 (q, *J =* 7.6 Hz, 2H), 2.67-2.43 (m, 1H), 2.30-1.82 (m, 3H), 1.81-1.68 (m, 8H), 1.53-1.50 (s, 2H), 1.46-1.17 (m, 12H), 1.14-0.83 (m, 9H)

### Synthesis of I-U9

To a solution of **I-U8** (3.0 g, 8.3 mmol) in THF (30 mL) was addded BH₃.Me₂S (2.5 mL, 10 M, 25.0 mmol). After stirring at 45°C for 1 h, ethanol (3.8 g, 83.1 mmol) was added at 15°C, followed by NaOH aqueous (16.6 mL, 5.0 M, 83.1 mmol) and then H₂O₂ (8.31 mL, 10 M, 83.1 mmol) dropwise. After stirring at 78°C for 1 h, the mixture was cooled to 15°C, diluted with water (150 mL) and extracted with EtOAc (2 x 100 mL). The combined organic solution was washed with saturated brine (2 x 200 mL), drive over anhydrous Na₂SO₄, filtered and concentrated in vacuum to give **I-U9** (3.0 g) as a solid.

### Synthesis of I-U10

To a solution of **I-U9** (3 g, 7.9 mmol) in DCM(100 mL) and was added PCC(3.4 g, 15.8 mmol) and silica gel (4 g). After stirring at 25°C for 2 h, the precipitate was filtered and the filtrate was concentrated. The residue was purified by flash column (0-30% of EtOAc in PE) to give **I-U10** (2.0 g, 69 %) as an oil. **¹H NMR** (400 MHz, CDCl₃) δ_{H} 3.54-3.48 (m, 2H), 3.46-3.36 (m, 2H), 2.75-2.55 (m, 1H), 2.50-2.35 (m, 1H), 2.15-2.11 (m, 3H), 1.88-1.61 (m, 8H), 1.52-1.28 (m, 8H), 1.27-1.15 (m, 7H), 1.10-0.94 (m, 3H), 0.91 (s, 3H)

### Synthesis of I-U11

To a solution of **I-U10** (2 g, 5.3 mmol) in MeOH (100mL) was added MeONa (4.3 g, 79.6 mmol). After stirring for 72 h at 70°C, the reaction mixture was concentrated. The residue was diluted with ethyl acetate (60 mL) and washed with water (50 mL) and brine (50 mL), dried over Na₂SO₄, filtered and concentrated. The residue was purified by flash column (0-30% of EtOAc in PE) to give **I-U11** (70 mg) as a solid. **¹H NMR** (400 MHz, CDCl₃) δ_{H} 3.53 (*q, J =* 6.4 Hz, 2H), 3.42 (q, *J=* 9.2 Hz, 2H), 2.71 (s, 1H), 2.30 (d, *J=* 12.8 Hz, 1H), 2.14 (s, 3H), 1.88-1.60 (m, 8H), 1.56-1.30 (m, 9H), 1.29-1.13 (m, 6H), 1.07-0.85 (m, 8H); **LC-ELSD/MS** purity 99%, MS ESI calcd. for C₂₄H₃₉O₂ [M-H₂O+H]⁺ 359.3, found 359.3

### Example 1-101 and I-101a: Synthesis of (1S,2aS,2bR,4aR,6R,8aS,8bR,10aS)-6-hydroxy-6,10a-dimethyl-N-phenylhexadecahydrocyclobuta[a]phenanthrene-1-carboxamide (I-AA6) & (1R,2aS,2bR4aR,6R,8aS,8bR,10aS)-6-hydroxy-6,10a-dimethyl-N-phenylhexadecahydrocyclobuta[a]phenanthrene-1-carboxamide (I-AA6a)

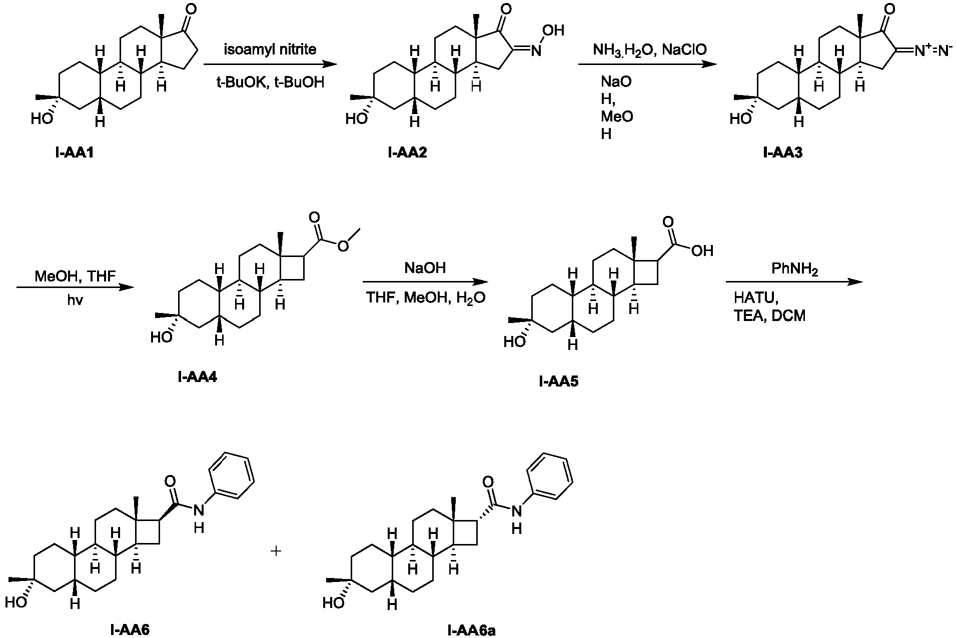

### Synthesis of I-AA2

To a solution of t-BuOK (22.1 g, 197 mmol) in t-BuOH (360 mL) was added **I-AA1** (7.2 g, 24.7 mmol, reported in patent 'WO2014/169833, 2014, A1') and isoamyl nitrite (11.5 g, 98.8 mmol). The mixture was stirred at 30°C for 1 h. To the mixture was added saturated NH₄Cl (100 mL). The mixture was extracted with EtOAc (500 mL). The organic layer was washed with water (200 mL), dried over Na₂SO₄, concentrated in vacuum and the residue was triturated from PE (500 mL) to give **I-AA2** (7.0 g, 88%) as a solid.

**¹H NMR** (400 MHz, CD₃OD) δ_{H} 2.86 (dd, *J* = 6.8, 17.6 Hz, 0.7H), 2.60-2.53 (m, 0.2H), 2.30-2.20 (m, 0.2H), 2.11 (dd, *J =* 13.2, 17.6 Hz, 0.7H), 1.95-1.65 (m, 5H), 1.60-1.35 (m, 10H), 1.30-1.10 (m, 7H), 1.05-0.85 (m, 3H).

### Synthesis of I-AA3

To a solution of **I-AA2** (5.0 g, 15.6 mmol) in MeOH (500 mL) was added NaOH (50 mL, 5 M aq), NH₃ (75 mL, 14 M aq.) at 20°C. NaClO (150 mL, 10% aq.) was added dropwise within 1 h. The mixture was stirred at 20°C for 4 h. The mixture was combined with another batch from 2.0 g of **I-AA2.** The mixture was concentrated to remove most MeOH and to the mixture was added saturated NH₄Cl (500 mL). The mixture was filtered. The solid was washed with water, dissolved in DCM (50 mL), dried over Na₂SO₄, filtered and purified by flash column (20~40% EtOAc in PE) to give **I-AA3** (3.5 g, 50%) as a solid.

**¹H NMR** (400 MHz, CDCl₃) δ_{H} 2.83 (dd, *J* = 6.8, 12.4 Hz, 1H), 2.58 (t, *J =* 12.0 Hz, 1H), 1.90-1.70 (m, 5H), 1.70-1.30 (m, 11H), 1.30-1.20 (m, 4H), 1.20-1.05 (m, 3H), 0.96 (s, 3H).

### Synthesis of I-AA4

A solution of **I-AA3** (500 mg, 1.6 mmol) in THF (60 mL) and MeOH (20 mL) under N₂ was irradiated with 300 W low pressure mercury lamps for 6 h at 20°C. The mixture was concentrated and purified by flash column (0~30% EtOAc in PE) to give **I-AA4** (450 mg, 89%, C-16 isomer mixture, the ratio is about 3: 1) as an oil.

**¹H NMR** (400 MHz, CDCl₃) δ_{H} 3.70 (s, 0.7H), 3.65 (s, 2.2H), 2.30-2.20 (m, 1H), 2.15-1.95 (m, 2H), 1.85-1.30 (m, 16H), 1.30-1.05 (m, 7.8H), 0.96 (s, 2.2H).

### Synthesis of I-AA5

To a solution of **I-AA4** (450 mg, 1.4 mmol, C-16 isomer mixture) in THF (5 mL) was added MeOH (5 mL) and NaOH (5 mL, 2 M aq, 10 mmol). The mixture was stirred at 20°C for 20 h. The mixture was concentrated in vacuum to remove most organic solvent and to the mixture was added HCl (6 mL, 2 M aq.). The mixture was extracted with EtOAc (10 mL). The organic layer was separated, dried over Na₂SO₄, filtered and concentrated in vacuum to give **I-AA5** (400 mg, 93%, C-16 isomer mixture) as a foaming-like solid.

**¹H NMR** (400 MHz, CDCl₃) δ_{H} 2.30-2.20 (m, 1H), 2.20-1.95 (m, 2H), 1.85-1.55 (m, 7H), 1.55-1.30 (m, 8H), 1.30-1.15 (m, 8H), 1.15-0.95 (m, 4H).

### Synthesis of I-AA6 & I-AA6a

To a solution of **I-AA5** (200 mg, 0.65 mmol, C-16 isomer mixture) in DCM (2 mL) was added aniline (121 mg, 1.3 mmol), TEA (329 mg, 3.3 mmol) and HATU (494 mg, 1.3 mmol). The mixture was stirred at 25°C for 16 h. The mixture was concentrated and purified by flash column (15~50% EtOAc in PE) to give 140 mg of **I-AA6** as a solid and 100 mg of **I-AA6a** as an oil. Crude **I-AA6** (140 mg) was triturated with MeCN (10 mL) to give **I-AA6** (69 mg, 28%) as a solid. The absolute structure of **I-AA6** was determined by NOE (H16 was correlated with H14; H16 was not correlated with H18). Crude **I-AA6a** (100 mg) was purified by HPLC and lyophilized to give **I-AA6a** (20 mg, 8%) as a solid. The absolute structure of **I-AA6a** was determined by NOE (H15-β was correlated with H16 and H18; H16 was correlated with H18).

### I-AA6:

**¹H NMR** (400 MHz, CDCl₃) δ_{H} 7.50 (d, *J =* 8.0 Hz, 2H), 7.38-7.27 (m, 2H), 7.09 (t, *J =* 7.6 Hz, 1H), 6.88 (br.s., 1H), 2.78 (dd, *J =* 6.4, 8.8 Hz, 1H), 2.25-2.15 (m, 1H), 2.10-2.00 (m, 1H), 1.85-1.75 (m, 4H), 1.70-1.60 (m, 3H), 1.55-1.35 (m, 8H), 1.35-1.10 (m, 8H), 1.06 (s, 3H); **MS ESI** calcd. for C₂₅H₃₆NO₂ [M+H]⁺ 382, found 382.

### I-AA6a:

**¹H NMR** (400 MHz, CDCl₃) δ_{H} 7.54 (d, *J =* 7.6 Hz, 2H), 7.32 (t, *J =* 7.6 Hz, 2H), 7.09 (t, *J =* 7.2 Hz, 1H), 6.98 (br.s., 1H), 2.74 (dd, *J =* 6.0 Hz, 1H), 2.40-2.30 (m, 1H), 2.25-2.05 (m, 2H), 1.70-1.55 (m, 5H), 1.55-1.30 (m, 8H), 1.30-1.05 (m, 12H); **MS ESI** calcd. for C₂₅H₃₆NO₂ [M+H]⁺ 382, found 382.

### Example I-102 and I-102a: Synthesis of 1-((1S,2aS,2bR,4aR,6R,8aS,8bR,10aS)-6-hydroxy-6,10a-dimethylhexadecahydrocyclobuta[a]phenanthren-1-yl)ethan-1-one (I-AA8) & 1-((1R,2aS,2bR,4aR,6R,8aS,8bR,10aS)-6-hydroxy-6,10a-dimethylhexadecahydrocyclobuta[a]phenanthren-1-yl)ethan-1-one (I-AA8a)

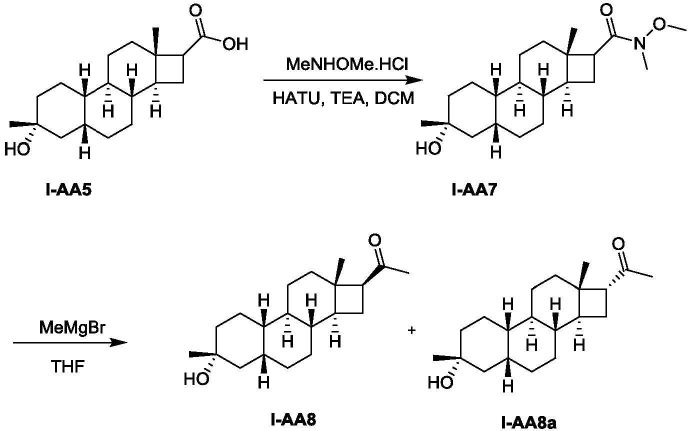

### Synthesis of I-AA7

To a solution of **I-AA5** (1.2 g, 3.91 mmol) in DCM (15 mL) was added MeONHMe.HCl (762 mg, 7.82 mmol), TEA (2.36 g, 23.4 mmol) and HATU (2.97 g, 7.82 mmol). The mixture was stirred at 25°C for 20 h. The mixture was combined with another batch from 0.2 g **I-AA7.** The mixture was washed with water (20 mL), dried over Na₂SO₄, filtered and purified by flash column (20~50% EtOAc in PE) to give **I-AA7** (1.7 g) as an oil.

**¹H NMR** (400 MHz, CDCl₃) δ_{H} 3.70-3.55 (m, 3H), 3.20-3.10 (m, 3H), 2.30-1.95 (m, 2H), 1.90-1.30 (m, 16H), 1.30-0.85 (m, 11H).

### Synthesis of I-AA8 & I-AA8a

To a solution of **I-AA7** (1.5 g, 4.3 mmol) in THF (40 mL) was added MeMgBr (7.1 mL, 21.4 mmol) at 0°C. The mixture was stirred at 0°C for 2 h. The mixture was quenched by saturated NH₄Cl (30 mL) and extracted with EtOAc (100 mL). The organic layer was separated, concentrated and purified by flash column (10~25% EtOAc in PE) to give **I-AA8** (700 mg, 54%) and **I-AA8a** (200 mg, 15%) both as solid. The absolute structure of **I-AA8** was determined by NOE (H16 was correlated with H14; H18 was correlated with H21). The absolute structure of **I-AA8a** was determined by NOE (H16 was correlated with H18.).

### I-AA8:

**¹H NMR** (400 MHz, CDCl₃) δ_{H} 2.80 (dd, *J =* 6.0, 9.2 Hz, 1H), 2.15-2.05 (m, 1H), 2.01 (s, 3H), 1.90-1.75 (m, 5H), 1.70-1.55 (m, 3H), 1.50-1.35 (m, 8H), 1.30-1.05 (m, 8H), 0.89 (s, 3H); **MS ESI** calcd. for C₂₀H₃₁O [M+H]⁺ 287, found 287.

### I-AA8a:

**¹H NMR** (400 MHz, CDCl₃) δ_{H} 2.94 (dd, *J =* 6.0 Hz, 1H), 2.25-2.15 (m, 1H), 2.07 (s, 3H), 1.90-1.75 (m, 5H), 1.65-1.30 (m, 11H), 1.30-1.05 (m, 11H); **MS ESI** calcd. for C₂₀H₃₁O [M+H]⁺ 287, found 287.

### Example I-103 and I-103a: Synthesis of 1-((1S,2aS,2bR,4aR,6R,8aS,8bS,10aS)-6-hydroxy-6,8a,10a-trimethylhexadecahydrocyclobuta[a]phenanthren-1-yl)ethan-1-one (I-103) and 1-((1R,2aS,2bR,4aR,6R,8aS,8bS,10aS)-6-hydroxy-6,8a,10a-trimethylhexadecahydrocyclobuta[a]phenanthren-1-yl)ethan-1-one (I-103a)

The following examples were made similar using the procedures in **Example I-101** & **Example I-102** with the listed starting material

| **Example** | **Starting Material** | **Compound Name** | **¹H NMR (400MHz, CDCl₃)** | **MS ESI** |
|---|---|---|---|---|
| **I-103** | (3R,5R,8R,9S,10S, 13S,14S)-3-hydroxy-3,10,13-trimethylhexadeca hydro-17H-cyclopenta[a]phen anthren-17-one | 1-((1S,2aS,2bR,4aR,6 R,8aS,8bS, 10aS)-6-hydroxy-6,8a,10a-trimethylhexadecah ydrocyclobuta[a]ph enanthren-1-yl)ethan-1-one **(I-103**) | δ_{H} 2.79 (dd, J=5.8, 9.3 Hz, 1H), 2.00 (s, 6H), 1.89 - 1.61 (m, 6H), 1.40 (s, 8H), 1.26 (s, 5H), 1.05 - 0.99 (m, 1H), 0.96 (s, 3H), 0.87 (s, 3H) | calcd. for C₂₁H₃₃O [M-H2O+H ]⁺301, found 301. |
| **I-103a** | (3R,5R,8R,9S,10S, 13S,14S)-3-hydroxy-3,10,13-trimethylhexadeca hydro-17H-cyclopenta[a]phen anthren-17-one | 1-((1R,2aS,2bR,4aR,6 R,8aS,8bS, 10aS)-6-hydroxy-6,8a,10a-trimethylhexadecah ydrocyclobuta[a]ph enanthren-1-yl)ethan-1-one **(I-103a**) | δ_{H} 2.93 (d, J=6.5 Hz, 1H), 2.25 - 2.19 (m, 1H), 2.07 (s, 3H), 1.96 - 1.72 (m, 4H), 1.68 - 1.31 (m, 12H), 1.23 (d, J=5.3 Hz, 8H), 1.05 - 0.97 (m, 1H), 0.95 (s, 3H) | calcd. for C₂₁H₃₃O [M-H2O+H]⁺ 301, found 301 |

### Example I-104: Synthesis of 1-(2-((1S,2aS,2bR,4aR,6R,8aS,8bR,10aS)-6-hydroxy-6,10a-dimethylhexadecahydrocyclobuta[a]phenanthren-1-yl)-2-oxoethyl)-1H-pyrazole-4-carbonitrile (I-AA10)

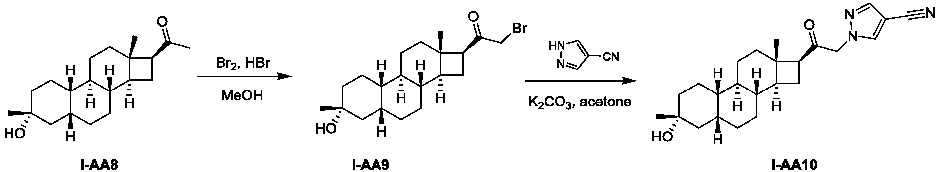

### Synthesis of I-AA9

To a solution of **I-AA8** (250 mg, 0.8 mmol) in MeOH (5 mL) was added HBr (33.2 mg, 0.16 mmol, 40% in water) and Br₂ (131 mg, 0.8 mmol) at 15°C. The mixture was stirred at 15°C for 2 hrs. The mixture was poured into saturated NaHCO₃ (10 mL), extracted with EtOAc (10 mL). The organic layer was separated, dried over Na₂SO₄, filtered and concentrated in vacuum to give **I-AA9** (350 mg) as a solid.

**¹H NMR** (400 MHz, CDCl₃) δ_{H} 3.90-3.70 (m, 2H), 3.13 (dd, *J =* 5.6, 9.2 Hz, 1H), 2.25-2.15 (m, 1H), 2.00-1.55 (m, 9H), 1.50-1.35 (m, 7H), 1.30-1.05 (m, 8H), 0.91 (s, 3H).

### Synthesis of I-AA10

To a solution of **I-AA9** (350 mg, 0.9 mmol) in acetone (10 mL) was added 4-cyanopyrazole (424 mg, 4.6 mmol) and K₂CO₃ (639 mg, 4.6 mmol). The mixture was stirred at 15°C for 16 h. To the mixture was added water (20 mL) and the mixture was extracted with EtOAc (50 mL). The organic layer was separated, concentrated, purified by flash column (25~50% EtOAc in PE) and triturated from hexane (30 mL) at 60°C. The mixture was filtered. The solid was washed with hexane (30 mL), dried in vacuum to give **I-AA10** (246 mg, 76 % above 2 steps) as a solid. The absolute structure of AA10 was determined by NOE (H18 was correlated with H15-β, H16 was correlated with H15-α.

**¹H NMR** (400 MHz, CDCl₃) δ_{H} 7.85 (s, 1H), 7.81 (s, 1H), 4.85 (s, 2H), 2.91 (dd, *J =* 6.0, 9.2 Hz, 1H), 2.23 (dt, *J* = 9.6, 12.4 Hz, 1H), 2.00-1.90 (m, 1H), 1.85-1.55 (m, 8H), 1.50-1.35 (m, 7H), 1.35-1.05 (m, 8H), 0.95 (s, 3H); **MS ESI** calcd. for C₂₄H₃₂N₃O [M+H-H₂O]⁺ 378, found 378.

### Example I-105, I-106, I-107 and I-107a: Synthesis of 1-(2-((1R,2aS,2bR,4aR,6R,8aS,8bR,10aS)-6-hydroxy-6,10a-dimethylhexadecahydrocyclobuta[a]phenanthren-1-yl)-2-oxoethyl)-1H-pyrazole-4-carbonitrile (I-105), 1-(2-((1S,2aS,2bR,4aR,6R,8aS,8bS,10aS)-6-hydroxy-6,8a,10a-trimethylhexadecahydrocyclobuta[a]phenanthren-1-yl)-2-oxoethyl)-1H-pyrazole-4-carbonitrile (I-106), 1-(2-((1S,2aS,2bR,4aR,6R,8aS,8bS,10aS)-6-hydroxy-6,8a,10a-trimethylhexadecahydrocyclobuta[a]phenanthren-1-yl)-2-oxoethyl)-1H-pyrazole-4-carbonitrile (I-107), 1-((1S,2aS,2bR,4aR,6R,8aS,8bS,10aS)-6-hydroxy-6,8a,10a-trimethylhexadecahydrocyclobuta[a]phenanthren-1-yl)-2-(1H-1,2,3-triazol-1-yl)ethan-1-one (I-107a).

The following examples were made similar to **Example I-23** with the listed ketone and ArylNH. Regioisomeric products such as **Example I-107** and **I-107a** were separated by flash chromatography.

| **Example** | **ketone** | **ArylN H** | **Compound Name** | **¹H NMR (400 MHz, CDCl₃)** | **MS ESI** |
|---|---|---|---|---|---|
| **I-105** | 1-((1R,2aS,2bR,4 aR,6R,8aS,8bR ,10aS)-6-hydroxy-6,10a-dimethylhexad ecahydrocyclob uta[a]phenanthr en-1-yl)ethan-1-one (**I**-AA8a) | 4-cyanop yrazole | 1-(2-((1R,2aS,2bR,4aR,6 R,8aS,8bR, 10aS)-6-hydroxy-6,10a-dimethylhexadecahy drocyclobuta[a]phen anthren-1-yl)-2-oxoethyl)-1H-pyrazole-4-carbonitrile **(I-105)** | δ_{H} 7.87 (s, 1H), 7.83 (s, 1H), 5.00-4.80 (m, 2H), 3.00-2.92 (m, 1H), 2.35-2.20 (m, 1H), 2.05-1.85 (m, 2H), 1.80-1.50 (m, 8H), 1.50-1.05 (m, 17H) | calcd. for C₂₄H₃₂N₃ O [M+H-H₂O]⁺ 378, found 378. |
| **I-106** | 1-((1 S,2aS,2bR,4 aR,6R,8aS,8bS, 10aS)-6-hydroxy-6,8a,10a-trimethylhexad ecahydrocyclob uta[a]phenanthr en-1-yl)ethan-1-one | 4-cyanop yrazole | 1-(2-((1S,2aS,2bR,4aR,6 R,8aS,8bS,10aS)-6-hydroxy-6,8a,10a-trimethylhexadecahy drocyclobuta[a]phen anthren-1-yl)-2-oxoethyl)-1H-pyrazole-4-carbonitrile (**I-106**) | δ_{H} 7.83 (d, *J*=15.8 Hz, 2H), 4.84 (d, *J*=1.0 Hz, 2H), 2.90 (dd, *J*=6.0, 9.3 Hz, 1H), 2.22 (d, *J*=11.3 Hz, 1H), 2.02 - 1.59 (m, 8H), 1.56 (s, 8H), 1.26 (s, 3H), 1.28 - 1.18 (m, 1H), 1.07 - 1.00 (m, 1H), 0.96 (s, 3H), 0.93 (s, 3H). | calcd. for C₂₅H₃₅N₃ O₂Na [M+Na]⁺ 432, found 432. |
| **I-107** | 1-((1 S,2aS,2bR,4 aR,6R,8aS,8bS, 10aS)-6-hydroxy-6,8a,10a-trimethylhexad ecahydrocyclob uta[a]phenanthr en-1-yl)ethan-1-one | 2H-1,2,3-triazole | 1-((1S,2aS,2bR,4aR,6 R,8aS,8bS,10aS)-6-hydroxy-6,8a,10a-trimethylhexadecahy drocyclobuta[a]phen anthren-1-yl)-2-(2H-1,2,3-triazol-2-yl)ethan-1-one **(I-107**) | δ_{H} 7.68 (s, 2H), 5.11 (d, *J*=15.3 Hz, 2H), 2.87 - 2.81 (m, 1H), 2.25 - 2.16 (m, 1H), 2.02 - 1.91 (m, 1H), 1.87 - 1.79 (m, 2H), 1.67 - 1.56 (m, 3H), 1.53 - 1.36 (m, 9H), 1.25 (s, 6H), 1.05 - 0.98 (m, 1H), 0.96 (d, *J*=3.3 Hz, 7H) | calcd. for C₂₃H₃₄N₃ O [M-H2O+H] ⁺ 368, found 368 |
| **I-107a** | 1-((1 S,2aS,2bR,4 aR,6R,8aS,8bS, 10aS)-6-hydroxy-6,8a,10a-trimethylhexad ecahydrocyclob uta[a]phenanthr en-1-yl)ethan-1-one | 2H-1,2,3-triazole | 1-((1S,2aS,2bR,4aR,6 R,8aS,8bS,10aS)-6-hydroxy-6,8a,10a-trimethylhexadecahy drocyclobuta[a]phen anthren-1-yl)-2-(1H-1,2,3-triazol-1-yl)ethan-1-one **(I-107a**) | δ_{H} 7.76 (s, 1H), 7.63 (s, 1H), 5.09 (s, 2H), 2.96 - 2.90 (m, 1H), 2.21 (s, 1H), 2.02 - 1.89 (m, 2H), 1.87 - 1.75 (m, 2H), 1.59 (br s, 5H), 1.38 (br s, 8H), 1.26 (s, 7H), 1.07 - 1.00 (m, 1H), 0.96 (s, 3H), 0.93 (s, 3H) | calcd. for C₂₃H₃₆N₃ O₂ [M+ H]⁺ 386, found 386 |

### Example I-108 and I-108a: Synthesis of (1S,2aS,2bR,4aR,6R,8aS,8bR,10aS)-1-(hydroxymethyl)-6,10a-dimethylhexadecahydrocyclobuta[a]phenanthren-6-ol (I-AB1) & (1R,2aS,2bR,4aR,6R,8aS,8bR,10aS)-1-(hydroxymethyl)-6,10a-dimethylhexadecahydrocyclobuta[a]phenanthren-6-ol (I-AB1a)

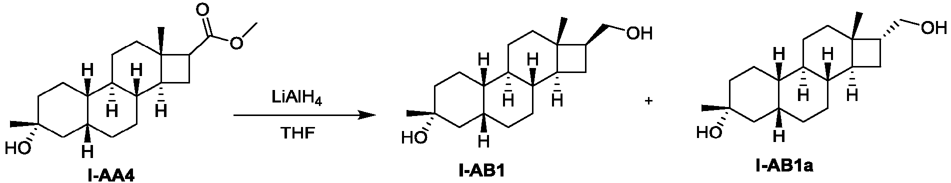

To a solution of **I-AA4** (900 mg, 2.8 mmol) in THF (30 mL) was added LiAlH₄ (1.06 g, 28 mmol) at 0°C. The mixture was stirred at 0°C for 1 h. The mixture was poured into water (50 mL) at 0°C and then HCl (60 mL, 2 M aq.) was added. The mixture was extracted with EtOAc (100 mL). The organic layer was separated, dried over Na₂SO₄, filtered and concentrated. The residue was triturated with MeCN (50 mL) to give 420 mg pure **I**-AB1 and 600 mg mixture of **I-AB1** & **I-AB1a.** The absolute structure of **I-AB1** was determined by NOE (H18 was correlated with H20; H18 was not correlated with H16).

The mixture (600 mg) was separated by flash column (25~50% EtOAc in PE) to give **I-AB1** (160 mg, total yield: 70%) and **I**-AB1a (200 mg, 24%) both as solid. The absolute structure of **I-AB1a** was determined by NOE (H16 was correlated with H18).

### I-AB1:

**¹H NMR** (400 MHz, CDCl₃) δ_{H} 3.65 (t, *J =* 10.8 Hz, 1H), 3.54 (dd, *J* = 6.0, 10.8 Hz, 1H), 2.15-2.00 (m, 1H), 1.90-1.70 (m, 4H), 1.65-1.50 (m, 3H), 1.50-1.35 (m, 10H), 1.30-1.15 (m, 7H), 1.15-1.00 (m, 2H), 0.97 (s, 3H).

### I-AB1a:

**¹H NMR** (400 MHz, CDCl₃) δ_{H} 3.95 (dd, *J =* 8.4, 10.8 Hz, 1H), 3.73 (dd, *J =* 7.2, 10.8 Hz, 1H), 2.15-1.95 (m, 2H), 1.85-1.70 (m, 4H), 1.65-1.45 (m, 4H), 1.45-1.15 (m, 16H), 1.15-1.00 (m, 4H).

### Example I-113 and I-113a: Synthesis of (1S,2aS,2bR,4aR,6R,8aS,8bR,10aS)-6-hydroxy-6-(methoxymethyl)-10a-methyl-N-phenylhexadecahydrocyclobuta[a]phenanthrene-1-carboxamide (I-AC8) & (1R,2aS,2bR,4aR,6R,8aS,8bR,10aS)-6-hydroxy-6-(methoxymethyl)-10a-methyl-N-phenylhexadecahydrocyclobuta[a]phenanthrene-1-carboxamide (I-AC8a)

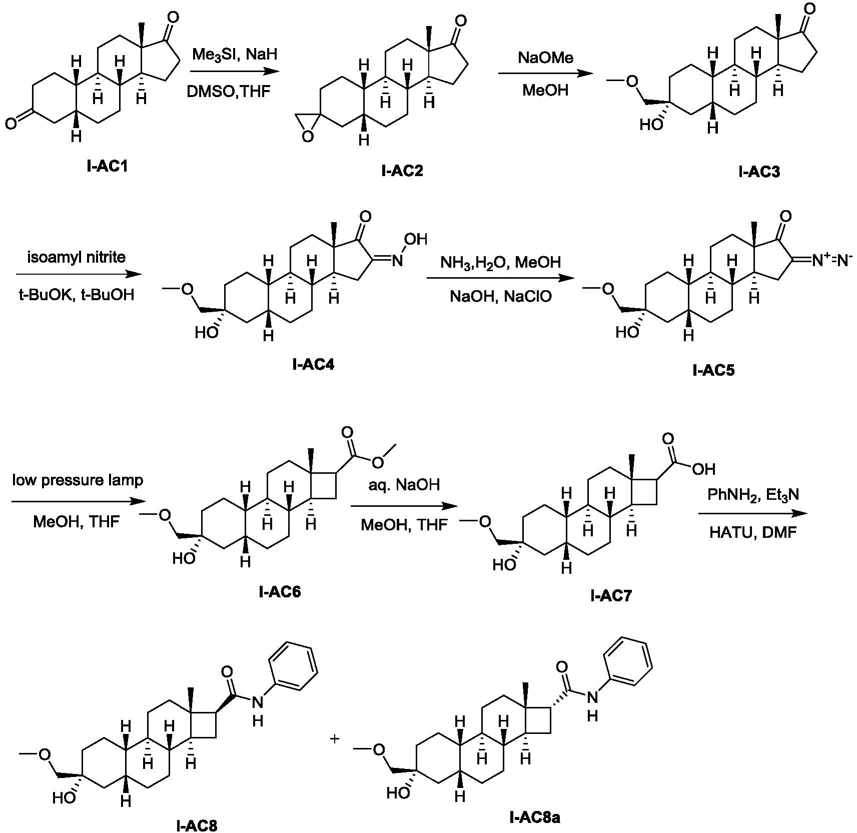

### Synthesis of I-AC2

To a solution of Me₃SI (10.3 g, 50.9 mmol) in DMSO (60 mL) and THF (30 mL) was added NaH (2.03g, 50.9 mmol, 60% in mineral oil) at 0 °C, the mixture was stirred at 0°C under N₂ for 1h. To a solution of **I-AC1** (CAS# 5696-51-5) (10.0 g, 36.4 mmol, reported in patent 'US5925630, 1999, A1) in DMSO (30mL) was added the mixture at 0 °C. Then the mixture was stirred at 15 °C for 6 h. The reaction was treated with water (80 mL). The mixture was extracted with EtOAc (2 x 80 mL). The combined organic phase was washed with water (2 x 50 mL), brine (50 mL), dried over anhydrous Na₂SO₄, filtered, and concentrated in vacuum to give **I-AC2** (13 g, 36.4 mmol) as a solid. The residue was used directly for the next step.

**¹H NMR** (400 MHz, CD₃OD) δ_{H} 2.63-2.53 (m, 2H), 2.47-2.38 (m, 1H), 2.27 - 2.02 (m, 3H), 1.96 - 1.44 (m, 10H), 1.43-1.22 (m, 6H), 1.19 - 0.90 (m, 3H), 0.88-8.84 (m, 3H).

### Synthesis of I-AC3

To a mixture of **I-AC2** (13 g, 36.4 mmol) in anhydrous MeOH (200 mL) was added NaOMe (12.1 g, 224 mmol) at 20°C. The reaction mixture was stirred at 65°C for 12 h. The mixture was poured into Water (300 mL). The reaction mixture was concentrated to remove most of the solvent and extracted with EtOAc (2 x 200 mL). The combined organic phase was washed with saturated brine (2 x 100 mL), dried over anhydrous Na₂SO₄, filtered, concentrated. The residue was purified by flash column (15~25% of EtOAc in PE) to give **I-AC3** (5.6g, 17.4 mmol, 48.2 %) as a solid.

**¹H NMR** (400 MHz, CD₃OD) δ_{H} = 3.43 - 3.32 (m, 5H), 2.63 (s, 1H), 2.46 - 2.36 (m, 1H), 2.14 - 2.03 (m, 1H), 1.96 - 1.87 (m, 1H), 1.85 - 1.71 (m, 5H), 1.65 - 1.30 (m, 10H), 1.26 - 1.17 (m, 4H), 1.12 - 0.99 (m, 1H), 0.84 (s, 3H).

### Synthesis of I-AC4

To a mixture of t-BuOK (15.5 g, 139 mmol) in t-BuOH (200 mL) was added **I-AC3** (5.6 g, 17.4 mmol) and isopentyl nitrite (8.15 g, 69.6 mmol) under N₂. The mixture was stirred at 30°C for 2 h. To the mixture was added NH₄Cl (100 mL, sat.). The mixture was extracted with EtOAc (2 x 200 mL). The organic layer was washed with water (2 x 100 mL), dried over Na₂SO₄, concentrated in vacuum and the residue was triturated from PE (30 mL) to give **I-AC4** (4.8 g, 78.9 %) as a solid.

**¹H NMR** (400 MHz, CD₃OD) δ_{H} 3.48-3.43 (m, 5H), 2.89 (dd, *J*=6.4, 17.6 Hz, 1H), 2.08 (dd, *J*=12.8, 17.2 Hz, 1H), 1.93 - 1.73 (m, 5H), 1.72 - 1.58 (m, 3H), 1.57 - 1.30 (m, 9H), 1.28 - 1.04 (m, 4H), 0.95-0.91 (m, 3H).

### Synthesis of I-AC5

To a solution of **I-AC4** (4.8 g, 13.7 mmol) in MeOH (500 mL) was added aq. NaOH (50 mL, 5 M), NH₃ (75 mL, 25%). Then to the mixture was added dropwised aq. NaClO (150 mL, 10%) within 30 min at 20 °C. The mixture was stirred at 20°C for 12 h. The mixture was concentrated to remove most MeOH and to the mxiture was added NH₄Cl (400 mL, sat.). The mixture was filtered. The solid was washed with water, dissolved in DCM (50 mL), dried over Na₂SO₄, filtered and purified by flash column (15~40% EtOAc in PE) to give **I-AC5** (2.9g, 61.1%) as a solid.

**¹H NMR** (400 MHz, CD₃OD) δ_{H} 3.41 - 3.34 (m, 5H), 2.840-2.785 (m, 1H), 2.67 (s, 1H), 2.56 (t, *J*=12.0 Hz, 1H), 1.79 - 1.53 (m, 8H), 1.47 - 1.33 (m, 6H), 1.29 - 1.03 (m, 5H), 0.95 (s, 3H).

### Synthesis of I-AC6

A solution of **I-AC5** (2.9 g, 8.37 mmol) in THF (300 mL) and MeOH (100 mL) under N₂ was irradiated with 300 W low pressrue mercury lamp for 16 h at 20°C. The mixture was concentrated and purified by flash column (5~25% EtOAc in PE) to give **I-AC6** (2.2g, 75%) as an oil.

**¹H NMR** (400 MHz, CD₃OD) δ_{H} 3.69-3.63 (m, 3H), 3.41 - 3.33 (m, 5H), 2.76 - 2.68 (m, 1H), 2.19 - 2.07 (m, 1H), 2.03 - 1.95 (m, 1H), 1.83 - 1.51 (m, 8H), 1.50 - 1.27 (m, 8H), 1.23 - 1.03 (m, 5H), 0.95 (s, 2H).

### Synthesis of I-AC7

To a solution of **I-AC6** (0.6g, 1.7mmol) in THF (4 mL) was added MeOH (4 mL) and aq. NaOH (4 mL, 5M). The mixture was stirred at 15°C for 20 h. The mixture was concentrated in vacuum to remove most organic solvent and to the mixture was added aq. HCl (13 mL, 2 M). The mixture was filtered and the filtered cake was combined to give **I-AC7** (450 mg, 78.2%) as a solid.

**¹H NMR** (400 MHz, CD₃OD) δ_{H} 3.45-3.35 (m, 5H), 2.82 - 2.73 (m, 1H), 2.17 - 2.05 (m, 1H), 2.03 - 1.97 (m, 1H), 1.90 - 1.54 (m, 8H), 1.53 - 1.28 (m, 8H), 1.23 - 1.09 (m, 1H), 1.24 - 1.07 (m, 5H), 1.03 (s, 2H).

### Synthesis of I-AC8 & I-AC8a

To a solution of **I-AC7** (200 mg, 0.60 mmol) in DCM (2 mL) was added aniline (109 mg, 1.18 mmol), TEA (300 mg, 3.0mmol) and HATU (448mg, 1.18 mmol). The mixtrue was stirred at 30°C for 16 h. The mixture was concentrated and purified by flash column (15~40% EtOAc in PE) to give 135 mg of **I-AC8** as a solid and 30mg of **I-AC8a** as an oil.

The **I-AC8** (135 mg) was purified by prep-HPLC (Instrument: FF; Column: YMC-Actus Triart C18 100*30mm*5um; Condition: water(0.05%HCl)-ACN; Begin B: 55; End B: 85; Gradient Time(min): 9; 100%B Hold Time(min): 2; FlowRate(ml/min): 25) and lyophilized to give **I-AC8** (42 mg, 16%) as a solid. The configuration of C16 was determined by NOE (H18 was correlated with Hₐ15, H16 was correlated with H_{b}15).

The **I-AC8a** (35 mg) was purified by prep-HPLC (Instrument: FF; Column: YMC-Actus Triart C18 100*30mm*5um; Condition: water(0.05%HCl)-ACN; Begin B: 55; End B: 85; Gradient Time(min): 9; 100%B Hold Time(min): 1; FlowRate (ml/min): 25) and lyophilized to give **I-AC8a** (5 mg, 2%) as a solid. The configuration of C16 was determined by NOE (H18 was correlated with H16).

### I-AC8:

**¹H NMR** (400 MHz, CD₃OD) δ_{H} 7.50 (d, *J*=7.2 Hz, 2H), 7.30 (t, *J*=7.2 Hz, 2H), 7.12-7.03 (m, 1H), 6.97 (s, 1H), 3.45-3.35 (m, 5H), 2.83-2.73 (m, 1H), 2.25 - 2.15 (m, 1H), 2.13 - 2.03 (m, 1H), 1.83 - 1.72 (m, 4H), 1.70 - 1.56 (m, 4H), 1.52 - 1.32 (m, 8H), 1.30 - 1.13 (m, 4H), 1.05 (s, 3H); **MS ESI** calcd. for C₂₆H₃₈NO₃ [M+H]⁺ 412, found 412.

### I-AC8a:

**¹H NMR** (400 MHz, CD₃OD) δ_{H} 7.54 (d, *J*=7.6 Hz, 2H), 7.32 (t, *J*=8.0 Hz, 2H), 7.09 (t, *J*=7.2 Hz, 1H), 6.94 (s, 1H), 3.40-3.36 (m, 5H), 2.74 (d, *J*=6.4 Hz, 1H), 2.32 (t, *J*=8.0 Hz, 1H), 2.21 - 2.08 (m, 2H), 1.80 - 1.61 (m, 7H), 1.53 - 1.38 (m, 7H), 1.30-1.15 (m, 8H); **MS ESI** calcd. for C₂₆H₃₈NO₃ [M+H]⁺ 412, found 412.

### Example I-114 and I-114a: Synthesis of ((1S,2aS,2bR,4aR,6R,8aS,8bR,10aS)-6-hydroxy-6-(methoxymethyl)-10a-methylhexadecahydrocyclobuta[a]phenanthren-1-yl)((S)-2-methylpiperidin-1-yl)methanone (I-AC9) & ((1R,2aS,2bR,4aR,6R,8aS,8bR,10aS)-6-hydroxy-6-(methoxymethyl)-10a-methylhexadecahydrocyclobuta[a]phenanthren-1-yl)((S)-2-methylpiperidin-1-yl)methanone (I-AC9a)

Following the procedure in **Example I-113** and using the acid **I-AC7,** the products **I-AC9** and **I-AC9a** were made using (2S)-2-methylpiperidine in place of aniline.

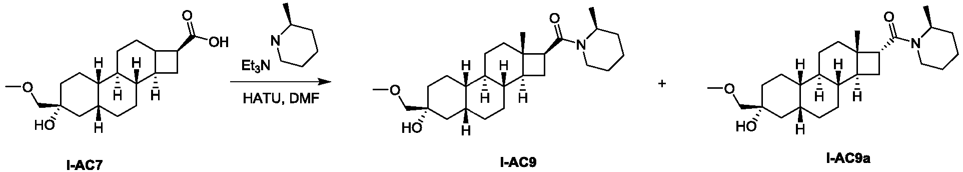

### I-AC9:

**¹H NMR** (400 MHz, DMSO-d₆) δ_{H} 4.92 - 4.23 (m, 1H), 3.61 - 3.40 (m, 1H), 3.35 - 3.27 (m, 5H), 3.22 - 2.91 (m, 1H), 2.90-2.83 (m, 1H), 2.18 - 2.07 (m, 1H), 1.82 - 1.45 (m, 16H), 1.41 - 1.31 (m, 5H), 1.24 - 1.04 (m, 9H), 0.89 (s, 3H); **MS ESI** calcd. for C₂₆H₄₄NO₃ [M+H]⁺ 418, found 418.

### I-AC9a:

**¹H NMR** (400 MHz, CD₃OD) δ_{H} 3.41-3.32 (m, 5H), 2.87-2.73 (m, 1H), 2.46-2.31 (m, 1H), 2.11 - 1.89 (m, 5H), 1.80 - 1.49 (m, 12H), 1.49 - 1.31 (m, 8H), 1.30 - 0.99 (m, 11H); **MS ESI** calcd. for C₂₆H₄₄NO₃ [M+H]⁺ 418, found 418.

### Example I-115 and I-115a: Synthesis of 1-((1S,2aS,2bR,4aS,6R,8aS,8bR,10aS)-6-hydroxy-6,10a-dimethylhexadecahydrocyclobuta[a]phenanthren-1-yl)ethan-1-one (I-AD8) & 1-((1R,2aS,2bR,4aS,6R,8aS,8bR,10aS)-6-hydroxy-6,10a-dimethylhexadecahydrocyclobuta[a]phenanthren-1-yl)ethan-1-one (I-AD8a)

Following the procedure in **Example I-101** & **I-102** and using the diastereomeric ketone (CAS #5696-58-2), the products **I-AD8** and **I-AD8a** were made.

### I-AD8:

**¹H NMR** (400 MHz, CDCl₃) δ_{H} 2.80 (dd, *J*=5.6, 9.2 Hz, 1H), 2.17-2.04 (m, 1H), 2.01 (s, 3H), 1.88-1.60 (m, 7H), 1.55-1.50 (m, 2H), 1.48-1.30 (m, 3H), 1.29-1.17 (m, 6H), 1.14-0.94 (m, 4H), 0.90 (s, 3H), 0.86-0.67 (m, 2H); **MS ESI** calcd. for C₂₀H₃₃O₂ [M+H]⁺ 305, found 305.

### I-AD8a:

**¹H NMR** (400 MHz, CDCl₃) δ_{H} 2.94 (d, *J*=6.0 Hz, 1H), 2.26-2.18 (m, 1H), 2.06 (s, 3H), 1.91-1.82 (m, 1H), 1.79-1.70 (m, 2H), 1.69-1.60 (m, 2H), 1.56-1.47 (m, 4H), 1.44-1.37 (m, 1H), 1.34-1.16 (m, 11H), 1.12-1.02 (m, 2H), 1.01-0.93 (m, 2H), 0.81 - 0.62 (m, 2H); **MS ESI** calcd. for C₂₀H₃₃O₂ [M+H]⁺ 305, found 305.

### Example I-116 and I-116a: Synthesis of 1-(2-((1S,2aS,2bR,4aS,6R,8aS,8bR,10aS)-6-hydroxy-6,10a-dimethylhexadecahydrocyclobuta[a]phenanthren-1-yl)-2-oxoethyl)-1H-pyrazole-4-carbonitrile (I-AD10) and 1-(2-((1R,2aS,2bR,4aS,6R,8aS,8bR,10aS)-6-hydroxy-6,10a-dimethylhexadecahydrocyclobuta[a]phenanthren-1-yl)-2-oxoethyl)-1H-pyrazole-4-carbonitrile (I-AD10a)

Following the procedure in **Example I-104** and using the diastereomeric ketones, the following examples were made.

| **Exa mpl e** | **ketone** | **ArylN H** | **Compound Name** | **¹H NMR (400 MHz, CDCl₃)** | **MS ESI** |
|---|---|---|---|---|---|
| **I-116** | 1-((1S,2aS,2bR, 4aS,6R,8aS,8b | 4-cyanop yrazole | 1-(2-((1S,2aS,2bR,4aS, 6R,8aS,8bR,10aS)- | δ_{H} 7.85 (s, 1H), 7.81 (s, 1H), 4.84 (s, 2H), 2.91 (dd, *J*=6.0, 9.2 Hz, 1H), | calcd. for C₂₄H₃₂N |
| | R,10aS)-6-hydroxy-6,10a-dimethylhexad ecahydrocyclo buta[a]phenan thren-1-yl)ethan-1-one (**I-AD8**) | | 6-hydroxy-6, 10a-dimethylhexadecah ydrocyclobuta[a]p henanthren-1-yl)-2-oxoethyl)-1H-pyrazole-4-carbonitrile (**I-AD10**) | 2.29-2.18 (m, 1H), 1.94-1.87 (m, 1H), 1.86-1.60 (m, 6H), 1.54-1.48 (m, 2H), 1.42-1.17 (m, 8H), 1.15-1.93 (m, 8H), 0.89-0.66 (m, 2H). | ₃O [M+H-H₂O]⁺ 378, found 378 |
| **I-116a** | 1-((1R,2aS,2bR, 4aS,6R,8aS,8b R,10aS)-6-hydroxy-6,10a-dimethylhexad ecahydrocyclo buta[a]phenan thren-1-yl)ethan-1-one (**I-AD8a**) | 4-cyanop yrazole | 1-(2-((1R,2aS,2bR,4aS, 6R,8aS,8bR,10aS)-6-hydroxy-6, 10a-dimethylhexadecah ydrocyclobuta[a]p henanthren-1-yl)-2-oxoethyl)-1H-pyrazole-4-carbonitrile (**I-AD10a**) | δ_{H} 7.87 (s, 1H), 7.83 (s, 1H), 4.96-4.79 (m, 2H), 2.96 (d, *J*=6.4 Hz, 1H), 2.34-2.22 (m, 1H), 2.05-1.96 (m, 1H), 1.90-1.76 (m, 2H), 1.70-1.60 (m, 3H), 1.54-1.41 (m, 4H), 1.35-1.15 (m, 11H), 1.12-0.92 (m, 4H), 0.82-0.63 (m, 2H). | calcd. for C₂₄H₃₂N ₃O [M+H-H₂O]⁺ 378, found 378 |

### Example I-117: Synthesis of (1S,2aS,2bR,4aR,6R,8aS,8bR,10aS)-1-(methoxymethyl)-6,10a-dimethylhexadecahydrocyclobuta[a]phenanthren-6-ol

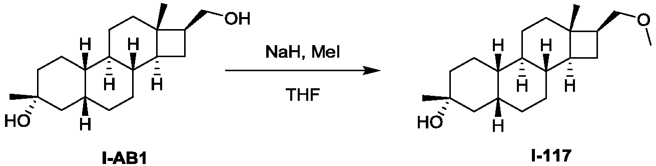

To a solution of **I-AB1** (200 mg, 0.7 mmol) in THF (5 mL) was added NaH (81.8 mg, 2.1 mmol) (60% in mineral oil) at 0°C, the mixture was stirred at 25°C for 30 min, then added CH₃I (106 mg, 0.8 mmoL) at 0°C. The mixture was stirred at 25°C for 16 hours. The mixture was quenched with H₂O (10 mL) and extracted with EtOAc (2 x 20 mL). The combined organic phase was dried over anhydrous Na₂SO₄, filtered and concentrated. The residue was purified by flash column (10~30% of EtOAc in PE) to give **Example I-117** (58 mg, 27.7%) as a solid.

**¹H NMR** (400 MHz, CDCl₃) δ_{H}.3.44-3.37 (m, 1H), 3.32-3.23 (m, 4H), 2.17-2.07 (m, 1H), 1.92-1.76 (m, 4H), 1.66-1.57 (m, 3H), 1.54-1.31 (m, 10H), 1.29-1.05 (m, 8H), 0.94 (s, 3H); **MS ESI** calcd. for C₂₀H₂₉ [M+H-H₂O-OCH₃]⁺ 257, found 257.

### Example I-118: Synthesis of (1R,2aS,2bR,4aR,6R,8aS,8bR,10aS)-1-(methoxymethyl)-6,10a-dimethylhexadecahydrocyclobuta[a]phenanthren-6-ol

Following the procedure in **Example I-117** and using the diastereomeric alcohol (**I-AB1a**), **Example I-118** was made.

**¹H NMR** (400 MHz, CDCl₃) δ_{H} 3.70-3.60 (m, 1H), 3.53-3.46 (m, 1H), 3.35 (s, 3H), 2.14 (q, J=7.6 Hz, 1H), 2.04-1.95 (m, 1H), 1.88-1.71 (m, 4H), 1.66-1.59 (m, 2H), 1.56-1.47 (m, 2H), 1.46- 1.31 (m, 8H), 1.30-1.15 (m, 7H), 1.12-1.09 (m, 4H); **MS ESI** calcd. for C20H29 [M+H-H2O-OCH3]+ 257, found 257.

### EXAMPLE I-119 & I-120: Synthesis of 1-((1S,2aS,2bR,4aR,6R,8aS,8bR,10aS)-6-hydroxy-6-(methoxymethyl)-10a-methylhexadecahydrocyclobuta[a]phenanthren-1-yl)ethan-1-one (I-AC11) & 1-((1R,2aS,2bR,4aR,6R,8aS,8bR,10aS)-6-hydroxy-6-(methoxymethyl)-10a-methylhexadecahydrocyclobuta[a]phenanthren-1-yl)ethan-1-one (I-AC12)

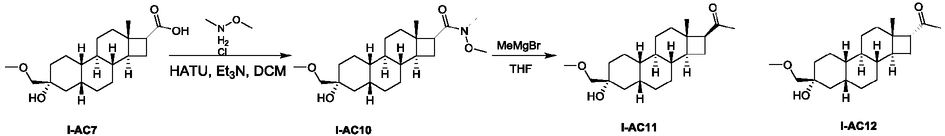

### Synthesis of I-AC10

To a solution of **I-AC7** (1.2 g, 3.56 mmol) and methoxy(methyl)amine hydrochloride (694 mg, 7.12 mmol) in DCM (10 mL) were added HATU (2.70 g, 7.12 mmol) and DIEA (2.15 g, 21.3 mmol) at 20 °C under N₂. After stirring at 20°C for 16 h, the mixture was concentrated, diluted with water (20 mL) and extracted with EtOAc (3 x 30 mL). The combined organic solution was washed with saturated brine (2 x 20 mL), dried over anhydrous Na₂SO₄, filtered and concentrated to give **I-AC10** (1.3 g) as a solid. **¹H NMR** (400 MHz, CDCl₃) δ_{H} 3.65 (s, 3H), 3.41-3.36 (m, 5H), 3.15 (s, 3H), 2.99-2.86 (m, 1H), 2.81 (s, 3H), 2.31-2.13 (m, 1H), 1.95-1.88 (m, 1H), 1.95-1.35 (m, 11H), 1.23-1.05 (m, 5H), 0.93 (s, 3H).

### Synthesis of I-AC11 & I-AC12

To a solution of **I-AC10** (1.3 g, 3.42 mmol) in THF (15 mL) was added MeMgBr (5.70 mL, 17.1 mmol, 3 M) at 0 °C. After stirring at 0 °C for 2 h, the mixture was quenched by NH₄Cl (20 mL, sat.) and extracted with EtOAc (3 x 50 mL). The combined organic solution was washed with saturated brine (2 x 20 mL), dried over anhydrous Na₂SO₄, filtered, concentrated and purified by flash column (0~20% EtOAc in PE) to give **I-AC11** (500 mg, 56%) as a solid and **I-AC12** (180 mg, 20%) as an oil.

**I-AC11: ¹H NMR** (400 MHz, CDCl₃) δ_{H} 3.47-3.31 (m, 5H), 2.89-2.76 (m, 1H), 2.61 (s, 1H), 2.17-2.03 (m, 1H), 2.01 (s, 3H), 1.90-1.72 (m, 5H), 1.70-1.58 (m, 3H), 1.55-1.31 (m, 8H), 1.28-1.09 (m, 4H), 0.89 (s, 3H); **LC-ELSD/MS** purity 99%, MS ESI calcd. for C₂₁H₃₄O₃ [M-H₂O+H]⁺ 317.2, found 317.2.

**I-AC12: ¹H NMR** (400 MHz, CDCl₃) δ_{H} 3.44-3.31 (m, 5H), 2.98-2.89 (m, 1H), 2.48 (s, 1H), 2.29-2.17 (m, 1H), 2.06 (s, 3H), 1.92-1.58 (m, 7H), 1.55-1.29 (m, 9H), 1.26 (s, 3H), 1.23-1.09 (m, 4H); **LC-ELSD/MS** purity 99%, MS ESI calcd. for C₂₀H₃₁O [M-H₂O +H]⁺ 317.2, found 317.2.

### EXAMPLE I-121: Synthesis of 1-(2-((1S,2aS,2bR,4aR,6R,8aS,8bR,10aS)-6-hydroxy-6-(methoxymethyl)-10a-methylhexadecahydrocyclobuta[a]phenanthren-1-yl)-2-oxoethyl)-1H-pyrazole-4-carbonitrile (I-AC14)

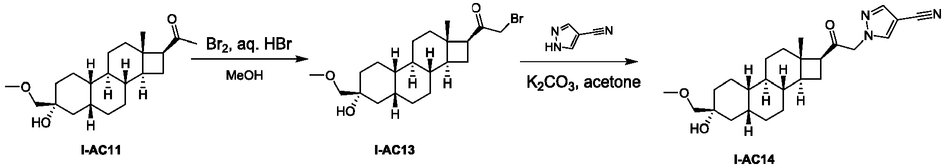

### Synthesis of I-AC13

To a solution of **I-AC11** (200 mg, 0.60 mmol) in MeOH (3 mL) was added HBr (0.01 mL, 0.18 mmol, 40% in water) and Br₂ (95.6 mg, 0.60 mmol) at 20°C. After stirring at 20°C for 2 h, the mixture was poured into Na₂S₂O₃ (10 mL, sat.) and extracted with EtOAc (10 mL). The organic solution was separated, dried over Na₂SO₄, filtered and concentrated in vacuum to give **I-AC13** (340 mg) as a solid. **¹H NMR** (400 MHz, CDCl₃) δ_{H} 3.85-3.70 (m, 2H), 3.41-3.36 (m, 5H), 3.13 (dd, *J =* 8.0, 8.0 Hz, 1H), 2.24-2.14 (m, 1H), 1.95-1.87 (m, 1H), 1.84-1.58 (m, 10H), 1.49-1.31 (m, 7H), 1.21-1.10 (m, 3H), 0.90 (s, 3H).

### Synthesis of I-AC14

To a solution of **I-AC13** (340 mg, 0.82 mmol) in acetone (3 mL) were added 1H-pyrazole-4-carbonitrile (114 mg, 1.23 mmol) and K₂CO₃ (339 mg, 2.46 mmol). After stirring at 20 °C for 16 h, the mixture was diluted with water (20 mL) and extracted with EtOAc (3 x 30 mL). The combined organic solution was separated, dried over Na₂SO₄, filtered and concentrated. The residue was purified by flash column (20~50% of EtOAc in PE) and lyophilized to give **I-AC14** (44.6 mg, 22%) as a solid. **¹H NMR** (400 MHz, CDCl₃) δ_{H} 7.85 (s, 1H), 7.81 (s, 1H), 4.84 (s, 2H), 3.41-3.36 (m, 5H), 2.92 (dd, *J =* 8.0, 4.0 Hz, 1H), 2.62 (brs, 1H), 2.30-2.17 (m, 1H), 1.97-1.85 (m, 1H), 1.81-1.73 (m, 4H), 1.71-1.58 (m, 4H), 1.49-1.31 (m, 7H), 1.26-1.11 (m, 4H), 0.95 (s, 3H); **LC-ELSD/MS** purity 99%, MS ESI calcd. for C₂₀H₃₁O [M +Na]⁺ 448.3, found 448.3.

### EXAMPLE I-122 & I-123: Synthesis of 1-((1S,2aS,2bR,4aR,6R,8aS,8bR,10aS)-6-hydroxy-6,10a-dimethylhexadecahydrocyclobuta[a]phenanthren-1-yl)-2-(2H-1,2,3-triazol-2-yl)ethan-1-one (I-AA11) & 1-((1S,2aS,2bR,4aR,6R,8aS,8bR,10aS)-6-hydroxy-6,10a-dimethylhexadecahydrocyclobuta[a]phenanthren-1-yl)-2-(1H-1,2,3-triazol-1-yl)ethan-1-one (I-AA12)

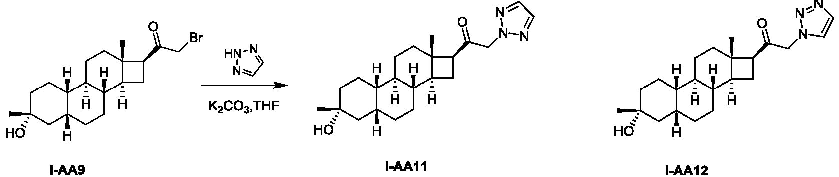

To a solution of **I-AA9** (100 mg, 0.3 mmol) in THF (3 mL) was added 2H-1,2,3-triazole (36 mg, 0.5 mmol) and K₂CO₃ (107 mg, 0.8 mmol). After stirring at 25°C for 12 h, the mixture was added water (40 mL) and extracted with EtOAc (2 x 50 mL). The organic layer was separated, dried over Na₂SO₄, filtered and concentrated. The residue (70 mg) was purified by flash column (30~80% EtOAc in PE) to give **I-AA11** (5 mg) and **I-AA12** (13 mg).

**I-AA11: ¹H NMR** (400 MHz, CDCl₃) δ_{H} 7.68 (s, 2H), 5.19-5.03 (m, 2H), 2.90-2.82 (m, 1H), 2.28-2.17 (m, 1H), 1.88-1.75 (m, 5H), 1.68-1.60 (m, 3H), 1.53-1.44 (m, 4H), 1.42-1.36 (m, 5H), 1.25 (s, 4H), 1.23-1.04 (m, 3H), 0.99 (s, 3H); **LC-ELSD/MS** purity 99%, MS ESI calcd. For C₂₂H₃₂N₃O [M+H-H₂O]+354.2, found 354.2.

**I-AA12: ¹H NMR** (400 MHz, CDCl₃) δ_{H} 7.76 (s, 1H), 7.64 (s, 1H), 5.09 (s, 2H), 2.99-2.90 (m, 1H), 2.29-2.18 (m, 1H), 1.95-1.87 (m, 1H), 1.88-1.74 (m, 4H), 1.68-1.60 (m, 5H), 1.45-1.34 (m, 6H), 1.31-1.18 (m, 8H), 0.95 (s, 3H); **LC-ELSD/MS** purity 99%, MS ESI calcd. For C₂₂H₃₄N₃O₂ [M+H]+372.3, found 372.3.

### Example I-201: Synthesis of (2R,4aS,4bR,6aS,11aS,11bR,13aR)-2-hydroxy-2,6a-dimethyloctadecahydro-7H-cyclohepta[a]phenanthren-7-one (I-BA3)

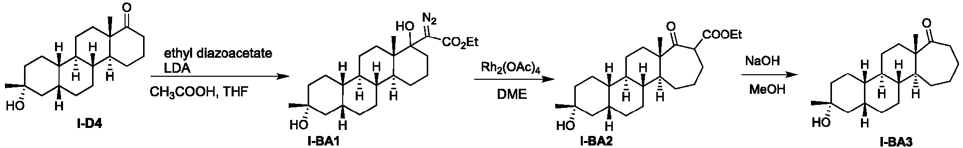

### Synthesis of I-BA1

A cold (-78°C) solution of lithium di-isopropylamide from addition of n-butyl-lithium in hexane (3.0 mL, 2.5 M, 7.5 mmol) to di-isopropylamine (1.1 mL, 0.72 g/mL, 7.9 mmol) in THF (5.0 mL) was added to a stirred solution of **I-D4** (400 mg, 1.3 mmol) and ethyl diazoacetate (896 mg, 7.9 mmol) in THF (5.0 mL) at -78°C. The mixture was stirred at -78°C for 1hour. Then acetic acid (471 mg, 9.9 mmol) in THF (5.0 mL) was added into the reaction mixture at -78°C, the mixture was then warm to 10°C and stirred for 16 hours. Water (50 mL) was added into the reaction mixture. The aqueous solution extracted with EtOAc (3 x 50 mL). The combined organic layers were washed with saturated brine (100 mL), dried over anhydrous Na₂SO₄, and evaporated under reduced pressure to give the product as an oil, and then purified by combi flash (0-10% of EtOAc in PE) to give **I-BA1** (420 mg, 77%) as a solid.

**¹H NMR** (400 MHz, CDCl₃) δ_{H} 4.25-4.18 (m, 2H), 1.95-1.59 (m, 15H), 1.51-1.29 (m, 15H), 1.25 (s, 3H), 0.87 (s, 3H).

### Synthesis of I-BA2

To a solution of **I-BA1** (420 mg, 1.0 mmol) in DME (5.0 mL) was added Rh₂(OAc)₄ (50 mg) at 10°C. The reaction mixture was stirred for 2 hours at 10°C. The reaction mixture was concentrated to give **I-BA2** (400 mg) as an oil.

**¹H NMR** (400 MHz, CDCl₃) δ_{H} 13.60 (s, 1H), 4.25-4.18 (m, 2H), 2.55-2.50 (m, 1H), 2.29-2.11 (m, 2H), 1.99-1.85 (m, 3H), 1.83-1.50 (m, 16H), 1.48-1.35 (m, 3H), 1.34-1.20 (m, 3H), 1.18 (s, 3H), 1.12-0.85 (m, 4H).

### Synthesis of I-BA3

To a solution of **I-BA2** (100 mg, 0.3 mmol) in MeOH (5 mL) and water (1 mL) was added NaOH (101 mg, 2.6 mmol) at 10°C. The reaction mixture was stirred for 18 hours at 70°C. The reaction mixture was added into saturated brine (50 mL), and the aqueous layer was extracted with DCM (2 x 50 mL). The combined organic layer was washed with HCl (100 mL, 1N), saturated NaHCO₃ (100 mL), saturated brine (100 mL), dried over Na₂SO₄, filtered and concentrated to give **I-BA3** (15 mg, 18%) as a solid.

**¹H NMR** (400 MHz, CDCl₃) δ_{H} 3.05-2.95 (m, 1H), 2.31-2.22 (m, 1H), 1.90-1.58 (m, 11H), 1.51-1.29 (m, 12H), 1.27 (s, 3H), 1.13-1.04 (m, 2H), 1.04 (s, 3H), 1.00-0.93 (m, 1H); **MS ESI** calcd. for C₂₁H₃₃O [M+H-H₂O]⁺ 301, found 301.

### Example I-202 and I-202a: Synthesis of (2R,4aS,4bR,6aS,7R,11aS,11bR,13aR)-7-(hydroxymethyl)-2,6a-dimethyloctadecahydro-1H-cyclohepta[a]phenanthren-2-ol (I-BASa) & (2R,4aS,4bR,6aS,7S,11aS,11bR,13aR)-7-(hydroxymethyl)-2,6a-dimethyloctadecahydro-1H-cyclohepta[a]phenanthren-2-ol (I-BA5b)

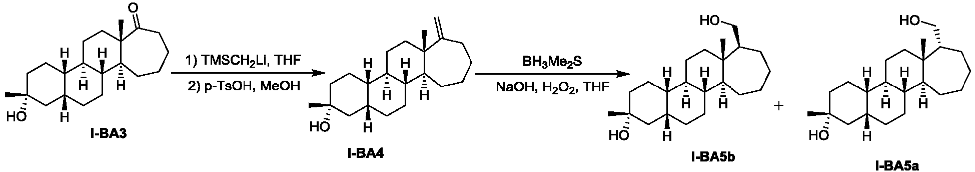

### Synthesis of I-BA4

To a solution of TMSCH₂Li (39.8 mL, 22.3 mmol, 0.56M) in THF (20 mL) was added a solution of **I-BA3** (1.4 g, 4.3 mmol) in THF (40 mL) at -40°C. The reaction mixture was stirred for 16 hours at 30°C. The reaction mixture was added into saturated NH₄Cl (100 mL). The aqueous layer was extracted with EtOAc (3 x 50 mL). The combined organic layer was washed with saturated brine (100 mL), dried over anhydrous Na₂SO₄, filtered and concentrated. The residue was dissolved in MeOH (20 mL). P-TsOH (200 mg) was added into the solution. The reaction mixture was stirred for 10min. Saturated NaHCO₃ (50 mL) was added to the mixture. The aqueous layer was extracted with EtOAc (3 x 50 mL). The combined organic layer was washed with saturated NH₄Cl (100 mL), saturated brine (100 mL), dried over anhydrous Na₂SO₄, filtered and concentrated. The residue was purified by flash column (0-20% of EtOAc in PE) to give **I-BA4** (650 mg) and recycled the starting material (360 mg) as an oil.

**¹H NMR** (400 MHz, CDCl₃) δ_{H} 4.81 (s, 1H), 4.74 (s, 1H), 2.49-2.35 (m, 1H), 2.25-2.11 (m, 1H), 1.95-1.65 (m, 8H), 1.51-1.31 (m, 6H), 1.27 (s, 3H), 1.07 (s, 3H), 1.05-0.80 (m, 12H).

### Synthesis of I-BA5a & I-BA5b

To a solution of **I-BA4** (650 mg, 2.1 mmol) in THF (10 mL) was added BH₃Me₂S (1.0 mL, 10 M, 10.0 mmol) at 0°C. The reaction mixture was stirred for 4 hours at 10°C. Ethanol (938 mg, 20.4 mmol) was added to the reaction mixture. To the mixture was added aqueous NaOH solution (5.00 mL, 5M, 25.0 mmol), followed by H₂O₂ (2.31 g, 30%, 20.4 mmol) at 0°C. The mixture was stirred for 1h at 70°C. The mixture was cooled to 15°C and extracted with EtOAc (3 x 50 mL). The combined organic layer was washed with saturated brine (100 mL), dried over anhydrous Na₂SO₄, filtered and concentrated to give **I-BA5** (700 mg) as an oil. The material was purified by flash column (0-30% of EtOAc in PE) to give **I-BA5a** (123 mg) and **I-BA5b** (230 mg) as solids.

### I-BA5b:

**¹H NMR** (400 MHz, CDCl₃) δ_{H} 3.86 (dd, *J* = 2.8, 10.8 Hz, 1H), 3.28 (t, *J =* 9.2 Hz, 1H), 2.00-1.65 (m, 9H), 1.50-1.28 (m, 13H), 1.26 (s, 3H), 1.16-0.75 (m, 8H), 0.72 (s, 3H); MS **ESI** calcd. for C₂₂H₃₇O [M+H-H₂O]⁺ 317, found 317.

The structure was confirmed by NOE.

### I-BA5a:

**¹H NMR** (400 MHz, CDCl₃) δ_{H} 3.76 (dd, *J* = 3.2, 10.0 Hz, 1H), 3.30 (t, *J =* 10.0 Hz, 1H), 2.00-1.60 (m, 12H), 1.50-1.28 (m, 8H), 1.26 (s, 3H), 1.23-0.95 (m, 10H), 0.93 (s, 3H); **MS ESI** calcd. for C₂₂H₃₇O [M+H-H₂O]⁺ 317, found 317.

The structure was confirmed by NOE.

### Example I-203 and I-203a: Synthesis of (2R,4aS,4bR,6aS,7S,11aS,11bR,13aR)-7-(methoxymethyl)-2,6a-dimethyloctadecahydro-1H-cyclohepta[a]phenanthren-2-ol (I-BA6) & (2R,4aS,4bR,6aS,7R,11aS,11bR,13aR)-7-(methoxymethyl)-2,6a-dimethyloctadecahydro-1H-cyclohepta[a]phenanthren-2-ol (I-BA6a)

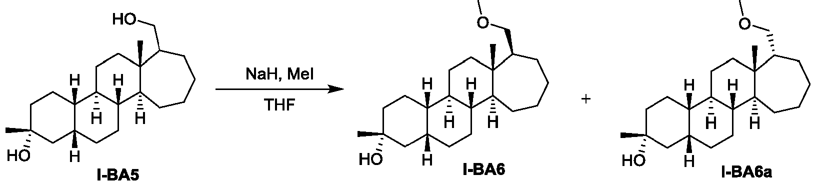

To a solution of **I-BA5** (300 mg) in THF (5 mL) was added NaH (72 mg, 60%, 1.8 mmol) at 0°C. The reaction mixture was stirred for 1h at 20°C. Then MeI (254 mg, 1.8 mmol) was added into the reaction mixture at 20°C. The reaction mixture was stirred for 16 hours at 20°C. The reaction mixture was added into saturated NH₄Cl (50 mL). The aqueous layer was extracted with EtOAc (2 x 50 mL). The combined organic layer was washed with saturated brine (100 mL), dried over anhydrous Na₂SO₄, filtered and concentrated. The residue was purified by flash column (0-5% of EtOAc in PE) to give **I-BA6a** (9 mg) and **I-BA6** (17 mg) both as solids.

The structure of **I**-BA6a was confirmed by NOE.

### I-BA6a:

**¹H NMR** (400 MHz, CDCl₃) δ_{H} 3.46-3.40 (m, 1H), 3.33 (s, 3H), 3.02 (t, *J =* 8.0 Hz, 1H), 1.91-1.61 (m, 9H), 1.51-1.28 (m, 9H), 1.27 (s, 3H), 1.25-0.95 (m, 10H), 0.92 (s, 3H), 0.90-0.80 (m, 1H); **MS ESI** calcd. for C₂₃H₃₉O [M+H-H₂O]⁺ 331, found 331.

### I-BA6:

**¹H NMR** (400 MHz, CDCl₃) δ_{H} 3.52 (dd, *J =* 2.8, 9.2 Hz, 1H), 3.30 (s, 3H), 3.00 (t, *J =* 8.0 Hz, 1H), 1.91-1.61 (m, 8H), 1.51-1.28 (m, 13H), 1.27 (s, 3H), 1.25-0.95 (m, 8H), 0.71 (s, 3H); **MS ESI** calcd. for C₂₃H₃₉O [M+H-H₂O]⁺ 331, found 331.

### Example I-204: Synthesis of 1-((2R,4aS,4bR,6aS,7R,11aS,11bR,13aR)-2-hydroxy-2,6a-dimethyloctadecahydro-1H-cyclohepta[a]phenanthren-7-yl)ethan-1-one (I-BA9)

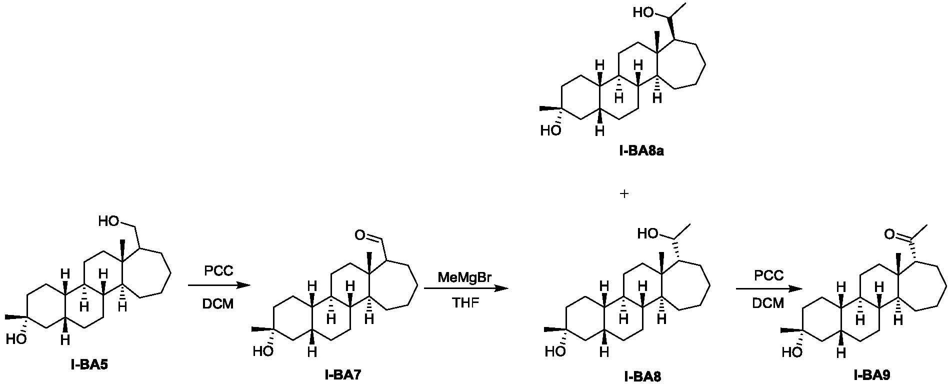

### Synthesis of I-BA7

To a solution of **I-BA5** (400 mg, 1.2 mmol) in DCM (5 mL) was added silica gel (1 g) and PCC (511 mg, 2.4 mmol) at 20°C. The reaction mixture was stirred for 1h at 20°C. The reaction mixture was filtered and the filter cake was washed with DCM (3 x 50 mL). The mother liquor was concentrated to give **I-BA7** (500 mg) as an oil, which was used directly for next step.

### Synthesis of I-BA8 and I-BA8a

To a solution of **I-BA7** (1.5 g) in THF (10 mL) was added MeMgBr (7.5 mL, 22.5 mmol, 3M in Et₂O) at 0°C. The reaction mixture was stirred for 1h at 20°C. The reaction mixture was added into saturated NH₄Cl (50 mL). The aqueous layer was extracted with EtOAc (3 x 50 mL). The combined organic layer was washed with saturated brine (100 mL), dried over anhydrous Na₂SO₄, filtered and concentrated. The residue was purified by flash column (0-25% of EtOAc in PE) to give **I-BA8a** (300 mg) and **I-BA8** (300 mg) as solids.

### I-BA8a:

**¹H NMR** (400 MHz, CDCl₃) δ_{H} 4.27-4.25 (m, 1H), 2.00-1.60 (m, 7H), 1.50-1.28 (m, 14H), 1.26 (s, 3H), 1.17 (d, *J =* 6.4 Hz, 3H), 1.08-0.92 (m, 4H), 0.90 (s, 3H), 0.88-0.75 (m, 5H).

### I-BA8:

**¹H NMR** (400 MHz, CDCl₃) δ_{H} 4.18-4.12 (m, 1H), 2.00-1.60 (m, 11H), 1.50-1.28 (m, 9H), 1.26 (s, 3H), 1.18 (d, *J =* 6.8 Hz, 3H), 1.15-0.88 (m, 10H), 0.85 (s, 3H).

### Synthesis of I-BA9

To a solution of **I-BA8** (100 mg, 0.2 mmol) in DCM (5 mL) was added silica gel (200 mg) and PCC (123 mg, 0.6 mmol) at 20°C. The reaction mixture was stirred for 2h. The mixture was filtered and the filter cake was washed with DCM (3 x 50 mL). The mother liquor was concentrated. The residue was purified by flash column (0-10% of EtOAc in PE) to give **I-BA9** (50 mg) as a solid.

**¹H NMR** (400 MHz, CDCl₃) δ_{H} 2.43 (d, *J =* 9.6 Hz, 1H), 2.10 (s, 3H), 1.95-1.60 (m, 9H), 1.50-1.28 (m, 8H), 1.26 (s, 3H), 1.24-1.07 (m, 6H), 1.05 (s, 3H), 1.03-0.80 (m, 5H).

### Example I-204a: 1-(2-((2R,4aS,4bR,6aS,7R,11aS,11bR,13aR)-2-hydroxy-2,6a-dimethyloctadecahydro-1H-cyclohepta[a]phenanthren-7-yl)-2-oxoethyl)-1H-pyrazole-4-carbonitrile (I-BA11)

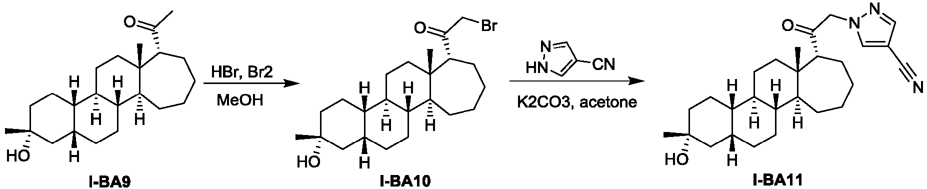

### Synthesis of I-BA10

To a solution of **I-BA9** (30 mg, 0.1 mmol) in MeOH (2 mL) was added HBr (2 mg, 24.7 µmol) and Br₂ (15 mg, 0.1 mmol) at 15°C. The reaction mixture was stirred for 1h at 15°C. The mixture was added into saturated NaHCO₃ (20 mL). The aqueous layer was extracted with EtOAc (3 x 20 mL). The combined organic layer was washed with saturated brine (50 mL), dried over anhydrous Na₂SO₄, filtered and concentrated to give **I-BA10** (20 mg) as an oil.

**¹H NMR** (400 MHz, CDCl₃) δ_{H} 4.00-3.92 (m, 2H), 2.80 (d, *J =* 9.2 Hz, 1H), 1.95-1.60 (m, 12H), 1.50-1.28 (m, 12H), 1.26 (s, 3H), 1.24-1.08 (m, 2H), 1.06 (s, 3H), 1.04-0.90 (m, 2H).

### Synthesis of I-BA11

To a solution of **I-BA10** (20 mg, 0.047 mmol) in acetone (2 mL) were added K₂CO₃ (13 mg, 0.1 mmol) and 1H-pyrazole-4-carbonitrile (9 mg, 0.1 mmol) at 15°C. The reaction mixture was stirred for 1h at 15°C. The reaction mixture was filtered and the mother liquor was concentrated. The residue was purified by flash column (0-30% of EtOAc in PE) to give **I-BA11** (10.3 mg, 50%) as a solid.

The structure was confirmed by NOE.

**¹H NMR** (400 MHz, CDCl₃) δ_{H} 7.86 (s, 1H), 7.81 (s, 1H), 5.10-4.90 (m, 2H), 2.60 (d, *J =* 8.8 Hz, 1H), 2.00-1.58 (m, 12H), 1.51-1.28 (m, 11H), 1.26 (s, 3H), 1.24-1.12 (m, 2H), 1.10 (s, 3H), 1.08-0.85 (m, 3H); **MS ESI** calcd. for C₂₇H₃₈N₃O [M+H-H₂O]⁺ 420, found 420.

### Example I-205a: Synthesis of 1-((2R,4aS,4bR,6aS,7S,11aS,11bR,13aR)-2-hydroxy-2,6a-dimethyloctadecahydro-1H-cyclohepta[a]phenanthren-7-yl)ethan-1-one (I-BA9a)

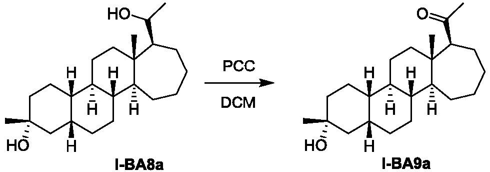

To a solution of **I-BA8a** (150 mg, 0.4 mmol) in DCM (10 mL) was added silica gel (200 mg) and PCC (185 mg, 0.8 mmol) at 20°C. The reaction mixture was stirred for 1h at 20°C. The mixture was filtered and the filter cake was washed with DCM (3 x 20 mL). The mother liquor was concentrated. The residue was purified by flash column (0-10% of EtOAc in PE) to give **I-BA9a** (100 mg, 67%) as a solid.

The structure was confirmed by NOE.

**¹H NMR** (400 MHz, CDCl₃) δ_{H} 2.40 (d, *J =* 10 Hz, 1H), 2.14 (s, 3H), 1.95-1.60 (m, 10H), 1.50-1.28 (m, 9H), 1.26 (s, 3H), 1.24-1.07 (m, 2H), 1.00 (s, 3H), 0.98-0.80 (m, 7H); **MS ESI** calcd. for C₂₃H₃₇O[M+H-H₂O]⁺ 329, found 329.

### Example I-205: Synthesis of 1-(2-((2R,4aS,4bR,6aS,7S,11aS,11bR,13aR)-2-hydroxy-2,6a-dimethyloctadecahydro-1H-cyclohepta[a]phenanthren-7-yl)-2-oxoethyl)-1H-pyrazole-4-carbonitrile (I-BA11a)

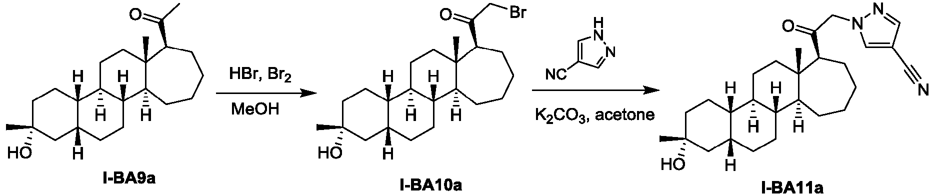

### Synthesis of I-BA10a

To a solution of **I-BA9a** (40 mg, 0.1 mmol) in MeOH (2 mL) was added HBr (10 mg, 0.049 mmol, 40%) and Br₂ (20 mg, 0.1 mmol) at 15°C. The reaction mixture was stirred for 1h to give an oil. The reaction mixture was added into saturated NaHCO₃ (20 mL). The aqueous layer was extracted with EtOAc (3 x 20 mL). The combined organic layer was washed with saturated brine (50 mL), dried over anhydrous Na₂SO₄, filtered and concentrated to give **I-BA10a** (50 mg) as an oil, which was used directly for next step.

### Synthesis of I-BA11a

To a solution of **I-BA10a** (50 mg, 0.2 mmol) in acetone (5 mL) was added K₂CO₃ (33 mg, 0.2 mmol) and 1H-pyrazole-4-carbonitrile (22 mg, 0.2 mmol) at 15°C. The reaction mixture was stirred for 2h at 15°C. The reaction mixture was filtered and the filter cake was washed with DCM (3 x 20 mL). The mother liquor was concentrated and purified by flash column (0-40% of EtOAc in PE) to give **I-BA11a** (10 mg, 19%) as a solid.

The structure was confirmed by NOE.

**¹H NMR** (400 MHz, CDCl₃) δ_{H} 7.82 (s, 2H), 4.99 (s, 2H), 2.41 (d, *J =* 11.6 Hz, 1H), 2.00-1.58 (m, 12H), 1.51-1.28 (m, 10H), 1.26 (s, 3H), 1.24-1.12 (m, 2H), 1.03 (s, 3H), 1.00-0.85 (m, 4H); **MS ESI** calcd. for C₂₇H₃₈N₃O [M+H-H₂O]⁺ 420, found 420.

### Example I-206: Synthesis of 1-((2R,4aS,4bR,6aS,7R,11aS,11bR,13aR)-2-hydroxy-2,6a-dimethyloctadecahydro-1H-cyclohepta[a]phenanthren-7-yl)ethan-1-one (I-BA9)

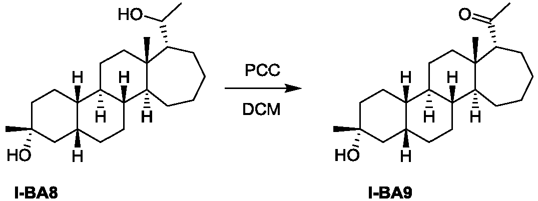

To a solution of **I-BA8** (100 mg, 0.2 mmol) in DCM (5 mL) was added silica gel (200 mg) and PCC (123 mg, 0.6 mmol) at 20°C. The reaction mixture was stirred for 2h. The mixture was filtered and the filter cake was washed with DCM (3 x 50 mL). The mother liquor was concentrated. The residue was purified by flash column (0-10% of EtOAc in PE) to give **I-BA9** (50 mg) as asolid.

**¹H NMR** (400 MHz, CDCl₃) δ_{H} 2.43 (d, *J =* 9.6 Hz, 1H), 2.10 (s, 3H), 1.95-1.60 (m, 9H), 1.50-1.28 (m, 8H), 1.26 (s, 3H), 1.24-1.07 (m, 6H), 1.05 (s, 3H), 1.03-0.80 (m, 5H).

### Example I-207: Synthesis of 1-(2-((2R,4aS,4bR,6aS,7S,11aS,11bR,13aR)-2-hydroxy-2,6a-dimethyloctadecahydro-1H-cyclohepta[a]phenanthren-7-yl)-2-oxoethyl)-1H-pyrazole-4-carbonitrile (I-BA11a)

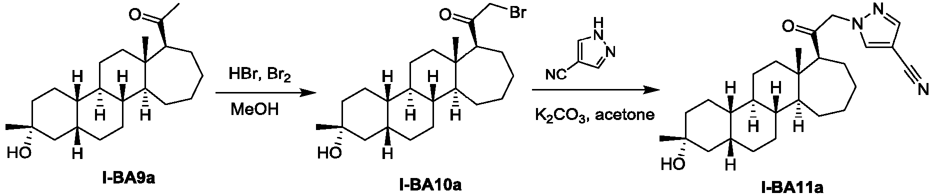

### Synthesis of I-BA10a

To a solution of **I-BA9a** (40 mg, 0.1 mmol) in MeOH (2 mL) was added HBr (10 mg, 0.049 mmol, 40%) and Br₂ (20 mg, 0.1 mmol) at 15°C. The reaction mixture was stirred for 1h. The reaction mixture was added into saturated NaHCO₃ (20 mL). The aqueous layer was extracted with EtOAc (3 x 20 mL). The combined organic layer was washed with saturated brine (50 mL), dried over anhydrous Na₂SO₄, filtered and concentrated to give **I-BA10a** (50 mg) as an oil, which was used directly for next step.

### Synthesis of I-BA11a

To a solution of **I-BA10a** (50 mg, 0.2 mmol) in acetone (5 mL) was added K₂CO₃ (33 mg, 0.2 mmol) and 1H-pyrazole-4-carbonitrile (22 mg, 0.2 mmol) at 15°C. The reaction mixture was stirred for 2h at 15°C. The reaction mixture was filtered and the filter cake was washed with DCM (3 x 20 mL). The mother liquor was concentrated and purified by flash column (0-40% of EtOAc in PE) to give **I-BA11a** (10 mg, 19%) as a solid.

The structure was confirmed by NOE.

**¹H NMR** (400 MHz, CDCl₃) δ_{H} 7.82 (s, 2H), 4.99 (s, 2H), 2.41 (d, *J =* 11.6 Hz, 1H), 2.00-1.58 (m, 12H), 1.51-1.28 (m, 10H), 1.26 (s, 3H), 1.24-1.12 (m, 2H), 1.03 (s, 3H), 1.00-0.85 (m, 4H); **MS ESI** calcd. for C₂₇H₃₈N₃O [M+H-H₂O]⁺ 420, found 420.

### Example I-208: Synthesis of (2R,4aS,4bR,6aS,7R,11aS,11bR,13aR)-2,6a-dimethyloctadecahydro-1H-cyclohepta[a]phenanthrene-2,7-diol (I-BA12)

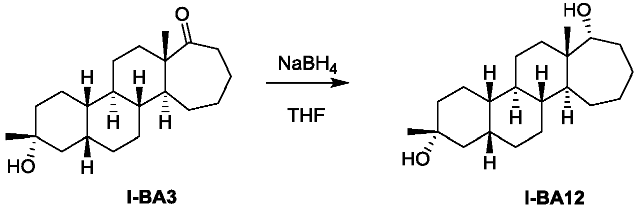

To a solution of **I-BA3** (200 mg, 0.6 mmol) in MeOH (5 mL) was added NaBH₄ (28.6 mg, 0.8 mmol) at 15°C. The reaction mixture was stirred for 1h at 15°C. Saturated NH₄ Cl (10 mL) was added to the reaction mixture. The aqueous layer was extracted with EtOAc (3 x 20 mL). The combined organic layer was washed with saturated brine (20 mL), dried over anhydrous Na₂SO₄, filtered and concentrated under reduced pressure. The residue was purified by flash column (50% EtOAc in PE) to afford **I-BA12** (190 mg, 95%) as a solid. **¹H NMR** (400 MHz, CDCl₃) δ_{H} 3.50-3.45 (m, 1H), 1.94-1.65 (m, 9H), 1.56-1.17 (m, 18H), 1.15-0.82 (m, 8H); **MS ESI** calcd. for C₂₁H₃₃ [M+H-H₂O- H₂O]⁺ 285, found 285. The structure was confirmed by NOE.

### Example I-209: Synthesis of (2R,4aS,4bR,6aS,7S,11aS,11bR,13aR)-2-hydroxy-2,6a-dimethyloctadecahydro-1H-cyclohepta[a]phenanthrene-7-carbonitrile (I-BA16) & (2R,4aS,4bR,6aS,7R,11aS,11bR,13aR)-2-hydroxy-2,6a-dimethyloctadecahydro-1H-cyclohepta[a]phenanthrene-7-carbonitrile (I-16a)

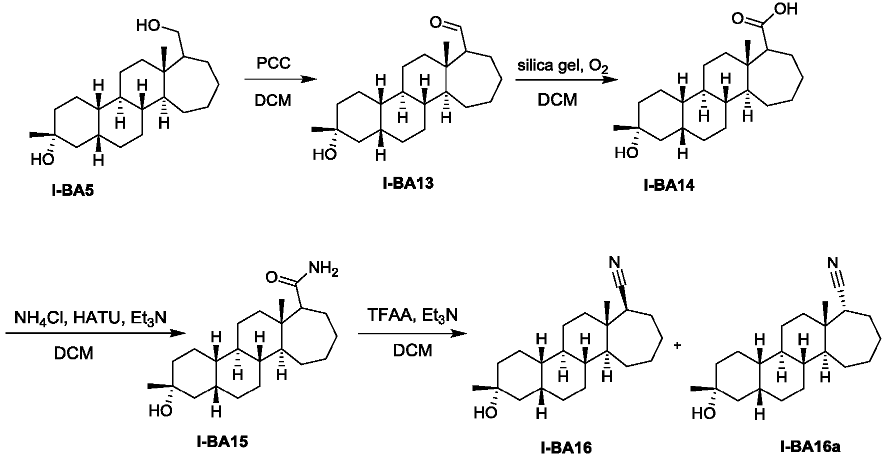

### Synthesis of I-BA13

To a solution of **I-BA5** (1.6 g, 4.8 mmol) in DCM (20 mL) was added silica gel (4 g) and PCC (2.05 g, 9.6 mmol) at 20°C. The reaction mixture was stirred for 1h. The reaction mixture was filtered and the filter cake was washed with DCM (3 x 50 mL). The mother liquor was concentrated to give **I-BA13** (2 g) as a solid, which was used directly for the next step without further purification.

### Synthesis of I-BA14

To a solution of **I-BA13** (2 g) in DCM (20 mL) was added silica gel (5 g). The reaction mixture was concentrated for 10 minutes at 50°C to give the product. The residue was purified by flash column (0-50% of EtOAc in PE) to give recovered **I-BA13** (120 mg) and **I-BA14** (800 mg) as both a solid.

**¹H NMR** (400 MHz, CDCl₃) δ_{H} 2.46-2.40 (m, 0.5H), 2.26 (dd, *J =* 4.0, 12.0 Hz, 0.5H), 2.00-1.60 (m, 12H), 1.53-1.40 (m, 11H), 1.38-1.26 (m, 4H), 1.24-1.15 (m, 2H), 1.14-1.10 (m, 1H), 1.08-0.95 (m, 2H), 0.93-0.80 (m, 3H).

### Synthesis of I-BA15

To a solution of **I-BA14** (400 mg, 1.1 mmol) in DCM (10 mL) were added HATU (649 mg, 1.7 mmol) and Et₃N (575 mg, 5.7 mmol) at 20°C. The reaction mixture was stirred for 30 minutes at 20°C. NH₄Cl (97.3 mg, 1.8 mmol) was added into the reaction mixture. The reaction mixture was stirred for 16 hours at 20°C. The reaction mixture was added into water (100 mL). The aqueous layer was extracted with DCM (3 x 50 mL). The combined organic layer was washed with saturated brine (100 mL), dried over anhydrous Na₂SO₄, filtered and concentrated. The residue was purified by flash column (0-60% of EtOAc in PE) to give **I-BA15** (200 mg, 51%) as a solid.

**¹H NMR** (400 MHz, CDCl₃) δ_{H} 5.46-5.31 (m, 2H), 2.24 (d, *J =* 9.2 Hz, 1H), 1.95-1.59 (m, 12H), 1.50-1.10 (m, 14H), 1.08 (s, 3H), 1.07-0.80 (m, 5H).

### Synthesis of I-BA16 and I-BA16a

To a solution of **I-BA15** (80 mg, 0.2 mmol) in DCM (5 mL) were added Et₃N (139 mg, 1.4 mmol) and TFAA (144 mg, 0.7 mmol) at 0°C. The reaction mixture was stirred at 20°C for 2 hours. The mixture was added into saturated NaHCO₃ (50 mL). The aqueous layer was extracted with DCM (3 x 50 mL). The combined organic layer was washed with saturated brine (100 mL), dried over anhydrous Na₂SO₄, filtered and concentrated. The residue was purified by flash column (0-10% of EtOAc in PE) to give **I-BA16** (50 mg) and **I-BA16a** (7 mg) both as a solid.

**I-BA16** (50 mg) was purified by prep-HPLC (Column: Xbridge 150*30mm*10µm, Condition: water (10mM NH₄HCO₃)-ACN, Begin B: 55, End B: 85, Gradient Time (min): 7, 100%B Hold Time (min): 1, FlowRate (mL/min): 25) to give **I-BA16** (4 mg) as a solid.

### I-BA16:

**¹H NMR** (400 MHz, CDCl₃) δ_{H} 2.27 (dd, *J* = 2.8, 11.2 Hz, 1H), 2.03-1.60 (m, 12H), 1.51-1.28 (m, 10H), 1.27 (s, 3H), 1.24-1.05 (m, 2H), 1.02 (s, 3H), 1.00-0.80 (m, 4H).

The structure was confirmed by NOE.

**LC-ELSD/MS** Rt = 1.085 min in 2.0 min chromatography, 30-90AB_2 min_E. (Mobile Phase: 1.5mL/4L TFA in water (solvent A) and 0.75mL/4L TFA in acetonitrile (solvent B), using the elution gradient 30%-90% (solvent B) over 0.9 minutes and holding at 90% for 0.6 minutes at a flow rate of 1.2 mL/min; Column: Xtimate C18 2.1*30mm,3µm; Wavelength: UV 220nm ; Column temperature: 50°C; MS ionization: ESI; Detector: PDA&ELSD), purity 99%, MS ESI calcd. for C₂₂H₃₄N [M+H-H₂O]⁺ 312, found 312.

### I-BA16a:

**¹H NMR** (400 MHz, CDCl₃) δ_{H} 2.58-2.52 (m, 1H), 2.00-1.60 (m, 14H), 1.51-1.28 (m, 10H), 1.26 (s, 3H), 1.24-0.98 (m, 4H), 0.96 (s, 3H); **MS ESI** calcd. for C₂₂H₃₄N [M+H-H₂O]⁺ 312, found 312

The structure was confirmed by NOE.

### Example I-210 and 1-210a: Synthesis of (2R,4aS,4bR,6aS,7S,11aS,11bR,13aR)-2-hydroxy-2,6a-dimethyl-N-phenyloctadecahydro-1H-cyclohepta[a]phenanthrene-7-carboxamide (I-BA17) & (2R,4aS,4bR,6aS,7R,11aS,11bR,13aR)-2-hydroxy-2,6a-dimethyl-N-phenyloctadecahydro-1H-cyclohepta[a]phenanthrene-7-carboxamide (I-BA17a)

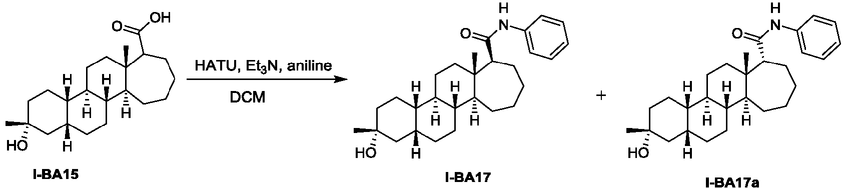

To a solution of **I-BA15** (200 mg, 0.6 mmol) in DCM (10 mL) were added HATU (327 mg, 0.9 mmol) and Et₃N (289 mg, 2.9 mmol) at 20°C. The reaction mixture was stirred at 20°C for 30 minutes. Aniline (85.4 mg, 0.9 mmol) was added into the reaction mixture. The reaction mixture was stirred for 48 hours at 20°C. The reaction mixture was diluted by DCM (50 mL) and added into water (100 mL). The aqueous layer was extracted with DCM (2 x 50 mL). The combined organic layer was washed with saturated brine (100 mL), dried over anhydrous Na₂SO₄, filtered and concentrated. The residue was purified by combi flash (0-20% of EtOAc in PE) to give a mixture of **I-BA17** and **I-BA17a** (200 mg) as a solid. The mixture of **I-BA17** and **I-BA17a** (200 mg) was purified by SFC (Column: Phenomenex-Amylose-1 (250mm*30mm, 5µm), Condition: 0.1%NH₃H₂O EtOH, Begin B: 40%, End B: 40%) to give **I-BA17** (27 mg) and **I-BA17a** (22 mg) both as a solid.

### I-BA17:

**¹H NMR** (400 MHz, CDCl₃) δ_{H} 7.51 (d, *J* = 7.2 Hz, 2H), 7.31 (t, *J =* 7.6 Hz, 2H), 7.08 (t, *J =* 7.6 Hz, 1H), 7.04 (s, 1H), 2.10-1.90 (m, 2H), 1.88-1.60 (m, 11H), 1.51-1.28 (m, 9H), 1.26 (s, 3H), 1.25-1.18 (m, 2H), 1.09 (s, 3H), 1.07-0.80 (m, 5H); **MS ESI** calcd. for C₂₈H₄₂NO₂ [M+H]⁺ 424, found 424

The structure was confirmed by NOE.

### I-B17a:

**¹H NMR** (400 MHz, CDCl₃) δ_{H} 7.50 (d, *J =* 8.0 Hz, 2H), 7.31 (t, *J =* 7.6 Hz, 2H), 7.09 (t, *J =* 7.2 Hz, 1H), 6.96 (s, 1H), 2.32 (d, *J =* 9.2 Hz, 1H), 2.00-1.60 (m, 12H), 1.50-1.28 (m, 10H), 1.26 (s, 3H), 1.24-1.15 (m, 2H), 1.13 (s, 3H), 1.12-0.85 (m, 4H); **MS ESI** calcd. for C₂₈H₄₀NO [M+H-H₂O]⁺ 406, found 406.

The structure was confirmed by NOE.

### Example I-211: Synthesis of (4aS,4bR,6aS,11aS,11bR,13aR)-6a-methylhexadecahydro-1H-cyclohepta[a]phenanthrene-2,7-dione (I-BB6)

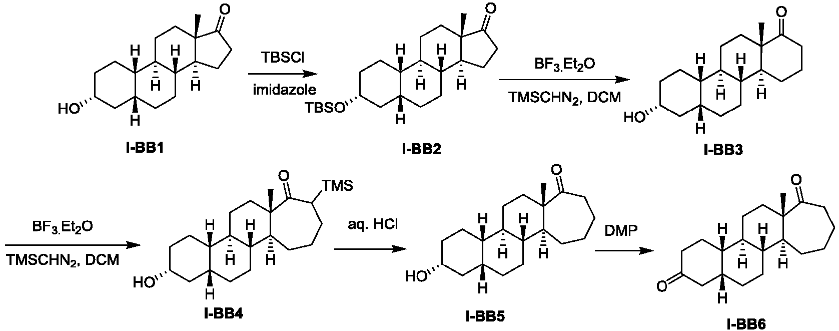

### Synthesis of I-BB2

To a solution of **I-BB1** (CAS# 33036-33-8) (50 g, 180 mmol) in DCM (500 mL) was added imidazole (18.3 g, 270 mmol) and TBSCl (40.6 g, 270 mmol) at 25°C. The solution was stirred at 25 °C for 16 hours. Water (300 mL) was added to the mixture. The aqueous phase was extracted with EtOAc (3 x 300 mL). The combined organic phase was washed with saturated brine (2 x 200 mL), dried over anhydrous Na₂SO₄, filtered and concentrated and was purified by silica gel chromatography (0-0.5% of EtOAc in PE) to afford **I-BB2** (100 g) as a solid.

### Synthesis of I-BB3

A solution of TMSCHN₂ (19.0 mL, 38.0 mmol, 2M in hexane) was added dropwise at -20°C to a stirred solution of **I-BB2** (5 g, 12.7 mmol) and BF₃·Et₂O (8.00 mL, 63.5 mmol) in dry CH₂Cl₂ (50 mL). The mixture was stirred at -15°C for 3 hrs under N₂. The reaction mixture was poured into ice-water (100 mL) and extracted with CH₂Cl₂ (2x100 mL). The combined organic phase was washed with brine (100 mL), dried over Na₂SO₄, concentrated and purified by flash column (0-30% EtOAc in PE) to give **I-BB3** (1.0 g, 27.1%) as an oil.

**¹H NMR** (400 MHz, CDCl₃) δ_{H} 3.71-3.53 (m, 1H), 2.70-2.55 (m, 1H), 2.25-2.15 (m, 1H), 2.11-1.90 (m, 2H), 1.88-1.48 (m, 13H), 1.46-1.15 (m, 8H), 1.08 (s, 3H), 1.07-0.83 (m, 3H).

### Synthesis of I-BB4

A solution of TMSCHN₂ (5.15 mL, 10.3 mmol, 2M in hexane) was added dropwise at -20°C to a stirred solution of **I-BB3** (1.0 g, 3.44 mmol) and BF₃·Et₂O (2.16 mL, 17.2 mmol) in dry CH₂Cl₂ (10 mL). The mixture was stirred at -15°C for 3 hrs under N₂. The reaction mixture was poured into ice-water (20 mL) and extracted with CH₂Cl₂ (2 x 20 mL). The combined organic phase was washed with brine (20 mL), dried over Na₂SO₄, and evaporated to give **I-BB4** (1.4 g) as an oil.

### Synthesis of I-BB5

To the solution of **I-BB4** (1.4 g, 3.7 mmol) in THF (20 mL) was added 2N HCl (1.85 mL). The mixture was stirred at 20 °C for 3 h. The mixture was quenched by NaHCO₃ (30 mL, saturated) and Na₂S₂O₃ (30 mL, saturated). The aqueous phase was extracted with EtOAc (2 x 50 mL). The combined organic phase was washed with water (2 x 50 mL), dried over anhydrous Na₂SO₄, filtered and concentrated and was purified by flash column (5~10% of EtOAc in PE) to give **I-BB5** (220 mg, 19.6%) as an oil.

**¹H NMR** (400 MHz, CDCl₃) δ_{H} 3.71-3.59 (m, 1H), 3.09-2.59 (m, 1H), 2.32-2.22 (m, 1H), 1.96-1.88 (m, 1H), 1.87-1.54 (m, 13H), 1.52-1.31 (m, 10H), 1.15-1.06 (m, 2H), 1.05 (s, 3H)

### Synthesis of I-BB6

To a solution of **I-BB5** (220 mg, 0.7 mmol) in DCM (10 mL) was added DMP (610 mg, 1.4 mmol). The mixture was stirred at 20°C for 0.5 hrs. The mixture was quenched by NaHCO₃ (30 mL, saturated) and Na₂S₂O₃ (30 mL, saturated). The organic layer was separated, dried over Na₂SO₄, filtered and concentrated and purified by flash column (0~30% EtOAc in PE) to give **I-BB6** (170 mg, 77.9%) as a solid. The solid (50 mg) was purified by HPLC separation (column:Xbridge 150*30mm*10um, gradient: 58-88% B (water(10mM NH₄HCO₃)-ACN), flow rate: 25 mL/min) to give **I-BB6** (7 mg, 14.0%) as a solid.

**¹H NMR** (400 MHz, CDCl₃) δ_{H} 3.05-3.00 (m, 1H), 2.65-2.55 (m, 1H), 2.35-2.08 (m, 7H), 1.86-1.61 (m, 8H), 1.55-1.39 (m, 6H), 1.29-1.10 (m, 4H), 1.09 (s, 3H); **MS ESI** calcd. for C₂₀H₃₁O₂ [M+H]+303, found 303.

### Example I-212: Synthesis of (2R,4aS,4bR,6aS,11aS,11bR,13aR)-2-hydroxy-2,6a-dimethyl-N-phenyl-2,3,4,4a,4b,5,6,6a,9,10,11,11a,11b,12,13,13a-hexadecahydro-1H-cyclohepta[a]phenanthrene-8-carboxamide (I-BC10)

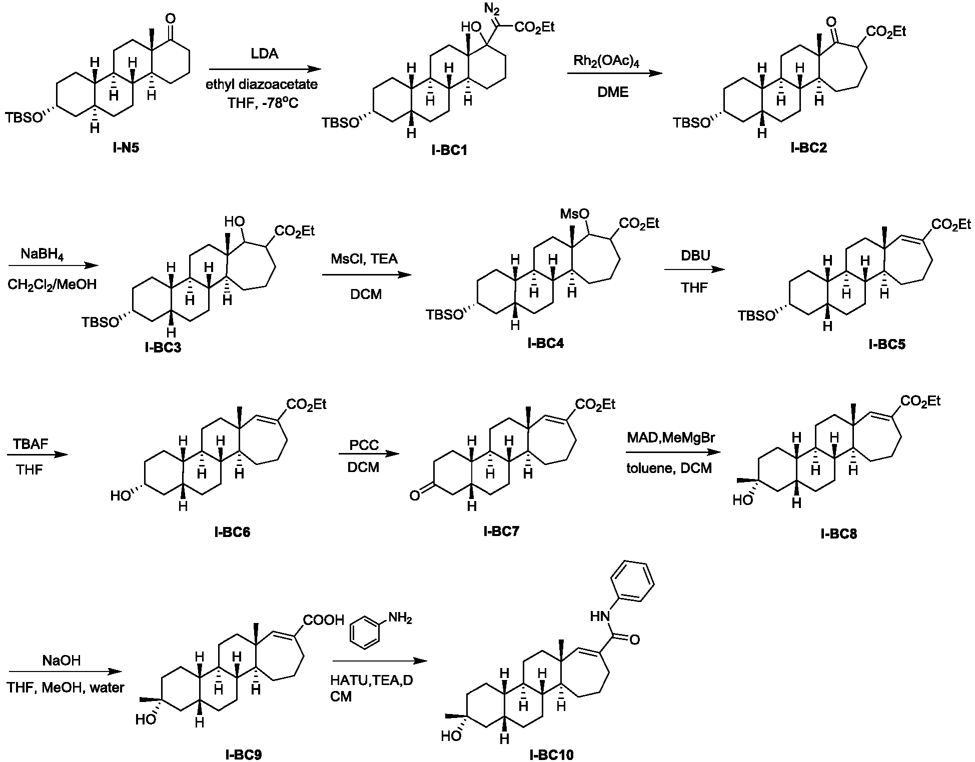

### Synthesis of I-BC1

A cold (-70°C) solution of di-isopropylamine (25.0 g, 247 mmol) in anhydrous THF (300 mL) was added butyllithium (98.8 mL, 247 mmol, 2.5M in n-hexane) drop-wise at -70°C over a period for 20 mins, during which the temperature was maintained below at -60°C. The internal temperature was allowed to 0 °C and stirred at 0°C for 1 h. To a stirred solution of **IN5** (20.0 g, 49.4 mmol) in anhydrous THF (240 mL) and ethyl diazoacetate (28.1 g, 247 mmol) was added LDA (247 mmol, 1 M) drop-wise at -70°C over a period for 30 min, during which the temperature was maintained below at -60°C. The mixture was stirred at -70°C for 3 hours. Then acetic acid (14.8 g, 247 mmol) in THF (120 mL) was added drop-wise at -70°C, then mixture reaction was then warm to 15°C and stirred for 12 h. Water (400 mL) was added, the aqueous solution extracted with EtOAc (2 x 400 mL). The combined organic layers were washed with brine (2 x 300 mL), dried over anhydrous Na₂SO₄, and evaporated under reduced pressure to give the product as an oil, and then purified by flash column (0~5% of EtOAc in PE) to give **I-BC1** (20.0 g, 78%) as a solid.

**¹H NMR** (400 MHz, CDCl₃) δ_{H} 4.28-4.15 (m, 2H), 3.63-3.53 (m, 1H), 1.95-1.50 (m, 13H), 1.35-1.20 (m, 20H), 0.93-0.80 (m, 8H), 0.05 (s, 6H).

### Synthesis of I-BC2

To a solution of **I-BC1** (20.0 g, 38.5 mmol) in DME (300 mL) was added 1, 1, 1-tris (acetyloxy) dirhodium-1-yl acetate (225 mg, 0.6 mmol) at 25°C. After stirring at 25°C for 2 h, the mixture was concentrated in vacuum to give **I-BC2** (18.0 g) as a solid.

**¹H NMR** (400 MHz, CDCl₃) δ_{H} 13.58 (s, 1H), 4.19 (q, *J =* 6.8 Hz, 1H), 3.62-3.58 (m, 1H), 2.54 (dd, *J* = 4, 16 Hz, 1H), 2.29-2.17 (m, 3H), 2.00-1.87 (m, 3H), 1.80-1.60 (m, 10H), 1.45-1.12 (m, 22H), 0.06-0.03 (m, 8H).

### Synthesis of I-BC3

To a solution of **I-BC2** (18.0 g, 36.6 mmol) in DCM (50 mL) and MeOH (150 mL) was added NaBH₄ (2.8 g, 73.2 mmol) in 5 portions at 0°C. After stirred at 0°C for 1 h, the reaction was quenched by saturated aqueous NH₄Cl (100 mL). The mixture was extracted with DCM (2 x 200 mL). The combined organic phase was washed with saturated brine (2 x 100 mL), dried over anhydrous Na₂SO₄, filtered and concentrated to give **I-BC3** (18.0 g) as an oil.

**¹H NMR** (400 MHz, CDCl₃) δ_{H} 4.18-4.14 (m, 2H), 3.57-3.49 (m, 2H), 2.25-1.25 (m, 20H), 1.20-0.80 (m, 20H), 0.05 (s, 6H).

### Synthesis of I-BC4

To a solution of **I-BC3** (18.0 g) in DCM (150 mL) was added TEA (73.7 g, 730 mmol) and 1-methyl-1H-imidazole (29.9 g, 365 mmol) at 20°C. The mixture was cooled to 0°C; MsCl (31.2 g, 273 mmol) in DCM (50 mL) was added. The reaction mixture was stirred at 20°C for 16 hours. The reaction mixture was quenched with water (300 mL). The mixture was extracted with DCM (2 x 200 mL). The combined organic phase was washed with brine (2 x 100 mL), dried over anhydrous Na₂SO₄, filtered and concentrated under vacuum. The residue was purified by flash column (0~5% of EtOAc in PE) to give **I-BC4** (3.1 g, 15%) as an oil. **¹H NMR** (400 MHz, CDCl₃) δ_{H} 4.82 (s, 1H), 4.18-4.14 (m, 2H), 3.64-3.52 (m, 1H), 3.10 (s, 3H), 2.99-2.93 (m, 1H), 2.05-1.63 (m, 10H), 1.35-1.12 (m, 15H), 0.92-0.81 (m, 15H), 0.05 (s, 7H).

### Synthesis of I-BC5

To a solution of **I-BC4** (3.0 g, 5.3 mmol) in THF (30 mL) was added DBU (7 mL) at 25°C. The reaction mixture was stirred at 25°C for 12 hours. The reaction mixture was poured into water (100 mL). The aqueous phase was extracted with EtOAc (3 x 100 mL). The combined organic layers was washed with saturated brine (2 x 100 mL), dried over anhydrous Na₂SO₄, filtered and concentrated. The residue was purified by flash column (0~2% of EtOAc in PE) to give **I-BC5** (2.0 g, 80%) as an oil.

**¹H NMR** (400 MHz, CDCl₃) δ_{H} 6.55 (s, 1H), 4.16 (q, *J =* 6.8 Hz, 2H), 3.65-3.50 (m, 1H), 2.58-2.48 (m, 1H), 2.38-2.25 (m, 1H), 2.12-1.89 (m, 2H), 1.87-1.78(m, 1H), 1.68-1.38 (m, 10H), 1.30-1.10 (m, 10H), 1.00-0.75 (m, 16H), 0.04 (s, 6H).

### Synthesis of I-BC6

To a solution of **I-BC5** (1.3 g, 2.7 mmol) in THF (20 mL) was added TBAF (4.26 g, 16.3 mmol) at 25°C. The reaction mixture was stirred at 55°C for 16 hours. The mixture was cooled and poured into saturated NH₄Cl (100 mL). The aqueous phase was extracted with EtOAc (2 x 100 mL). The combined organic phase was washed saturated brine (2 x 100 mL), dried over anhydrous Na₂SO₄, filtered and concentrated in vacuum to give **I-BC6** (1 g) as an oil.

**¹H NMR** (400 MHz, CDCl₃) δ_{H} 6.56 (s, 1H), 4.16 (q, *J =* 7.2 Hz, 2H), 3.70-3.55 (m, 1H), 2.55-2.45 (m, 1H), 2.35-2.25 (m, 1H), 1.90-1.59 (m, 11H), 1.50-1.35 (m, 4H), 1.31-1.16 (m, 9H), 1.05-0.91 (m, 6H).

### Synthesis of I-BC7

To a solution of **I-BC6** (1 g) in DCM (20 mL) were added silica gel (3 g) and PCC (1.19 g, 5.5 mmol) at 25°C. The reaction mixture was stirred at 25°C for 2 hours. The reaction mixture was filtered and the filter cake was washed with DCM (3 x 50 mL). The mother liquor was concentrated. The residue was purified by flash column (0~10 EtOAc in PE) to give **I-BC7** (660 mg, 66%) as an oil.

**¹H NMR** (400 MHz, CDCl₃) δ_{H} 6.57 (d, *J =* 1.2 Hz, 1H), 4.16 (q, *J =* 7.2 Hz, 2H), 2.61-2.50 (m, 2H), 2.40-2.06 (m, 6H), 1.95-1.59 (m, 7H), 1.51-1.29 (m, 11H), 1.27 (s, 3H), 1.21-0.95 (m, 2H).

### Synthesis of I-BC8

To a solution of BHT (2.46 g, 11.2 mmol) in toluene (5 mL) under nitrogen at 0°C was added trimethylaluminum (2 M in toluene, 2.8 mL, 5.6 mmol) dropwise. The mixture was stirred at 25°C for 1 h and used directly as a solution of MAD without further purification. To the MAD solution (5.60 mmol) was added a solution of **I-BC7** (650 mg, 1.8 mmol) in anhydrous DCM (10 mL) drop-wise at -70°C. After stirring at -70°C for 1 h under N₂, MeMgBr (1.8 mL, 5.4 mmol, 3M in ethyl ether) was added drop wise at -70°C. The resulting solution was stirred at -70°C for another 2 hrs. The reaction mixture was poured into saturated aqueous citric acid (50 mL) below 10°C and extracted with EtOAc (3 x 50 mL). The combined organic layer was dried over anhydrous Na₂SO₄, filtered and concentrated in vacuum to give the product which was purified by column (PE/EtOAc= 6/1) to give **I-BC8** (540 mg, 80%) as an oil.

**¹H NMR** (400 MHz, CDCl₃) δ_{H} 6.56 (s, 1H), 4.16 (q, *J =* 6.8 Hz, 2H), 2.58-2.48 (m, 1H), 2.35-2.25 (m, 1H), 2.03-1.95 (m, 1H), 1.92-1.59 (m, 6H), 1.51-1.20 (m, 18H), 1.04 (s, 3H), 1.02-0.85 (m, 5H).

### Synthesis of I-BC9

A solution of **I-BC8** (540 mg, 1.4 mmol) and NaOH (287 mg, 7.2 mmol) in THF/MeOH/water (5 mL/5 mL/5 mL) was stirred for 16 hours at 40°C. The reaction mixture was added into satyrated NH₄Cl (50 mL). The aqueous layer was extracted with EtOAc (3 x 20 mL). The combined organic layer was washed with saturated brine (100 mL), dried over anhydrous Na₂SO₄, filetered and concentrated to give **I-BC9** (450 mg, 90%) as a solid.

**¹H NMR** (400 MHz, CDCl₃) δ_{H} 6.69 (s, 1H), 2.58-2.48 (m, 1H), 2.35-2.25 (m, 1H), 1.95-1.59 (m, 9H), 1.50-1.35 (m, 6H), 1.34-1.20 (m, 10H), 1.06 (s, 3H), 1.05-0.80 (m, 3H).

### Synthesis of I-BC10

To a solution of **I-BC9** (200 mg, 0.6 mmol) in DCM (10 mL) were added HATU (329 mg, 0.9 mmol) and Et₃N (291 mg, 2.9 mmol) at 25°C. The reaction mixture was stirred at 25°C for 30 minutes. Aniline (85.9 mg, 0.9 mmol) was added into the reaction mixture. The reaction mixture was stirred at 20°C for 5 hours. The reaction mixture was partitioned between DCM (50 mL) and water (100 mL). The aqueous layer was extracted with DCM (2 x 50 mL). The combined organic layer was washed with saturated brine (100 mL), dried over anhydrous Na₂SO₄, filtered and concentrated. The residue was purified by combi flash (0-20% of EtOAc in PE) to give **I-BC10** (100 mg, 41%) as a solid.

**¹H NMR** (400 MHz, CDCl₃) δ_{H} 7.53 (d, *J =* 7.6 Hz, 2H), 7.33 (t, *J =* 8.4 Hz, 3H), 7.10 (t, *J =* 7.6 Hz, 1H), 6.06 (d, *J =* 1.6 Hz, 1H), 2.60-2.50 (m, 1H), 2.45-2.35 (m, 1H), 2.15-2.05 (m, 1H), 1.95-1.65 (m, 9H), 1.51-1.26 (m, 14H), 1.09-0.85 (m, 6H); **MS ESI** calcd. for C₂₈H₄₀NO₂ [M+H]⁺ 422, found 422.

### Example 1-213 and I-213a: Synthesis of (2R,4aS,4bR,6aR,8S,11aS,11bR,13aR)-2-hydroxy-2,6a-dimethyl-N-phenyloctadecahydro-1H-cyclohepta[a]phenanthrene-8-carboxamide (I-BC11) and (2R,4aS,4bR,6aR,8S,11aS,11bR,13aR)-2-hydroxy-2,6a-dimethyl-N-phenyloctadecahydro-1H-cyclohepta[a]phenanthrene-8-carboxamide (I-BC11a)

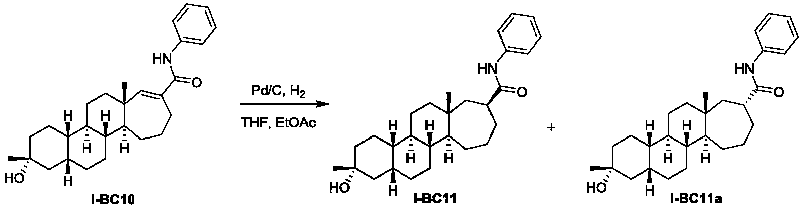

To a solution of **I-BC10** (70 mg, 0.17 mmol) in THF (5 mL) was added Pd/C (20 mg, dry). 103421 Pa (15 psi) of hydrogen at 25°C for 16 hrs was applied to the resulting solution. The reaction mixture was filtered and concentrated under vacuum to give a solid (70 mg, 100%). The mixture was purified by SFC (Column: Phenomenex-Amylose-1 (250mm*30mm, 5mm), Condition: 0.1%NH₃H₂O EtOH, Begin B: 40%, End B: 40%, Flow Rate(mL/min): 50) to give **I-BC11** (rt = 3.284 min, 42 mg, 60%) and **I-BC11a** (10 mg, 14%) both as solids.

### I-BC11:

**¹H NMR** (400 MHz, CDCl₃) δ_{H} 7.51 (d, *J =* 8.0 Hz, 2H), 7.31 (t, *J =* 8.0 Hz, 2H), 7.12-7.06 (m, 1H), 7.05-7.00 (m, 1H), 2.51-2.48 (m, 1H), 2.10-1.60 (m, 12H), 1.50-1.25 (m, 14H), 1.24-0.94 (m, 5H), 0.88 (s, 3H); **MS ESI** calcd. for C₂₈H₄₂NO₂ [M+H]⁺ 424, found 424.

### I-BC11a:

**¹H NMR** (400 MHz, CDCl₃) δ_{H} 7.51 (d, *J =* 7.6 Hz, 2H), 7.31 (t, *J =* 8.0 Hz, 2H), 7.12-7.00 (m, 2H), 2.52-2.49 (m, 1H), 1.96-1.60 (m, 11H), 1.50-1.24 (m, 14H), 1.15-0.80 (m, 9H); **MS ESI** calcd. for C₂₈H₄₀NO [M+H-H₂O]⁺ 406, found 406. NOE signal between H1 and H3 indicates H1 and H3 should be cis-.

### Example I-214 and I-214a: Synthesis of 1-((2R,4aS,4bR,6aR,8S,11aS,11bR,13aR)-2-hydroxy-2,6a-dimethyloctadecahydro-1H-cyclohepta[a]phenanthren-8-yl)ethan-1-one (I-BC14a) and 1-((2R,4aS,4bR,6aR,8R,11aS,11bR,13aR)-2-hydroxy-2,6a-dimethyloctadecahydro-1H-cyclohepta[a]phenanthren-8-yl)ethan-1-one (I-BA14b)

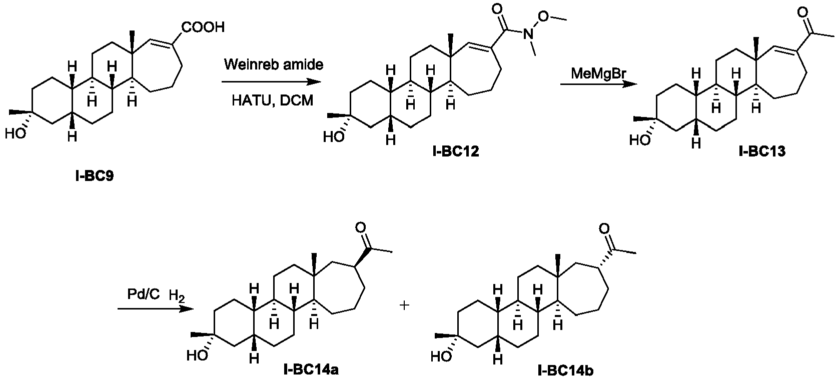

### Synthesis of I-BC12

To a solution of **I-BC9** (250 mg, 0.7 mmol) in DCM (20 mL) were added HATU (410 mg, 1.1 mmol), TEA (363 mg, 3.6 mmol) and methoxy(methyl)amine (139 mg, 1.4 mmol) at 25°C. The mixture was stirred at 40°C for 16 hrs. The reaction was poured into water (50 mL). The aqueous phase was extracted with DCM (2 x 50 mL). The combined organic phase was washed with saturated brine (100 mL), dried over anhydrous Na₂SO₄, filtered and concentrated in vacuum to afford **I-BC12** (250 mg) as an oil.

**¹H NMR** (400 MHz, CDCl₃) δ_{H} 5.62 (s, 1H), 3.65 (s, 3H), 3.21 (s, 3H), 2.40-2.20 (m, 2H), 2.10-1.95 (m, 1H), 1.95-1.59 (m, 8H), 1.45-1.28 (m, 12H), 1.27 (s, 3H), 1.04 (s, 3H), 1.00-0.85 (m, 3H).

### Synthesis of I-BC13

To a solution of **I-BC12** (250 mg, 0.6 mmol) in anhydrous THF (5 mL) was added MeMgBr (1 mL, 3.0 mmol, 3M in ether) drop-wise at 0°C under N₂. The reaction mixture was stirred at 25°C for 30 min. The reaction was slowly poured into saturated citric acid (50 mL). The aqueous phase was extracted with EtOAc (2 x 50 mL). The combined organic phase was washed with saturated brine (2 x 50 mL), dried over anhydrous Na₂SO₄, filtered and concentrated. The residue was purified by flash column (0~20% of EtOAc in PE) to afford **I-BC13** (150 mg, 70%) as a solid.

**¹H NMR** (400 MHz, CDCl₃) δ_{H} 6.39 (s, 1H), 2.60-2.50 (m, 1H), 2.29 (s ,3H), 1.95-1.60 (m, 9H), 1.50-1.24 (m, 14H), 1.08 (s, 3H), 1.05-0.75 (m, 5H).

### Synthesis of I-BC14a and I-BC14b

To a solution of **I-BC13** (150 mg, 0.4 mmol) in THF (5 mL) and EtOAc (5 mL) was added dry Pd/C (20 mg) at 25°C. The reaction mixture was stirred at 25°C for 16 hours under 103421 Pa (15 psi).

The mixture was filtered and the mother liquor was concentrated to give an oil (150 mg, 100%). The oil (50 mg, 0.14 mmol) was purified by flash column (0-12% of EtOAc in PE) to give **I-BC14a** (2 mg, 4%) and **I-BC14b** (15 mg, 30%) both as a solid.

### I-BC14a:

**¹H NMR** (400 MHz, CDCl₃) δ_{H} 2.75-2.60 (m, 1H), 2.13 (s, 3H), 1.90-1.60 (m, 7H), 1.51-1.35 (m, 9H), 1.34-1.15 (m, 9H), 1.10-0.80 (m, 9H); **MS ESI** calcd. for C₂₃H₃₇O[M+H-H₂O]⁺ 329, found 329.

NOE signal between H1 and H3 indicates H1 and H3 should be cis-.

### I-BC14b:

**¹H NMR** (400 MHz, CDCl₃) δ_{H} 2.75-2.60 (m, 1H), 2.13 (s, 3H), 1.90-1.60 (m, 9H), 1.51-1.35 (m, 8H), 1.34-1.15 (m, 9H), 1.14-0.94 (m, 5H), 0.84 (s, 3H); **MS ESI** calcd. for C₂₃H₃₇O [M+H-H₂O]⁺ 329, found 329.

### Example I-215 and I-215a: Synthesis of 1-(2-((2R,4aS,4bR,6aR,8S,11aS,11bR,13aR)-2-hydroxy-2,6a-dimethyloctadecahydro-1H-cyclohepta[a]phenanthren-8-yl)-2-oxoethyl)-1H-pyrazole-4-carbonitrile (I-BC17) and 1-(2-((2R,4aS,4bR,6aR,8R,11aS,11bR,13aR)-2-hydroxy-2,6a-dimethyloctadecahydro-1H-cyclohepta[a]phenanthren-8-yl)-2-oxoethyl)-1H-pyrazole-4-carbonitrile (I-BC17a)

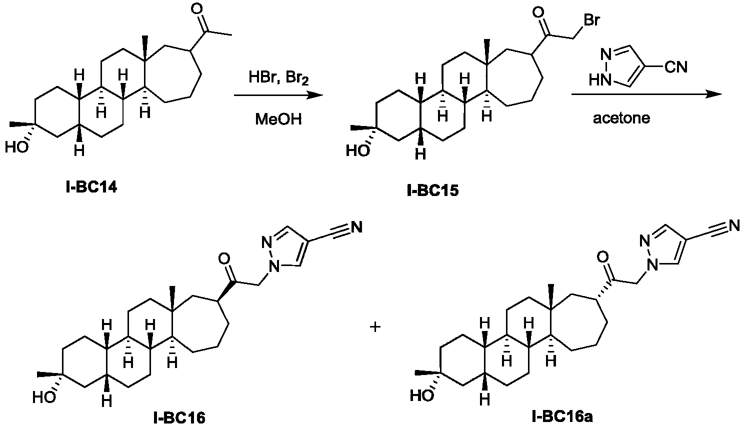

### Synthesis of I-BC15

To a solution of **I-BC14** (100 mg, 0.29 mmol) in MeOH (10 mL) were added HBr (10 mg, 0.05 mmol, 40%) and Br₂ (48.4 mg, 0.3 mmol) at 0°C. The reaction mixture was stirred at 25°C for 16 hours. The mixture was added into saturated NaHCO₃/water (20 mL/20 mL). The aqueous layer was extracted with EtOAc (3 x 20 mL). The combined organic layer was washed with saturated brine (100 mL), dried over anhydrous Na₂SO₄, filtered and concentrated to give **I-BC15** (120 mg), which was used directly for the next step without further purification.

### Synthesis of I-BC16 & I-BC16a

To a solution of **I-BC15** (120 mg) in acetone (5 mL) were added K₂CO₃ (77.8 mg, 0.56 mmol) and 1H-pyrazole-4-carbonitrile (52.5 mg, 0.56 mmol) at 25°C. The reaction mixture was stirred at 25°C for 16 hours. The mixture was added into saturated NH₄Cl (100 mL). The aqueous layer was extracted with EtOAc (3 x 50 mL). The combined organic layer was washed with water (100 mL) and saturated brine (100 mL), dried over anhydrous Na₂SO₄, filtered and concentrated. The residue was purified by pre-HPLC (Column: Waters Xbridge 150*25 5µ, Condition: water(10mM NH₄HCO₃)-ACN, Begin B: 58, End B: 88, Gradient Time(min): 8, 100%B Hold Time(min):2, Flow Rate(mL/min): 25) to give **I-BC16a** (13 mg, 11%) and **I-BC16** (13 mg, 11%) both as a solid.

### I-BC16a:

**¹H NMR** (400 MHz, CDCl₃) δ_{H} 7.87 (s, 1H), 7.82 (s, 1H), 5.04 (s, 2H), 2.70-2.60 (m, 1H), 1.95-1.60 (m, 11H), 1.51-1.28 (m, 12H), 1.26 (s, 3H), 1.15-0.90 (m, 5H), 0.87 (s, 3H); **MS ESI** calcd. for C₂₇H₃₈N₃O [M+H-H₂O]⁺ 420, found 420. NOE signal between H1 and H2 indicates H1 and H2 should be cis-.

### I-BC16:

**¹H NMR** (400 MHz, CDCl₃) δ_{H} 7.87 (s, 1H), 7.82 (s, 1H), 5.04 (q, *J =* 18.0 Hz, 2H), 2.70-2.60 (m, 1H), 1.95-1.60 (m, 11H), 1.51-1.20 (m, 14H), 1.15-0.90 (m, 6H), 0.87 (s, 3H); **MS ESI** calcd. for C₂₇H₃₈N₃O [M+H-H₂O]⁺ 420, found 420.

### Example I-215: Biological data

### Steroid Inhibition of TBPS Binding

[³⁵S]-t-Butylbicyclophosphorothionate (TBPS) binding assays using rat brain cortical membranes in the presence of 5 mM GABA has been described (Gee et al, J. Pharmacol. Exp. Ther. 1987, 241, 346-353; Hawkinson et al, Mol. Pharmacol. 1994, 46, 977-985; Lewin, A.H et al., Mol. Pharmacol. 1989, 35, 189-194).

Briefly, cortices are rapidly removed following decapitation of carbon dioxide-anesthetized Sprague-Dawley rats (200-250 g). The cortices are homogenized in 10 volumes of ice-cold 0.32 M sucrose using a glass/teflon homogenizer and centrifuged at 1500 x g for 10 min at 4 °C. The resultant supernatants are centrifuged at 10,000 x *g* for 20 min at 4 °C to obtain the P2 pellets. The P2 pellets are resuspended in 200 mM NaCl/50 mM Na-K phosphate pH 7.4 buffer and centrifuged at 10,000 x g for 10 min at 4 °C. This washing procedure is repeated twice and the pellets are resuspended in 10 volumes of buffer. Aliquots (100 mL) of the membrane suspensions are incubated with 3 nM [³⁵S]-TBPS and 5 mL aliquots of test drug dissolved in dimethyl sulfoxide (DMSO) (final 0.5%) in the presence of 5 mM GABA. The incubation is brought to a final volume of 1.0 mL with buffer. Nonspecific binding is determined in the presence of 2 mM unlabeled TBPS and ranged from 15 to 25 %. Following a 90 min incubation at room temp, the assays are terminated by filtration through glass fiber filters (Schleicher and Schuell No. 32) using a cell harvester (Brandel) and rinsed three times with ice-cold buffer. Filter bound radioactivity is measured by liquid scintillation spectrometry. Non-linear curve fitting of the overall data for each drug averaged for each concentration is done using Prism (GraphPad). The data are fit to a partial instead of a full inhibition model if the sum of squares is significantly lower by F-test. Similarly, the data are fit to a two component instead of a one component inhibition model if the sum of squares is significantly lower by F-test. The concentration of test compound producing 50% inhibition (IC₅₀) of specific binding and the maximal extent of inhibition (Iₘₐₓ) are determined for the individual experiments with the same model used for the overall data and then the means ± SEM.s of the individual experiments are calculated. Picrotoxin serves as the positive control for these studies as it has been demonstrated to robustly inhibit TBPS binding.

Various compounds are or can be screened to determine their potential as modulators of [³⁵S]-TBPS binding *in vitro.* These assays are or can be performed in accordance with the above.

**In Tables I-4, I-5,** and **I-6** below, A indicates a µTBPS IC₅₀ (uM) < 0.01 µM, B indicates a TBPS IC₅₀ (uM) of 0.01 µM to < 0.1 µM, C indicates a TBPS IC₅₀ (µM) of 0.1 µM to < 1.0 µM, D indicates a TBPS IC₅₀ (µM) of 1.0 µM to < 10 µM, and E means ≥ 10 µM.

**Table I-4**

| **Example** | **ID** | **STRUCTURE** | **TBPS IC₅₀ (µM)** |
|---|---|---|---|
| **I-1** | **I-A7** | 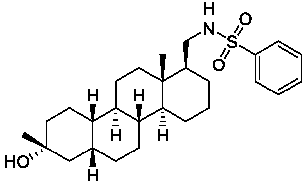 | B |
| **I-2** | **I-A8** | 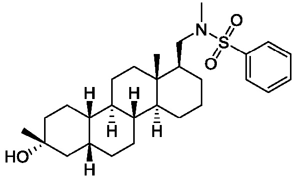 | B |
| **I-3** | **I-B6** | 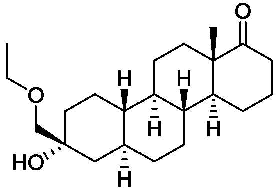 | D |
| **I-4** | **I-C4** | 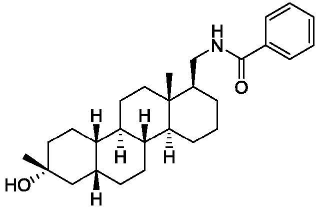 | C |
| **I-5** | **I-C5** | 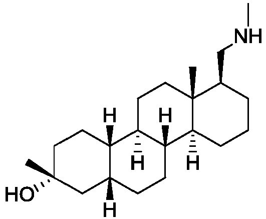 | D |
| **I-6** | **I-C6** | 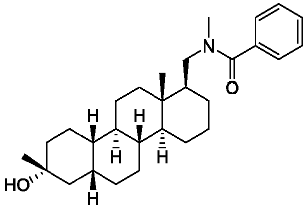 | C |
| **I-7** | **I-7** | 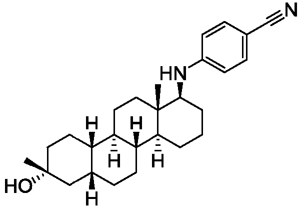 | B |
| **I-8** | **I-8** | 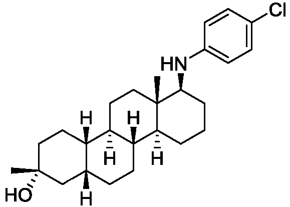 | B |
| **I-9** | **I-9** | 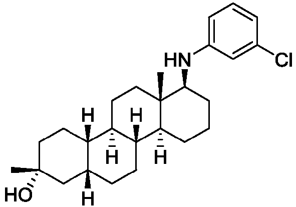 | B |
| **I-10** | **I-10** | 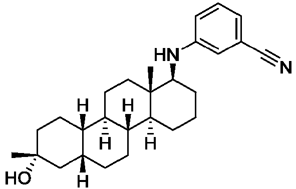 | B |
| **I-11** | **I-11** | 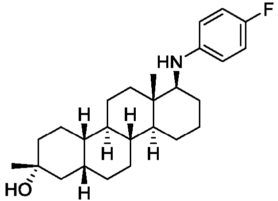 | C |
| **I-12** | **I-12** | 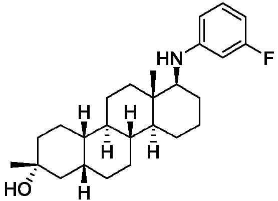 | B |
| **I-13** | **I-E4** | 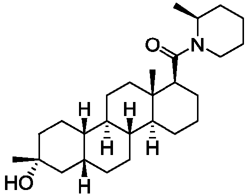 | C |
| **I-14** | **I-14** | 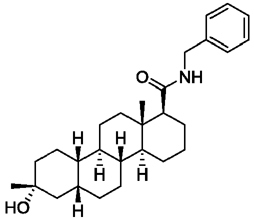 | B |
| **I-15** | **I-15** | 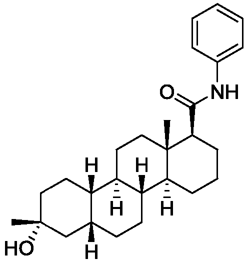 | B |
| **I-16** | **I-16** | 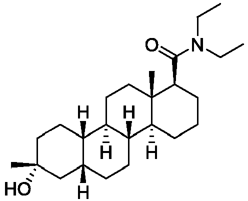 | D |
| **I-17** | **I-17** | 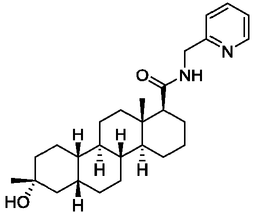 | B |
| **I-18** | **I-D7** | 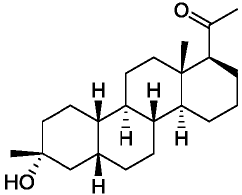 | A |
| **I-19** | **I-D9** | 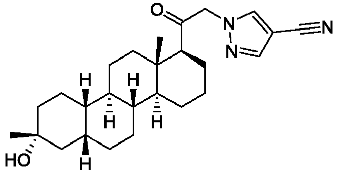 | A |
| **I-20** | **I-20** | 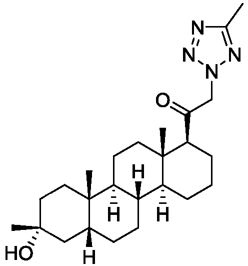 | C |
| **I-21** | **I-G14** | 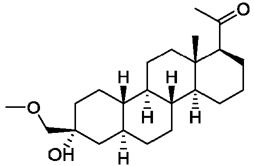 | B |
| **I-22** | **I-G16** | 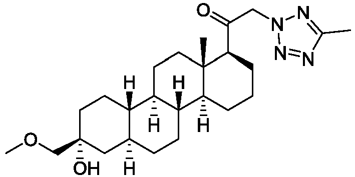 | A |
| **I-23** | **I-23** | 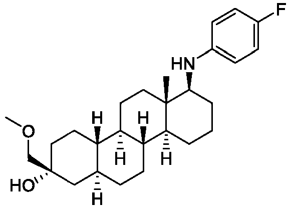 | B |
| **I-24** | **I-M3** | 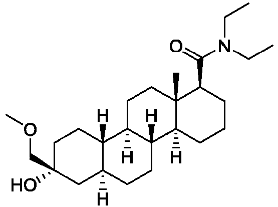 | C |
| **I-25** | **I-25** | 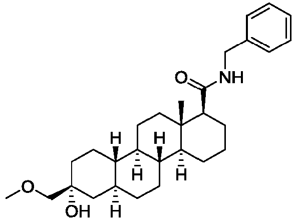 | B |
| **I-26** | **I-26** | 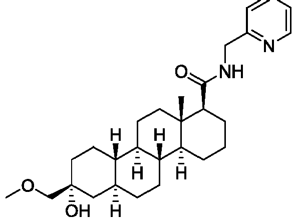 | C |
| **I-27** | **I-27** | 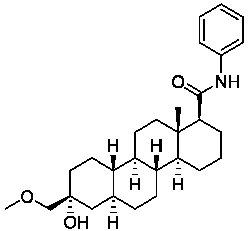 | B |
| **I-28** | **I-H5** | 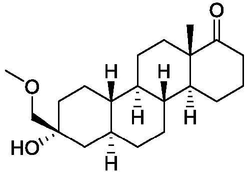 | D |
| **I-34** | **I-K2** | 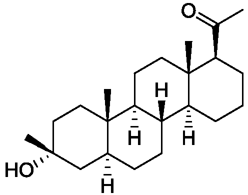 | B |
| **I-35** | **I-L6** | 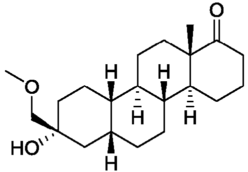 | D |
| **I-37** | **I-L8** | 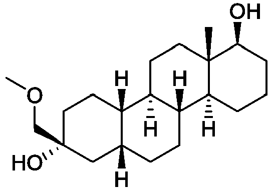 | D |
| **I-38** | **I-L9** | 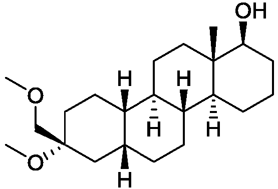 | D |
| **I-39** | **I-39** | 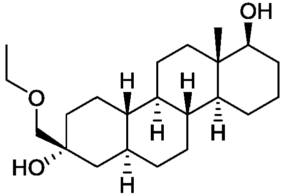 | D |
| **I-40** | **I-40** | 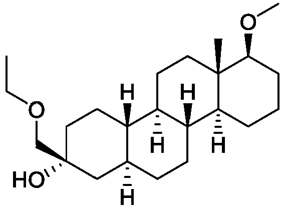 | C |
| **I-49** | **I-N11** | 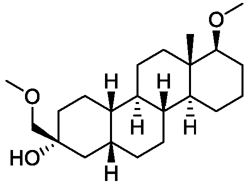 | B |
| **I-50** | **I-50** | 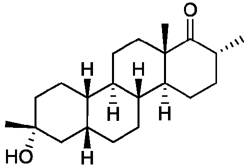 | D |
| **I-53** | **I-53** | 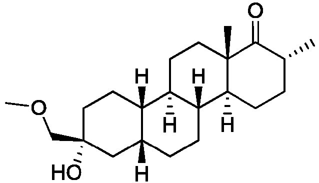 | D |
| **I-57** | **I-B11** | 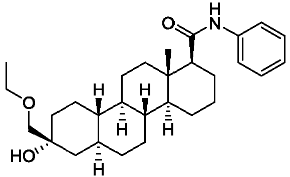 | B |
| **I-58** | **I-P9** | 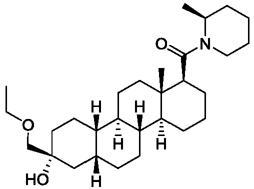 | C |
| **I-59** | **I-L14** | 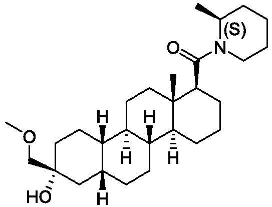 | C |
| **I-60** | **I-L17** | 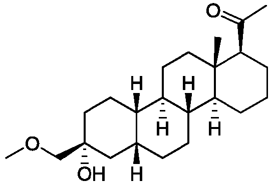 | B |
| **I-61** | **I-L19** | 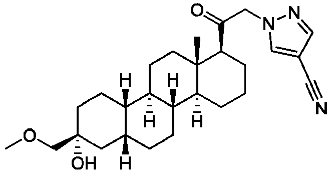 | A |
| **I-62** | **I-Q8a** | 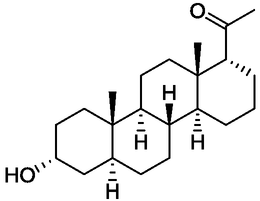 | |
| **I-63** | **I-Q8** | 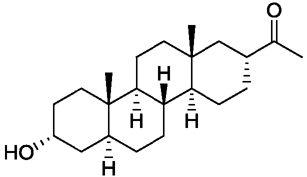 | |
| **I-65** | **I-R14** | 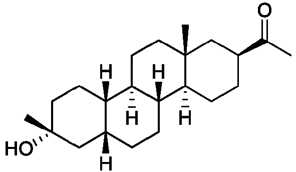 | C |
| **I-67** | **I-R17** | 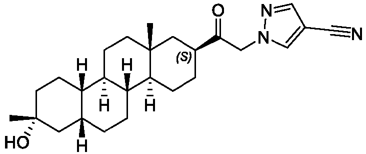 | D |
| **I-69** | **I-R21** | 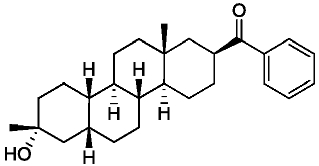 | C |
| **I-70** | **I-S3** | 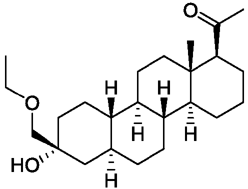 | C |
| **I-72** | **I-R24** | 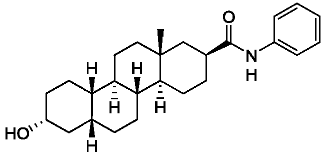 | D |
| **I-73** | **I-R26** | 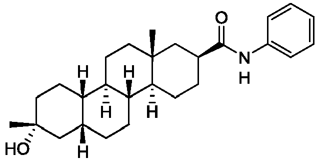 | |
| **I-74** | **I-T6a** | 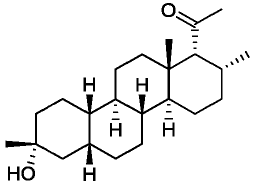 | C |
| **I-75** | **I-U11** | 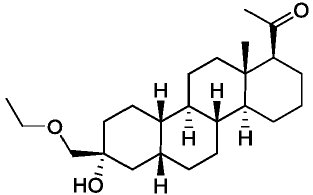 | C |
| **I-10a** | **I-10a** | 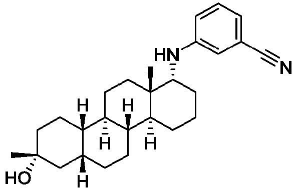 | C |
| **I-11a** | **I-11a** | 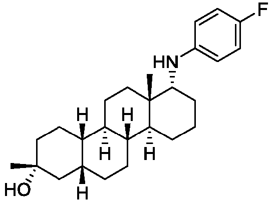 | D |
| **I-18a** | **I-D7a** | 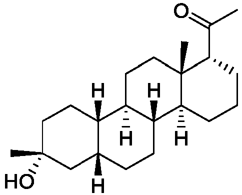 | C |
| **I-20a** | **I-20a** | 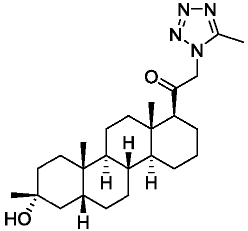 | C |
| **I-22a** | **I-G16a** | 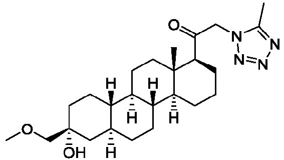 | B |
| **I-23a** | **I-23a** | 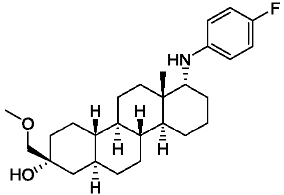 | D |
| **I-49a** | **I-N11a** | 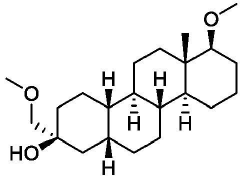 | D |
| **I-67a** | **I-R17a** | 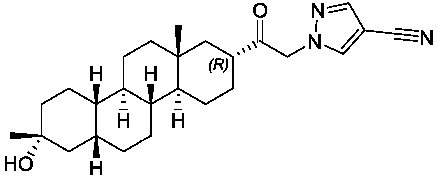 | D |
| **I-70a** | **I-S3a** | 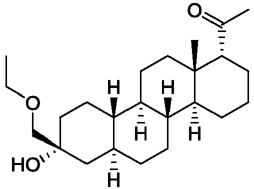 | |
| **I-72a** | **I-R24a** | 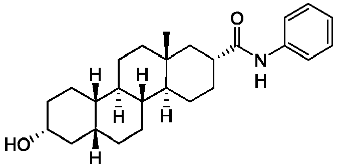 | |
| **I-1a** | **I-A3** | 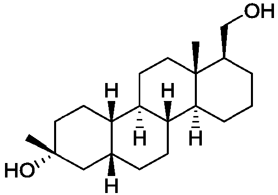 | C |

**Table I-5**

| **Example** | **Compound ID** | **STRUCTURE** | **TBPS IC₅₀ (**µ**M)** |
|---|---|---|---|
| **I-101** | **I-AA6** | 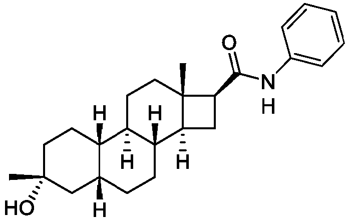 | D |
| **I-101a** | **I-AA6a** | 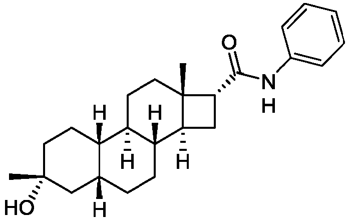 | D |
| **I-102** | **I-AA8** | 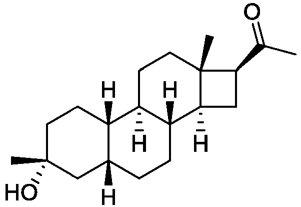 | C |
| **I-102a** | **I-AA8a** | 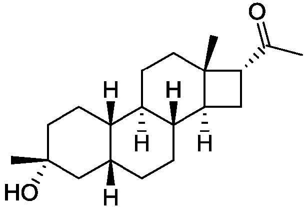 | |
| **I-103** | **I-103** | 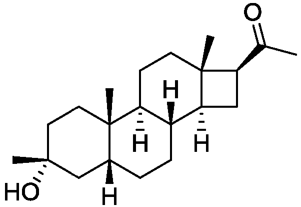 | C |
| **I-103a** | **I-103a** | 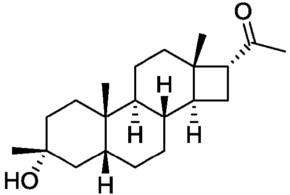 | D |
| **I-104** | **I-AA10** | 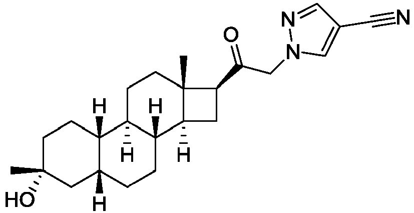 | C |
| **I-105** | **I-105** | 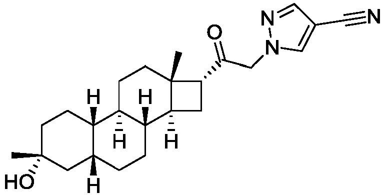 | D |
| **I-106** | **I-106** | 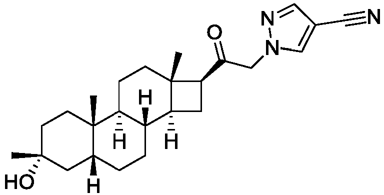 | C |
| **I-107** | **I-107** | 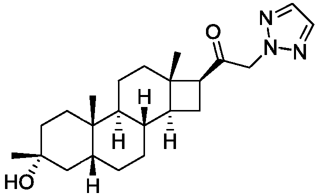 | D |
| **I-107a** | **I-107a** | 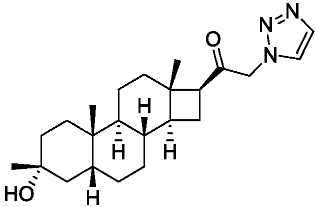 | D |
| **I-108** | **I-AB1** | 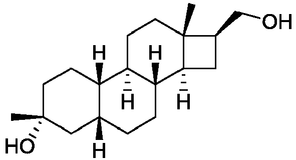 | D |
| **I-108a** | **I-AB1a** | 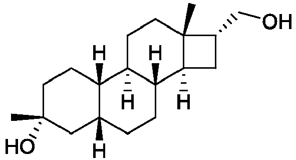 | D |
| **I-113** | **I-AC8** | 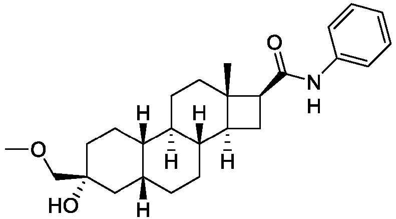 | D |
| **I-113a** | **I-AC8a** | 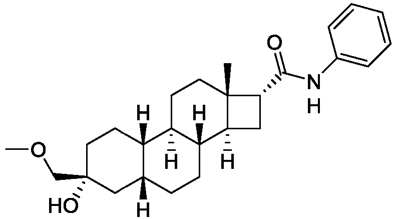 | D |
| **I-114** | **I-AC9** | 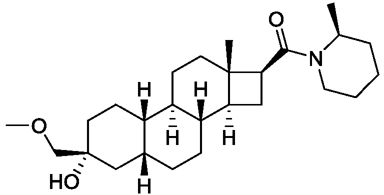 | D |
| **I-114a** | **I-AC9a** | 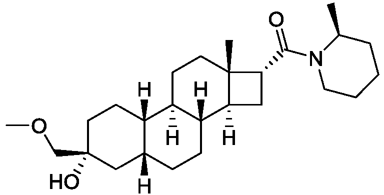 | |
| **I-115** | **I-AD8** | 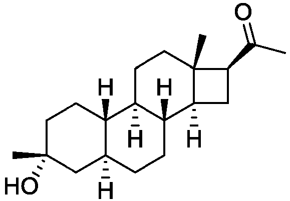 | D |
| **I-115a** | **I-AD8a** | 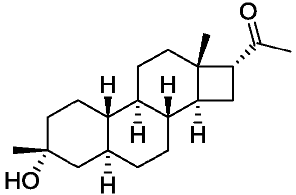 | D |
| **I-116** | **I-AD10** | 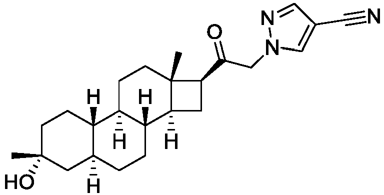 | D |
| **I-116a** | **I-AD10a** | 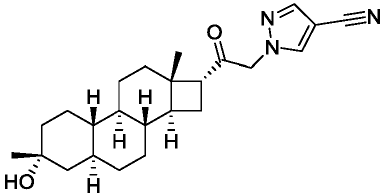 | |
| **I-117** | **I-117** | 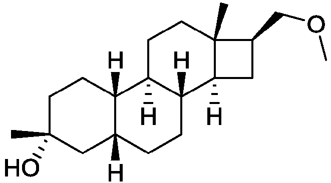 | C |
| **I-118** | **I-118** | 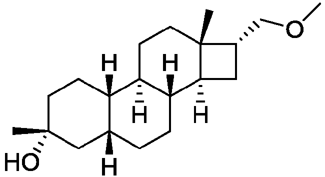 | D |
| **I-119** | **I-AC11** | 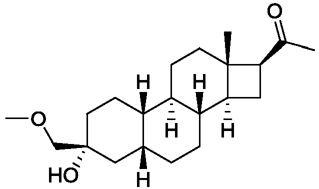 | C |
| **I-120** | **I-AC12** | 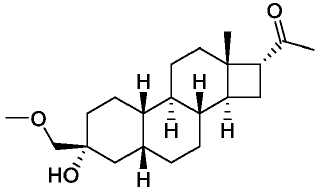 | |
| **I-121** | **I-AC14** | 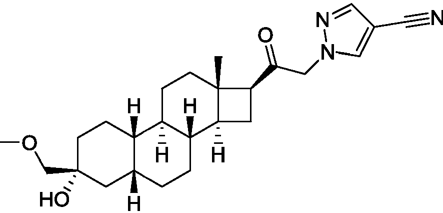 | C |
| **I-122** | **I-AA11** | 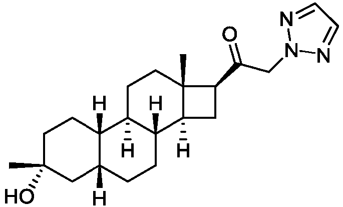 | D |
| **I-123** | **I-AA12** | 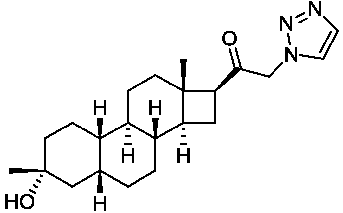 | D |

**Table I-6**

| **Example** | **Compound ID** | **STRUCTURE** | **TBPS IC₅₀ (µM)** |
|---|---|---|---|
| **I-201** | **I-BA3** | 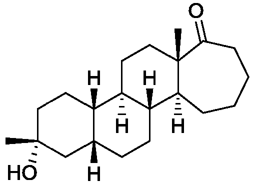 | C |
| **I-202** | **I-BA5a** | 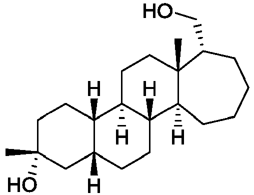 | D |
| **I-202a** | **I-BA5b** | 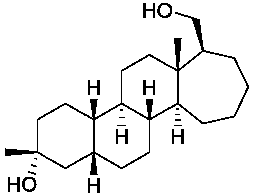 | C |
| **I-203** | **I-BA6a** | 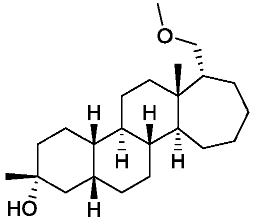 | D |
| **I-203a** | **I-BA6** | 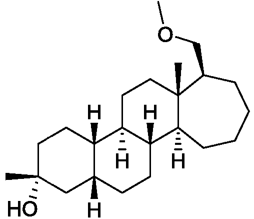 | C |
| **I-206** | **I-BA9a** | 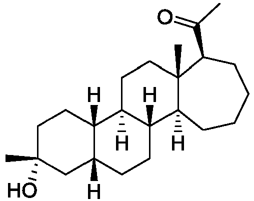 | B |
| **I-205** | **I-BA11** | 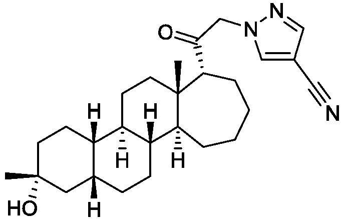 | B |
| **I-207** | **I-BA11a** | 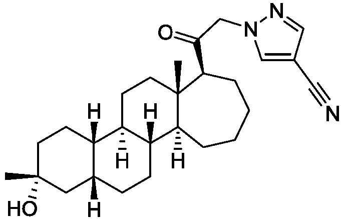 | B |
| **I-208** | **I-BA12** | 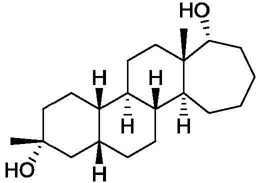 | D |
| **I-209** | **I-BA16a** | 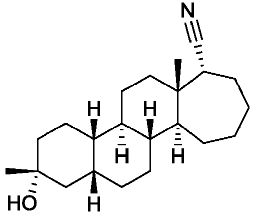 | D |
| **I-209** | **I-BA16** | 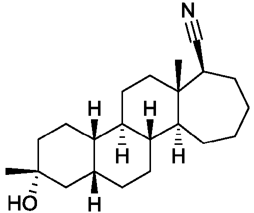 | B |
| **I-210** | **I-BA17** | 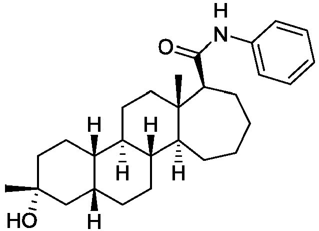 | B |
| **I-210a** | **I-BA17a** | 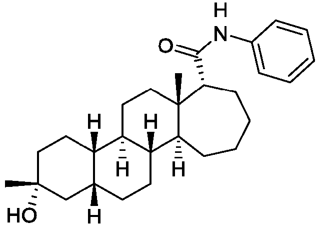 | C |
| **I-211** | **I-BB6** | 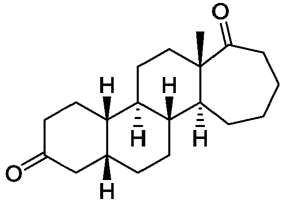 | |
| **I-213** | **I-BC11** | 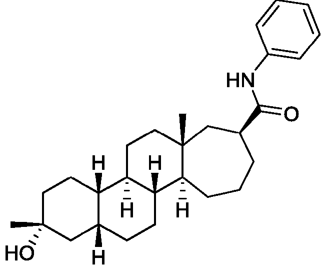 | D |
| **I-213a** | **I-BC11a** | 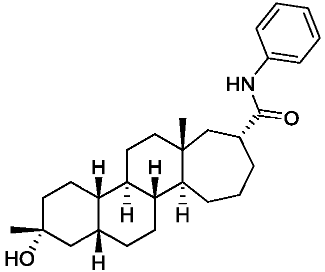 | D |
| **I-214** | **I-BC14a** | 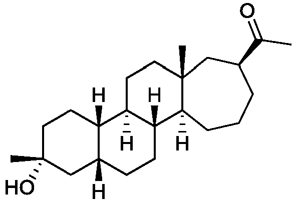 | C |
| **I-214a** | **I-BC14b** | 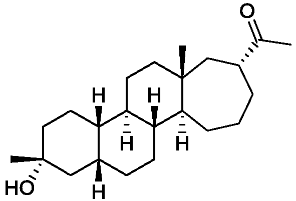 | D |
| **I-215** | **I-BC16** | 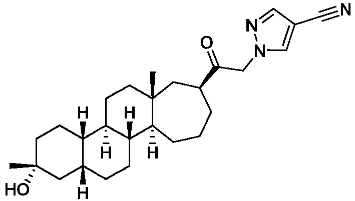 | C |
| **I-215a** | **I-BC16a** | 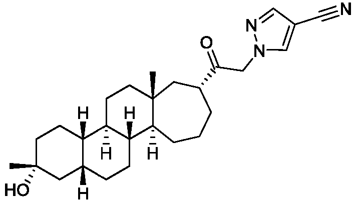 | C |

### Example II-1 & II-2: Synthesis of 1-((1S,3aS,3bS,8S,10aR,10bS,12aS)-8-hydroxy-8,10a,12a-trimethyl-1,2,3,3a,3b,4,6,7,8,9,10,10a,10b,11,12,12a-hexadecahydrocyclohepta[a]cyclopenta[f]naphthalen-1-yl)ethan-1-one (II-C2a) & 1-((1S,3aS,3bS,8R,10aR,10bS,12aS)-8-hydroxy-8,10a,12a-trimethyl-1,2,3,3a,3b,4,6,7,8,9,10,10a,10b,11,12,12a-hexadecahydrocyclohepta[a]cyclopenta[f]naphthalen-1-yl)ethan-1-one (II-C2b)

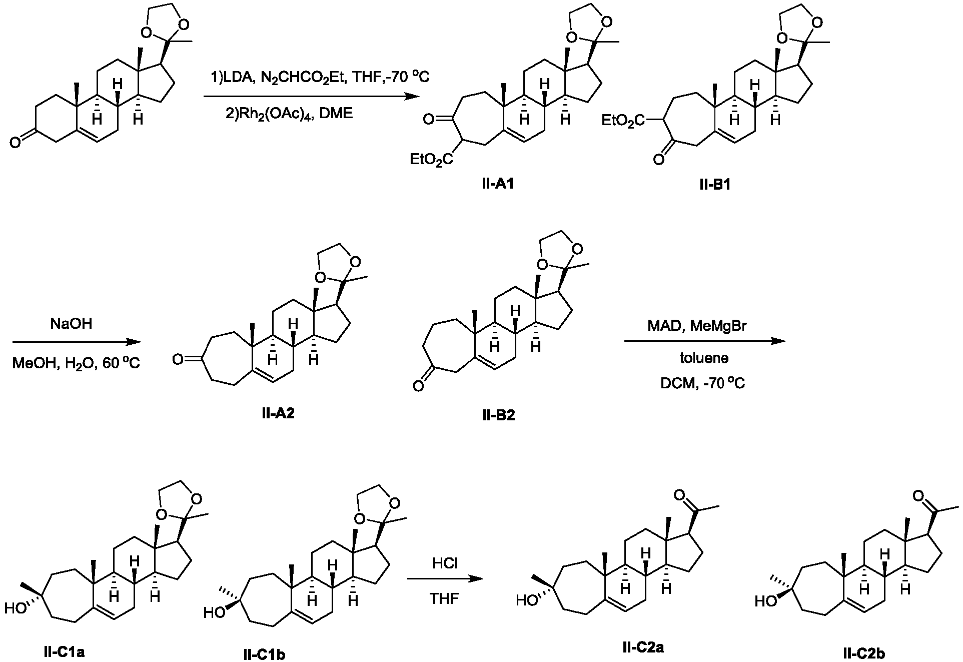

### Synthesis of II-A1 & II-B1

A cold (-70 °C) LDA solution (139 mL, 1.0 M, 139 mmol, fresh prepared) was added to a stirred solution of Pregn-5-ene-3,20-dione, cyclic 20-(1,2-ethanediyl acetal) (CAS # 1427208-28-3) (10 g, 27.8 mmol) and ethyl diazoacetate (15.8 g, 139 mmol) in THF (160 mL) at -70°C. The mixture was stirred at -70°C for 2h. Then acetic acid (8.34 g, 139 mmol) in THF (40 mL) was added, the mixture was then warmed to 20°C and stirred for 16 hrs. Water (300 mL) and PE (200 mL) was added, the organic phase was separated and the aqueous phase was extracted with EtOAc (150 mL). The combined organic layers were washed with saturated brine (200 mL), dried over anhydrous Na₂SO₄, filtered and concentrated to give the product (13 g) as an oil, which was used directly in next step.

To a solution of the product (12 g, 25.3 mmol) in DME (100 mL) was added Rh₂(OAc)₄ (335 mg, 0.76 mmol). The reaction mixture was stirred at 25 °C for 16 hrs. The reaction mixture was concentrated. The residue was purified by silica gel chromatography (0-20% of EtOAc in PE) to give the mixture **II-A1 & II-B1** (6.8 g) as a solid.

**¹H NMR** (400 MHz, CDCl₃) δ_{H} 12.8-12.6 (m, 0.2H), 5.69-5.44 (m, 1H), 4.29-4.10 (m, 2H), 4.04-3.82 (m, 4H), 3.44-3.17 (m, 0.8H), 2.99-2.65 (m, 1H), 2.48-1.96 (m, 4H), 1.85-1.61 (m, 6H), 1.55-1.40 (m, 3H), 1.34-1.14 (m, 12H), 1.02-0.96 (m, 3H), 0.79 (s, 3H).

### Synthesis of II-A2 & II-B2

To a mixture of **II-A1 & II-B1** (6.8 g, 15.2 mmol) in MeOH (150 mL) were added H₂O (50 mL) and NaOH (6.08 g, 152 mmol). The reaction mixture was stirred at 60 °C for 16 hrs. The reaction mixture was concentrated. Then H₂O (150 mL) was added. The mixture was extracted with EtOAc (3 x 150 mL). The combined organic phase was washed with saturated brine (150 mL), dried over anhydrous Na₂SO₄, filtered and concentrated. The residue was purified by silica gel chromatography (0-2% of EtOAc in DCM) to give the product **II-A2** (1.5 g, 26% yield) as a solid and the product **II-B2** (900 mg, 16% yield) as a solid, and the mixture products **II-A2 & II-B2** (1.5 g) as a solid.

### II-A2:

**¹H NMR** (400 MHz, CDCl₃) δ_{H} 5.57 (d, *J =* 4.4 Hz, 1H), 4.04-3.82 (m, 4H), 2.67-2.56 (m, 1H), 2.49-2.25 (m, 4H), 2.20-1.97 (m, 3H), 1.84-1.59 (m, 7H), 1.56-1.37 (m, 3H), 1.30 (s, 3H), 1.26-1.03 (m, 4H), 1.00 (s, 3H), 0.79 (s, 3H).

### II-B2:

**¹H NMR** (400 MHz, CDCl₃) δ_{H} 5.56 (d, *J =* 4.4 Hz, 1H), 4.05-3.83 (m, 4H), 3.25 (d, *J =* 14.0 Hz, 1H), 2.82 (d, *J =* 14.4 Hz, 1H), 2.65-2.53 (m, 1H), 2.24-2.01 (m, 3H), 1.89-1.60 (m, 7H), 1.57-1.39 (m, 4H), 1.30 (s, 3H), 1.28-1.02 (m, 5H), 0.99 (s, 3H), 0.79 (s, 3H).

### Synthesis of II-C1a & II-C1b

To a solution of BHT (10.6 g, 48.3 mmol) in toluene (100 mL) under nitrogen at 0°C was added trimethylaluminum (2 M in toluene, 12.0 mL, 24.1 mmol) dropwise. The mixture was stirred at 25°C for 1 h and used directly as a solution of MAD without further purification. To the MAD solution was added a solution **of II-A2** (3.0 g, 8.0 mmol) in anhydrous DCM (20 mL) dropwise at -70°C. After stirring at -70°C for 1h under N₂, MeMgBr (8.03 mL, 24.1 mmol, 3M in ethyl ether) was added dropwise at -70°C. The resulting solution was stirred at - 70°C for another 2h. The reaction mixture was poured into saturated aqueous citric acid (100 mL) below 10°C and extracted with EtOAc (2 x 30 mL). The combined organic layer was dried over anhydrous Na₂SO₄, filtered and concentrated in vacuum to give the product. The residue was triturated with PE (20 mL) at 20°C to give **II-C1a & II-C1b** (3.3 g) as a solid, which was used without further purification and used directly for the next step.

### Synthesis of II-C2a & II-C2b

To a solution of **II-C1a & II-C2b** (3.3 g, 8.5 mmol) in THF (50 mL) was added 12M HCl (3 mL, 36.0 mmol). The reaction mixture was stirred at 20°C for 16 hours. The reaction mixture was diluted with H₂O (50 mL) and adjust to pH = 9 with solid Na₂CO₃ (20 g). The product was extracted with EtOAc (3 x 30 mL). The combined organic layers were dried over anhydrous Na₂SO₄, filtered and concentrated to give the product. The product was purified by flash column (20~100% of EtOAc in PE) to afford **II-C1a** (600 mg) as a solid and **II-C2b** (1.0 g, 34%) as a solid.

### II-C2b:

**¹H NMR** (400 MHz, CDCl₃) δ_{H} 5.40 (d, *J* = 3.6 Hz, 1H), 2.53 (t, *J =* 8.8 Hz, 1H), 2.28-2.17 (m, 2H), 2.12 (s, 3H), 2.10-2.01 (m, 2H), 1.92-1.50 (m, 8H), 1.49-1.32 (m, 6H), 1.30-1.19 (m, 7H), 0.91 (s, 3H), 0.63 (s, 3H); ELSD purity 99%, **MS ESI** calcd. for C₂₃H₃₅O [M-H₂O+H]⁺ 327, found 327.

### II-C1a:

**¹H NMR** (400 MHz, CDCl₃) δ_{H} 5.45 (d, *J* = 4.0 Hz, 1H), 2.54 (t, *J =* 8.8 Hz, 1H), 2.22-1.85 (m, 9H), 1.78-1.60 (m, 4H), 1.55-1.35 (m, 7H), 1.32-1.16 (m, 8H), 0.88 (s, 3H), 0.63 (s, 3H); ELSD, purity 99%, **MS ESI** calcd. for C₂₃H₃₅O [M-H₂O+H]⁺ 327, found 327.

### Example II-3: Synthesis of 1-((1S,3aS,3bR,5aS,8S,10aS,10bS,12aS)-8-hydroxy-8,10a,12a-trimethyloctadecahydrocyclohepta[a]cyclopenta[f]naphthalen-1-yl)ethan-1-one (II-C3)

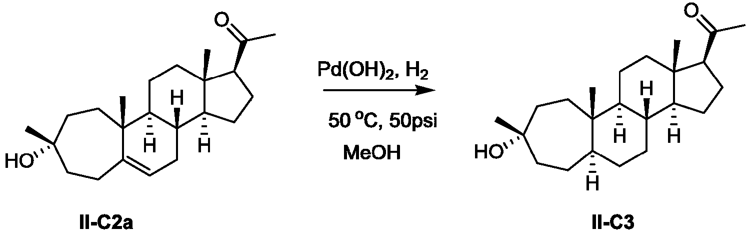

To a mixture of **II-C2a** (300 mg, 0.9 mmol) in MeOH (30 mL) was added Pd(OH)₂/C (dry, 30 mg) under Ar. 344738 Pa (50 psi) of hydrogen at 50°C for 2 hrs was applied to the resulting solution. The reaction mixture was filtered through a pad of Celite and washed with THF (2 x 50 mL). The filtrate was concentrated under reduced pressure. The product was purified by flash column (15~20% of EtOAc in PE) to give **II-C3** (113 mg, 38%) as a solid.

**¹H NMR** (400 MHz, CDCl₃) δ_{H} 2.52 (t, *J =* 8.8 Hz, 1H), 2.20-2.10 (m, 4H), 2.05-1.94 (m, 1H), 1.90-1.80 (m, 1H), 1.74-1.49(m, 7H), 1.48-1.00 (m, 16H), 0.95-0.80 (m, 1H), 0.78-0.20 (m, 4H), 0.60 (s, 3H); ELSD purity, 99%; **MS ESI** calcd. for C₂₃H₃₇O [M-H₂O+H]⁺ 329, found 329.

### Example II-4: Synthesis of 1-(2-((1S,3aS,3bR,5aS,8S,10aS,10bS,12aS)-8-hydroxy-8,10a,12a-trimethyloctadecahydrocyclohepta[a]cyclopenta[f]naphthalen-1-yl)-2-oxoethyl)-1H-pyrazole-4-carbonitrile (II-C5)

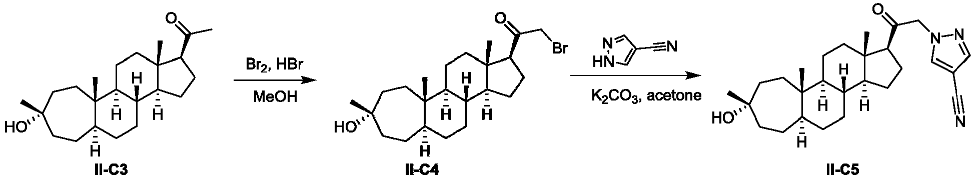

### Synthesis of II-C4

To a solution of **II-C3** (90 mg, 0.3 mmol) in MeOH (5 mL) was added HBr (10.3 mg, 0.05 mmol, 40%) and Br₂ (41.5 mg, 0.3 mmol) at 15°C. The reaction mixture was stirred for 1 hour at 15°C. The reaction mixture was added into saturated NaHCO₃ (30 mL), then extracted with EtOAc (3 x 20 mL). The combined organic layer was washed with saturated brine (30 mL), dried over anhydrous Na₂SO₄, filtered and concentrated to give **II-C4** (110 mg) as an oil, which was used directly for the next step without further purification.

**¹H NMR** (400 MHz, CDCl₃) δ_{H} 3.96-3.85 (m, 2H), 2.81 (t, *J =* 8.8 Hz, 1H), 2.25-2.13 (m, 1H), 1.91-1.62 (m, 9H), 1.34-1.15 (m, 15H), 1.00-0.86 (m, 2H), 0.79-0.74 (m, 4H), 0.62 (s, 3H).

### Synthesis of II-C5

To a solution of **II-C4** (110 mg, 0.3 mmol) and 1H-pyrazole-4-carbonitrile (48.1 mg, 0.5 mmol) in acetone (5 mL) was added K₂CO₃ (71.3 mg, 0.52 mmol). The reaction mixture was stirred at 20°C for 16 hrs. The reaction mixture was quenched with saturated NH₄Cl (30 mL) and extracted with EtOAc (3 x 20 mL). The combined organic phase was washed with saturated brine (20 mL), dried over anhydrous Na₂SO₄, filtered and concentrated to give the product (100 mg) as an oil. The product was purified by flash column (0-30% of EtOAc in PE) to give **II-C5** (52.8 mg, 59%) as a solid.

**¹H NMR** (400 MHz, CDCl₃) δ_{H} 7.85 (s, 1H), 7.81 (s, 1H), 4.95 (dd, *J =* 18.0, 48.8 Hz, 2H), 2.59 (t, *J =* 8.4 Hz, 1H), 2.26-2.15 (m, 1H), 2.08-2.02 (m, 1H), 1.91-1.84 (m, 1H), 1.82-1.58 (m, 7H), 1.53-1.34 (m, 4H), 1.33-1.07 (m, 12H), 1.00-0.88 (m, 1H), 0.87-0.77 (m, 1H), 0.76 (s, 3H), 0.65 (s, 3H); ELSD, purity 99%; **MS ESI** calcd. for C₂₇H₃₈ON₃ [M+H-H₂O]⁺ 420, found 420.

### Example II-5: Synthesis of 1-((1S,3aS,3bS,7R,10aR,10bS,12aS)-7-hydroxy-10a,12a-dimethyl-1,2,3,3a,3b,4,6,7,8,9,10,10a,10b,11,12,12a-hexadecahydrocyclohepta[a]cyclopenta[f]naphthalen-1-yl)ethan-1-one (II-B4)

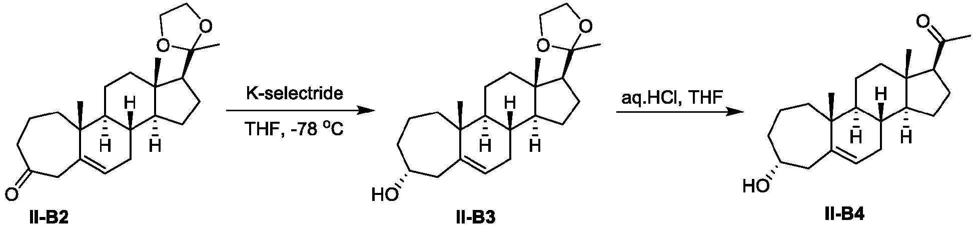

### Synthesis of II-B3

To a solution of **II-B2** (700 mg, 1.9 mmol) in THF (20 mL) at -70°C was added K-selectride (5.6 mL, 5.6 mmol, 1M in THF). The reaction mixture was stirred at -70°C for 2 hours. The reaction mixture was quenched with saturated NH₄Cl (50 mL) and extracted with EtOAc (2 x 30 mL). The combined organic phase was washed with saturated brine (30 mL), dried over anhydrous Na₂SO₄, filtered and concentrated. The residue was purified by silica gel chromatography (0-30% of EtOAc in PE) to give the product **II-B3** (500 mg, 71%) as a solid.

**¹H NMR** (400 MHz, CDCl₃) δ_{H} 5.51 (d, *J* = 3.6 Hz, 1H), 4.02-3.86 (m, 5H), 2.45 (d, *J* = 13.6 Hz, 1H), 2.31-2.16 (m, 2H), 2.12-1.93 (m, 3H), 1.85-1.61 (m, 7H), 1.38-1.27 (m, 7H), 1.18-1.00 (m, 6H), 0.94 (s, 3H), 0.79 (s, 3H).

### Synthesis of II-B4

To a solution of **II-B3** (500 mg, 1.3 mmol) in THF (20 mL) was added HCl (3.30 mL, 13.2 mmol, 4M in H₂O). The reaction mixture was stirred at 20°C for 16 hrs. The reaction mixture was quenched with saturated NaHCO₃ (50 mL) and extracted with EtOAc (3 x 30 mL). The combined organic phase was washed with saturated brine (30 mL), dried over anhydrous Na₂SO₄, filtered and concentrated to give the product **II-B4** (450 mg) as a solid.

**¹H NMR** (400 MHz, CDCl₃) δ_{H} 5.51 (d, *J* = 4.0 Hz, 1H), 3.93 (s, 1H), 2.54 (t, *J=* 9.2 Hz, 1H), 2.45 (d, *J =* 13.6 Hz, 1H), 2.27-2.17 (m, 3H), 2.13 (s, 3H), 2.10-2.05 (m, 1H), 2.02-1.93 (m, 1H), 1.91-1.82 (m, 1H), 1.74-1.60 (m, 5H), 1.51-1.29 (m, 5H), 1.23-0.98 (m, 5H), 0.93 (s, 3H), 0.64 (s, 3H).

### Example II-6: Synthesis of 1-((1S,3aS,3bR,5aS,7R,10aS,10bS,12aS)-7-hydroxy-10a,12a-dimethyloctadecahydrocyclohepta[a]cyclopenta[f]naphthalen-1-yl)ethan-1-one (II-B5)

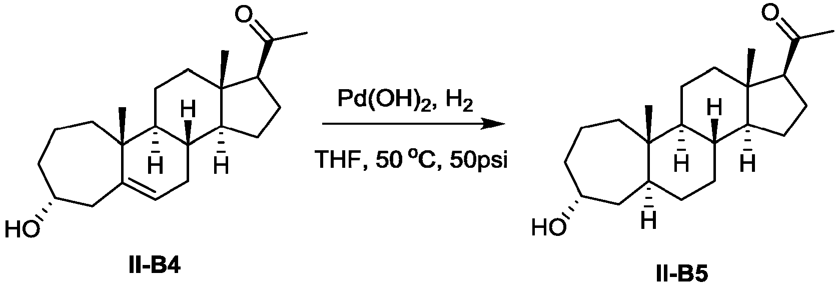

To a solution of **II-B4** (200 mg, 0.6 mmol) in THF (10 mL) was added dry Pd(OH)₂/C (100 mg) under N₂. The reaction mixture was degassed and refilled with H₂. 344738 Pa (50 psi) of hydrogen at 50°C for 16 hrs was applied to the resulting solution. The reaction mixture was filtered to remove Pd(OH)₂/C and eluted with EtOAc (10 mL). The filtrate was concentrated. The residue was purified by silica gel chromatography (0-30% of EtOAc in PE) to give the product **II-B5** (66.8 mg, 33%) as a solid.

**¹H NMR** (400 MHz, CDCl₃) δ_{H} 4.09-3.98 (m, 1H), 2.52 (t, *J =* 8.8 Hz, 1H), 2.21-2.08 (m, 4H), 2.07-1.93 (m, 3H), 1.72-1.60 (m, 6H), 1.53-1.09 (m, 13H), 1.01-0.88 (m, 1H), 0.83-0.72 (m, 4H), 0.59 (s, 3H); ELSD purity, 99%; **MS ESI** calcd. for C₂₂H₃₅O [M+H-H₂O]⁺ 315, found 315.

### Example II-7: Synthesis of 1-((15,3aS,3bR,5aS,8R,10aS,10bS,12aS)-8-hydroxy-8,10a,12a-trimethyloctadecahydrocyclohepta[a]cyclopenta[f]naphthalen-1-yl)ethan-1-one (II-C6)

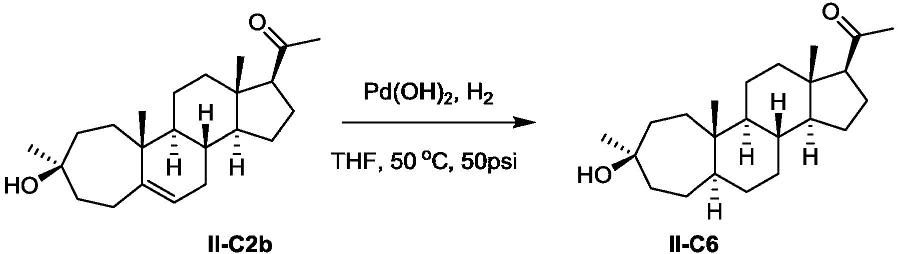

To a mixture of **II-C2b** (350 mg, 1.0 mmol) in THF (10 mL) was added Pd(OH)₂/C (dry, 100 mg) under Ar. The suspension was degassed under vacuum and purged with H₂ for three times. 344738 Pa (50 psi) of hydrogen at 50°C for 12 hrs was applied to the resulting solution. The reaction mixture was filtered through a pad of Celite and washed with THF (2 x 50 mL). The filtrate was concentrated under reduced pressure to give the product. The product was purified by flash column (15~20% of EtOAc in PE) to give **II-C6** (260.9 mg, 75%) as a solid. **¹H NMR** (400 MHz, CDCl₃) δ_{H} 2.51 (t, *J =* 8.8 Hz, 1H), 2.11-2.06 (m, 4H), 2.04-1.96 (m, 1H), 1.81-1.59 (m, 9H), 1.53-1.28 (m, 5H), 1.25-0.84 (m, 11H), 0.82-0.72 (m, 4H), 0.60 (s, 3H); ELSD purity, 99%; **MS ESI** calcd. for C₂₃H₃₇O [M-H₂O+H]⁺329, found 329.

### Example II-8: Synthesis of 1-(2-((1S,3aS,3bR,5aS,8R,10aS,10bS,12aS)-8-hydroxy-8,10a,12a-trimethyloctadecahydrocyclohepta[a]cyclopenta[f]naphthalen-1-yl)-2-oxoethyl)-1H-pyrazole-4-carbonitrile (II-C8)

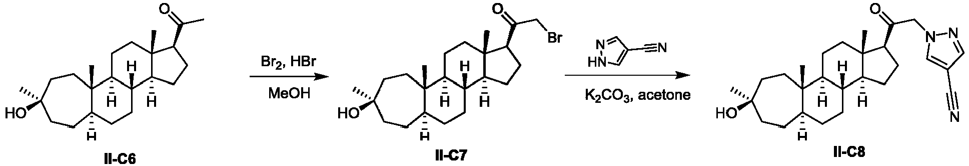

### Synthesis of II-C7

To a solution of **II-C6** (200 mg, 0.56 mmol) in MeOH (5 mL) were added HBr (23.0 mg, 0.1 mmol) and Br₂ (92.3 mg, 0.6 mmol) at 15°C. The reaction mixture was stirred for 2 hours at 15°C. The reaction mixture was added into saturated NaHCO₃ (20 mL), then extracted with EtOAc (3 x 30 mL). The combined organic layer was washed with saturated brine (30 mL), dried over Na₂SO₄, filtered and concentrated to give **II-C7** (245 mg) as an oil. It was used directly for the next step without further purification.

### Synthesis of II-C8

To a solution of **II-C7** (245 mg, 0.6 mmol) and 1H-pyrazole-4-carbonitrile (53.6 mg, 0.6 mmol) in acetone (5 mL) was added K₂CO₃ (158 mg, 1.12 mmol). The reaction mixture was stirred at 20 °C for 16 hrs. The reaction mixture was extracted with EtOAc (3 x 30 mL). The combined organic layer was washed with saturated brine (30 mL), dried over Na₂SO₄, filtered and concentrated to give the product. The product was purified by flash column (0-30% of EtOAc in PE) to give **II-C8** (39.5 mg, 16%) as a solid.

**¹H NMR** (400 MHz, CDCl₃) δ_{H} 7.85 (s, 1H), 7.81 (s, 1H), 4.95 (dd, *J =* 18.0, 44.8 Hz, 2H), 2.58 (t, *J* = 8.4 Hz, 1H), 2.25-2.15 (m, 1H), 2.08-1.99 (m, 1H), 1.82-1.60 (m, 9H), 1.45-1.15 (m, 13H), 1.05-0.79 (m, 7H), 0.66 (s, 3H); ELSD purity, 99%; **MS ESI** calcd. for C₂₇H₄₀N₃O₂ [M+H]⁺ 438, found 438.

### Example II-9 & II-10: Synthesis of 1-((1S,3aS,3bS,7R,10aR,10bS,12aS)-7-hydroxy-7,10a,12a-trimethyl-1,2,3,3a,3b,4,6,7,8,9,10,10a,10b,11,12,12a-hexadecahydrocyclohepta[a]cyclopenta[f]naphthalen-1-yl)ethan-1-one (II-D2a) & 1-((1S,3a8,3bS,7S,10aR,10bS,12aS)-7-hydroxy-7,10a,12a-trimethyl-1,2,3,3a,3b,4,6,7,8,9,10,10a,10b,11,12,12a-hexadecahydrocyclohepta[a]cyclopenta[f]naphthalen-1-yl)ethan-1-one (II-D2b)

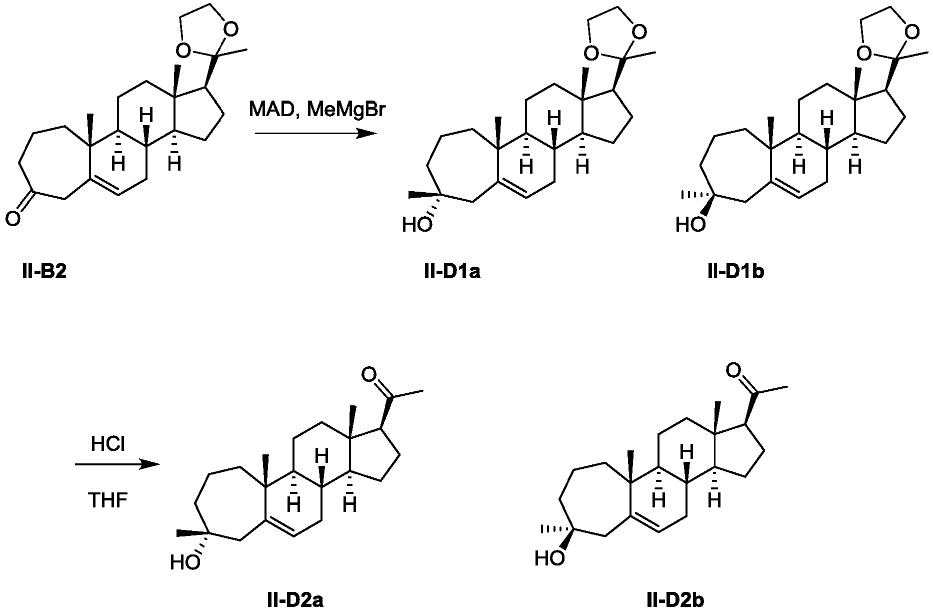

### Synthesis of II-D1a & II-D1b

To a solution of **II-B2** (2.6 g, 7.0 mmol) in anhydrous THF (100 mL) at 0°C under N₂ was added MeMgBr (6.96 mL, 20.9 mmol, 3M in ethyl ether) was added drop wise at 0°C. The resulting solution was stirred at 20°C for another 2h. The reaction mixture was poured into saturated aq.NH₄Cl (100 mL) below 10°C and extracted with EtOAc (2 x 50 mL). The combined organic layer was dried over anhydrous Na₂SO₄, filtered and concentrated in vacuum to give **II-D1a & II-D1b** (2.5 g) as a solid, which was used directly for the next step without further purification.

### Synthesis of II-D2a & II-D2b

To a solution of **II-D1a & II-D1b** (2.5 g, 6.4 mmol) in anhydrous THF (100 mL) was added HCl (10 mL, 6.4 mmol). The resulting solution was stirred at 25°C for 16h. The reaction mixture was diluted with H₂O (100 mL) and adjust to pH = 9 with solid Na₂CO₃ (30 g). The product was extracted with EtOAc (3 x 50 mL). The combined organic layers were dried over anhydrous Na₂SO₄, filtered and concentrated to give the product. The residue was purified by combi flash (0-20% of EtOAc in PE) to give **II-D2a** (1.1 g) and **II-D2b** (520 mg) as solids. **II-D2a** (100 mg) was purified by prep-HPLC (Column: Agela DuraShell 150mm_25mm_5µm; Condition: water (0.04%NH₃H₂O+10mM NH₄HCO₃)-ACN; Begin B: 66; End B: 96; Gradient Time (min): 8.5; 100%B Hold Time (min): 2) to afford **II-D2a** (65 mg) as a solid.

**II-D2a: ¹H NMR** (400 MHz, CDCl₃) δ_{H} 5.53 (d, *J* = 3.6 Hz, 1H), 2.57-2.50 (m, 2H), 2.40 (d, *J= 13.6* Hz, 1H), 2.25-1.93 (m, 8H), 1.76-1.55 (m, 6H), 1.53-1.15 (m, 10H), 1.14-1.00 (m, 2H), 0.93 (s, 3H), 0.64 (s, 3H); ELSD purity, 99%; **MS ESI** calcd. for C₂₃H₃₅O [M+H]⁺ 327, found 327.

**II-D2b: ¹H NMR** (400 MHz, CDCl₃) δ_{H} 5.42 (d, *J* = 3.6 Hz, 1H), 2.53 (t, *J* = 9.2 Hz, 1H), 2.45 (d, *J =* 12.4 Hz, 1H), 2.25-1.95 (m, 8H), 1.85-1.56 (m, 6H), 1.52-1.15 (m, 10H), 1.14-1.00 (m, 2H), 0.93 (s, 3H), 0.64 (s, 3H); ELSD purity, 99%; **MS ESI** calcd. for C₂₃H₃₅O [M+H]⁺ 327, found 327.

### Example II-11: Synthesis of 1-((1S,3aS,3bR,5aS,7S,10aS,10bS,12aS)-7-hydroxy-7,10a,12a-trimethyloctadecahydrocyclohepta[a]cyclopenta[f]naphthalen-1-yl)ethan-1-one (II-D3b)

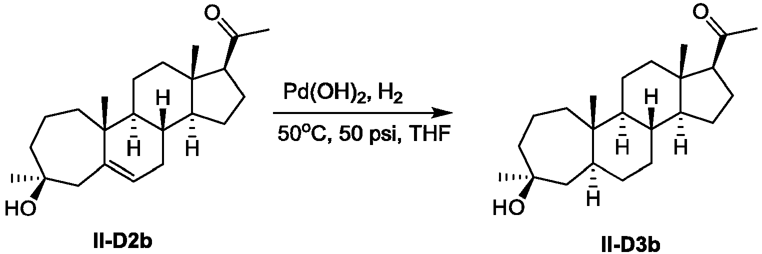

To a solution of **II-D2b** (120 mg, 0.3 mmol) in THF (50 mL) was added Pd(OH)₂/C (dry, 30 mg) under Ar. The suspension was degassed under vacuum and purged with H₂ for three times. The mixture was stirred under H₂ (344738 Pa = 50 psi) at 50°C for 16 hours. The reaction mixture was filtered through a pad of Celite and washed with THF (2 x 50 mL). The filtrate was concentrated under reduced pressure. The product was purified by flash column (15~20% of EtOAc in PE) to give **II-D3b** (25.3 mg, 21%) as a solid.

**¹H NMR** (400 MHz, CDCl₃) δ_{H} 2.50 (t, *J =* 8.8 Hz, 1H), 2.21-1.94 (m, 6H), 1.83-1.60 (m, 6H), 1.55-1.30 (m, 7H), 1.25-0.85 (m, 11H), 0.81 (s, 3H), 0.76-0.65 (m, 1H), 0.59 (s, 3H); ELSD purity, 99%; **MS ESI** calcd. for C₂₃H₃₇O [M+H]⁺ 329, found 329.

### Example II-12: Synthesis of 1-((15,3aS,3bR,5aS,7R,10aS,10bS,12aS)-7-hydroxy-7,10a,12a-trimethyloctadecahydrocyclohepta[a]cyclopenta[f]naphthalen-1-yl)ethan-1-one (II-D3a)

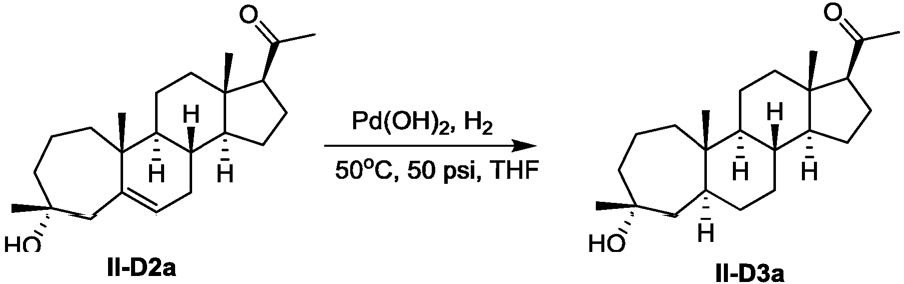

To a mixture of **II-D2a** (150 mg, 0.4 mmol) in THF (30 mL) was added Pd(OH)₂/C (dry, 30 mg) under Ar. The suspension was degassed under vacuum and purged with H₂ for three times. The mixture was stirred under H₂ (344738 Pa = 50 psi) at 50°C for 2 hours. The reaction mixture was filtered through a pad of Celite and washed with THF (2 x 50 mL). The filtrate was concentrated under reduced pressure. The product was purified by flash column (15~20% of EtOAc in PE) to give **II-D3a** (104 mg, 69%) as a solid.

**¹H NMR** (400 MHz, CDCl₃) δ_{H} 2.51 (t, *J* = 8.8 Hz, 1H), 2.18-2.11 (m, 4H), 2.03-1.96 (m, 1H), 1.92-1.84 (m, 1H), 1.79-1.47 (m, 7H), 1.45-1.07 (m, 15H), 1.02-0.88 (m, 2H), 0.84-0.73 (m, 4H), 0.59 (s, 3H); ELSD purity, 99%; MS ESI calcd. for C₂₃H₃₇O [M+H]⁺ 329, found 329.

### Example II-13: Synthesis of 1-(2-((1S,3aS,3bR,5aS,7R,10aS,10bS,12aS)-7-hydroxy-7,10a,12a-trimethyloctadecahydrocyclohepta[a]cyclopenta[f]naphthalen-1-yl)-2-oxoethyl)-1H-pyrazole-4-carbonitrile (II-D5a)

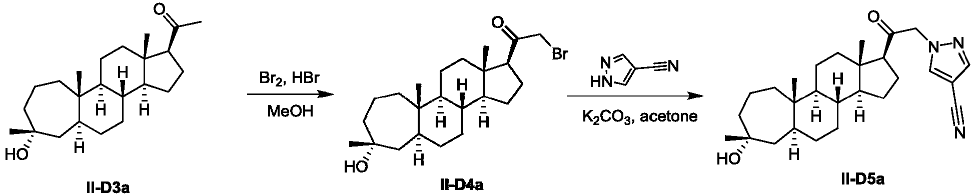

### Synthesis of II-D4a

To a solution of **II-D3a** (150 mg, 0.4 mmol) in MeOH (5 mL) was added HBr (17.3 mg, 0.09 mmol, 40% in water) and Br₂ (69.2 mg, 0.4 mmol) at 25°C. The reaction mixture was stirred for 1 hour at 25°C. The reaction mixture was added into saturated NaHCO₃ (50 mL), then extracted with EtOAc (3 x 30 mL). The combined organic layer was washed with saturated brine (50 mL), dried over anhydrous Na₂SO₄, filtered and concentrated to give **II-D4a** (180 mg) as an oil, which was used directly to the next step without further purification.

**¹H NMR** (400 MHz, CDCl₃) δ_{H} 3.96-3.87 (m, 2H), 2.81 (t, *J =* 8.8 Hz, 1H), 2.17 (q, *J=* 11.2 Hz, 1H), 1.95-1.82 (m, 2H), 1.80-1.60 (m, 7H), 1.55-1.10 (m, 16H), 1.05-0.75 (m, 5H), 0.62 (s, 3H).

### Synthesis of II-D5a

To a solution of **II-D4a** (180 mg, 0.4 mmol) in acetone (5 mL) were added K₂CO₃ (116 mg, 0.8 mmol) and 1H-pyrazole-4-carbonitrile (78.7 mg, 0.8 mmol) at 25°C. The reaction mixture was stirred for 12 hours at 25°C. The reaction mixture was added into saturated NH₄Cl (30 mL) and the aqueous layer was extracted with EtOAc (3 x 20 mL). The combined organic layer was washed with saturated brine (50 mL), dried over anhydrous Na₂SO₄, filtered and concentrated to give product. The residue was purified by flash column (10~20% of EtOAc in PE) to afford the product. The product was purified by prep-HPLC to afford **II-D5a** (59.6 mg, 32%) as a solid.

**¹H NMR** (400 MHz, CDCl₃) δ_{H} 7.85 (s, 1H), 7.81 (s, 1H), 4.95 (dd, *J =* 18.0, 49.6 Hz, 2H), 2.59 (t, *J =* 8.8 Hz, 1H), 2.25-2.13 (m, 1H), 2.07-2.01 (m, 1H), 1.92-1.82 (m, 1H), 1.80-1.60 (m, 7H), 1.55-1.12 (m, 15H), 1.05-0.90 (m, 2H), 0.85-0.75 (m, 4H), 0.65 (s, 3H); ELSD purity, 99%; **MS ESI** calcd. for C₂₇H₄₀N₃O₂ [M+H]⁺ 438, found 438.

### Example II-14: Synthesis of 1-(2-((1S,3aS,3bR,5aS,7S,10aS,10bS,12aS)-7-hydroxy-7,10a,12a-trimethyloctadecahydrocyclohepta[a]cyclopenta[f]naphthalen-1-yl)-2-oxoethyl)-1H-pyrazole-4-carbonitrile (II-D5b)

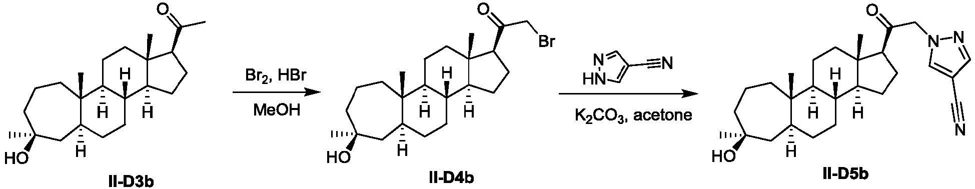

### Synthesis of II-D4b

To a solution of **II-D3b** (150 mg, 0.4 mmol) in MeOH (5 mL) was added HBr (17.3 mg, 0.09 mmol, 40% in water) and Br₂ (69.2 mg, 0.4 mmol) at 25°C. The reaction mixture was stirred for 1 hour at 25°C. The reaction mixture was added into saturated NaHCO₃ (50 mL), then extracted with EtOAc (3 x 30 mL). The combined organic layer was washed with saturated brine (50 mL), dried over anhydrous Na₂SO₄, filtered and concentrated to give **II-D4b** (180 mg) as an oil, which was used directly to the next step without further purification.

**¹H NMR** (400 MHz, CDCl₃) δ_{H} 3.96-3.87 (m, 2H), 2.80 (t, *J =* 8.8 Hz, 1H), 2.25-2.12 (m, 1H), 2.00-1.88 (m, 2H), 1.82-1.60 (m, 7H), 1.53-0.82 (m, 17H), 0.81 (s, 3H), 0.78-0.66 (m, 1H), 0.62 (s, 3H).

### Synthesis of II-D5b

To a solution of **II-D4b** (180 mg, 0.4 mmol) in acetone (5 mL) were added K₂CO₃ (116 mg, 0.8 mmol) and 1H-pyrazole-4-carbonitrile (78.7 mg, 0.8 mmol) at 25°C. The reaction mixture was stirred for 12 hours at 25°C. The reaction mixture was added into saturated NH₄Cl (30 mL), and the aqueous layer was extracted with EtOAc (3 x 20 mL). The combined organic layer was washed with saturated brine (50 mL), dried over anhydrous Na₂SO₄, filtered and concentrated to give product. The residue was purified by flash column (10~20% of EtOAc in PE) to afford the product. The product was purified by prep-HPLC to afford **II-D5b** (86.2 mg, 46%) as a solid.

**¹H NMR** (400 MHz, CDCl₃) δ_{H} 7.85 (s, 1H), 7.81 (s, 1H), 4.95 (dd, *J =* 18.0, 49.6 Hz, 2H), 2.57 (t, *J =* 8.8 Hz, 1H), 2.25-2.15 (m, 1H), 2.07-1.92 (m, 2H), 1.85-1.62 (m, 6H), 1.55-1.20 (m, 12H), 1.19-0.82 (m, 9H), 0.85-0.67 (m, 1H), 0.65 (s, 3H); ELSD purity, 99%; **MS ESI** calcd. for C₂₇H₄₀N₃O₂ [M+H]⁺ 438, found 438.

### Example II-15 & II-16: Synthesis of (1S,3aS,3bR,5aR,10aS,10bR,12aS)-1-acetyl-12a-methylhexadecahydrocyclohepta[a]cyclopenta[f]naphthalen-8(1H)-one (II-E7a) & (1S,3aS,3bR,5aR,10aS,10bR,12aS)-1-acetyl-12a-methylhexadecahydrocyclohepta[a]cyclopenta[f]naphthalen-7(1H)-one (II-E7b)

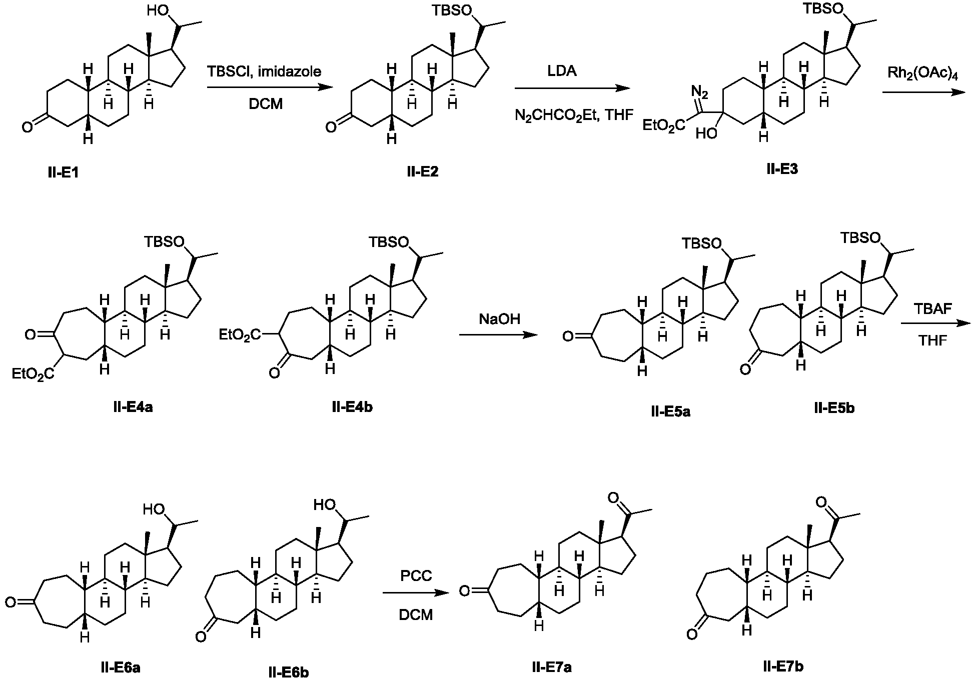

### Synthesis of II-E2

To a solution of **II-E1** (CAS# 1430063-75-4) (10 g, 32.8 mmol) in DCM (40 mL) were added imidazole (4.46 g, 65.6 mmol) and TBSCl (9.88 g, 65.6 mmol) at 20 °C. The reaction mixture was stirred at 20 °C for 1h. The reaction mixture was filtered and the filtrate was washed with saturated NH₄Cl (2 x 60 mL), dried over anhydrous Na₂SO₄, filtered and concentrated to give the product. The product was purified by flash column (0-15% of EtOAc in PE) to give **II-E2** (8.2 g, 82%) as a solid.

**¹H NMR** (400 MHz, CDCl₃) δ_{H} 3.73-3.62 (m, 1H), 2.60 (t, *J =* 14.0 Hz, 1H), 2.29-2.06 (m, 5H), 1.96-1.85 (m, 2H), 1.77-1.66 (m, 2H), 1.61-1.44 (m, 6H), 1.36-1.09 (m, 11H), 0.87 (s, 9H), 0.67 (s, 3H), 0.06-0.03 (m, 6H)

### Synthesis of II-E3

To a solution of DIPA (2.40 g, 23.8 mmol) in THF (15 mL) was added BuLi (9.5 mL, 2.5 M in hexane, 23.8 mmol) at -70°C. The mixture was warmed to 0°C and stirred at 0°C for 1 h. A cold (-70°C). LDA solution (23.8 mmol) was added to a stirred solution of **II-E2** (2 g, 4.8 mmol) and ethyl 2-diazoacetate (2.71 g, 23.8 mmol) in THF (20 mL) at -78°C. The mixture was stirred at -70°C for 1h. Then acetic acid (1.42 g, 23.8 mmol) in THF (20 mL) was added and the mixture was then warm to 20°C. Water (60 mL) was added and the aqueous solution was extracted with EtOAc (3 x 50 mL). The combined organic layers were washed with saturated brine (50 mL), dried over anhydrous Na₂SO₄, and concentrated under reduced pressure to give **II-E3** (2.54 g) as an oil.

### Synthesis of II-E4a & II-E4b

To a solution of **II-E3** (2.54 g) in DME (10 mL) was added Rh₂(OAc)₄ (31.5mg, 0.071 mmol) at 25°C. The reaction mixture was stirred at 25°C for 12 hours. The reaction mixture was extracted with ethyl acetate (3 x 20 mL). The combined organic phase was washed with water (30 mL), saturated brine (30 mL), dried over anhydrous Na₂SO₄, filtered and concentrated under vacuum to give the product. The product was purified by flash column (0-15% of EtOAc in PE) to give **II-E4a & II-E4b** (2.8 g) as a solid.

### Synthesis of II-E5a & II-E5b

To a mixture of a mixture of **II-E4a & II-E4b** (2.8 g, 5.5 mmol) in MeOH/THF/H₂O (20 mL/20 mL/5 mL) was added NaOH (2.21 g, 55.4 mmol). The reaction mixture was stirred at 70°C for 12 hour. The reaction mixture was extracted with ethyl acetate (4 x 50 mL). The combined organic phase was washed with water (50 mL), saturated brine (60 mL), dried over anhydrous Na₂SO₄, filtered and concentrated under vacuum to afford the product. The product was purified by flash column (0-15% of EtOAc in PE) to give **II-E5a & II-E5b** (1.6 g) as a solid.

### Synthesis of II-E6a & II-E6b

To a solution of a mixture of **II-E5a & II-E5b** (1.6 g, 3.7 mmol) in THF (50 mL) was added TBAF.3H₂O (5.74 g, 18.4 mmol) at 15°C. After the solution was stirring at 55°C for 12 hours. The mixture was poured into water (50 mL) and extracted with EtOAc (2 x 30 mL). The organic layer was washed with saturated brine (2 x 50 mL), dried over anhydrous Na₂SO₄, filtered and concentrated in vacuum to give the product. The product was purified by flash column (0-15% of EtOAc in PE) to give **II-E6a & II-E6b** (900 mg, 76%) as a solid.

### Synthesis of II-E7a & II-E7b

To a solution of a mixture of **II-E6a & II-E6b** (900 mg, 2.8 mmol) in DCM (30 mL) were added silica gel (1.8 g) and PCC (1.21 g, 5.64 mmol) at 20°C. The reaction mixture was stirred at 20°C for 12 h. The mixture was filtered and the filter cake was washed with DCM (3 x 20 mL). The filtrate was concentrated. The residue was purified by flash column (0-10% of EtOAc in PE) to give the product. The product was purified by SFC to afford II-E7a (336.5 mg, 37%) and II-E7b (336.5 mg, 37%) as solids.

### II-E7a:

**¹H NMR** (400 MHz, CDCl₃) δ_{H} 2.63-2.43 (m, 4H), 2.38-2.24 (m, 1H), 2.20-2.06 (m, 4H), 2.05-1.85 (m, 4H), 1.79-1.60 (m, 7H), 1.53-1.36 (m, 3H), 1.28-1.02 (m, 6H), 0.63 (s, 3H); ELSD purity, 98.30%; MS ESI calcd. for C₂₁H₃₃O₂[M+H]⁺ 317, found 317.

### II-E7b:

**¹H** NMR (400 MHz, CDCl₃) δ_{H} 3.03 (t, J=12.8 Hz, 1H), 2.54 (t, *J =* 8.8 Hz, 1H), 2.50-2.35 (m, 2H), 2.20-2.00 (m, 7H), 1.96-1.86 (m, 1H), 1.73-1.58 (m, 8H), 1.50-0.96 (m, 9H), 0.63 (s, 3H); ELSD purity, 99%; MS ESI calcd. for C₂₁H₃₃O₂[M+H]⁺ 317, found 317.

### Example II-17a & Example II-17: Synthesis of 1-((1S,3aS,3bR,5aR,7R,10aS,10bR,12aS)-7-hydroxy-7,12a-dimethyloctadecahydrocyclohepta[a]cyclopenta[f]naphthalen-1-yl)ethan-1-one (II-E8a) & 1-((1S,3aS,3bR,5aR,7S,10aS,10bR,12aS)-7-hydroxy-7,12a-dimethyloctadecahydrocyclohepta[a]cyclopenta[f]naphthalen-1-yl)ethan-1-one (II-E8)

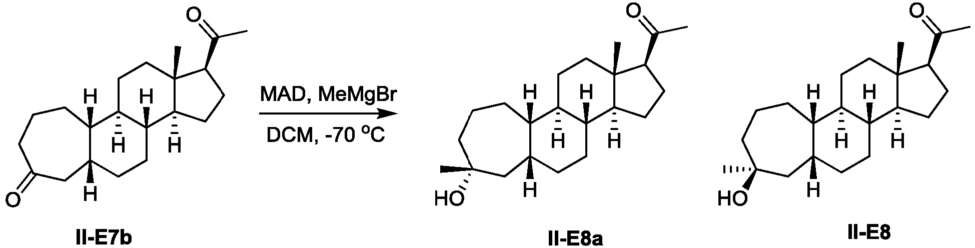

To a solution of 2, 6-di-tert-butyl-4-methylphenol (1.25 g, 5.7 mmol) in toluene (2 mL) was added drop-wise AlMe₃ (1.42 mL, 2.8 mmol, 2 M in toluene) at 0 °C. The mixture was stirred at 25 °C for 30 min. The MAD reaction was used for next step. A solution of II-E7b (300mg, 0.9 mmol) in anhydrous DCM (5 mL) was added drop-wise to MAD (1.36 g, 2.8 mmol) solution at -70°C. After stirring at -70°C for 1h, MeMgBr (0.946 mL, 2.8 mmol, 3M in ethyl ether) was added drop-wise at -70°C and stirring at -70°C for 1h. The reaction mixture was poured into saturated aqueous citric acid (15 mL) below 10°C. The aqueous solution was extracted with EtOAc (3 x 10 mL). The combined organic layers were washed with brine (20 mL), dried over anhydrous Na₂SO₄, and evaporated under reduced pressure to give the product. The product was purified by flash column (10~30% of EtOAc in PE) to give **II-E8a** (160 mg) & **II-E8** (120 mg) as solids. **II-E8** (120 mg) was purified by pre-HPLC (Column: Xbridge 150*30mm*10µm); Condition: water (10mM NH₄HCO₃)-ACN; Begin B: 60%; End 90%) to give **II-E8** (37.5 mg) as a solid.

**II-E8a: ¹H** NMR (400 MHz, CDCl₃) δ_{H} 2.54 (t, *J =* 8.8 Hz, 1H), 2.20-2.08 (m, 4H), 2.04-1.94 (m, 2H), 1.90-1.59 (m, 7H), 1.52-1.34 (m, 6H), 1.32-0.88 (m, 13H), 0.62 (s, 3H); LC-ELSD purity 95.68%, MS ESI calcd. for C₂₂H₃₅O[M-H₂O+H]⁺ 315.27, found 315.3.

II-E8: **¹H** NMR (400 MHz, CDCl₃) δ_{H} 2.52 (t, *J* = 9.2 Hz, 1H), 2.45-2.35 (m, 1H), 2.22-1.95 (m, 6H), 1.92-1.55 (m, 7H), 1.49-1.28 (m, 6H), 1.27-0.76 (m, 12H), 0.62 (s, 3H); LC-ELSD purity 99%, MS ESI calcd. for C₂₂H₃₅O[M-H₂O+H]⁺ 315.27, found 315.3.

### Example II-18a: Synthesis of 1-(2-((1S,3aS,3bR,5aR,7R,10aS,10bR,12aS)-7-hydroxy-7,12a-dimethyloctadecahydrocyclohepta[a]cyclopenta[f]naphthalen-1-yl)-2-oxoethyl)-1H-pyrazole-4-carbonitrile (II-E10a)

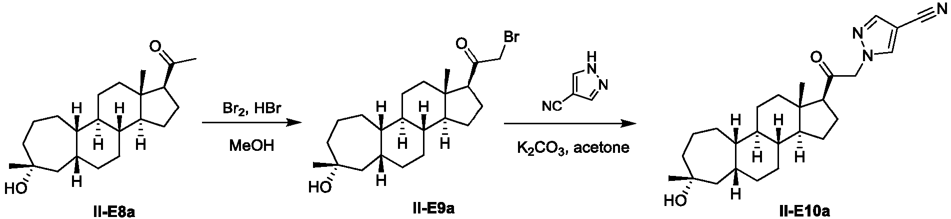

### Synthesis of II-E9a

To a solution of II-E8a (85 mg, 0.3 mmol) in MeOH (2 mL) were added HBr (10.2 mg, 0.05 mmol) and Br₂ (40.8 mg, 0.3 mmol) at 15°C. The reaction mixture was stirred at 15°C for 2 hours. The reaction mixture was added into saturated NaHCO₃ (20 mL), then extracted with EtOAc (3 x 10 mL). The combined organic layer was washed with saturated brine (20 mL), dried over anhydrous Na₂SO₄, filtered and concentrated to give II-E9a (105 mg) as an oil, which was used directly to the next step without further purification.

### Synthesis of II-E10a

To a solution of II-E9a (105 mg, 0.3 mmol) and 1H-pyrazole-4-carbonitrile (47.5 mg, 0.5 mmol) in acetone (1 mL) was added K₂CO₃ (70.4 mg,0.5 mmol). The reaction mixture was stirred at 20 °C for 16 hrs. The reaction mixture was extracted with EtOAc (3 x 30 mL). The combined organic layer was washed with saturated brine (30 mL), dried over Na₂SO₄, filtered and concentrated to give the product. The product was purified by flash column (0-30% of EtOAc in PE) to give II-E10a (30.5 mg, 28%) as a solid.

**¹H** NMR (400 MHz, CDCl₃) δ_{H} 7.85 (s, 1H), 7.81 (s, 1H), 4.96 (dd, *J* = 17.6, 47.6 Hz, 2H), 2.70-2.56 (m, 1H), 2.28-2.12 (m, 1H), 2.09-1.94 (m, 2H), 1.93-1.70 (m, 5H), 1.54-1.36 (m, 7H), 1.32-1.12 (m, 11H), 1.09-0.80 (m, 4H), 0.68 (s, 3H); LC-ELSD purity, 99.30%, MS ESI calcd. for C₂₆H₃₈N₃O₂[M-H₂O+H]⁺ 424.29, found 424.3.

### Example II-18: Synthesis of 1-(2-((1S,3aS,3bR,5aR,7S,10aS,10bR,12aS)-7-hydroxy-7,12a-dimethyloctadecahydrocyclohepta[a]cyclopenta[f]naphthalen-1-yl)-2-oxoethyl)-1H-pyrazole-4-carbonitrile (II-E10)

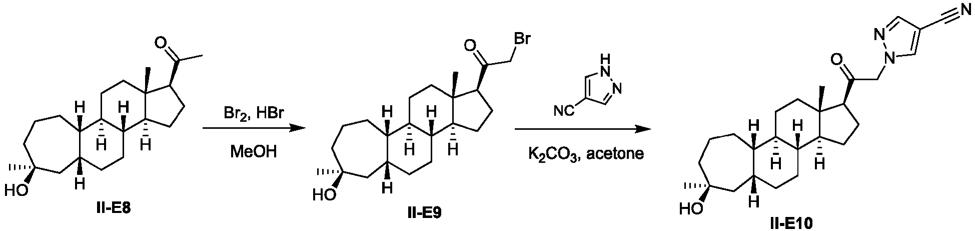

### Synthesis of II-E9

To a solution of II-E8 (55 mg, 0.2 mmol) in MeOH (1 mL) were added HBr (6.60 mg, 0.03 mmol) and Br₂ (26.4 mg, 0.2 mmol) at 15°C. The reaction mixture was stirred at 25°C for 2 hours. The reaction mixture was added into saturated NaHCO₃ (10 mL), then extracted with EtOAc (3 x 10 mL). The combined organic layer was washed with saturated brine (20 mL), dried over anhydrous Na₂SO₄, filtered and concentrated to give II-E9 (68.0 mg) as an oil, which was used directly to the next step without further purification.

### Synthesis of II-E10

To a solution of II-E9 (68.0 mg, 0.2 mmol) and 1H-pyrazole-4-carbonitrile (30.7 mg, 0.3 mmol) in acetone (2 mL) was added K₂CO₃ (45.5mg,0.3 mmol). The reaction mixture was stirred at 20°C for 16 hrs. The reaction mixture was extracted with EtOAc (3 x 30 mL). The combined organic layer was washed with saturated brine (30 mL), dried over anhydrous Na₂SO₄, filtered and concentrated to give the product. The product was purified by flash column (0-30% of EtOAc in PE) to give II-E10 (10 mg) as a solid. The solid (10 mg) was further purified by pre-HPLC (Column: Waters Xbridge 150*25 5µm); Condition: water (10mM NH₄HCO₃)-ACN; Begin B: 53%; End 79%) to give II-E10 (8.2 mg) as a solid. **¹H** NMR (400 MHz, CDCl₃) δ_{H} 7.86 (s, 1H), 7.81 (s, 1H), 4.95 (dd, *J =* 17.6, 44.8 Hz, 2H), 2.59 (t, *J =* 8.8 Hz, 1H), 2.46-2.35 (m, 1H), 2.27-2.14 (m, 1H), 2.10-1.98 (m, 2H), 1.90-1.67 (m, 5H), 1.65-1.58 (m, 2H), 1.49-1.38 (m, 4H), 1.34-0.79 (m, 14H), 0.68 (s, 3H); LC-ELSD purity ≥99%; MS ESI calcd. for C₂₆H₃₈N₃O₂[M-H₂O+H]⁺ 424.29, found 424.3.

### Example II-19 & II-20: Synthesis of 1-((1S,3aS,3bR,5aS,8S,10aS,10bS,12aS)-8-hydroxy-8,10a,12a-trimethyloctadecahydrocyclohepta[a]cyclopenta[f]naphthalen-1-yl)-2-(5-methyl-1H-tetrazol-1-yl)ethan-1-one (II-C10) & 1-((1S,3aS,3bR,5aS,8S,10aS,10bS,12aS)-8-hydroxy-8,10a,12a-trimethyloctadecahydrocyclohepta[a]cyclopenta[f]naphthalen-1-yl)-2-(5-methyl-2H-tetrazol-2-yl)ethan-1-one (II-C11)

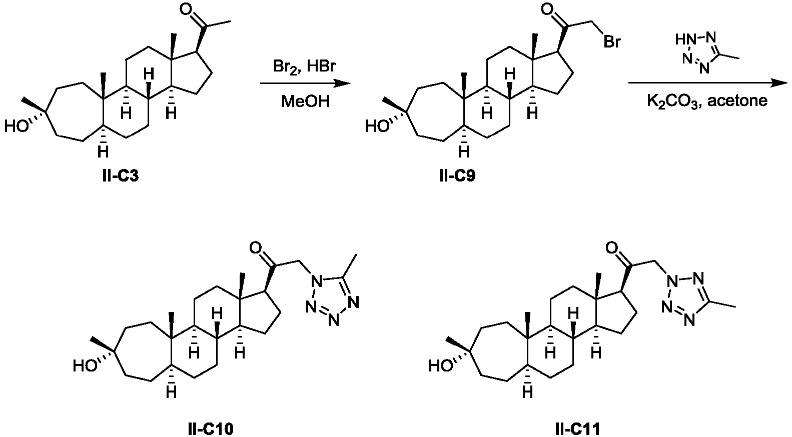

### Synthesis of II-C9

To a solution of **II-C3** (90 mg, 0.3 mmol) in MeOH (5 mL) were added HBr (10.3 mg, 0.05 mmol, 40%) and Br₂ (41.5 mg, 0.3 mmol) at 15°C. The reaction mixture was stirred for 1 hour at 15°C. The reaction mixture was added into saturated NaHCO₃ (30 mL), then extracted with EtOAc (3 x 20 mL). The combined organic layer was washed with saturated brine (30 mL), dried over anhydrous Na₂SO₄, filtered and concentrated to give **II-C9** (110 mg) as an oil, which was used directly for the next step without further purification.

**¹H NMR** (400 MHz, CDCl₃) δ_{H} 3.96-3.85 (m, 2H), 2.81 (t, *J =* 8.8 Hz, 1H), 2.25-2.13 (m, 1H), 1.91-1.62 (m, 9H), 1.34-1.15 (m, 15H), 1.00-0.86 (m, 2H), 0.79-0.74 (m, 4H), 0.62 (s, 3H).

### Synthesis of II-C10 & II-C11

To a solution of **II-C9** (250 mg, 0.59 mmol) and 5-methyl-2H-1,2,3,4-tetrazole (98.3 mg, 1.17 mmol) in acetone (20 mL) was added K₂CO₃ (161 mg, 1.17 mmol). The reaction mixture was stirred at 20 °C for 16 hrs. The reaction mixture was quenched with saturated NH₄Cl (30 mL) and extracted with EtOAc (3 x 20 mL). The combined organic phase was washed with saturated brine (20 mL), dried over anhydrous Na₂SO₄, filtered and concentrated to give the mixture product (280 mg) as a solid. The mixture product was purified by silica gel chromatography (20-100% of EtOAc in PE) to give the product **II-C10** (62.8 mg, 22%) as a solid and the product **II-C11** (29.4 mg, 11%) as a solid.

**II-C10: ¹H NMR** (400 MHz, CDCl₃) δ_{H} 5.09 (dd, *J* = 18.0, 34.4 Hz, 2H), 2.65 (t, *J* = 9.2 Hz, 1H), 2.47 (s, 3H), 2.24-2.14 (m, 1H), 2.10-2.01 (m, 1H), 1.95-1.60 (m, 8H), 1.56-1.36 (m, 4H), 1.34-1.09 (m, 12H), 1.05-0.82 (m, 2H), 0.77 (s, 3H), 0.67 (s, 3H); ELSD purity, 99%; **MS ESI** calcd. for C₂₅H₄₁N₄O₂ [M+H]⁺ 429, found 429.

**II-C11: ¹H NMR** (400 MHz, CDCl₃) δ_{H} 5.34 (d, *J* = 2.8 Hz, 2H), 2.61(t, *J =* 9.2 Hz, 1H), 2.56 (s, 3H), 2.27-2.16 (m, 1H), 2.11-2.03 (m, 1H), 1.91-1.59 (m, 8H), 1.53-1.37 (m, 4H), 1.33-1.11 (m, 12H), 0.99-0.81 (m, 2H), 0.77 (s, 3H), 0.70 (s, 3H); ELSD purity, 99%; **MS ESI** calcd. for C₂₅H₄₁N₄O₂ [M+H]⁺ 429, found 429.

### Example II-21: Synthesis of 1-((1S,3aS,3bR,5aS,8R,10aS,10bS,12aS)-8-hydroxy-8,10a,12a-trimethyloctadecahydrocyclohepta[a]cyclopenta[f]naphthalen-1-yl)-2-(5-methyl-2H-tetrazol-2-yl)ethan-1-one (II-C12)

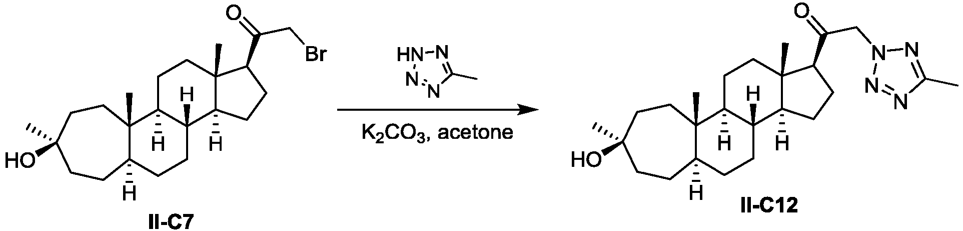

To a solution of **II-C7** (190 mg, 0.4 mmol) and 5-methyl-2H-tetrazole (75 mg, 0.9 mmol) in acetone (5 mL) was added K₂CO₃ (123 mg,0.9 mmol). The reaction mixture was stirred at 20°C for 16 hrs. The reaction mixture was extracted with EtOAc (3 x 30 mL). The combined organic layer was washed with saturated brine (30 mL), dried over anhydrous Na₂SO₄, filtered and concentrated to give the product. The product as purified by flash column (0-30% of EtOAc in PE) to give **II-C12** (52 mg, 27%) as a solid.

**¹H NMR** (400 MHz, CDCl₃) δ_{H} 5.34 (d, *J* = 1.2 Hz, 2H), 2.65-2.54 (m, 4H), 2.26-2.15 (m, 1H), 2.11-2.02 (m, 1H), 1.80-1.59 (m, 9H), 1.59-1.15 (m, 13H), 1.12-0.85 (m, 3H), 0.98-0.77 (m, 4H), 0.70 (s, 3H); LC-ELSD purity 99%; **MS ESI** calcd. for C₂₅H₄₁N₄O₂ [M+H]⁺ 429 found 429.

### EXAMPLE II-22: Synthesis of 1-(2-((1S,3aS,3bS,8S,10aR,10bS,12aS)-8-hydroxy-8,10a,12a-trimethyl-1,2,3,3a,3b,4,6,7,8,9,10,10a,10b,11,12,12a-hexadecahydrocyclohepta[a]cyclopenta[f]naphthalen-1-yl)-2-oxoethyl)-1H-pyrazole-4-carbonitrile (II-F3)

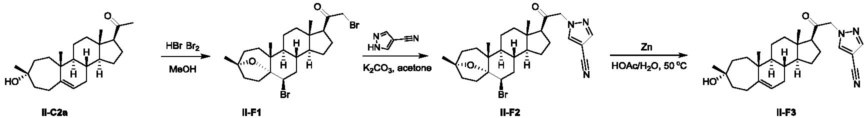

### Synthesis of II-F1

To a solution of **II-C2a** (150 mg, 0.4 mmol) in MeOH (5 mL) was added HBr (17.4 mg, 0.09 mmol, 40%) and Br₂ (139 mg, 0.9 mmol) at 20°C. After stirring at 20°C for 1 h, the reaction mixture was diluted with NaHCO₃ (20 mL) and extracted with EtOAc (3 x 15 mL). The combined organic solution was washed with saturated brine (30 mL), dried over Na₂SO₄, filtered and concentrated to give **II-F1** (200 mg) as an oil, which was used directly for the next step without further purification.

### Synthesis of II-F2

To a solution of **II-F1** (200 mg, 0.4 mmol) in acetone (5 mL) was added K₂CO₃ (109 mg, 0.8 mmol) and 1H-pyrazole-4-carbonitrile (74.1 mg, 0.8 mmol) at 20°C. After stirring at 20 °C for 16 h, the reaction mixture was diluted with saturated NH₄Cl (20 mL) and extracted with EtOAc (3 x 20 mL). The combined organic solution was washed with saturated brine (30 mL), dried over anhydrous Na₂SO₄, filtered and concentrated. The residue was purified by flash column (0-20% of EtOAc in PE) to give **II-F2** (120 mg, 59%) as a solid.

**¹H NMR** (400 MHz, CDCl₃) δ_{H} 7.86-7.81 (m, 2H), 5.15-4.80 (m, 2H), 4.20-4.00 (m, 1H), 2.85-2.55 (m, 1H), 2.40-2.15 (m, 2H), 2.10-1.65 (m, 11H), 1.50-1.15 (m, 14H), 1.00 (s, 1H), 0.71 (s, 2H).

### Synthesis of II-F3

To a solution of **II-F2** (120 mg, 0.2 mmol) in AcOH (10 mL) and water (0.5 mL) was added zinc (0.763 mg, 0.01 mmol) at 20 °C. After stiring at 50°C for 1 h, the reaction mixture was diluted with NH₄Cl (20 mL) and extracted with EtOAc (3 x 20 mL). The combined organic solution was washed with saturated brine (50 mL), dried over anhydrous Na₂SO₄, filtered and concentrated. The residue was purified by flash column (0-20% of EtOAc in PE) to give **II-F3** (75.1 mg, 74%) as a solid.

**¹H NMR** (400 MHz, CDCl₃) δ_{H} 7.86 (s, 1H), 7.81 (s, 1H), 5.46 (d, *J =* 4.0 Hz, 1H), 4.95 (dd, *J =* 18.0, 49.2 Hz, 2H), 2.61 (t, *J =* 8.8 Hz, 1H), 2.30-2.15 (m, 1H), 2.08-1.65 (m, 10H), 1.60-1.40 (m, 5H), 1.35-1.15 (m, 9H), 0.89 (s, 3H), 0.69 (s, 3H); **LC-ELSD/MS** purity 99%, MS ESI calcd. for C₂₇H₃₈N₃O₂ [M+H]⁺ 436, found 436.

### EXAMPLE II-23 & II-24: Synthesis of 1-((1S,3aS,3bS,8S,10aR,10bS,12aS)-8-hydroxy-8,10a,12a-trimethyl-1,2,3,3a,3b,4,6,7,8,9,10,10a,10b,11,12,12a-hexadecahydrocyclohepta[a]cyclopenta[f]naphthalen-1-yl)-2-(5-methyl-2H-tetrazol-2-yl)ethan-1-one (II-F6) & 1-((1S,3aS,3bS,8S,10aR,10bS,12aS)-8-hydroxy-8,10a,12a-trimethyl-1,2,3,3a,3b,4,6,7,8,9,10,10a,10b,11,12,12a-hexadecahydrocyclohepta[a]cyclopenta[f]naphthalen-1-yl)-2-(5-methyl-1H-tetrazol-1-yl)ethan-1-one (II-F7)

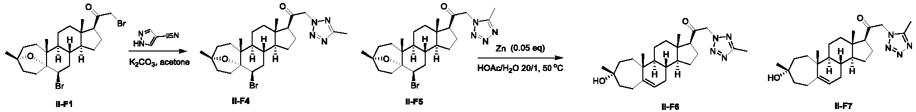

### Synthesis of II-F4 & II-F5

To a solution of **II-F1** (350 mg, 0.7 mmol) in acetone (20 mL) was added K₂CO₃ (191 mg, 1.4 mmol) and 5-methyl-2H-1,2,3,4-tetrazole (116 mg, 1.4 mmol) at 20°C. After stirring for 2 h at 20 °C, the reaction mixture was diluted with saturated NH₄Cl (100 mL) and extracted with EtOAc (3 x 50 mL). The combined organic solution was washed with saturated brine (100 mL), dried over anhydrous Na₂SO₄, filtered and concentrated to give a mixture of **II-F4 & II-F5** (300 mg) as an oil, which was used directly for next step without further purification.

### Synthesis of II-F6 & II-F7

To a solution of a mixture of **II-F4 & II-F5** (200 mg) in AcOH/water (10 mL/0.5 mL) was added zinc (5 mg) at 20°C. After stirring for 16h at 50 °C, he reaction mixture was filtered. The filtrate was diluted with saturated NaHCO₃ (100 mL) and extracted with EtOAc (3 x 50 mL). The combined organic solution was washed with saturated brine (100 mL), dried over anhydrous Na₂SO₄, filtered and concentrated to give a mixture of **II-F6** & **II-F7** (200 mg) as an oil. The mixture was purified by flash column (0-60% of EtOAc in PE) to give **II-F6** (60 mg) and **II-F7** (25 mg) as solids.

**II-F6** (60 mg) was further purified by pre-HPLC (Column: Agela DuraShell 150mm_25mm_5um, Condition: water (0.04%NH₃H₂O+10mM NH₄HCO₃)-ACN, Begin B: 56, End B: 86, Gradient Time (min): 8.5, 100% B Hold Time (min): 2) to give **II-F6** (10 mg) as a solid.

**II-F7: ¹H NMR** (400 MHz, CDCl₃) δ_{H} 5.46 (d, *J=* 3.6 Hz, 1H), 5.10 (dd, *J =* 18.4 Hz, 37.6 Hz, 2H), 2.67 (t, *J =* 8.4 Hz, 1H), 2.48 (s, 3H), 2.33-1.63 (m, 12H), 1.52-1.28 (m, 10H), 1.26 (s, 3H), 0.90 (s, 3H), 0.71 (s, 3H); **LC-ELSD/MS** purity 99%, MS ESI calcd. for C₂₅H₃₇N₄O [M+H-H₂O]⁺ 409, found 409.

**II-F6: ¹H NMR** (400 MHz, CDCl₃) δ_{H} 5.46 (d, *J* = 4.0 Hz, 1H), 5.37 (d, *J =* 1.6 Hz, 2H), 2.63 (t, *J =* 8.4 Hz, 1H), 2.57 (s, 3H), 2.33-1.63 (m, 12H), 1.52-1.28 (m, 8H), 1.26 (s, 3H), 1.24-1.15 (m, 2H), 0.90 (s, 3H), 0.73 (s, 3H); **LC-ELSD/MS** purity 99%, MS ESI calcd. for C₂₅H₃₇N₄O [M+H-H₂O]⁺ 409, found 409.

### EXAMPLE II-25 & II-26: Synthesis of 1-((1S,3aS,3bR,5aR,8S,10aS,10bR,12aS)-8-hydroxy-8,12a-dimethyloctadecahydrocyclohepta[a]cyclopenta[f]naphthalen-1-yl)ethan-1-one (II-F8) & 1-((1S,3aS,3bR,5aR,8R,10aS,10bR,12aS)-8-hydroxy-8,12a-dimethyloctadecahydrocyclohepta[a]cyclopenta[f]naphthalen-1-yl)ethan-1-one (II-F9)

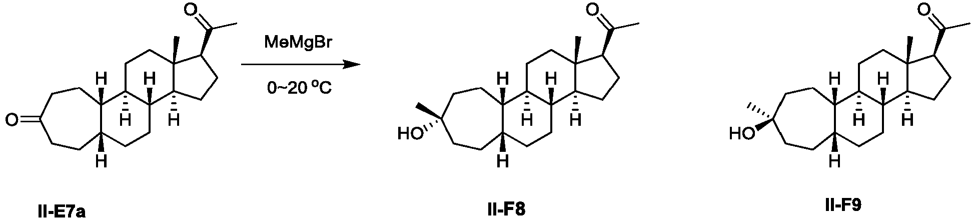

To a solution of **II-E7a** (200 mg, 0.6 mmol) in anhydrous THF (2 mL) at 0°C under N₂ was added MeMgBr (0.25 mL, 0.8 mmol, 3M in ethyl ether) dropwise at 0°C. After stirring at 20°C for another 2 h, the reaction mixture was diluted with saturated aq.NH₄Cl (20 mL) below 10°C and extracted with EtOAc (2 x 20 mL). The combined organic solution was dried over anhydrous Na₂SO₄, filtered and concentrated in vacuum. The residue was purified by flash column (10~20% EtOAc in PE) to give **II-F8 & II-F9** (100 mg) as a solid which was separated by pre-HPLC (Column: Xbridge 150*30mm*10um; Condition: water (10mM NH₄HCO₃)-ACN; Begin B: 60%; End 90%) to give **II-F8** (15.8 mg) and **II-F9** (8.0 mg) both as solids. The structure of **II-F8** was confirmed by X-ray.

**II-F8: ¹H NMR** (400 MHz, CDCl₃) δ_{H} 2.53 (t, *J =* 8.4 Hz, 1H), 2.21-2.07 (m, 4H), 2.03-1.93 (m, 1H), 1.86-1.59 (m, 9H), 1.54-1.35 (m, 7H), 1.26-0.89 (m, 11H), 0.61 (s, 3H); **LC-ELSD/MS** purity≥99%, MS ESI calcd. for C₂₂H₃₅O[M-H₂O+H]⁺ 315.27, found 315.2.

**II-F9: ¹H NMR** (400 MHz, CDCl₃) δ_{H} 2.54 (t, *J =* 8.8 Hz, 1H), 2.20-2.08 (m, 4H), 2.04-1.95 (m, 1H), 1.89-1.77 (m, 2H), 1.71-1.58 (m, 7H), 1.53-1.34 (m, 7H), 1.29-0.90 (m, 11H), 0.62 (s, 3H); **LC-ELSD/MS** purity≥99%, MS ESI calcd. for C₂₂H₃₅O[M-H₂O+H]⁺ 315.27, found 315.3.

### EXAMPLE II-27: Synthesis of 1-(2-((1S,3aS,3bR,5aR,8S,10aS,10bR,12aS)-8-hydroxy-8,12a-dimethyloctadecahydrocyclohepta[a]cyclopenta[f]naphthalen-1-yl)-2-oxoethyl)-1H-pyrazole-4-carbonitrile (II-F11)

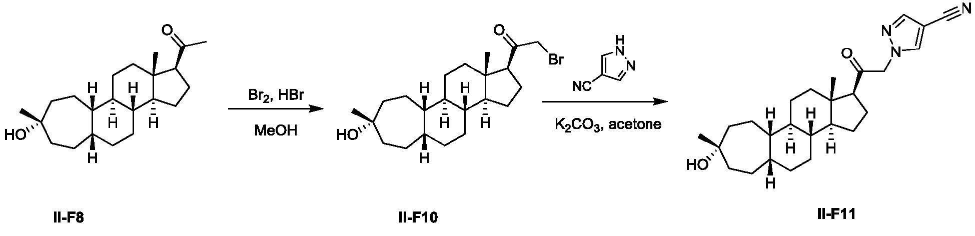

### Synthesis of II-F10

To a solution of **II-F8** (40 mg, 0.1 mmol) in MeOH (2 mL) were added HBr (4.80 mg, 0.024 mmol) and Br₂ (19.2 mg, 0.1 mmol) at 15°C. After stirring at 25°C for 2 h, the reaction mixture was diluted with saturated NaHCO₃ (10 mL) and extracted with EtOAc (3 x 10 mL). The combined organic solution was washed with saturated brine (15 mL), dried over anhydrous Na₂SO₄, filtered and concentrated to give **II-F10** (49.4 mg) as an oil, which was used directly for the next step without further purification.

**¹H NMR** (400 MHz, CDCl₃) δ_{H} 3.91 (dd, *J* =13.2*,* 16.8 Hz, 2H), 2.81 (t, *J* = 8.8 Hz, 1H), 2.24-2.09(m, 1H), 1.92-1.61 (m, 10H), 1.55-1.32 (m, 10H), 1.24-1.22 (m, 3H), 1.14-0.89 (m, 5H), 0.64 (s, 3H)

### Synthesis of II-F11

To a solution of **II-F10** (49.4 mg, 0.1 mmol) and 1H-pyrazole-4-carbonitrile (22.3 mg, 0.2 mmol) in acetone (2 mL) was added K₂CO₃ (33.1 mg, 0.24 mmol). After stirring at 20 °C for 16 h, the reaction mixture was extracted with EtOAc (3 x 30 mL). The combined organic solution was washed with saturated brine (30 mL), dried over anhydrous Na₂SO₄, filtered and concentrated. The residue was purified by flash column (0-20% of EtOAc in PE) to give **II-F11 (28.5** mg, 56%) as a solid.

**¹H NMR** (400 MHz, CDCl₃) δ_{H} 7.85 (s, 1H), 7.81 (s, 1H), 5.06-4.86 (dd, *J =* 17.6, 47.6 Hz, 2H) , 2.60 (t, *J =* 8.8 Hz), 2.27-2.15 (m, 1H), 2.06-1.99 (m, 1H), 1.84-1.67 (m, 6H), 1.56-1.37 (m, 8H), 1.33-0.80 (m, 13H), 0.67 (s, 3H); **LC-ELSD/MS** purity 99.30%, MS ESI calcd. for C₂₆H₃₆N₃O[M-H₂₀+H]⁺ 406.29, found 406.3.

### EXAMPLE II-28 & II-29: Synthesis of 1-((1S,3aS,3bR,5aR,8S,10aS,10bR,12aS)-8-hydroxy-8,12a-dimethyloctadecahydrocyclohepta[a]cyclopenta[f]naphthalen-1-yl)-2-(5-methyl-2H-tetrazol-2-yl)ethan-1-one (II-F12) & 1-((1S,3aS,3bR,5aR,8S,10aS,10bR,12aS)-8-hydroxy-8,12a-dimethyloctadecahydrocyclohepta[a]cyclopenta[f]naphthalen-1-yl)-2-(5-methyl-1H-tetrazol-1-yl)ethan-1-one (II-F13)

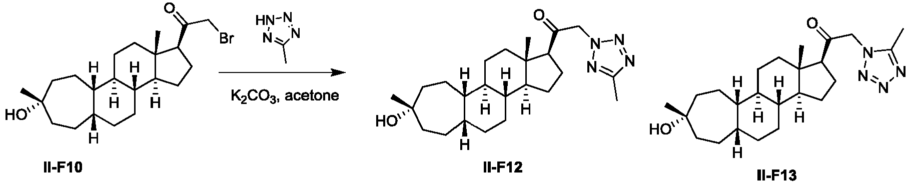

To a solution of **II-F10** (130 mg, 0.32 mmol) and 5-methyl-2H-1,2,3,4-tetrazole (39.8 mg, 0.47 mmol) in acetone (10 mL) was added K₂CO₃ (88.5 mg, 0.63 mmol). After stirring at 25 °C for 16 h, the reaction mixture was diluted with saturated NH₄Cl (30 mL) and extracted with EtOAc (3 x 20 mL). The combined organic solution was washed with saturated brine (20 mL), dried over anhydrous Na₂SO₄, filtered and concentrated. The residue was purified by silica gel chromatography (20-100% of EtOAc in PE) to give **II-F12** (35 mg) and **II-F13** (48 mg) as solids. **II-F12** (35 mg) was further purified by prep-HPLC (Column: Boston Prime C18 150*30mm 5 µm; Condition: water (0.05% ammonia hydroxide v/v)-ACN; Begin B: 65%; End B: 95%; Gradient Time(min): 9; 100%B Hold Time(min): 2) to give **II-F12** (22.2 mg, 64%) as a solid.

**II-F12: ¹H NMR** (400 MHz, CDCl₃) δ_{H} 5.42-5.27 (m, 2H), 2.67-2.59 (m, 1H), 2.56 (s, 3H), 2.27-2.15 (m, 1H), 2.10-2.02 (m, 1H), 1.83-1.69 (m, 6H), 1.67-1.58 (m, 5H), 1.52-1.39 (m, 5H), 1.29-1.20 (m, 5H), 1.18-0.91 (m, 6H), 0.72 (s, 3H); **LC-ELSD/MS** purity≥99%, MS ESI calcd. for C₂₄H₃₇N₄O [M-H₂O+H]⁺ 397.30, found 397.3.

**II-F13: ¹H NMR** (400 MHz, CDCl₃) δ_{H} 5.10 (dd, *J=* 18.0, 33.6 Hz, 2H), 2.71-2.60 (m, 1H), 2.47 (s, 3H), 2.27-2.14 (m, 1H), 2.08-2.00 (m, 1H), 1.85-1.70 (m, 6H), 1.67-1.59 (m, 4H), 1.55-1.36 (m, 6H), 1.32-1.26 (m, 2H), 1.25-1.21 (m, 3H), 1.19-0.90 (m, 6H), 0.69 (s, 3H); **LC-ELSD/MS** purity≥99%, MS ESI calcd. for C₂₄H₃₇N₄O [M-H₂O+H]⁺ 397.30, found 397.3.

### EXAMPLE II-30: Synthesis of 1-((1S,3aS,3bR,5aR,8S,10aS,10bR,12aS)-8-hydroxy-8-(methoxymethyl)-12a-methyloctadecahydrocyclohepta[a]cyclopenta[f]naphthalen-1-yl)ethan-1-one (II-F19)

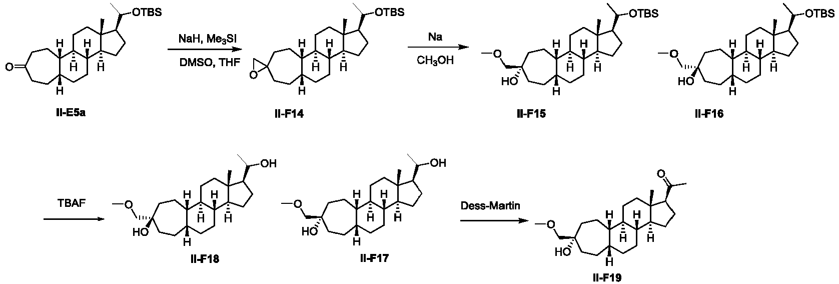

### Synthesis of II-F14

To a stirred solution of Me₃SI (1.40 g, 6.9 mmol) in DMSO (6 mL) and THF (3 mL) was added NaH (275 mg, 6.9 mmol, 60% in oil) at 0°C. After stirring for 1 h under N₂, the mixture was then added to a solution of **II-E5a** (600 mg, 1.4 mmol) in DMSO (2 mL). After stirring at 25°C for 16 h, the reaction mixture was poured into ice-water (10 mL) and extracted with EtOAc (2 x 20 mL). The combined organic solution was washed with water (2 x 10 mL), brine (10 mL), dried over anhydrous Na₂SO₄, filtered, and concentrated. The residue was purified by silica gel chromatography (0-40% of EtOAc in PE) to give **II-F14** (450 mg) as a solid.

**¹H NMR** (400 MHz, CDCl₃) δ_{H} 3.74-3.57 (m, 1H), 2.68-2.52 (m, 2H), 2.02-1.76 (m, 6H), 1.74-1.56 (m, 5H), 1.54-1.30 (m, 7H), 1.28-0.93 (m, 11H), 0.88 (s, 9H), 0.65 (s, 3H), 0.07-0.01 (m, 6H).

### Synthesis of II-F15 & II-F16

Fresh Na (240 mg, 10.0 mmol) was carefully added to MeOH (10 mL) in portions. After stirring at 20°C for 3h, **II-F14** (450 mg, 1.0 mmol) in MeOH (5 mL) was added. After stirring at 75°C for 16 h, water (30 mL) was added. The reaction mixture was concentrated to remove most of the solvent and then extracted with EtOAc (2 x 30 mL). The combined organic solution was washed with saturated brine (50 mL), dried over anhydrous Na₂SO₄, filtered and concentrated. The residue was purified by flash chromatography (0-40% of EtOAc in PE) to give **II-F16** (90 mg) and **II-F15** (250 mg) as oils.

**II-F15: ¹H NMR** (400 MHz, CDCl₃) δ_{H} 3.70-3.61 (m, 1H), 3.38 (s, 3H), 3.16 (s, 2H), 1.94-1.58 (m, 10H), 1.56-1.21 (m, 10H), 1.18-0.96 (m, 10H), 0.87 (s, 9H), 0.63 (s, 3H), 0.04 (s, 3H), 0.03 (s, 3H).

**II-F16: ¹H NMR** (400 MHz, CDCl₃) δ_{H} 3.71-3.62 (m, 1H), 3.40 (s, 3H), 3.22 (s, 2H), 1.99-1.63 (m, 6H), 1.56-1.32 (m, 13H), 1.20-0.96 (m, 10H), 0.89-0.83 (m, 10H), 0.64 (s, 3H), 0.05 (s, 3H), 0.04 (s, 3H).

### Synthesis of II-F17 & II-F18

To a solution of **II-F15 & II-F16** (240 mg, 0.5 mmol) in THF (3 mL) was added TBAF.3H₂O (780 mg, 2.5 mmol) at 15°C. After the solution stirring at 55°C for 12 h, the mixture was poured into water (50 mL) and extracted with EtOAc (2 x 30 mL). The combined organic solution was washed with saturated brine (2 x 50 mL), dried over anhydrous Na₂SO₄, filtered and concentrated. The residue was purified by flash column (0-15% of EtOAc in PE) and then purified by pre-HPLC (Column: Xbridge 150*30mm*10µm; Condition: water (10mM NH₄HCO₃)-ACN; Begin B: 55%; End 85%) to give **II-F18** (30 mg) and **II-F17** (40 mg) as solids.

**II-F18: ¹H NMR** (400 MHz, CDCl₃) δ_{H} 3.76-3.63 (m, 1H), 3.39 (s, 3H), 3.25-3.19 (m, 2H), 2.23 (s, 1H), 1.97-1.76 (m, 4H), 1.73-1.29 (m, 13H), 1.24-0.85 (m, 13H), 0.67 (s, 3H); **LC-ELSD/MS** purity 99%, MS ESI calcd. for C₂₃H₄₀O₃Na [M+Na]⁺ 387, found 387 **II-F17: ¹H NMR** (400 MHz, CDCl₃) δ_{H} 3.76-3.61 (m, 1H), 3.38 (s, 3H), 3.16 (s, 2H), 2.07 (s, 1H), 1.95-1.28 (m, 18H), 1.27-0.87 (m, 12H), 0.67 (s, 3H); **LC-ELSD/MS** purity 99%, MS ESI calcd. for C₂₃H₄₀O₃Na [M+Na]⁺ 387, found 387

### Synthesis of II-F19

To a solution of **II-F17** (150 mg, 0.4 mmol) in DCM (2 mL) was added Dess-Martin periodinane (521 mg, 1.2 mmol). After stirring at 20 °C for 1 h, the reaction mixture was quenched with saturated NaHCO₃ (15 mL) and saturated Na₂S₂O₃ (20 mL) at 0°C and stirred for 20 min. The mixture was extracted with DCM (2 x 20 mL). The combined organic solution was washed with saturated NaHCO₃ (2 x 20 mL), brine (20 mL), dried over anhydrous Na₂SO₄, filtered and concentrated to give **II-F19** (110 mg, 74%) as a solid.

**¹H NMR** (400 MHz, CDCl₃) δ_{H} 3.38 (s, 3H), 3.16 (s, 2H), 2.54 (t, *J* = 9.2 Hz, 1H), 2.19-2.08 (m, 4H), 2.02-1.82 (m, 2H), 1.80-1.59 (m, 9H), 1.56-1.29 (m, 7H), 1.27-0.91 (m, 7H), 0.61 (s, 3H); **LC-ELSD/MS** purity 99%, MS ESI calcd. for C₂₃H₃₇O₂ [M-H₂O+H]⁺ 345, found 345

### EXAMPLE II-31: Synthesis of 1-(2-((1S,3aS,3bR,5aR,8S,10aS,10bR,12aS)-8-hydroxy-8-(methoxymethyl)-12a-methyloctadecahydrocyclohepta[a]cyclopenta[f]naphthalen-1-yl)-2-oxoethyl)-1H-pyrazole-4-carbonitrile (II-F21)

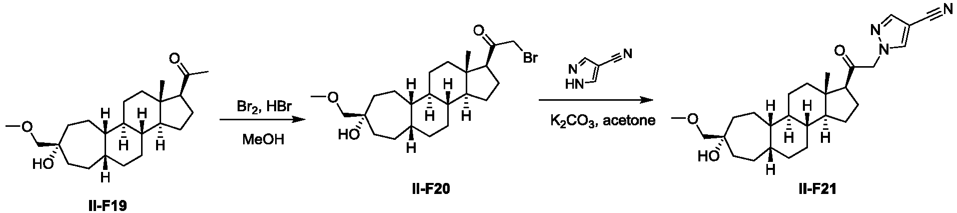

### Synthesis of II-F20

To a solution of **II-F19** (100.5 mg, 0.3 mmol) in MeOH (2 mL) were added HBr (11.0 mg, 0.06mmol) and Br₂ (44.1 mg, 0.3 mmol) at 25°C. After stirring at 25°C for 2 h, the reaction mixture was added into saturated NaHCO₃ (20 mL) and extracted with EtOAc (3 x 10 mL). The combined organic solution was washed with saturated brine (20 mL), dried over anhydrous Na₂SO₄, filtered and concentrated to give **II-F20** (121 mg) as an oil, which was used directly to the next step without further purification.

### Synthesis of II-F21

To a solution of **II-F20** (121 mg, 0.3 mmol) and 1H-pyrazole-4-carbonitrile (51.0 mg, 0.5 mmol) in acetone (1 mL) was added K₂CO₃ (75.6 mg, 0.5 mmol). After stirring at 20 °C for 16 h, the reaction mixture was extracted with EtOAc (3 x 30 mL). The combined organic solution was washed with saturated brine (30 mL), dried over anhydrous Na₂SO₄, filtered and concentrated. The residue was purified by flash column (0-30% of EtOAc in PE) to give II-**F21** (48 mg, 39%) as a solid.

**¹H NMR** (400 MHz, CDCl₃) δ_{H} 7.85 (s, 1H), 7.81 (s, 1H), 4.95 (dd, *J* = 18.0, 48.8 Hz, 2H), 3.38 (s, 3H), 3.17 (s, 2H), 2.61 (t, *J* = 8.8 Hz, 1H), 2.27-1.98 (m, 3H), 1.95-1.63 (m, 7H), 1.52-0.86 (m, 15H), 0.67 (s, 3H); **LC-ELSD/MS** purity 99%, MS ESI calcd. for C₂₇H₄₀N₃O₃ [M+H]⁺ 454, found 454.

### EXAMPLE II-32: Synthesis of 1-((1S,3aS,3bR,5aR,7S,8aS,8bR,10aS)-7-hydroxy-7,10a-dimethylhexadecahydrodicyclopenta[a,f]naphthalen-1-yl)ethan-1-one (II-G8)

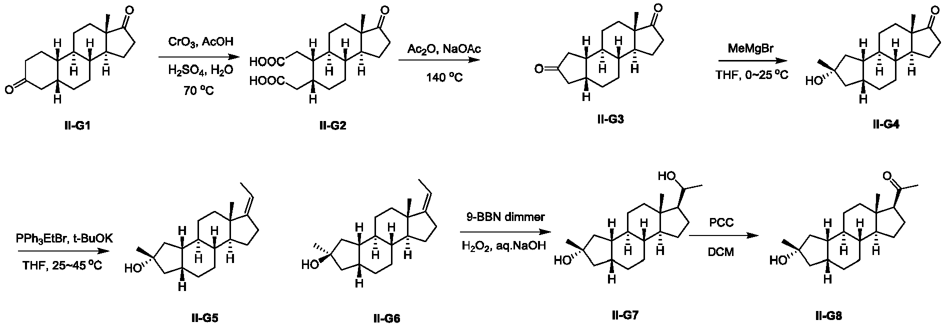

### Synthesis of II-G2

A solution of CrO₃ (20.7 g, 207 mmol) in H₂SO₄ (98%, 30 mL) and water (145 mL) was added dropwise to a stirred solution of **II-G1** (15 g, 54.6 mmol) in AcOH (150 mL) at 65°C. After stirring at 70°C for 1 h, crushed ice and water (500 mL) were added and the mixture was stirred for 1 h at 25°C. The aqueous solution was extracted with CH₂Cl₂ (2 x 200 mL) and the combined extracts solutions were washed with brine (2 x 200 mL), dried over anhydrous Na₂SO₄, filtered and the filtrate was evaporated to afford **II-G2** (17 g) as an oil, which was used as is.

**¹H NMR** (400 MHz, CDCl₃) δ_{H} 2.95-2.65 (m, 1H), 2.60-2.20 (m, 7H), 2.10-1.70 (m, 4H), 1.60-1.10 (m, 9H), 1.05-0.80 (m, 5H).

### Synthesis of II-G3

To a solution of **II-G2** (5.6 g, 17.3 mmol) in Ac₂O (50 mL) was added sodium acetate (1.41 g, 17.3 mmol). After stirring at 140°C for 16 h, the reaction was concentrated and water (200 mL) was added. The aqueous solution was extracted with CH₂Cl₂ (2 x 100 mL) and the combined extracts were washed with saturated brine (100 mL), dried over anhydrous Na₂SO₄, filtered and the filtrate was evaporated. The product was purified together with another batch (1g). The residue was purified by flash column (30~40% of EtOAc in PE) to afford **II-G3** (1.3 g) as a solid.

**¹H NMR** (400 MHz, CDCl₃) δ_{H} 2.60-2.40 (m, 2H), 2.35-2.25 (m, 2H), 2.20-2.00 (m, 3H), 2.00-1.90 (m, 2H), 1.90-1.60 (m, 5H), 1.55-1.45 (m, 1H), 1.40-1.20 (m, 3H), 1.15-1.10 (m, 2H), 0.90 (s, 3H), 0.75-0.65 (m, 1H). **LC-ELSDMS** purity 99%, MS ESI calcd. for C₁₈H₂₄O₂ [M+H]⁺ 261, found 261. The structure was confirmed by COSY NMR.

### Synthesis of II-G4

To a solution of **II-G3** (13 g, 49.9 mmol) in anhydrous THF (130 mL) under N₂ was added MeMgBr (18.2 mL, 54.8 mmol, 3M in ethyl ether) dropwise at 0°C. After stirring at 25°C for 2 h, the reaction mixture was poured into saturated aq.NH₄Cl (300 mL) below 10°C and extracted with EtOAc (2 x 100 mL). The combined organic solutions were dried over anhydrous Na₂SO₄, filtered and concentrated in vacuum. The product was purified by flash column (20~35% of EtOAc in PE) to give **II-G4** (3.4 g) as a solid.

**¹H NMR** (400 MHz, CDCl₃) δ_{H} 2.43 (dd, *J* = 8.4, 19.2 Hz, 1H), 2.20-1.60 (m, 11H), 1.60-1.40 (m, 3H), 1.40-1.20 (m, 6H), 1.20-0.95 (m, 4H), 0.88 (s, 3H). **NOE** confirms H18 and H4 as cis orientation. **LC-ELSD/MS** purity 99%, MS ESI calcd. for C₁₈H₂₇O [M-H₂O+H]⁺ 259, found 259.

### Synthesis of II-G5 & II-G6

To a suspension of bromo(ethyl)triphenylphosphorane (7.49 g, 20.2 mmol) in THF (40 mL) was added t-BuOK (2.26 g, 20.2 mmol) at 25°C under N₂. After stirring 45°C and for 1 h, a solution of**II-G4** (1.4 g, 5.06 mmol) in THF (10 mL) was added. After stirring at 45°C for 16 h, the mixture was treated with NH₄Cl (80 mL) and extracted with EtOAc (2 x 50 mL). The combined organic solution was washed with brine (2 x 50 mL), dried over anhydrous Na₂SO₄, filtered, concentrated in vacuum. The residue was purified by flash column (0~15% of EtOAc in PE) to give **II-G5** (500 mg, 34%) and **II-G6** (500 mg) as solids. The stereochemistry was confirmed by 2D NMR (NOE and HMBC). **II-G5: ¹H NMR** (400 MHz, CDCl₃) δ_{H} 5.20-5.05 (m, 1H), 2.45-2.30 (m, 1H), 2.25-2.05 (m, 3H), 1.90 (t, *J* = 12.8 Hz, 1H), 1.85-1.60 (m, 3H), 1.60-1.55 (m, 8H), 1.55-1.45 (m, 3H), 1.34 (s, 3H), 1.30-0.95 (m, 6H), 0.88 (s, 3H); **LC-ELSD/MS** purity 99%, MS ESI calcd. for C₂₀H₃₁ [M-H₂O+H]⁺ 271, found 271.

### Synthesis of II-G7

To a solution of **II-G5** (0.5 g, 1.73 mmol) in THF (15 mL) was added 9-BBN dimer (844 mg, 3.46 mmol) at 25°C. After stirring at 25°C for 16 h, the reaction was cooled to 0°C and ethanol (795 mg, 17.3 mmol) and aqueous NaOH (3.46 mL, 5 M, 17.3 mmol) were added to the solution very slowly. After the addition was complete, H₂O₂ (1.73 mL, 17.3 mmol, 30% in water) was added slowly and the inner temperature was maintained below 15°C. After stirring at 75°C for 1 h, saturated aqueous Na₂S₂O₃ (300 mL) was added. After stirring at 0°C for 1 h, the mixture was extracted with EtOAc (3 x 30 mL). The combined organic solution was washed with saturated brine (50 mL), dried over anhydrous Na₂SO₄, filtered and concentrated under vacuum to give **II-G7** (500 mg) as an oil, which was used directly for the next step without further purification.

**¹H NMR** (400 MHz, CDCl₃) δ_{H} 4.00-3.70 (m, 1H), 2.20-2.05 (m, 1H), 2.00-1.60 (m, 10H), 1.50-1.20 (m, 9H), 1.20-0.90 (m, 10H), 0.67 (s, 3H).

### Synthesis of II-G8

To a solution of **II-G7** (500 mg, 1.63 mmol) in DCM (20 mL) were added PCC (1.05 g, 4.89 mmol) and silica gel (1.5 g) at 25°C. After stirring at 25°C for 1h, the reaction mixture was filtered, and the residue was washed with anhydrous DCM (2 x 20 mL). The combined filtrate was concentrated in vacuum and purified by flash column (0~20% of EtOAc in PE) to give **II-G8** (232 mg, 47%) as a solid.

**¹H NMR** (400 MHz, CDCl₃) δ_{H} 2.54 (t, *J* = 8.8 Hz, 1H), 2.20-2.05 (m, 5H), 2.00-1.85 (m, 2H), 1.80-1.60 (m, 7H), 1.60-1.35 (m, 8H), 1.30-0.95 (m, 6H), 0.62 (s, 3H); **LC-ELSD/MS** purity 99%, MS ESI calcd. for C₂₀H₃₁O [M-H₂O+H]⁺ 287, found 287.

### EXAMPLE II-33: Synthesis of 1-(2-((1S,3aS,3bR,5aR,7S,8aS,8bR,10aS)-7-hydroxy-7,10a-dimethylhexadecahydrodicyclopenta[a,f]naphthalen-1-yl)-2-oxoethyl)-1H-pyrazole-4-carbonitrile (II-G10)

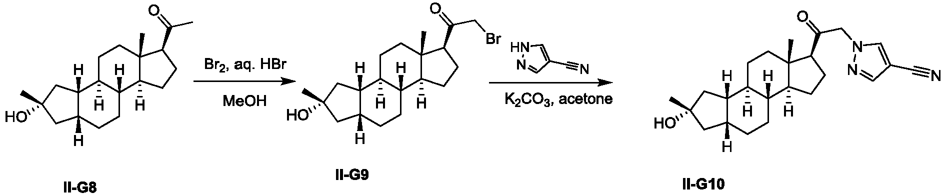

### Synthesis of II-G9

To a solution of **II-G8** (200 mg, 0.66 mmol) in MeOH (5 mL) were added HBr (26.2 mg, 0.13 mmol, 40% in HOAc) and Br₂ (105 mg, 0.66 mmol) at 25°C. After stirring at 25°C for 2 h, the reaction mixture was added to saturated NaHCO₃ (20 mL) and extracted with EtOAc (3 x 20 mL). The combined organic solution was washed with saturated brine (50 mL), dried over anhydrous Na₂SO₄, filtered and concentrated to give **II-G9** (200 mg) as an oil, which was used directly for the next step without further purification.

### Synthesis of II-G10

To a solution of **II-G9** (200 mg, 0.52 mmol) in acetone (5 mL) were added K₂CO₃ (143 mg, 1.04 mmol) and 1H-pyrazole-4-carbonitrile (96.8 mg, 1.04 mmol) at 25°C. After stirring at 25°C for 16 h, the reaction mixture was added to saturated NH₄Cl (20 mL) and extracted with EtOAc (3 x 20 mL). The combined organic solution was washed with saturated brine (50 mL), dried over anhydrous Na₂SO₄, filtered and concentrated to give product. The residue was purified by prep-HPLC (Column: Xtimate C18 150*25mm*5µm; Condition: water (10mM NH₄HCO₃)-ACN; Begin B: 52; End B: 82; Gradient Time (min): 8; 100%B Hold Time (min): 2) to give **II-G10** (46.7 mg, 23%) as a solid. **¹H NMR** (400 MHz, CDCl₃) δ_{H} 7.85 (s, 1H), 7.80 (s, 1H), 4.96 (dd, *J =* 18.0, 46.0 Hz, 2H), 2.60 (t, *J* = 8.8 Hz, 1H), 2.30-2.10 (m, 2H), 2.10-2.00 (m, 1H), 1.90 (t, *J* = 12.8 Hz, 1H), 1.85-1.60 (m, 5H), 1.60-1.35 (m, 6H), 1.35 (s, 3H), 1.34-0.95 (m, 7H), 0.68 (s, 3H). LC-ELSD/MS purity 99%, MS ESI calcd. for C₂₄H₃₄N₃O₂ [M+H]⁺ 396, found 396. Structure confirmed by xray.

### EXAMPLE II-34: Synthesis of 1-((1S,3aS,3bR,5aR,6R,8aR,8bS,10aS)-6-hydroxy-6,10a-dimethylhexadecahydrodicyclopenta[a,f]naphthalen-1-yl)ethan-1-one (II-H11)

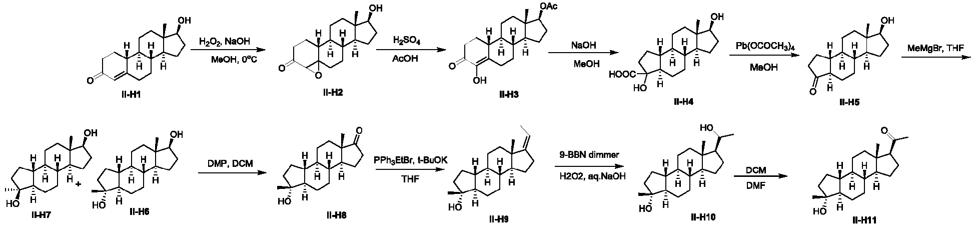

### Synthesis of II-H2

To a solution of **II-H1** (10.0 g, 36.4 mmol) in methanol (100 ml) was added 30% hydrogen peroxide (62 ml) and 10% NaOH (21 mL) at 0°C. After stirring at 0°C for 16 h, the reaction was diluted with water (500 mL) and extracted with EtOAc (2 x 200mL). The combined organic solution was washed with 10% Na₂S₂O₃ (300 mL), brine (100 mL), dried over Na₂SO₄ and filtered, concentrated in vacuum to afford **II-H2** (9.2 g, 87.6%) as a solid, which was used as is.

**¹H NMR** (400 MHz, CDCl3) δ_{H} 4.53 (t, *J* = 8.0 Hz, 1H), 3.02 (s, 1H), 2.35-2.26 (m, 1H), 2.13-1.94 (m, 5H), 1.89-1.80 (m, 2H), 1.74-1.56 (m, 4H), 1.48-1.28 (m, 5H), 1.12-0.90 (m, 4H), 0.77 (s, 3H).

### Synthesis of II-H3

To a solution of **II-H2** (8.2 g, 28.2 mmol) in CH₃COOH (82 ml) was added concentrated H₂SO₄ (8.2 ml). After stirring at 25°C for 18 h, the reaction mixture was diluted with ice water (300 mL), neutralized with sat. NaHCO₃ and extracted with EtOAc (2 x 200 mL). The combined organic solution was washed with brine (200 mL), dried over Na₂SO₄ and filtered, concentrated in vacuum to afford **II-H3** (8.6 g, 92%) as a solid, which was used as is.

**¹H NMR** (400 MHz, DMSO-d₆) δ_{H} 7.84 (s, 1H), 4.53 (t, *J* = 8.0 Hz, 1H), 3.04-2.97 (m, 1H), 2.40-2.27 (m, 2H), 2.21-2.03 (m, 6H), 1.85-1.57 (m, 4H), 1.51-1.06 (m, 8H), 0.93-0.74 (m, 5H).

### Synthesis of II-H4

A solution of **II-H3** (8.6 g, 25.8 mmol) in methanol (86 ml) and 5% aqueous NaOH (86 ml) was stirred at 100°C for 16 h under nitrogen. The reaction was cooled to rt, the pH adjusted to pH = 3 with HCl (2M) and extracted with EtOAc (100 mL). The organic solution was washed with brine (100 mL), dried over Na₂SO₄, filtered and concentrated in vacuum to give **II-H4** (8.2 g) as a solid, which was used as is.

**¹H NMR** (400 MHz, DMSO-d₆) δ_{H} 12.17 (s, 1H), 4.79-4.25 (m, 2H), 3.42 (s, 1H), 2.14-2.07 (m, 1H), 1.84-1.56 (m, 6H), 1.48-1.10 (m, 9H), 1.00-0.77 (m, 4H), 0.70-0.57 (m, 4H).

### Synthesis of II-H5

To a solution of **II-H4** (8.2 g, 26.5 mmol) in MeOH (150 mL) was added tris(acetyloxy)plumbyl acetate (23.5 g, 53 mmol) at 25°C. After stirring at 25°C for 16 h, the mixture was poured into water (400 mL) and extracted with ethyl acetate (2 x 100 mL). The combined organic solution was washed with brine (200 mL), dried over Na₂SO₄, filtered and concentrated in vacuum. The residue was purified by flash chromatography (10-25% EtOAc in PE) to give **II-H5** (3 g, 43%) as a solid.

**¹H NMR** (400 MHz, CDCl3) δ_{H} 3.67 (t, *J* = 8.0 Hz, 1H), 2.40-2.29 (m, 1H), 2.16-1.99 (m, 4H), 1.85-1.71 (m, 3H), 1.65-1.54 (m, 2H), 1.48-1.22 (m, 6H), 1.17-1.10 (m, 4H), 0.94-0.83 (m, 2H), 0.76 (s, 3H); **LC-ELSDE/MS** purity 99%, MS ESI calcd. for C₁₇H₂₇O₂ [M+H]⁺263.2, found 263.2.

### Synthesis of II-H6 & II-H7

To a solution of **II-H5** (3.3 g, 12.5 mmol) in THF (50 mL) was added dropwise MeMgBr (20.8 mL, 3 M in Et2O) at 0°C. After stirring at 25°C for 16 h, the mixture was poured into water (200 mL) and extracted with EtOAc (2 x 50 ml). The combined organic solution was washed with brine (100 mL), dried over Na₂SO₄ and concentrated in vacuum. The residue was purified by flash column (0-20% EtOAc in PE) to give **II-H6** (1.5 g) and **II-H7** (1.1 g) as solids. The absolute stereochemistry was determined after the next step.

**II-H6: ¹H NMR** (400 MHz, CDCl3) δ_{H} 3.65 (t, *J* = 8.0 Hz, 1H), 2.10-2.01 (m, 1H), 1.84-1.68 (m, 6H), 1.65-1.54 (m, 2H), 1.46-1.34 (m, 4H), 1.32-1.21 (m, 4H), 1.15 (s, 3H), 1.11-0.96 (m, 4H), 0.95-0.83 (m, 1H), 0.80-0.65 (m, 4H).

**II-H7: ¹H NMR** (400 MHz, CDCl3) δ_{H} 3.65 (t, *J* = 8Hz, 1H), 2.12-2.01 (m, 1H), 1.88-1.68 (m, 6H), 1.65-1.60 (m, 1H), 1.46-1.34 (m, 5H), 1.32-1.19 (m, 6H), 1.11-0.86 (m, 6H), 0.80-0.65 (m, 4H).

### Synthesis of II-H8

To a solution of **II-H6** (1.0 g, 3.59 mmol) in DCM (20 mL) was added DMP (3.04 g, 7.18 mmol). After stirring at 30°C for 30 min, the reaction mixture was quenched with saturated NaHCO₃ aqueous (50 mL) until pH ~ 9 and filtered. The DCM solution was separated, and the aqueous solution was extracted with DCM (50 mL). The combined organic solution was washed with saturated Na₂S₂O₃ aqueous (2 x 50mL), sat. NaHCO₃ (50 mL), brine (50 mL), dried over Na₂SO₄, filtered and concentrated. The residue was purified by flash column (0-30% EtOAc in PE) to give **II-H8** (650 mg, 66%) as a solid.

**¹H NMR** (400 MHz, CDCl3) δ_{H} 2.47-2.40 (m, 1H), 2.12-2.03 (m, 1H), 1.98-1.83 (m, 3H), 1.80-1.68 (m, 5H), 1.65-1.47 (m, 2H), 1.38-1.18 (m, 6H), 1.16 (s, 3H), 1.13-0.92 (m, 3H), 0.88 (s, 3H), 0.85-0.77 (m, 1H); **LC-ELSDE/MS** purity 99%, MS ESI calcd. for C₁₈H₂₇O [M-H₂O+ H]⁺259.1, found 259.1.

### Synthesis of II-H9

To a solution of PPh₃EtBr (4.00 g, 10.8 mmol) in THF (15 mL) was added t-BuOK (1.21 g, 10.8 mmol) at 25°C. After stirring at 50°C for 1 h, a solution **of II-H8** (600 mg, 2.17 mmol) in THF (5 mL) was added drop wise at 50°C. After stirring at 50°C for 16 h, the mixture was poured into saturated NH₄Cl (50 mL) and extracted with EtOAc (2 x 20 mL). The combined organic solution was washed with brine (50 mL), dried over Na₂SO₄ and filtered, concentrated in vacuum. The residue was purified by flash column (0~30% of EtOAc in PE) to give **II-H9** (410 mg, 66%) as a solid.

**¹H NMR** (400 MHz, DMSO-d₆) δ_{H} 5.14-5.08 (m, 1H), 2.42-2.30 (m, 1H), 2.23-2.12 (m, 2H), 1.82-1.61 (m, 11H), 1.33-0.98 (m, 13H), 0.88 (s, 3H), 0.81-0.72 (m, 1H).

### Synthesis of II-H10

To a solution of **II-H9** (410 mg, 1.42 mmol) in THF (10 mL) was added 9-BBN dimer (692 mg, 2.84 mmol). After stirring at 25°C for 5 h, ethanol (8 mL) at 15°C, followed by NaOH aqueous (2.83 mL, 5.0 M, 14.2 mmol) and finally hydrogen peroxide (1.41 mL, 10 M, 14.2 mmol) were added dropwise at 0°C. After stirring at 78°C for 1 h, the mixture was cooled, diluted with water (50 mL) and extracted with EtOAc (2 x 20 mL). The combined organic solution was washed with Na₂S₂SO₃ (2 x 50 mL) and brine (100 mL), dried over Na₂SO₄, filtered and concentrated in vacuum to give **II-H10** (390 mg) as an oil.

**¹H NMR** (400 MHz, CDCl3) δ_{H} 3.77-3.68 (m, 1H), 2.02-1.66 (m, 8H), 1.47-1.32 (m, 3H), 1.27-1.03 (m, 15H), 0.97-0.82 (m, 3H), 0.76-0.67 (m, 4H).

### Synthesis of II-H11

To a solution of **II-H10** (390 mg, 1.27 mmol) in DCM (10 mL) was added DMP (1.07 g, 2.54 mmol). After stirring at 30°C for 30 min, the reaction mixture was quenched with saturated NaHCO₃ aqueous (50 mL) until pH ~ 9 and filtered. The DCM solution was separated, and the aqueous solution was extracted with DCM (20 mL). The combined organic solution was washed with saturated Na₂S₂O₃ aqueous (2 x 20mL), sat. NaHCO₃ (40 mL), brine (40 mL), dried over Na₂SO₄, filtered and concentrated. The residue was purified by flash column (0-15% EtOAc in PE) to give **II-H11** (276 mg, 72%) as a solid.

**¹H NMR** (400 MHz, CDCl3) δ_{H} 2.57-2.52 (m, 1H), 2.22-2.13 (m, 1H), 2.11 (s, 3H), 2.00-1.94 (m, 1H), 1.84-1.61 (m, 8H), 1.49-1.37 (m, 2H), 1.34-1.18 (m, 5H), 1.15 (s, 3H), 1.13-0.77 (m, 5H), 0.62 (s, 3H); **LC-ELSDE/MS** purity 99%, MS ESI calcd. for C₂₀H₃₂O [M-H₂O +H]⁺287.2, found 287.2.

### EXAMPLE II-35: Synthesis of 1-(2-((1S,3aS,3bR,5aR,6R,8aR,8bS,10aS)-6-hydroxy-6,10a-dimethylhexadecahydrodicyclopenta[a,f]naphthalen-1-yl)-2-oxoethyl)-1H-pyrazole-4-carbonitrile (II-H13)

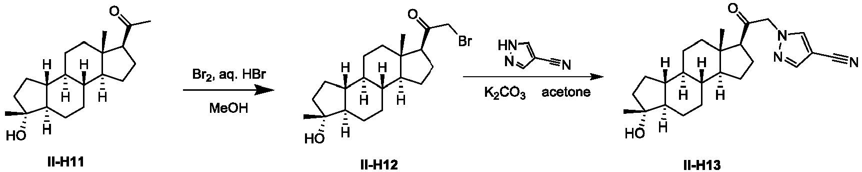

### Synthesis of II-H12

To a solution of **II-H11** (100 mg, 0.33 mmol) in MeOH (2 ml) was added HBr (0.01 mL, 0.33 mmol, 40% in water) and Br₂ (62.9 mg, 0.39 mmol) at 25°C. After stirring at 25°C for 2 h, the mixture was diluted with sat.aq NaHCO₃ (10 mL) and water (20 mL) and extracted with EtOAc (2 x 30 mL). The combined organic solution was washed with brine (30 mL), dried over anhydrous Na₂SO₄, filtered, concentrated in vacuum to afford **II-H12** (150 mg) as a solid used directly for the next step.

### Synthesis of II-H13

To a solution of **H12** (150 mg, 0.39 mmol) in acetone (3 mL) were added 1H-pyrazole-4-carbonitrile (43.6 mg, 0.47 mmol) and K₂CO₃(108 mg, 0.78 mmol). After stirring at 25°C for 14 h, the mixture was added water (20 mL) and extracted with EtOAc (2 x 30 mL). The combined organic solution was dried over Na₂SO₄, filtered and concentrated. The residue was purified by flash column (20~50% of EtOAc in PE) to give **II-H13** (34 mg, 22%) as a solid. **¹H NMR** (400 MHz, CDCl3) δ_{H} 7.85 (s, 1 H), 7.81 (s, 1 H), 5.06-4.86 (m, 2 H), 2.66-2.56 (m, 1 H), 2.26 - 2.13 (m, 1 H), 2.04-1.98 (m, 1 H), 1.84-1.66 (m, 8 H) 1.55-1.44 (m, 2 H), 1.37-1.17 (m, 6 H), 1.15 (s, 7 H), 0.68 (s, 3 H); **LC-ELSDE/MS** purity 99%, MS ESI calcd. for C₂₄H₃₄N₃O₂ [M+H]⁺396.2, found 396.2.

### EXAMPLE II-36: Synthesis of 1-((1S,3aS,3bR,5aR,6S,8aR,8bS,10aS)-6-hydroxy-6,10a-dimethylhexadecahydrodicyclopenta[a,f]naphthalen-1-yl)ethan-1-one (II-H17)

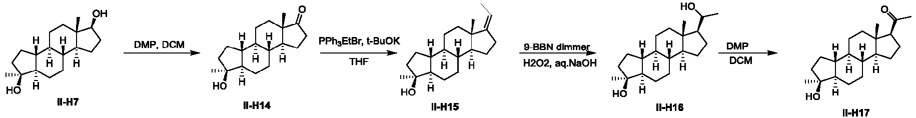

### Synthesis of II-H14

To a solution of **II-H7** (1.1 g, 3.95 mmol) in DCM (20 mL) was added DMP (3.34 g, 7.90 mmol). After stirring at 30°C for 30 min, the reaction mixture was quenched with saturated NaHCO₃ aqueous (50 mL) until pH ~ 9 and filtered. The DCM solution was separated, and the aqueous solution was extracted with DCM (20 mL). The combined organic solution was washed with saturated Na₂S₂O₃ aqueous (2 x 50mL), sat. NaHCO₃ (50 mL), brine (50 mL), dried over Na₂SO₄, filtered and concentrated. The residue was purified by flash column (0-30% EtOAc in PE) to give **II-H14** (850 mg, 78%) as a solid. The absolute structure was determined by NOE.

**¹H NMR** (400 MHz, CDCl3) δ_{H} 2.48-2.41 (m, 1H), 2.12-2.02 (m, 1H), 1.98-1.66 (m, 8H), 1.52-1.44 (m, 3H), 1.38-1.18 (m, 8H), 1.13-0.91 (m, 3H), 0.88 (s, 3H), 0.82-0.70 (m, 1H). **LC-ELSDE/MS** purity 99%, MS ESI calcd. for C₁₈H₂₇O [M-H₂O +H]⁺259.2, found 259.2.

### Synthesis of II-H15

To a solution of PPh₃EtBr (5.34 g, 14.4 mmol) in THF (15 mL) was added t-BuOK (1.61 g, 14.4 mmol) at 25°C. After stirring at 50°C for 1 h, a solution of **II-H14** (800 mg, 2.89 mmol) in THF (5 mL) was added dropwise at 50°C. After stirring at 50°C for 16 h, the mixture was poured into saturated NH₄Cl (50 mL) and extracted with EtOAc (2 x 20 mL). The combined organic solution was washed with brine (100 mL), dried over Na₂SO₄ and filtered, concentrated in vacuum. The residue was purified by flash column (0~30% of EtOAc in PE) to give **II-H15** (660 mg, 79%) as a solid.

**¹H NMR** (400 MHz, CDCl3) δ_{H} 5.15-5.09 (m, 1H), 2.40-2.33 (m, 1H), 2.24-2.13 (m, 2H), 1.89-1.70 (m, 5H), 1.68-1.62 (m, 5H), 1.55-1.38 (m, 3H), 1.32-1.16 (m, 8H), 1.13-1.02 (m, 3H), 0.99-0.92 (m, 1H), 0.90-0.83 (m, 4H), 0.78-0.69 (m, 1H).

### Synthesis of II-H16

A solution of **II-H15** (660 mg, 2.28 mmol) in THF (15 mL) was added 9-BBN dimer (1.11g, 4.56 mmol). After stirring at 25°C for 16 h, the resulting mixture was added ethanol (12 mL) at 15°C, followed by addition of NaOH aqueous (4.56 mL, 5.0 M, 22.8 mmol) at 0°C. Hydrogen peroxide (2.28 mL, 10 M, 22.8 mmol) was then added drop-wise at 0°C. After stirring at 78°C for 1 h, the mixture was cooled to rt, diluted with water (50 mL) and extracted with EtOAc (2 x 20 mL). The combined organic solution was washed with Na₂S₂O₃ (2 x 50 mL) and brine (80 mL), dried over Na₂SO₄, filtered and concentrated in vacuum. The residue was purified by flash column (0-15% of EtOAc in PE) to give **II-H16** (450 mg, 64.4%) as an oil.

**¹H NMR** (400 MHz, CDCl3) δ_{H} 3.77-3.68 (m, 1H), 2.02-1.66 (m, 8H), 1.47-1.32 (m, 3H), 1.27-1.03 (m, 15H), 0.97-0.82 (m, 3H), 0.76-0.67 (m, 4H).

### Synthesis of II-H17

To a solution of **II-H16** (450 mg, 1.46 mmol) in DCM (10 mL) was added DMP (1.23 g, 2.92 mmol). After stirring at 30°C for 30 min, the reaction mixture was quenched with saturated NaHCO₃ aqueous (50 mL) until pH ~ 9 and filtered. The DCM solution was separated, and the aqueous solution was extracted with DCM (20 mL). The combined organic solution was washed with saturated Na₂S₂O₃ aqueous (2 x 20mL), sat. NaHCO₃ (40 mL), brine (40 mL), dried over Na₂SO₄, filtered and concentrated. The residue was purified by flash column (0-15% EtOAc in PE) to give **II-H17** (261 mg, 58.7%) as a solid.

**¹H NMR** (400 MHz, CDCl3) δ_{H} 2.54 (t, *J* = 8Hz, 1H), 2.20-2.14 (m, 1H), 2.11 (s, 3H), 2.00-1.95 (m, 1H), 1.89-1.61 (m, 8H), 1.49-1.39 (m, 2H), 1.32-1.16 (m, 7H), 1.13-1.02 (m, 3H), 0.99-0.86 (m, 2H), 0.79-0.70 (m, 1H), 0.62 (s, 3H); **LC-ELSDE/MS** purity 99%, MS ESI calcd. for C₂₀H₃₂O [M-H₂O +H]⁺287.2, found 287.2.

### EXAMPLE II-37: Synthesis of 1-(2-((1S,3aS,3bR,5aR,6S,8aR,8bS,10aS)-6-hydroxy-6,10a-dimethylhexadecahydrodicyclopenta[a,f]naphthalen-1-yl)-2-oxoethyl)-1H-pyrazole-4-carbonitrile (II-H19)

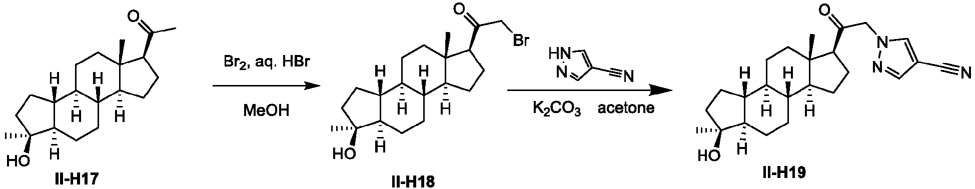

### Synthesis of II-H18

To a solution of **II-H17** (100 mg, 0.33 mmol) in MeOH (2 ml) was added HBr (0.01 mL, 0.33 mmol, 40% in water) and Br₂ (62.9 mg, 0.39 mmol) at 25°C. After stirring at 25°C for 2 h, the mixture was quenched by sat.aq NaHCO3 (10 mL), diluted with water (20 mL), and extracted with EtOAc (2 x 30 mL). The combined organic solution was washed with brine (30 mL), dried over anhydrous Na₂SO₄, filtered, concentrated in vacuum to afford **II-H18** (150 mg) as a solid, which used directly for the next step.

### Synthesis of II-H19

To a solution of **II-H18** (150 mg, 0.39 mmol) in acetone (3 mL) were added 1H-pyrazole-4-carbonitrilev (43.6 mg, 0.47 mmol) and K₂CO₃(108 mg, 0.78 mmol). After stirring at 25°C for 14 h, the mixture was diluted with water (20 mL) and extracted with EtOAc (2 x 30 mL). The organic solution was separated, dried over Na₂SO₄, filtered and concentrated. The residue was purified by flash column (20~50% of EtOAc in PE) to give **II-H19** (76 mg) as a solid. The solid was further purified by HPLC separation (column: Xtimate C18 150*25mm*5um, gradient: 67-87% B (water(0.225%FA)-ACN), flow rate: 25 mL/min) to give **II-H19** (43 mg) as a solid.

**¹H NMR** (400 MHz, CDCl3) δ_{H} 7.86 (s, 1 H), 7.81 (s, 1 H), 5.05-4.84 (m, 2 H), 2.66-2.57 (m, 1 H), 2.30-2.13 (m, 1 H), 2.10-2.05 (m, 1 H), 1.90-1.95 (m, 7H), 1.53-1.40 (m, 3H), 1.36-1.22 (m, 8 H), 1.14 - 0.80 (m, 4 H), 0.82 - 0.72 (m, 1 H), 0.68 (s, 3 H); **LC-ELSDE/MS** purity 99%, MS ESI calcd. for C₂₄H₃₃N₃O₂ [M+H]⁺396.2, found 396.2.

### EXAMPLE II-38: Synthesis of 1-((1S,3aS,3bR,5aR,7S,8aS,8bR,10aS)-7-hydroxy-7-(methoxymethyl)-10a-methylhexadecahydrodicyclopenta[a,f]naphthalen-1-yl)ethan-1-one (II-J9)

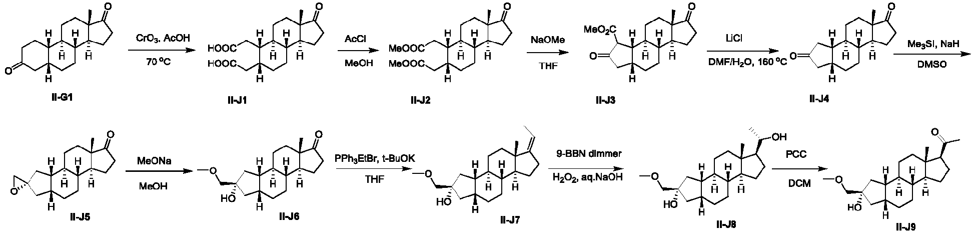

### Synthesis of II-J1

A solution of CrO₃ (143 g, 1.4 mol) in H₂SO₄ (98%, 200 mL) and water (960 mL) was added dropwise to a stirred solution **of II-G1** (99 g, 360 mmol) in HOAc (1000 mL) at 70°C. After stirring at 70°C for 16h, the mixture was added to crushed ice and water (1000 mL), stirred at 25°C for 1h and extracted with EtOAc (2 x 800 mL). The combined organic solution was washed with brine (2 x 600 mL), dried over anhydrous Na₂SO₄, filtered and concentrated to give **II-J1** (100 g) as an oil.

**¹H NMR** (400 MHz, CDCl₃) δ_{H} 2.94-2.76 (m, 1H), 2.66-2.10 (m, 7H), 2.09-1.74 (m, 5H), 1.73-1.17 (m, 9H), 1.00-0.88 (m, 4H).

### Synthesis of II-J2

To a suspension of **II-J1** (50 g, 155 mmol) in MeOH (500 mL) was added drop-wise AcCl (40 mL) at 25°C. After stirring at 25°C for 2h, the reaction mixture was poured to ice-cooled aqueous citric acid (400 mL) and extracted with EtOAc (2 x 300 mL). The combined organic solution was washed with NaHCO₃ (200 mL) and brine (2 x 200 mL), dried over anhydrous sodium sulfate, filtered and concentrated to give **II-J2** (45 g) as an oil. The material (purified together with another batch (50 g)) was purified by silica gel chromatography (PE/EtOAc=5/1) to afford **II-J2** (80 g, 74%) as an oil.

### Synthesis of II-J3

Fresh Na (1.63 g, 70.9 mmol) was carefully added to MeOH (20 mL) in portions. After stirring at 25°C for 3h, a solution of **II-J2** (5 g, 14.2 mmol) in dry THF (20 mL) was added. After stirring at 100°C for 1h under N₂, the mixture was cooled to rt, 1M HCl (77 mL) was slowly added and then extracted with CH₂Cl₂ (2 x 50 ml). The combined organic solution was washed with brine (70 mL), dried over anhydrous Na₂SO₄, filtered and concentrated under reduced pressure. The residue was purified by silica gel chromatography (PE/EtOAc=5/1) to afford **II-J3** (1.74 g, 39%) as a solid.

**¹H NMR** (400 MHz, CDCl₃) δ_{H} 3.80-3.65 (m, 3H), 3.31-3.10 (m, 1H), 3.00-2.75 (m, 1H), 2.59-2.34 (m, 2H), 2.34-2.21 (m, 2H), 2.17-1.92 (m, 3H), 1.86-1.67 (m, 4H), 1.36-0.99 (m, 6H), 0.90 (s, 3H), 0.82-0.57 (m, 1H).

### Synthesis of II-J4

To a solution of **II-J3** (1.7 g, 5.5 mmol) in DMF (15 mL) and H₂O (1 mL) was added LiCl (924 mg, 21.8 mmol) at 25°C. After stirring at 160°C and for 35 min, the reaction mixture was poured to ice-cooled aqueous citric acid (35 mL) and extracted with DCM (2 x 20 mL). The combined organic solution was washed with brine (2 x 20 mL), dried over anhydrous sodium sulfate, filtered and concentrated to give **II-J4** (1.3 g) as a solid.

**¹H NMR** (400 MHz, CDCl₃) δ_{H} 2.60-2.40 (m, 2H), 2.29 (d, *J* = 4.4 Hz, 2H), 2.21-2.03 (m, 3H), 2.01-1.91 (m, 2H), 1.88-1.63 (m, 5H), 1.56-1.48 (m, 1H), 1.33-1.23 (m, 3H), 1.20-1.05 (m, 2H), 0.90 (s, 3H), 0.80-0.65 (m, 1H).

### Synthesis of II-J5

To a stirred solution of trimethylsulfonium iodide (1.56 g, 7.7 mmol) in DMSO (10 mL) and THF (5 mL) was added NaH (306 mg, 7.7 mmol, 60 % in oil). After stirring at 0°C for 1.0 h under N₂. a solution of **II-J4** (2 g, 7.7 mmol) in DMSO (1 mL) was added at 0°C. After stirring at 25 °C for 16 h, the reaction was diluted with water (5 mL) and extracted with EtOAc (2 x 3 mL). The combined organic solution was washed with water (2 x 3 mL) and brine (3 mL), dried over anhydrous Na₂SO₄, filtered and concentrated in vacuum to give **II-J5** (2.1 g, 7.7 mmol) as an oil, which was used directly for the next step.

### Synthesis of II-J6

Fresh Na (1.67 g, 72.8 mmol) was carefully added to MeOH (20 mL) in portions. After stirring at 25 °C for 3h, a solution **of II-J5** (2 g, 7.3 mmol) in anhydrous MeOH (15 mL) was added. After stirring at 75°C for 16 h, the reaction was diluted with water (50 mL), concentrated to remove most of the solvent, and extracted with EtOAc (2 x 30 mL). The combined organic solution was washed with saturated brine (50 mL), dried over anhydrous Na₂SO₄, filtered and concentrated. The residue was purified by silica gel chromatography (20% EtOAc in PE) to afford **II-J6** (600 mg, 33%) as a solid.

**¹H NMR** (400 MHz, CDCl₃) δ_{H} 3.45-3.18 (m, 5H), 2.44 (dd, *J* = 8.8, 19.2 Hz, 2H), 2.10-1.66 (m, 7H), 1.45-0.95 (m, 11H), 0.92-0.80 (m, 5H). The structure was confirmed by NOE.

### Synthesis of II-J7

To a suspension of bromo (ethyl) triphenylphosphorane (3.6 g, 9.8 mmol) in THF (800 mL) was added t-BuOK (1.1 g, 9.8 mmol) at 25°C under N₂. After stirring at 50°C for 1h, a solution of **II-J6** (600 mg, 2.0 mmol) in THF (100 mL) was added. After stirring at 50 °C for 16 h, the mixture was treated with NH₄Cl (300 mL) and extracted with EtOAc (2 x 100 mL). The combined organic solution was washed with brine (2 x 100 mL), dried over anhydrous Na₂SO₄, filtered, concentrated in vacuum. The residue was purified by silica gel chromatography (20% EtOAc in PE) to afford **II-J7** (300 mg, 50%) as a solid.

**¹H NMR** (400 MHz, CDCl₃) δ_{H} 5.20-5.05 (m, 1H), 3.41 (s, 3H), 3.35-3.18 (m, 2H), 2.54-2.28 (m, 2H), 2.26-2.11 (m, 2H), 2.08-1.92 (m, 1H), 1.92-1.60 (m, 10H), 1.52-1.48 (m, 1H), 1.30-1.00 (m, 8H), 0.93-0.84 (m, 4H)

### Synthesis of II-J8

9-BBN (458 mg, 1.9 mmol) was added to a solution of **II-J7** (300 mg, 0.9 mmol) in THF (100 mL) at 20°C. After stirring at 50°C for 16 h, ethanol (433 mg, 9.4 mmol) was added at 25°C, followed by aqueous NaOH solution (1.9 mL, 5.0 M, 9.4 mmol) at 0°C. Hydrogen peroxide (0.9 mL, 10 M, 9.4 mmol) was then added dropwise at 0°C. After stirring at 78°C for 1 h, the mixture was cooled to 25°C. The mixture was filtered, and the precipitate was washed with water (3 x 50 mL). The residue was purified by silica gel chromatography (10% of EtOAc in PE) to afford **II-J8** (220 mg, 74%) as a solid.

**¹H NMR** (400 MHz, CDCl₃) δ_{H} 3.76-3.63 (m, 1H), 3.40 (s, 3H), 3.24 (dd, *J* = 8.8, 26.0 Hz, 2H), 2.48 (s, 1H), 2.11-1.98 (m, 1H), 1.95-1.60 (m, 9H), 1.50-0.85 (m, 16H), 0.67 (s, 3H); **LC-ELSD/MS** purity≥99%, MS ESI calcd. for C₂₁H₃₃O [M-2H2O+H]⁺ 301.3, found 301.3.

### Synthesis of II-J9

To a solution of **II-J8** (200 mg, 0.6 mmol) in DCM (20 mL) were added PCC (253 mg, 1.2 mmol) and silica gel (0.5 g). After stirring at 25°C for 2 h, the reaction was filtered and the filtrate concentrated. The residue was purified by silica gel chromatography (0-20% of EtOAc in PE) to give **II-J9** (150 mg, 76%) as a solid.

**¹H NMR** (400 MHz, CDCl₃) δ_{H} 3.40 (s, 3H), 3.25 (dd, *J* = 8.8, 25.6 Hz, 2H), 2.60-2.45 (m, 2H), 2.20-1.90 (m, 6H), 1.85-1.60 (m, 9H), 1.55-1.40 (m, 3H), 1.30-0.95 (m, 6H), 0.62 (s, 3H); **LC-ELSD/MS** purity≥99%, MS ESI calcd. for C₂₁H₃₃O₂ [M-H₂O+H]⁺ 317.3, found 317.2.

### EXAMPLE II-39: Synthesis of 1-(2-((1S,3aS,3bR,5aR,7S,8aS,8bR,10aS)-7-hydroxy-7-(methoxymethyl)-10a-methylhexadecahydrodicyclopenta[a,f]naphthalen-1-yl)-2-oxoethyl)-1H-pyrazole-4-carbonitrile (II-J11)

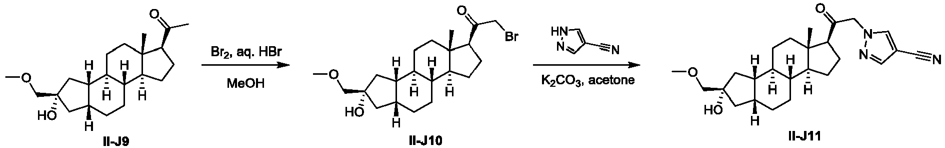

### Synthesis of II-J10

To a solution of **II-J9** (60 mg, 0.2 mmol) in MeOH (5 mL) were added HBr (7.2 mg, 0.1 mmol) and Br₂ (28.6 mg, 0.2 mmol) at 25°C. After stirring at 25°C for 2 h, the reaction mixture was added into saturated NaHCO₃ (10 mL) and extracted with EtOAc (3 x 10 mL). The combined organic solution was washed with saturated brine (20 mL), dried over anhydrous Na₂SO₄, filtered and concentrated to give **II-J10** (74 mg) as an oil, which was used directly for the next step without further purification.

**¹H NMR**(400 MHz, CDCl₃) δ_{H} 3.95-3.80 (m, 2H), 3.40 (s, 3H), 3.25 (dd, *J* = 8.8, 25.2 Hz, 2H), 2.90-2.75 (m, 1H), 2.51 (s, 1H), 2.25-2.10 (m, 1H), 1.90-1.75 (m, 3H), 1.70-1.40 (m, 10H), 1.20-0.75 (m, 7H), 0.65 (s, 3H).

### Synthesis of II-J11

To a solution of **II-J10** (74 mg, 0.2 mmol) and 1H-pyrazole-4-carbonitrile (33.3 mg, 0.4 mmol) in acetone (5 mL) was added K₂CO₃ (49.4 mg, 0.4 mmol). After stirring at 25°C for 16 h, the reaction mixture was extracted with EtOAc (3 x 30 mL). The combined organic solution was washed with saturated brine (30 mL), dried over anhydrous Na₂SO₄, filtered and concentrated to give the product. The residue was purified by Prep-HPLC (Column: Xtimate C18 150*25mm*5µm; Condition: water (10mM NH₄HCO₃)-ACN; Begin B: 50; End B: 80; Gradient Time (min): 10; 100%B Hold Time (min): 0) to give **II-J11** (15 mg, 20%) as a solid.

**¹H NMR** (400 MHz, CDCl₃) δ_{H} 7.85 (s, 1H), 7.81 (s, 1H), 4.95 (dd, *J* = 18.0, 48.8 Hz, 2H), 3.41 (s, 3H), 3.26 (dd, *J* = 8.8, 25.2 Hz, 2H), 2.65-2.45 (m, 2H), 2.30-2.15 (m, 1H), 2.10-1.95 (m, 2H), 1.90-1.75 (m, 4H), 1.70-1.40 (m, 8H), 1.35-0.95 (m, 6H), 0.68 (s, 3H); **LC-ELSD/MS** purity≥99%, MS ESI calcd. for C₂₅H₃₆N₃O₃ [M+H]⁺ 426.3, found 426.3.

### EXAMPLE II-40 & II-41: Synthesis of 1-((1S,3aS,3bR,5aR,7S,8aS,8bR,10aS)-7-hydroxy-7,10a-dimethylhexadecahydrodicyclopenta[a,f]naphthalen-1-yl)-2-(2H-1,2,3-triazol-2-yl)ethan-1-one (II-G11) & 1-((1S,3aS,3bR,5aR,7S,8aS,8bR,10aS)-7-hydroxy-7,10a-dimethylhexadecahydrodicyclopenta[a,f]naphthalen-1-yl)-2-(1H-1,2,3-triazol-1-yl)ethan-1-one (II-G12)

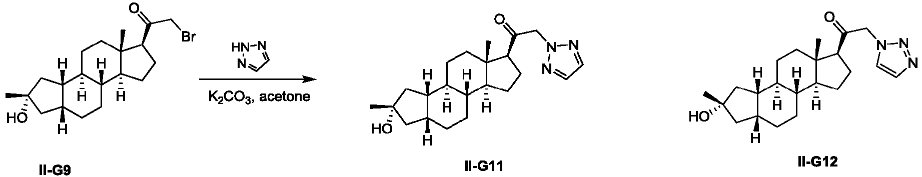

To a solution of **II-G9** (113 mg, 0.3 mmol) and 1H-pyrazole-4-carbonitrile (40.7 mg, 0.6 mmol) in acetone (3 mL) was added K₂CO₃ (81.3 mg, 0.6 mmol). After stirring at 20 °C for 16 h, the reaction mixture was extracted with EtOAc (3 x 30 mL). The combined organic layer was washed with saturated brine (30 mL), dried over anhydrous Na₂SO₄, filtered and concentrated (100 mg). The residue was purified by pre-HPLC (Column: Waters Xbridge 150*25 5µm; Condition: water (10mM NH₄HCO₃)-ACN; Begin B: 45%; End 75%) to give **II-G11** (10 mg) and **II-G12** (5.2 mg, 5%) as solids.

**II-G11: ¹H NMR** (400 MHz, CDCl₃) δ_{H} 7.68 (s, 2H), 5.34-5.14 (m, 2H), 2.59 (t, *J* = 9.2 Hz, 1H), 2.27-2.02 (m, 3H), 1.98-1.64 (m, 8H), 1.38-0.80 (m, 14H), 0.72 (s, 3H); **LC-ELSD/MS** purity≥99%, 100% de based on H-NMR; MS ESI calcd. for C₂₂H₃₄N₃O₂ [M+H]⁺ 372.3, found 372.3

**II-G12: ¹H NMR** (400 MHz, CDCl₃) δ_{H} 7.76 (s, 1H), 7.64 (s, 1H), 5.20 (dd, *J* = 18.0, 54.8 Hz, 2H), 2.65 (t, *J* = 8.8 Hz, 1H), 2.28-2.03 (m, 3H), 1.96-1.59 (m, 9H), 1.51-0.94 (m, 13H), 0.69 (s, 3H); **LC-ELSD/MS** purity≥99%, 100% de based on H-NMR; MS ESI calcd. for C₂₂H₃₄N₃O₂ [M+H]⁺ 372.3, found 372.3

### EXAMPLE II-42: Biological Data

In **Table II-2** below, A indicates a TBPS IC₅₀ (µM) < 0.01 µM, B indicates a TBPS IC₅₀ (µM) of 0.01 µM to < 0.1 µM, C indicates a TBPS IC₅₀ (µM) of 0.1 µM to < 1.0 µM, D indicates a TBPS IC₅₀ (µM) of 1.0 µM to < 10 µM, and E means ≥ 10 µM.

**Table II-2**

| **Example** | **Compound ID** | **Structure** | **IC₅₀ (µm)** |
|---|---|---|---|
| **II-1** | **II-C2a** | 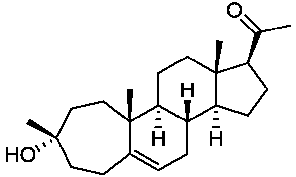 | B |
| **II-2** | **II-C2b** | 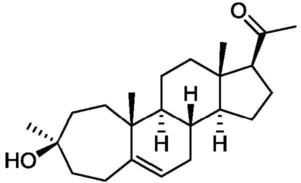 | D |
| **II-3** | **II-C3** | 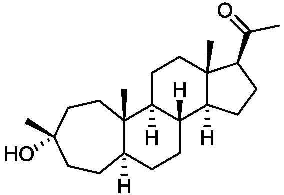 | B |
| **II-4** | **II-C5** | 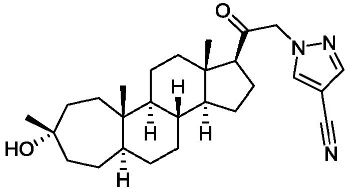 | B |
| **II-6** | **II-B5** | 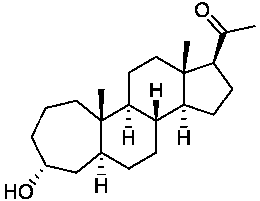 | C |
| **II-7** | **II-C6** | 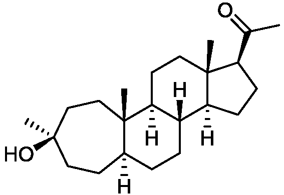 | |
| **II-8** | **II-C8** | 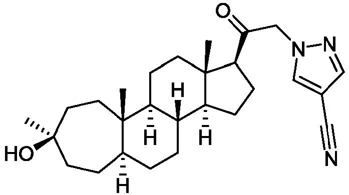 | C |
| **II-9** | **II-D2a** | 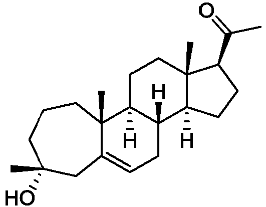 | D |
| **II-10** | **II-D2b** | 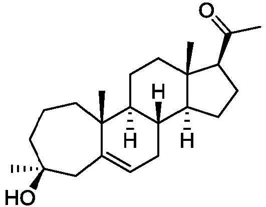 | |
| **II-11** | **II-D3b** | 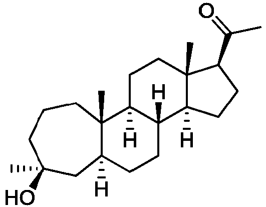 | |
| **II-12** | **II-D3a** | 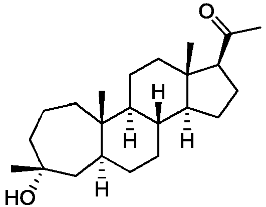 | D |
| **II-13** | **II-D5a** | 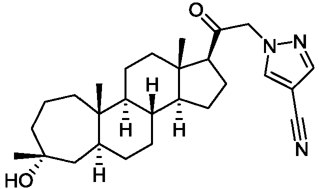 | B |
| **II-14** | **II-D5b** | 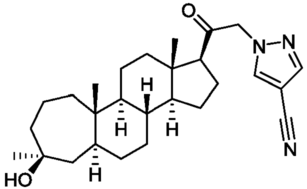 | C |
| **II-15** | **II-E7a** | 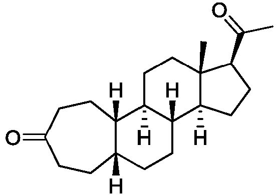 | D |
| **II-16** | **II-E7b** | 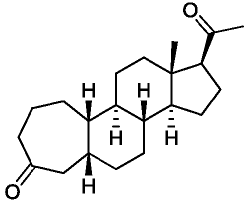 | D |
| **II-17** | **II-E8** | 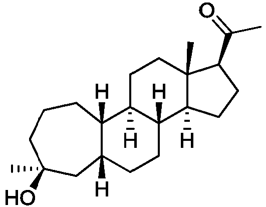 | |
| **II-17a** | **II-E8a** | 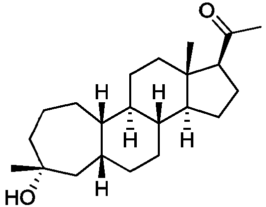 | B |
| **II-18** | **II-E10** | 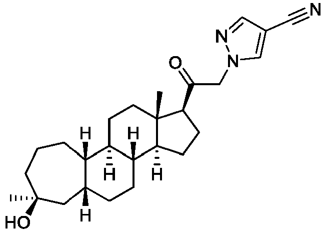 | C |
| **II-18a** | **II-E10a** | 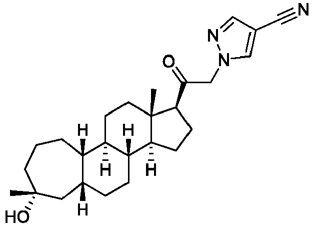 | B |
| **II-19** | **II-C10** | 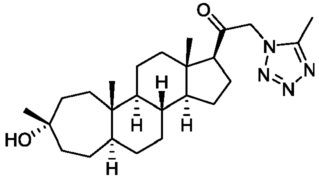 | C |
| **II-20** | **II-C11** | 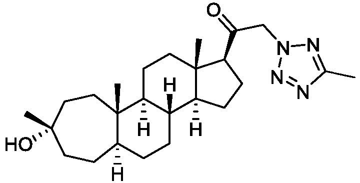 | B |
| **II-21** | **II-C12** | 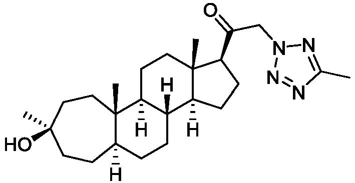 | |
| **II-22** | **II-F3** | 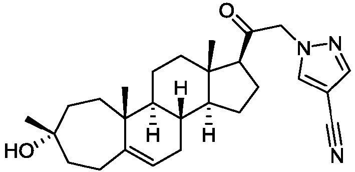 | A |
| **II-23** | **II-F6** | 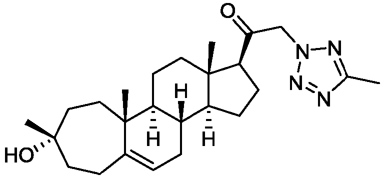 | B |
| **II-24** | **II-F7** | 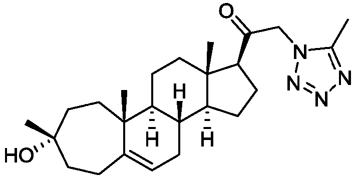 | B |
| **II-25** | **II-F8** | 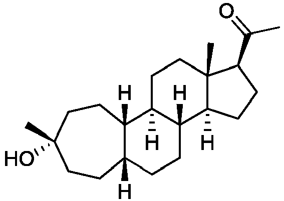 | C |
| **II-26** | **II-F9** | 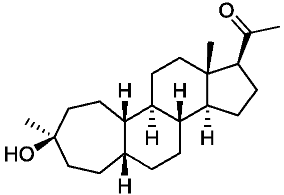 | |
| **II-27** | **II-F11** | 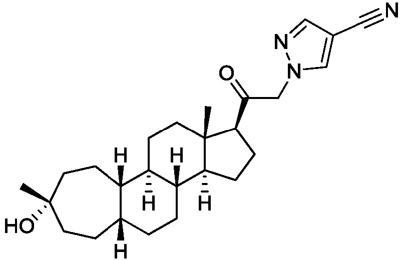 | C |
| **II-28** | **II-F12** | 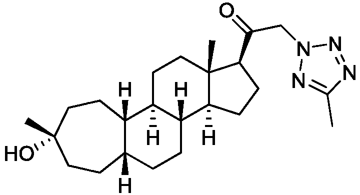 | C |
| **II-29** | **II-F13** | 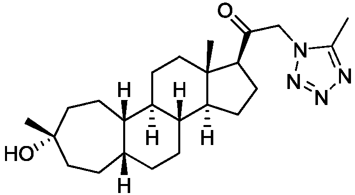 | D |
| **II-30** | **II-F19** | 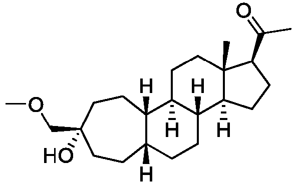 | C |
| **II-31** | **II-F21** | 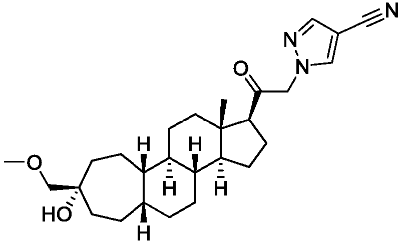 | C |
| **II-32** | **II-G8** | 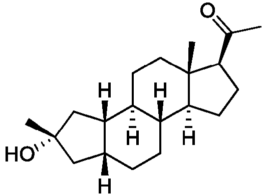 | C |
| **II-33** | **II-G10** | 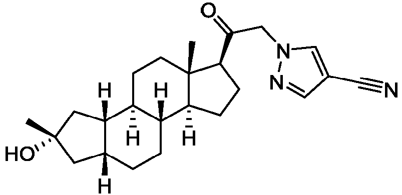 | C |
| **II-34** | **II-H11** | 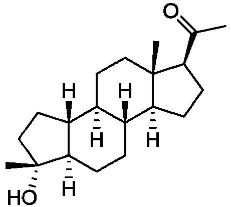 | D |
| **II-35** | **II-H13** | 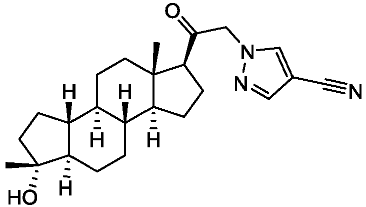 | D |
| **II-36** | **II-H17** | 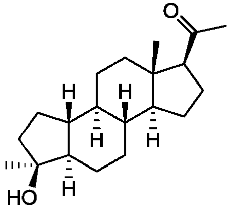 | |
| **II-37** | **II-H19** | 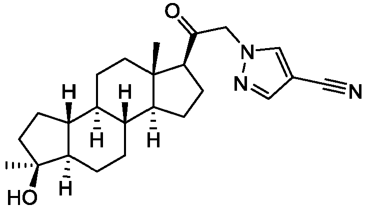 | D |
| **II-38** | **II-J9** | 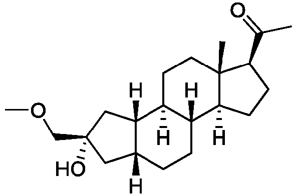 | C |
| **II-39** | **II-J11** | 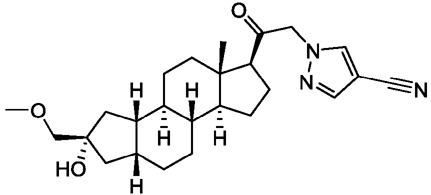 | C |
| **II-40** | **II-G11** | 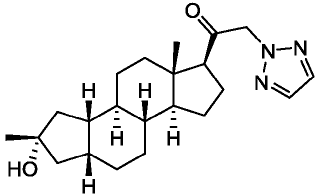 | C |
| **II-41** | **II-G12** | 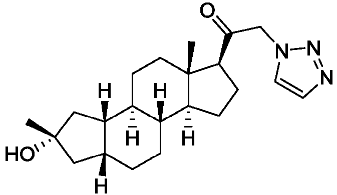 | C |

### EXAMPLE III-1: Synthesis of (3aS,3bR,6aS,8R,10aS,10bR,12aS)-8-hydroxy-8,12a-dimethylhexadecahydrobenzo[3,4]cyclohepta[1,2-e]inden-1(2H)-one (III-A10)

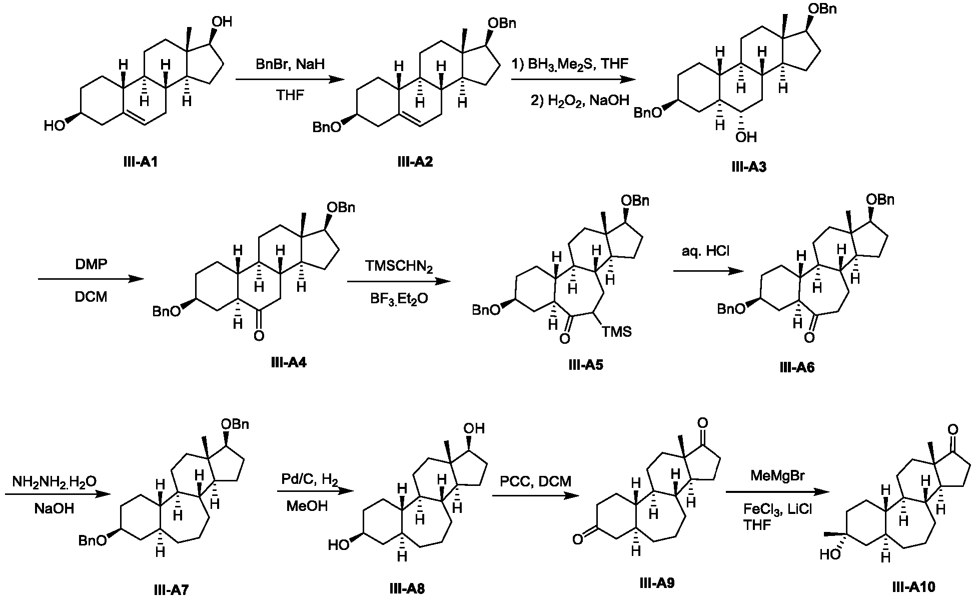

### Synthesis of III-A2

To a suspension of **III-A1** (70 g, 0.25 mol, reported in reference 'Tetrahedron vol. 62 (2006) p. 4384-4392') in THF (1.5 L) was added NaH (40.3 g, 60%, 1.0 mol) and BnBr (173 g, 1.0 mol) at 20°C. The mixture was stirred at 40°C for 40 h. The mixture was quenched by aq. NH₄Cl (200 mL). The mixture was concentrated to remove THF. The residue was extracted with PE/EtOAc (10:1, 500 mL). The organic layer was separated, dried over Na₂SO₄, filtered and concentrated in vacuum (170 g). The material was purified by flash column (0~10% EtOAc in PE) to give **III-A2** as a solid.

### Synthesis of III-A3

To a solution of **III-A2** (65 g, 0.14 mol) in THF (650 mL) was added BH₃.Me₂S (42.6 mL, 10 M, 0.42 mol) and the mixture was stirred at 15°C for 16 h. To the mixture was added EtOH (65.4 g, 1.42mol) dropwise followed by NaOH (283 mL, 5 M) and H₂O₂ (141 mL, 10 M). The mixture was stirred at 60°C for 5 h. The mixture was quenched by Na₂SO₃ (2 L, 10%) and extracted with EtOAc (1 L). The organic layer was separated, dried Na₂SO₄, filtered and concentrated in vacuum to give **III-A3** as a solid.

### Synthesis of III-A4

To a solution of **III-A3** (180 g, 379 mmol) in DCM (2 L) was added DMP (240 g, 568 mmol) and water (6.59 g, 365 mmol). The mixture was stirred at 20°C for 0.5h. The mixture was quenched by aq. NaHCO₃ (500 mL) and aq.Na₂S₂O₃ (500 mL). The organic layer was separated, dried over Na₂SO₄, filtered, concentrated in vacuum, and purified by flash column (0~30% EtOAc in PE) to give **III-A4** as a solid.

**¹H NMR** (400 MHz, CDCl₃) δ_{H} 7.45-7.20 (m, 10H), 4.59-4.37 (m, 4H), 3.40-3.30 (m, 1H), 3.28-3.20 (m, 1H), 2.45-2.20 (m, 2H), 2.11-1.83 (m, 6H), 1.55-1.37 (m, 3H), 1.25-0.70 (m, 9H), 0.75 (s, 3H)

### Synthesis of III-A5

A solution of TMSCHN₂ in hexane (31.6 mL,63.3 mmol,2M in hexane) was added dropwise at -20°C to a stirred solution of **III-A4** (10 g,21.1 mmol) and BF₃·Et₂O (13.2 mL, 105 mmol) in dry CH₂Cl₂ (100 mL). The mixture was stirred at -15°C for 3 h under N₂. The reaction mixture was poured into ice-water (100 mL) and extracted with DCM (2 x 100 mL). The combined organic phase was washed with brine (100 mL), dried over Na₂SO₄, and evaporated to give **III-A5** (12 g) as an oil.

### Synthesis of III-A6

To the solution of **III-A5** (12 g, 21.4 mmol) in THF (80 mL) was added 2N HCl (8 mL). The mixture was stirred at 20 °C for 3 h. The mixture was quenched by NaHCO₃ (50 mL, saturated) and Na₂S₂O₃ (50 mL, saturated.). The aqueous phase was extracted with EtOAc (2 x 100 mL). The combined organic phase was washed with water (2 x 100 mL), dried over anhydrous Na₂SO₄, filtered and concentrated and was purified by flash column (5~10% of EtOAc in PE) to give **III-A6** (5 g, 48.0%) as an oil.

**¹H NMR** (400 MHz, CDCl₃) δ_{H} 7.32-7.01 (m, 10H), 4.64-4.46 (m, 4H), 3.46-3.28 (m, 2H), 2.52-2.38 (m, 2H), 2.23-1.85 (m, 6H), 1.83-1.50 (m, 5H), 1.45-0.90 (m, 10H), 0.88-0.75 (m, 3H).

### Synthesis of III-A7

To the mixture of **III-A6** (5.0 g, 10.2 mmol) in diethylene glycol (90 mL) was added hydrazine hydrate (5.05 g, 101 mmol) and the mixture was heated to 120 °C for 2 hours. The mixture was cooled to 75 °C. Potassium hydroxide (11.3 g, 203 mmol) was added to the mixture and heated to 200 °C for 20 hours. The mixture was cooled to room temperature, poured into water (500 mL), and adjusted to pH = 6 with 2N hydrochloric acid. The aqueous phase was extracted with DCM (3 x 300 mL). The combined organic phase was washed with water (2 x 200 mL), dried over anhydrous Na₂SO₄, filtered and concentrated and was purified by flash column (0~5% of EtOAc in PE) to give **III-A7** (2.7 g, 56.0%) as an oil.

**¹H NMR** (400 MHz, CDCl₃) δ_{H} 7.35-7.10 (m, 10H), 4.57-4.39 (m, 4H), 3.40-3.16 (m, 2H), 2.14-1.71 (m, 7H), 1.65-1.25 (m, 10H), 1.24-1.85 (m, 9H), 0.83-0.77 (m, 3H), 0.76-0.57 (m, 2H)

### Synthesis of III-A8

To a mixture of **III-A7** (2.7 g, 5.71 mmol) in MeOH (50 mL) was added Pd/C (wet, 537 mg, 10%). The mixture was degassed under vacuum and purged with H₂ for three times. The mixture was stirred under H₂ (103421 Pa = 15 psi) at 20°C for 16 hours. The mixture was filtered through a pad of celite and concentrated to afford **III-A8** (1.3 g, 78.3%) as a solid. A portion (100 mg, 0.3 mmol) was purified by HPLC separation to give **III-A8** (32 mg, 3.45%) as a solid.

**¹H NMR** (400 MHz, CDCl₃) δ_{H} 3.71-3.50 (m, 2H), 2.02-1.92 (m, 3H), 1.90-1.79 (m, 2H), 1.77-1.65 (m, 3H), 1.51-1.26 (m, 10H), 1.25-0.86 (m, 8H), 0.75 (s, 3H), 0.73-0.68 (m, 1H); **LC-ELSD/MS** purity 99%, **MS ESI** calcd. for C₁₉H₂₉ [M-2H₂O+H]+257, found 257

### Synthesis of III-A9

To a mixture of **III-A8** (1.3 g, 4.44 mmol) in DCM (50 mL) was added silica gel (5.32 g) and PCC (4.77 g, 22.2 mmol) at 0°C. The reaction mixture was stirred at 20 °C for 3 hrs. Then, the mixture was filtered and the filter pad was washed with DCM (2 x 100 ml). The combined solvents were evaporated. The residue was purified by flash column (15-35% of EtOAc in PE) to give **III-A9** (1.2 g, 93.7%) as an oil.

**¹H NMR** (400 MHz, CDCl₃) δ_{H} 2.47-2.21 (m, 6H), 2.21-2.06 (m, 2H), 2.00-1.68 (m, 7H), 1.67-1.51 (m, 5H), 1.45-1.28 (m, 5H), 0.92-0.84 (m, 4H)

### Synthesis of III-A10

A suspension of LiCl (801 mg, 18.9 mmol, anhydrous) in THF (40 mL, anhydrous) was stirred at 10°C for 30 min under N₂. FeCl₃ (1.6 g, 9.9 mmol, anhydrous) was added to the mixture at 10°C. The mixture was cooled to -30°C. To the mixture was added MeMgBr (12.0 mL, 3M in THF) dropwise at -30°C. The mixture was stirred at -30°C for 30 min. **III-A9** (1.3 g, 4.5 mmol) was added to the mixture at -30°C. The mixture was stirred at -15°C for 2 h. To the mixture was added sat. citric acid (50 mL). The mixture was extracted with EtOAc (2 x 100 mL). The combined organic phase was washed with brine (2 x 50 mL), dried over Na₂SO₄, filtered, concentrated to give **III-A10** (1.1 g, 80.2%) as an oil. **III-A10** (100 mg, 0.3 mmol) was purified by HPLC separation, flow rate: 25 mL/min) to give **III-A10** (38 mg) as a solid.

**¹H NMR** (400 MHz, CDCl₃) δ_{H} 2.55-2.37 (m, 1H), 2.15-1.92 (m, 3H), 1.86-1.70 (m, 3H), 1.66-1.58 (m, 3H), 1.55-1.35 (m, 6H), 1.34-1.15 (m, 11H), 0.89 (s, 3H), 0.85-0.70 (m, 2H); **LC-ELSD/MS** purity 99%; **MS ESI** calcd. for C₂₀H₃₁O [M-H₂O+H]⁺ 287.2, found 287.2.

### EXAMPLE III-2: Synthesis of 1-((1S,3aS,3bR,6aS,8R,10aS,10bR,12aS)-8-hydroxy-8,12a-dimethyloctadecahydrobenzo[3,4]cyclohepta[1,2-e]inden-1-yl)ethan-1-one (III-A13)

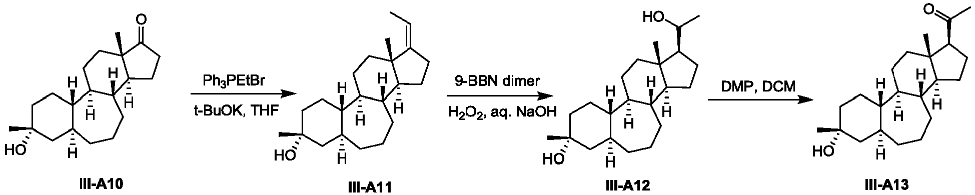

### Synthesis of III-A11

To a mixture of EtPPh₃Br (3.65 g, 9.8 mmol) in THF (15 mL) was added t-BuOK (1.10 g, 9.8 mmol) at 25°C under N₂. The resulting mixture was stirred at 40°C for 30 min. **III-A10** (1 g, 3.28 mmol) was added at 40°C. The reaction mixture was stirred at 40°C for 3 hour to give a suspension. The reaction mixture was quenched with saturated NH₄Cl aqueous (30 mL) at 20°C. The aqueous was extracted with EtOAc (2 x 50 mL). The combined organic phase was concentrated. The residue was purified by flash column (0~20% of EtOAc in PE) to give **III-A11** (810 mg, 78.6%) as a solid.

**¹H NMR** (400 MHz, CDCl₃) δ_{H} 5.15-4.96 (m, 1H), 2.40-2.26 (m, 1H), 2.21-2.09 (m, 2H), 1.94-1.79 (m, 2H), 1.76-1.50 (m, 11H), 1.48-1.27 (m, 8H), 1.21-1.11 (m, 6H), 0.88 (s, 3H), 0.85-0.65 (m, 2H).

### Synthesis of III-A12

To a solution of **III-A11** (770 mg, 2.4 mmol) in anhydrous THF (10 mL) was added 9-BBN dimer (1.18 g,4.9 mmol) at 25°C under N₂. After the reaction mixture was stirring at 25°C for 1 h. Then, the mixture was cooled, quenched by EtOH (1.4 mL, 24.3 mmol) at 0°C. NaOH (4.86 mL, 5M, 24.3 mmol) was added very slowly. After addition, H₂O₂ (2.43 mL, 24.3 mmol, 30% in water) was added slowly until the inner temperature no longer rises and the inner temperature was maintained below 30°C. After stirring at 50°C for another 1 h and the mixture was cooled. The mixture was poured into water (50 mL). The aqueous phase was extracted with EtOAc (3 x 50 mL). The combined organic phase was washed with saturated brine (2 x 50 mL), dried over anhydrous Na₂SO₄, filtered and concentrated. The residue was purified by flash column (0~30% of EtOAc in PE) to give **III-A12** (650 mg, 80.0%)} as a solid.

**¹H NMR** (400 MHz, CDCl₃) δ_{H} 3.55-3.52 (m, 1H), 1.95-1.45 (m, 5H), 1.70-1.38 (m, 12H), 1.36-1.25 (m, 7H), 1.17-1.07 (m, 4H), 0.82-0.74 (m, 1H), 0.70-0.63 (m, 3H).

### Synthesis of III-A13

To a solution of **III-A12** (600 mg, 1.8 mmol) in DCM (20 mL) was added DMP (1.51 g, 3.6 mmol). The mixture was stirred at 20°C for 0.5 hrs. The mixture was quenched by NaHCO₃ (50 mL, saturated) and Na₂S₂O₃ (50 mL, saturated). The organic layer was separated, dried over Na₂SO₄, filtered and concentrated and purified by flash column (0~30% EtOAc in PE) to give **III-A13** (240 mg, 40.3%) as a solid.

**¹H NMR** (400 MHz, CDCl₃) δ_{H} 2.53 (t, 1H), 2.19-2.07 (m, 4H), 1.97-1.79 (m, 3H), 1.69-1.52 (m, 14H), 1.47-1.25 (m, 9H), 0.89-0.66 (m, 2H), 0.63 (s, 3H); **LC-ELSD/MS** purity 99%, **MS ESI** calcd. for C₂₂H₃₅O [M-H₂O+H]⁺ 315.2, found 315.2

### EXAMPLE III-3: Synthesis of 1-(2-((1S,3aS,3bR,6aS,8R,10aS,10bR,12aS)-8-hydroxy-8,12a-dimethyloctadecahydrobenzo[3,4]cyclohepta[1,2-e]inden-1-yl)-2-oxoethyl)-1H-pyrazole-4-carbonitrile (III-A15)

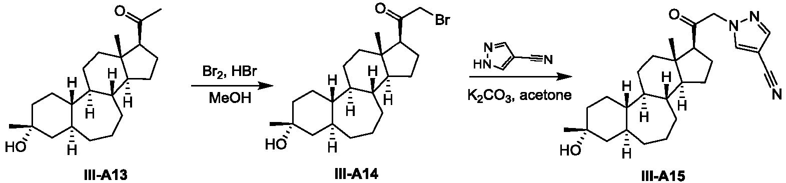

### Synthesis of III-A14

To a solution of **III-A13** (100 mg, 0.3 mmol) in MeOH (3 mL) was added HBr (12.1 mg, 0.1 mmol, 40% in water) and Br₂ (48.0 mg, 0.3 mmol) at 25°C. The mixture was stirred at 25°C for 2 hrs. The mixture was poured into NaHCO₃ (10 mL, saturated), extracted with EtOAc (2 x 10 mL). The organic layer was separated, dired over Na₂SO₄, filtered and concetrated in vacuum to give **III-A14** (140 mg) as a solid.

**¹H NMR** (400 MHz, CDCl₃) δ_{H} 4.01-3.55 (m, 2H), 2.83 (t, 1H), 2.26-2.07 (m, 1H), 1.96-1.78 (m, 4H), 1.77-1.59 (m, 5H), 1.54-1.34 (m, 7H), 1.33-1.13 (m, 13H), 0.89-0.70 (m, 2H), 0.66 (s, 3H)

### Synthesis of III-A15

To a solution of **III-A14** (140 mg, 0.3 mmol) in acetone (2 mL) was added 4-cyanopyrazole (47.5 mg, 0.5 mmol) and K₂CO₃ (243 mg, 1.7 mmol). The mixture was stirred at 25°C for 16 hrs. To the mixture was added water (20 mL) and the mixture was extracted with EtOAc (2 x 20 mL). The organic layer was separated, concentrated and purified by flash column (25~50% EtOAc in PE) to give **III-A15** (130 mg) as a solid. The material (130 mg) was stirred in water (30 mL) at 80°C for 2 hrs. The mixture was extracted with EtOAc (2 x 50 mL). The organic layer was separated, concentrated to give **III-A15** (61 mg, 47.2%) as a solid.

**¹H NMR** (400 MHz, CDCl₃) 7.85 (s, 1H), 7.81 (s, 1H), 4.95 (dd, 2H), 2.61 (t, 1H), 2.27-2.12 (m, 1H), 2.02-1.58 (m, 8H), 1.53-1.23 (m, 12H), 1.22-1.09 (m, 6H), 0.92-0.80 (m, 2H), 0.70 (s, 3H); **LC-ELSD/MS** purity 99%, **MS ESI** calcd. for C₂₆H₃₈N₃O₂ [M +H]+ 424.3, found 424.3

### EXAMPLE III-4: Synthesis of (1S,3aS,3bR,6aS,8R,10aS,10bR,12aS)-1-methoxy-8,12a-dimethyloctadecahydrobenzo[3,4]cyclohepta[1,2-e]inden-8-ol (III-A20)

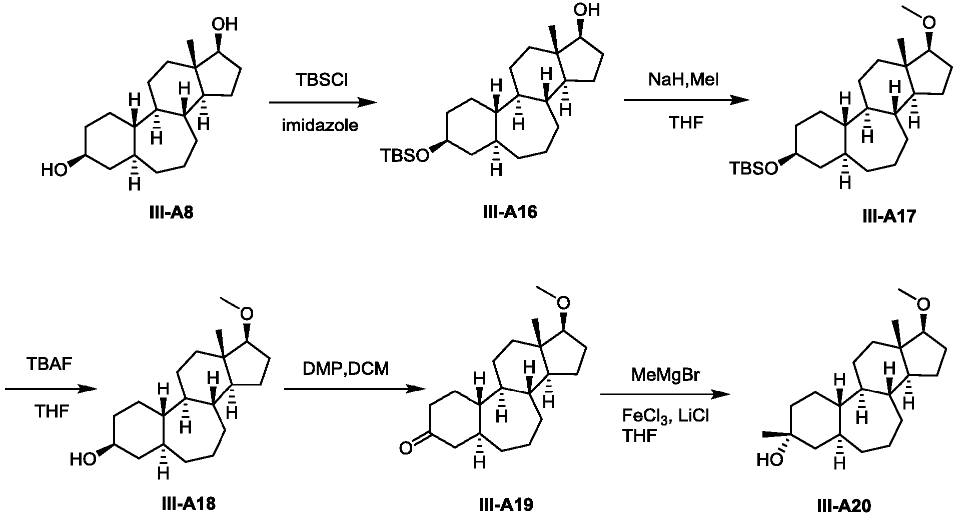

### Synthesis of III-A16

To a solution of **III-A8** (300 mg, 1.0 mmol) in DCM (10 mL) was added imidazole (138 mg, 2.0 mmol) at 25 °C. The mixture was cooled to 0 °C was added TBSCl (183 mg, 1.2 mmol). The reaction mixture was stirred at 25 °C for 16 hrs to give a mixture. To the mixture was added water (50 mL) and the mixture was extracted with EtOAc (2 x 50 mL). The organic layer was separated, concentrated and purified by flash column (0~10% EtOAc in PE) to give **III-A16** (60 mg, 14.4%) as a solid.

**¹H NMR** (400 MHz, CDCl₃) δ_{H} 3.68-3.45 (m, 2H), 2.04-1.93 (m, 2H), 1.87-1.61 (m, 6H), 1.53-1.28 (m, 9H), 1.22-0.96 (m, 6H), 0.93-0.85 (m, 11H), 0.79-0.69 (m, 4H), 0.15-0.01 (m, 7H)

### Synthesis of III-A17

To a solution of **III-A16** (540 mg, 1.3 mmol) in THF (10 mL) was added NaH (158 mg, 3.96 mmol, 60%) at 25°C. The reaction mixture was stirred for 30 min. MeI (562 mg, 3.96 mmol) was added into the reaction mixture at 25°C. The reaction mixture was stirred for 16 hours at 25°C. The residue was poured into water (50 mL). The aqueous phase was extracted with EtOAc (2 x 30 mL). The combined organic phase was washed with water (2 x 30 mL), dried over anhydrous Na₂SO₄, filtered and concentrated and was purified by flash column (0-30% of EtOAc in PE) to give **III-A17** (320 mg, 57.6%) as an oil.

**¹H NMR** (400 MHz, CDCl₃) δ_{H} 3.60-3.45 (m, 1H), 3.34 (s, 3H), 3.25-3.13 (m, 1H), 2.03-1.92 (m, 2H), 1.90-1.78 (m, 3H), 1.74-1.62 (m, 3H), 1.52-1.36 (m, 5H), 1.34-1.10 (m, 8H), 1.09-0.95 (m, 2H), 0.90-0.85 (m, 10H), 0.80-0.70 (m, 4H), 0.09-0.17 (m, 6H)

### Synthesis of III-A18

To a solution of **III-A17** (320 mg, 0.8 mmol) in THF (3 mL) was added TBAF (596 mg, 2.3 mmol). The mixture was stirred at 25°C for another 16 hrs. The mixture was concentrated in vacuum. The residue was dissolved in EtOAc (50 mL), washed with water (2 x 30 mL), brine (30 mL), dried over Na₂SO₄, filtered, concentrated in vacuum to give **III-A18** (300 mg) as a solid.

**¹H NMR** (400 MHz, CDCl₃) δ_{H} 3.65-3.49 (m, 1H), 3.39-3.30 (m, 4H), 3.25-3.14 (m, 1H), 2.01-1.78 (m, 7H), 1.77-1.31 (m, 13H), 1.28-0.95 (m, 10H), 0.91-0.83 (m, 3H), 0.78-0.67 (m, 4H), 0.11-0.06 (m, 1H)

### Synthesis of III-A19

To a solution of **III-A18** (300 mg, 0.98 mmol) in DCM (10 mL) was added DMP (826 mg, 1.95 mmol). The mixture was stirred at 25°C for 0.5hrs. The mixture was quenched by NaHCO₃ (50 mL, saturated) and Na₂S₂O₃ (50 mL, saturated). The organic layer was separated, dried over Na₂SO₄, filtered and concentrated and purified by flash column (0~30% EtOAc in PE) to give **III-A19** (180 mg, 60.6%) as a solid.

**¹H NMR** (400 MHz, CDCl₃) δ_{H} 3.34 (s, 3H), 3.27-3.16 (m, 1H), 2.42-2.11 (m, 5H), 2.04-1.94 (m, 1H), 1.93-1.76 (m, 3H), 1.72-1.52 (m, 4H), 1.49-1.32 (m, 6H), 1.30-1.01 (m, 5H), 0.92-0.84 (m, 1H), 0.81-0.72 (m, 3H)

### Synthesis of III-A20

A suspension of LiCl (210 mg, 4.96 mmol, anhydrous) in THF (8 mL, anhydrous) was stirred at 10°C for 30 min under N₂ and the solid was dissolved. FeCl₃ (421 mg, 2.6 mmol, anhydrous) was added to the mixture at 10°C. The mixture was cooled to -30°C. To the mixture was added MeMgBr (3.15 mL, 9.5 mmol, 3M in THF) dropwise at -30°C. The mixture was stirred at -30°C for 30 min. **III-A19** (180 mg, 0.6 mmol) was added to the mixture at -30°C. The mixture was stirred at -15°C for 2 hrs. To the mixture was added satd. citric acid (20 mL). The mixture was extracted with EtOAc (2 x 50 mL). The combined organic phase was washed with brine (2 x 50 mL), dried over Na₂SO₄, filtered, concentrated to give **III-A20** (90 mg) as an oil. The material (90 mg) was purified by HPLC separation to give **III-A20** (4 mg, 2.11%) as a solid.

**¹H NMR** (400 MHz, CDCl₃) δ_{H} 3.34 (s, 3H), 3.26-3.15 (m, 1H), 2.08-1.91 (m, 2H), 1.90-1.78 (m, 3H), 1.73-1.58 (m, 4H), 1.50-1.14 (m, 17H), 1.09-0.97 (m, 2H), 0.77 (s, 3H), 0.74-0.70 (m, 1H); **LC-ELSD/MS** purity 99%, **MS ESI** calcd. for C₂₁H₃₅O [M-H₂O+H]+303.3, found 303.3.

**EXAMPLE III-5: Biological Data**

In **Table III-1** below, A indicates a TBPS IC₅₀ (µM) < 0.01 µM, B indicates a TBPS IC₅₀ (µM) of 0.01 µM to < 0.1 µM, C indicates a TBPS IC₅₀ (µM) of 0.1 µM to < 1.0 µM, D indicates a TBPS IC₅₀ (µM) of 1.0 µM to < 10 µM, and E means ≥ 10 µM.

**Table III-1:**

| **Example** | **Compound ID** | **STRUCTURE** | **IC50 (µM)** |
|---|---|---|---|
| **III-1** | **III-A10** | 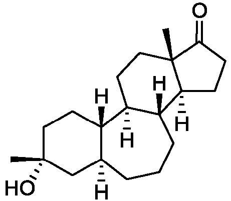 | |
| **III-2** | **III-A13** | 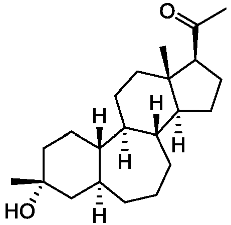 | D |
| **III-3** | **III-A15** | 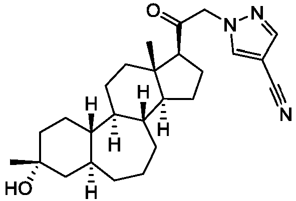 | C |
| **III-4** | **III-A20** | 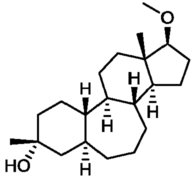 | |

### Example IV-1: Synthesis of 1-((3S,3aS,5aR,5bS,8R,9aR,10aR,10bS)-8-hydroxy-3a,8-dimethylhexadecahydrocyclopenta[a]fluoren-3-yl)ethan-1-one (IV-A14)

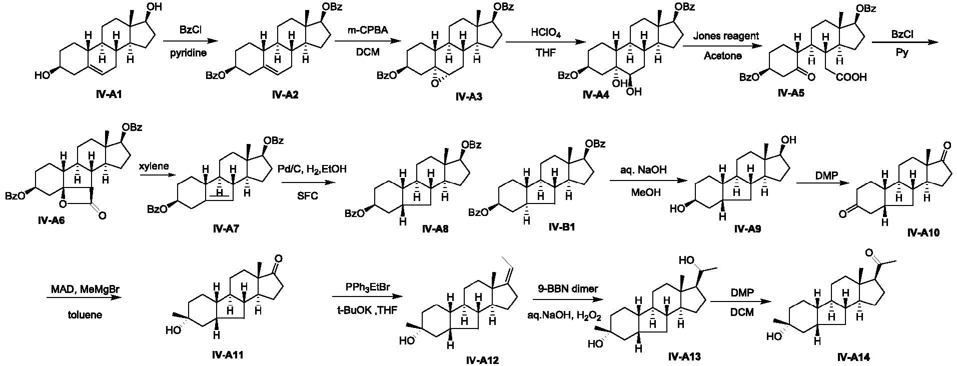

### Synthesis of IV-A2

To a solution of **IV-A1** (20 g, 72.3 mmol, CAS: 25975-59-1) and DMAP (8.83 g, 72.3 mmol) in pyridine (200 mL) was added dropwise benzoyl chloride (30.3 g, 216 mmol). After stirring at 80°C for 12 h, the mixture was poured into ice water (200 mL). The aqueous phase was extracted with EtOAc (3 x 200 mL). The combined organic solution was washed with brine (2 x 50 mL), dried over anhydrous Na₂SO₄, filtered and concentrated. The residue was purified by flash column (0~5% of EtOAc in PE), then was triturated from PE (50 mL) to give **IV-A2** (10.6 g, 30%) as a solid.

**¹HNMR**(400 MHz, CDCl₃) δ_{H} 8.01-7.92 (m, 4H), 7.52-7.44 (m, 2H), 7.42-7.30 (m, 4H), 5.51-5.34 (m, 1H), 4.89-4.64 (m, 2H), 2.65-2.42 (m, 1H), 2.31-1.74 (m, 7H), 1.70-1.49 (m, 5H), 1.41-1.04 (m, 6H), 0.91 (s, 3H), 0.88-0.69 (m, 1H).

### Synthesis of IV-A3

To a mixture of **IV-A2** (9.6 g, 19.8 mmol) in DCM (160 ml) was added m-CPBA (8.01 g, 39.6 mmol,85% purity). After stirring at 20°C for 12 h, the reaction was quenched with saturated aqueous Na₂S₂O₃ (30 mL) at 0°C and extracted with DCM (3 x 50 mL). The combined organic solution was washed with brine (20 mL), dried over anhydrous Na₂SO₄, filtered and concentrated. The residue was purified by flash column (0~10% of EtOAc in PE), then was triturated from PE (30 mL) to give to give **IV-A3** (3.8 g, 38%) as a solid. **¹HNMR**(400 MHz, CDCl₃) δ_{H} 8.06-7.99 (m, 4H), 7.58-7.51 (m, 2H), 7.43 (td, *J* = 7.6, 2.4 Hz, 4H), 5.29-5.16 (m, 1H), 4.88-4.78 (m, 1H), 3.03 (d, *J* = 5.6 Hz, 1H), 2.38-2.23 (m, 2H), 2.09-1.93 (m, 3H), 1.84-1.73 (m, 3H), 1.64-0.98 (m, 12H), 0.93 (s, 3H).

### Synthesis of IV-A4

To a solution of **IV-A3** (6.5 g, 6.2 mmol) in THF (30 mL) was added perchloric acid (7%, 6.5 mL) at 25°C. After stirring at 25°C for 16 h, the reaction was quenched with saturated aqueous Na₂S₂O₃ (10 mL) and extracted with EtOAc (3 x 30 mL). The combined organic solution was washed with brine (5 mL), dried over anhydrous Na₂SO₄, filtered and concentrated. The residue was triturated from PE (20 mL) to give **IV-A4** (5.7 g, 85%) as a solid.

**¹HNMR**(400 MHz, CDCl₃) δ_{H} 8.06-7.99 (m, 4H), 7.57-7.51 (m, 2H), 7.45-7.40 (m, 4H), 5.36-5.25 (m, 1H), 4.86 (t, *J* = 8.4 Hz, 1H), 3.61-3.56 (m, 1H), 2.36-2.24 (m, 1H), 2.23-2.13 (m, 1H), 2.07-1.74 (m, 4H), 1.72-1.62 (m, 4H), 1.55-1.42 (m, 3H), 1.41-1.33 (m, 1H), 1.32-1.08 (m, 5H), 0.98 (s, 3H).

### Synthesis of IV-A5

To a stirred solution of **IV-A4** (4.0 g, 7.7 mmol) in acetone (80 ml) at 50 °C was added Jones reagent (3.5 ml, 9.3 mmol) in portions over an hour. After stirring at 50°C for 0.5 h, i-PrOH (25 mL) was added and the mixture was concentrated. The residue was diluted with water (30 mL) and extracted with EtOAc (3 x 40 mL). The combined organic solution was washed with brine (20 mL), dried over anhydrous Na₂SO₄, filtered and concentrated. The residue was purified by flash column (0~30% of EtOAc in PE) to give **IV-A5** (1.9 g, 46%) as an oil. **¹HNMR**(400 MHz, CDCl₃) δ_{H} 8.06-7.98 (m, 4H), 7.59-7.52 (m, 2H), 7.48-7.41 (m, 4H), 5.26-5.14 (m, 1H), 4.85 (t, *J* = 8.4 Hz, 1H), 2.97-2.86 (m, 1H), 2.61-2.42 (m, 3H), 2.41-2.23 (m, 3H), 2.13-1.96 (m, 4H), 1.90-1.75 (m, 3H), 1.70-1.58 (m, 2H), 1.57-1.44 (m, 3H), 1.40-1.29 (m, 2H), 0.97 (s, 3H).

### Synthesis of IV-A6

To a solution of **IV-A5** (1.9 g, 3.6 mmol) in pyridine (10 mL) was added benzoyl chloride (2.50 g, 17.8 mmol) drop-wise at 0°C under N₂. After stirring at 25°C for 16 h, the reaction mixture was poured onto ice (20 ml) and extracted with ethyl acetate (3 × 20 ml). The combined organic solution was washed with a cold solution of sodium hydroxide (0 °C, 2%) (20 mL), water (20 mL) and brine (10 mL), dried over anhydrous Na₂SO₄, filtered and concentrated. The residue was purified by flash column (0~25% of EtOAc in PE) to give **IV-A6** (1.1 g, 60%) as a solid.

**¹HNMR**(400 MHz, CDCl₃) δ_{H} 8.08-8.01 (m, 4H), 7.60-7.53 (m, 2H), 7.44 (td, *J* = 7.6, 2.8 Hz, 4H), 5.42-5.34 (m, 1H), 4.92 (dd, *J* = 8.8, 7.2 Hz, 1H), 3.26 (d, *J* = 7.6 Hz, 1H), 2.49-2.28 (m, 2H), 2.22-2.00 (m, 5H), 1.93-1.85 (m, 1H), 1.82-1.64 (m, 5H), 1.48-1.22 (m, 5H), 1.01 (s, 3H).

### Synthesis of IV-A7

A solution of **IV-A6** (1.8 g, 3.5 mmol) in xylene (30 mL) was stirred at 160°C for 12 hours. The mixture was concentrated and purified by flash column (0~8% of EtOAc in PE) to give **IV-A7** (1.45 g, 88%) as a solid.

**¹HNMR**(400 MHz, CDCl₃) δ_{H} 8.08-8.02 (m, 4H), 7.58-7.52 (m, 2H), 7.44 (td, *J*=7.6, 2.0 Hz, 4H), 5.58-5.55 (m, 1H), 4.98-4.86 (m, 2H), 2.97-2.89 (m, 1H), 2.42-2.02 (m, 6H), 1.89-1.59 (m, 5H), 1.55-1.32 (m, 3H), 1.30-1.19 (m, 1H), 1.17-1.02 (m, 2H), 0.98 (s, 3H).

### Synthesis of IV-A8 & IV-B1

To a solution of **IV-A7** (1.35 g, 2.9 mmol) in EtOH (36 mL) and THF (27 mL) was added Pd/C (wet, 10%, 300 mg) under N₂. The suspension was degassed under vacuum and purged with H₂ for three times. After stirring under H₂ (103421 Pa = 15 psi) at 25°C for 24 h, the reaction mixture was filtered through a pad of Celite and washed with THF (3 x 30 mL). The filtrate was concentrated and purified by flash column (0~5% of EtOAc in PE) to give a mixture of **IV-A8** and **IV-B1** (1.2 g, 89%) as a solid. The mixture was purified by SFC (Column: DAICEL CHIRALCEL OJ(250mm*50mm, 10um), gradient: 30-30% B 0.1%NH3H2O ETOH-CO2, flow rate: 200 mL/min) to afford **IV-B1** (0.46 g, 36%) and **IV-A8** (0.52 g, 40%) as solids. Both structures regiochemistry were confirmed by next step.

**IV-B1: ¹HNMR**(400 MHz, CDCl₃) δ_{H} 8.08-8.02 (m, 4H), 7.58-7.52 (m, 2H), 7.47-7.40 (m, 4H), 5.07-4.97 (m, 1H), 4.89 (dd, *J* = 8.8, 7.6 Hz, 1H), 2.37-2.24 (m, 2H), 2.21-2.13 (m, 1H), 1.98-1.90 (m, 1H), 1.86-1.81 (m, 1H), 1.76-1.61 (m, 4H), 1.55-1.37 (m, 7H), 1.24-0.80 (m, 8H). Analytical **SFC:** 100% de.

**IV-A8: ¹HNMR**(400 MHz, CDCl₃) δ_{H} 8.08-8.02 (m, 4H), 7.58-7.52 (m, 2H), 7.48-7.40 (m, 4H), 5.32-5.23 (m, 1H), 4.89 (dd, *J* = 9.2, 7.6 Hz, 1H), 2.43-2.25 (m, 2H), 2.04-1.85 (m, 5H), 1.75-1.61 (m, 5H), 1.53-1.18 (m, 8H), 0.95 (s, 3H), 0.82-0.71 (m, 1H). Analytical **SFC:** 99.3% de.

### Synthesis of IV-A9

To a suspension of **IV-A8** (0.51 g, 1.1 mmol) in MeOH (5 mL) and THF (5 mL) was added NaOH aq (4 mL, 2M) at 25°C under N₂. After stirring at 50°C for 12 h, the solution was diluted with H₂O (8 mL), concentrated slightly to remove MeOH, and extracted with EtOAc (3 x 15 mL). The combined organic solution was washed with brine (5 mL), dried over anhydrous Na₂SO₄, filtered and concentrated. The residue was purified by flash column (0~30% of EtOAc in PE) to give **IV-A9** (0.27 g, 96%) as a solid. The structure was confirmed by NOE.

**¹HNMR**(400 MHz, CDCl₃) δ_{H} 4.10-4.02 (m, 1H), 3.72-3.62 (m, 1H), 2.34-2.21 (m, 1H), 2.15-2.01 (m, 1H), 1.99-1.90 (m, 1H), 1.89-1.75 (m, 3H), 1.74-1.65 (m, 2H), 1.63-1.49 (m, 3H), 1.46-1.26 (m, 6H), 1.24-0.97 (m, 4H), 0.92-0.78 (m, 1H), 0.73 (s, 3H), 0.71-0.62 (m, 1H). **LC-ELSD/MS** purity 99%, MS ESI calcd. for C₁₇H₂₅ [M-2H₂O+H]⁺ 229.1, found 229.1.

### Synthesis of IV-A10

To a solution of **IV-A9** (270 mg, 1.0 mmol) in DCM (10 mL) was added Dess-martin periodinane (1.29 g, 3.1 mmol) at 30°C. After stirring at 30°C for 1 hour. The mixture was quenched by saturated NaHCO₃ aqueous (10 mL). The DCM phase was separated and washed with saturated NaHCO₃/Na₂S₂O₃ aqueous (1:1, 2 x 10 mL), brine (10 mL), dried over Na₂SO₄, filtered and concentrated under vacuum to give a solid, which was purified by trituration in PE/EtOAc (10/1, 10 mL) to give **IV-A10** (265 mg, 100%) as a solid.

**¹HNMR** (400 MHz, CDCl₃) δ_{H} 2.52-2.31 (m, 4H), 2.29-2.02 (m, 5H), 1.95-1.79 (m, 4H), 1.71-1.63 (m, 1H), 1.57-1.34 (m, 3H), 1.32-1.20 (m, 2H), 1.00-0.81 (m, 5H); **LC-ELSD/MS** purity 99%, MS ESI calcd. for C₁₇H₂₄O₂Na [M+Na]⁺ 283.0, found 283.0.

### Synthesis of IV-A11

To the MAD (7.8 mmol) solution was added a solution of **IV-A10** (0.34 g, 1.3 mmol) in DCM (3 mL) dropwise at -70°C. After stirring at -70°C for 1 h under N₂, MeMgBr (1.73 mL, 5.2 mmol, 3M in ethyl ether) was added drop wise at -70°C. After stirring at -70°C for another 2 h, the reaction mixture was poured into saturated aqueous citric acid (30 mL) below 10°C and extracted with EtOAc (2 x 40 mL). The combined organic solution was dried over Na₂SO₄, filtered and concentrated. The residue was purified by flash column (0~35% of EtOAc in PE) to afford **IV-A11** (70 mg, 19%) as an oil. The configuration of C3 was assigned by NOE.

**¹HNMR** (400 MHz, CDCl₃) δ_{H} 2.43 (dd, *J* = 19.2, 8.0 Hz, 1H), 2.18-1.98 (m, 3H), 1.93-1.72 (m, 3H), 1.71-1.36 (m, 10H), 1.31-1.09 (m, 6H), 1.08-0.97 (m, 1H), 0.92-0.76 (m, 4H). **LC-ELSD/MS** purity 99%, MS ESI calcd. for C₁₈H₂₇O [M-H₂O+H]⁺ 259.2, found 259.2.

### Synthesis of IV-A12

To a mixture of PPh₃EtBr (1.34 g, 3.6 mmol) in THF (3 mL) was added t-BuOK (404 mg, 3.61 mol) at 15°C under N₂. After stirring at 40°C for 1h, a solution of **IV-A11** (0.10 g, 0.4 mmol) in THF (1 mL) was added in portions below 40°C. After stirring at 40°C for 12 h, the reaction mixture was quenched with saturated NH₄Cl aqueous (10 mL) at 15°C and extracted with EtOAc (2×25 mL). The combined organic solution was concentrated. The residue was purified by flash column (0~10% of EtOAc in PE) to give **IV-A12** (70 mg, 67%) as a solid. **¹HNMR**(400 MHz, CDCl₃) δ_{H} 5.25-4.97 (m, 1H), 2.43-2.31 (m, 1H), 2.30-2.13 (m, 2H), 2.10-1.92 (m, 2H), 1.76-1.61 (m, 6H), 1.56-1.36 (m, 6H), 1.34-1.11 (m, 9H), 1.06-0.94 (m, 1H), 0.84 (s, 3H), 0.80-0.68 (m, 1H).

### Synthesis of IV-A13

To a solution of **IV-A12** (70 mg, 0.2 mmol) in THF (4 mL) was added 9-BBN dimer (177 mg, 0.7 mmol) at 0°C. After stirring at 45°C for 12 h, ethanol (1 mL) was added at 15°C, followed by NaOH aqueous (0.7 mL, 5.0 M) at 0°C and then hydrogen peroxide (0.36 mL, 10 M, 25 mmol) drop-wise at 0°C. After stirring at 78°C for 1 h, the mixture was cooled to 15°C and saturated aqueous Na₂S₂O₃ (20 mL) was added. The aqueous phase was extracted with EtOAc (2×30 mL). The combined organic solution was washed with brine (2 x 10 mL), dried over anhydrous Na₂SO₄, filtered and concentrated. The residue was purified by flash column (0~30% of EtOAc in PE) to afford **IV-A13** (60 mg, 81%) as an oil.

**¹HNMR** (400 MHz, CDCl₃) δ_{H} 3.78-3.68 (m, 1H), 2.11-1.80 (m, 4H), 1.69-1.52 (m, 8H), 1.46-1.31 (m, 4H), 1.29-1.05 (m, 12H), 1.03-0.91 (m, 1H), 0.78-0.58 (m, 4H).

### Synthesis of IV-A14

To a solution of **IV-A13** (60 mg, 0.2 mmol) in DCM (3 mL) was added Dess-martin periodinane (166 mg, 0.4 mmol) at 30°C. After stirring at 30°C for 1 h, the mixture was diluted with DCM (20 mL) and quenched by saturated NaHCO₃ aqueous (10 mL). The DCM phase was separated and washed with saturated NaHCO₃/Na₂S₂O₃ aqueous (1:1, 2 x 10 mL), brine (10 mL), dried over Na₂SO₄, filtered and concentrated. The residue was purified by flash column (0~18% of EtOAc in PE) to give **IV-A14** (7 mg, 12%) as a solid.

**¹HNMR** (400 MHz, CDCl₃) δ_{H} 2.59 - 2.47 (m, 1H), 2.27 - 1.92 (m, 7H), 1.59 - 1.34 (m, 7H), 1.32 - 0.66 (m, 14H), 0.59 (s, 3H).

### EXAMPLE IV-2: Synthesis of 1-(2-((3S,3aS,5aR,5bS,8R,9aR,10aR,10bS)-8-hydroxy-3a,8-dimethylhexadecahydrocyclopenta[a]fluoren-3-yl)-2-oxoethyl)-1H-pyrazole-4-carbonitrile (IV-A16)

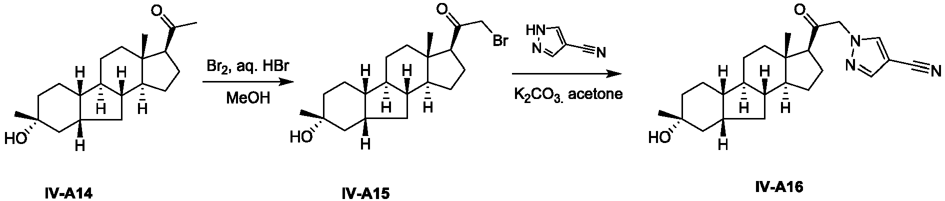

### Synthesis of IV-A15

To a solution of **IV-A14** (7 mg, 0.023 mmol) in MeOH (1.0 ml) was added HBr (0.93 mg, 0.005 mmol, 40% in water) and Br₂ (4 mg, 0.03 mmol) at 25°C. After stirring at 25°C for 2 h, the mixture was quenched by sat.aq Na₂S₂O₃ (1 mL), sat.aq NaHCO₃ (1 mL) and water (3 mL), and extracted with EtOAc (2 x 5 mL). The combined organic solution was washed with brine (2 mL), dried over anhydrous Na₂SO₄, filtered, concentrated in vacuum to afford **IV-A15** (8.8 mg) as a solid used directly for the next step.

### Synthesis of IV-A16

To a solution of **IV-A15** (8.8 mg, 0.02 mmol) in acetone (1 mL) was added 1H-pyrazole-4-carbonitrile (3 mg, 0.03 mmol) and K₂CO₃ (9.5 mg, 0.07 mmol). After stirring at 25°C for 12 h, the mixture was treated with water (3 mL), extracted with EtOAc (2 x 8 mL). The combined organic solution was washed with brine (2 mL), dried over anhydrous Na₂SO₄, filtered, concentrated. The residue was purified by flash column (0~45% of EtOAc in PE), then was purified by HPLC separation (column: Waters Xbridge 150*25*5u, gradient: 45-75% B (water(10mM NH₄HCO₃)-ACN), flow rate: 25 mL/min) to give **IV-A16** (1.2 mg, 13%) as a solid.

**¹HNMR**(400 MHz, CDCl₃) δ_{H} 7.86 (s, 1H), 7.81 (s, 1H), 5.09-5.02 (m, 1H), 4.95-4.88 (m, 1H), 2.61 (t, *J* = 8.8 Hz, 1H), 2.25-2.17 (m, 1H), 2.09-1.96 (m, 3H), 1.83-1.59 (m, 6H), 1.47-1.36 (m, 3H), 1.36-1.12 (m, 10H), 1.10-0.99 (m, 1H), 0.80-0.71 (m, 1H), 0.65 (s, 3H). **LC-ELSD/MS** purity 99%, MS ESI calcd. for C₂₄H₃₄N₃O₂[M +H]⁺ 396.2, found 396.2.

### EXAMPLE IV-3: Synthesis of 1-((3S,3aS,5aR,5bS,8R,9aS,10aR,10bS)-8-hydroxy-3a,8-dimethylhexadecahydrocyclopenta[a]fluoren-3-yl)ethan-1-one (IV-B7)

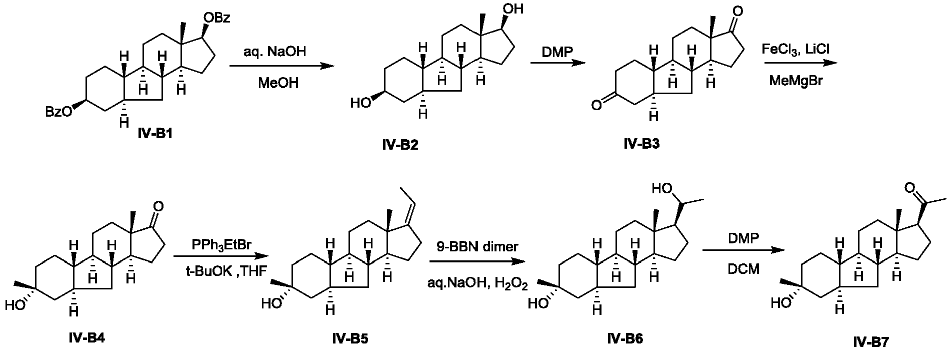

### Synthesis of IV-B2

To a suspension of **IV-B2** (0.45 g, 1.0 mmol) in MeOH (5 mL) and THF (5 mL) was added NaOH aq (4 mL, 2M) at 25°C under N₂. After stirring at 50°C for 12 h, the reaction mixture was diluted with H₂O (8 mL), concentrated to remove the MeOH, then was extracted with EtOAc (3 x 15 mL). The combined organic solution was washed with brine (5 mL), dried over anhydrous Na₂SO₄, filtered and concentrated. The residue was purified by flash column (0~30% of EtOAc in PE) to give **IV-B2** (0.24 g, 96%) as a solid.

**¹HNMR** (400 MHz, CDCl₃) δ_{H} 3.71-3.59 (m, 2H), 2.17-1.97 (m, 3H), 1.891.76 (m, 2H), 1.72-1.65 (m, 1H), 1.56-1.47 (m, 2H), 1.46-1.10 (m, 11H), 1.06-0.84 (m, 3H), 0.77-0.66 (m, 4H). **LC-ELSD/MS** purity 99%, MS ESI calcd. for C₁₇H₂₅[M -2H₂O+H]⁺229.2, found 229.2.

### Synthesis of IV-B3

To a solution of **IV-B2** (240 mg, 0.9 mmol) in DCM (8 mL) was added Dess-martin periodinane (1.15 g, 2.7 mmol) at 30°C. After stirring at 30°C for 1 h, the mixture was quenched by saturated NaHCO₃ aqueous (10 mL). The DCM phase was separated and washed with saturated NaHCO₃/Na₂S₂O₃ aqueous (1:1, 2 x 10 mL), brine (10 mL), dried over Na₂SO₄, filtered and concentrated. The residue was purified by flash column (0~15% of EtOAc in PE) to give **IV-B3** (210 mg, 89%) as a solid.

**¹HNMR** (400 MHz, CDCl₃) δ_{H} 2.62-2.54 (m, 1H), 2.50-2.39 (m, 2H), 2.31-2.21 (m, 2H), 2.15-2.03 (m, 2H), 1.94-1.86 (m, 1H), 1.86-1.72 (m, 4H), 1.68-1.60 (m, 2H), 1.58-1.47 (m, 2H), 1.46-1.20 (m, 4H), 0.97-0.83 (m, 4H). **LC-ELSD/MS** purity 99%, MS ESI calcd. for C₁₇H₂₅O₂ [M +H]⁺ 261.1, found 261.1.

### Synthesis of IV-B4

After stirring at 10°C for 30 minutes under N₂ a suspension of LiCl (178 mg, 4.2 mmol, anhydrous) in THF (5 mL, anhydrous), FeCl₃ (1.29 g, 8.1 mmol, anhydrous) was added. After cooling to -30°C, MeMgBr (5.1 mL, 3M in diethyl ether) was added drop-wise. After stirring at-30°C for 10 min, **IV-B3** (200 mg, 0.77 mmol) was added. After stirring at -15°C for 2 h, citric acid (10 mL, 20% aq.) was added and the mixture was extracted with EtOAc (2 x 20 mL). The combined organic solution was washed with saturated brine (10 mL), dried over anhydrous Na₂SO₄, filtered and concentrated. The residue was purified by flash column (0~20% of EtOAc in PE) to give **IV-B4** (130 mg, 61%) as a solid. The configuration of C3 was assigned by NOE.

**¹HNMR** (400 MHz, CDCl₃) δ_{H} 2.47-2.37 (m, 1H), 2.13-2.01 (m, 1H), 1.93-1.78 (m, 4H), 1.75-1.48 (m, 8H), 1.41-1.17 (m, 9H), 0.92-0.83 (m, 5H). **LC-ELSD/MS** purity 99%, MS ESI calcd. for C₁₈H₂₇O [M -H₂O+H]⁺259.2, found 259.2.

### Synthesis of B5

To a mixture of PPh₃EtBr (1.61 g, 4.3 mmol) in THF (4 mL) was added t-BuOK (486 mg, 4.3 mol) at 15°C under N₂. After stirring at 40°C for 1 h, a solution of **IV-B4** (0.12 g, 0.43 mmol) in THF (1 mL) was added in portions below 40°C. After stirring at 40°C for 12 h, the reaction mixture was quenched with saturated NH₄Cl aqueous (10 mL) at 15°C and extracted with EtOAc (2×25 mL). The combined organic solution was concentrated. The residue was purified by flash column (0~10% of EtOAc in PE) to give **IV-B5** (90 mg, 72%) as a solid. **¹HNMR** (400 MHz, CDCl₃) δ_{H} 5.18-5.02 (m, 1H), 2.41-2.31 (m, 1H), 2.28-2.13 (m, 2H), 1.83-1.65 (m, 8H), 1.55-1.29 (m, 8H), 1.23 (s, 7H), 0.90-0.78 (m, 5H).

### Synthesis of IV-B6

To a solution of **IV-B5** (90 mg, 0.31 mmol) in THF (5 mL) was added 9-BBN dimer (228 mg, 0.94 mmol) at 0°C. After stirring at 45°C for 12 h, ethanol (1 mL) at 15°C, followed by NaOH aqueous (0.93 mL, 5.0 M) at 0°C and H₂O₂ (0.47 mL, 10 M, 25 mmol) at 0°C were added dropwise. After stirring at 78°C for 1 h, the mixture was cooled to 15°C and saturated aqueous Na₂S₂O₃ (20 mL) was added. The reaction mixture was extracted with EtOAc (2×30 mL). The combined organic solution was washed with brine (2 x 10 mL), dried over anhydrous Na₂SO₄, filtered and concentrated. The residue was purified by flash column (0~30% of EtOAc in PE) to afford **IV-B6** (75 mg, 79%) as a solid.

**¹HNMR** (400 MHz, CDCl₃) δ_{H} 3.78-3.68 (m, 1H), 1.98-1.74 (m, 4H), 1.73-1.62 (m, 5H), 1.57-1.48 (m, 2H), 1.47-1.32 (m, 5H), 1.28-1.18 (m, 11H), 1.18-1.13 (m, 1H), 0.88-0.75 (m, 2H), 0.64 (s, 3H)

### Synthesis of IV-B7

To a solution of **IV-B6** (75 mg, 0.24 mmol) in DCM (3 mL) was added Dess-martin periodinane (207 mg, 0.49 mmol) at 30°C. After stirring at 30°C for 1 h, the mixture was diluted with DCM (20 mL) and quenched by saturated NaHCO₃ aqueous (10 mL). The organic phase was separated and washed with saturated NaHCO₃/Na₂S₂O₃ aqueous (1:1, 2 x 10 mL), brine (10 mL), dried over Na₂SO₄, filtered and concentrated. The residue was purified by flash column (0~18% of EtOAc in PE) to give **IV-B7** (40 mg, 54%) as a solid. **¹HNMR** (400 MHz, CDCl₃) δ_{H} 2.55 (t, *J* = 8.8 Hz, 1H), 2.21-2.10 (m, 4H), 2.04-1.94 (m, 1H), 1.82-1.61 (m, 7H), 1.48-1.33 (m, 5H), 1.32-1.19 (m, 9H), 0.91-0.79 (m, 2H), 0.59 (s, 3H). **LC-ELSD/MS** purity 99%, MS ESI calcd. for C₂₀H₃₁O [M -H₂O+H]⁺287.2, found 287.2.

### EXAMPLE IV-4: Synthesis of 1-(2-((3S,3aS,5aR,5bS,8R,9aS,10aR,10bS)-8-hydroxy-3a,8-dimethylhexadecahydrocyclopenta[a]fluoren-3-yl)-2-oxoethyl)-1H-pyrazole-4-carbonitrile (IV-B9)

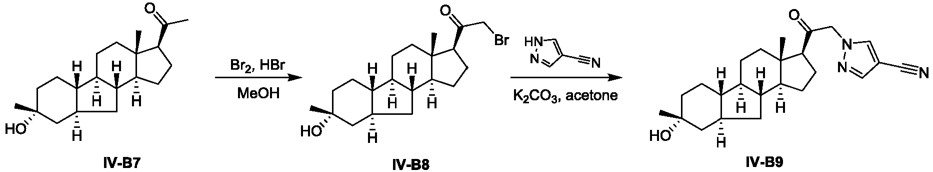

### Synthesis of B8

To a solution of **IV-B7** (35 mg, 0.11 mmol) in MeOH (1.5 ml) was added HBr (4.62 mg, 0.023 mmol, 40% in water) and Br₂ (20.1 mg, 0.13 mmol) at 25°C. After stirring at 25°C for 2 h, the mixture was quenched by sat.aq Na₂S₂O₃ (2 mL) and sat.aq NaHCO₃ (2 mL), diluted with water (5 mL), and extracted with EtOAc (2 x 10 mL). The combined organic solution was washed with brine (5 mL), dried over anhydrous Na₂SO₄, filtered, concentrated in vacuum to afford **IV-B8** (44 mg) as a solid used directly for the next step.

**¹HNMR** (400 MHz, CDCl₃) δ_{H} 3.97-3.88 (m, 2H), 2.84 (t, *J* = 8.8 Hz, 1H), 2.24-2.14 (m, 1H), 1.94-1.87 (m, 1H), 1.81-1.65 (m, 8H), 1.38-1.21 (m, 12H), 0.91-0.80 (m, 3H), 0.62 (s, 3H).

### Synthesis of IV-B9

To a solution of **IV-B8** (44 mg, 0.11 mmol) in acetone (2 mL) was added 1H-pyrazole-4-carbonitrile (16 mg, 0.17 mmol) and K₂CO₃ (48 mg, 0.34 mmol). After stirring at 25°C for 12 h, the mixture was diluted with water (5 mL) and extracted with EtOAc (2 x 10 mL). The combined organic solution was washed with brine (5 mL), dried over anhydrous Na₂SO₄, filtered and concentrated. The residue was purified by flash column (0~43% of EtOAc in PE) to give **IV-B9** (22 mg, 49%) as a solid.

**¹HNMR** (400 MHz, CDCl₃) δ_{H} 7.86 (s, 1H), 7.81 (s, 1H), 5.08-5.02 (m, 1H), 4.94-4.87 (m, 1H), 2.63 (t, *J* = 8.8 Hz, 1H), 2.26-2.16 (m, 1H), 2.06-1.99 (m, 1H), 1.82-1.70 (m, 6H), 1.46-1.17 (m, 15H), 0.91-0.83 (m, 2H), 0.65 (s, 3H). **LC-ELSD/MS** purity 99%, MS ESI calcd. for C₂₄H₃₃N₃O₂ [M+H]⁺396.2, found 396.2

### EXAMPLE IV-5:

### Steroid Inhibition of TBPS Binding

In **Table IV-2** below, A indicates a TBPS IC₅₀ (µM) < 0.01 µM, B indicates a TBPS IC₅₀ (µM) of 0.01 µM to < 0.1 µM, C indicates a TBPS IC₅₀ (µM) of 0.1 µM to < 1.0 µM, D indicates a TBPS IC₅₀ (µM) of 1.0 µM to < 10 µM, and E means ≥ 10 µM.

**Table IV-2.**

| **Example** | **Intermediate** | **STRUCTURE** | **IC50** |
|---|---|---|---|
| IV-1 | IV-A14 | 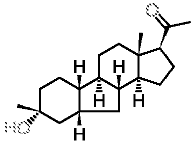 | |
| IV-2 | IV-A16 | 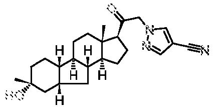 | B |
| IV-3 | IV-B7 | 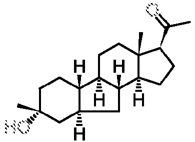 | B |
| IV-4 | IV-B9 | 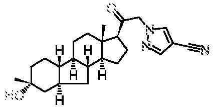 | B |

### EXAMPLE V-1: Synthesis of 1-((3aS,4S,6aS,6bR,8aS,10R,12aS,12bS)-10-hydroxy-3a,10,12a-trimethyloctadecahydronaphtho[2,1-e]azulen-4-yl)ethan-1-one (V-A8)

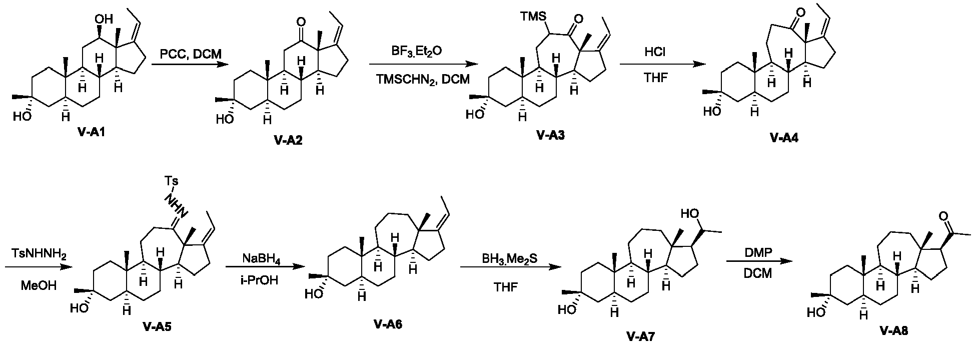

### Synthesis of V-A2

To a solution of **V-A1** (4 g, 12 mmol, reported in WO 2018013615) in DCM (50 mL) at 0°C was added silica gel (5 g) and PCC (5.17 g, 24 mmol). The mixture was stirred at 25°C for 2 h. PE (50 mL) was added to the reaction mixture. The resulting mixture was filtered through a pad of celite and the filter cake was washed with DCM (80 mL). The filtrate was concentrated and the residue was purified by flash column (5%~30% of EtOAc in PE) to give **V-A2** (2.5 g, 63.1%) as a solid.

**¹H NMR** (400 MHz, CDCl₃) δ_{H} 5.51-5.41 (m, 1H), 2.60 (t, *J*=12.8 Hz, 1H), 2.41-2.24 (m, 3H), 1.95-1.78 (m, 2H), 1.75-1.68 (m, 1H), 1.63-1.59 (m, 3H), 1.55-1.48 (m, 3H), 1.46-1.27 (m, 9H), 1.23 (s, 3H), 1.21 (s, 3H), 1.15-0.98 (m, 2H), 0.86 (s, 3H).

### Synthesis of V-A3

To a solution of **V-A2** (2.4 g, 7.26 mmol) and BF₃·Et₂O (4.57 mL, 36.3 mmol) in dry CH₂Cl₂ (30 mL) was added TMSCHN₂ (10.8 mL, 21.7 mmol) (2 M in hexane) dropwise at -20°C. After stirring at -15°C for 4 h under N₂, the reaction mixture was poured into ice-water (80 mL) and extracted with CH₂Cl₂ (2 x 100 mL). The combined organic phase was washed with brine (50 mL), dried over Na₂SO₄, filtered and concentrated to give **V-A3** (2.5 g) as a solid.. **¹H NMR** (400 MHz, CDCl₃) δ_{H}. 5.42-4.32 (m, 1H), 2.80-2.70 (m, 1H), 2.64-2.52 (m, 1H), 2.34-2.23 (m, 2H), 1.99-1.81 (m, 3H), 1.76-1.65 (m, 4H), 1.54-1.43 (m, 6H), 1.40 (s, 3H), 1.35-1.09 (m, 11H), 0.72 (s, 3H), 0.10-1.06 (m, 9H).

### Synthesis of V-A4

To a solution of **V-A3** (2.5 g, 5.99 mmol) in THF (25 mL) was added 2N HCl (3.59 mL, 7.18 mmol). After stirring at 25°C for 3 h, the mixture was added saturated NaHCO₃ (50 mL) and extracted with EtOAc (2 x 100 mL). The combined organic phase was dried over anhydrous Na₂SO₄, filtered and concentrated. The residue was purified by flash column (5~20% of EtOAc in PE) to give **V-A4** (1 g, 48.5%) as a solid.

**¹H NMR** (400 MHz, CDCl₃) δ_{H} 5.41-5.32 (m, 1H), 2.80-2.70 (m, 1H), 2.64-2.51 (m, 1H), 2.35-2.22 (m, 2H), 2.00-1.80 (m, 3H), 1.75-1.61 (m, 4H), 1.54-1.44 (m, 6H), 1.40 (s, 3H), 1.37-1.06 (m, 11H), 0.92-0.83 (m, 1H), 0.72 (s, 3H).

### Synthesis of V-A5

To solution of **V-A4** (650 mg, 1.88 mmol) in MeOH (10 mL) was added TsNHNH₂ (525 mg, 2.82 mmol). After refluxing at 80°C for 20 h, the mixture was added to water (50 mL) and the aqueous phase was extracted with EtOAc (3 x 50 mL). The combined organic phase was washed with brine (50 mL), dried over Na₂SO₄, filtered and concentrated to give **V-A5** (960 mg) as a solid.

### Synthesis of V-A6

To a solution of **V-A5** (960 mg, 1.87 mmol) in i-PrOH (10 mL) was added NaBH₄ (141 mg, 3.74 mmol). After stirring at 80°C reflux for 16 h, the mixture was added water (80 mL) and extracted with EtOAc (3 x 50 mL). The organic layer was dried over Na₂SO₄, filtered and concentrated and the residue was purified by flash column (0~10% of EtOAc in PE) to give **V-A6** (300 mg) as an oil.

### Synthesis of V-A7

To a solution of **V-A6** (300 mg) in THF (10 mL) was added BH₃.Me₂S (0.5 mL, 10 M, 5 mmol). After stirring at 40°C for 12 h. EtOH (2 mL) dropwise follow by NaOH (1.81 mL, 5 M) and H₂O₂ (1 mL, 10 M) were added to the mixture. After stirring at 60°C for 1 h, the mixture was quenched by Na₂S₂O₃ (50 mL, 10%) and extracted with EtOAc (2 x 80 mL). The combined organic solution was dried over Na₂SO₄, filtered and concentrated and the residue was purified by flash column (0~15% of EtOAc in PE) to give **V-A7** (60 mg, 18.9%) as an oil.

**¹H NMR** (400 MHz, CDCl₃) δ_{H} 3.92-3.66 (m, 1H), 2.07-1.97 (m, 1H), 1.82-1.59 (m, 6H), 1.55-1.35 (m, 10H), 1.34-1.10 (m, 13H), 1.07-0.90 (m, 3H), 0.87-0.81 (m, 3H), 0.84-0.71 (m, 3H).

### Synthesis of V-A8

To a solution of **V-A7** (60 mg, 0.17 mmol) in DCM (2 mL) was added DMP (145 mg, 0.34 mmol). After stirring at 25°C for 1 h, the mixture was quenched with saturated NaHCO₃ (30 mL) and saturated Na₂S₂O₃ (20 mL) and extracted with DCM (2 x 50 mL). The organic layer was dried over Na₂SO₄, filtered, concentrated. The residue was purified by flash column (0~10% of EtOAc in PE) to give **V-A8** (40 mg, 64.4%) as a solid.

**¹H NMR** (400 MHz, CDCl₃) δ_{H} 2.62 (t, *J*=9.6 Hz, 1H), 2.12 (s, 3H), 2.02-1.86 (m, 2H), 1.81-1.74 (m, 2H), 1.72-1.56 (m, 5H), 1.50-1.30 (m, 9H), 1.28-1.14 (m, 8H), 1.05- 0.93 (m, 2H), 0.80 (s, 3H), 0.68 (s, 3H); **LC-ELSD/MS** purity 99%, **MS ESI** calcd. for C₂₃H₃₇O [M-OH]⁺ 329.3, found 329.3.

### EXAMPLE V-2: Synthesis of 1-(2-((3aS,4S,6aS,6bR,8aS,10R,12aS,12bS)-10-hydroxy-3a,10,12a-trimethyloctadecahydronaphtho[2,1-e]azulen-4-yl)-2-oxoethyl)-1H-pyrazole-4-carbonitrile (V-A10)

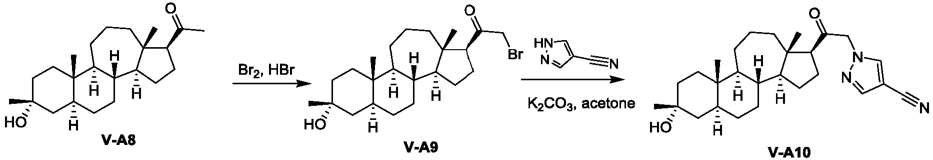

### Synthesis of V-A9

To a solution of **V-A8** (30 mg, 0.087 mmol) in MeOH (2 mL) was added HBr (4 mg, 0.02 mmol, 40% in water) and Br₂ (20 mg, 0.13 mmol) at 25°C. After stirring for 3 h, the mixture was quenched by saturated aqueous NaHCO₃ (10 mL), treated with water (20 mL). The aqueous solution was extracted with EtOAc (2 x 30 mL) and the combined organic solution was washed with brine (30 mL), dried over anhydrous Na₂SO₄, filtered, concentrated to give **V-A9** (35 mg, 95.1%) as an oil which was used directly for the next step.

**¹H NMR** (400 MHz, CDCl₃) δ_{H} 3.92 (s, 2H), 2.91 (t, *J*=9.2 Hz, 1H), 2.01-1.92 (m, 1H), 1.83-1.59 (m, 8H), 1.50-1.38 (m, 6H), 1.37-1.14 (m, 10H), 1.01-0.91 (m, 2H), 0.89-0.80 (m, 4H), 0.67 (s, 3H).

### Synthesis of V-A10

To a solution of **V-A9** (35 mg, 0.08 mmol) in acetone (2 mL) was added K₂CO₃ (34 mg, 0.25 mmol) and 1*H*-pyrazole-4-carbonitrile (12 mg, 0.13 mmol). After stirring at 25°C for 14 h, the mixture was added water (20 mL) and extracted with EtOAc (2 x 30 mL). The organic layer was separated, dried over Na₂SO₄, filtered and concentrated. The residue was purified by flash column (5~30% of EtOAc in PE) to give **V-A10** (19 mg, 52.9%) as a solid.

**¹H NMR** (400 MHz, CDCl₃) δ_{H} 7.84 (s, 1H), 7.81 (s, 1H), 5.06-4.88 (m, 2H), 2.69 (t, *J*=9.2 Hz, 1H), 2.04-1.95 (m, 1H), 1.93-1.82 (m, 2H), 1.79-1.61 (m, 5H), 1.55-1.41 (m, 8H), 1.38-1.14 (m, 10H), 1.08-0.95 (m, 2H), 0.86 (s, 3H), 0.69 (s, 3H); **LC-ELSD** purity 99%, **MS** ESI calcd. for C₂₇H₃₉N₃O₂ [M+1]⁺ 437.3, found 438.3.

### EXAMPLE V-3: Biological Data

### Steroid Inhibition of TBPS Binding

In **Table V-2** below, A indicates a **TBPS IC₅₀ (µM)** < 0.01 µM, B indicates a TBPS IC₅₀ (µM) of 0.01 µM to < 0.1 µM, C indicates a TBPS IC₅₀ (µM) of 0.1 µM to < 1.0 µM, D indicates a TBPS IC₅₀ (µM) of 1.0 µM to < 10 µM, and E means ≥ 10 µM.

**Table V-2:**

| **Example** | **Compound ID** | **STRUCTURE** | **IC50 (µM)** |
|---|---|---|---|
| V-1 | V-**A8** | 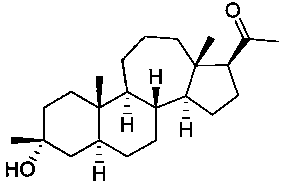 | C |
| V-2 | V**-A10** | 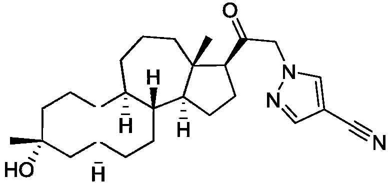 | C |

## Claims

1. A compound of Formula **(I-Ia):** 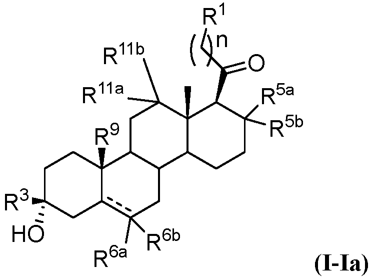
or a pharmaceutically acceptable salt thereof;
wherein:
R³ is substituted or unsubstituted C₁₋₃ alkyl;
R⁹ is hydrogen or unsubstituted C₁₋₃ alkyl;
each of R^{5a}, R^{5b}, R^{6a}, R^{6b}, R^{11a}, and R^{11b} is hydrogen;
R¹ is substituted or unsubstituted alkyl, substituted or unsubstituted carbocyclyl, substituted or unsubstituted heterocyclyl, substituted or unsubstituted aryl, substituted or unsubstituted heteroaryl, -OR^{A1}, -SR^{A1}, -N(R^{A1})₂, -N(R^{A1}), -OC(=O)R^{A1}, -OC(=O)OR^{A1}, - OC(=O)SR^{A1}, -OC(=O)N(R^{A1})₂, -SC(=O)R^{A2}, -SC(=O)OR^{A1}, -SC(=O)SR^{A1}, -SC(=O)N(R^{A1})₂, -NHC(=O)R^{A1}, -NHC(=O)OR^{A1}, -NHC(=O)SR^{A1}, -NHC(=O)N(R^{A1})₂, -OS(=O)₂R^{A2}, -OS(=O)₂OR^{A1}, -S-S(=O)₂R^{A2}, -S-S(=O)₂OR^{A1}, -S(=O)R^{A2}, -SO₂R^{A2}, or -S(=O)₂OR^{A1}, wherein each instance of R^{A1} is independently hydrogen, substituted or unsubstituted alkyl, substituted or unsubstituted carbocyclyl, substituted or unsubstituted heterocyclyl, substituted or unsubstituted aryl, substituted or unsubstituted heteroaryl, or two R^{A1} groups are joined to form an substituted or unsubstituted heterocyclic or heteroaryl ring; and R^{A2} is substituted or unsubstituted alkyl, substituted or unsubstituted carbocyclyl, substituted or unsubstituted heterocyclyl, substituted or unsubstituted aryl, or substituted or unsubstituted heteroaryl; and
n is 0, 1, 2, or 3; and
wherein - - - - - - represents a single bond.

2. The compound or pharmaceutically acceptable salt of claim 1, wherein R³ is substituted C₁₋₃ alkyl, optionally methoxymethyl or ethoxymethyl.

3. The compound or pharmaceutically acceptable salt of claim 1, wherein R³ is unsubstituted C₁₋₃ alkyl, optionally methyl.

4. The compound or pharmaceutically acceptable salt of any one of claims 1-3, wherein R⁹ is hydrogen.

5. The compound or pharmaceutically acceptable salt of any one of claims 1-3, wherein R⁹ is methyl.

6. The compound or pharmaceutically acceptable salt of claim 1, wherein the compound of Formula (I-Ia) is a compound of Formula **(I-Ib)** 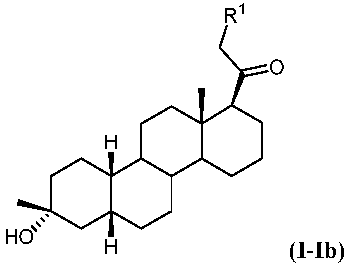 or a pharmaceutically acceptable salt thereof.

7. The compound or pharmaceutically acceptable salt of claim 1 or claim 6, wherein R¹ is 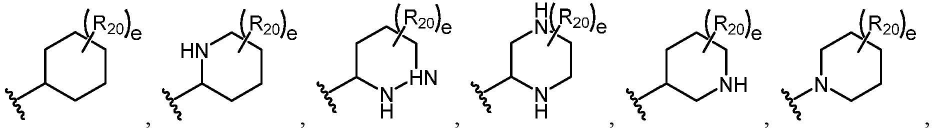 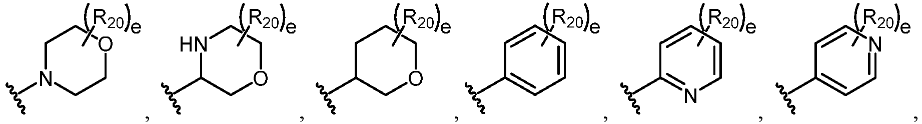 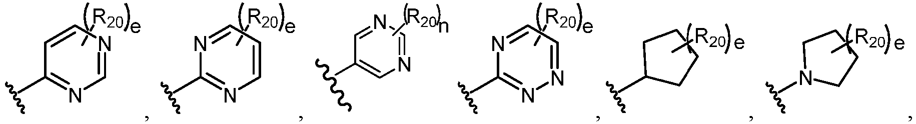 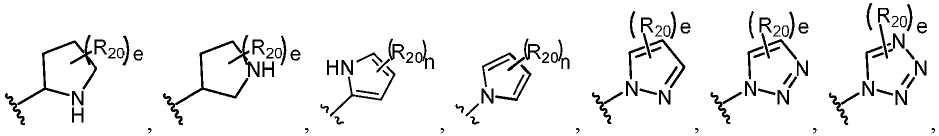 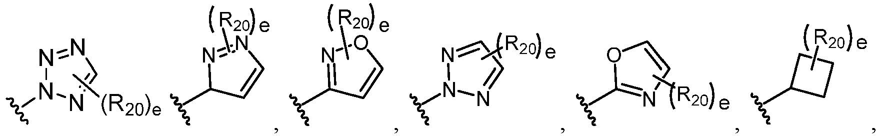 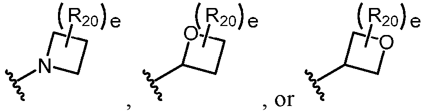 wherein each instance of R₂₀ is, independently, halogen, -NO₂, -CN, -OR^{GA}, -N(R^{GA})₂, -C(=O)R^{GA}, -C(=O)OR^{GA}, -OC(=O)R^{GA}, -OC(=O)OR^{GA}, -C(=O)N(R^{GA})₂, -N(R^{GA})C(=O)R^{GA}, -OC(=O)N(R^{GA})₂, -N(R^{GA})C(=O)OR^{GA}, -S(=O)₂R^{GA}, -S(=O)₂OR^{GA}, -OS(=O)₂R^{GA}, -S(=O)₂N(R^{GA})₂, or -N(R^{GA})S(=O)₂R^{GA}; substituted or unsubstituted C₁₋₆ alkyl, substituted or unsubstituted C₃₋₄ carbocylyl, or substituted or unsubstituted 3- to 4- membered heterocylyl;
wherein each instance of R^{GA} is independently hydrogen, substituted or unsubstituted C₁₋₆ alkyl, substituted or unsubstituted C₃₋₆ carbocylyl, substituted or unsubstituted 3- to 6- membered heterocylyl, substituted or unsubstituted aryl, substituted or unsubstituted heteroaryl, or two R^{GA} groups are taken with the intervening atoms to form a substituted or unsubstituted carbocyclic or heterocyclic ring; and
n or e is 0, 1, 2, 3, 4, or 5; optionally 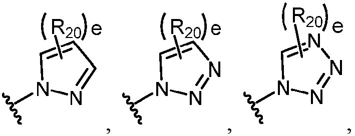 or 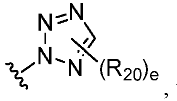 wherein each instance of R₂₀ is independently -CN or unsubstituted C₁₋₆ alkyl; and
e is 0 or 1.

8. The compound or pharmaceutically acceptable salt of claim 1, wherein the compound of Formula (**I-Ia**) is a compound of Formula (**I-Id**) 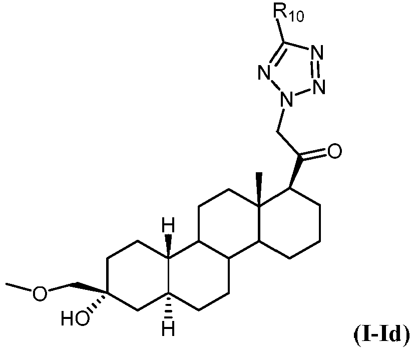 or a pharmaceutically acceptable salt thereof,
wherein R₁₀ is independently hydrogen, halogen, substituted or unsubstituted alkyl, hydroxyl, or cyano.

9. The compound or pharmaceutically acceptable salt of claim 1, wherein the compound of Formula (**I-Ia**) is a compound of Formula (**I-Ie**) 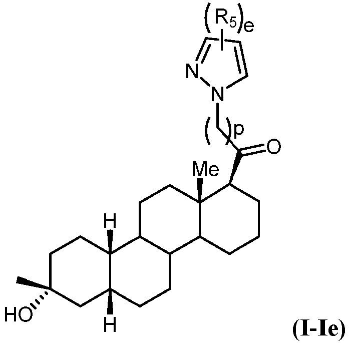
or a pharmaceutically acceptable salt thereof,
wherein e is 0, 1, 2 or 3;
p is 0, 1, 2 or 3; and
each R₅ is independently halogen, alkyl, hydroxyl, or cyano.

10. The compound or pharmaceutically acceptable salt of claim 1, wherein the compound of Formula (**I-Ia**) is a compound of Formula (**I-If**) 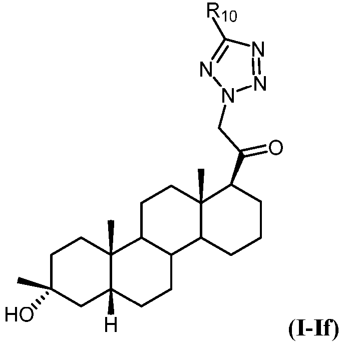 or a pharmaceutically acceptable salt thereof,
wherein R₁₀ is hydrogen, halogen, substituted or unsubstituted alkyl, hydroxyl, or cyano.

11. A compound selected from
| | |
|---|---|
| 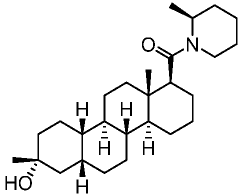 | **I-E4** |
| 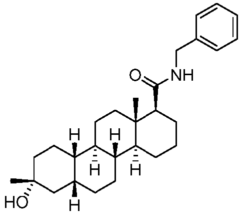 | **I-14** |
| 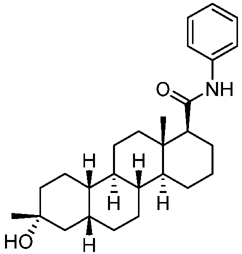 | **I-15** |
| 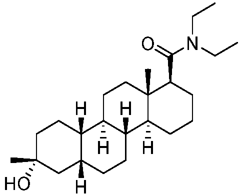 | **I-16** |
| 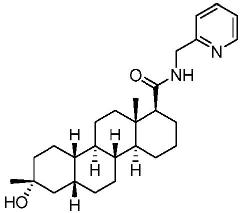 | **I-17** |
| 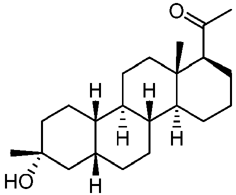 | **I-D7** |
| 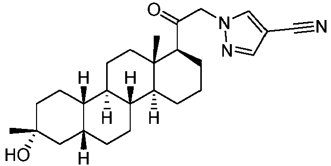 | **I-D9** |
| 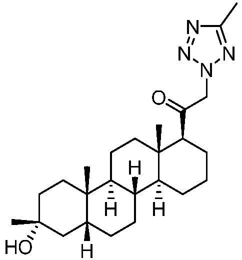 | **I-20** |
| 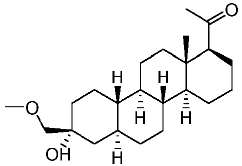 | **I-G14** |
| 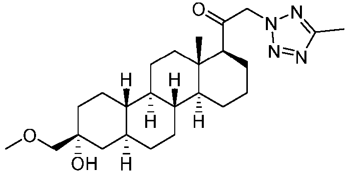 | **I-G16** |
| 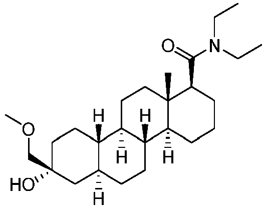 | **I-M3** |
| 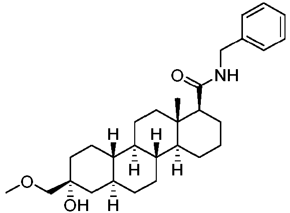 | **I-25** |
| 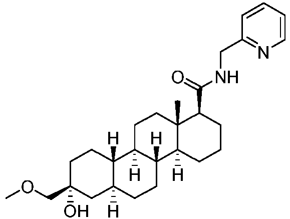 | **I-26** |
| 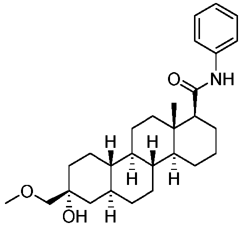 | **I-27** |
| 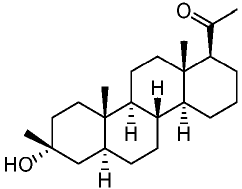 | **I-K2** |
| 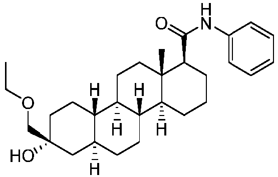 | **I-B11** |
| 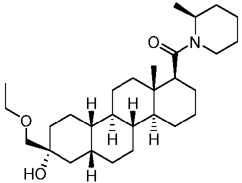 | **I-P9** |
| 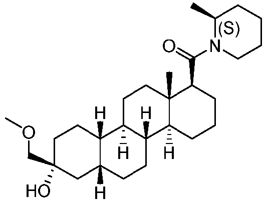 | **I-L14** |
| 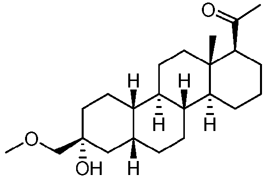 | **I-L17** |
| 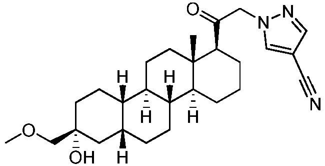 | **I-L19** |
| 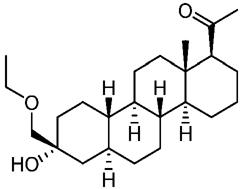 | **I-S3** |
| 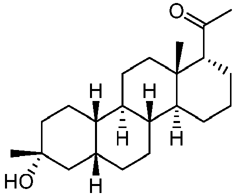 | **I-D7a** |
| 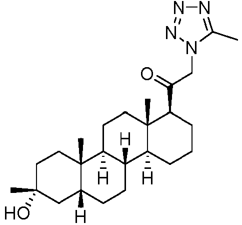 | **I-20a** |
| 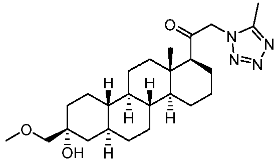 | **I-G16a** |
| 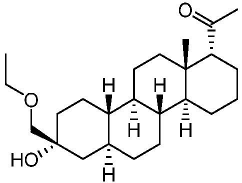 | **I-S3a** |
| 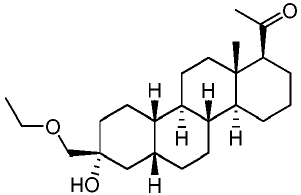 | **I-U11** |
or a pharmaceutically acceptable salt thereof.

12. A compound of claim 11, wherein the compound is selected from
| | |
|---|---|
| 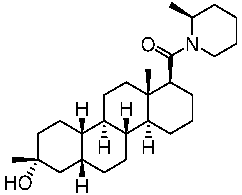 | **I-E4** |
| 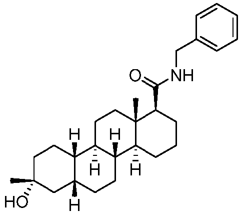 | **I-14** |
| 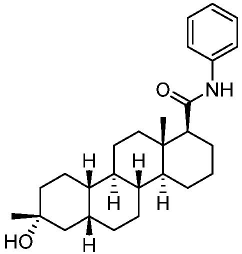 | **I-15** |
| 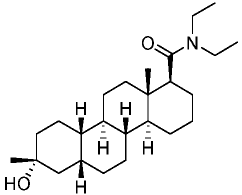 | **I-16** |
| 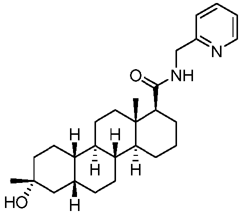 | **I-17** |
| 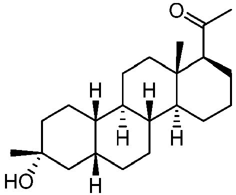 | **I-D7** |
| 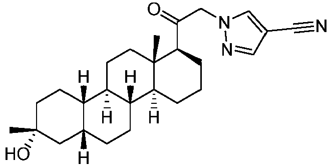 | **I-D9** |
| 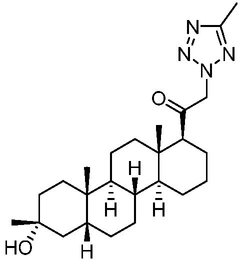 | **I-20** |
| 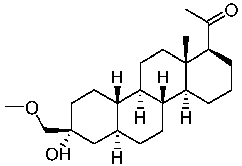 | **I-G14** |
| 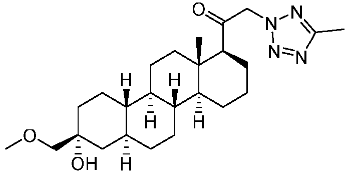 | **I-G16** |
| 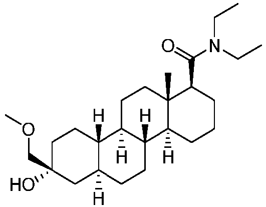 | **I-M3** |
| 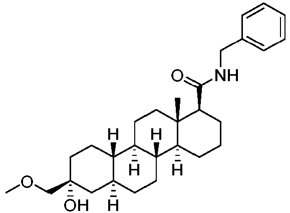 | **I-25** |
| 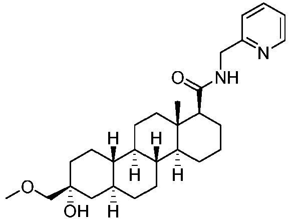 | **I-26** |
| 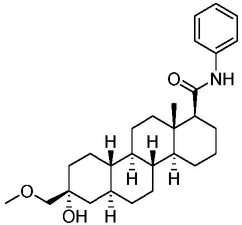 | **I-27** |
| 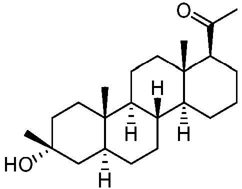 | **I-K2** |
| 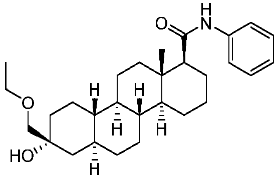 | **I-B11** |
| 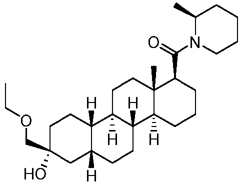 | **I-P9** |
| 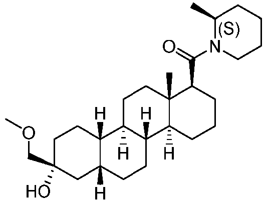 | **I-L14** |
| 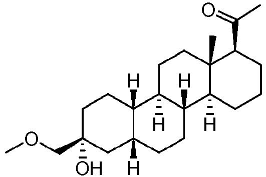 | **I-L17** |
| 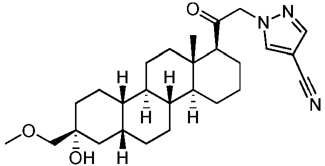 | **I-L19** |
| 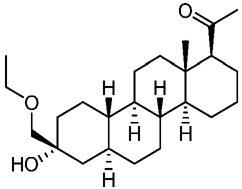 | **I-S3** |
| 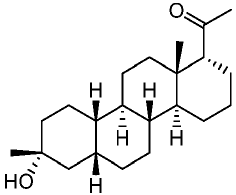 | **I-D7a** |
| 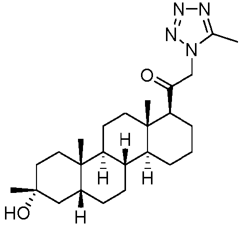 | **I-20a** |
| 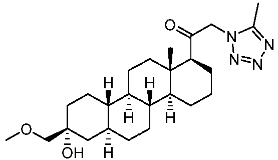 | **I-G16a** |
| 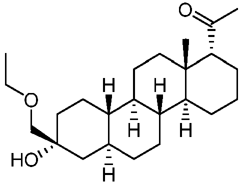 | **I-S3a** |
| 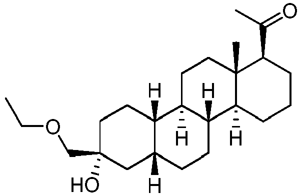 | **I-U11** |

13. A pharmaceutical composition comprising a compound or pharmaceutically acceptable salt of any one of claims 1-12, and a pharmaceutically acceptable excipient.

14. A compound or pharmaceutically acceptable salt of any one of claims 1-12, for use in treating a CNS-related disorder, wherein the CNS-related disorder is a sleep disorder, a mood disorder, a schizophrenia spectrum disorder, a convulsive disorder, a disorder of memory and/or cognition, a movement disorder, a personality disorder, autism spectrum disorder, pain, traumatic brain injury, a vascular disease, a substance abuse disorder and/or withdrawal syndrome, tinnitus, or status epilepticus.

15. A compound or pharmaceutically acceptable salt of any one of claims 1-12, for use according to claim 14, wherein the CNS-related disorder is depression.

16. A compound or pharmaceutically acceptable salt of any one of claims 1-12, for use according to claim 14, wherein the CNS-related disorder is postpartum depression.

17. A compound or pharmaceutically acceptable salt of any one of claims 1-12, for use according to claim 14, wherein the CNS-related disorder is major depressive disorder.

18. A compound or pharmaceutically acceptable salt of any one of claims 1-12, for use according to claim 17, wherein the major depressive disorder is moderate major depressive disorder.

19. A compound or pharmaceutically acceptable salt of any one of claims 1-12, for use according to claim 17, wherein the major depressive disorder is severe major depressive disorder.

## Patentansprüche

1. Verbindung der Formel (I-Ia): 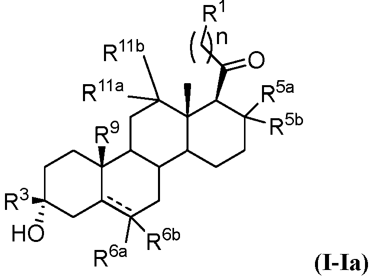 oder ein pharmazeutisch unbedenkliches Salz davon; wobei:
R³ für substituiertes oder unsubstituiertes C₁₋₃-Alkyl steht;
R⁹ für Wasserstoff oder unsubstituiertes C₁₋₃-Alkyl steht; R^{5a}, R^{5b}, R^{6a}, R^{6b}, R^{11a} und R^{11b} jeweils für Wasserstoff stehen;
R¹ für substituiertes oder unsubstituiertes Alkyl, substituiertes oder unsubstituiertes Carbocyclyl, substituiertes oder unsubstituiertes Heterocyclyl, substituiertes oder unsubstituiertes Aryl, substituiertes oder unsubstituiertes Heteroaryl, -OR^{A1}, - SR^{A1}, -N(R^{A1})₂, -N(R^{A1}), -OC(=O)R^{A1}, -OC(=O)OR^{A1}, - OC(=O)SR^{A1}, -OC(=O)N(R^{A1})₂, -SC(=O)R^{A2}, -SC(=O)OR^{A1}, - SC (=O) SR^{A1}, -SC(=O)N(R^{A1})₂, -NHC(=O)R^{A1}, -NHC(=O)OR^{A1}, - NHC(=O)SR^{A1}, -NHC(=O)N(R^{A1})₂, -OS(=O)₂R^{A2}, -OS(=O)₂OR^{A1}, -S-S(=O)₂R^{A2}, -S-S(=O)₂OR^{A1}, -S(=O)R^{A2}, -SO₂R^{A2} oder -S(=O)₂OR^{A1} steht, wobei jedes Vorkommen von R^{A1} unabhängig für Wasserstoff, substituiertes oder unsubstituiertes Alkyl, substituiertes oder unsubstituiertes Carbocyclyl, substituiertes oder unsubstituiertes Heterocyclyl, substituiertes oder unsubstituiertes Aryl, substituiertes oder unsubstituiertes Heteroaryl steht oder zwei R^{A1}-Gruppen zusammen einen substituierten oder unsubstituierten heterocyclischen Ring oder Heteroarylring bilden und R^{A2} für substituiertes oder unsubstituiertes Alkyl, substituiertes oder unsubstituiertes Carbocyclyl, substituiertes oder unsubstituiertes Heterocyclyl, substituiertes oder unsubstituiertes Aryl oder substituiertes oder unsubstituiertes Heteroaryl steht; und
n für 0, 1, 2 oder 3 steht; und
wobei - - - - - - für eine Einfachbindung steht.

2. Verbindung oder pharmazeutisch unbedenkliches Salz nach Anspruch 1, wobei R³ für substituiertes C₁₋₃-Alkyl, gegebenenfalls Methoxymethyl oder Ethoxymethyl, steht.

3. Verbindung oder pharmazeutisch unbedenkliches Salz nach Anspruch 1, wobei R³ für unsubstituiertes C₁₋₃-Alkyl, gegebenenfalls Methyl, steht.

4. Verbindung oder pharmazeutisch unbedenkliches Salz nach einem der Ansprüche 1-3, wobei R⁹ für Wasserstoff steht.

5. Verbindung oder pharmazeutisch unbedenkliches Salz nach einem der Ansprüche 1-3, wobei R⁹ für Methyl steht.

6. Verbindung oder pharmazeutisch unbedenkliches Salz nach Anspruch 1, wobei es sich bei der Verbindung der Formel (I-Ia) um eine Verbindung der Formel (I-Ib) 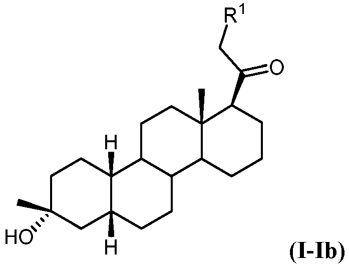 oder ein pharmazeutisch unbedenkliches Salz davon handelt.

7. Verbindung oder pharmazeutisch unbedenkliches Salz nach Anspruch 1 oder Anspruch 6, wobei R¹ für Folgendes steht: 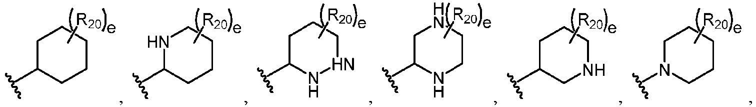 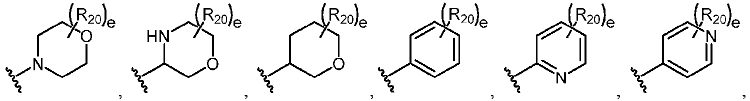 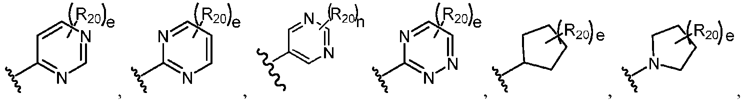 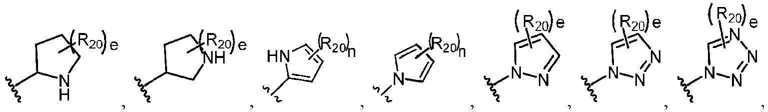 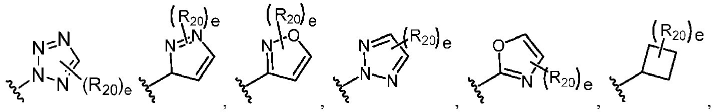 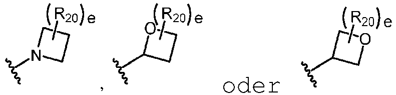
wobei jedes Vorkommen von R₂₀ unabhängig für Halogen, - NO₂, -CN, -OR^{GA}, -N(R^{GA})₂, -C(=O)R^{GA}, -C(=O) OR^{GA}, -OC(=O)R^{GA}, -OC(=O)OR^{GA}, -C(=O)N(R^{GA})₂, -N(R^{GA})C(=O)R^{GA}, -OC(=O)N(R^{GA})₂, -N(R^{GA})C(=O)OR^{GA}, -S(=O)₂R^{GA}, -S(=O)₂OR^{GA}, -OS(=O)₂R^{GA}, - S(=O)₂N(R^{GA})₂ oder -N(R^{GA})S(=O)2R^{GA}; substituiertes oder unsubstituiertes C₁₋₆-Alkyl, substituiertes oder unsubstituiertes C₃₋₄-Carbocylyl oder substituiertes oder unsubstituiertes 3- bis 4-gliedriges Heterocylyl steht; wobei jedes Vorkommen von R^{GA} unabhängig für Wasserstoff, substituiertes oder unsubstituiertes C₁₋₆-Alkyl, substituiertes oder unsubstituiertes C₃₋₆-Carbocylyl, substituiertes oder unsubstituiertes 3- bis 6-gliedriges Heterocyclyl, substituiertes oder unsubstituiertes Aryl, substituiertes oder unsubstituiertes Heteroaryl steht oder zwei R^{GA}-Gruppen zusammen mit den dazwischenliegenden Atomen einen substituierten oder unsubstituierten carbocyclischen oder heterocyclischen Ring bilden und
n oder e für 0, 1, 2, 3, 4 oder 5 steht; gegebenenfalls 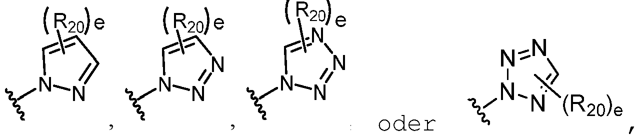 wobei jedes Vorkommen von R₂₀ unabhängig für -CN oder unsubstituiertes C₁₋₆-Alkyl steht und
e für 0 oder 1 steht.

8. Verbindung oder pharmazeutisch unbedenkliches Salz nach Anspruch 1, wobei es sich bei der Verbindung der Formel (I-Ia) um eine Verbindung der Formel (I-Id) 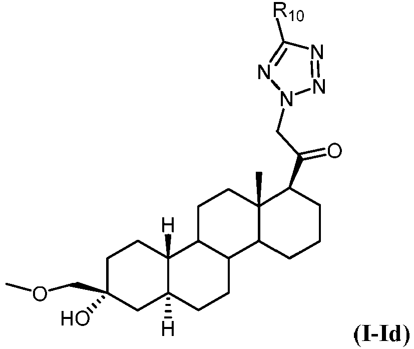 oder pharmazeutisch unbedenkliches Salz davon handelt, wobei R₁₀ jeweils unabhängig für Wasserstoff, Halogen, substituiertes oder unsubstituiertes Alkyl, Hydroxyl oder Cyano steht.

9. Verbindung oder pharmazeutisch unbedenkliches Salz nach Anspruch 1, wobei es sich bei der Verbindung der Formel (I-Ia) um eine Verbindung der Formel (I-Ie) 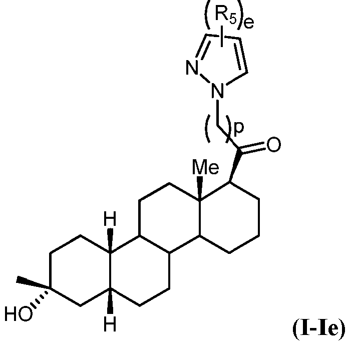
oder pharmazeutisch unbedenkliches Salz davon handelt,
wobei e für 0, 1, 2 oder 3 steht;
p für 0, 1, 2 oder 3 steht und
R₅ jeweils unabhängig für Halogen, Alkyl, Hydroxyl oder Cyano steht.

10. Verbindung oder pharmazeutisch unbedenkliches Salz nach Anspruch 1, wobei es sich bei der Verbindung der Formel (I-Ia) um eine Verbindung der Formel (I-If) 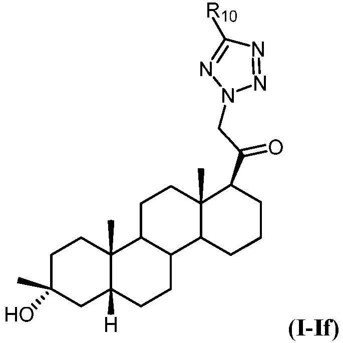 oder pharmazeutisch unbedenkliches Salz davon handelt, wobei R₁₀ für Wasserstoff, Halogen, substituiertes oder unsubstituiertes Alkyl, Hydroxyl oder Cyano steht.

11. Verbindung, ausgewählt aus
| | |
|---|---|
| | I-E4 |
| 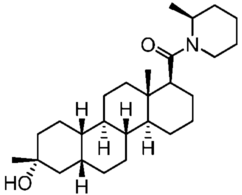 | |
| 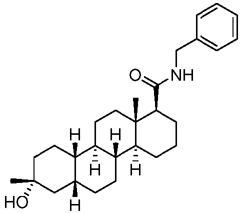 | I-14 |
| 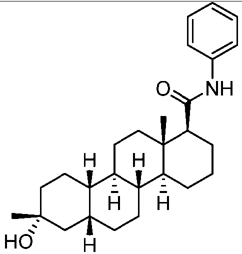 | I-15 |
| 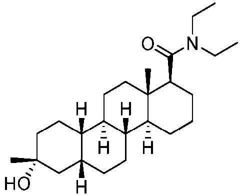 | I-16 |
| 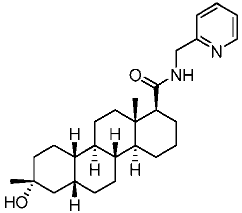 | I-17 |
| 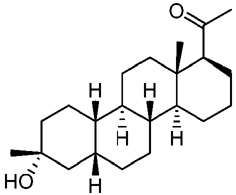 | I-D7 |
| 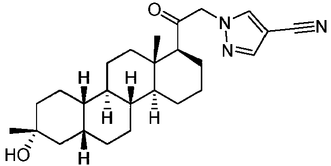 | I-D9 |
| 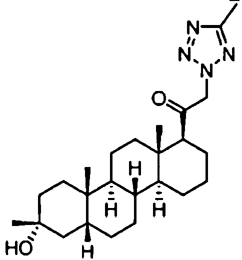 | I-20 |
| 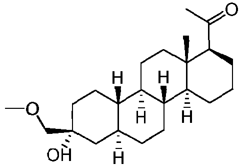 | I-G14 |
| 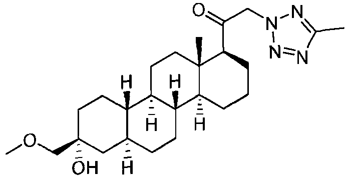 | I-G16 |
| 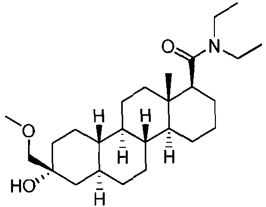 | I-M3 |
| 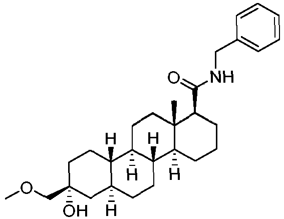 | I-25 |
| 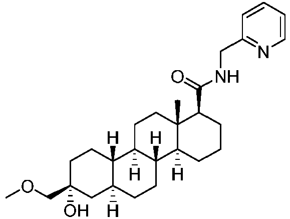 | I-26 |
| 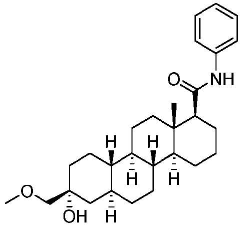 | I-27 |
| 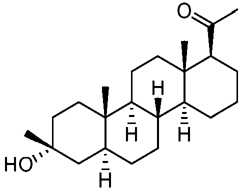 | I-K2 |
| 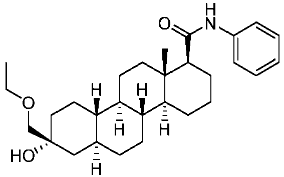 | I-B11 |
| 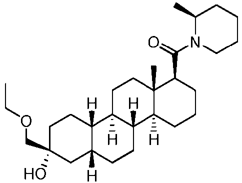 | I-P9 |
| 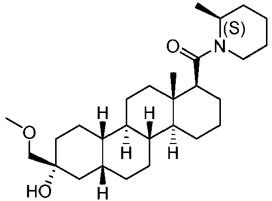 | I-L14 |
| 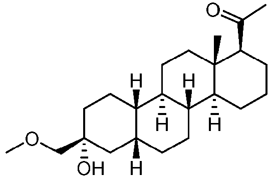 | I-L17 |
| 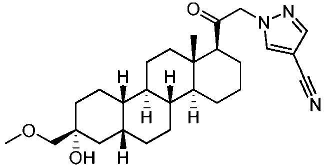 | I-L19 |
| 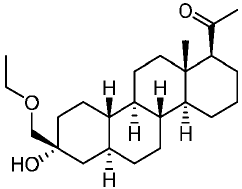 | I-S3 |
| 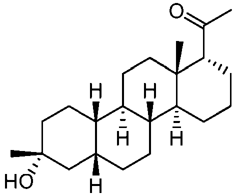 | I-D7a |
| 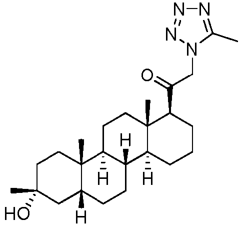 | I-20a |
| 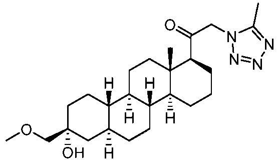 | I-G16a |
| 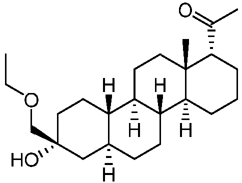 | I-S3a |
| 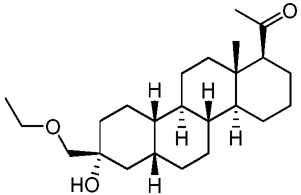 | I-U11 |
oder ein pharmazeutisch unbedenkliches Salz davon handelt.

12. Verbindung nach Anspruch 11, wobei die Verbindung ausgewählt ist aus
| | |
|---|---|
| 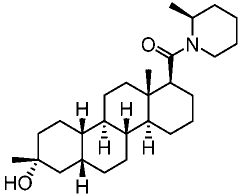 | I-E4 |
| 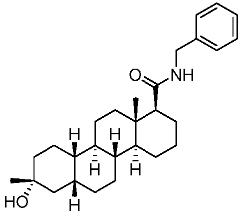 | I-14 |
| 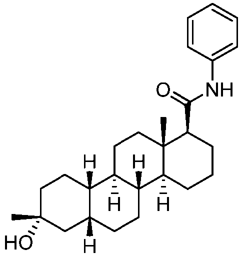 | I-15 |
| 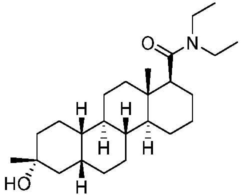 | I-16 |
| 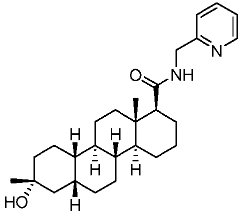 | I-17 |
| 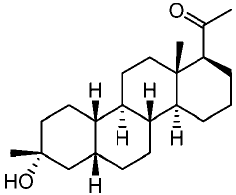 | I-D7 |
| 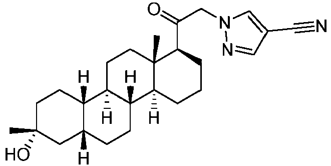 | I-D9 |
| 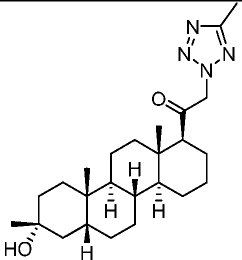 | I-20 |
| 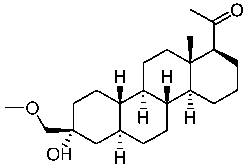 | I-G14 |
| 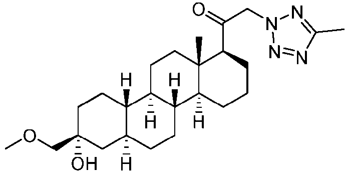 | I-G16 |
| 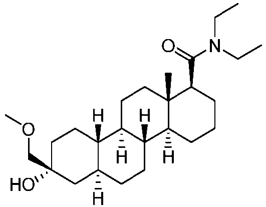 | I-M3 |
| 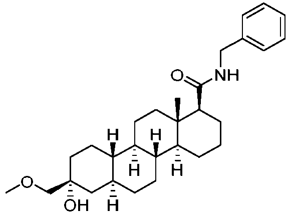 | I-25 |
| 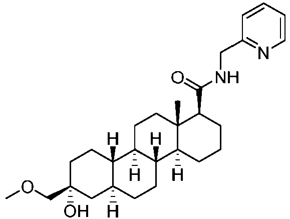 | I-26 |
| 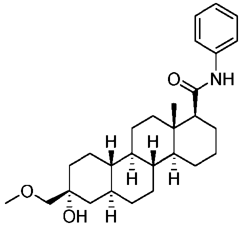 | I-27 |
| 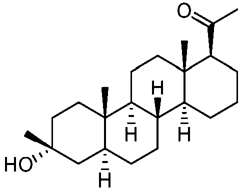 | I-K2 |
| 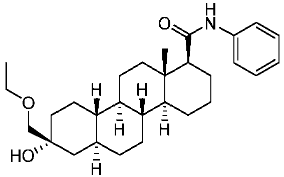 | I-B11 |
| 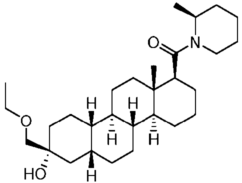 | I-P9 |
| 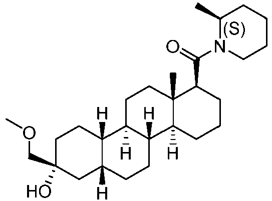 | I-L14 |
| 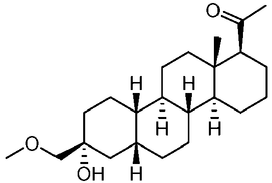 | I-L17 |
| 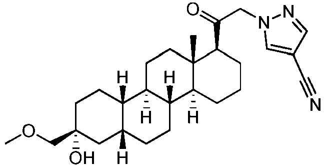 | I-L19 |
| 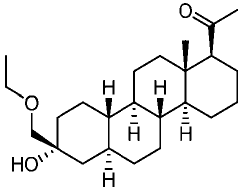 | I-S3 |
| 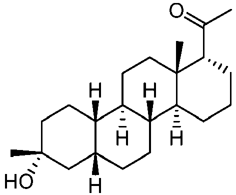 | I-D7a |
| 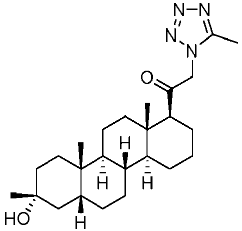 | I-20a |
| 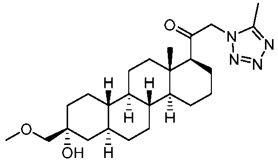 | I-G16a |
| 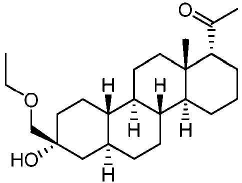 | I-S3a |
| 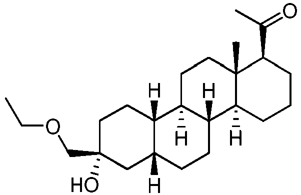 | I-U11 |

13. Pharmazeutische Zusammensetzung, umfassend eine Verbindung oder ein pharmazeutisch unbedenkliches Salz nach einem der Ansprüche 1-12 und einen pharmazeutisch unbedenklichen Hilfsstoff.

14. Verbindung oder pharmazeutisch unbedenkliches Salz nach einem der Ansprüche 1-12 zur Verwendung bei der Behandlung einer mit dem ZNS in Zusammenhang stehenden Störung, wobei es sich bei der mit dem ZNS in Zusammenhang stehenden Störung um eine Schlafstörung, eine affektive Störung, eine Störung des Schizophrenie-Spektrums, eine konvulsive Störung, eine Gedächtnis- und/oder Kognitionsstörung, eine Bewegungsstörung, eine Persönlichkeitsstörung, eine Störung des Autismus-Spektrums, Schmerzen, eine traumatische Hirnverletzung, eine Gefäßerkrankung, eine Substanzmissbrauchsstörung und/oder ein Entzugssyndrom, Tinnitus oder Status epilepticus handelt.

15. Verbindung oder pharmazeutisch unbedenkliches Salz nach einem der Ansprüche 1-12 zur Verwendung nach Anspruch 14, wobei es sich bei der mit dem ZNS in Zusammenhang stehenden Störung um Depression handelt.

16. Verbindung oder pharmazeutisch unbedenkliches Salz nach einem der Ansprüche 1-12 zur Verwendung nach Anspruch 14, wobei es sich bei der mit dem ZNS in Zusammenhang stehenden Störung um postpartale Depression handelt.

17. Verbindung oder pharmazeutisch unbedenkliches Salz nach einem der Ansprüche 1-12 zur Verwendung nach Anspruch 14, wobei es sich bei der mit dem ZNS in Zusammenhang stehenden Störung um majore depressive Störung handelt.

18. Verbindung oder pharmazeutisch unbedenkliches Salz nach einem der Ansprüche 1-12 zur Verwendung nach Anspruch 17, wobei es sich bei der majoren depressiven Störung um moderate majore depressive Störung handelt.

19. Verbindung oder pharmazeutisch unbedenkliches Salz nach einem der Ansprüche 1-12 zur Verwendung nach Anspruch 17, wobei es sich bei der majoren depressiven Störung um schwere majore depressive Störung handelt.

## Revendications

1. Composé de formule (I-Ia) : 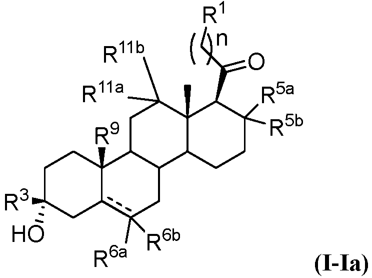 ou un sel pharmaceutiquement acceptable de celui-ci ; dans laquelle :
R³ représente un groupe alkyle en C₁₋₃ substitué ou non substitué ;
R⁹ représente un atome d'hydrogène ou un groupe alkyle en C₁₋₃ non substitué ;
chacun de R^{5a}, R^{5b}, R^{6a}, R^{6b}, R^{11a} et R^{11b} représente un atome d'hydrogène ;
R¹ représente un groupe alkyle substitué ou non substitué, carbocyclyle substitué ou non substitué, hétérocyclyle substitué ou non substitué, aryle substitué ou non substitué, hétéroaryle substitué ou non substitué, -OR^{A1}, -SR^{A1}, -N(R^{A1})₂, -N(R^{A1}), -OC(=O)R^{A1}, -OC(=O)OR^{A1}, - OC (=O) SR^{A1}, -OC(=O)N(R^{A1})₂, -SC (=O) R^{A2}, -SC(=O)OR^{A1}, - SC(=O)SR^{A1}, -SC(=O)N(R^{A1})₂, -NHC(=O)R^{H1}, -NHC(=O)OR^{A1}, - NHC (=O) SR^{A1}, -NHC(=O)N(R^{A1})₂, -OS(=O)₂R^{A2}, -OS(=O)₂OR^{A1}, -S-S(=O)₂R^{A2}, -S-S(=O)₂OR^{A1}, -S(=O)R^{A2}, -SO₂R^{A2} ou -S(=O)₂OR^{A1}, dans laquelle chaque occurrence de R^{A1} représente indépendamment un atome d'hydrogène, un groupe alkyle substitué ou non substitué, carbocyclyle substitué ou non substitué, aryle substitué ou non substitué, hétéroaryle substitué ou non substitué, ou deux groupes R^{A1} sont reliés pour former un cycle hétérocyclique ou hétéroaryle substitué ou non substitué ; et R^{A2} représente un groupe alkyle substitué ou non substitué, carbocyclyle substitué ou non substitué, hétérocyclyle substitué ou non substitué, aryle substitué ou non substitué ou hétéroaryle substitué ou non substitué ; et
n est 0, 1, 2 ou 3 ; et
dans laquelle - - - - - - représente une simple liaison.

2. Composé ou sel pharmaceutiquement acceptable selon la revendication 1, dans lequel R³ représente un groupe alkyle en C₁₋₃ substitué, facultativement méthoxyméthyle ou éthoxyméthyle.

3. Composé ou sel pharmaceutiquement acceptable selon la revendication 1, dans lequel R³ représente un groupe alkyle en C₁₋₃ non substitué, facultativement méthyle.

4. Composé ou sel pharmaceutiquement acceptable selon l'une quelconque des revendications 1 à 3, dans lequel R⁹ représente un atome d'hydrogène.

5. Composé ou sel pharmaceutiquement acceptable selon l'une quelconque des revendications 1 à 3, dans lequel R⁹ représente un groupe méthyle.

6. Composé ou sel pharmaceutiquement acceptable selon la revendication 1, le composé de formule (I-Ia) étant un composé de formule (I-Ib) 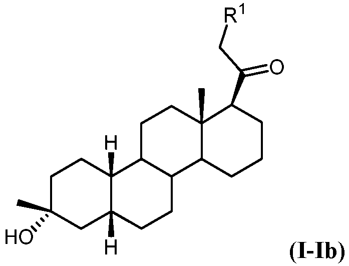 ou un sel pharmaceutiquement acceptable de celui-ci.

7. Composé ou sel pharmaceutiquement acceptable selon la revendication 1 ou la revendication 6, dans lequel R¹ représente 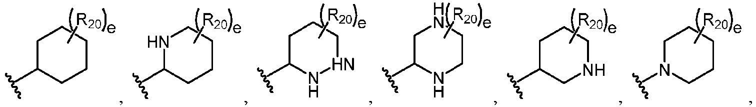 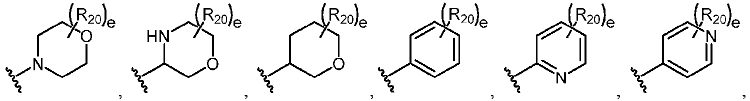 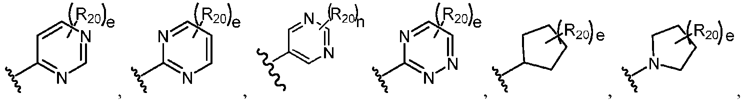 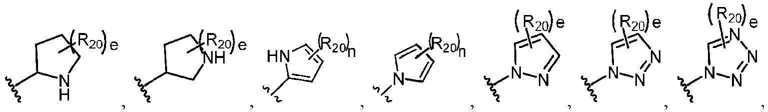 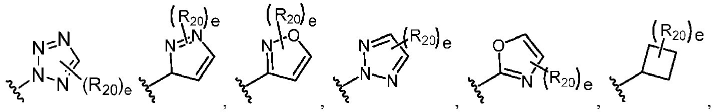 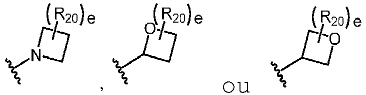
dans lequel chaque occurrence de R₂₀ représente indépendamment un atome d'halogène, -NO₂, -CN, -OR^{GA}, - N(R^{GA})₂, -C (=O) R^{GA}, -C (=O) OR^{GA}, -OC (=O) R^{GA}, -OC(=O)OR^{GA}, - C(=O)N(R^{GA})₂, -N(R^{GA})C(=O)R^{GA}, -OC(=O)N(R^{GA})₂, - N(R^{GA})C(=O)OR^{GA}, -S(=O)₂R^{GA}, -S(=O)₂OR^{GA}, -OS(=O)₂R^{GA}, - S(=O)₂N(R^{GA})₂ ou -N(R^{GA})S(=O)₂R^{GA} ; un groupe alkyle en C₁-₆ substitué ou non substitué, carbocyclyle en C₃₋₄ substitué ou non substitué ou hétérocyclyle de 3 à 4 chaînons, substitué ou non substitué ;
dans lequel chaque occurrence de R^{GA} représente indépendamment un atome d'hydrogène, un groupe alkyle en C₁₋₆ substitué ou non substitué, carbocyclyle en C₃₋₆ substitué ou non substitué ou hétérocyclyle de 3 à 6 chaînons, aryle substitué ou non substitué, hétéroaryle substitué ou non substitué, ou deux groupes R^{GA}, avec les atomes intermédiaires, forment un cycle carbocyclique ou hétérocyclique substitué ou non substitué ; et
n ou e est 0, 1, 2, 3, 4 ou 5 ; facultativement 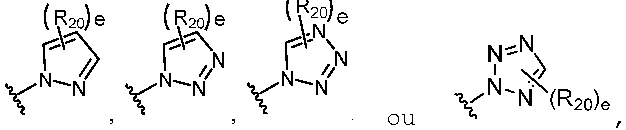 dans lequel chaque occurrence de R₂₀ représente indépendamment -CN ou un groupe alkyle en C₁₋₆ non substitué ; et
e est 0 ou 1.

8. Composé ou sel pharmaceutiquement acceptable selon la revendication 1, le composé de formule (I-Ia) étant un composé de formule (I-Id) 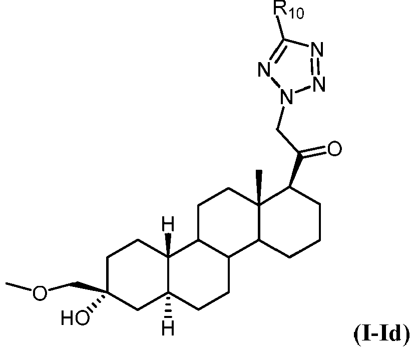 ou un sel pharmaceutiquement acceptable de celui-ci, dans lequel R₁₀ représente indépendamment un atome d'hydrogène, un atome d'halogène, un groupe alkyle substitué ou non substitué, hydroxyle ou cyano.

9. Composé ou sel pharmaceutiquement acceptable selon la revendication 1, le composé de formule (I-Ia) étant un composé de formule (I-Ie) 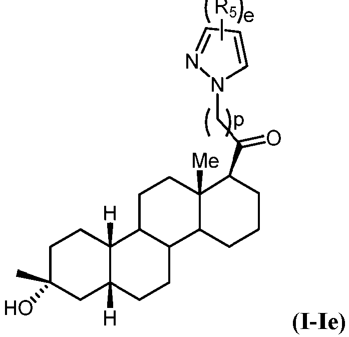
ou un sel pharmaceutiquement acceptable de celui-ci,
dans lequel e est 0, 1, 2 ou 3 ;
p est 0, 1, 2 ou 3 ; et
chaque R₅ représente indépendamment un atome d'halogène, un groupe alkyle, hydroxyle ou cyano.

10. Composé ou sel pharmaceutiquement acceptable selon la revendication 1, le composé de formule (I-Ia) étant un composé de formule (I-If) 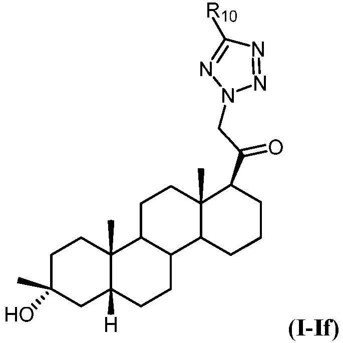
ou un sel pharmaceutiquement acceptable de celui-ci,
dans lequel R₁₀ représente indépendamment un atome d'hydrogène, un atome d'halogène, un groupe alkyle substitué ou non substitué, hydroxyle ou cyano.

11. Composé choisi parmi
| | |
|---|---|
| 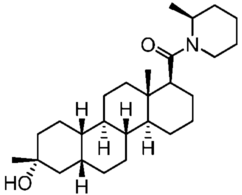 | I-E4 |
| 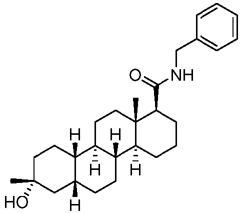 | I-14 |
| 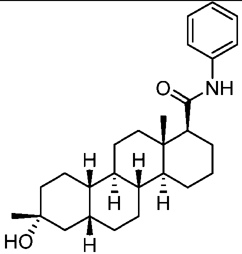 | I-15 |
| 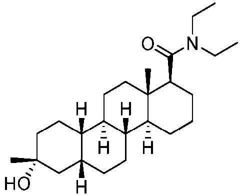 | I-16 |
| 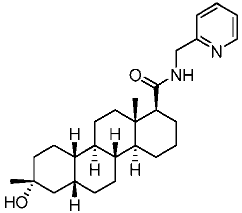 | I-17 |
| 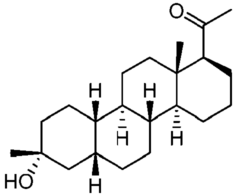 | I-D7 |
| 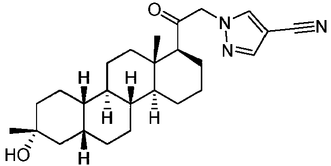 | I-D9 |
| 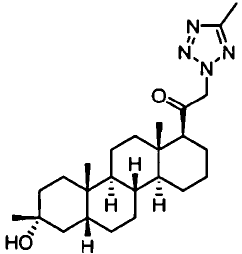 | I-20 |
| 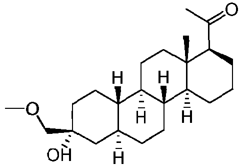 | I-G14 |
| 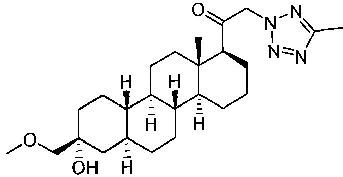 | I-G16 |
| 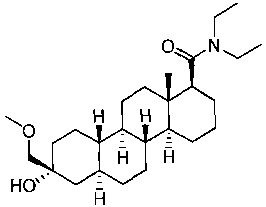 | I-M3 |
| 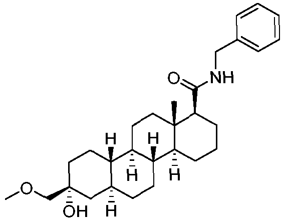 | I-25 |
| 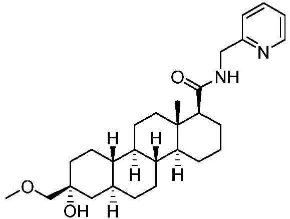 | I-26 |
| 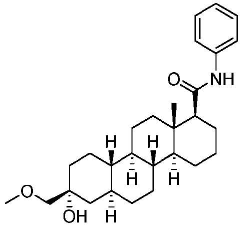 | I-27 |
| 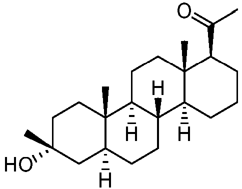 | I-K2 |
| 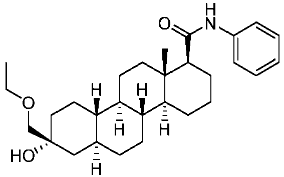 | I-B11 |
| 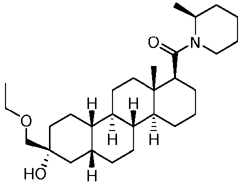 | I-P9 |
| 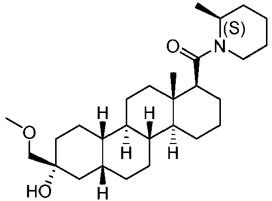 | I-L14 |
| 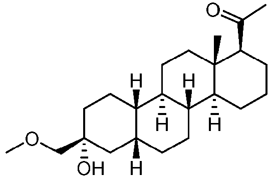 | I-L17 |
| 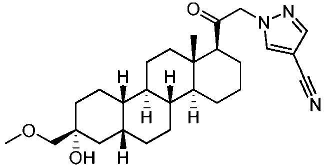 | I-L19 |
| 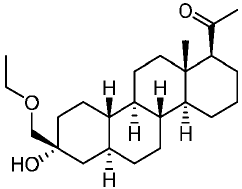 | I-S3 |
| 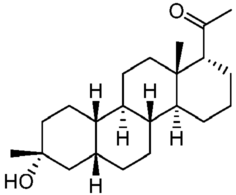 | I-D7a |
| 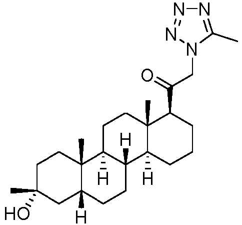 | I-20a |
| 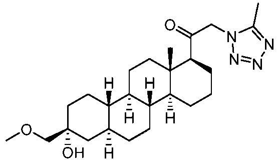 | I-G16a |
| 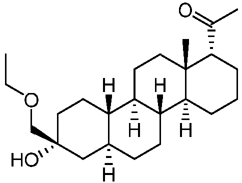 | I-S3a |
| 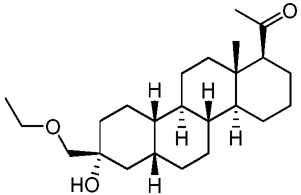 | I-U11 |
ou un sel pharmaceutiquement acceptable de celui-ci.

12. Composé selon la revendication 11, le composé étant choisi parmi :
| | |
|---|---|
| 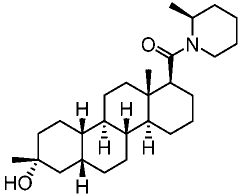 | I-E4 |
| 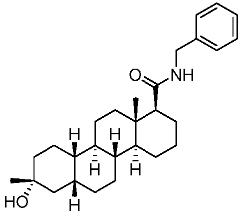 | I-14 |
| 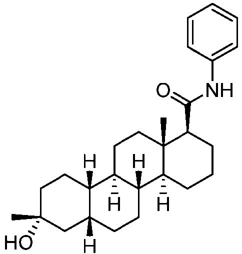 | I-15 |
| 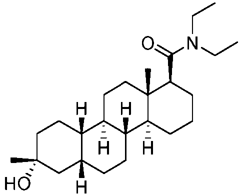 | I-16 |
| 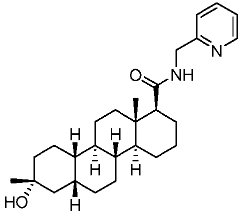 | I-17 |
| 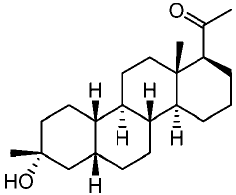 | I-D7 |
| 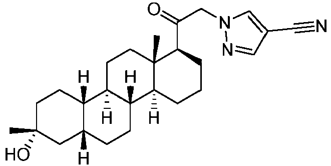 | I-D9 |
| 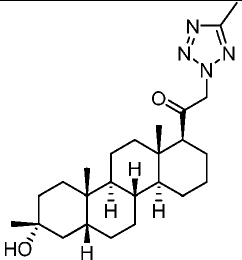 | I-20 |
| 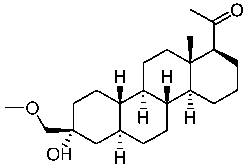 | I-G14 |
| 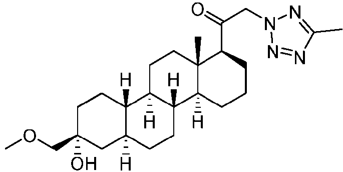 | I-G16 |
| 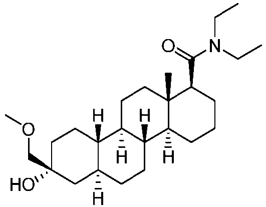 | I-M3 |
| 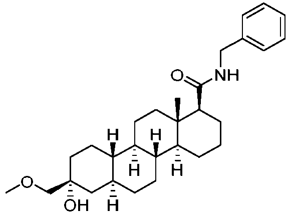 | I-25 |
| 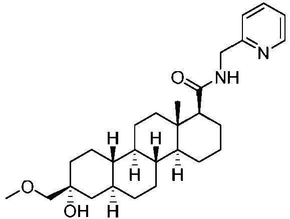 | I-26 |
| 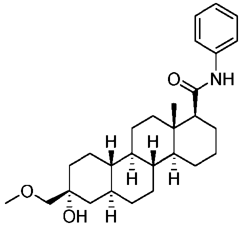 | I-27 |
| 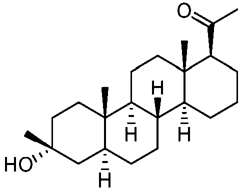 | I-K2 |
| 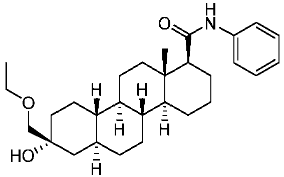 | I-B11 |
| 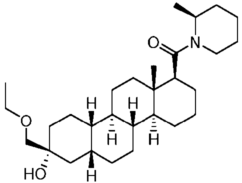 | I-P9 |
| 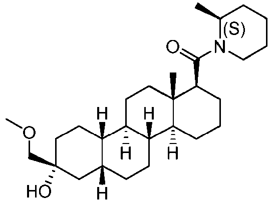 | I-L14 |
| 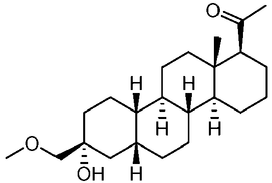 | I-L17 |
| 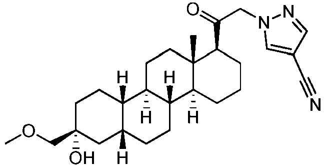 | I-L19 |
| 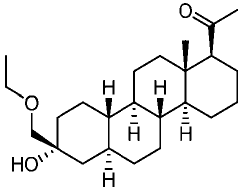 | I-S3 |
| 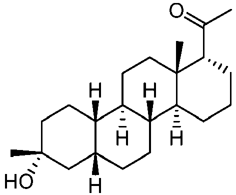 | I-D7a |
| 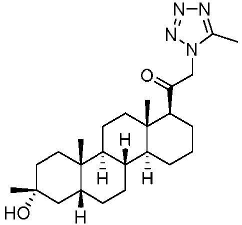 | I-20a |
| 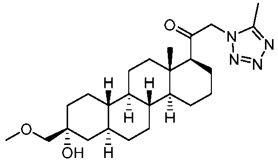 | I-G16a |
| 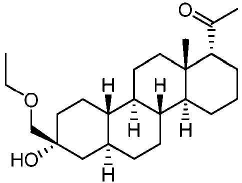 | I-S3a |
| 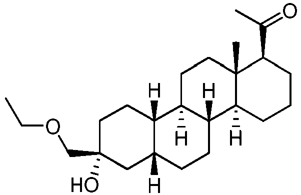 | I-U11 |

13. Composition pharmaceutique comprenant un composé ou un sel pharmaceutiquement acceptable selon l'une quelconque des revendications 1 à 12, et un excipient pharmaceutiquement acceptable.

14. Composé ou sel pharmaceutiquement acceptable selon l'une quelconque des revendications 1 à 12, pour une utilisation dans le traitement d'un trouble lié au SNC, le trouble lié au SNC étant un trouble du sommeil, un trouble de l'humeur, un trouble du spectre de la schizophrénie, un trouble convulsif, un trouble de la mémoire et/ou cognitif, un trouble du mouvement, un trouble de la personnalité, un trouble du spectre autistique, la douleur, une lésion traumatique du cerveau, une maladie vasculaire, un trouble lié à l'abus de substances et/ou un syndrome de sevrage, des acouphènes ou un état de mal épileptique.

15. Composé ou sel pharmaceutiquement acceptable correspondant selon l'une quelconque des revendications 1 à 12, pour une utilisation selon la revendication 14, le trouble lié au SNC étant la dépression.

16. Composé ou sel pharmaceutiquement acceptable correspondant selon l'une quelconque des revendications 1 à 12, pour une utilisation selon la revendication 14, le trouble lié au SNC étant la dépression.

17. Composé ou sel pharmaceutiquement acceptable correspondant selon l'une quelconque des revendications 1 à 12, pour une utilisation selon la revendication 14, le trouble lié au SNC étant une dépression post-partum ou un trouble dépressif majeur.

18. Composé ou sel pharmaceutiquement acceptable selon l'une quelconque des revendications 1 à 12, pour une utilisation selon la revendication 17, le trouble dépressif majeur étant un trouble dépressif majeur modéré.

19. Composé ou sel pharmaceutiquement acceptable correspondant selon l'une quelconque des revendications 1 à 12, pour une utilisation selon la revendication 17, le trouble dépressif majeur étant un trouble dépressif majeur sévère.
